# EUROPEAN PATENT APPLICATION

(11) **EP 4 530 355 A2**
(43) Date of publication of application: **02.04.2025**
(21) Application number: 24214942.5
(22) Date of filing: 12.10.2022
(51) Int. Cl.: C12N 15/867

(54) **LENTIVIRAL VECTORS**

(30) Priority: 12.10.2021 GB 202114529
(62) Divisional of application: 22790351.5
(71) Applicant: Oxford BioMedica (UK) Limited, Oxford, OX4 6LT (GB)
(72) Inventor: FARLEY, Daniel, Oxford, OX4 6LT (GB); WRIGHT, Jordan, Oxford, OX4 6LT (GB); NOGUEIRA, Cristina, Oxford, OX4 6LT (GB)
(74) Representative: D Young & Co LLP

(57) **Abstract**

The invention relates to the production of lentiviral vectors. More specifically, the present invention relates to nucleotide sequences encoding a lentiviral vector genome which comprises any one or more of a modified 3' LTR; a modified 5' LTR; a vector intron; at least one cis-acting sequence; and/or an interfering RNA. Methods and uses of such nucleotide sequences are also encompassed by the invention.

## Description

### FIELD OF THE INVENTION

The invention relates to lentiviral vectors designed to improve in their efficiency of production, transgene capacity, safety profile and utility in target cells. More specifically, the present invention relates to nucleotide sequences encoding a lentiviral vector genome which comprises any one or more of a modified 3' LTR; a modified 5' LTR; a vector intron; at least one cis-acting sequence; and/or an interfering RNA. The invention also relates to a lentiviral vector genome comprising any one or more of the modifications described above. Methods and uses involving such a nucleotide sequence or lentiviral vector genome are also encompassed by the invention.

### BACKGROUND TO THE INVENTION

The development and manufacture of viral vectors towards vaccines and human gene therapy over the last several decades is well documented in scientific journals and in patents. The use of engineered viruses to deliver transgenes for therapeutic effect is wide-ranging. Contemporary gene therapy vectors based on RNA viruses such as γ-retroviruses and lentiviruses (Muhlebach, M.D. et al., 2010, Retroviruses: Molecular Biology, Genomics *and* Pathogenesis, 13:347-370; Antoniou, M.N., Skipper, K.A. & Anakok, O., 2013, Hum. Gene Ther., 24:363-374), and DNA viruses such as adenovirus (Capasso, C. et al., 2014, Viruses, 6:832-855) and adeno-associated virus (AAV) (Kotterman, M.A. & Schaffer, D.V., 2014, Nat. Rev. Genet., 15:445-451) have shown promise in a growing number of human disease indications. These include *ex vivo* modification of patient cells for hematological conditions (Morgan, R.A. & Kakarla, S., 2014, Cancer J., 20:145-150; Touzot, F. et al., 2014, Expert Opin. Biol. Ther., 14:789-798), and *in vivo* treatment of ophthalmic (Balaggan, K.S. & Ali, R.R., 2012, Gene Ther., 19:145-153), cardiovascular (Katz, M.G. et al., 2013, Hum. Gene Ther., 24:914-927), neurodegenerative diseases (Coune, P.G., Schneider, B.L. & Aebischer, P., 2012, Cold Spring Harb. Perspect. Med., 4:a009431) and tumour therapy (Pazarentzos, E. & Mazarakis, N.D., 2014, Adv. Exp. Med Biol., 818:255-280).

As the underlying causes of many genetic diseases are being revealed, it is clear that the delivery of more functionality to the genetic payload (rather than a single gene) within vector genomes is becoming extremely desirable. Thus, there is expectation that transgene cassettes will become more complex, requiring the delivery of more functions, for example in delivering more genes, transgene control (e.g. gene switch systems or inverted transgene expression cassettes) or suicide switches.

The current 'limits' of lentiviral vector capacity have not changed significantly over the last 20 years, and remain in the region of ~7kb of transgene space when employing standard genome *cis*-acting sequences such as the typical packaging sequence, rev-response element (RRE) and post-transcriptional regulatory elements (PREs) such as that from the woodchuck hepatitis virus (wPRE). Intrinsically, some aspect of this restriction is defined by the size of the wild type HIV-1 genome of ~9.5kb from which these vector systems are derived. Generally, the specific titres of lentiviral vectors diminish substantially in proportion to their payload size over-and-above this 'limit'. Several aspects of lentiviral vectorology are likely to contribute to the limit: [1] steady-state pool of vector genomic RNA (vRNA) in the production cell, [2] efficiency of conversion of vRNA to dsDNA by reverse transcriptase, and [3] efficiency of nuclear import and/or integration into host DNA. The desire to minimize lentiviral vector backbone sequences has recently lead to attempts to alter the arrangement of existing *cis-*elements (Sertkaya et al., 2021; Vink et al., 2017) as well as the generation of novel genome configurations to minimize RRE and the packaging signal (WO 2021/181108 A1).

As discussed above, inverted transgene expression cassettes may be desirable. The principal problem with retroviral vectors carrying inverted transgene cassettes that are active during vector production is the production of long dsRNA that forms by base pairing between the viral RNA genome (vRNA) and the mRNA encoding the transgene. The presence of dsRNA within the production cell triggers innate dsRNA sensing pathways, such as those involving oligoadenylate synthetase-ribonuclease L (OAS-RNase L), protein kinase R (PKR), and interferon (IFN)/ melanoma differentiation-associated protein 5 (MDA-5). One solution to avoid this response is to knock-down or knock-out endogenous PKR in the LV production cell, or over-express protein factors shown to inhibit dsRNA sensing mechanisms, as indeed others have shown is possible (Hu et al. (2018) Gene Ther, 25: 454-472; Maetzig et al. (2010), Gene Ther. 17: 400-411; and Poling et al. (2017), RNA Biol. 14: 1570-1579). However, knock-down/-out of these factors may be laborious or difficult, or it may be impossible to achieve the required reduction/loss in activity, and over-expression of protein factors may alter other aspects of the vector production cell, such as viability/vitality, leading to generally less healthy vector production cells.

Retroviruses typically do not utilize strong polyA sequences because there needs to be a balance of transcriptional activity driven from the 5' LTR and efficient polyadenylation at the 3' LTR, despite the LTRs being identical in sequence. U3-deleted LTRs have been shown to have less polyadenylation activity compared to wild-type, non-U3-deleted LTRs (Yang et al. (2007), Retrovirology 4:4), indicating that SIN-LTRs within LVs would be limited in the same fashion.

There are several consequences of weak polyadenylation sites within the LTRs of LVs, such as SIN-LTR-containing LVs. In summary, transcriptional read-out of the vector genome expression cassette and/or transgene expression cassette through the polyA sequence within the 3' LTR into downstream sequences is not efficiently prevented at either the vRNA stage (i.e. in the vector production cell) or the transgene mRNA transcription stage (i.e. in the transduced cell). In addition, transcriptional read-in from cellular genes through the polyA sequence within the 5' LTR into the vector genome expression cassette is not efficiently prevented at the transgene mRNA transcription stage (i.e. in the transduced cell). Transcriptional read-out and read-in each have deleterious consequences.

In view of the above, there is an ever-present need in the art for viral vectors with improved safety profiles in administration to patients (for example, in the context of vaccination and gene therapy), and/or for improved viral vectors for larger payloads (whilst maintaining suitable titre and safety profiles) and/or for viral vectors with improved efficiency of production. In particular, viral vectors with improved safety profiles, increased payload capacity and improved efficiency of production are urgently needed.

### SUMMARY OF THE INVENTION

The present invention is based on the development of lentiviral vectors (LVs) with improved safety profiles, increased payload capacity and/or improved efficiency of production.

As described further herein below, it is intended that one or more of the aspects of the lentiviral vectors according to the invention may be combined in the same vector. It is also intended that one or more of the aspects of the invention may be combined during the production of the same lentiviral vector. Each of the aspects may be used either alone, for example to achieve a particular effect or improvement, or in combination, for example to achieve one or more particular effects or improvements, as required.

### Improved safety profiles

In a first aspect, the present inventors surprisingly found that employing modified polyadenylation (polyA) sequences within LV genome expression cassettes results in simplified production of vector genomic RNA for packaging, improved transgene expression and reduced transcriptional read-in and -out (both of the vector genome expression cassette and transgene expression cassette) in transduced cells. The use of the modified polyA sequences of the invention is particularly advantageous for LVs flanked by SIN-LTRs due to the reduced polyadenylation activity in SIN-LTRs. The modified polyA sequences of the invention lead to efficient polyadenylation and thus reduced transcriptional read-in and -out of the LV genome expression cassette. The ability to modify polyadenylation sequences and thereby reduce transcriptional read-in and -out of the LV genome expression cassette offers safety advantages over current LV systems.

A foundational element to the invention is, in effect, re-positioning of a polyadenylation signal (PAS) across the U3/R boundary within the 3' LTR such that the PAS is copied from the 3' LTR to the 5' LTR during integration of LVs. Thus, the R region is embedded within the modified polyA sequence. The resulting efficient polyadenylation at the modified polyadenylation sequence will occur at the vRNA 3' polyA cleavage site, which will be located at the 3' end of the embedded R region sequence. Sufficient homology (~20 nucleotides of homology) between the R regions at both 5' and 3' ends of the vRNA is provided to allow for efficient first strand transfer during reverse transcription.

The inventors surprisingly found that this modified polyA sequence configuration can be employed to improve transcription termination at the 3' LTR, whilst simultaneously ensuring that vRNA cleavage (prior to polyadenylation) allows sufficient R homology with the 5' R region to retain first strand synthesis. When such modified polyA sequences are used in this context, it is found that the requirement for a back-up heterologous polyA sequence downstream of the vRNA expression cassette is avoided. This permits minimization and/or simplification of LV genome constructs (e.g. plasmids) used during vector production.

Further synthetic versions of the modified R-embedded heterologous polyA sequences of the invention can be made by pairing different USEs inserted upstream of the PAS and embedded R sequence with different GU-rich DSE elements inserted downstream of the embedded R sequence. Whilst the USE-PAS sequence residing within the 3' U3 region will be copied to the 5' LTR upon integration, the heterologous GU-rich DSE will not be copied. Therefore, to provide the PAS with an efficient DSE in a close position within the LTRs of the integrated LV genome cassette, the 5' R region of the vRNA is engineered to contain a GU-rich sequence that functions as a DSE in the recapitulated LTRs. Therefore, after reverse transcription and the LTR-copying process, the USE-PAS sequence residing within the U3 region in both 5' and 3' LTRs will be 'serviced' by this new DSE (i.e. the DSE will act upon the USE-PAS sequence). Optionally, the DSE-modified R sequence can also be employed at the 3' LTR as part of a synthetic R-embedded heterologous polyA sequence, functioning as the DSE for the 3' polyA sequence as well.

Altogether, these improvements are referred to herein as "sequence-upgraded pA LTRs (supA-LTRs)". The supA-LTRs impart improved transcriptional termination to the transgene cassette in target cells (i.e. reduced transcriptional 'read-out') and reduced transcriptional 'read-in' from upstream cellular promoters, leading to reduced mobilisation of vRNA backbone sequences, and reduced likelihood of transgene cassette interference. In the preferred combination of sequences, the native HIV-1 PAS can be functionally mutated since transcriptional termination within the supA-LTRs is no longer dependent on any HIV-1 sequences present. This ensures that no premature transcription termination is possible in the LV expression cassettes employing the supA-LTRs.

Accordingly, in one aspect, the invention provides a nucleotide sequence comprising a lentiviral vector genome expression cassette, wherein the 3' LTR of the lentiviral vector genome comprises a modified polyadenylation sequence, and wherein the modified polyadenylation sequence comprises a polyadenylation signal which is 5' of the 3' LTR R region.

In a further aspect, the invention provides a nucleotide sequence comprising a lentiviral vector genome expression cassette, wherein the lentiviral vector genome comprises a modified 5' LTR, and wherein the R region of the modified 5' LTR comprises at least one polyadenylation downstream enhancer element (DSE).

### Increased payload capacity

In a second aspect, the present inventors have generated viral vectors with novel short *cis-*acting sequences in the 3' UTR of a transgene expression cassette. They have identified two novel short cis-acting sequences that can be introduced into the 3' UTR of a transgene expression cassette, either alone, or in combination. These novel short nucleotide sequences (and combinations thereof) can either be used in addition to traditional post-transcriptional regulatory elements (PREs e.g. from woodchuck hepatitis virus; wPRE) to boost transgene expression in target cells or to replace these longer PREs entirely, enabling increased transgene capacity whilst maintaining high levels of transgene expression in target cells.

The inventors have surprisingly found that Cytoplasmic Accumulation Region (CAR) sequences previously identified to function within 5'UTR sequences of heterologous mRNA provide enhanced gene expression when incorporated into the 3'UTR of a viral vector transgene expression cassette. Moreover, when located within the transgene expression cassette 3' UTR, the initially reported 160bp CAR sequence (composed of 16x repeats of a 10bp core sequence) could be further minimized to fewer than 16 repeats without loss of the benefit to transgene expression. Surprisingly, these CAR sequences are shown to enhance the transgene expression from transgene cassettes utilizing introns, as well as boosting expression from cassettes already containing a full length wPRE.

The inventors have also identified a minimal ZCCHC14 protein-binding sequence that can be incorporated into the 3' UTR of a transgene expression cassette to improve transgene expression. The inventors have shown that these minimal ZCCHC14 protein-binding sequences can be combined with the CAR sequences described herein to further enhance transgene expression.

The novel cis-acting sequences described herein can be used to minimize the size of functional cis-acting sequences of all viral vectors such that payloads can be increased and/or titres of vectors containing larger payloads can be improved, whilst maintaining transgene expression levels in target cells. The invention may therefore be employed [1] within viral vector genomes where 'cargo' space is not limiting, such that the novel cis-acting sequences further enhance expression of a transgene cassette containing another 3'UTR element, such as the wPRE, or [2] within viral vector genomes where cargo space is limiting (i.e. at or above or substantially above the packaging 'limit' of the viral vector system employed), where the novel cis-acting sequences may be used instead of a larger 3'UTR element, such as the wPRE, thus reducing vector genome size, whilst also imparting an increase to transgene expression in target cells compared to a vector genome lacking any 3'UTR cis-acting element.

Accordingly, in a further aspect, the invention provides a nucleotide sequence comprising a transgene expression cassette wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.

In a third aspect, the present invention is based on the concept of introducing an intron into the vector genome expression cassette in order to enable reduction of the viral backbone sequence. In this regard, the inventors have surprisingly found that introduction of such an intron facilitates removal of the rev-response element (RRE), which allows for more transgene capacity in the vector.

As the resulting vector genomic RNA (vRNA) packaged into vector virions does not contain the intronic sequence, this so-called 'Vector-Intron' (VI) is not counted against available 'space' on the vRNA. Thus, more space is available for transgene sequences. This new lentivirus (LV) genome configuration is simple to employ, surprisingly does not require rev or an exogenous vRNA-export factor, and may be a more attractive option in moving away from current LV genomes, since most other aspects of LV genome biology remains the same. Additionally, VI may be of further benefit as it is expected that, since the VI is not present in the final integrated LV genome, the potential for mobilisation of vRNA will be reduced compared to RRE-containing LVs, thereby improving the safety profile of the vector.

As described herein, dsRNA species may be formed during production of viral vectors comprising an inverted transgene expression cassette. The present invention solves this problem by providing LV genome expression cassettes that comprise transgene mRNA self-destabilization or self-decay elements, or transgene mRNA nuclear retention signals, that function to reduce the amount of dsRNA formed when the LV genome expression cassette comprises an inverted transgene expression cassette. The transgene mRNA self-destabilization or self-decay elements, or transgene mRNA nuclear retention signals are located within the VI in the LV genome expression cassettes.

The inventors have previously shown (see WO 2021/160993) that the MSD and cryptic splice donor (crSD) in stem loop 2 (SL2) of the HIV-1 packaging sequence within lentiviral vector genome expression cassettes can be extremely promiscuous, leading to aberrant splicing into transgene sequences and resulting in reduction in production of full length vRNA. Surprisingly, as much as 95% of the detectable cytoplasmic mRNA derived from the external promoter driving vRNA production is spliced depending on internal sequences. For efficient vector production, unspliced packageable vRNA is the most desirable product. In addition, the presence of the MSD in the vector backbone delivered in transduced (patient) cells has been shown by others to be utilised by the splicing machinery, when read-through transcription from upstream cellular promoters occurs (lentiviral vectors target active transcription sites), leading to potential aberrant splice-products with cellular exons. Functional ablation of the MSD and crSD appeared to ablate most of this aberrant splicing.

The same functional ablation of this aberrant splicing may be employed in the present invention in order to avoid unwanted spliced products (e.g. MSD to the VI splice acceptor). Moreover, it is surprisingly found that the ability of the VI to impart full RRE-independence of LV genomes is improved by the MSD mutation.

It has been found previously that potential titre losses associated with such mutations can be recovered by supplying a modified U1 snRNA that targets the packaging sequence of the vRNA (WO 2021/160993). Surprisingly, the present inventors also show that the VI is sufficient to recover LV titres without the need to employ the modified U1 snRNA. Therefore, the invention also encompasses use of the VI to increase titres of MSD-mutated LVs. Surprisingly, therefore, it appears that the VI feature and MSD/crSD mutations are functionally symbiotic in generating RRE-deleted LVs. Moreover, the previous finding that MSD-mutated LVs are less prone to transcriptional read-in from cellular gene transcription at the sites of integration is likely to be synergized with the reduced ability for mobilization of VI LV sequences as a consequence of the VI not being present in the final integrated cassette.

Accordingly, in a further aspect, the invention provides a nucleotide sequence comprising a lentiviral vector genome expression cassette, wherein the lentiviral vector genome expression cassette comprises a transgene expression cassette and a vector intron as described herein.

In one aspect, the present invention provides a nucleotide sequence comprising a lentiviral vector genome expression cassette, wherein:
i) the major splice donor site in the lentiviral vector genome expression cassette is inactivated;
ii) the lentiviral vector genome expression cassette does not comprise a rev-response element;
iii) the lentiviral vector genome expression cassette comprises a transgene expression cassette and a vector intron.

In a further aspect, the present invention provides a nucleotide sequence comprising a lentiviral vector genome expression cassette, wherein:
i) the major splice donor site in the lentiviral vector genome expression cassette is inactivated;
ii) the lentiviral vector genome expression cassette does not comprise a rev-response element;
iii) the lentiviral vector genome expression cassette comprises a transgene expression cassette and a vector intron; and
iv) when the transgene expression cassette is inverted with respect to the lentiviral vector genome expression cassette, the vector intron is not located between the promoter of the transgene expression cassette and the transgene.

In another aspect, the present invention provides a nucleotide sequence comprising a lentiviral vector genome expression cassette, wherein:
i) the major splice donor site in the lentiviral vector genome expression cassette is inactivated;
ii) the lentiviral vector genome expression cassette does not comprise a rev-response element;
iii) the lentiviral vector genome expression cassette comprises a transgene expression cassette and a vector intron; and
iv) when the transgene expression cassette is inverted with respect to the lentiviral vector genome expression cassette, the nucleotide sequence comprises a sequence as set forth in any of SEQ ID NOs: SEQ ID NOs: 2, 3, 4, 6, 7, and/or 8, and/or the sequences CAGACA, and/or GTGGAGACT.

In another aspect, the present invention provides a nucleotide sequence comprising a lentiviral vector genome expression cassette, wherein:
i) the major splice donor site in the lentiviral vector genome expression cassette is inactivated;
ii) the lentiviral vector genome expression cassette does not comprise a rev-response element; and
iii) the lentiviral vector genome expression cassette comprises a transgene expression cassette and a vector intron, and
iv) when the transgene expression cassette is inverted with respect to the lentiviral vector genome expression cassette, the 3' UTR of the transgene expression cassette comprises the vector intron.

In another aspect, the the present invention provides a nucleotide sequence comprising a lentiviral vector genome expression cassette, wherein:
i) the major splice donor site and cryptic splice donor site adjacent to the 3' end of the major splice donor site in the lentiviral vector genome expression cassette are inactivated;
ii) the lentiviral vector genome expression cassette does not comprise a rev-response element;
iii) the lentiviral vector genome expression cassette comprises a transgene expression cassette and a vector intron.

### Improved efficiency of production

In a fourth aspect, the present inventors surprisingly found that RNA interference (RNAi) targeting a nucleotide of interest (NOI) can be employed in retroviral vector production cells during production of retroviral vectors comprising the NOI without impeding effective expression of the NOI in target cells, the native pathway of virion assembly and the resulting functionality of the viral vector particles. This is not straightforward because the NOI expression cassette and the vector genome molecule that will be packaged into virions are operably linked. Thus, modification of the NOI expression cassette may have adverse consequences on the ability to produce the vector genome molecule in the cell.

As described above, expression of the transgene protein during retroviral vector production may have unwanted effects on vector virion assembly, vector virion activity, process yields and/or final product quality. Furthermore, the formation of double-stranded (ds) RNA (which typically results from opposed transcription within cells) triggers innate dsRNA sensing pathways within the cell leading to loss of *de novo* protein synthesis. If this occurs during retroviral vector production (e.g. when the retroviral vector genome comprises an inverted transgene expression cassette), this leads to a loss in expression of vector components, and consequently loss in titre.

The present inventors show that RNAi can be employed in retroviral vector production cells to suppress the expression of the NOI (i.e. transgene) during retroviral vector production in order to minimize unwanted effects of the transgene protein on vector virion assembly, vector virion activity, process yields and/or final product quality. Advantageously, the use of RNAi in retroviral vector production cells also permits the rescue of titres of retroviral vectors harbouring an actively transcribed inverted transgene cassette (wherein the transgene expression cassette is all or in part inverted with respect to the retroviral vector genome expression cassette). The inventors surprisingly found that RNAi can be employed during vector production to minimize/eliminate transgene mRNA but not vector genome RNA (vRNA) required for packaging. Thus, the present invention is particularly advantageous for the improved production of retroviral vectors harbouring an actively transcribed inverted transgene cassette.

Accordingly, the present invention provides a single approach to both mediating transgene repression and rescuing titres of vectors containing actively expressed inverted transgene cassettes by the use of RNAi to target the transgene mRNA during retroviral vector production.

Accordingly, in one aspect, the invention provides a nucleotide sequence encoding a lentiviral vector genome, wherein the 3' LTR of the lentiviral vector genome comprises a modified polyadenylation sequence, and wherein the modified polyadenylation sequence comprises a polyadenylation signal which is 5' of the 3' LTR R region.

In a further aspect, the invention provides a nucleotide sequence encoding a lentiviral vector genome, wherein the lentiviral vector genome comprises a modified 5' LTR, and wherein the R region of the modified 5' LTR comprises a polyadenylation downstream enhancer element (DSE).

In a further aspect, the invention provides a nucleotide sequence encoding a lentiviral vector genome, wherein the 3' LTR of the lentiviral vector genome is a modified 3' LTR as described herein and the 5' LTR of the lentiviral vector genome is a modified 5' LTR as described herein.

In a further aspect, the invention provides a nucleotide sequence comprising a lentiviral vector genome, wherein the lentiviral vector genome comprises a modified 3' LTR and a modified 5' LTR, wherein the modified 3' LTR comprises a modified polyadenylation sequence comprising a polyadenylation signal which is 5' of the R region within the 3'LTR and wherein the R region within the 3' LTR comprises a polyadenylation DSE, and wherein the modified 5' LTR comprises a modified polyadenylation sequence comprising a polyadenylation signal which is 5' of the R region within the 5'LTR and wherein the R region within the 5' LTR comprises a polyadenylation DSE.

### Improved safety profiles, increased payload capacity and/or improved efficiency of production

The present inventors have surprisingly found that all of the above aspects of the invention can be used in combination. In particular, two or more aspects of the invention may be used in combination whilst maintaining suitable titre during lentiviral vector production and high levels of transgene expression in target cells. Advantageously, this provides a lentiviral vector having the improvements associated with each individual aspect of the invention which are utilised, i.e. a lentiviral vector having the corresponding improved properties associated with the relevant aspect of the invention. Thus, the present invention provides lentiviral vectors having improved safety profiles and increased payload capacity, improved safety profiles and improved efficiency of production, increased payload capacity and improved efficiency of production, and improved safety profiles, increased payload capacity and improved efficiency of production.

Accordingly, in one aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette, and wherein the 3' UTR of the transgene expression cassette comprises at least one cis-acting sequence as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome expression cassette comprises a transgene expression cassette and a vector intron, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome expression cassette comprises a transgene expression cassette and a vector intron, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, and wherein the lentiviral vector genome comprises a modified 5' LTR as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette, and wherein the 3' UTR of the transgene expression cassette comprises at least one cis-acting sequence as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, and wherein the lentiviral vector genome expression cassette comprises a transgene expression cassette and a vector intron as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette and a vector intron as described herein, and wherein the 3' UTR of the transgene expression cassette comprises at least one cis-acting sequence as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette and a vector intron as described herein, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a set of nucleotide sequences comprising nucleotide sequences encoding lentiviral vector components and a nucleotide sequence of the invention.

In a further aspect, the invention provides a set of nucleotide sequences comprising nucleotide sequences encoding lentiviral vector components and a nucleic acid sequence encoding an interfering RNA of the invention.

In a further aspect, the invention provides a set of nucleotide sequences comprising nucleotide sequences encoding lentiviral vector components, a nucleotide sequence of the invention and a nucleic acid sequence encoding an interfering RNA of the invention.

In some embodiments, the set of nucleic acid sequences comprises a first nucleic acid sequence encoding the lentiviral vector genome and at least a second nucleic acid sequence encoding the interfering RNA. Preferably, the first and second nucleic acid sequences are separate nucleic acid sequences. Suitably, the nucleic acid encoding the lentiviral vector genome may not comprise the nucleic acid sequence encoding the interfering RNA.

In some embodiments, the nucleic acid encoding the lentiviral vector genome comprises the nucleic acid sequence encoding the interfering RNA.

In some embodiments, the lentiviral vector components include gag-pol, env, and optionally rev.

In a further aspect, the invention provides a lentiviral vector genome encoded by the nucleotide sequence of the invention.

In a further aspect, the invention provides a lentiviral vector genome as described herein.

In a further aspect, the invention provides an expression cassette comprising a nucleotide sequence of the invention.

Accordingly, in a further aspect, the invention provides an expression cassette encoding a lentiviral vector genome comprising:
(i) a transgene expression cassette; and
(ii) a vector intron comprising at least one interfering RNA as described herein.

In a further aspect, the invention provides a viral vector production system comprising a set of nucleotide sequences of the invention.

In a further aspect, the invention provides a cell comprising the nucleotide sequence of the invention, the expression cassette of the invention, the set of nucleotide sequences of the invention or the vector production system of the invention.

In a further aspect, the invention provides a cell for producing lentiviral vectors comprising:
(a)
   (i) nucleotide sequences encoding vector components including gag-pol and env, and optionally rev, and the nucleotide sequence of the invention, or the expression cassette of the invention, or the set of nucleotide sequences of the invention; or
   (ii) the viral vector production system of the invention; and
(b) optionally, a nucleotide sequence encoding a modified U1 snRNA and/or optionally a nucleotide sequence encoding TRAP.

In a further aspect, the invention provides a method for producing a lentiviral vector, comprising the steps of:
(a) introducing:
   (i) nucleotide sequences encoding vector components including gag-pol and env, and optionally rev, and the nucleotide sequence of the invention, or the expression cassette of the invention, or the set of nucleic acid sequences of the invention; or
   (ii) the viral vector production system of the invention,
      into a cell; and
(b) optionally selecting for a cell that comprises nucleotide sequences encoding vector components and the RNA genome of the lentiviral vector; and
(c) culturing the cell under conditions suitable for the production of the lentiviral vector.

In a further aspect, the invention provides a lentiviral vector produced by the method of the invention.

In a further aspect, the invention provides the use of the nucleotide sequence of the invention, the expression cassette of the invention, the set of nucleotide sequences of the invention, the viral vector production system of the invention, or the cell of the invention, for producing a lentiviral vector.

In a further aspect, the invention provides a lentiviral vector comprising the lentiviral vector genome as described herein.

### DESCRIPTION OF THE FIGURES

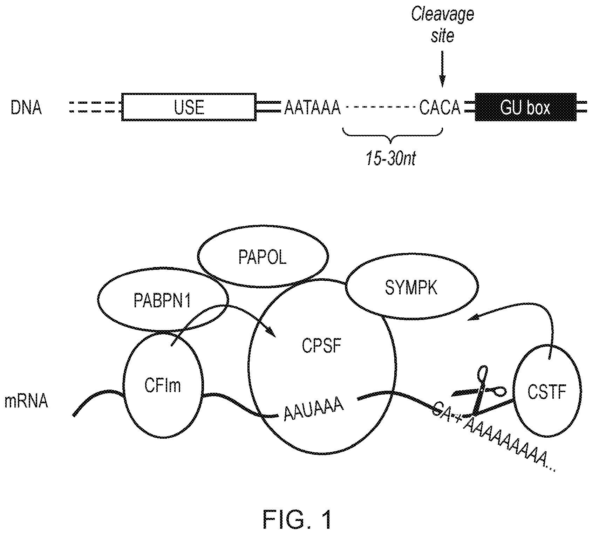

**Figure 1****. The general nucleotide domains comprising typical polyadenylation sequences and the protein complexes that interrogate them, leading to cleavage and addition of polyA tails to mRNA.** The Cleavage and Polyadenylation Specificity Factor (CPSF) protein complex binds to the poly(A) signal (PAS; AAUAAA); it contains CPSF1-4 and the associated factors FIP1L and Symplekin (SYMPK). The CPSF3 subunit is the endonuclease acting at the cleavage site. The Cleavage Factor 1 complex (CFIm) recognizes the upstream element (USE); it is composed of NUDT21, CPSF6, and CPSF7. The CSTF complex recognizes the GU- or U-rich downstream element (DSE). CPSF, CSTF, SYMPK, and CFIm interact at the protein level, stabilizing the RNA binding, thus promoting correct cleavage (typically 15-30nt downstream of the PAS, often at 'CA' dinucleotides), and PAS recognition and recruitment of the poly(A) polymerase (PAPOLA or PAPOLG). The nuclear poly(A) binding protein (PABPN1) interacts with CFIm and PAPOLA and contributes to the efficiency of polyadenylation.

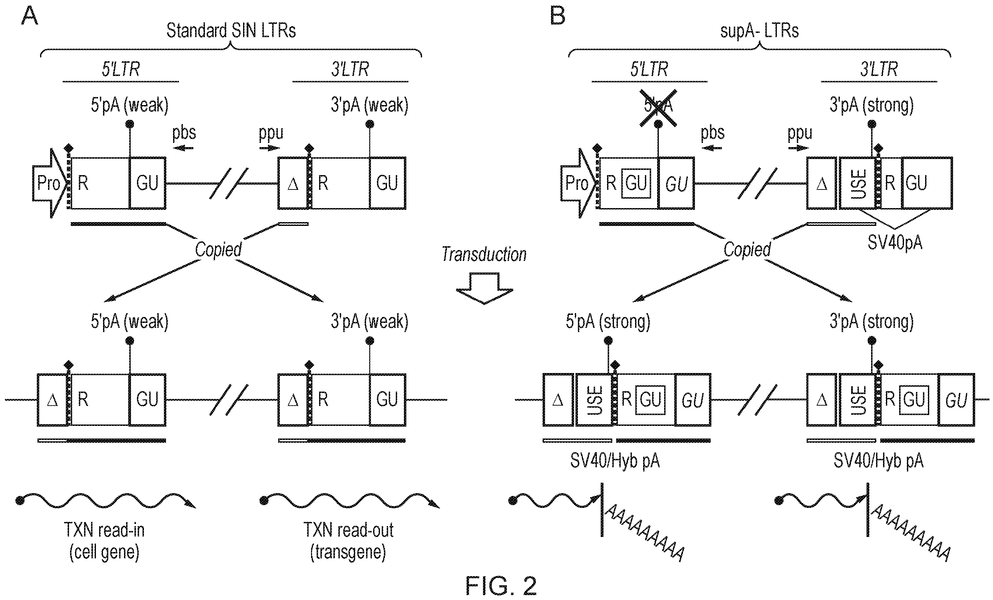

**Figure 2****. The configuration of LTRs (top panel) and resolution of LTRs following reverse transcription (bottom panel) within lentiviral vectors harbouring standard or novel SIN LTRs of the invention (supA-LTRs).** Typical 3^{rd} generation lentiviral vector genomic RNA (vRNA) is generated in production cells by transcription from a powerful promoter; the 3'LTR is typically deleted within the U3 promoter region (Δ) so that no transcription can occur from the LTRs in transduced cells. No 'internal' sequences are shown for clarity. **[A]** Standard lentiviral vector LTRs. Apart from the SIN modification, standard lentiviral vector LTRs are generally not modified further from wild type HIV-1. In the course of reverse transcription, the denoted sequences are copied resulting in two identical LTRs flanking the transgene cassette in the integrated cassette; at the 5'LTR, the sequence between the first nucleotide of R region (dotted lollipop) to the primer binding site (pbs) is copied to the 3'LTR, and at the 3'LTR, the sequence between the polypurine tract (ppu) and the first nucleotide of R region (dotted lollipop) is copied to the 5'LTR. The result of this that the 5'pA site is effectively copied to the 3'LTR. The HIV-1 polyA site is not as strong as some cellular polyAs; however, the deletion of U3 sequences has resulted in removal of polyA USE sequences, making the HIV-1 pA site even weaker. Since lentiviral vectors integrate into transcriptionally active gene regions, the consequence of harbouring a weak pA within the 5'LTR is the potential for transcription read-through ('read-in') into the integrated cassette. The consequence of harbouring a weak pA within the 3'LTR is transcription read-through ('read-out') into the downstream chromatin. **[B]** Lentiviral vector LTRs comprising the sequence upgraded polyAs of the invention. To generate 'sequence upgraded' pAs ('supA') within the flanking LTRs in order to make lentiviral vectors transcriptionally 'stealthy', the present invention shows that the 3'LTR can be modified in that a minimal 'R' sequence is inserted between the 3'pA and the polyA cleavage site; this modification can be done within a strong heterologous polyA (e.g. SV40 pA) or with a synthetic polyA, and therefore the the wild type R-U5 region can be entirely deleted from the 3'LTR. The consequence of this is that the 3'pA site is effectively repositioned upstream of the first nucleotide of R region (dotted lollipop) - within the U3 or ΔU3/SIN region - and copied from the 3'LTR to the 5'LTR during reverse-transcription. The use of an USE within the U3 or ΔU3/SIN region may optionally be included to enhance polyadenylation efficiency. In order to associate the new pA site within the ΔU3/SIN region with a DSE (GU-rich box), the 5'LTR is additionally modified to include a new GU-rich box within the first (TAR) loop of the vRNA. This new DSE/GU box is copied to the 3'LTR during reverse-transcription, resulting in stronger pAs within the flanking LTRs, leading to reduced transcription read-in or read-out of the lentiviral vector genome expression cassette. Optionally, the endogenous 5'pA site within the R region of the 5'LTR may be functionally mutated, since it no longer required.

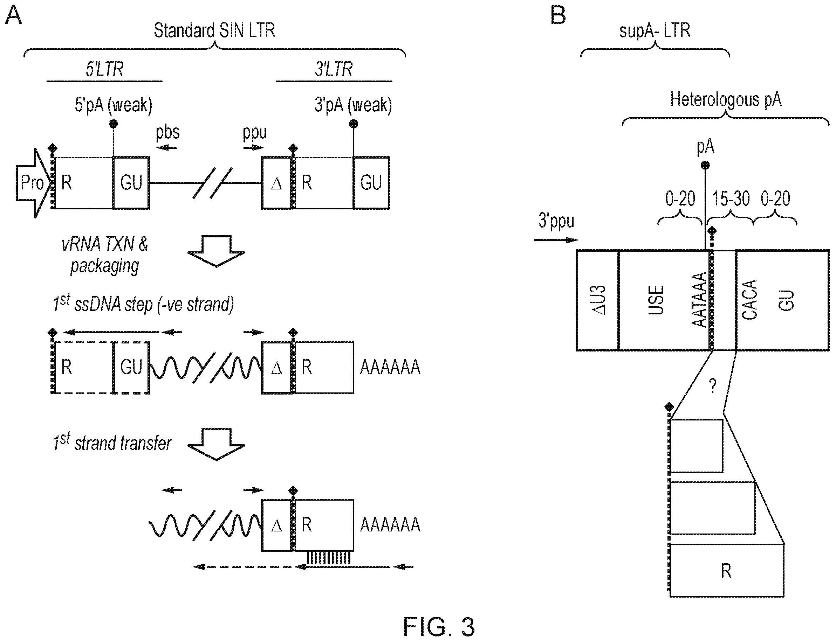

**Figure 3****. Modifications to the 3'(SIN)LTR in supA-LTR genomes and the importance of the minimal 'R' region. [A]** Typical 3^{rd} generation lentiviral vector genomic RNA (vRNA) is generated in production cells by transcription from a powerful promoter; the vRNA comprises from the first nucleotide of 5' R region (dotted lollipop) to the end of the 3' R region, down to the polyA cleavage site (and includes a polyA tail added at the cleaved 3'terminus). The vRNA recruits an endognous tRNA to the primer binding site (pbs), which is used as a primer for cDNA synthesis as the first step of reverse-transcription. The RNA sequence of the resulting DNA:RNA hybrid product is degraded by the RNAseH domain of the RT enzyme, allowing 1^{st} strand transfer of the 'free' cDNA to the 3'end of the vRNA, where the cDNA anneals to the complementary 3'R region. The remainder of the negative strand cDNA synthesis now proceeds. **[B]** An example of 'embedding' a minimal sequence of the R region between a heterologous pA signal (AATAAA) and a polyA cleavage site within the 3'(SIN)LTR to give a 'supA-LTR' in accordance with the invention. Given the approximate distance tollerances of polyA sequences (USE, pA signal, cleavage site, DSE/GU box) reported to enable highly efficient polyadenylation, prior to the invention it was not known if sufficient 'R' sequence could be inserted between the pA signal and the cleavage site to allow both efficient polyadenylation and first strand transfer during the RT step.

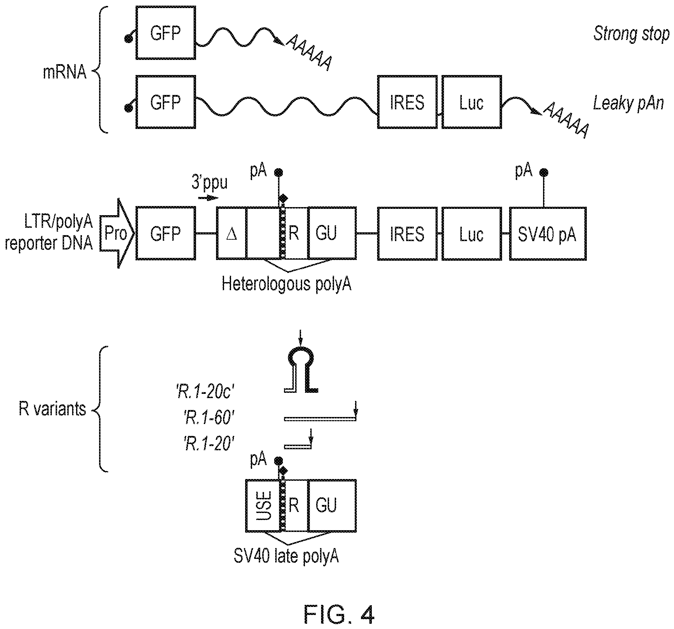

**Figure 4****. LTR reporter constructs used to assess polyadenylation efficiency during testing of novel supA-LTRs.** A simple GFP reporter construct was modified at the 3'end to include test LTRs containing polyA sequences, and an internal ribosomal entry site (IRES) and luciferase reporter ORF inserted downstream of this (top panel). Therefore, the level of luciferase expression detected in cells transfected with the reporter constructs inversely correlated with the strength of the test polyA sequences. The general structure of the LTR tested reflected the positions of the heterologous pA signal placed upstream of the R sequence (the first nucleotide of R represented by the dotted lollipop) and downstream of the SIN-U3 sequence (Δ). Other heterologous polyA sequences flanked the the pA signal and R region i.e. USE and GU-rich DSE (middle panel) . Example 'R variants' are shown, which were tested to identify the different lengths of R that could be inserted whilst maintaining polyA activity (bottom panel); R.1-20 comprised the first 20 nucleotides of the R region, R.1-60 comprised the first 60 nucleotides of the R region, and R.1-20c was generated with downstream complementary so that a stable hairpin could be generated. The arrows indicate the likely/possible polyA cleavage region.

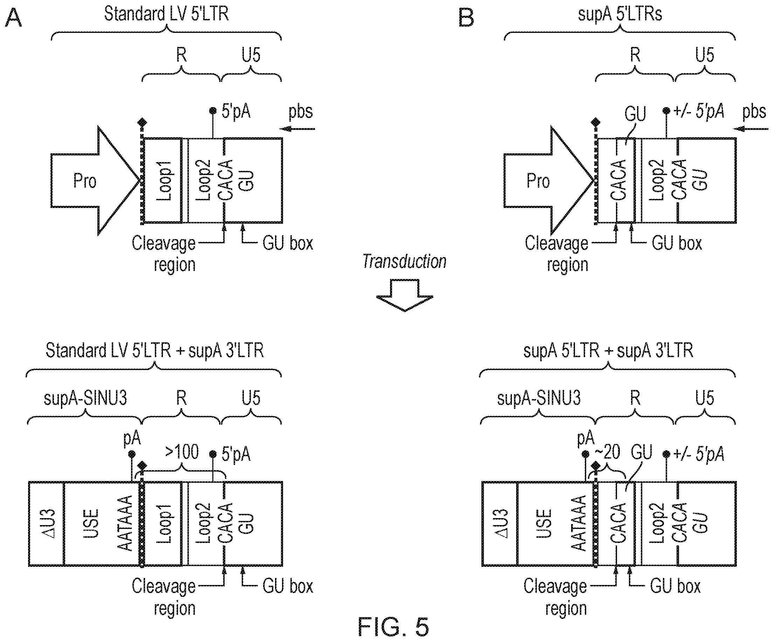

**Figure 5****. Consequences of partnering the a 3' supA-LTR modification with either a standard 5'LTR or a 5' supA-LTR modfication. [A]** The schematic shows the sequence order of a standard lentiviral vector 5'LTR in production (i.e. unmodified, wild type HIV-1 RU5 sequence) showing the first nucleotide of the R region (dotted lollipop), loop 1 (the TAR stem loop), loop 2 (the polyA stem loop), the polyA cleavage site (CACA) and GU rich DSE (top panel). If a lentiviral vector harbouring this standard 5'LTR was partnered with the modified 3'LTR of the invention (modified 3' LTR not shown in the to panel), the resolution of LTRs after reverse transcription is as shown (post-transduction); i.e. this configuration exists at both ends of the integrated cassette (bottom panel). The repositioned pA signal (now upstream of R) of the supA-SIN/U3 region is over 100 nucleotides upstream of the endogenous GU-rich DSE in the native R-U5 sequence. **[B]** In order to associate a DSE with the repositioned 3' pA signal, the 5'LTR can be modified such that a new cleavage site and GU-rich DSE is engineered into loop 1, so that the stem loop structure of loop 1 is maintained (top panel)., Therefore, after LTR resolution by reverse transcription a strong polyA sequence is generated with (optional) USE, pA signal, cleavage site and GU-rich DSE all in optimal positioning relative to each other (bottom panel). Consequently, the native 5'pA signal within loop 2 can be funcationally mutated.

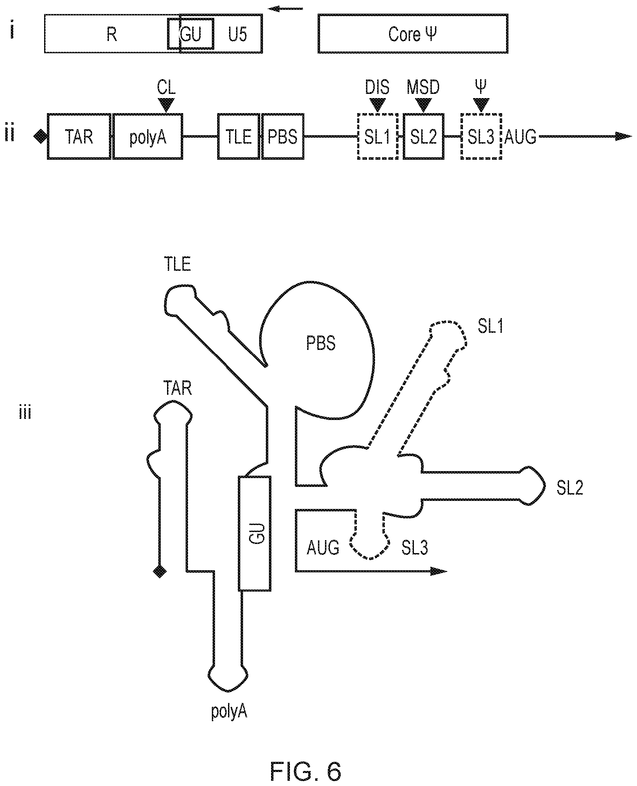

**Figure 6****. A schematic to show the functional domains of RNA within the 5'UTR of the wild type HIV-1 genome. [i]** A simplified view of the HIV-1 5'UTR indicating the location of the GU-rich DSE embedded between the R and U5 region (primer binding site [arrow] and core packaging region shown). **[ii]** A more detailed description of the blocks of functional sequences; trans-activation response (TAR) element to which tat binds, enhancing transcription elongation; the polyA region [approximate pA cleavage site denoted]; tRNA-like element (TLE), which loads the tRNA primer onto the primer binding site (PBS); stem-loop 1 (SL1), contains the dimerisation initiation sequence (DIS)); stem-loop 2 (SL2), contains the major splice donor (MSD), and stem-loop 3 (SL3) contains the 'GxG' nucleocapsid binding sequence (Ψ) - the AUG being the start codon of Gag/Gagpol. **[iii]** The generalised structure of the entire 5'UTR that approximates the 'dimerised' conformation.

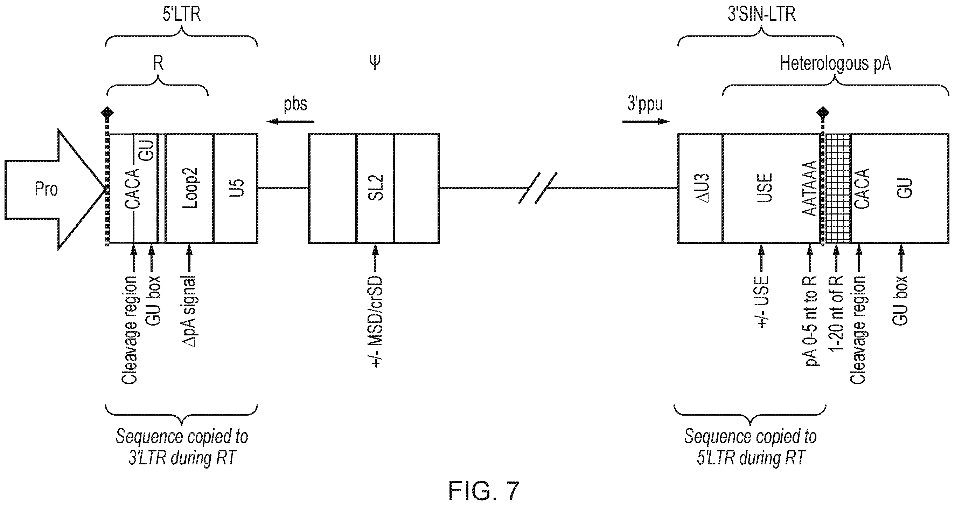

**Figure 7****. A schematic to show the general features of the improved 5' and 3' LTRs of the invention as encoded within a lentiviral vector genome expression cassette.** The position of the promoter (Pro) is shown at the 5'LTR, with the transcription start site (TSS) at nucleotide 1 of the R region as indicated by the dotted lollipop. The 5' R region is modified to include a new GU-rich box (the DSE) downstream of the polyA cleavage region. In this nonlimiting example, the region of R comprising 1-20nt is the hashed box region comprising the sequence between the TSS and the cleavage region (this same sequence is embedded in the heterologous pA sequence at the 3'LTR). The loop 2 region contains the native pA signal, which may be optionally mutated/deleted. At the 3'LTR, the self-inactivating (SIN) modification to the U3 region is shown downstream of the 3'polypurine (ppu) tract. Downstream of the SIN-U3 is positioned a heterologous pA sequence (i.e. the native R-U5 sequence is deleted), wherein the 1-20 nucleotide R region sequence - identical to the 1-20 nucleotides of R at the 5' end - is postioned between the heterologous pA signal and the cleavage region/GU-rich box (DSE) zone. Optionally, an upstream pA enhancer element may be placed between the SIN-U3 and the heterologous pA signal. In effect, relative to standard LV 3'LTRs, the 3'pA signal is repositioned upstream of the first nucleotide of of the R region; this means that the 3'pA signal will be copied to the 5'LTR during reverse transcription. The new GU-rich box (DSE) will be copied to the 3'LTR, and will 'service' the pA signals of both LTRs. The new LTRs may be optionally used in lentiviral vector harbouring mutations within the major/cryptic splice donor region (MSD/crSD).

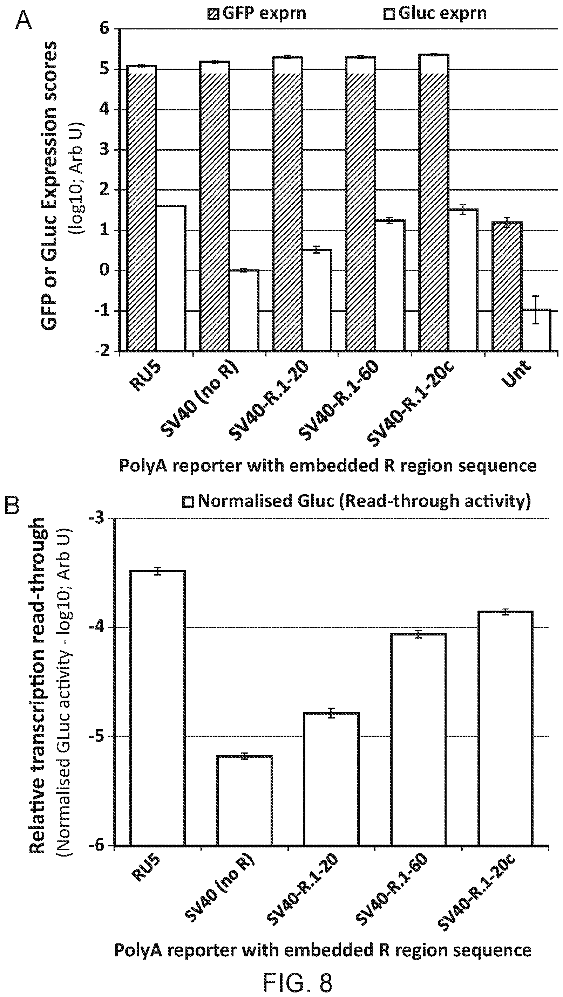

**Figure 8****. Testing three R region lengths embedded within the SV40 late polyA sequence with regard to polyadenylation efficiency using GFP/Luciferase reporters.** The PolyA reporter plasmid described in Figure 4 was used to test polyadenylation efficiency of 'R-embedded' SV40 polyA sequences. HEK293T cells were transfected with EF1a-driven GFP-wPRE-SINLTR-IRES-Gluc containing plasmids, wherein each SINLTR sequence harboured R variants 'R.1-20', 'R.1-60', 'R.1-20c' or 'no R' (i.e. just the SV40 pA), or just a standard RU5 containing its own pA and GU-rich DSE in the U5. **[A]** GFP expression scores were generated by multiplying percentage GFP-positve by the MFI, and Gluc activity was measured in cell lysates. **[B]** Gluc activity was divided by GFP expression scores to generate normalised Gluc values, which reflected transcriptional read-through of the SINLTR regions.

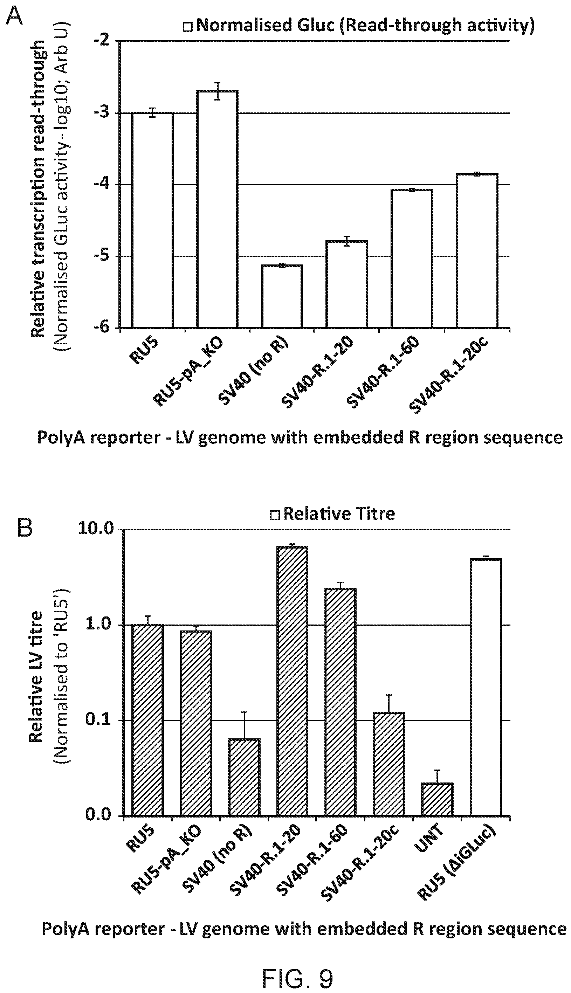

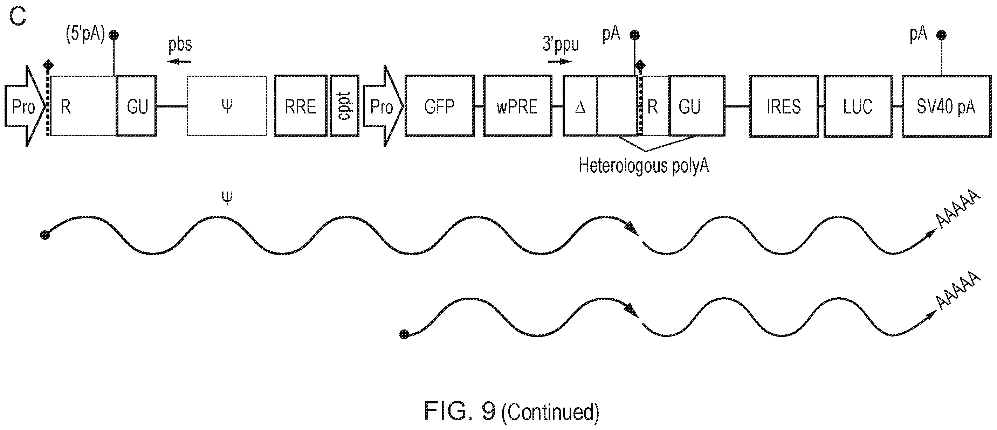

**Figure 9****. Testing three R region lengths embedded within the SV40 late polyA sequence with regard to polyadenylation efficiency and LV titres using GFP/Luciferase LV genome reporters.** The R variants 'R.1-20', 'R-160, and 'R.1-20c', as well as non-R containing variant 'SV40 (no R)' and the native HIV-1 pA knock-out RU5 variant, were cloned into an LV genome polyA reporter construct. This construct is capable of producing LV vector and also report on polyA activity by Gluc assay. HEK293T cells were transfected with the LV genome reporters described, and with LV packaging components to generate LV-GFP/VSVG vector particles. A pPGK-DsRedX plasmid was spiked in to all transfection mixes to measure transfection efficiency. A 'normal' LV vector genome was used as a control (lacking the IRES-Gluc-SV40pA sequence). **[A]** Post-production cells were measured for GFP and DsRedX expression by flow cytometry and cell lysates were measured for Gluc activity; Gluc activity was normalised by DsRedX expression. **[B]** Clarified crude LV harvests were titrated on HEK293T cells by flow cytometry to generated TU/mL titres, which were then normalised setting 100% at the 'RU5' variant (harbouring a standard 3'LTR).

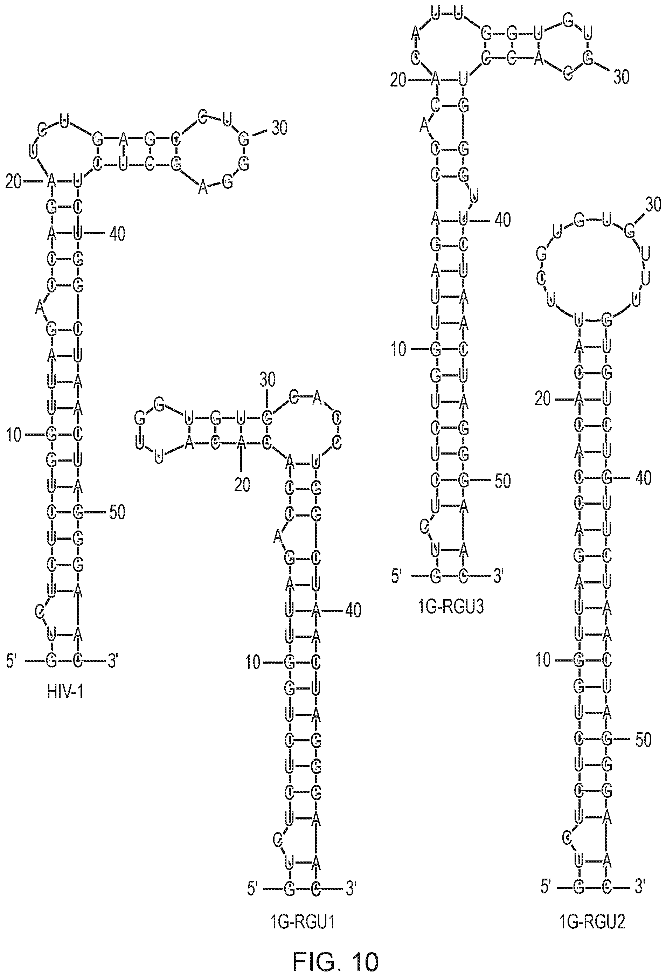

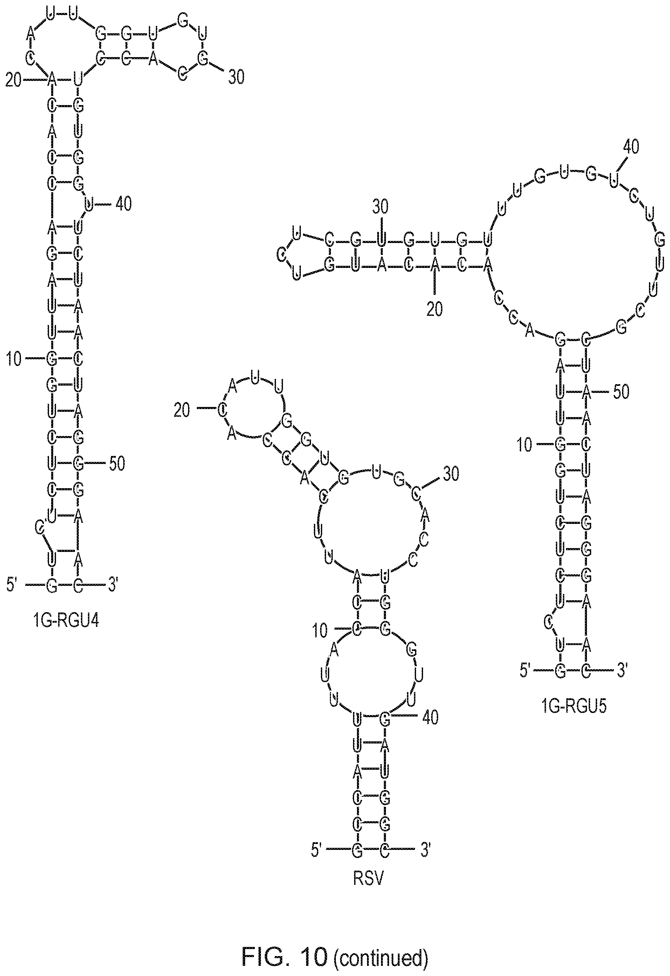

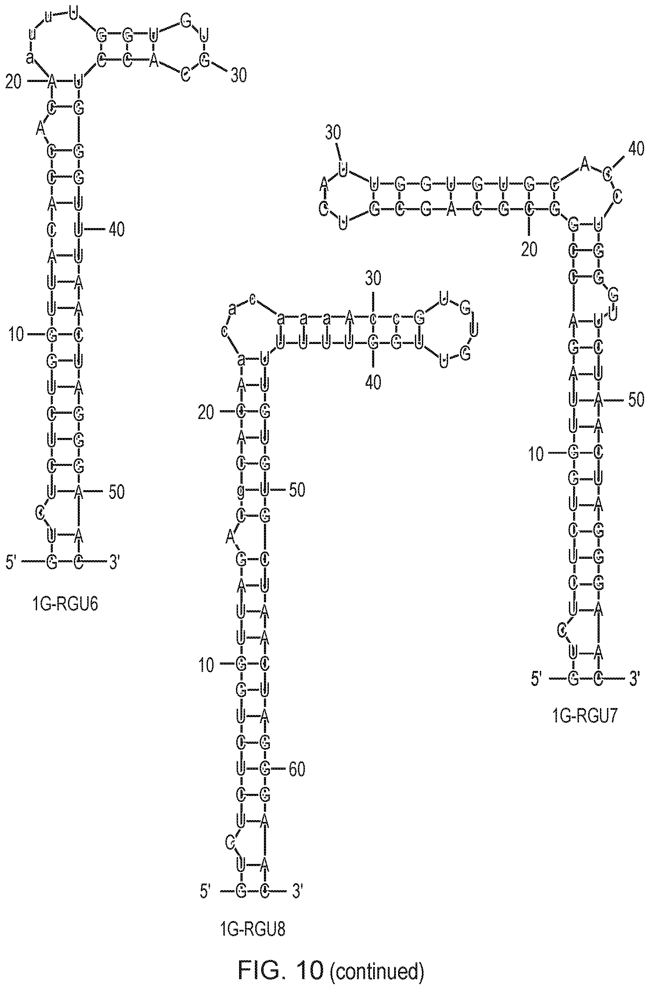

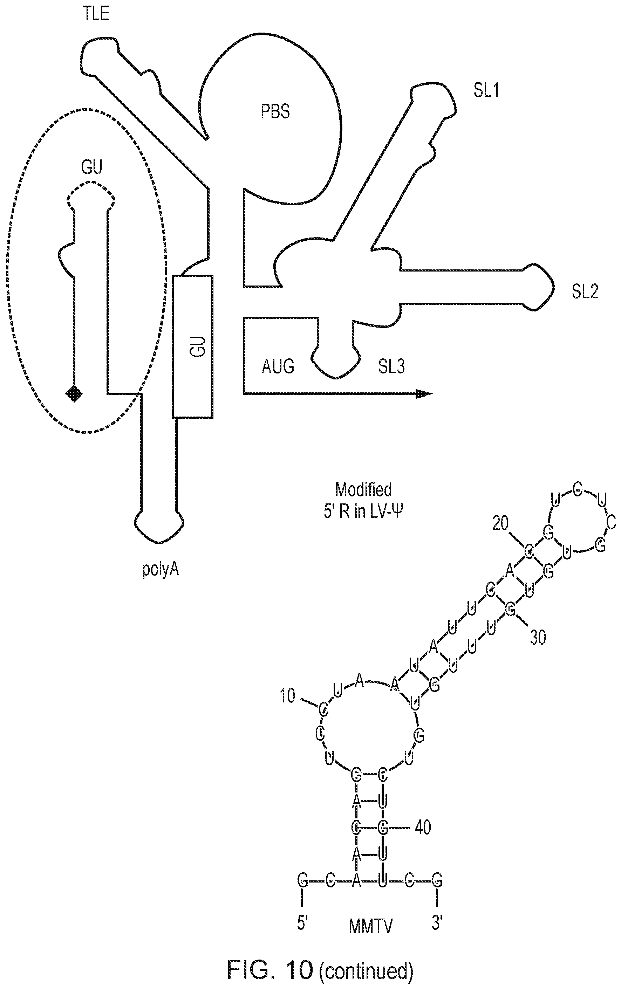

**Figure 10****. Predicted RNA structure of stem loops (SL) found at the 5'end of retroviruses compared to those in engineered lentiviral vector genomes tested in the invention.** HIV-1 genomic RNA contains a stem loop at the 5' terminus called the trans-activation response (TAR) element to which binds tat. Other retroviruses also harbour stem loop structures at their 5' terminus; RSV and MMTV contain a SL harbouring the GU-rich DSE that 'services' a polyA signal encoded between the U3 TATA box and transcription start site. Hybrid TAR/SLs were designed to incorporate RSV or MMTV or a beta-globin polyA based GU-rich DSEs into the HIV-1 TAR stem; the first 18-20 nucleotides of HIV-1 R were retained in order to maintain sequence that may be important for transcription initiation, as well as ensuring at least 18 nucleotides for homology driven first strand transfer. The hybrid structures are drawn based on the '1G' sequence, which is thought to be the primary packaged form of genomic RNA (see Table 1). The GU-rich DSE-modified TAR loop position is depicted in context to the secondary structure of the LV packaging signal (Psi).

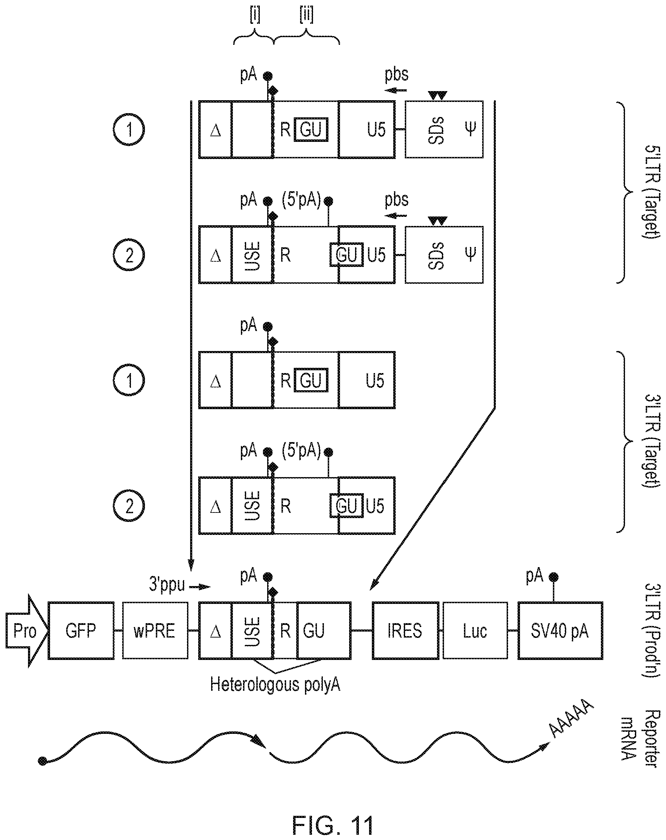

**Figure 11****. PolyA reporter constructs to assess polyadenylation of different LTR variants in different expression contexts.** The schematic displays subtly different polyA reporter constructs used in the study, which were used to assess polyA activity in different settings. The original polyA reporter (shown in its entirity) tests the 3' supA-(SIN)LTR variants/configurations reflecting polyadenylation at the 3'end of the LV genome cassette in production cells. The 'R-embedded' heterologous polyA sequence is shown with a USE and GU-rich DSE. Above this indicates the alternative LTR variants/configurations to reflect either 5' or 3' LTRs after reverse transcription and integration into target cells, depending on whether a USE within the SIN-U3 region **[i]** is included (e.g. ②) or whether loop 1 of the 5'R region of the LV genome (copied to 3'LTR) has been modified to include a new GU-rich DSE [ii], which will 'service' the new pA signal upstream of the R region (e.g. ①); the native HIV-1 pA may be optionally deleted from variant LTRs. The 5'LTR reporter constructs also contained extentded sequence from the LV genome into the packaging region, including the major splice donor region (SDs). Thus, the 5'LTR reporters modelled transcription 'read-in' from upstream cellular promoters and the 3'LTR reporters modelled transcription 'read-out' from the transene cassette.

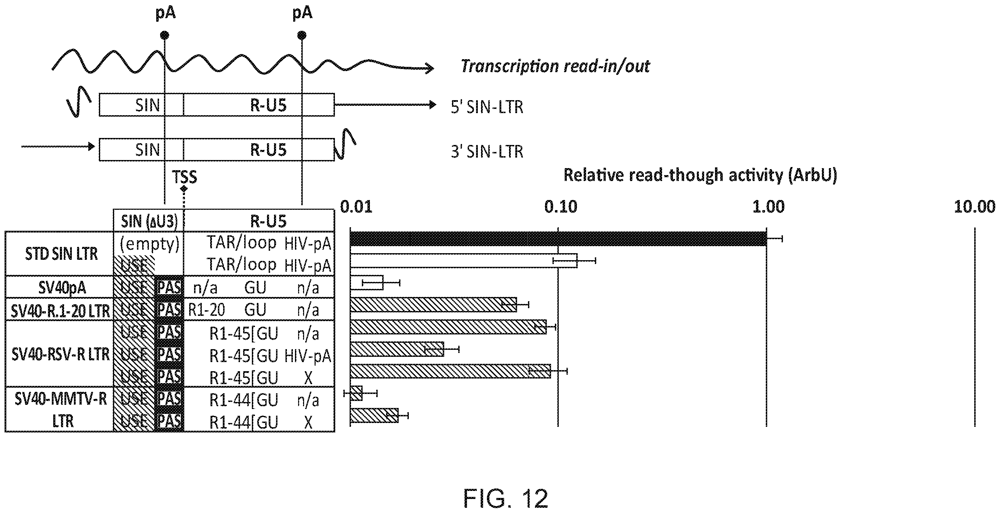

**Figure 12****. PolyA reporter constructs to assess polyadenylation of different SIN-LTR variants containing different R region sequences from different retroviruses.** The polyA reporter cassette (see Figure 11) was tested with SIN-LTR variants containing functional sequences as denoted in the grid to the left, modelling either transcriptional 'read-in' (5'SIN-LTR) from a cellular promoter or 'read-out' (3'SIN-LTR) from the transgene promoter in the context of an integrated cassette. The variants were compared to the standard SIN-LTR (STD SIN-LTR), which just has the deletion in the U3 followed by the native R-U5, comprising the TAR/SL1^{®} and native polyA signal (grey lollypop)/GU-rich DSE (R-U5). The variants were the R-embedded SV40 polyA sequence, wherein the sequence between the pA signal (PAS; black lollypop)) and GU-rich DSE was replaced with [1] nts 1-20 of the HIV-1 R region (TAR/SL1) or [2] nts 1-45 of RSV R region (containing its own GU-rich DSE) or [3] nts 1-44 of MMTV R region (containing its own GU-rich DSE). The latter two variants were also tested with or without downstream HIV pA signal mutation. The position of the transcription start site (TSS; defines U3-RU5 boundary) is shown by the dotted lollypop. Relative read-through activity was measured in normalised luciferase units (arbitrary units).

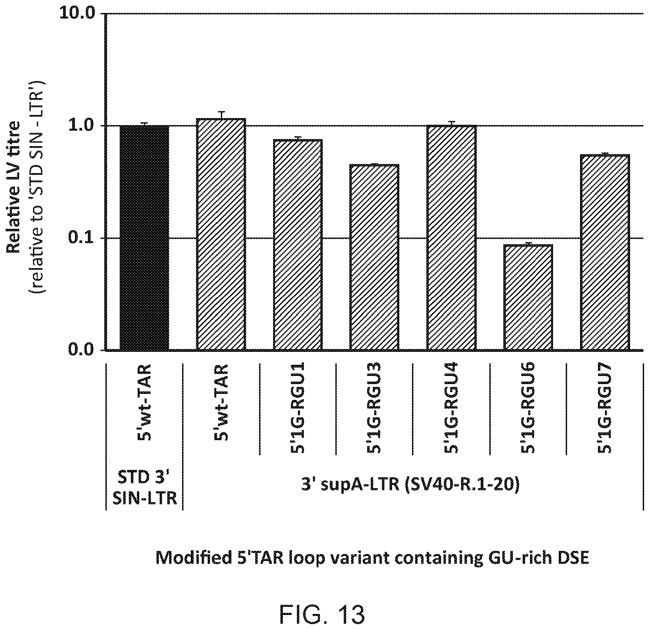

**Figure 13****. Relative LV titres of vector genomes harbouring GU-rich DSE modified 5' TAR/SL1 sequences paired with the 3'supA-LTR.** Having previously shown that the minimal nt1-20 R-embedded SV40 polyA sequence could be used to generate high titre LV, some of the GU-rich DSE modified hybrid TAR/SL1 variants based on RSV or MMTV R region (see Figure 10, Table 1) were cloned into the 5'end of the LV genome to assess the impact of both 5' and 3' LTR changes on LV titres. LVs encoded GFP were produced in adherent HEK293T cells and titrated by flow cytometry. Titres were normalised to a standard LV vector control (with unmodified 5' TAR/SL1 and SIN-LTR) and plotted on a log-10 scale.

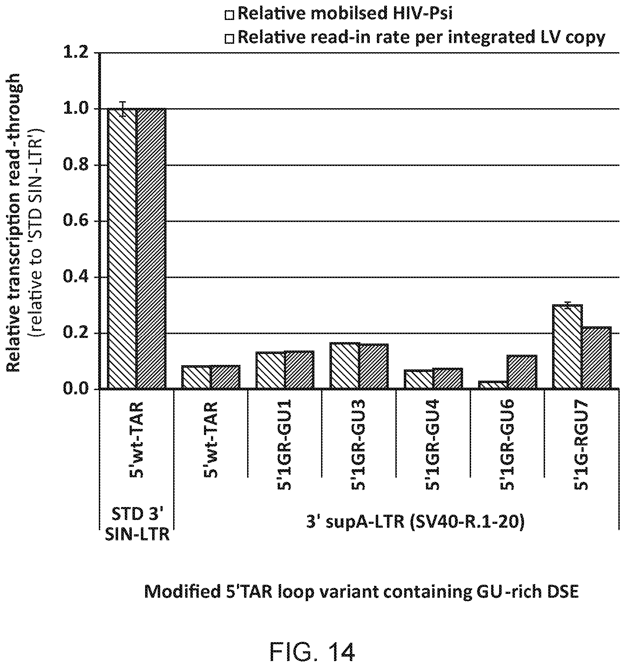

**Figure 14****. Assessment of transcriptional read-in into integrated supA-LTR LV variants of the invention.** The supA-LTR variant LVs produced and titrated in Figure 13, were used to transduce HEK293T cells at matched MOIs, cells passaged for 10 days to ensure unintegrated cDNA was diluted out, and transcription read-in from cellular gene promoters upstream of the 5'SIN/supA-LTR measured by RT-qPCR using primers/probe binding in the packaging region. RNA signal was displayed relative to the standard LV either as total 'mobilised RNA signal' or normalised to integrated copy-number.

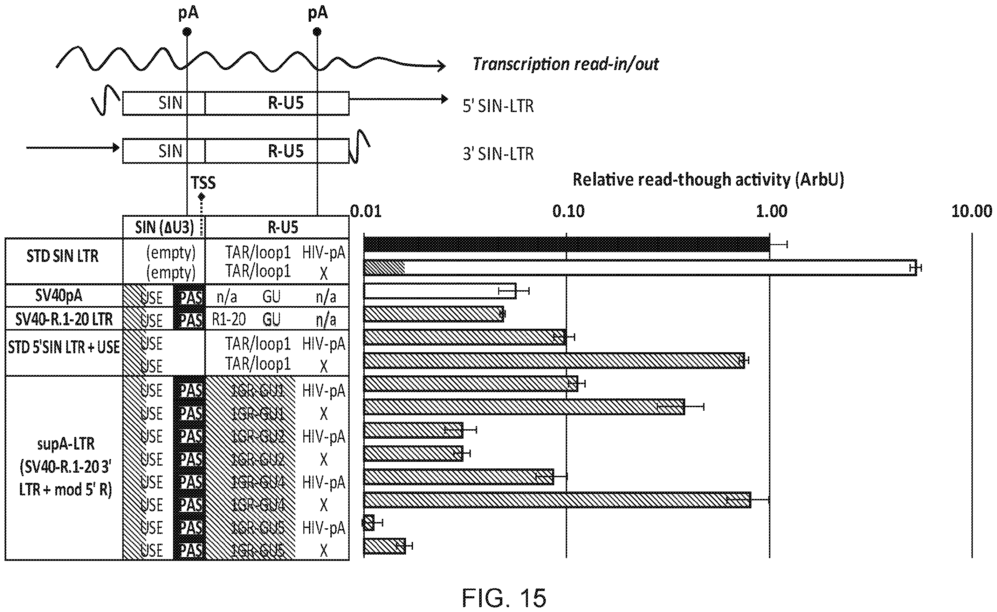

**Figure 15****. PolyA reporter constructs to assess polyadenylation of different supA-LTR variants containing different TAR/SL1-GU/DSE hybrids.** The polyA reporter cassette (see Figure 11) was tested with supA-LTR variants containing functional sequences as denoted in the grid to the left, modelling either transcriptional 'read-in' (5' supA-LTR) from a cellular promoter or 'read-out' (3' supA-LTR) from the transgene promoter in the context of an integrated cassette. The variants were compared to the standard SIN-LTR (STD SIN-LTR), which just has the deletion in the U3 followed by the native R-U5, comprising the TAR/SL1^{®} and native polyA signal (grey lollypop)/GU-rich DSE (R-U5). The variants were the R-embedded SV40 polyA sequence (SV40-R.1-20 3'LTR), wherein the sequence between the pA signal (PAS; black lollypop)) and GU-rich DSE was replaced with the stated TAR/SL1-GU/DSE variants (Figure 10, Table 1). The variants were also tested with or without downstream HIV pA signal mutation. The position of the transcription start site (TSS; defines U3-RU5 boundary) is shown by the dotted lollypop. Relative read-through activity was measured in normalised luciferase units (arbitrary units).

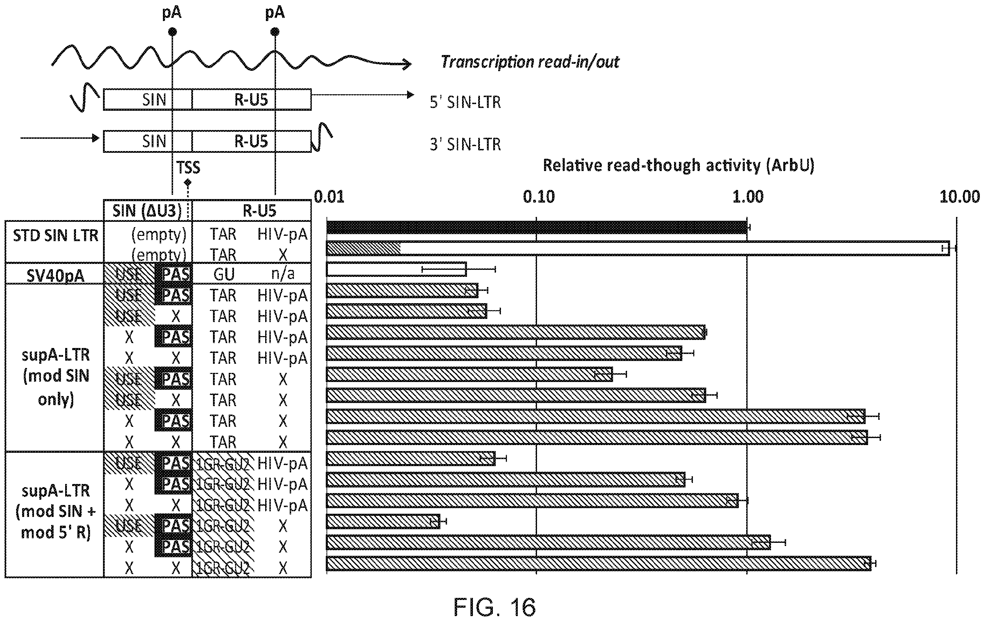

**Figure 16****. PolyA reporter constructs to assess relative contribution of polyadenylation activity by the different modifications introduced in to supA-LTR.** The polyA reporter cassette (see Figure 11) was tested with supA-LTR variants containing functional sequences as denoted in the grid to the left, modelling either transcriptional 'read-in' (5' supA-LTR) from a cellular promoter or 'read-out' (3' supA-LTR) from the transgene promoter in the context of an integrated cassette. The variants were compared to the standard SIN-LTR (STD SIN-LTR), which just has the deletion in the U3 followed by the native R-U5, comprising the TAR/SL1^{®} and native polyA signal (grey lollypop)/GU-rich DSE (R-U5). Variants containing just the 3' modifications (mod SIN only) were compared to the full recapitulated supA-LTR (mod SIN + mod 5'R), containing the 1GR-GU2 variant. These were also tested with or without downstream HIV pA signal mutation. The position of the transcription start site (TSS; defines U3-RU5 boundary) is shown by the dotted lollypop. Relative read-through activity was measured in normalised luciferase units (arbitrary units).

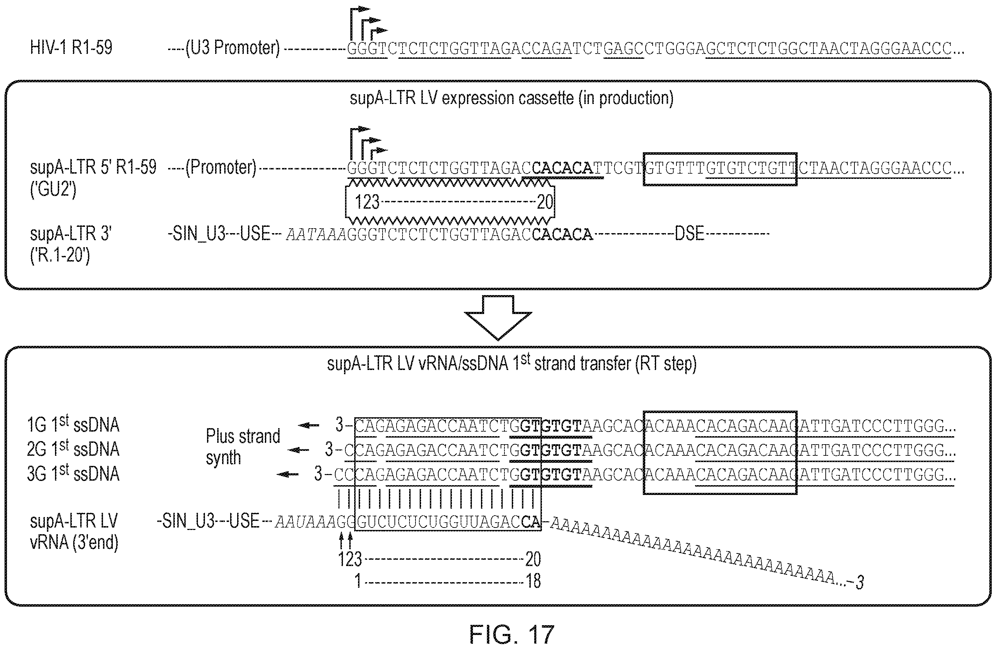

**Figure 17****. A detailed view of the modified 5' R region and 3' 'R-embedded heterolgous polyadenylation sequence in the modified 3'SIN-LTR as part of the supA-LTR configuration.** The HIV-1 R region from nucleotide 1 to 59 is shown, indicating the three alternative TSSs at the first three nucleotides. Underlined sequence indicates the nucleotides base-paired in stem loop 1 (the TAR loop) and the light grey sequence indicates the loop. The modified 5' R region exemplified in the invention retains 18-20 nucleotides of the first HIV-1 R 1-20 nucleotides, and introduces additional 'CA' motifs downstream to 'offer' cleavage sites. The GU-rich DSE of variant 'GU2' (see Table 1) is shown as boxed sequence. Also shown as part of the LV expression cassette is the general structure/sequence of the 3' supA-LTR containing the SIN-U3, optional USE, the PAS (italic) position upstream of the retained ~20 nucleotides of R.1-20, and a DSE. Since typical LV expression cassettes retain the 3xGs at the TSS, then expression of 3G, 2G and 1G vRNA can occur in production. Whilst it has been shown for HIV-1 that 1G vRNA preferentially dimerises and is most efficiently packaged of the three variants, the invention allows for all three vector vRNA species to be potential substrates for packaging and subsequent reverse transcription (RT) steps, by ensuring that the 3xGs are retained downstream of the PAS. However, the invention also discloses a novel promoter that enables just 1G vRNA to be transcribed, and in this case the first 18 nucleotides (i.e. nucleotides 3-to-20 of HIV-1) of the 5' R region can be inserted directly after the PAS. In this specific case, the two additional Gs (vertical arrows) downstream of the PAS would not *necessarily* be required. Thus, during 1^{st} strand transfer of the new minus strand ssDNA, the 18-20 nucleotides of homology between 5' and 3' R regions results in complementarity sufficient to allow annealing between ssDNA and vRNA, and plus strand synthesis initiation with no mismatches at the primer-extention point. The graphic provides further clarity of how the new DSE (boxed) encoded in the 5' R region is positioned and copied into DNA as a consequence of the RT step, resulting in the USE-PAS-clv-DSE sequences - all within desirable position within respect to each other in both the 5' and 3' supA-LTRs.

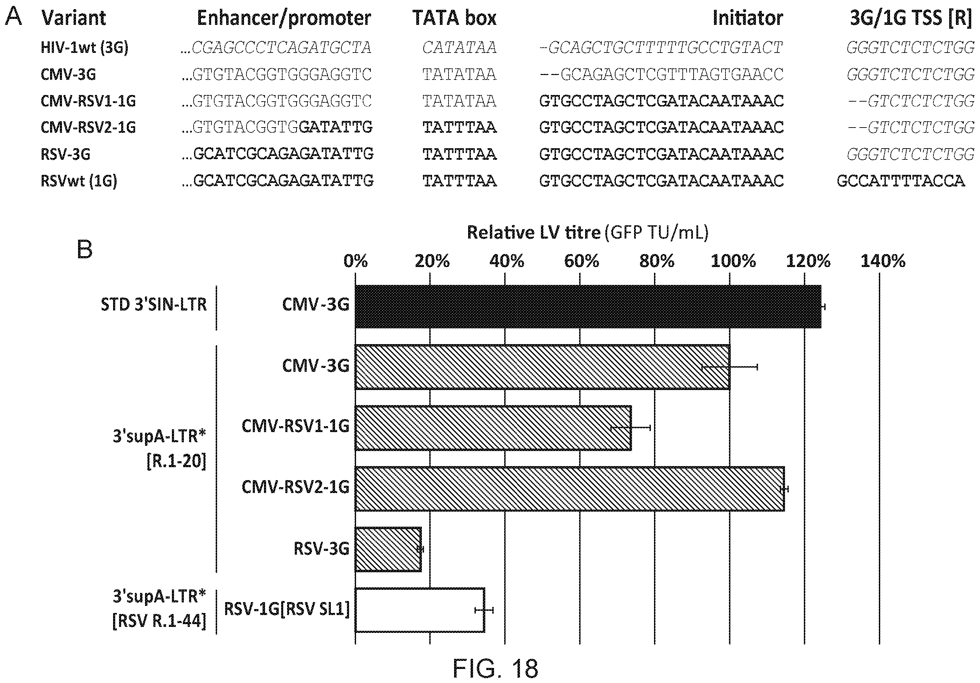

**Figure 18****. Development of a novel CMV/RSV hybrid promoter that generates '1G' LV genomic vRNA. [A]** A comparison of the core promoter and transcriptional start site (TSS) sequences for wild type HIV-1 (top sequence), wild type RSV (bottom sequence), and the novel variants engineered as part of the invention. The core TATA box is shown with immediate upstream and downstream flanking sequence. The presence of the 3x G or 1x G at the 5' terminus of the LV genome vRNA is indicated in each case. Italised sequence is from HIV-1 and bold sequence is from RSV. All other sequence is from the CMV promoter, with spaces indicated by dashes. Variants 'CMV-RSV1-G' and 'CMV-RSV2-1G' are two hybrid promoters comprising mostly the CMV promoter but with sequence exchanged for the RSV promoter in the core region where indicated. Variant 'RSV-3G' is an expression cassette driven by the full RSV promoter i.e. has the standard '3G' 5' R region of the LV genome. Variant 'RSV-1G[RSV SL1]' is also driven by the full length RSV promoter but the RSV R region replaces the HIV-1 5' R region; this was cloned into an alternative 3'supA-LTR cassette habouring an 'R-embedded' SV40 late polyA sequence where the RSV R.1-44 was emdedded instead of R from HIV-1 so that 1^{st} strand transfer could occur. All other variants were cloned into an LV genome expression cassette with the 3'supA-LTR containing the HIV-1 R.1-20 embedded SV40 late polyA. *Note that all these variant genome cassettes also contained the 'IRES-Luc' reporter after the 3'SIN-LTR region and so were longer constructs compared to the standard, unmodifed control (black bar). Therefore, relative LV titres are compared to the 'CMV-3G' variant, which contained the standard CMV promoter-LTR configuration. [B] Relative LV titres compared to the 'CMV-3G' variant.

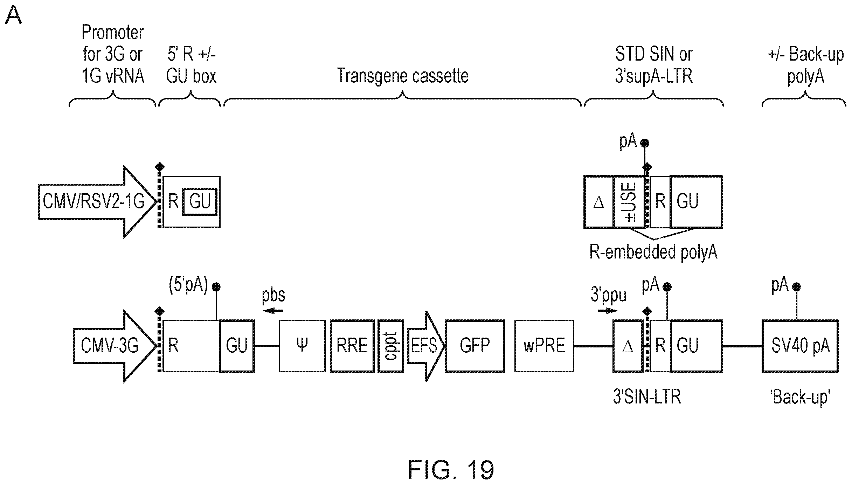

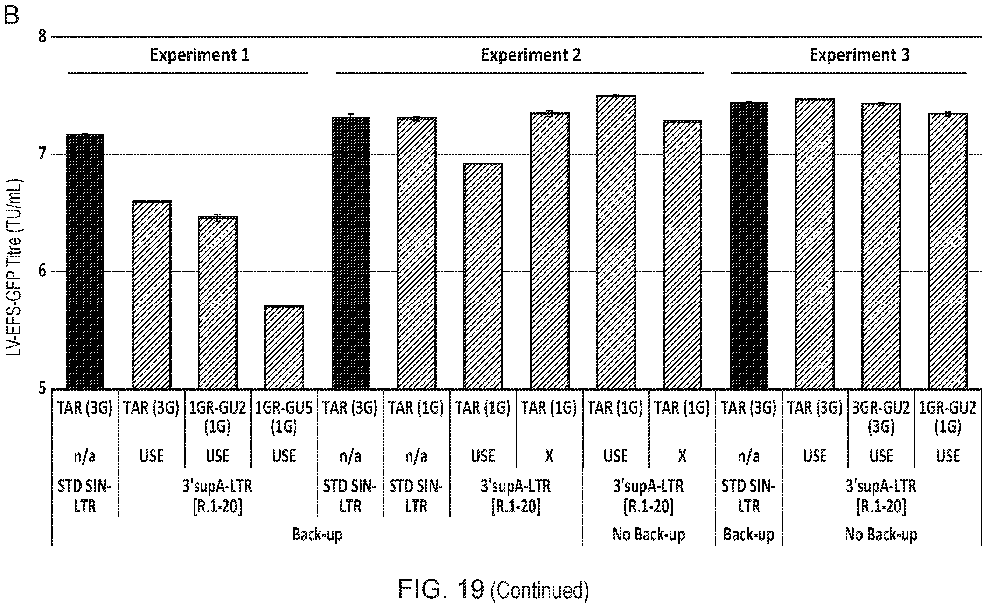

**Figure 19****. Further optimisation of the supA-LTR LV genome expression cassette. [A]** A schematic indicating the type/positions of modifications introducing to a standard SIN-LTR LV genome expression cassette to generate a supA-LTR LV genome expression cassette. A typical SIN-LTR encoding LV genome cassette is shown with [1] CMV promoter driving expression of an vRNA genome from a '3G' TSS, standard 5'/3' RU5 sequences and a 'back-up' polyadenylation sequence (SV40 late polyA shown). A series of three independent experiments was performed wherein LVs were produced using genome expresion cassettes employing some or all of the supA-LTR features. The novel CMV-RSV2-1G hybrid promoter is employed to generate a '1G' TSS, the 5' R region modification with GU-rich sequence (1GR-GU2 and 1GR-GU5 variants were tested), the 3'supA-LTR (R.1-20 embedded SV40 late polyA) with or without the USE, and optionally the back-up polyA sequence. **[B]** The vector titres produced in each of the three separate experiments (STD SIN-LTR in black bars) from using LV genome expression cassettes with the stated variant features (log10 values plotted on a linear scale).

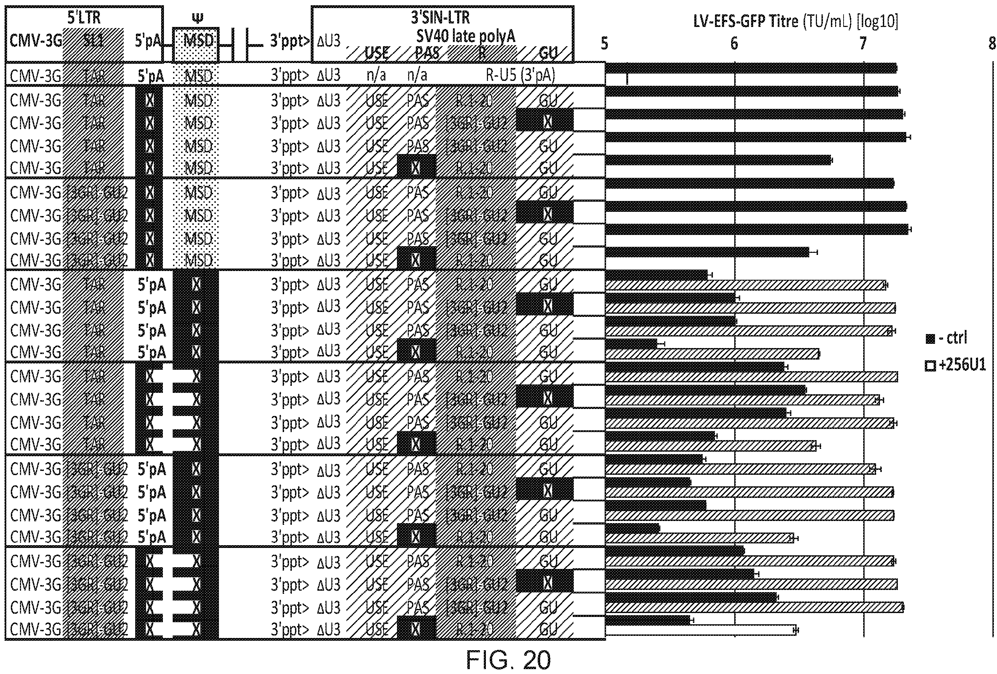

**Figure 20****. The supA-LTR LV genomes enable mutation of the native 5'LTR PAS site and can be paired with 'MSD2-KO' LV genomes.** The configurations of 5' and 3' LTRs are indicated with or without different elements of the invention; all constructs were driven by the CMV promoter (with the '3G' TSS) and the 'back-up' polyadenylation sequence was absent except for the standard control (had SV40 late polyA downstream of the 3'SIN-LTR - not shown). The '1GR-GU2' DSE element was positioned in at the 5' position, and when also optionally used as the embedded 'R' sequence in the 3' supA-LTR sequence the SV40 late polyA GU-rich DSE was deleted to assess the ability of the '1GR-GU2' DSE to functionally replace it. Deletions are indicated by a white X/black box. All combinations of elements were also evaluated in 'MSD-2KO' LV genomes, wherein aberrant splicing from the packaging region is eliminated; such genomes produce lower titres but are recovered by co-expression of a modified U1 snRNA (256U1).

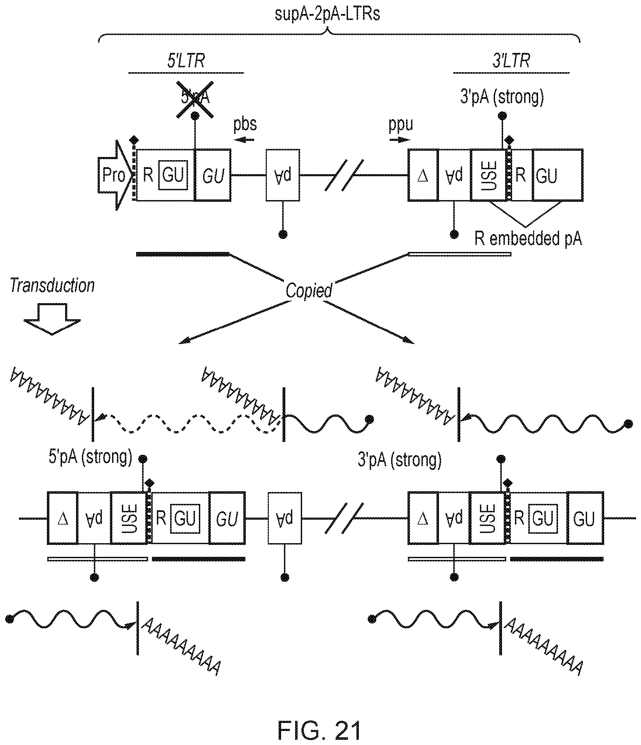

**Figure 21****. Overview of 'supA-2pA-LTRs' employed within LV genomes.** The schematic describes how additional polyadenylation sequences can be inserted within the 3'SIN region in the anti-sense orientation, between the ΔU3 region and the R-embedded heterologous polyA sequence, such that it is copied to the 5'SIN-LTR after transcription.

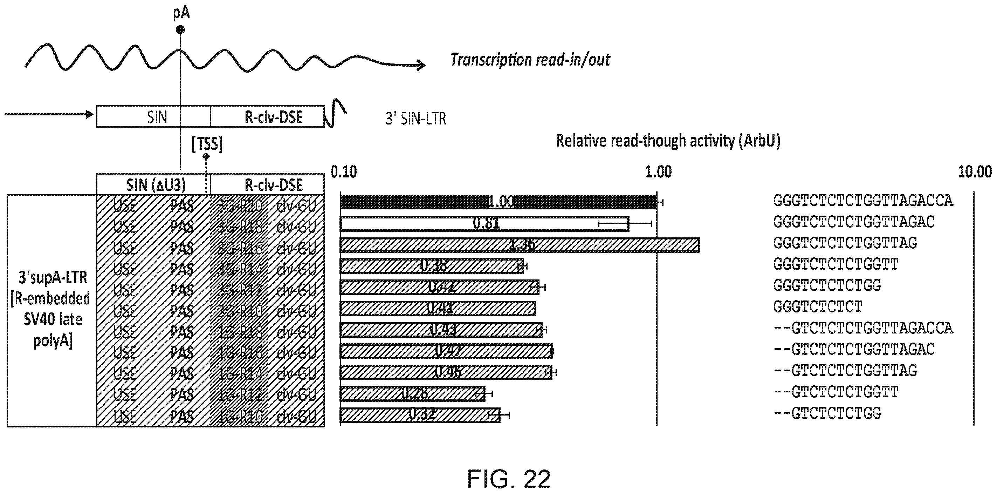

**Figure 22****. Defining a minimum R region sequence embedded within a heterologous polyadenylation sequence in the 3' supA-LTR: effects on polyadenylation/transcriptional read-through.** The heterologous SV40 late polyadenylation signal was positioned downstream of the 3'ppt/ΔU3 region within an EF1a-GFP reporter cassette, and upstream of an IRES-luciferase reporter sequence. R region sequences composed of up to 20 nucleotides of HIV-1 R region were inserted between the heterologous PAS and the cleavage site/GU-rich DSE element. The '3G-R20' sequence is the same as 'R.1-20' referred to elsewhere in the invention. Truncated variants were generated based on including either the 3x Gs or a single G ('1G) immediately downstream of the heterologous PAS (modelling use of genomes with '3G' or '1G' vRNAs). Sequences of the embedded R sequences are displayed. Read-through data are normalised luciferase activities, and displayed relative to the 3G-R20/R.1-20 control.

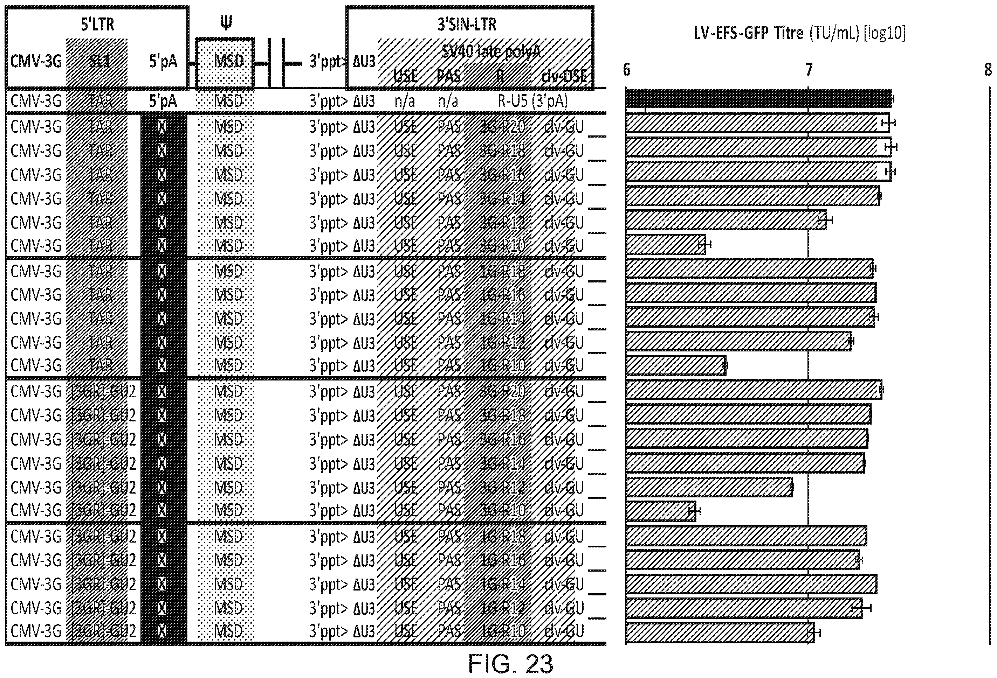

**Figure 23****. Defining a minimum R region sequence embedded within a heterologous polyadenylation sequence in the 3' supA-LTR: effects on LV titres.** The configurations of 5' and 3' LTRs are indicated with or without different elements of the invention; all constructs were driven by the CMV promoter (with the '3G' TSS) and the 'back-up' polyadenylation sequence was absent except for the standard control (had SV40 late polyA downstream of the 3'SIN-LTR - not shown). All 5' LTRs were mutated in the native HIV-1 5' PAS and an internal EFS-GFP-wPRE cassette (not shown) but retained the major splice donor. The 5' R region was either the wild type/standard TAR/SL1 or the modified 5' R SL1 comprised the 3GR-GU2 variant. The heterologous SV40 late polyadenylation signal was positioned downstream of the 3'ppt/ΔU3 region. R region sequences composed of up to 20 nucleotides of HIV-1 R region were inserted between the heterologous PAS and the cleavage site/GU-rich DSE element. The '3G-R20' sequence is the same as 'R.1-20' referred to elsewhere in the invention. Truncated variants were generated based on including either the 3x Gs or a single G ('1G) immediately downstream of the heterologous PAS (modelling use of genomes with '3G' or '1G' vRNAs). Sequences of the embedded R sequences are displayed in Figure 22. Vector supernatants were titrated on adherent HEK293T cells, followed by flow cytometry, and data plotted on a log10 scale.

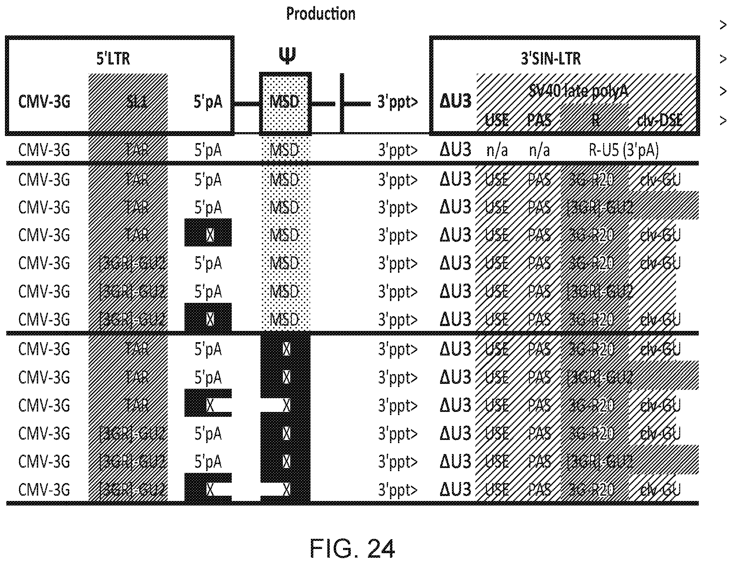

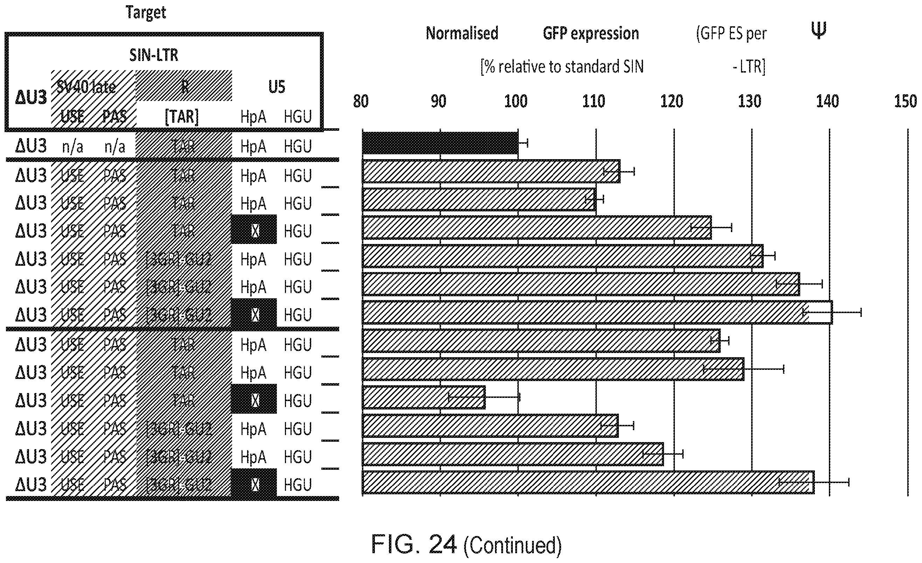

**Figure 24****. Use of supA-LTRs can increase transgene expression in target cells.** The configurations of 5' and 3' LTRs of the LV genome expression cassettes used during production - as well as the resulting final SIN-LTR generated in target cells - are indicated with or without different elements of the invention. All constructs were driven by the CMV promoter (with the '3G' TSS) and the 'back-up' polyadenylation sequence was absent except for the standard control (had SV40 late polyA downstream of the 3'SIN-LTR - not shown). The native HIV-1 5' PAS and the major splice donor (MSD) were optionally mutated, and an internal EFS-GFP-wPRE cassette (not shown). The 5' R region was either the wild type/standard TAR/SL1 or the modified 5' R SL1 comprised the 3GR-GU2 variant. At the 3' supA(SIN)-LTR, the heterologous SV40 late polyadenylation signal was positioned downstream of the 3'ppt/ΔU3 region. R region sequences composed of 20 nucleotides of HIV-1 R region were inserted between the heterologous PAS and the cleavage site/GU-rich DSE element. The '3G-R20' sequence is the same as 'R.1-20' referred to elsewhere in the invention. Alternatively, the 3GR-GU2 sequence was also employed in the 3' supA-LTR, effectively providing the embedded R sequence, the cleavage site (not shown) and the GU-rich DSE. LVs were produced in suspension (serum-free) HEK293T cells and used to transduce adherent HEK293T cells, followed by analysis of GFP expression by flow cytometry to generate titre values (GFP TU/mL). Adherent HEK293T cells were then transduced at matched MOI, and cell passaged for 10 days, followed by integration assay and flow cytometry. GFP Expression Scores (ES) were generated by multiplyin percent positive cells by the median fluorescence intensity (arbitrary units). These were normalised according to average packaging (Ψ) copy-number per cell, and then compared to the standard LV set to 100%.

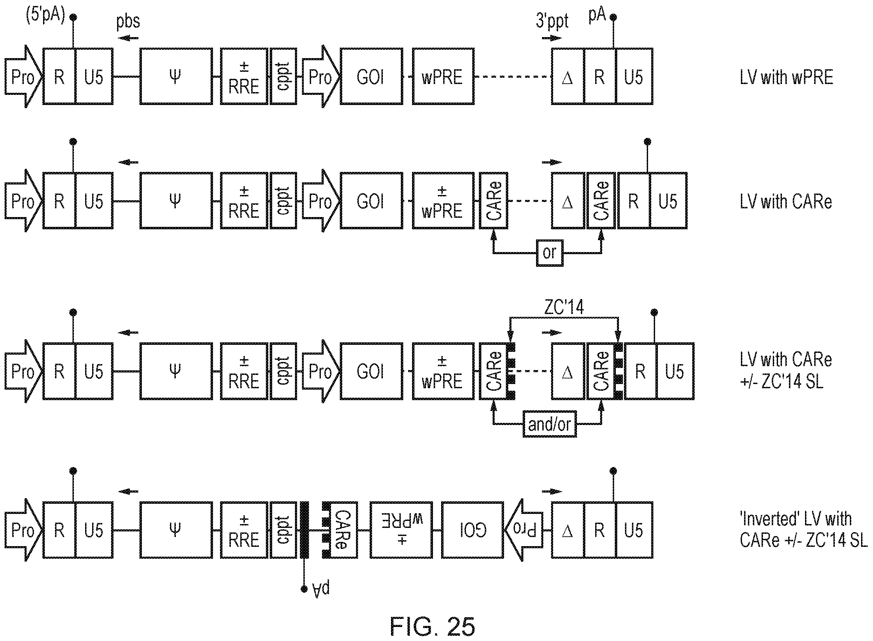

**Figure 25****. An overview of the positional use of CARe cis-acting elements for use alone or in combination with ZCCHC14 stem loop(s) and/or a PRE within lentiviral vector genomes.** The schematic shows the generalized structure of a lentiviral vector genome containing the RRE or a Vector-Intron (i.e. deleted for RRE) and internal transgene expression cassette encoding a gene of interest (GOI); such genomes typically utilize a PRE (such as wPRE) within the transgene 3' UTR. The PRE may optionally be entirely replaced with minimal CARe sequences alone or in combination with ZCCHC14 stem-loops (ZC'14 SL) up or downstream of the 3'ppt in order to reduce the size of the transgene cassette. For transgene cassettes inverted with respect to the forward directionality of the vector genomic RNA, the same cis-acting element options can be employed in the transgene 3'UTR, except there is no 3'ppt to consider.

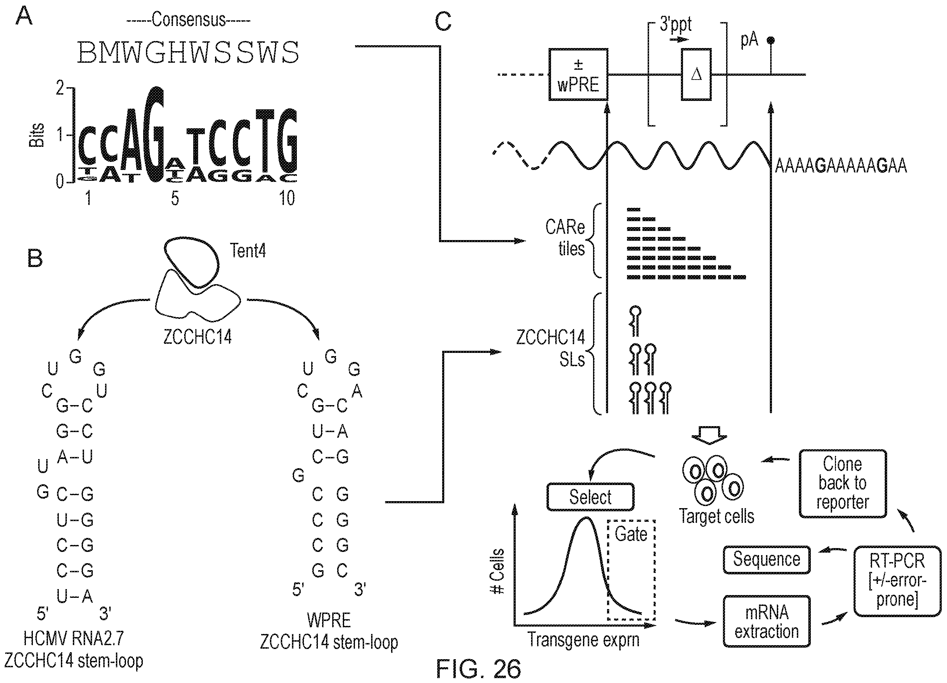

**Figure 26****. A detailed view of the CARe and ZCCHC14 stem loop sequences and their incorporation into the 3'UTR region of transgene cassettes within viral vectors.** A. The consensus sequence for the 10bp CARe core sequence (or 'tile' referred herein). B. Two nonlimiting examples of ZCCHC14 binding stem loops found within HCMV (RNA2.7) and WHV (wPRE). ZCCHC14 recruitment leads to formation of a complex with Tent4, which promotes mixed tailing in polyA tails of mRNAs, stabilizing them. C. The concept of insertion of CARe sequences into the 3' UTR of a viral vector transgene cassette (DNA at top, RNA shown as curvy line below DNA), optionally together with ZCCHC14 stem loops, taking care in retro/lentiviral vectors not to disrupt 3'ppt or *att* ('Δ'[i.e. ΔU3]) integration sequences required for reverse transcription and integration respectively. CARe sequences and optionally ZCCHC14 stem loops can be designed rationally or by library design, and screened empirically in target cells. For example screening can be done by viral vector transduction followed by selection of high-expressing cells (e.g. GFP FACS), followed by RT-PCR and sequencing of target mRNA to identify transcripts containing combinations of the cis-acting elements that lead to greater transgene expression and mRNA steady-state pools. This process can be repeated to enrich the best variants, whilst also optionally including error-prone RT-PCR to fine-tune sequences.

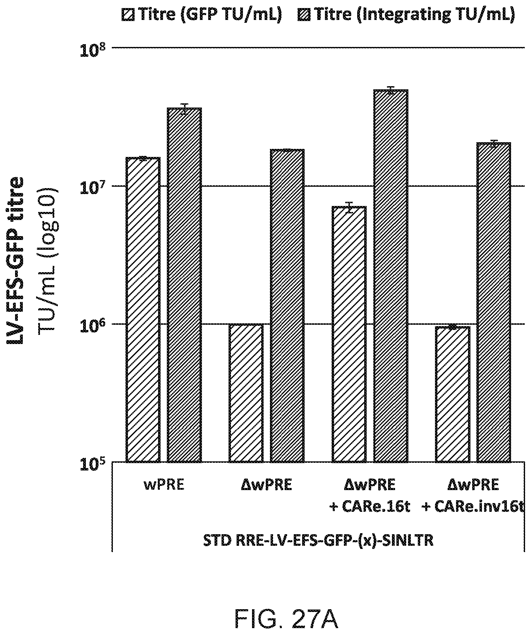

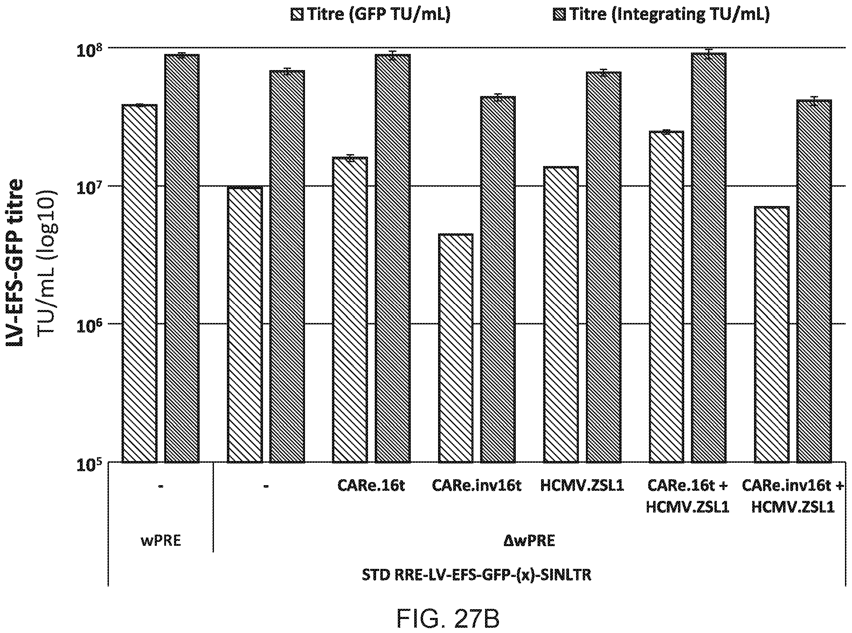

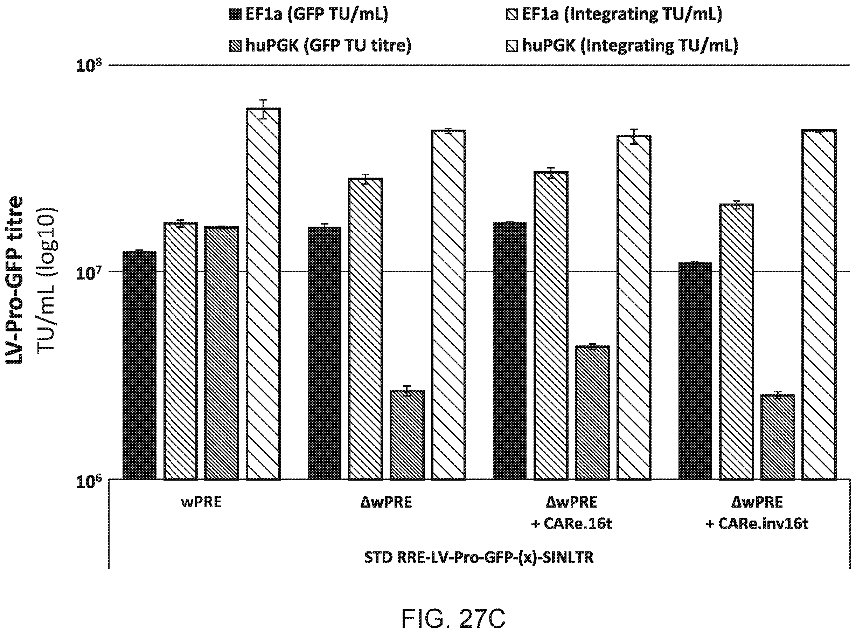

**Figure 27****. Production titres in suspension (serum-free) HEK293T cells of lentiviral vectors harbouring different transgene promoters combined with 3' UTR cis-acting elements. A** and **B** present data from two independent experiments for LV-RRE-EFS-GFP vectors containing different 3' UTR cis-acting elements: wPRE, ΔwPRE (wPRE deleted), 16x 10bp CARe sequences in sense (CARe.16t) or antisense (CARe.inv16t) and/or single copy of the ZCCHC14 stem loop from HCMV RNA2.7 (HCMV.ZSL1). C shows data for output titres of LV-RRE-EF1a-GFP (EF1a contains an intron) and LV-RRE-huPGK-GFP. Titres were measured by transduction of adherent HEK293T cells followed by flow cytometry based assay after 3 days (GFP TU/mL) or qPCR to LV DNA after 10 days (Integrating TU/mL). The data shows that integrating titres of LVs are comparable irrespective of the presence/absence of any of the cis-acting elements but that GFP titres vary, reflecting the expression levels in transduced adherent HEK293T cells. The 16x 10bp CARe tile (only) in the sense orientation provided a boost to LV GFP TU/ml titres lacking the wPRE.

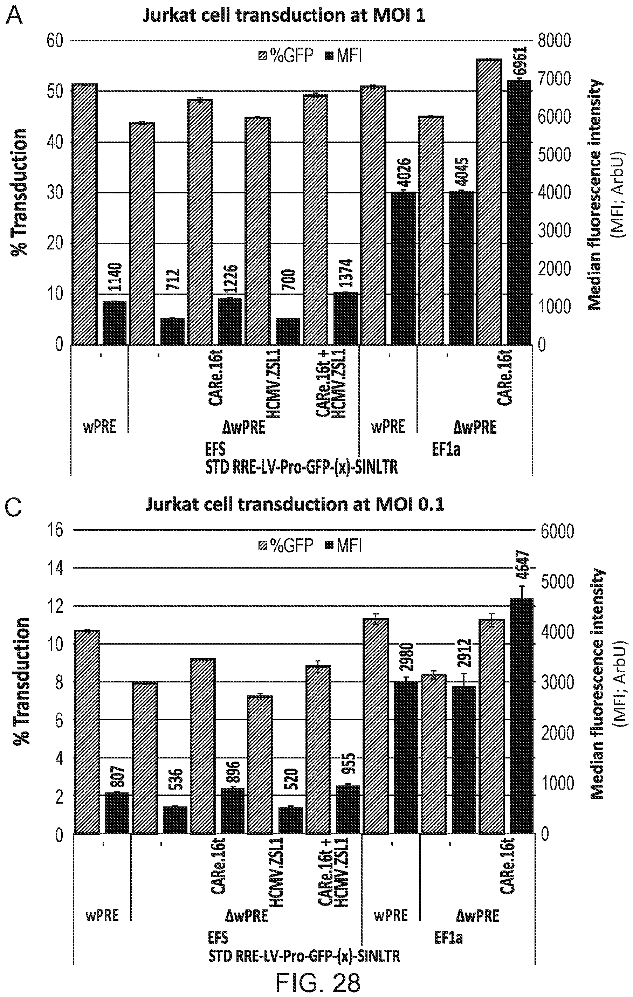

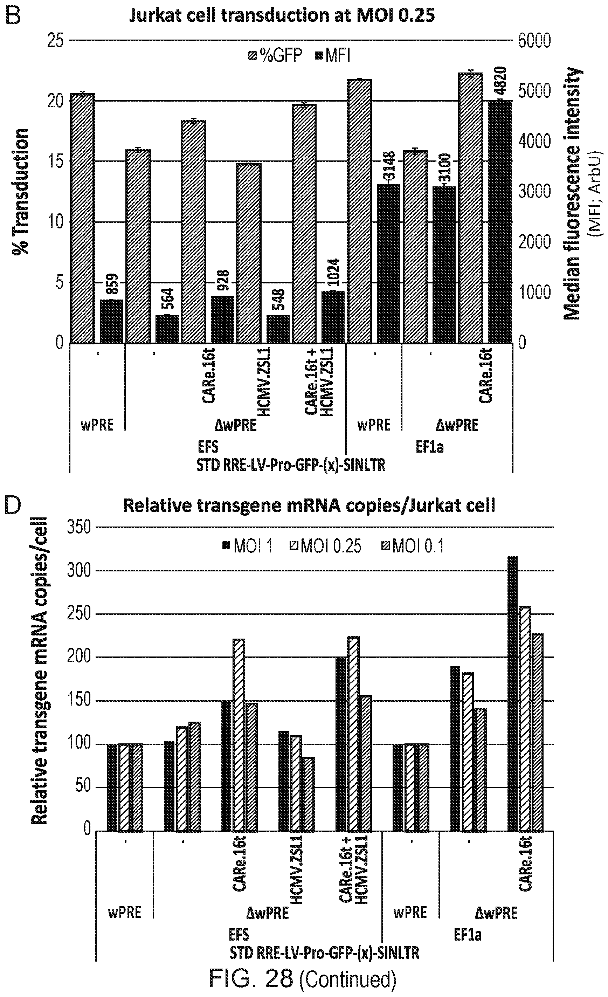

**Figure 28****. The 16x 10bp CARe tile boosts transgene expression from lentiviral vectors lacking wPRE in a T-cell line.** LV-RRE-EFS-GFP and LV-RRE-EF1a-GFP vector stocks produced in suspension (serum-free) HEK293Ts were used to transduce Jurkat cells at matched multiplicity of infection (MOI): MOI 1 **[A],** MOI 0.25 **[B]** and MOI 0.1 **[C].** Transduced cells were analysed by flow cytometry to obtain % GFP-positive values and median fluorescence intensity values (Arbitrary units). **D** displays data from normalized RT-PCR of extracted mRNA from the transduced cells, where the 100% level is set for each EFS-GFP or EF1a-GFP cassette containing the wPRE in each case. The data show that the 16x 10bp CARe tile (only) in the sense orientation restores transgene expression levels to those observed with wPRE-only, and for EF1a-GFP surprisingly boosts transgene expression levels higher the wPRE-only. The 16x 10bp CARe tile (only) in the sense orientation increase the levels of transgene mRNA above wPRE-only in all conditions.

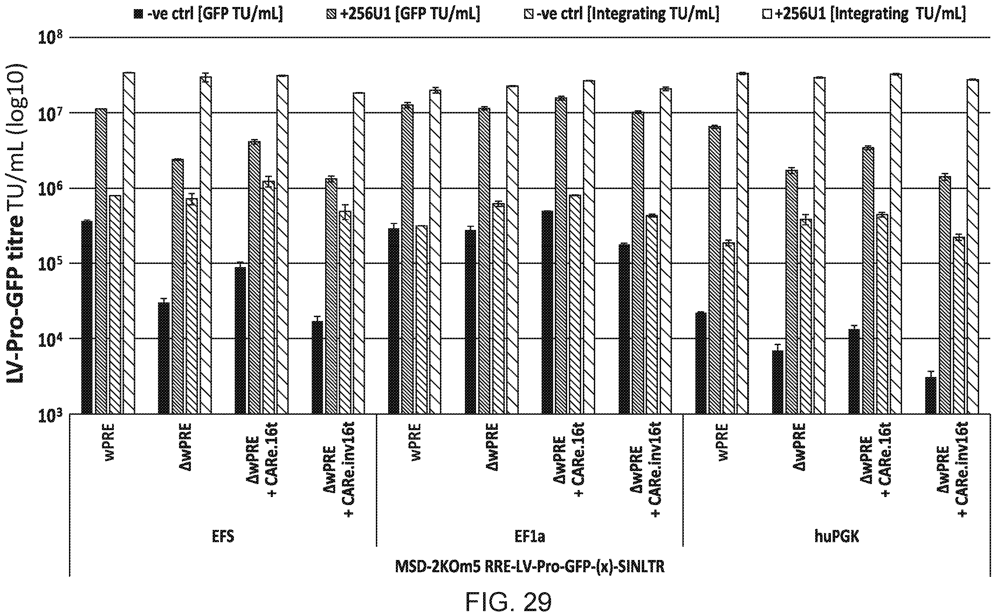

**Figure 29****. Production titres in suspension (serum-free) HEK293T cells of 'MSD-2KO'/'U1-dependent' lentiviral vectors harbouring different transgene promoters combined with 3' UTR cis-acting elements.** LV-RRE-Pro-GFP vectors containing mutations in the SL2 loop of the packaging signal (thus ablating aberrant splicing from this region) were produced +/- 256U1, a modified U1 snRNA that binds to the vector genomic RNA to restore titres. Three different transgene promoters (EFS, EF1a, and huPGK) and different 3' UTR cis-acting elements were employed: wPRE, ΔwPRE (wPRE deleted), and 16 x 10bp CARe sequences in sense (CARe.16t) or antisense (CARe.inv16t). Titres were measured by transduction of adherent HEK293T cells followed by flow cytometry based assay after 3 days (GFP TU/mL) or qPCR to LV DNA after 10 days (Integrating TU/mL).

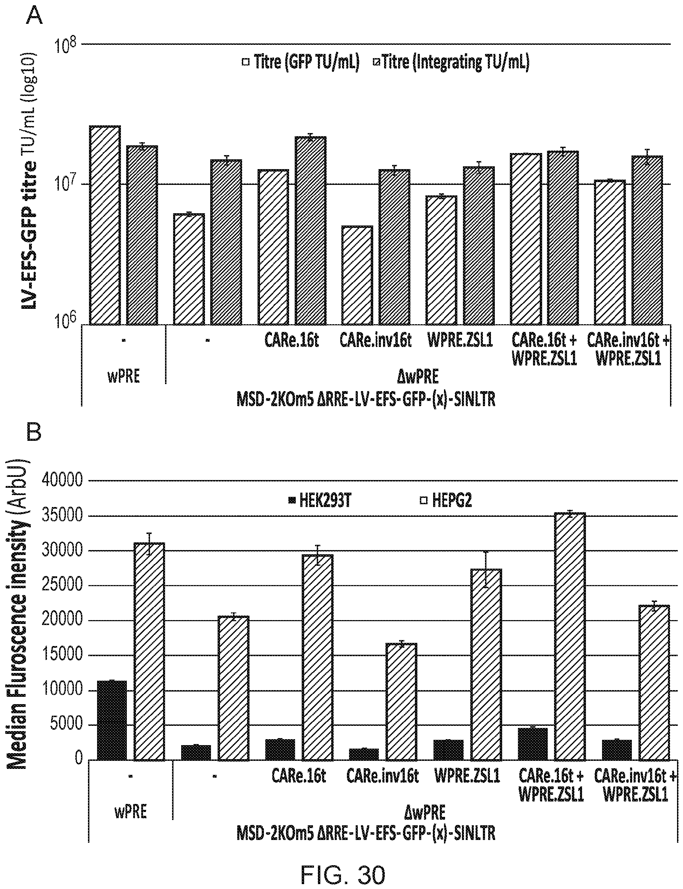

**Figure 30****. Production titres in suspension (serum-free) HEK293T cells of 'MSD-2KO'/' /ΔRRE' lentiviral vectors harbouring different 3' UTR cis-acting elements and transgene expression in target cells.** LV-VI(ΔRRE)-EFS-GFP vectors containing mutations in the SL2 loop of the packaging signal (thus ablating aberrant splicing from this region) were produced. Different 3' UTR cis-acting elements were employed: wPRE, ΔwPRE (wPRE deleted), 16x 10bp CARe sequences in sense (CARe.16t) or antisense (CARe.inv16t), and/or single copy of the ZCCHC14 stem loop from either HCMV RNA2.7 (HCMV.ZSL1) or WHV wPRE (WPRE.ZSI1). **A.** Titres were measured by transduction of adherent HEK293T cells followed by flow cytometry based assay after 3 days (GFP TU/mL) or qPCR to LV DNA after 10 days (Integrating TU/mL). **B.** Transgene expression in transduced adherent HEK293T or HEPG2 cells was measured by flow cytometry three days post-transduction at match MOI.

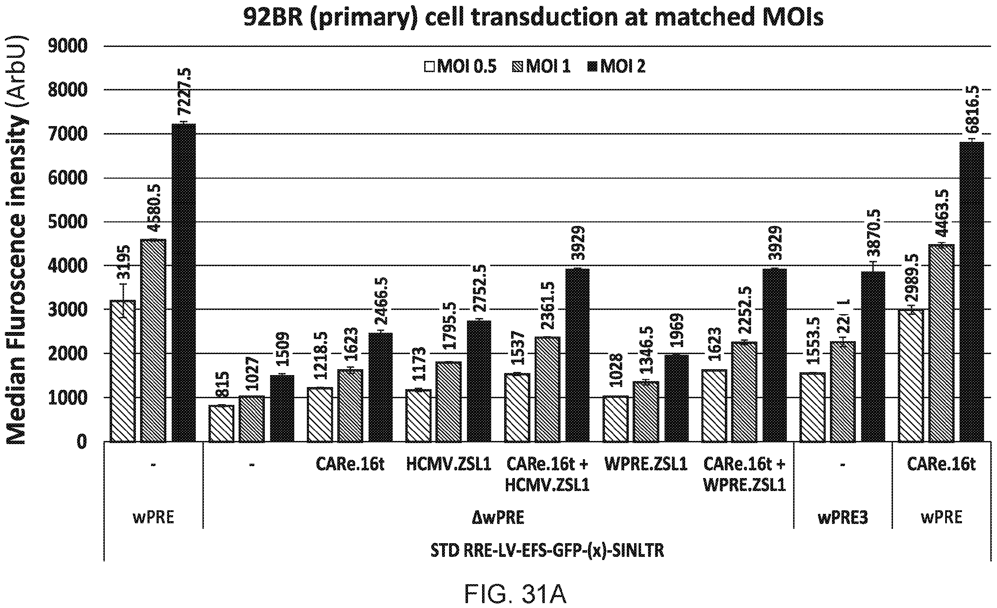

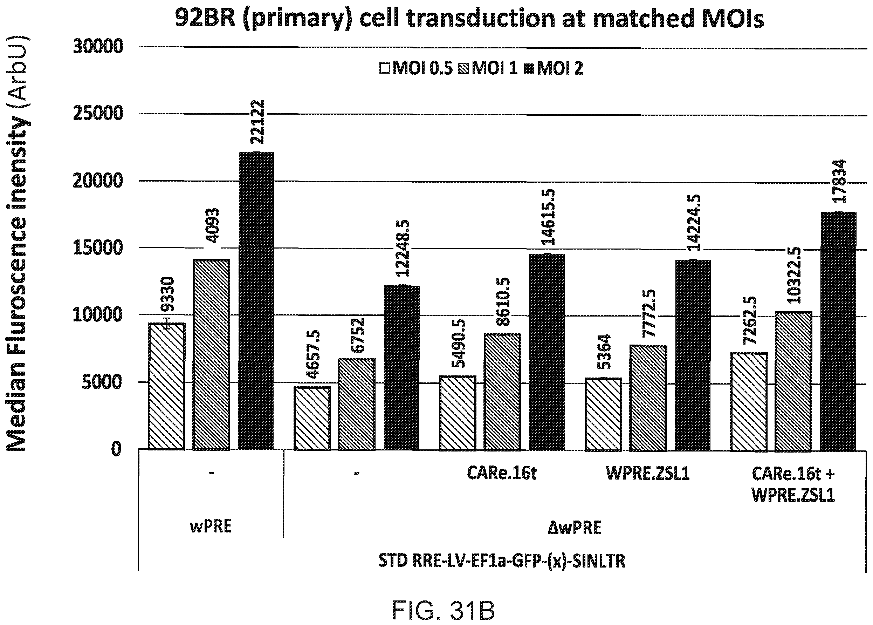

**Figure 31****. Transgene expression levels in primary cells transduced with RRE/rev-dependent lentiviral vectors harbouring different 3' UTR cis-acting elements.** LV-RRE-EFS-GFP **[A]** or LV-RRE-EF1a-GFP **[B]** vector stocks produced in suspension (serum-free) HEK293Ts were used to transduce primary cells (92BR) at matched multiplicity of infection (MOI): MOI 2, 1 or 0.5. Different 3' UTR cis-acting elements were employed: wPRE, wPRE3 (shortened wPRE), ΔwPRE (wPRE deleted), 16x 10bp CARe sequences in sense (CARe.16t) or antisense (CARe.inv16t), and/or single copy of the ZCCHC14 stem loop from either HCMV RNA2.7 (HCMV.ZSL1) or WHV wPRE (WPRE.ZSI1). Transgene expression in transduced adherent 92BR cells was measured by flow cytometry three days post-transduction at match MOI.

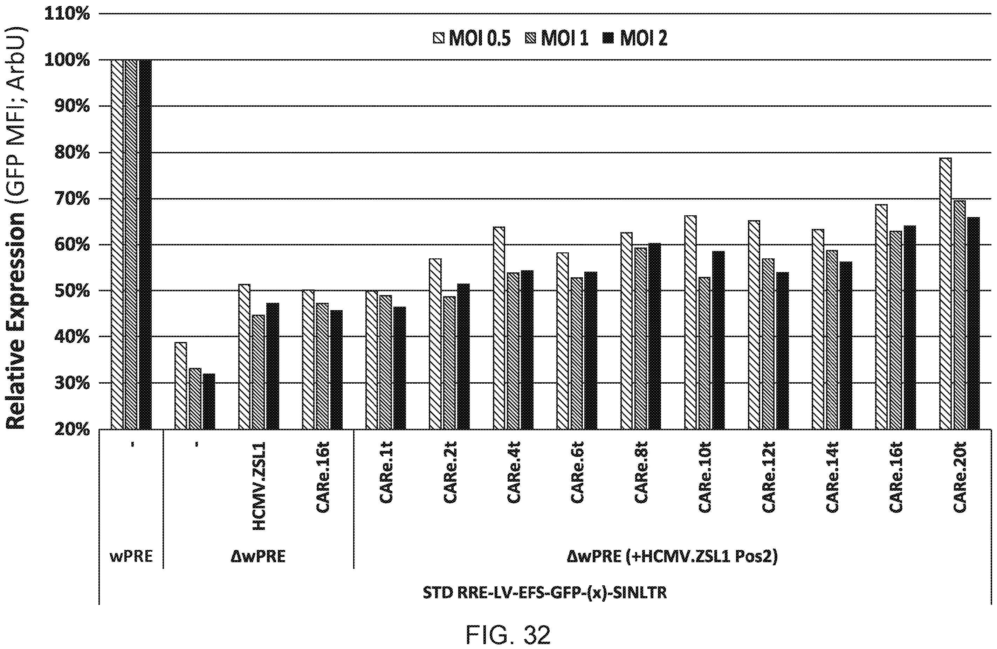

**Figure 32****. Transgene expression levels in adherent HEK293T cells transduced with RRE/rev-dependent lentiviral vectors harbouring different 3' UTR cis-acting elements at matched MOIs.** LV-RRE-EFS-GFP vector stocks produced in suspension (serum-free) HEK293Ts were initially titrated on adherent HEK293T cells to generate integrating titres (TU/mL). Vector stocks were used to transduce fresh adherent HEK293T cells at matched multiplicity of infection (MOI): MOI 2, 1 or 0.5. Different 3' UTR cis-acting elements were employed as 'stand-alone' elements: wPRE, 16x 10bp CARe tiles (CARe.16t) or a single copy of the ZCCHC14 stem loop (HCMV.ZSL1), compared to no element (ΔwPRE). Additionally, variants deleted for wPRE but containing a single copy of the ZCCHC14 stem loop were also paired with increasing numbers of CARe tile, from 1x to 20x 10bp copies. Transgene (GFP) expression in transduced adherent HEK293T cells was measured by flow cytometry three days post-transduction and median fluorescence intensities (Arbitrary units) normalised to that achieved with the standard wPRE-containing LV (set to 100%).

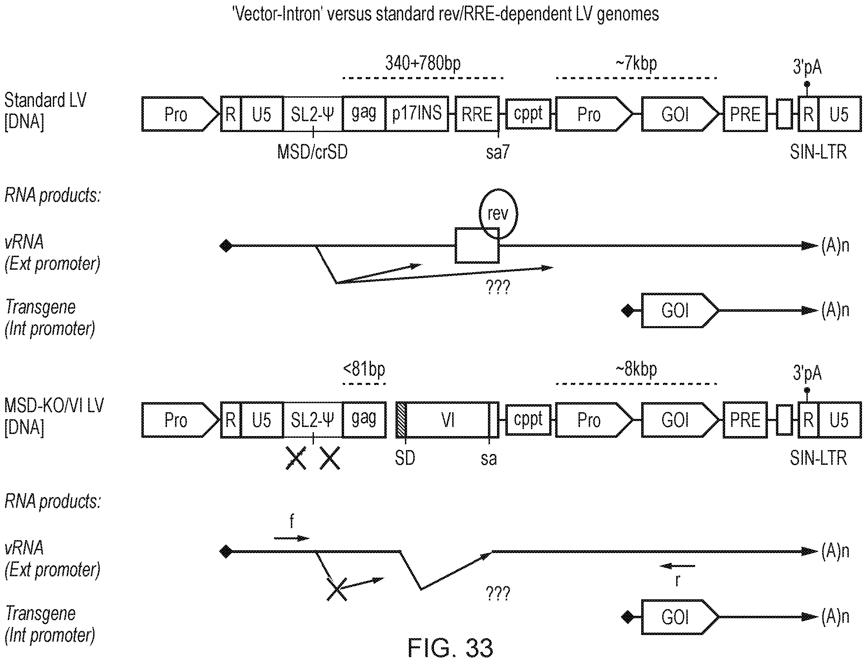

**Figure 33****: A schematic comparing DNA expression cassettes for standard and Vector-Intron containing LV genomes and the mRNAs transcribed therefrom.** The general structure of typical standard 3^{rd} generation LV genomes is shown, containing: a U3-deleted, tat-independent heterologous promoter driving transcription (Pro), the broad packaging sequence from R-U5 to the gag region, the RRE, the central polypurine tract (cppt), an internal transgene expression cassette (Pro-GOI), a post-transcriptional regulatory element (PRE) and a self-inactivating 3'LTR. The SL1 loop of the broad packaging sequence contains the MSD and adjacent crSD. The core packaging motif (Ψ) is within SL3. The amount of retained gag sequence can vary but is typically between ~340 and ∼690 nts from the primary ATG codon of gag, and includes the p17 instability element (p17-INS). The RRE is typically in the region of 780bp, and includes the splice acceptor '7' site (sa7) from HIV-1. For standard LV genome cassettes, apart from the main transgene mRNA (assuming the internal promoter is active in production cells), the primary transcript produced and exported to steady-state levels in the cytoplasm by rev was thought to be the full length vRNA. However, the inventors have shown elsewhere that promiscuous or aberrant splicing from the MSD or the crSD in the SL2 loop occurs to (cryptic) splice acceptors downstream of sa7 even in the presence of rev. The amount of spliced product compared to full length vRNA can be 20:1, especially when the transgene cassette contains a strong splice acceptor such as the one present in the EF1a promoter. The novel LV genome of the present invention replaces the RRE entirely with a single intron in order to increase transgene payload, since the intronic sequence will be absent from the full length vRNA. The MSD/crSD mutation ensures that no aberrant splicing from SL2 can occur with the VI splice acceptor. Surprisingly, not only does the act of splicing out of the VI allow vRNA to be stabilized in a rev/RRE-independent manner, it also abrogates the attenuating effect of MSD/crSD mutation on LV titres. Further, it is shown that RRE-deleted VI genomes achieve greatest titres in a rev-independent manner when the MSD/crSD is mutated. The novel LV genome may also incorporate a deletion of the p17-INS, therefore also increasing transgene capacity by a total of ~1kb.

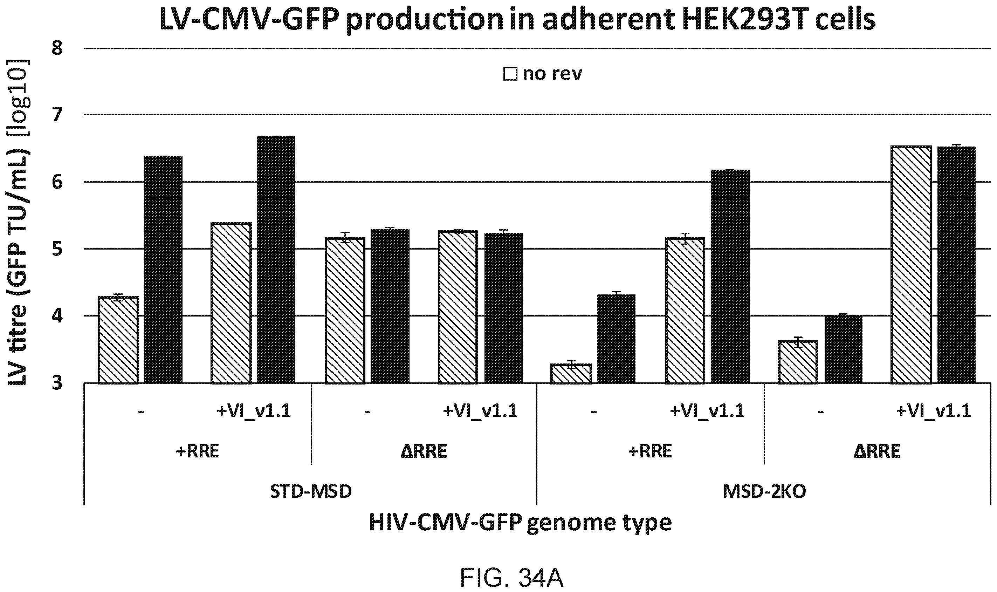

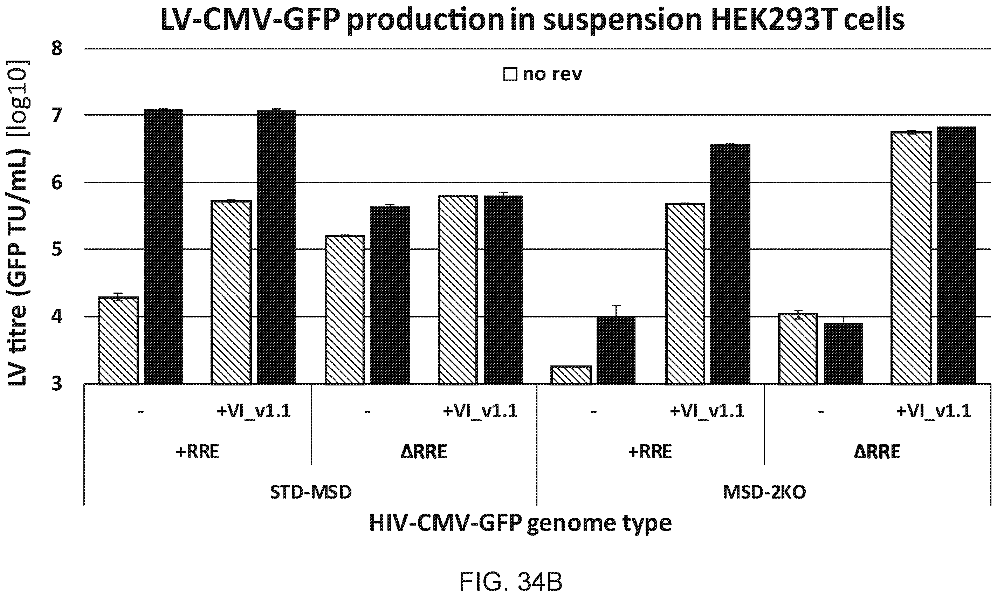

**Figure 34****: Rev/RRE-independent HIV-1 based LVs containing a Vector-Intron are improved by mutation of the major splice donor and cryptic splice donor sites in SL2 of the packaging signal.** HIV-1 based LV genomes (with an internal CMV-GFP cassette) were generated containing various combinations of either standard or mutated/deleted *cis-*acting elements (STD-MSD or MSD-2KO, ±RRE, ± Vector-Intron; see Figure 33). These genome plasmids were used to produce LV-CMV-GFP vectors in either adherent (A) or suspension [serum-free] **(B)** HEK293T cells in the presence or absence of a rev-expression plasmid. Clarified vector supernatants were titrated on adherent HEK293T cells using flow cytometry, and vector titres plotted on a log10 scale.

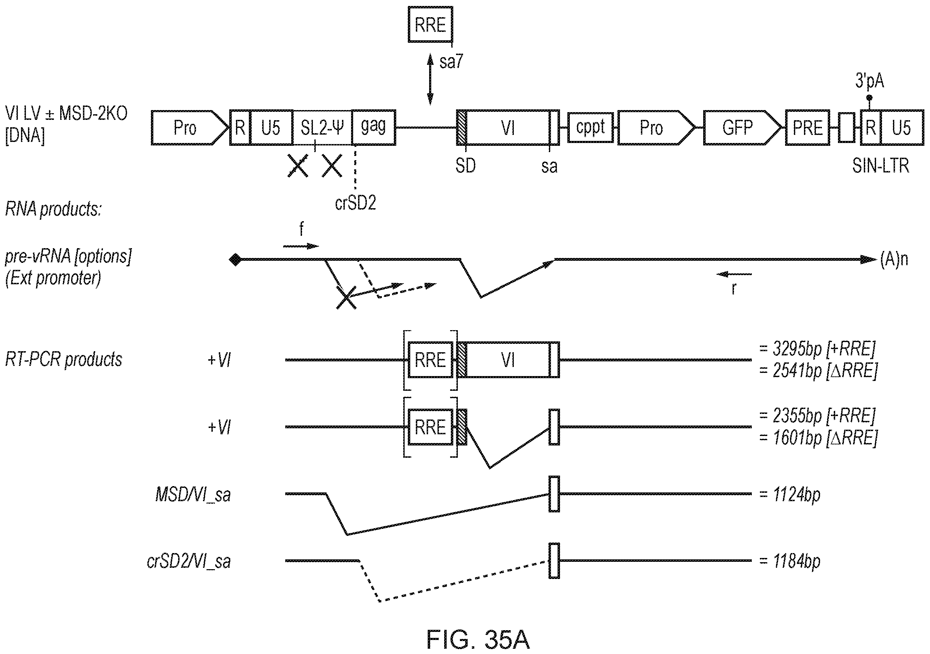

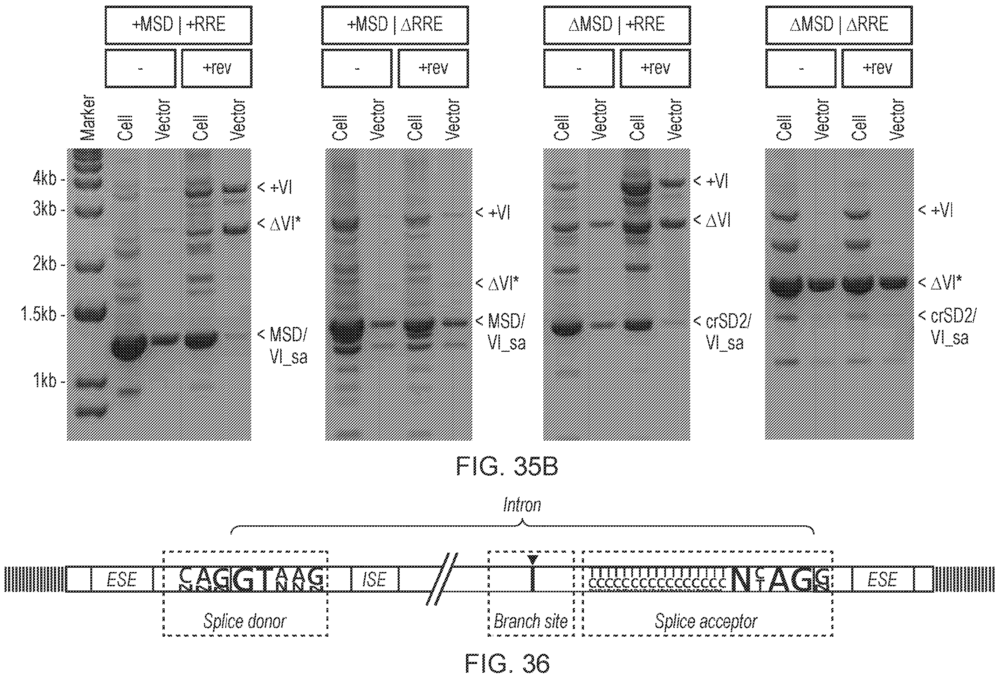

**Figure 35****: Analysis of vector cassette-derived RNA in adherent production cells and in resulting vector particles for variant genomes containing a Vector-Intron in combination with other *cis*-elements/mutations.** Total extracted RNA from production cells and vector particles from the adherent cell production run of Vector-Intron (VI_v1.1) genomes described for Figure 34A was subjected to RT-PCR to assess the species produced by each genome variant (panel B). The DNA, pre-RNA and main splicing products for these four Vector-Intron genome variants is shown schematically in panel A. The splicing of the Vector-Intron is denoted as well as the potential aberrant splicing of the MSD to the VI splice acceptor. The optional presence of the RRE is also denoted, as well as the positions of the PCR primers (grey arrows) used for the RT-PCR analysis (oligo-dT primer was used for the cDNA step).
**Figure 36****: A schematic showing the core features of exon-intron-exon sequences important for splicing.** The schematic shows a representative single intron (grey block) between two exons (black blocks), indicating the position of key consensus sequences required for splicing-out of the intron, as well as the position of enhancers. The termini of the intron are defined by GT-AG dinucleotides at the 5' and 3' ends respectively. The GT dinucleotide is the least variable sequence of the broader splice donor consensus sequence; the consensus sequence is the target of U1 snRNA, which anneals to the donor site early on during exon/intron boundary recognition. The AG dinucleotide is the least variable sequence of the broader splice acceptor consensus sequence, which typically comprises a polypurine tract of ~12-to-20 nts upstream. The Branch site (consensus = TNCTRAC, wherein "N" means any nucleotide and "R" means A or G) is located 20-to-35 nts upstream of the spliced acceptor site and is the target of U2 snRNA, which anneals to the branch site during the splicing reaction. The Branch site is also the anchor point for the linkage of the 5' end of the intron to form the lariat structure. The length of the intron can be short or many thousands of nucleotides, and may contain other cis-acting elements, with some partaking in enhancing or regulating splicing efficiency. Intronic splicing enhancers (ISEs) may be located closer to the ends of the intron so as to be in close proximity to the core elements described above. Exonic splicing enhancers (ESEs) may also be located close to the exon-intron junction in order to mediate effects. In the present invention, a number of these functional elements from different organisms were evaluated towards the optimization of the Vector-Intron approach.

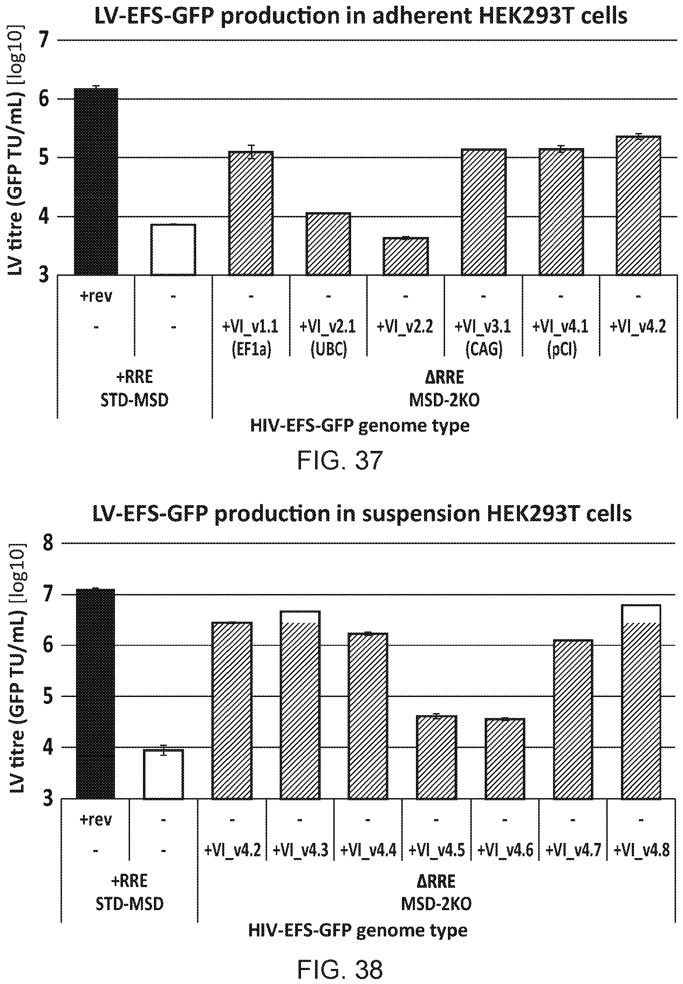

**Figure 37****: Assessment of initial Vector-Intron variants in HIV-1 based LVs in adherent HEK293T production cells.** Genome plasmids harbouring an EFS-GFP transgene cassette but lacking the RRE were constructed to contain the MSD-2KO mutations and six variant Vector-Introns, as per Table 7. These were based on native introns from the EF1a or Ubiquitin (UBC) promoter-introns, or the CAG promoter-intron or the small chimeric intron (Syn) from the pCI series of expression plasmids by Promega. These genome plasmids were used to produce LV-EFS-GFP vectors in adherent HEK293T cells in the absence of a rev-expression plasmid, whereas a standard LV vector was made +/- rev. Clarified vector supernatants were titrated on adherent HEK293T cells using flow cytometry, and vector titres plotted on a log10 scale.
**Figure 38****. Further development of Vector-Intron variants based on a chimeric intron in HIV-1 based LVs in suspension (serum-free) HEK293T production cells.** Genome plasmids harbouring an EFS-GFP transgene cassette but lacking the RRE were constructed to contain the MSD-2KO mutations and seven variant Vector-Introns VI_v4.2-4.8, as per Table 7. These were based on the small chimeric intron from the pCI series of expression plasmids by Promega but varied mainly in the presence/type of upstream ESE and/or splice donor sequence. These genome plasmids were used to produce LV-EFS-GFP vectors in suspension (serum-free) HEK293T cells in the absence of a rev-expression plasmid, whereas a standard LV vector was made +/- rev. Clarified vector supernatants were titrated on adherent HEK293T cells using flow cytometry, and vector titres plotted on a log10 scale.

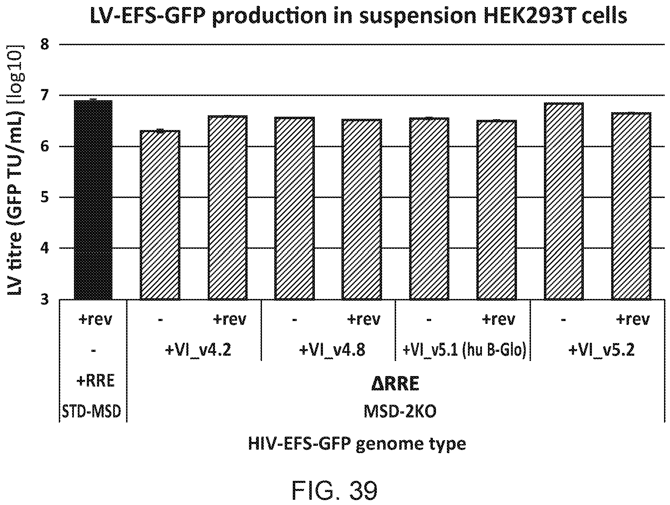

**Figure 39****. Further development of Vector-Intron variants based on the human β-globin intron-2 in HIV-1 based LVs in suspension (serum-free) HEK293T production cells.** Genome plasmids harbouring an EFS-GFP transgene cassette but lacking the RRE were constructed to contain the MSD-2KO mutations and two Vector-Introns VI_v5.1/5.2 based on the second (truncated) intron of the human β-globin gene, as per Table 7. These were compared to two of the previous Vector-Intron variants based on the chimeric intron from the pCI series of Promega plasmids (VI_4.2/4.8). These genome plasmids were used to produce LV-EFS-GFP vectors in suspension (serum-free) HEK293T cells in the presence/absence of a rev-expression plasmid, whereas a standard LV vector was made +rev. Clarified vector supernatants were titrated on adherent HEK293T cells using flow cytometry, and vector titres plotted on a log10 scale.

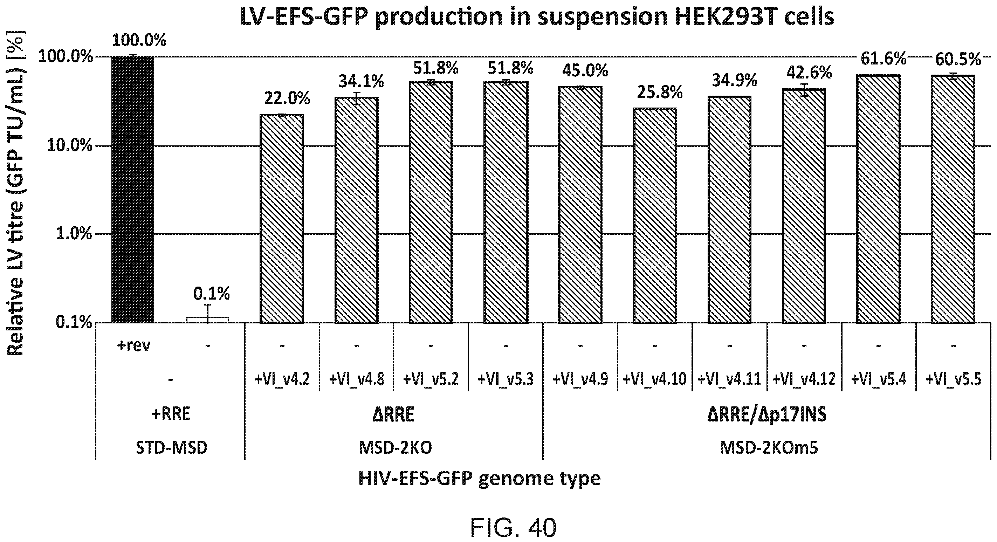

**Figure 40****. Testing of LV genomes with Vector-Introns in combination with different MSD-mutations and p17-INS(gag) deletion.** Vector-Intron variants from two series (v4 [pCl] and v5 [hu β-Globin]) were tested in LV genomes in the context of two different MSD-mutation variants ('MSD-2KO' and 'MSD-2KOm5'), and additionally with the p17-INS deleted from the gag region of the packaging sequence. These genome plasmids were used to produce LV-EFS-GFP vectors in suspension (serum-free) HEK293T cells in the absence of a rev-expression plasmid, whereas a standard LV vector was made +/-rev. Clarified vector supernatants were titrated on adherent HEK293T cells using flow cytometry, and titres normalized to the Standard LV-GFP vector prep made with rev.

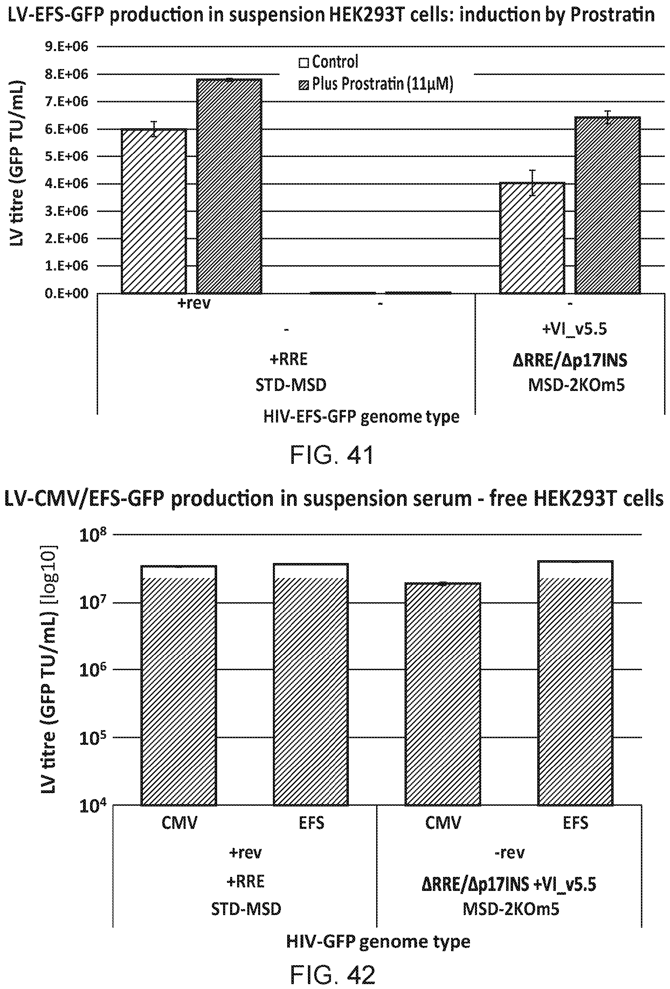

**Figure 41****. Evaluation of titre increase by Prostratin on Vector-Intron LV genomes.** Standard or Vector-Intron/MSD-2KO/ΔRREΔp17-INS genome plasmids were used to produce LV-EFS-GFP vectors in suspension (serum-free) HEK293T cells in the absence of a rev-expression plasmid, whereas a standard LV vector was made +/- rev. Vectors were made in the presence or absence of 11µM Prostratin ~20 hours post-transfection (at sodium butyrate induction step). Clarified vector supernatants were titrated on adherent HEK293T cells using flow cytometry, and vector titres plotted on a linear scale.
**Figure 42****. Rev-independent production of Vector-Intron LVs containing different transgene promoters.** HIV-1 based LVs containing CMV/EFS driven transgene cassettes within either standard or Vector-Intron backbones were produced to high titres in suspension (serum-free) HEK293T cells, in the presence or absence of rev, respectively. Vector titres are plotted on a log10 scale.

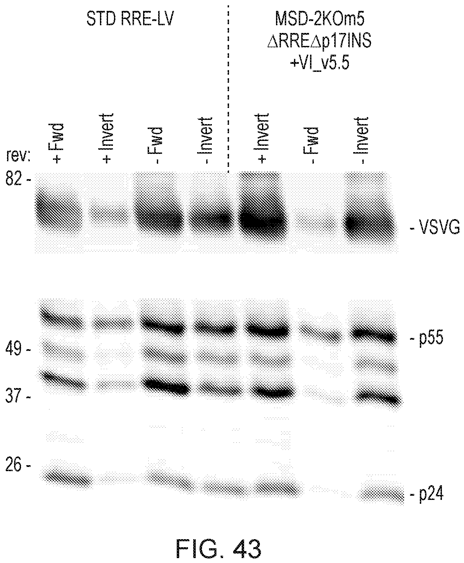

**Figure 43****. Utilisation of LV genome cassettes containing inverted transgene cassettes expressed during LV production leads to suppression of *de novo* vector component protein expression via a cytoplasmic dsRNA sensing mechanism.** Standard RRE-containing LV genome plasmids (STD RRE-LV) or Vector-Intron genome plasmids (MSD-2KOm5/ΔRRE/Δp17-INS+VI_v5.5) [both 3G TSS constructs unless indicated] were generated with either forward (Fwd) or inverted (Invert) EFS-GFP transgene cassettes, and used to produce LV harvest supernatants in suspension (serum-free) HEK293T cells. Packaging plasmids (pGagPol and pVSVG) were co-transfected together with or without pRev were indicated. Supernatants were analysed by SDS-PAGE/immunoblotting to VSVG and p24 (capsid). The data indicate that inverted transgene cassettes induce suppression of *de novo* LV component synthesis, consistent with a cytoplasmic dsRNA sensing mechanism e.g. PKR.

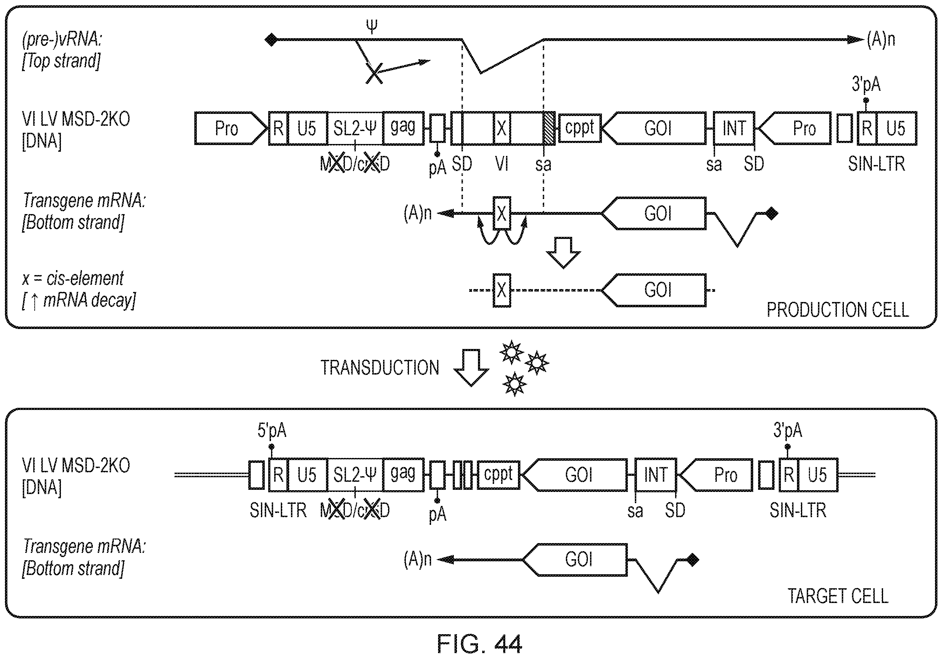

**Figure 44****. A schematic showing an example of a Vector-Intron LV genome with inverted transgene cassette.** A Vector-Intron LV genome with a reverse facing transgene cassette containing an intron is shown. Since the VI stimulates intron loss only from the vRNA (top strand-copied), the transgene cassette will retain its own intron. Depending on the strength of the transgene cassette promoter, a significant amount of double-stranded RNA may form between the vRNA and the transgene mRNA during LV production. This can potentially lead to a PKR response, cleavage by Dicer or deamination by ADAR; any or all of these mechanisms can contribute to reduced vector titres. This can be avoided by utilizing the unique features of the VI by inserting into it cis-acting element(s) (X) within the 3'UTR of the inverted transgene cassette. Such cis-acting elements are those that would reduce the abundance of only the transgene mRNA e.g. AU-rich [instability] elements (AREs), miRNAs, and/or self-cleaving ribozymes. The action of these reduce the amount of transgene mRNA available for pairing with the complementary vRNA to generate dsRNA. In addition, reduced transgene mRNA (and resultant protein) can be advantageous for LV production. Importantly, the cis-acting element(s) will not be present within the final integrated transgene cassette due to out-splicing of the VI, and therefore transgene mRNA stability in the transduced cell will be efficient.

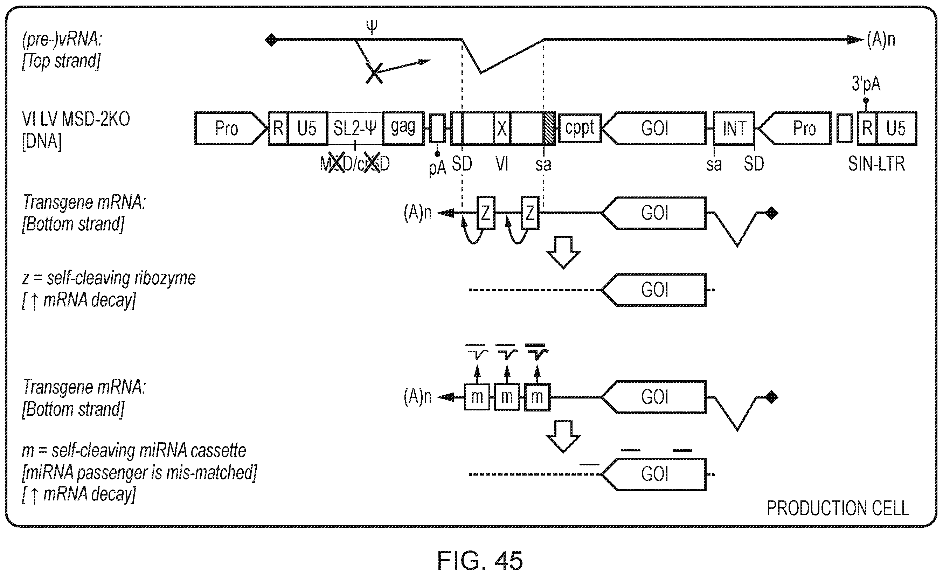

**Figure 45****. A schematic showing an example of a Vector-Intron LV genome with inverted transgene cassette and further details of cis-acting elements within the 3'UTR of the transgene cassette that mediate transgene mRNA degradation.** A Vector-Intron LV genome with a reverse facing transgene cassette containing an intron is shown during LV production. The use of 'functional' cis-acting elements ('X') within the 3'UTR of the transgene cassette - and located within the anti-sense VI sequence - can be used to achieve transgene repression and to avoid dsRNA responses during LV production. Two examples of functional cis-acting sequences are shown. Firstly, one or multiple self-cleaving ribozymes ('Z') can be inserted within the anti-sense VI sequence of the 3'UTR, leading to self-cleavage of pre-mRNA, resulting in RNA lacking a polyA tail and degradation in the nucleus. Secondly, one or multiple pre-miRNAs ('m') can be inserted within the anti-sense VI sequence of the 3'UTR, leading to pre-miRNA cleavage/processing resulting in cleavage of the pre-mRNA. Importantly, the miRNAs can be targeted to the transgene mRNA so that any mRNA that does locate to the cytoplasm is a target for microRNA-mediated cleavage (the guide strand should be 100% matched to its target). The vRNA will not be targeted by the guide strand. The passenger strand should be mis-matched with regard to the vRNA sequence to avoid cleavage of the vRNA should the passenger strand become a legitimate microRNA effector. Thus, both of these examples can be used to reduce/eliminate transgene mRNA (and dsRNA) only in LV production, since these functional cis-acting elements will be lost from the packaged vRNA due to loss of the VI.

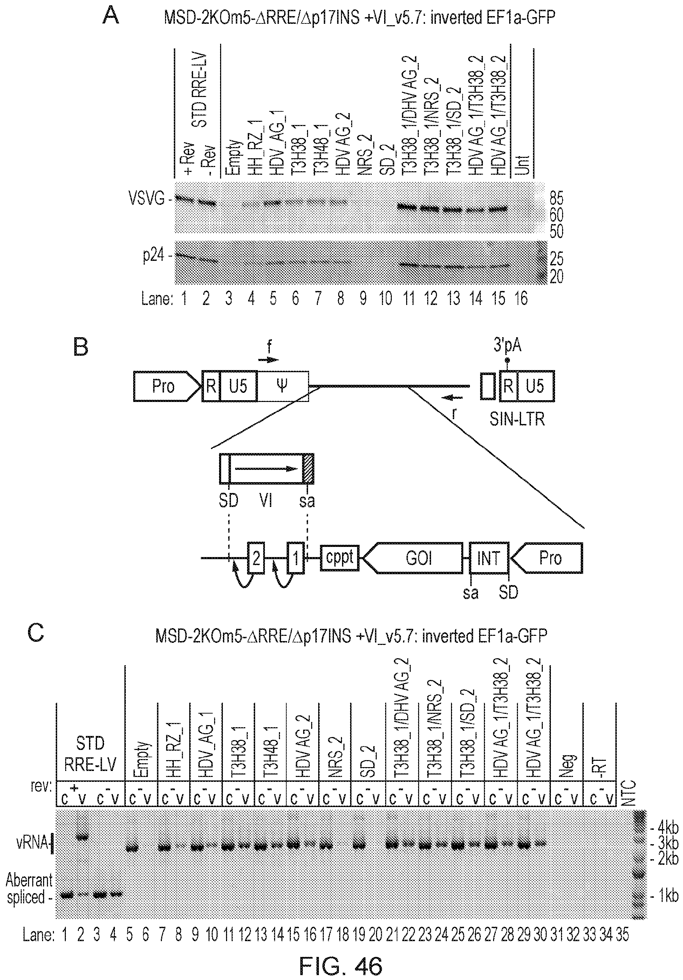

**Figure 46****. Use of self-cleaving ribozymes within the 3'UTR of inverted transgene cassettes to enhance LV virion production.** LV genome cassettes containing an inverted EF1a-GFP transgene with or without 'functionalized' 3' UTRs were used to produce LVs in suspension (serum-free) HEK293Ts, and vector proteins components within clarified harvest material analysed (panel A). Levels of vRNA were assessed by RT-PCR in both post-production cells ('C') and vector supernatant harvest ('V'). Expression of an inverted transgene cassette during LV production leads to double-stranded (ds) RNA, since the mRNA will be complementary to the majority of the LV vRNA. dsRNA is likely triggering at least one sensing mechanism during production (e.g. PKR), leading to a substantial reduction in detectable VSVG and p24 (capsid) in harvest material (panel A) and vRNA (panel C) - see 'Empty' lanes. Four different self-cleaving ribozymes were tested within the Vector-Intron (VI) region of the 3'UTR of the inverted transgene cassette: Hammerhead ribozyme (HH_RZ), Hepatitis delta virus ribozyme (HDV-AG), and modified *Schistosoma mansoni* hammerhead ribozymes (T3H38/T3H48) (see panel [B] for schematics and [A, C for data]. Additionally, variants were made harbouring the 'negative regulator of splicing' (NRS) element from RSV, or a splice donor site, as these have been shown to impart destabilization effects on mRNA. Other variants included several of these cis-acting elements in the same 3'UTR, with upstream/downstream positions noted as [1] or [2] respectively. (The positions of the forward [f] and reverse [r] primers for RT-PCR analysis is indicated in panel B; other features such as cppt and wPRE are not shown). These elements were cloned into an LV genome containing the VI_5.7 variant, the MSD-2KOm5 modification and deletions in the gag-Psi and RRE (full deletion) regions. Vectors were produced alongside the standard, RRE/rev-dependent LV genome containing the cassette in the forward direction, which produces aberrant splice products (see panel C). The data show that use of self-cleaving ribozymes enables recovery of VSVG, p24 and vRNA with LV supernatants.

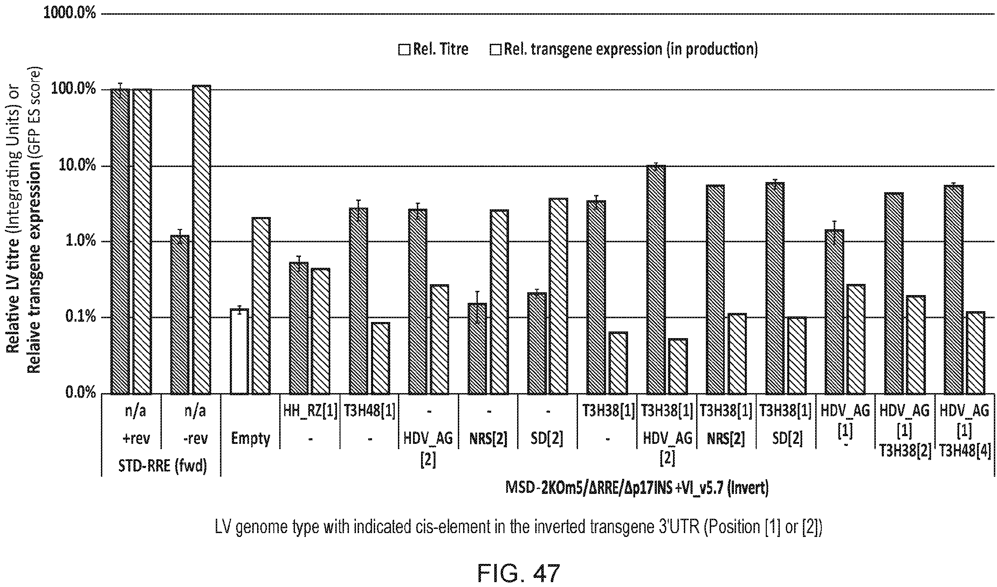

**Figure 47****. Use of self-cleaving ribozymes within the 3'UTR of inverted transgene cassettes to enhance LV titres.** LV harvest supernatants described in Example 20 and Figure 46 were titrated by integration assay in HEK293T cells. The data demonstrated that titres of VL LV genomes harbouring active inverted transgene cassettes are -1000-fold lower than STD RRE-LVs containing the same transgene in the forward (fwd) orientation (see 'Empty'). However, the use of self-cleaving ribozymes within the 3' UTR of the inverted transgene cassette enables ~100-fold recovery in titres. The presence of other cis-elements NSR and a splice donor site had no/minimal effect on titres.

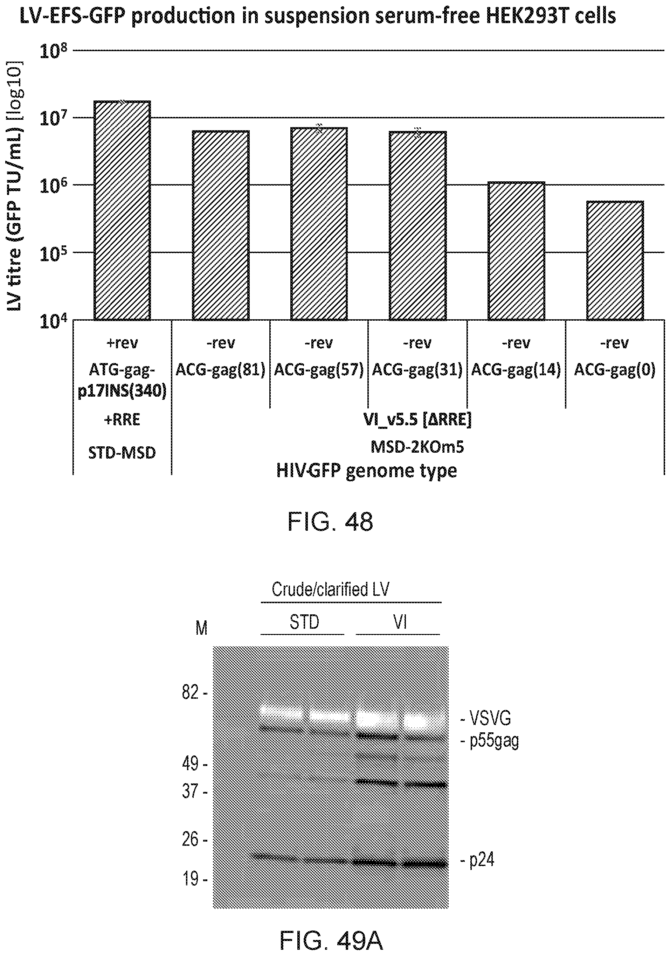

**Figure 48****. Use of minimal gag sequences as part of the packaging signal within Vector-Intron LV genomes.** The retained gag sequence within the packaging region of Vector-Intron genomes (reduced to 81 in other examples) was further minimized, resulting in constructs harbouring 57, 31, 14 or zero nucleotides of gag. All constructs harboured an ATG>ACG mutation in the primary initiation codon of retained gag sequence. All variants were presented within an MSD-2KOm5 LV genome containing VI_5.5 in place of the RRE. The standard LV genome contained the MSD, RRE and the first 340 nucleotides of gag (including the p17INS). LVs were produced in suspension (serum-free) HEK293T cells by transient transfection, and clarified harvests titrated on adherent HEK293T cells followed by flow cytometry.

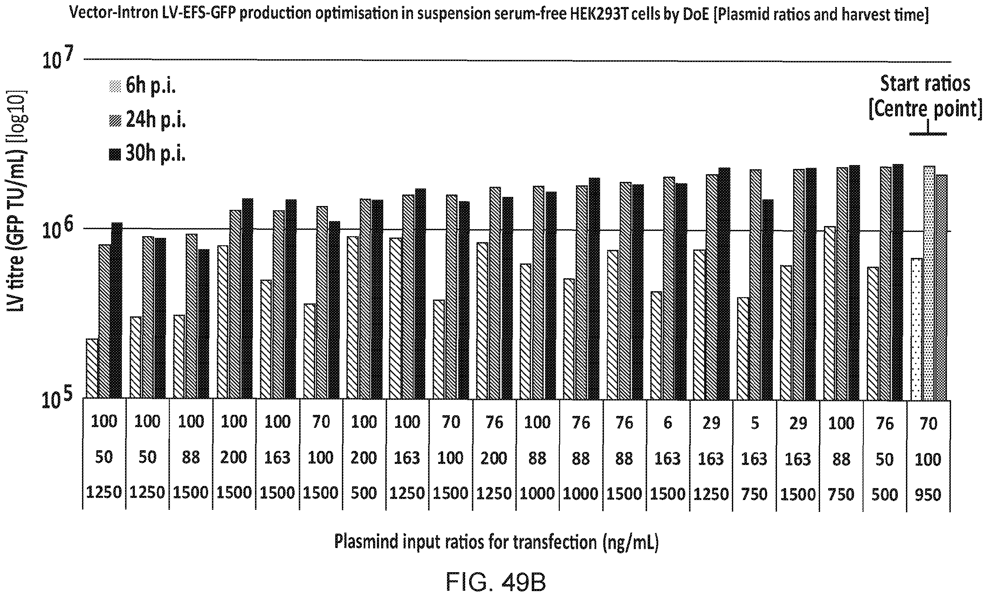

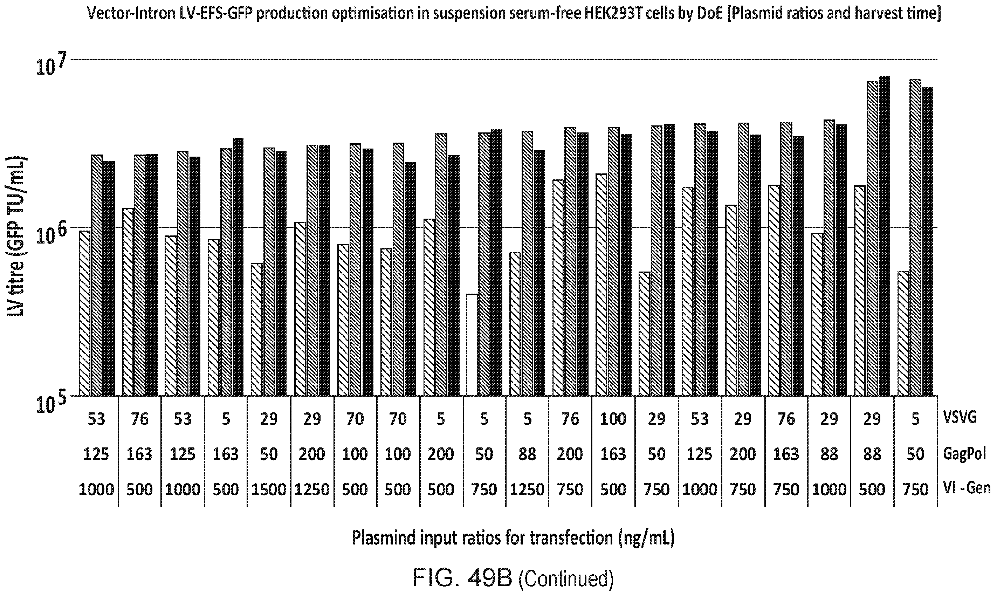

**Figure 49****. Optimisation of rev-independent production of Vector-Intron LVs: fine-tuning of vector component input levels. A.** Clarified standard (STD) or Vector-Intron (VI) LV vector supernatants (duplicate) were immunoblotted using antibodies to VSVG (white) or p24/capsid (black). M = molecular weight markers (kDa). **B.** A Design-of-Experiment (DoE) multivariate analysis experiment was performed using an MSD-2KOm5/Δp17INS/VI_5.5 LV genome encoding EFS-GFP. The control/centre point set of ratios (genome:gagPol:VSVG of 950:100:70 ng/mL) was the optimized ratio used for standard RRE/rev-dependent LVs (and all previous examples assessing VI LVs). LVs were produced LVs were produced in suspension (serum-free) HEK293T cells by transient transfection, and vector was harvested at the time points (hours) stated post-induction with sodium butyrate. Clarified harvests titrated on adherent HEK293T cells followed by flow cytometry.

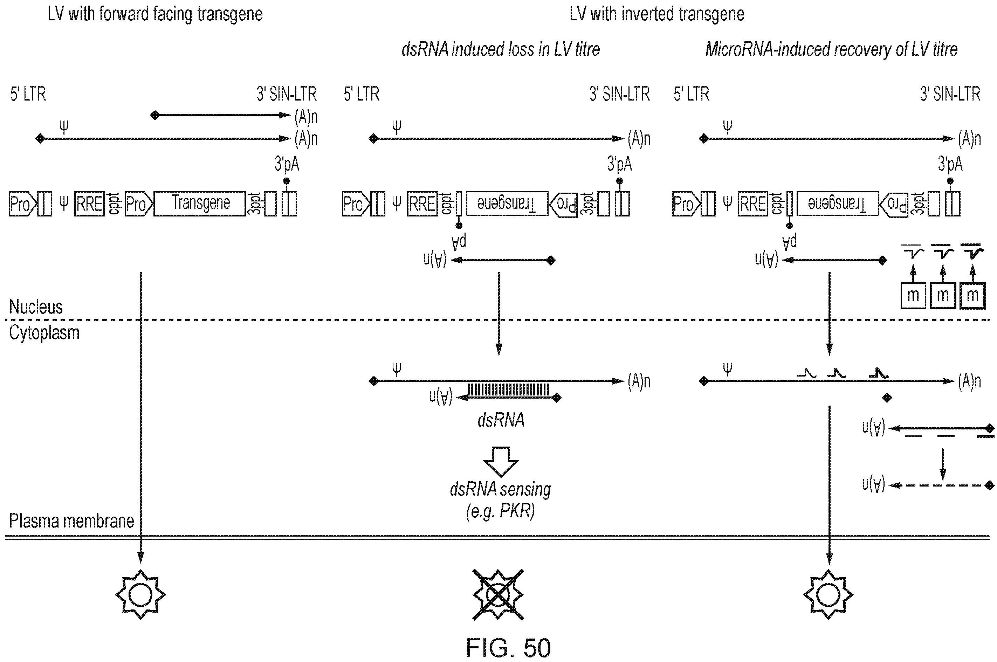

**Figure 50****. A schematic showing how microRNA targeted against the transgene mRNA** of **a lentiviral vector (LV) containing an inverted transgene cassette can be used to avoid production of dsRNA, and to reduce transgene expression.** The configurations of both forward facing and inverted transgene cassettes with LV genome expression cassettes are indicated, as are the packaged vRNA (Ψ) and transgene mRNAs in each case. Use of inverted transgene cassettes within retroviral vectors typically leads to a reduction in vector production due to the generation of long dsRNA; this typically induces dsRNA sensing pathways in the cell (such as PKR-mediated translation suppression), leading to reduction in vector component protein expression. To avoid this, one or more microRNAs can be co-expressed during vector production (e.g. by co-transfection with siRNA or with a microRNA expression cassette), wherein the microRNA targets the transgene mRNA for cleavage. Use of a mis-matched passenger strand can avoid loss of vRNA due to low level loading of the passenger strand as the guide within the RISC. Cleavage (and resultant degradation) of transgene mRNA leads to reduction in transgene protein expression during LV production, which can be advantage in achieving maximal titres and/or product recovery/purity.

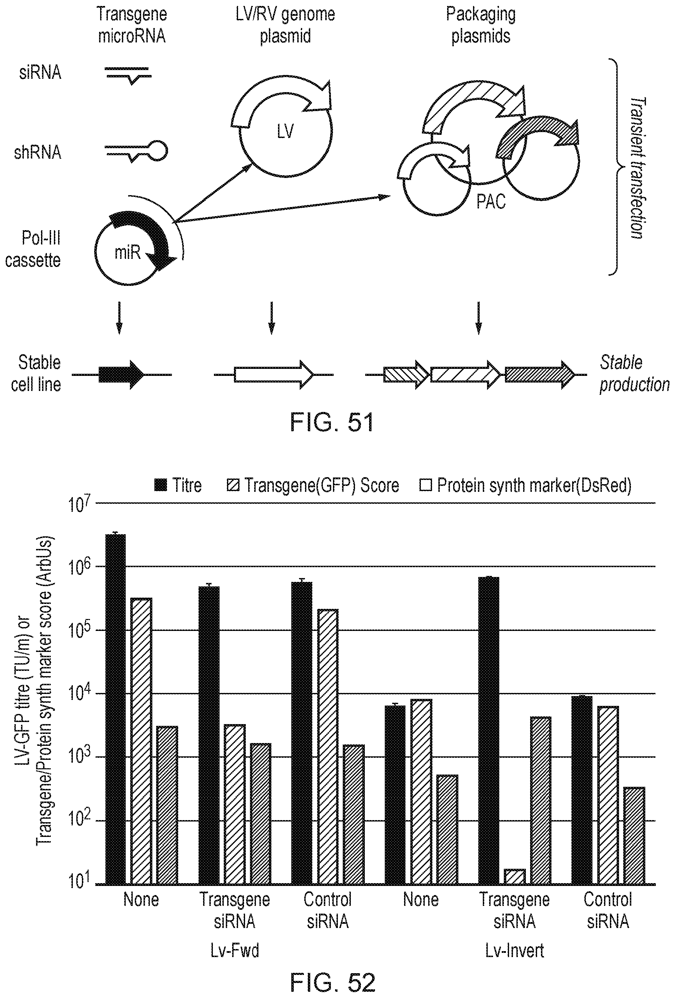

**Figure 51****. A schematic showing the different microRNA 'modalities' that can be adopted in the invention.** The transgene-targeting microRNA can be part of a 'transient' or 'stable' vector process using cell transfection or stable cell lines, respectively. For transient transfection approaches the microRNA can be delivered as siRNA or shRNA, or as a miR expression cassette, where the microRNA is transcribed *de novo,* for example, from a polymerase-III promoter such as U6 or a tRNA promoter. The miR cassette may be a separate plasmid or alternatively could be inserted within the vector genome plasmid or packaging plasmids. The miR may also be stably integrated into the production cell, which itself may or may not also contain the all or some of the vector components.
**Figure 52****. Production of LVs using siRNA to repress transgene expression from forward facing or inverted transgene cassettes.** LVs containing an EFS-promoter driven GFP cassette either in the forward (Fwd) or inverted (Invert) orientation were produced in suspension (serum-free) HEK293T cells. Production cells were co-transfected with LV genome and packaging plasmids with or without the stated siRNAs, as well as a DsRed-Xprs reporter plasmid control, and post-production cells analysed by flow cytometry for GFP/DsRed-Xprs expression levels (% positive gate x median fluorescence intensity; Arbitrary units). Clarified vector supernatants were titrated by transduction of adherent HEK293T cells followed by flow cytometry (Titre in TU/mL). The control siRNA was directed to Luciferase (not present), and the DsRed-Xprs reporter was present to assess the impact of dsRNA production on *de novo* protein synthesis.

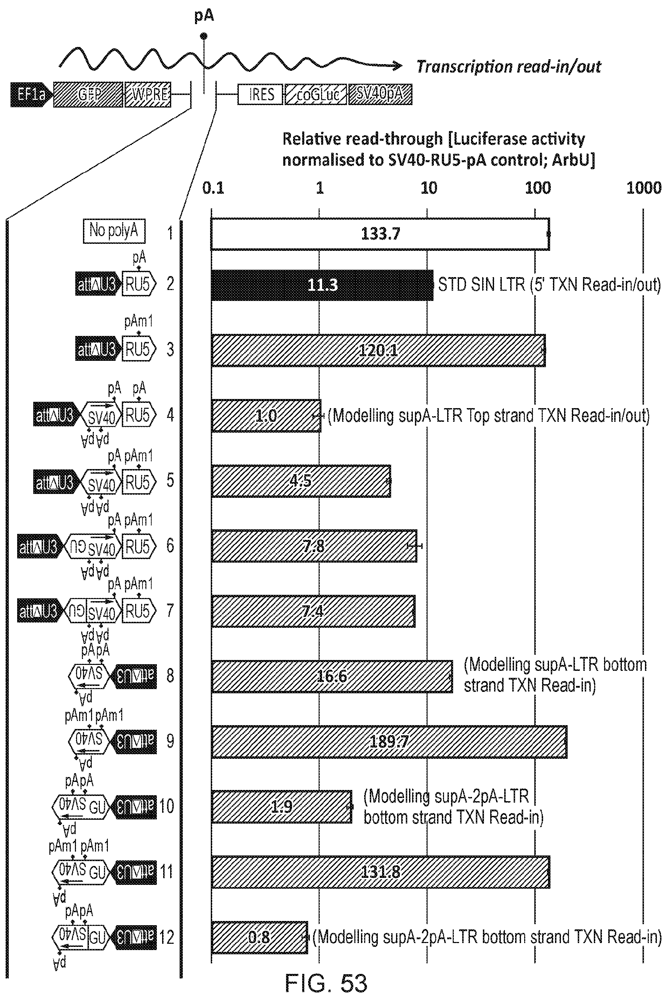

**Figure 53****. Building a supA-2pA-LTR: insertion of inverted polyadenylation signal and inverted GU-rich DSE within the SINΔU3 region to reduce transcriptional read-in from 3' end of integrated LVs.** A number of variants of SIN-LTR-like composite sequences were generated based upon positioning differing lengths of the SV40 polyadenylation sequence downstream of the SINΔU3 (*att*ΔU3) sequence, and harbouring mutations in different polyA signals (pAm1) present upstream of the RU5 (where indicated) or the native HIV-1 polyA signal (where indicated). The SV40 polyadenylation sequence is bi-directional, with the arrow indicating the direction of the late sequence, which included the late USE and polyA signal but not the late GU-rich DSE. Note that the modified (5') R - containing the optimal GU-rich DSE - could in principle have been use in place of the TAR to improve polyadenylation as shown elswehre where but was not in this initial example. In previous examples of supA-LTR sequences the late SV40 polyA sequence (containing the late USE-PAS sequence) includes the two polyA signals of the early SV40 polyA sequence on the bottom strand (i.e. inverted) but the early GU-rich DSE is omitted. Constructs 2-7 model the 'top-strand' orientation (i.e. transcriptional read-in from upstream cellular promoters), whereas constructs 8-12 model 'bottom-strand' orientation (i.e. transcription read-in from downstream cellular promoters), although the inverted RU5 sequence was not present in these inverted variants in this example. To provide a GU-rich DSE for the early SV40 polyA sequence, the native SV40 early polyA sequence was simply extended to include the native GU-rich DSE (contructs 6, 10, 11) or a variant GU-rich DSE based on the MMTV GU-rich DSE was inserted downstream of the two early PAS's (contructs 7, 12). These sequences were inserted into the luciferase polyA reporter and suspension (serum-free) HEK293T cells transfected, followed by luciferase assay of cell lysates to measure transcriptional read-in/out. The standard SIN-LTR and no sequence controls were included as positive and negative controls respectively. Luciferase activity was normalised to that of construct 4 (set at 1.0) and data displayed on a log10 scale (Arbritray units).

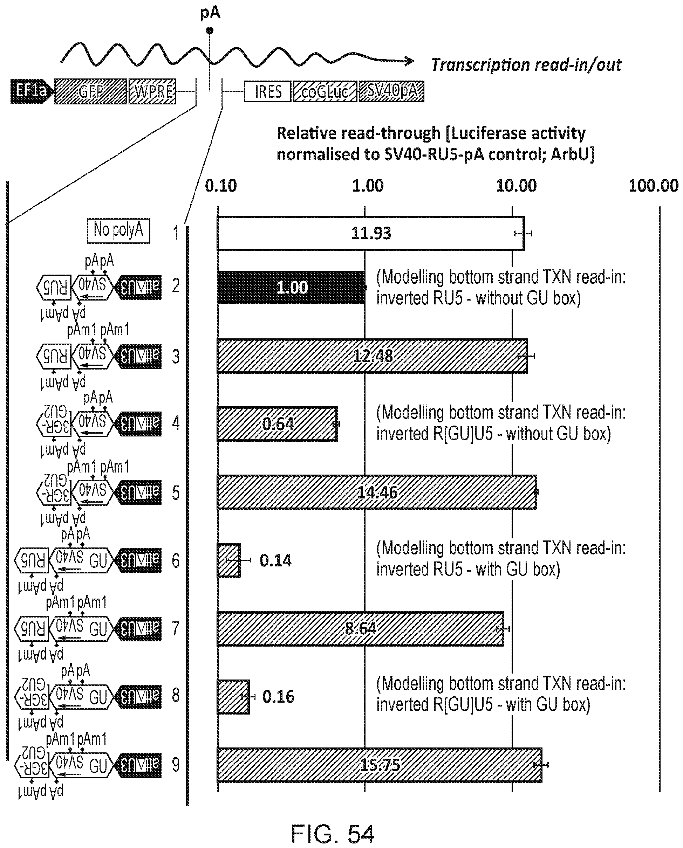

**Figure 54****. Modelling transcriptional read-in on the bottom strand of an intact 'supA-2pA-LTR'.** As per Figure 53, supA-LTR sequence was inverted and inserted into a luciferase reporter cassette, except that the inverted RU5 was also included so that an entire supA-/2pA-LTR was present. All constructs 2 - 9 model the 'bottom-strand' orientation (i.e. transcription read-in from downstream cellular promoters). The present construct 1 is identical to construct 1 of Figure 53 (Example 7); present constructs 2, 4 and correspond to construct 8 in Figure 53 (Example 7), except that the modified (5') RU5 or the modified (5') RU5 containing the optimal GU-rich DSE ('GU2') has been included where indicated to assess impact of these sequences; and present constructs 3, 5 and 9 correspond to construct 9 in Figure 53 (Example 7), except that the modified (5') RU5 or the modified (5') RU5 containing the optimal GU-rich DSE ('GU2') has been included where indicated to assess impact of these sequences. To provide a GU-rich DSE for the early SV40 polyA sequence, the native SV40 early polyA sequence was simply extended to include the native GU-rich DSE (constructs 6 - 9). Note the GU box is 'GU-1' as noted in Table 2. These sequences were inserted into the luciferase polyA reporter and suspension (serum-free) HEK293T cells transfected, followed by luciferase assay of cell lysates to measure transcriptional read-in/out. Luciferase activity was normalised to that of construct 2 (set at 1.0) and data displayed on a log10 scale (arbitrary units).

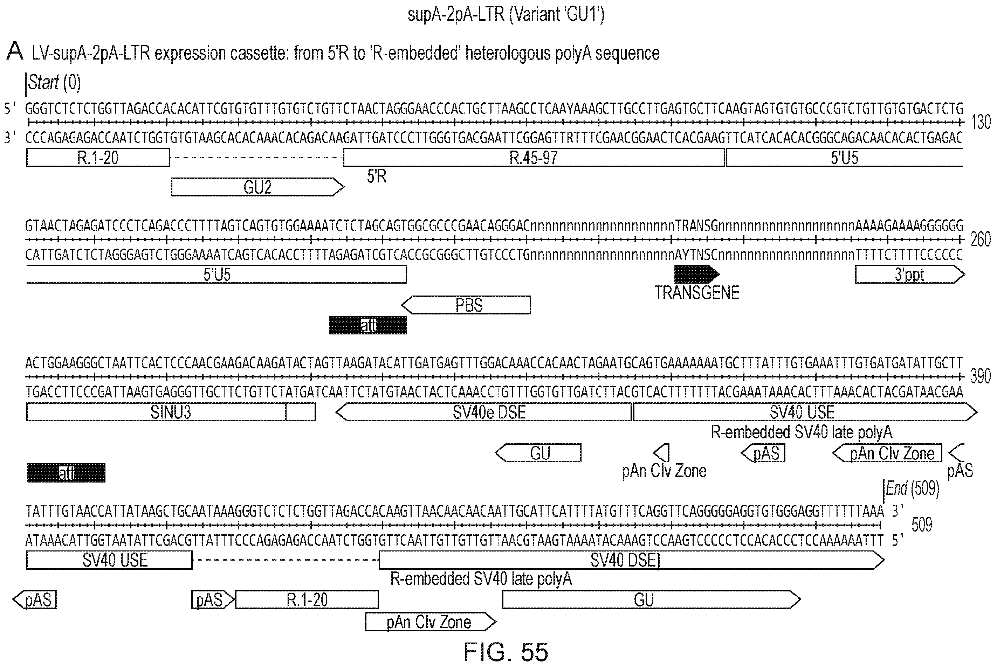

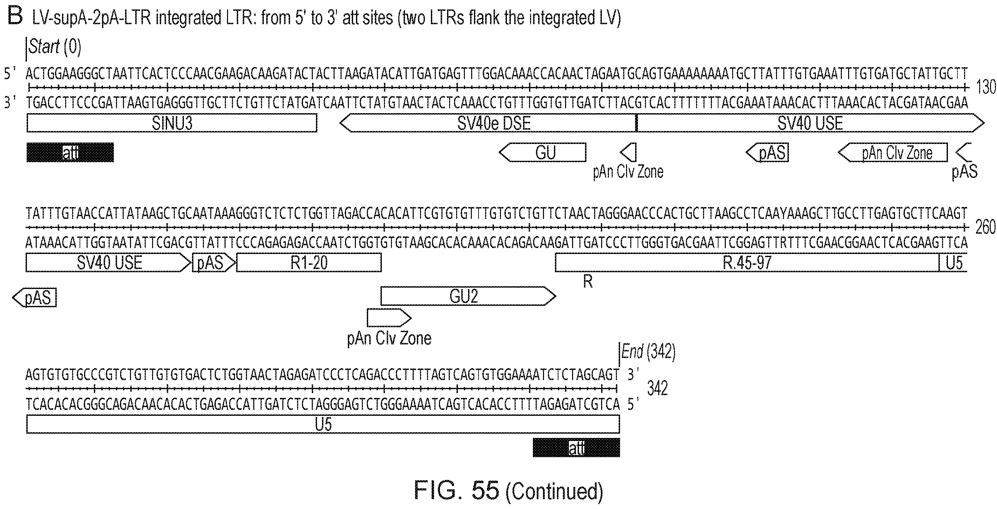

**Figure 55****. Detailed schematic of example supA-2pA-LTR as part of LV expression cassette and resultant LTR in target cells.** The sequences displayed conform to SEQ ID No: 200 **[A]** and 201 **[B],** as shown in Table 9. **[A]** gives the 5'R-to-PBS, and 3'ppt-to-R-embedded heterologous polyadenylation sequence (in this case the SV40 polyA), with the LV backbone and transgene sequences 'abbreviated' in between (no promoter sequence driving the cassette is shown). Grey features are typical HIV-1 based LV sequences of RU5 regions, the PBS and 3'ppt. The attachment sites (for integration) are shown in black ('att'). Modified sequences of the invention are shown in white features, the direction of which are indicated by arrows, showing whether the sequence functions on the top strand (pointing rightwards) or on the bottom strand (pointing leftwards). PolyA signals (pAS), Upstream enhancer (USE), polyA cleavage zone/region (pAn Clv Zone) and downstream enhancer/GU-rich box (DSE, GU) indicated. **[B]** displays the resultant LTR in target cells, after the completion of reverse transcription/cDNA synthesis; only one LTR is shown for simplicity, since both LTRs flanking the LV will be identical. The same features convention is used as per panel **[A].**

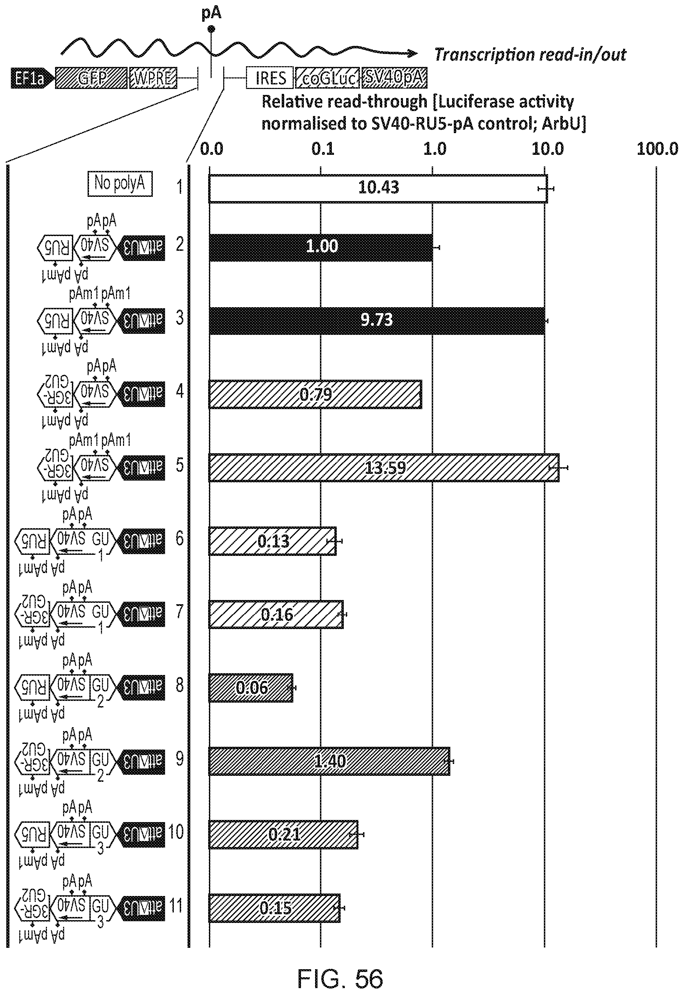

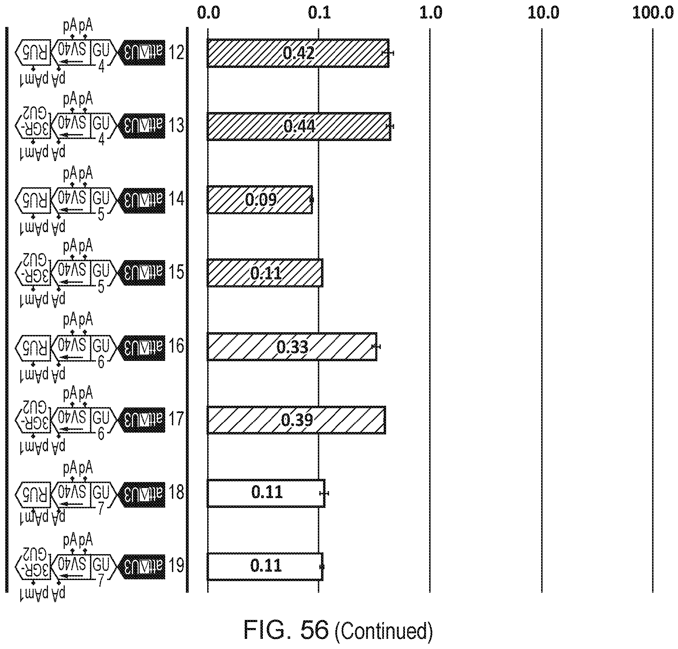

**Figure 56****. Further modelling transcriptional read-in on the bottom strand of a 'supA-2pA-LTR'; evaluating alternative GU boxes.** As per Figure 54, supA-LTR sequence was inverted and inserted into a luciferase reporter cassette, so that an entire supA-/2pA-LTR was present. All constructs 2-19 model the 'bottom-strand' orientation (i.e. transcription read-in from downstream cellular promoters). Bottom strand variants included seven different GU boxes from difference sources as denoted in Table 2, and the inverted RU5 variably encoded the GU2 modification where indicated. These sequences were inserted into the luciferase polyA reporter and suspension (serum-free) HEK293T cells transfected, followed by luciferase assay of cell lysates to measure transcriptional read-in/out. Luciferase activity was normalised to that of construct 2 (set at 1.0) and data displayed on a log10 scale (arbitrary units).

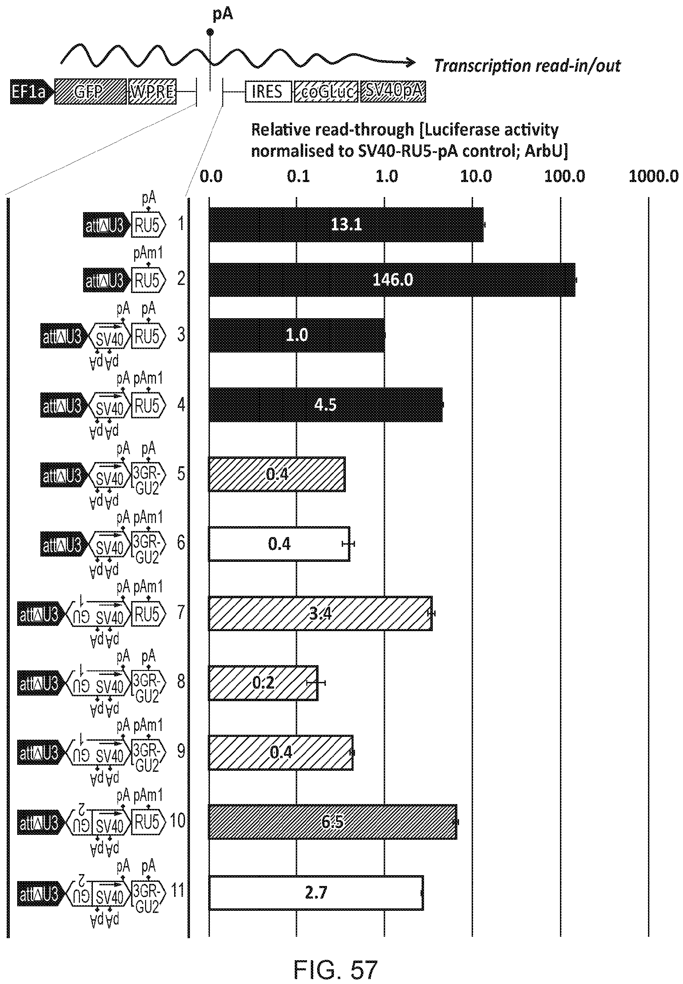

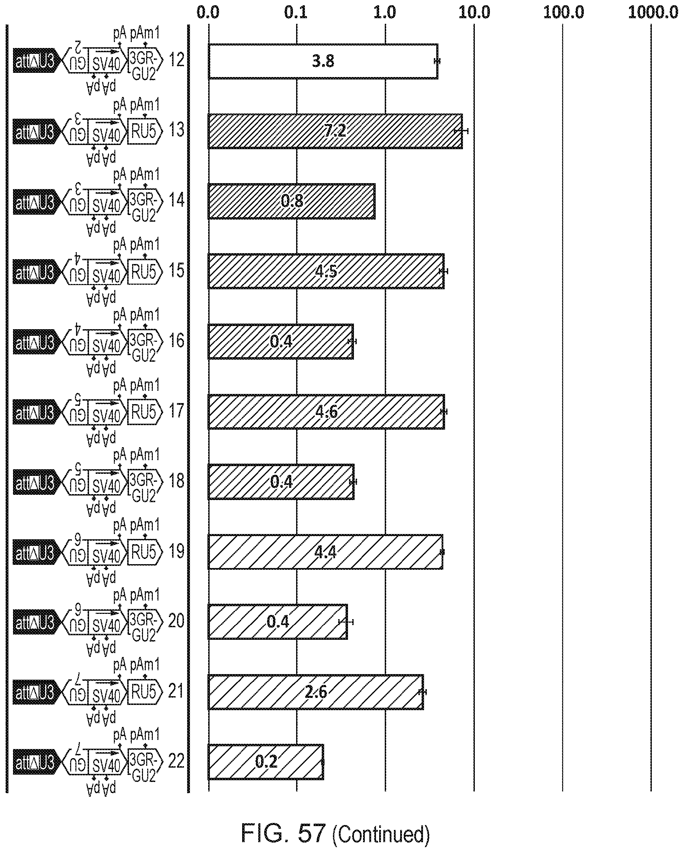

**Figure 57****. Assessing the impact of the inverted GU boxes on 'top strand' transcriptional termination.** The supA-2pA-LTR variants described in Table 10 and tested in the inverted orientation (i.e. bottom strand termination) in Figure 56, were flipped within the luciferase reporter cassettes so that these novel LTRs could be assessed for transcriptional termination efficiency in the forward direction (i.e. on the top strand). This allowed the assessment of any impact of the inverted GU boxes (servicing the SV40 early polyA sequence on the bottom strand) on top strand transcriptional termination. These variants were compared to a standard SIN-LTR (construct 1 and 2) or optimal supA-LTR (construct 5 and 6) with or without native HIV-1 polyA signal, respectively. These sequences were inserted into the luciferase polyA reporter and suspension (serum-free) HEK293T cells transfected, followed by luciferase assay of cell lysates to measure transcriptional read-in/out. Luciferase activity was normalised to that of construct 3 (set at 1.0) and data displayed on a log10 scale (arbitrary units).

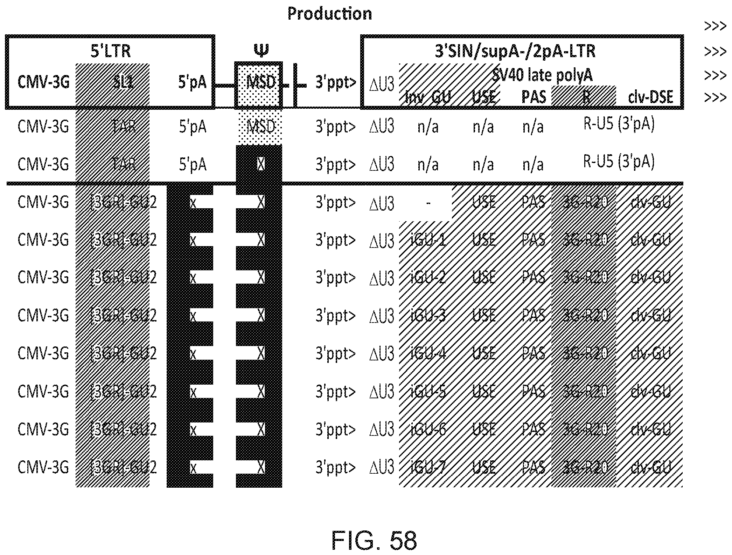

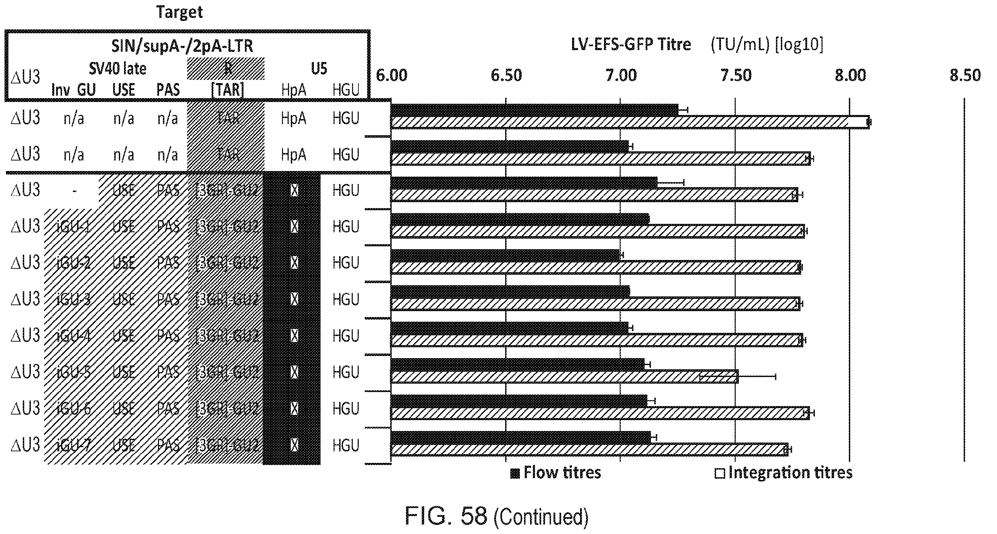

**Figure 58****. Production of lentiviral vectors containing variant supA-2pA-LTRs.** The configurations of 5' and 3' LTRs (for production expression cassettes) are indicated with or without different elements of the invention; all constructs were driven by the CMV promoter (with the '3G' TSS) and the 'back-up' polyadenylation sequence was absent except for the standard/MSD controls using a standard SIN-LTR (had SV40 late polyA downstream of the 3'SIN-LTR - not shown). Mutated native HIV-1 5' PAS (5'pA) and/or major splice donor site (MSD) are indicated by a cross. An internal EFS-GFP-wPRE cassette was present (not shown). The 5' R region was either the wild type/standard TAR/SL1 or the modified 5' R SL1 comprised the 3GR-GU2 variant. The heterologous SV40 late polyadenylation signal was positioned downstream of the 3'ppt/ΔU3 region. R region sequences composed of up to 20 nucleotides of HIV-1 R region were inserted between the heterologous PAS and the cleavage site/GU-rich DSE element. The '3G-R20' sequence is the same as the 'R.1-20' sequence referred to herein elsewhere. The optional presence of the inverted GU boxes (iGU-1 to iGU-7; see Table 10) are noted, to provide a DSE for the inverted polyA sequences. The structure of the final SIN/supA/supA-2pA LTRs in the 'target' cell are provided. LVs were produced in serum-free, suspension HEK293T cells as described elsewhere in the invention, with p256U1 provided *in trans* for the MSD-mutated LVs. Vector supernatants were titrated on adherent HEK293T cells, followed by flow cytometry (GFP positive cells) and integration assay, and data plotted on a log10 scale.

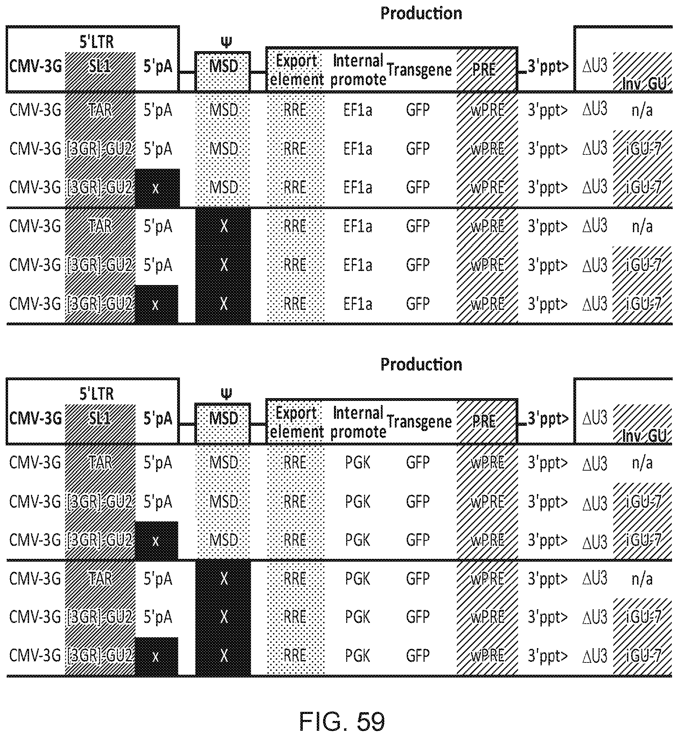

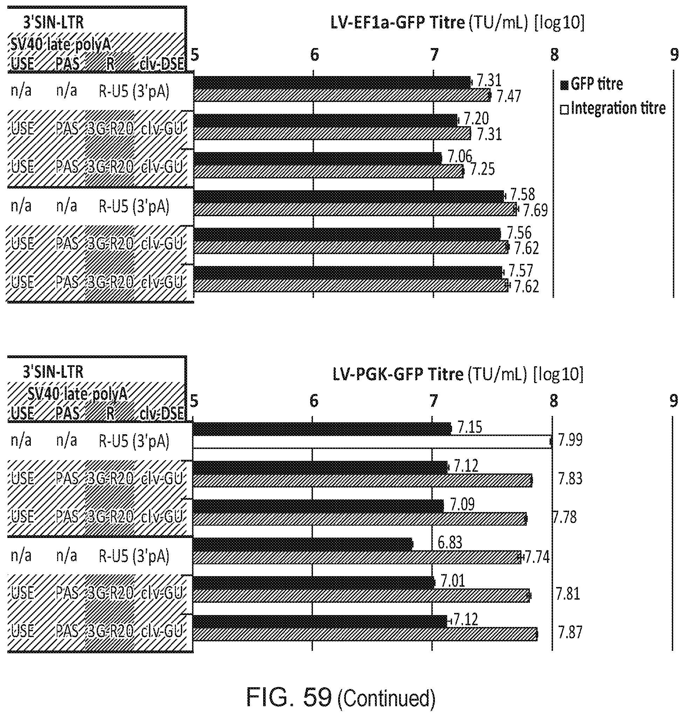

**Figure 59****. Production of different types of lentiviral vectors containing a supA-2pA-**LTR. The configurations of 5' and 3' LTRs are indicated with or without different elements of the invention; all constructs were driven by the CMV promoter (with the '3G' TSS) and the 'back-up' polyadenylation sequence was absent except for the standard/MSD controls using a standard SIN-LTR (had SV40 late polyA downstream of the 3'SIN-LTR - not shown). Mutated native HIV-1 5' PAS (5'pA) and/or major splice donor site (MSD) are indicated by a cross. An internal EF1a- or huPGKpromoter driven GFP cassette was present. The 5' R region was either the wild type/standard TAR/SL1 or the modified 5' R SL1 comprised the 3GR-GU2 variant. The heterologous SV40 late polyadenylation signal was positioned downstream of the 3'ppt/ΔU3 region. R region sequences composed of up to 20 nucleotides of HIV-1 R region were inserted between the heterologous PAS and the cleavage site/GU-rich DSE element. The '3G-R20' sequence is the same as the 'R.1-20' sequence referred to herein elsewhere . The inverted GU box 'GU-7' (see Table 10) was used, to provide a DSE for the inverted polyA sequences. LVs were produced in serum-free, suspension HEK293T cells, with p256U1 provided *in trans* for the MSD-mutated LVs. Vector supernatants were titrated on adherent HEK293T cells, followed by flow cytometry (GFP positive cells) and integration assay, and data plotted on a log10 scale.

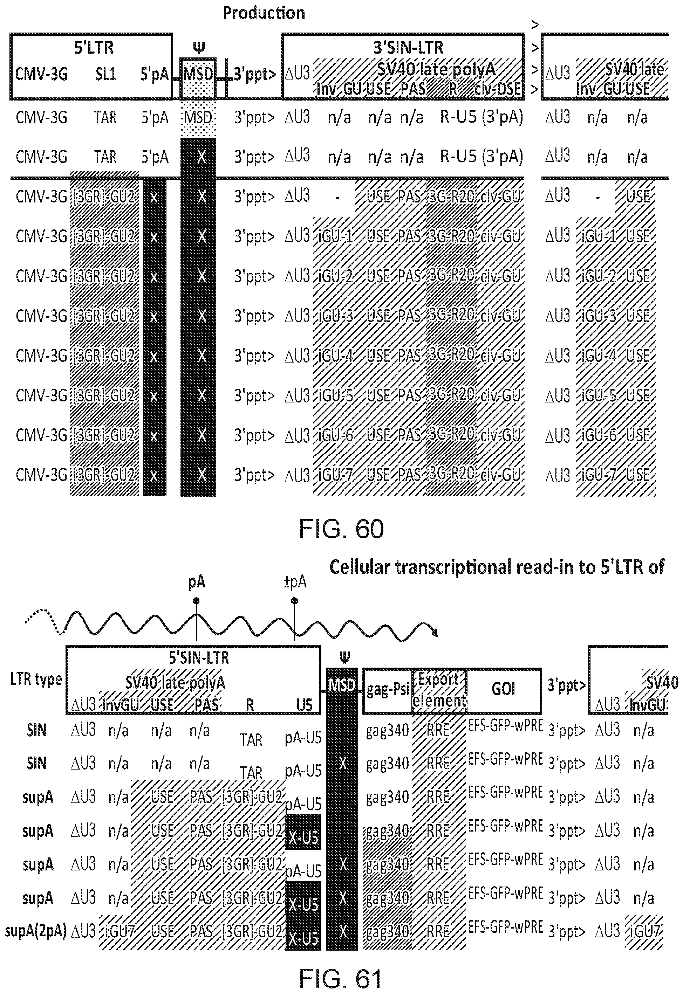

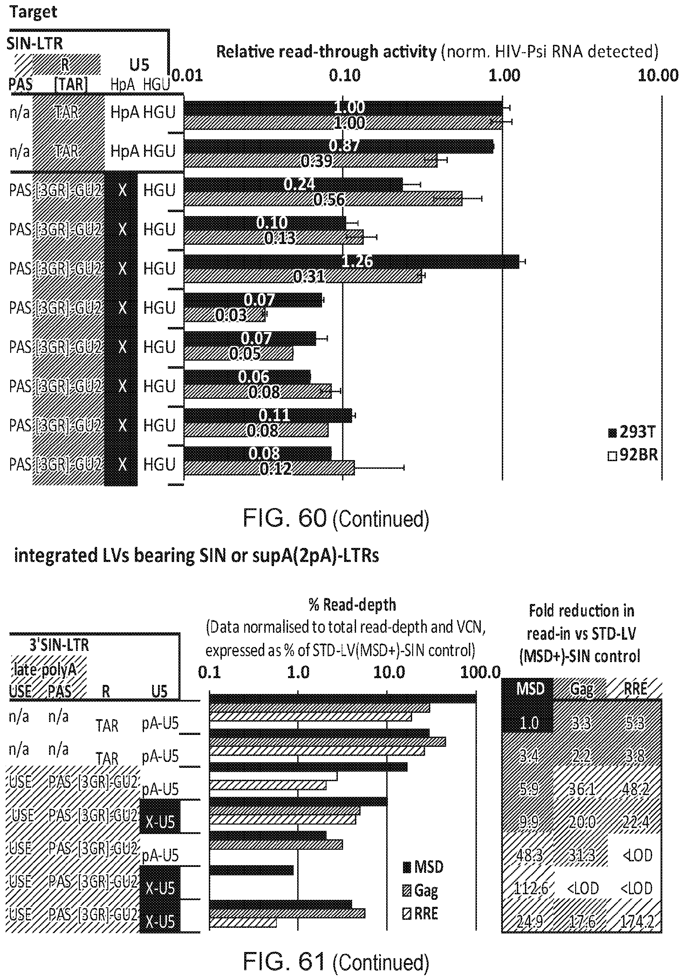

**Figure 60****. Measuring read-through the 5'LTR of integrated LVs bearing SIN-, supA or supA-2pA-LTRs.** LVs produced in Example 26 (Figure 58) were used to transduce adherent HEK293T cells or primary donkey fibroblasts (92BR) at MOI 1, followed by passaging for 10 days and integration assay to obtain vector-copy number (HIV Psi qPCR). Total RNA was extracted and HIV-Psi RNA and GAPDH mRNA quantified by RT-qPCR, and a relative HIV-Psi RNA ratio to GAPDH mRNA generated to provide a measure of mobilised HIV-Psi RNA (i.e. read-through the 5'LTR) in each culture. This value was then divided by average copy number per cell, which ranged from 0.8 to 3.6 copies across both cell types.
**Figure 61****. Comparison of transcriptional read-in from chromatin upstream of 5'LTR into integrated LV cassettes bearing either standard SIN-LTRs or supA(2pA)-LTRs.** Configuration of integrated LVs bearing either SIN-LTRs or supA(2pA)-LTRs, with optional mutation of the major splice donor (both types) and/or optional mutation of the native HIV-1 pA signal (supA(2pA)-LTR only). LVs were used to transduce adherent HEK293T cells at MOI of 1, and after a 10 day passage host cell genomic DNA extracted for integration assay to determine vector copy number (qPCR to HIV-Psi). PolyA-selected RNA was purified and subjected to RNAseq. Read coverage was mapped to templates for the integrated cassette for each genome. Read counts from regions indicated (MSD[core-Psi], Gag-Psi, and RRE) were initially normalised to total read counts across the GFP transgene. The data were further normalised to vector copy number. Data were finally expressed as % of the MSD reads-depth of the control genome (STD-LV(MSD+)-SIN). Fold-reduction in detected read-through RNA (relative MSD reads of STD-LV(MSD+)-SIN control) is tabulated (LOD = limit of detection).

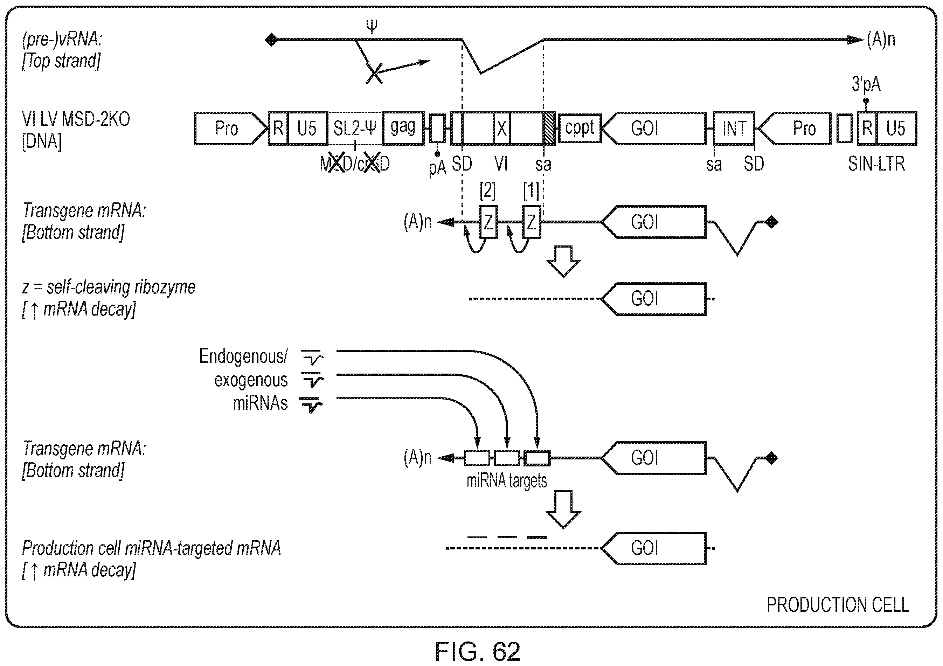

**Figure 62****. Vector-Intron LVs harbouring self-cleaving elements within the transgene 3'UTR: use of production cell derived microRNA target sites.** The inverted transgene cassette comprises self-cleaving elements within the 3'UTR sequence that is encompassed by the Vector-Intron sequence on the top strand, and thus such elements are spliced out of packaged vRNA and not delivered to target cells. Self-cleaving elements (such as ribozymes [Z]) eliminate transgene mRNA, and therefore avoid triggering dsRNA-sensing pathways that otherwise reduce LV titres, as well as leading to suppression of transgene protein expression that might otherwise impact on LV titres. In this case, one or more microRNA target sequences are inserted into the 3'UTR, optionally with other self-cleaving elements such as ribozymes. These target sequences may be synthetic, and be targeted by a miRNA expressed exogenously (e.g. by a U6-driven cassette introduced into the production cell) or by endogenous miRNAs.

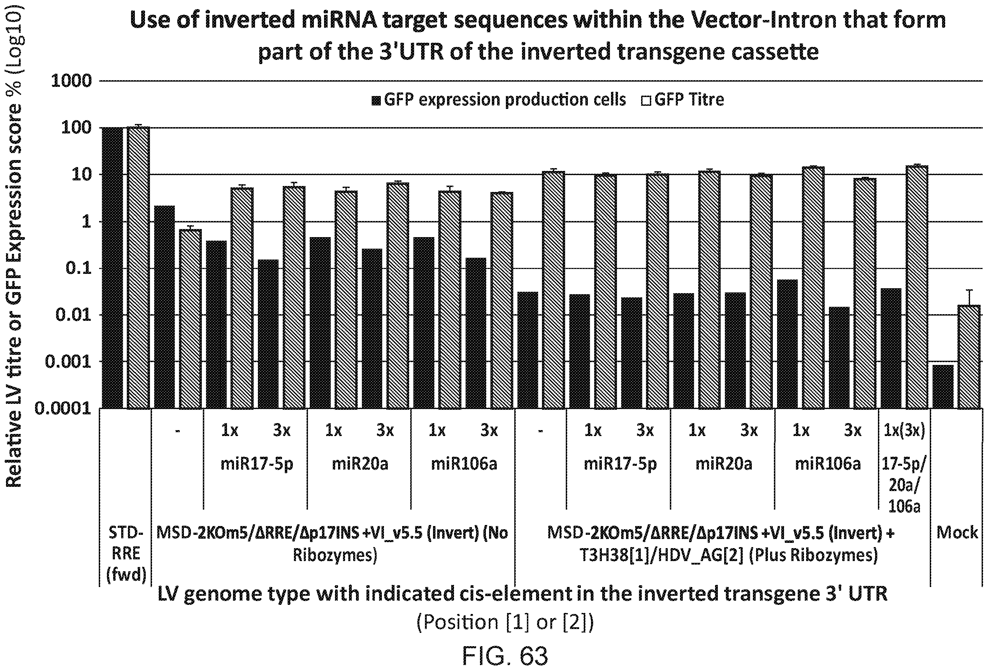

**Figure 63****. Production cell transgene expression and output titres of Vector-Intron LVs harbouring self-cleaving elements within the transgene 3'UTR: use of production cell derived microRNA target sites.** Vector-Intron LVs harbouring an inverted EF1a-GFP cassette were generated in a similar format as per Figure 62. Specifically, the 3'UTR of the inverted transgene that is encompassed by the VI on the top strand had 1x or 3x copies of three different target sequences of miRNAs found to be endogenously expressed in HEK293(T) cells (miR17-5p, miR20a and mi106a). Two sets of variants were produced in which the ribozymes T3H38 and HDV_AG were additionally present within the VI-encompassed 3'UTR region (at positions [1] and [2] respectively). For the variants containing both ribozymes and miRNA target sequence(s), the miRNA target sequences were positioned between the two ribozymes. A third variant type was generated in which a single copy of all three miRNAs were present between the ribozymes (17-5p/20a/106a). LVs were produced in suspension (serum-free) HEK293T cells alongside a standard LV, containing the EF1a-GFP cassette in the forward orientation. Post-production cells were analysed by flow cytometry to generate GFP Expression scores (%GFP x MFI), and resultant vector supernatants were titrated on adherent HEK293T cells by flow cytometry to yield GFP TU/mL values. Titre values and GFP expression scores were normalised to that attained by the standard LV (set to 100%).

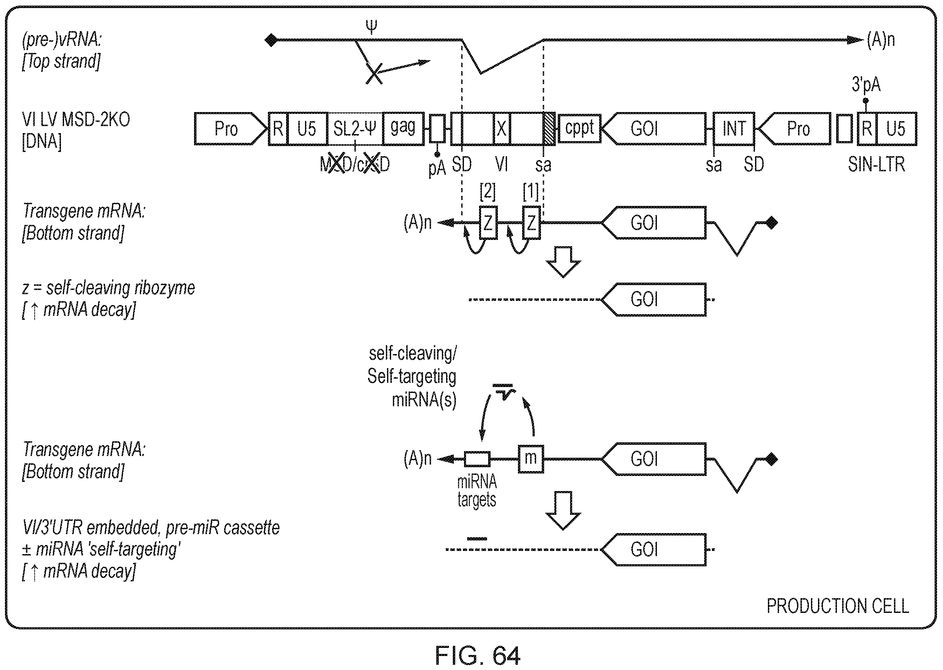

**Figure 64****. Vector-Intron LVs harbouring self-cleaving elements within the transgene 3'UTR: use of Vector-Intron embedded microRNAs.** The figure displays a similar LV production system to that described in Figures 44 and 45. The inverted transgene cassette comprises self-cleaving elements within the 3'UTR sequence that is encompassed by the Vector-Intron sequence on the top strand, and thus such elements are spliced out of packaged vRNA and not delivered to target cells. Self-cleaving elements (such as ribozymes [Z]) eliminate transgene mRNA, and therefore avoid triggering dsRNA-sensing pathways that otherwise reduce LV titres, as well as leading to suppression of transgene protein expression that might otherwise impact on LV titres. In this case, one or more microRNA cassettes are inserted into the 3'UTR (processing of which will cleave the transgene mRNA), and optionally the miRNAs produced from processing target sites within the transgene mRNA (in this case 3'UTR sequence). Optionally, these miRs/miRNA targets are combined with other self-cleaving elements such as ribozymes.

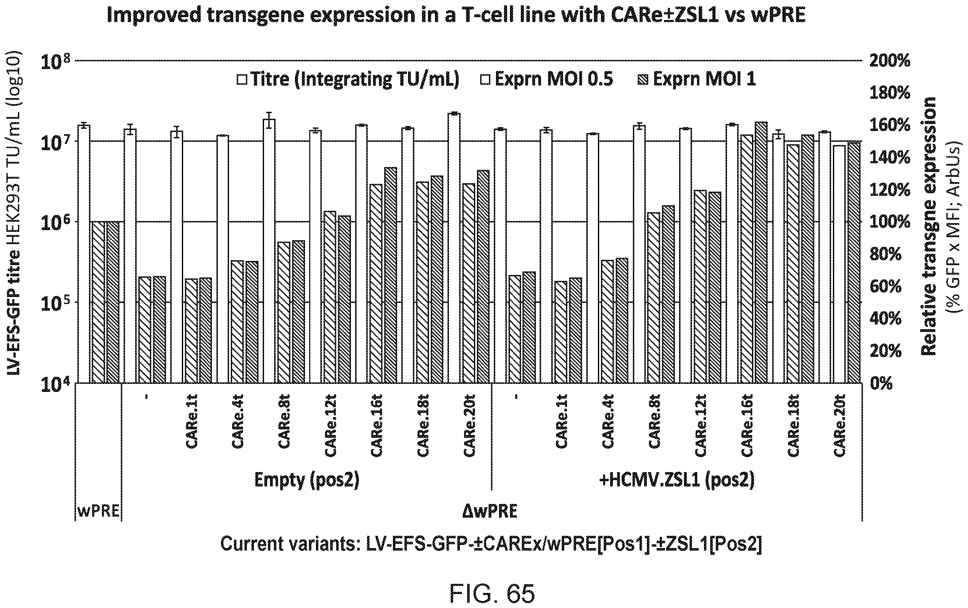

**Figure 65****. Transgene expression levels in suspension Jurkat cells (T-cell line) transduced with RRE/rev-dependent lentiviral vectors harbouring different 3' UTR cis-acting elements at matched MOIs (diagonal lines).** LV-RRE-EFS-GFP vector stocks produced in suspension (serum-free) HEK293Ts were initially titrated on adherent HEK293T cells to generate integrating titres (open bars; TU/mL). Vector stocks were used to transduce fresh a Jurkat cells at matched multiplicity of infection (MOI): MOI 1 or 0.5. Different 3' UTR cis-acting elements were employed as 'stand-alone' elements: wPRE, 16x 10bp CARe tiles (CARe.16t) or a single copy of the ZCCHC14 stem loop (HCMV.ZSL1), compared to no element (ΔwPRE). Additionally, variants deleted for wPRE but containing a single copy of the ZCCHC14 stem loop (at position 2) were also paired with increasing numbers of CARe tile, from 1x to 20x 10bp copies (at position 1 i.e. upstream of position 2). Transgene (GFP) expression in transduced Jurkat cells was measured by flow cytometry three days post-transduction and median fluorescence intensities (Arbitrary units) normalised to that achieved with the standard wPRE-containing LV (set to 100%).

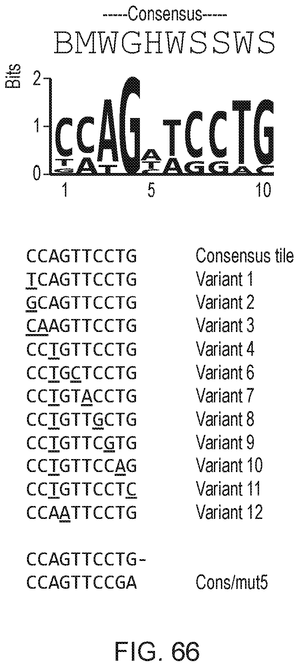

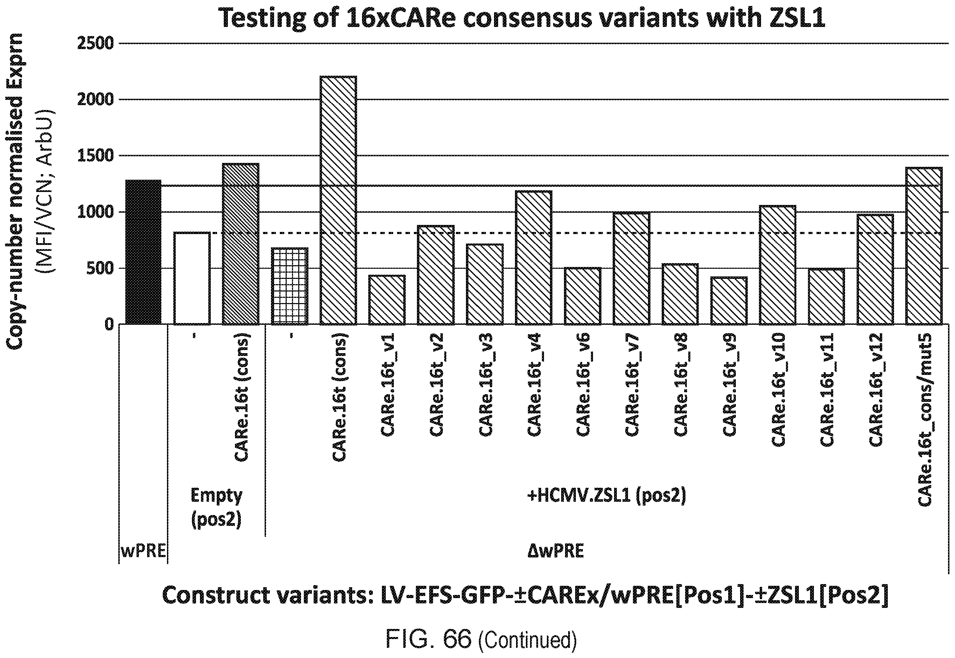

**Figure 66****. Transgene expression levels in suspension Jurkat cells (T-cell line) transduced with RRE/rev-dependent lentiviral vectors harbouring different 3' UTR cis-acting elements at matched MOI.** LV-RRE-EFS-GFP vector stocks produced in suspension (serum-free) HEK293Ts were initially titrated on adherent HEK293T cells to generate integrating titres (not shown). Vector stocks were used to transduce fresh Jurkat cells at matched multiplicity of infection of 1. Different 3' UTR cis-acting elements were employed as 'stand-alone' elements: wPRE (black bar), 16x 10bp CARe tiles (CARe.16t; dark grey bar) or a single copy of the ZCCHC14 stem loop (HCMV.ZSL1; striped light grey bar), compared to no element (ΔwPRE; white bar). Additionally, variants deleted for wPRE but containing a single copy of the ZCCHC14 stem loop (at position 2) were also paired with 16x 10bp CARe tiles that contained synthetic variant sequences of the consensus (CARe.16t_vX; at position 1 i.e. upstream of position 2) as shown (grey bars). Transgene (GFP) expression in transduced Jurkat cells was measured by flow cytometry ten days post-transduction and median fluorescence intensities (Arbitrary units) normalised to vector copy-number (VCN), which was measured by qPCR against HIV-Psi on extracted host cell DNA. The solid horizontal line indicates expression level achieved by the larger wPRE element, and the dotted horizontal line indicates expression levels without any 3'UTR element.

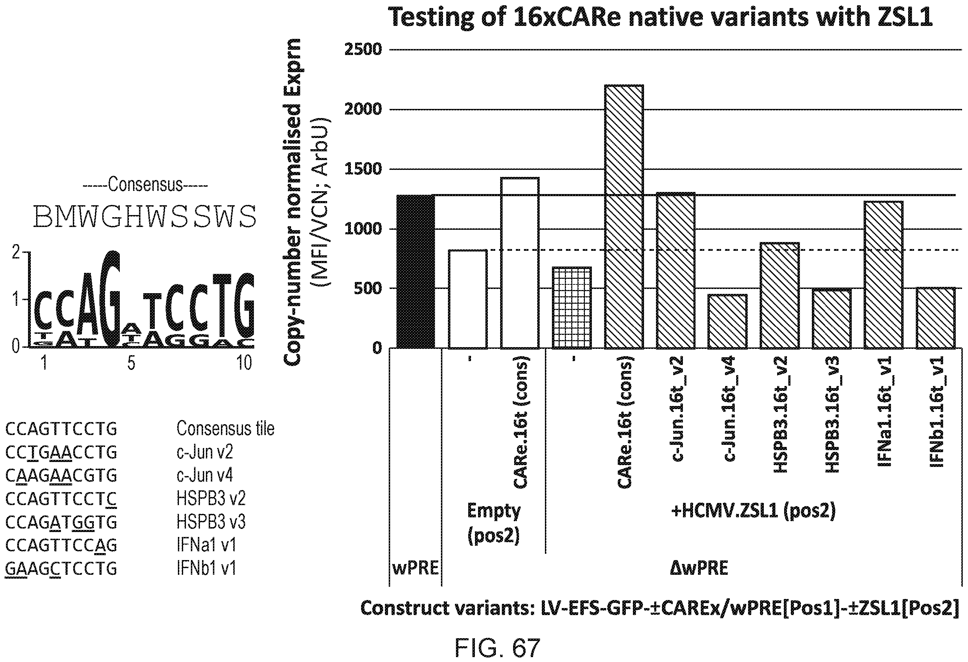

**Figure 67****. Transgene expression levels in suspension Jurkat cells (T-cell line) transduced with RRE/rev-dependent lentiviral vectors harbouring different 3' UTR cis-acting elements at matched MOI.** LV-RRE-EFS-GFP vector stocks produced in suspension (serum-free) HEK293Ts were initially titrated on adherent HEK293T cells to generate integrating titres (not shown). Vector stocks were used to transduce fresh Jurkat cells at matched multiplicity of infection of 1. Different 3' UTR cis-acting elements were employed as 'stand-alone' elements: wPRE (black bar), 16x 10bp CARe tiles (CARe.16t; dark grey bar) or a single copy of the ZCCHC14 stem loop (HCMV.ZSL1; striped light grey bar), compared to no element (ΔwPRE; white bar). Additionally, variants deleted for wPRE but containing a single copy of the ZCCHC14 stem loop (at position 2) were also paired with 16x 10bp CARe tiles that contained native variant sequences of the consensus (CARe.16t_vX; at position 1 i.e. upstream of position 2) as shown (grey bars). These were from c-Jun, HSPB3, IFN-alpha and IFN-beta mRNAs. Transgene (GFP) expression in transduced Jurkat cells was measured by flow cytometry ten days post-transduction and median fluorescence intensities (Arbitrary units) normalised to vector copy-number (VCN), which was measured by qPCR against HIV-Psi on extracted host cell DNA. The solid horizontal line indicates expression level achieved by the larger wPRE element, and the dotted horizontal line indicates expression levels without any 3'UTR element.

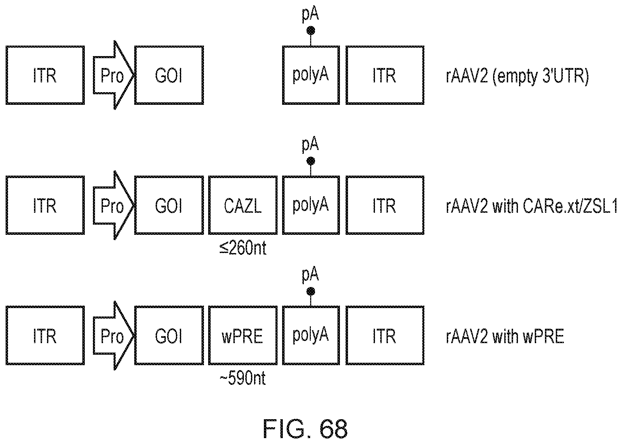

**Figure 68****. Example rAAV vector genomes containing no (empty) or 3'UTR elements to enhance transgene expression in target cells.** The 'CAZL' element (a composite of tandem CARe 10bp consensus tiles [CARe.xt] and the ZCCHC14 stem loop [ZSL1]) is ~140-260 nts in length (depending on use of 4x to 16x CARe tiles). The wPRE is ~590 nts in length, and therefore occupies more of the rAAV vector genome, size being a critical limitation for rAAVs. Key - Inverted terminal repeat (ITR), Promoter (Pro), Gene of interest (GOI), polyadenylation signal (polyA).

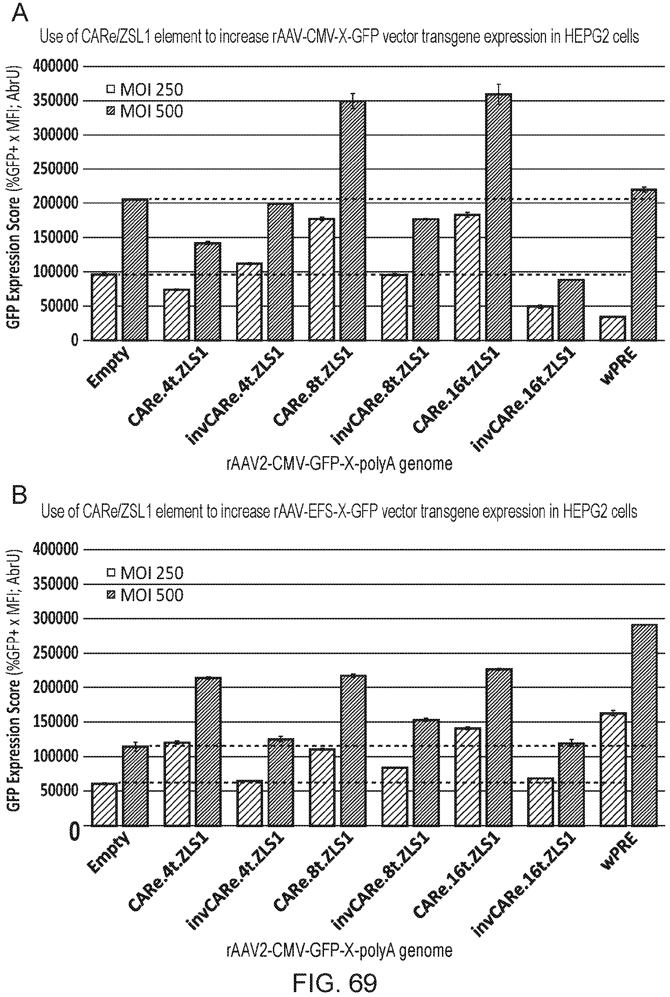

**Figure 69****. The results of an experiment wherein rAAV vectors containing either CMV- or EFS-promoter driven GFP, paired with either variant CARe/ZSL1 ('CAZL') elements from ∼140-to-260 nts in length or with the wPRE (~590 nts) at position 'x' (i.e. in 3'UTR).** Controls for the CARe/ZSL1 were inverted elements ('inv') to control for potential effects of different genome sizes, which ranged from 2.0-to-2.6 kb (CMV) and 1.6-to-2.2 kb (EFS) across all genomes. An empty rAAV vector was used as a negative control. rAAVs were made by co-transfection of HEK293T suspension cells with pGenome/pRepCap/pHelper plasmids at 1:1:1 ratio, and harvest 72 hours post-transfection. Vector harvest material was titrated by qPCR against the GFP sequence to generated vg/mL physical titre values. HEPG2 cells were transduced at the denoted MOls, and then 72 hours post-transduction cells analysed by flow cytometry, and GFP Expression scores (%GFP+ x MFI; ArbUs) generated.

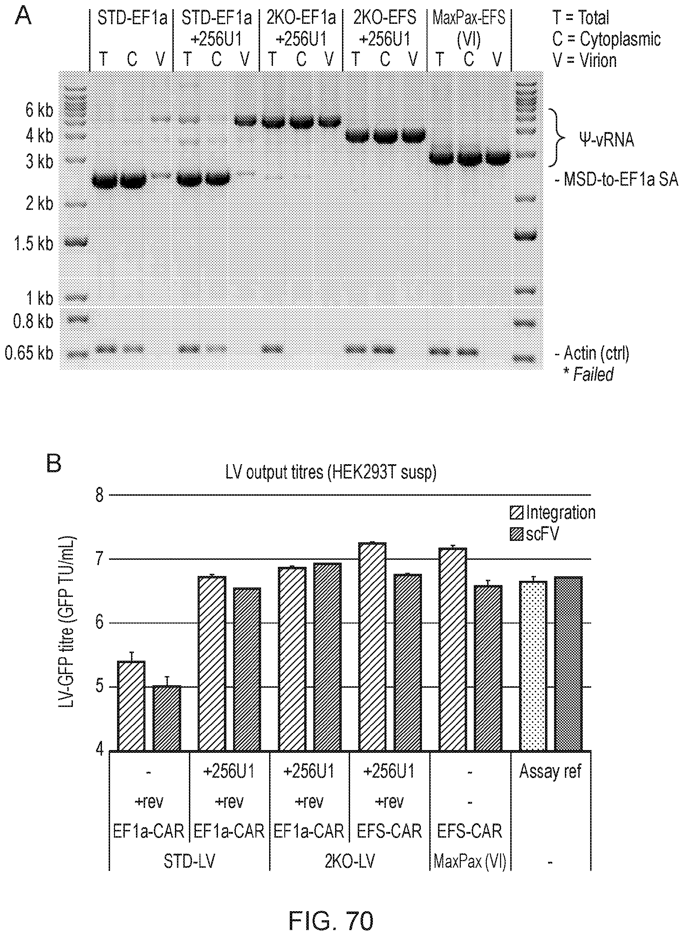

**Figure 70****. High titre production of an LV encoding a chimeric antigen receptor (CAR) transgene cassette using an optimised Vector-Intron efficiently spliced out of packaged vRNA. [A]** RT-PCR analysis of vRNA-derived species within production cells (total and cytoplasmic) and resultant LV virions (V) for four different types of LV vector backbone expression cassettes. 'STD' refers to standard 3^{rd} Gen LVs, harbouring all the typical cis-acting elements, including the major splice donor (MSD) and rev-response element (RRE). '2KO' refers to newer generation LVs wherein the MSD has been mutated; these also contain the RRE and require expression of a modified U1 snRNA (256U1) molecule to fully restore output titres. 256U1 used with STD LVs also increases packaged vRNA and titres, see **[B].** The 'MaxPax' LV contains the v5.6 variant of the Vector-Intron of the present invention, and harbours both mutation in the MSD and deletion of the RRE and gag-p17INS sequences. Promoters used were either EF1a or the short EF1a (EFS). The RT-PCR use primers upstream of the MSD (fwd) and downstream within the GFP transgene so that aberrant or 'correct' VI splicing could be monitored. The presence of packageable/packaged vRNA is shown (ψ-vRNA); note that the size of RT-PCR product reflects the size of promoter (EFS is ~1kb shorter than EF1a; see 2KO-EF1a vs 2KO-EFS) and the increase in capacity of the VI-derived vRNA of ∼1kb (see 2KO-EFS vs MaxPax-EFS). An RT-PCR to actin mRNA was used as a positive control. Panel B displays the output titres of the vectors descrive in A; both integration and 'biological' (scFV) titres are shown against the assay reference control (stripes). VI-derived MaxPax LVs were produced in the absence of rev.

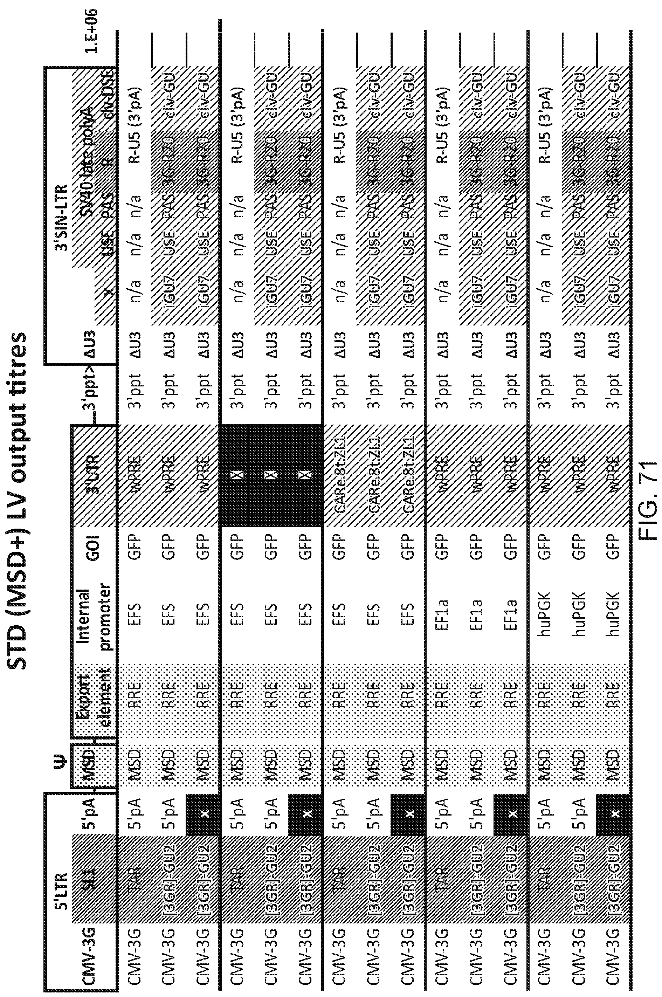

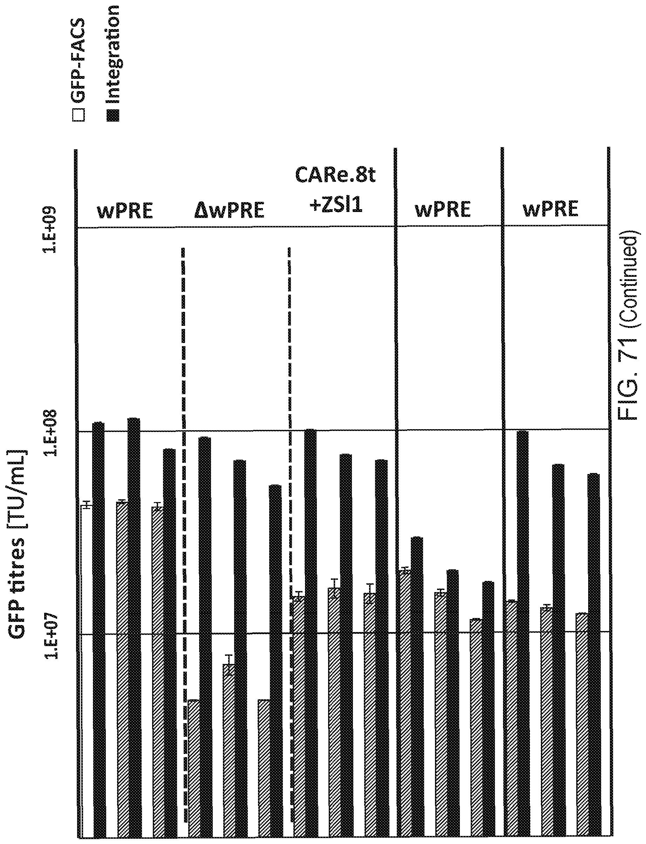

**Figure 71****. Production titres of standard LV-GFPs harbouring different transgene promoters, SIN or supA-2pA LTRs, and with alternative 3'UTR elements.** The figure displays the structure of the LV DNA expression cassette in each case, from 5' to 3' LTR. At the 5' LTR postion the CMV promoter is used with the 3Gs at the transcription start site (CMV-3G), has either the standard R region (TAR) or the supA(2pA)-ITR 5' modification 'GU2', and is optionally mutated for the native 5' polyA (white X). All standard LVs contained an intact major splice donor (MSD) and rev response element (RRE). The GFP transgene was driven by either EF1a, EFS (short EF1a i.e. lacking intron A) or human PGK promoters. The 3' UTR was the either the wPRE or a CARe/ZSL1 element containing 8x tiles of the CARe 10bp consensus element link to a ZCCHC14 protein-binding stem loop, or did not contain an element (white X). The 3' LTR were either standard, self-inactivating (SIN) or used the 3' R-embedded heterologous polyA adenylation sequence (in this case SV40 late polyA), with inverted polyA (not shown; in this case the SV40 early polyA) and GU-box (iGU7; in this case from the 'SPA' polyA, based on beta-globin polyA) upstream of the Usptream enhancer (USE; in this case from SV40 late polyA). Note that constructs using the SIN-LTR also utilised a 'back-up' polyA downstream (in this case the SV40 pA), wherease supA(2p)-LTRs did not.The embedded R region was downstream of the heterologous polyA signal (PAS) and comprised the first 20 nucleotides of the R region, including the 3Gs (3G-R20). The heterologous polyA cleavage zone and downstream DSE/GU rich sequence are alos indicated (clv-DSE/GU). LVs were produced in suspension (serum-free) HEK293T cells and titrated on adherent HEK293T cells by flow cytometry (GFP-FACS; grey bars) and by integration assay (qPCR for HIV-Psi, on host cell DNA; black bars) on days 3 and 10 post-transduction.

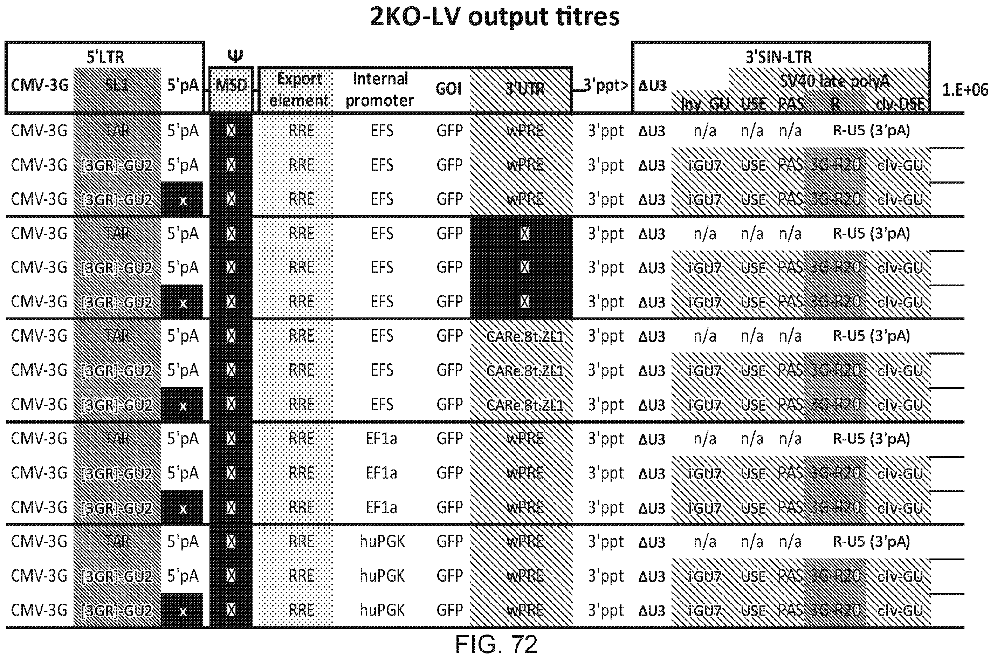

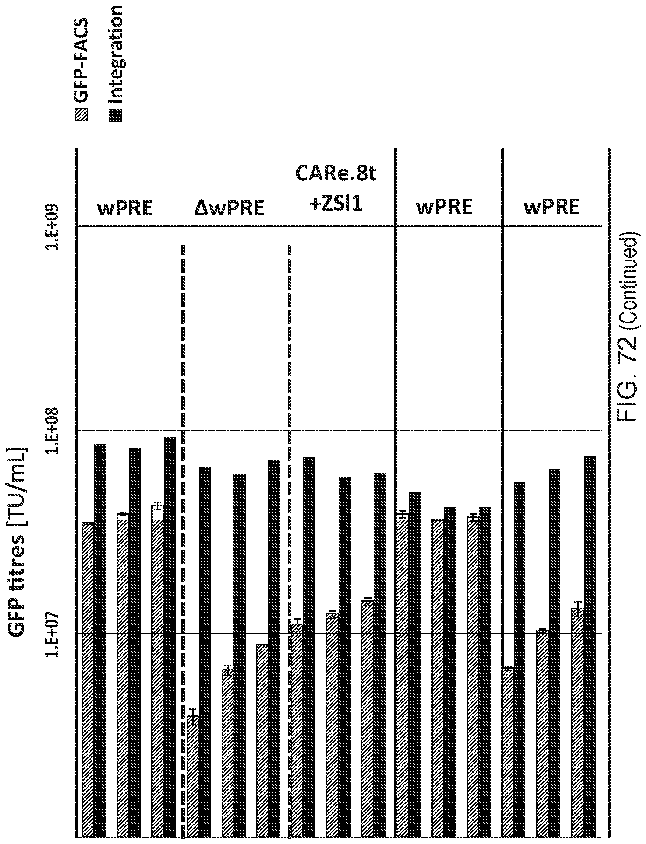

**Figure 72****. Production titres of '2KO'-LV-GFPs harbouring different transgene promoters, SIN or supA-2pA LTRs, and with alternative 3'UTR elements.** The figure displays the structure of the LV DNA expression cassette in each case, from 5' to 3' LTR. At the 5' LTR postion the CMV promoter is used with the 3Gs at the transcription start site (CMV-3G), has either the standard R region (TAR) or the supA(2pA)-ITR 5' modification 'GU2', and is optionally mutated for the native 5' polyA (white X). All 2KO-LVs contained a mutated major splice donor (MSD; mutant '2KOm5' was used) and rev response element (RRE). The GFP transgene was driven by either EF1a, EFS (short EF1a i.e. lacking intron A) or human PGK promoters. The 3' UTR was the either the wPRE or a CARe/ZSL1 element containing 8x tiles of the CARe 10bp consensus element link to a ZCCHC14 protein-binding stem loop, or did not contain an element (white X). The 3' LTR were either standard, self-inactivating (SIN) or used the 3' R-embedded heterologous polyA adenylation sequence (in this case SV40 late polyA), with inverted polyA (not shown; in this case the SV40 early polyA) and GU-box (iGU7; in this case from the 'SPA' polyA, based on beta-globin polyA) upstream of the Usptream enhancer (USE; in this case from SV40 late polyA). Note that constructs using the SIN-LTR also utilised a 'back-up' polyA downstream (in this case the SV40 pA), wherease supA(2p)-LTRs did not.The embedded R region was downstream of the heterologous polyA signal (PAS) and comprised the first 20 nucleotides of the R region, including the 3Gs (3G-R20). The heterologous polyA cleavage zone and downstream DSE/GU rich sequence are alos indicated (clv-DSE/GU). LVs were produced in suspension (serum-free) HEK293T cells and titrated on adherent HEK293T cells by flow cytometry (GFP-FACS; grey bars) and by integration assay (qPCR for HIV-Psi, on host cell DNA; black bars) on days 3 and 10 post-transduction.

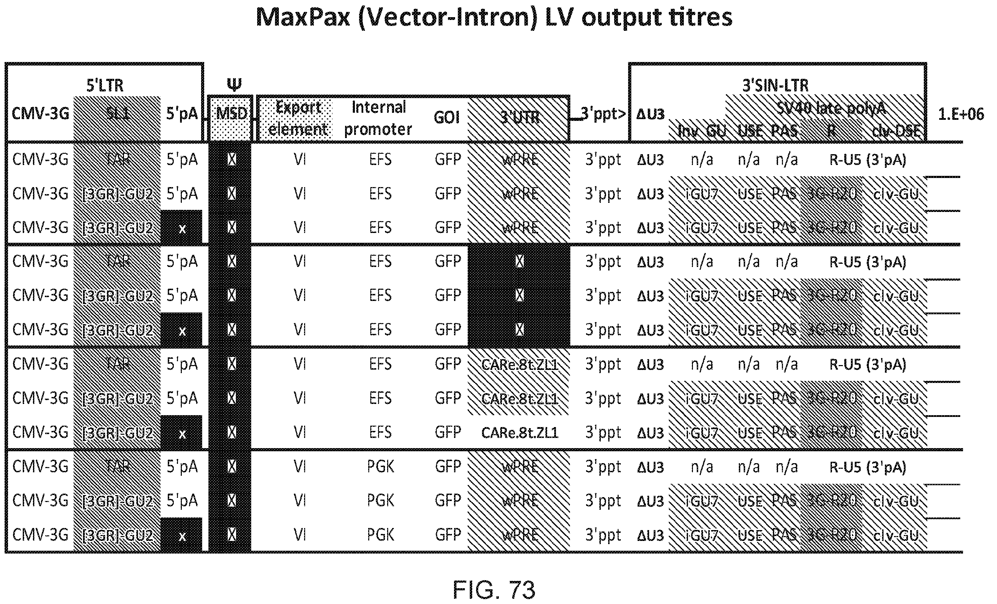

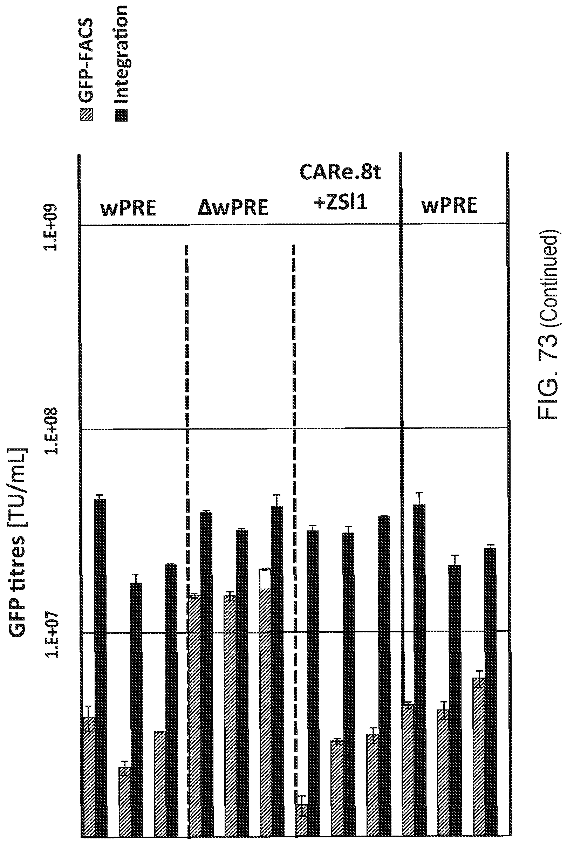

**Figure 73****. Production titres of 'MaxPax' (Vector-Intron) LV-GFPs harbouring different transgene promoters, SIN or supA-2pA LTRs, and with alternative 3'UTR elements.** The figure displays the structure of the LV DNA expression cassette in each case, from 5' to 3' LTR. At the 5' LTR postion the CMV promoter is used with the 3Gs at the transcription start site (CMV-3G), has either the standard R region (TAR) or the supA(2pA)-ITR 5' modification 'GU2', and is optionally mutated for the native 5' polyA (white X). All MaxPax-LVs contained a mutated major splice donor (MSD; mutant '2KOm5' was used) and Vector-Intron (VI) variant v5.5, as well as truncated gag-Psi region (not shown). The GFP transgene was driven by either EFS (short EF1a i.e. lacking intron A) or human PGK promoters. The 3' UTR was the either the wPRE or a CARe/ZSL1 element containing 8x tiles of the CARe 10bp consensus element link to a ZCCHC14 protein-binding stem loop, or did not contain an element (white X). The 3' LTR were either standard, self-inactivating (SIN) or used the 3' R-embedded heterologous polyA adenylation sequence (in this case SV40 late polyA), with inverted polyA (not shown; in this case the SV40 early polyA) and GU-box (iGU7; in this case from the 'SPA' polyA, based on beta-globin polyA) upstream of the Usptream enhancer (USE; in this case from SV40 late polyA). Note that constructs using the SIN-LTR also utilised a 'back-up' polyA downstream (in this case the SV40 pA), wherease supA(2p)-LTRs did not.The embedded R region was downstream of the heterologous polyA signal (PAS) and comprised the first 20 nucleotides of the R region, including the 3Gs (3G-R20). The heterologous polyA cleavage zone and downstream DSE/GU rich sequence are alos indicated (clv-DSE/GU). LVs were produced in suspension (serum-free) HEK293T cells and titrated on adherent HEK293T cells by flow cytometry (GFP-FACS; grey bars) and by integration assay (qPCR for HIV-Psi, on host cell DNA; black bars) on days 3 and 10 post-transduction.

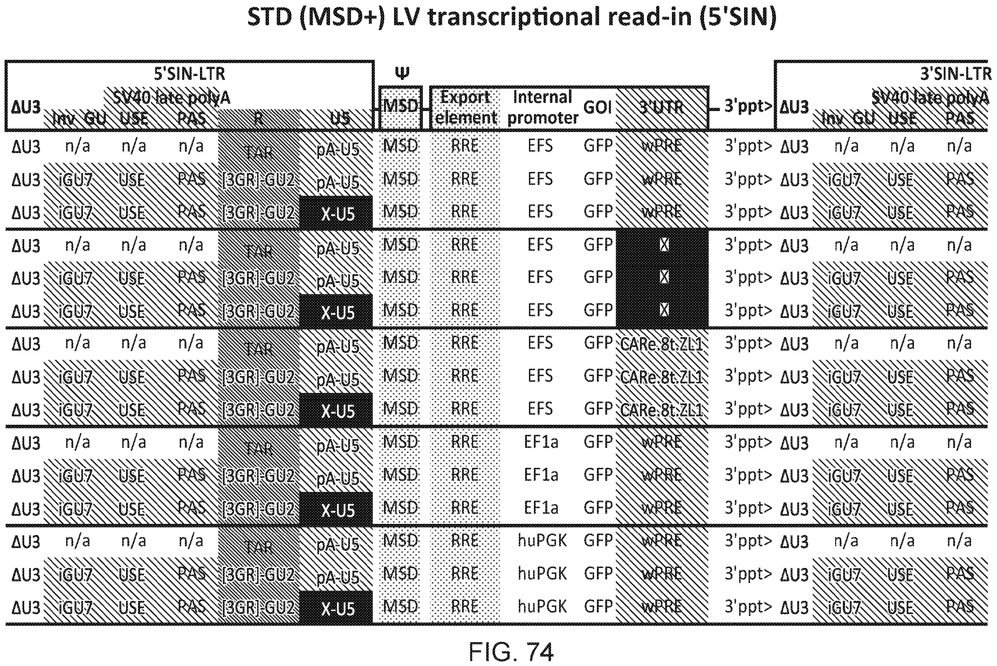

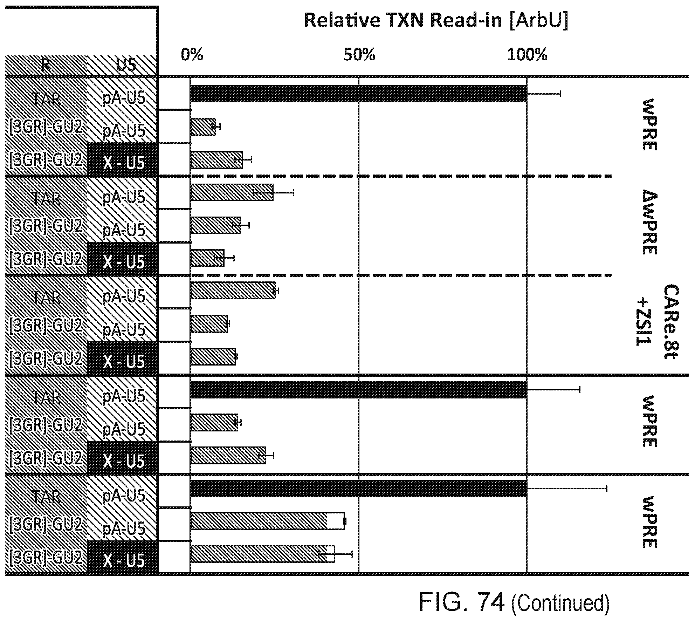

**Figure 74****. Transcriptional read-in to the 5' LTR of integrated standard LV-GFPs harbouring different transgene promoters, SIN or supA-2pA LTRs, and with alternative 3'UTR elements.** The figure displays the structure of the integrated LV DNA cassette in each case, from 5' to 3' LTR, resulting from reverse transcription and integrated of the LVs in Figure 71. Therefore, both 5' and 3' LTRs are identical. The standard SIN LTR contains the deleted U3 region (ΔU3), and R-U5, which contains the native HIV-1 polyA signal. The supA(2pA) LTRs harbour an inverted polyA (not shown; in this case the SV40 early polyA) and GU-box (iGU7; in this case from the 'SPA' polyA, based on beta-globin polyA) upstream of the Usptream enhancer (USE; in this case from SV40 late polyA). Downstream of the USE is the heterologus polyA signal (SV40 late PAS), the GU2-modified R region, and is optionally mutated for the native HIV-1 polyA signal in the U5 region (white X). All other aspects between the LTRs were the same as described in Figure 71. LVs were used to transduce adherent HEK293T cells at MOI 1, followed by passaging for 10 days and integration assay to obtain vector-copy number (HIV Psi qPCR). Total RNA was extracted, and HIV-Psi RNA and GAPDH mRNA quantified by RT-qPCR. The relative HIV-Psi RNA ratio to GAPDH mRNA generated to provide a measure of mobilised HIV-Psi RNA (i.e. read-through the 5'LTR) in each culture (normalised to vector copy-number); these are plotted in comparison to read-in values for the LV harbouring the standard wPRE/SIN-LTR variant for each LV bearing the same internal promoter driving the transgene (set to 100%, black bars).

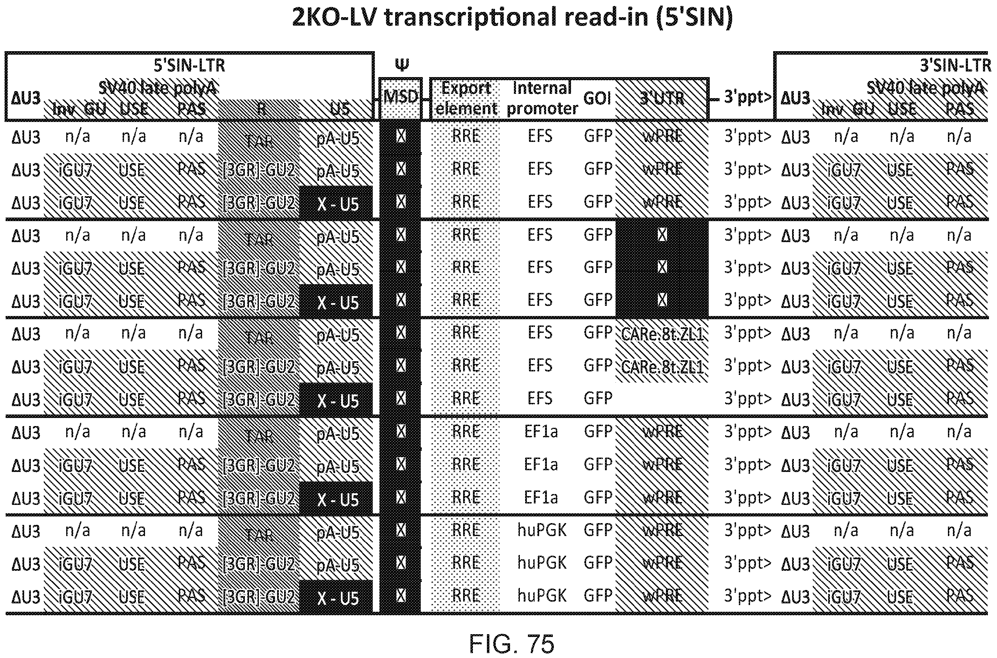

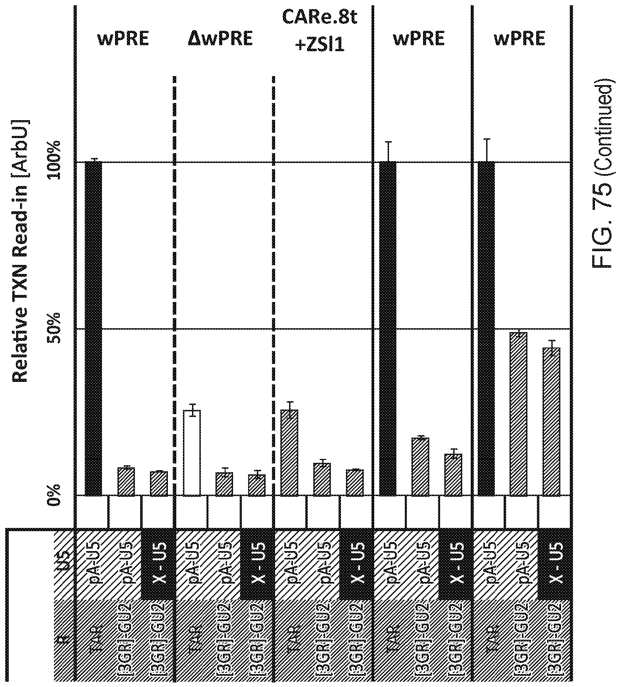

**Figure 75****. Transcriptional read-in to the 5' LTR of integrated '2KO' LV-GFPs harbouring different transgene promoters, SIN or supA-2pA LTRs, and with alternative 3'UTR elements.** The figure displays the structure of the integrated LV DNA cassette in each case, from 5' to 3' LTR, resulting from reverse transcription and integrated of the LVs in Figure 72. Therefore, both 5' and 3' LTRs are identical. The standard SIN LTR contains the deleted U3 region (ΔU3), and R-U5, which contains the native HIV-1 polyA signal. The supA(2pA) LTRs harbour an inverted polyA (not shown; in this case the SV40 early polyA) and GU-box (iGU7; in this case from the 'SPA' polyA, based on beta-globin polyA) upstream of the Usptream enhancer (USE; in this case from SV40 late polyA). Downstream of the USE is the heterologus polyA signal (SV40 late PAS), the GU2-modified R region, and is optionally mutated for the native HIV-1 polyA signal in the U5 region (white X). All other aspects between the LTRs were the same as described in Figure 72, including the mutated MSD. LVs were used to transduce adherent HEK293T cells at MOI 1, followed by passaging for 10 days and integration assay to obtain vector-copy number (HIV Psi qPCR). Total RNA was extracted, and HIV-Psi RNA and GAPDH mRNA quantified by RT-qPCR. The relative HIV-Psi RNA ratio to GAPDH mRNA generated to provide a measure of mobilised HIV-Psi RNA (i.e. read-through the 5'LTR) in each culture (normalised to vector copy-number); these are plotted in comparison to read-in values for the LV harbouring the standard wPRE/SIN-LTR variant for each LV bearing the same internal promoter driving the transgene (set to 100%, black bars).

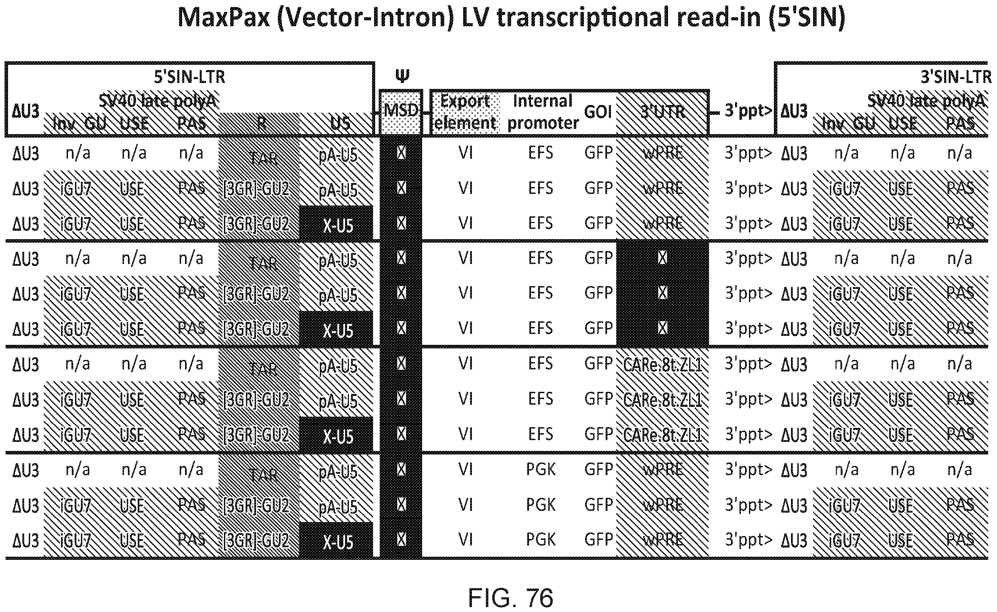

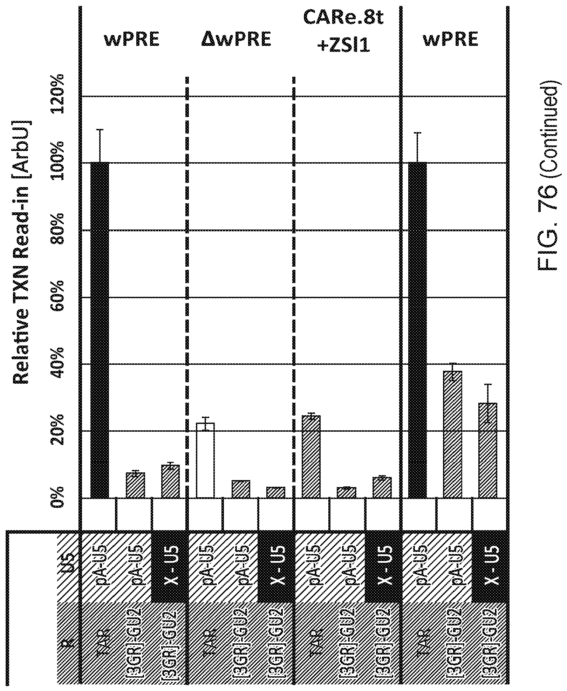

**Figure 76****. Transcriptional read-in to the 5' LTR of integrated 'MaxPax' (Vector-Intron) LV-GFPs harbouring different transgene promoters, SIN or supA-2pA LTRs, and with alternative 3'UTR elements.** The figure displays the structure of the integrated LV DNA cassette in each case, from 5' to 3' LTR, resulting from reverse transcription and integrated of the LVs in Figure 73. Therefore, both 5' and 3' LTRs are identical. The standard SIN LTR contains the deleted U3 region (ΔU3), and R-U5, which contains the native HIV-1 polyA signal. The supA(2pA) LTRs harbour an inverted polyA (not shown; in this case the SV40 early polyA) and GU-box (iGU7; in this case from the 'SPA' polyA, based on beta-globin polyA) upstream of the Usptream enhancer (USE; in this case from SV40 late polyA). Downstream of the USE is the heterologus polyA signal (SV40 late PAS), the GU2-modified R region, and is optionally mutated for the native HIV-1 polyA signal in the U5 region (white X). All other aspects between the LTRs were the same as described in Figure 73, including the mutated MSD and replacement of RRE with VI, and truncated gag-Psi (not shown). LVs were used to transduce adherent HEK293T cells at MOI 1, followed by passaging for 10 days and integration assay to obtain vector-copy number (HIV Psi qPCR). Total RNA was extracted, and HIV-Psi RNA and GAPDH mRNA quantified by RT-qPCR. The relative HIV-Psi RNA ratio to GAPDH mRNA generated to provide a measure of mobilised HIV-Psi RNA (i.e. read-through the 5'LTR) in each culture (normalised to vector copy-number); these are plotted in comparison to read-in values for the LV harbouring the standard wPRE/SIN-LTR variant for each LV bearing the same internal promoter driving the transgene (set to 100%, black bars).

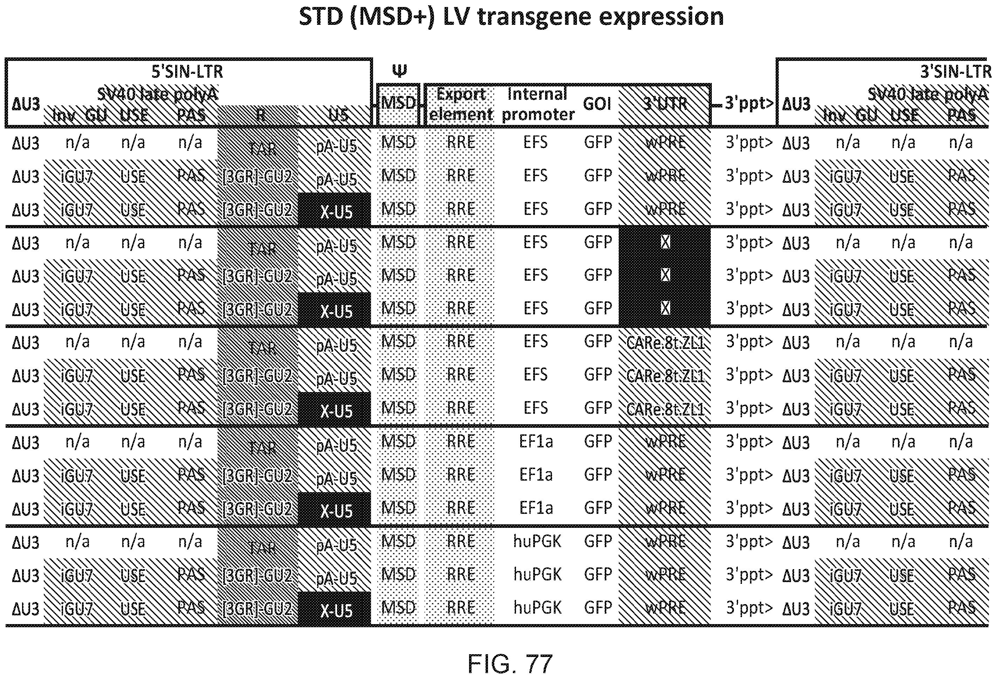

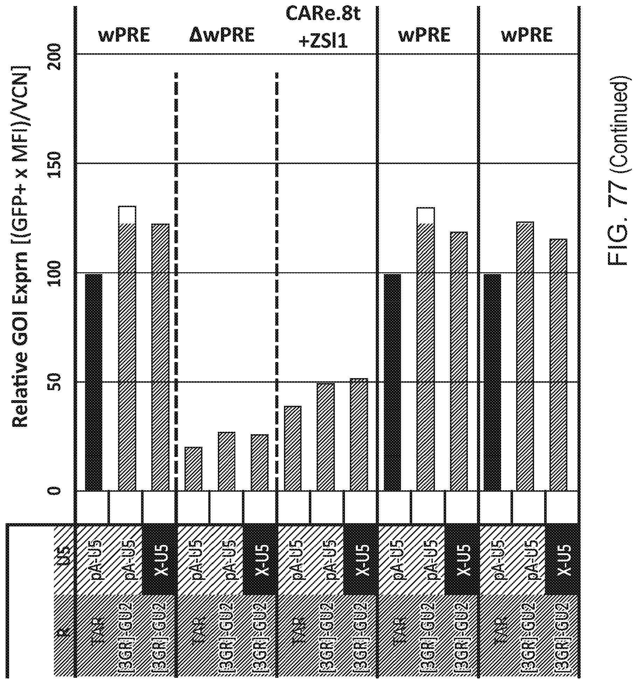

**Figure 77****. Relative transgene expression of integrated standard LV-GFPs harbouring different transgene promoters, SIN or supA-2pA LTRs, and with alternative 3'UTR elements.** The figure re-states the configuration of the integrated LV as described in Figure 74. Adherent HEK293T cells transduced with the LVs at MOI of 1 (as per Figures 71 and 74) were analysed by flow cytometry at day 3 post-transduction and GFP Expression scores generated by multiplying %GFP-postive cells and median fluoresence values (MFI). These ES scores were divided by the vector-copy number generated at day 10 post-transduction (by qPCR to HIV Psi). The resulting 'relative GOI Exprn' values are plotted in comparison to those of the LV harbouring the standard wPRE/SIN-LTR for each LV bearing the same internal promoter driving the transgene (set to 100, black bars).

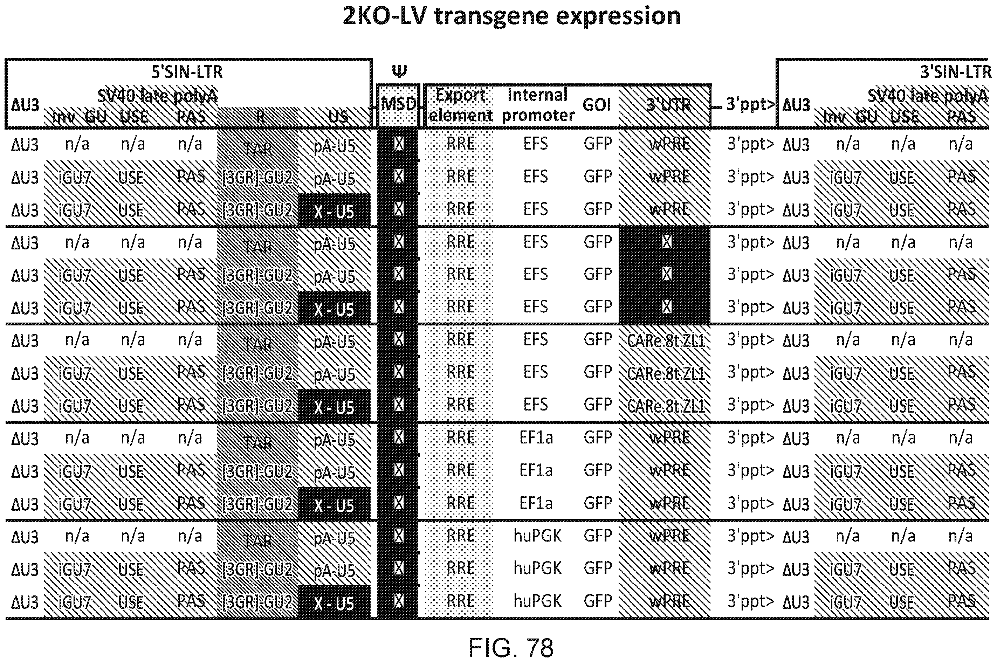

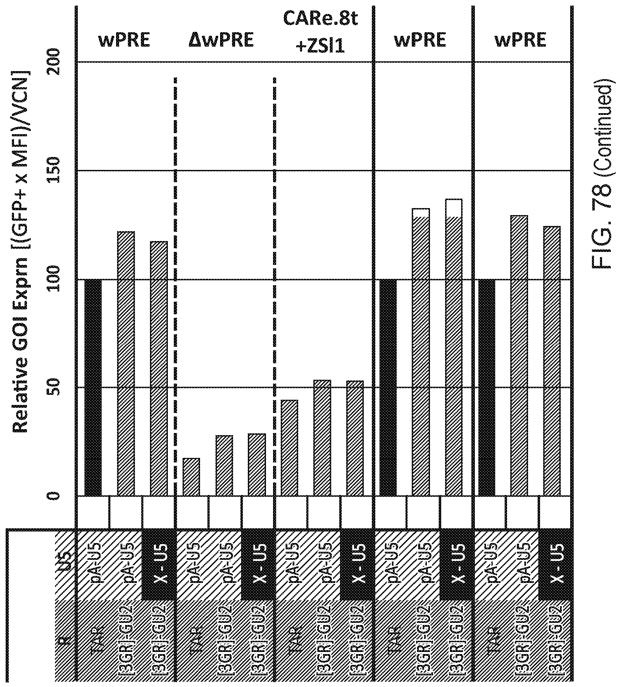

**Figure 78****. Relative transgene expression of integrated standard LV-GFPs harbouring different transgene promoters, SIN or supA-2pA LTRs, and with alternative 3'UTR elements.** The figure re-states the configuration of the integrated LV as described in Figure 75. Adherent HEK293T cells transduced with the LVs at MOI of 1 (as per Figures 72 and 75) were analysed by flow cytometry at day 3 post-transduction and GFP Expression scores generated by multiplying %GFP-postive cells and median fluoresence values (MFI). These ES scores were divided by the vector-copy number generated at day 10 post-transduction (by qPCR to HIV Psi). The resulting 'relative GOI Exprn' values are plotted in comparison to those of the LV harbouring the standard wPRE/SIN-LTR for each LV bearing the same internal promoter driving the transgene (set to 100, black bars).

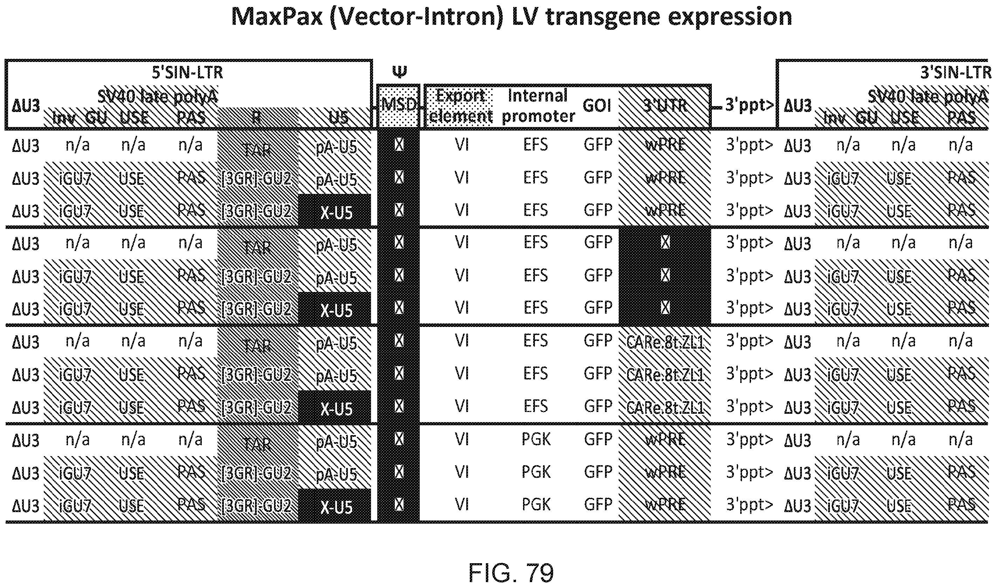

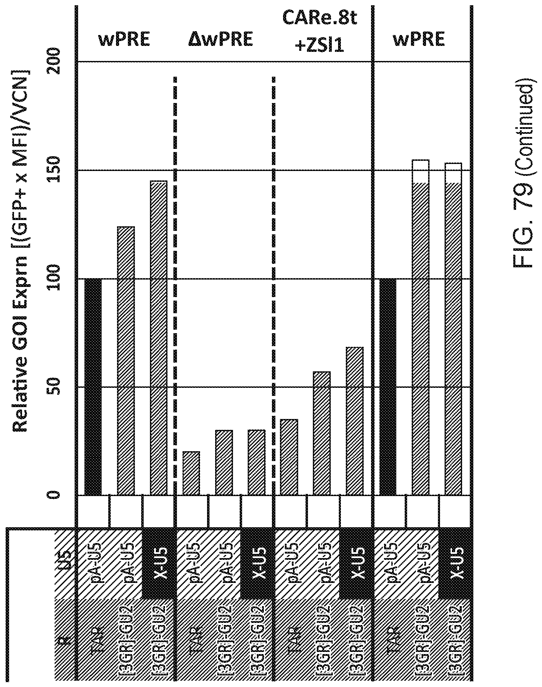

**Figure 79****. Relative transgene expression of integrated standard LV-GFPs harbouring different transgene promoters, SIN or supA-2pA LTRs, and with alternative 3'UTR elements.** The figure re-states the configuration of the integrated LV as described in Figure 76. Adherent HEK293T cells transduced with the LVs at MOI of 1 (as per Figures 73 and 76) were analysed by flow cytometry at day 3 post-transduction and GFP Expression scores generated by multiplying %GFP-postive cells and median fluoresence values (MFI). These ES scores were divided by the vector-copy number generated at day 10 post-transduction (by qPCR to HIV Psi). The resulting 'relative GOI Exprn' values are plotted in comparison to those of the LV harbouring the standard wPRE/SIN-LTR for each LV bearing the same internal promoter driving the transgene (set to 100, black bars).

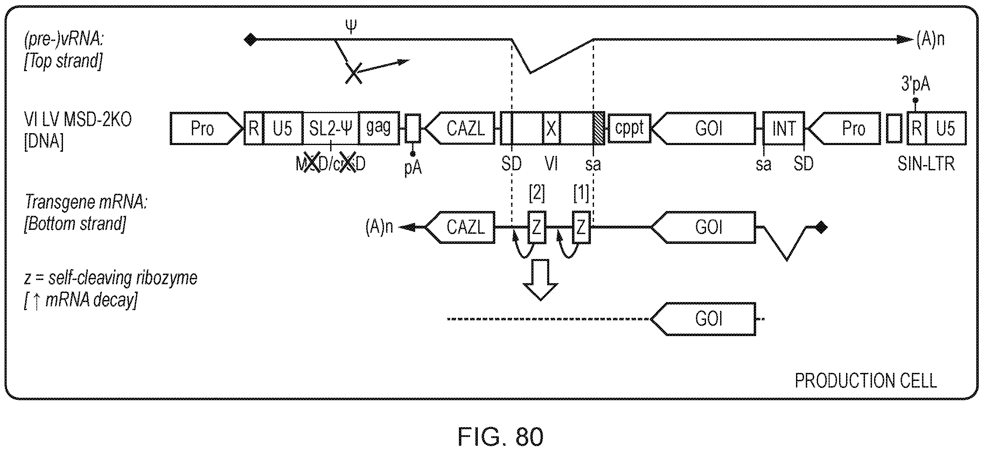

**Figure 80****. Use of the CARe/ZSL1 3'UTR element within the inverted transgene cassette of a Vector-Intron genome with 'functionalised 3'UTR'.** The figure provides an alternative structure to that of Figures 68 and 69. The inverted transgene configuration within a Vector-Intron LV is desirable to enable on-boarding of intron-containing cassettes. The functionalised 3'UTR in this instance contains two self-cleaving ribozymes (Z). In this case, the transgene is used with the CARe.8t/ZSL1 element (CAZL), which is positioned outside of the VI-excised sequence, and so will remain in the delivered LV transgene cassette.

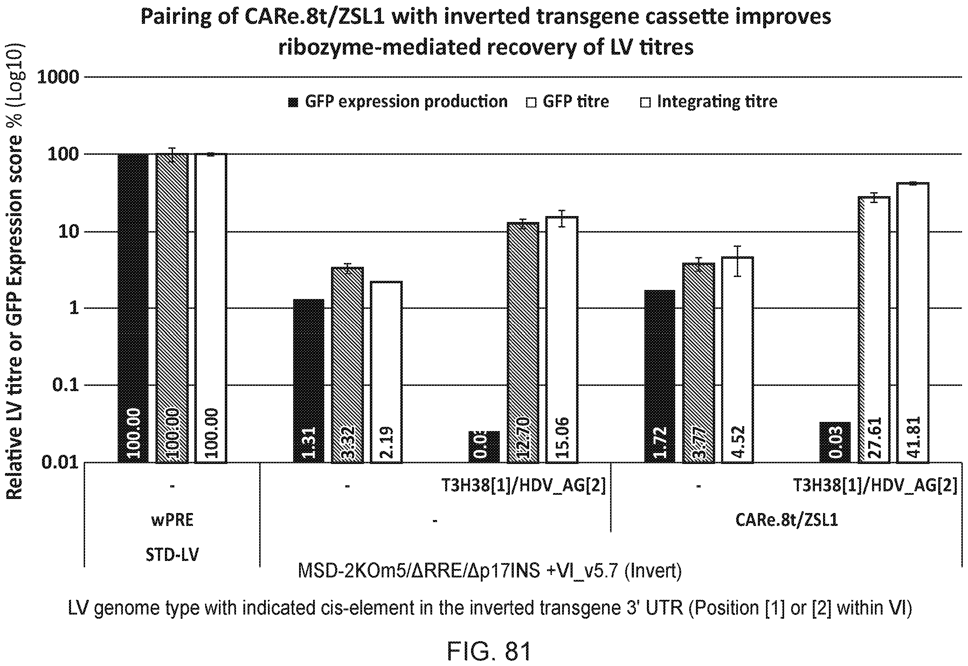

**Figure 81****. Use of the CARe/ZSL1 3'UTR element within the inverted transgene cassette of a Vector-Intron genome with 'functionalised 3'UTR' improves output titres.** The Vector-Intron LVs based on Figure 80 were made +/- the CARe/ZSL1 3'UTR element (aka 'CAZL' and +/- the dual ribozymes functionalising the 3'UTR within the VI encoded on the top strand. LVs were produced in suspension (serum-free) HEK293T cells and post-production levels of GFP expression measured by flow cytometry (%GFP x MFI). Vector supernatants were titrated on adherent HEK293T cells by flow cytometry and integration assay (qPCR to HIV Psi) at day 3 and 10 post-transduction respectively. Data was normalised to a standard LV containing a forward facing transgene cassette (EF1a-GFP) and the wPRE (set to 100%).

### DETAILED DESCRIPTION OF THE INVENTION

### Nucleotide sequence and set of nucleotide sequences

The present inventors surprisingly found that:
1) employing modified polyadenylation (polyA) sequences within LV genome expression cassettes results in simplified production of vector genomic RNA for packaging, improved transgene expression and reduced transcriptional read-in and -out (both of the vector genome expression cassette and transgene expression cassette) in transduced cells;
2) viral vectors with novel short cis-acting sequences in the 3' UTR of a transgene expression cassette either in addition to traditional PREs to boost transgene expression in target cells or to replace these longer PREs entirely, enabling increased transgene capacity whilst maintaining high levels of transgene expression in target cells;
3) introduction of an intron into the vector genome expression cassette facilitates removal of the rev-response element (RRE), which allows for more transgene capacity in the vector; and
4) RNAi can be employed in retroviral vector production cells to suppress the expression of the NOI (i.e. transgene) during retroviral vector production in order to minimize unwanted effects of the transgene protein and to rescue of titres of retroviral vectors harbouring an actively transcribed inverted transgene cassette (wherein the transgene expression cassette is all or in part inverted with respect to the retroviral vector genome expression cassette).

Accordingly, in one aspect, the invention provides a nucleotide sequence comprising a lentiviral vector genome expression cassette, wherein the 3' LTR of the lentiviral vector genome comprises a modified polyadenylation sequence, and wherein the modified polyadenylation sequence comprises a polyadenylation signal which is 5' of the 3' LTR R region.

In a further aspect, the invention provides a nucleotide sequence comprising a lentiviral vector genome expression cassette, wherein the lentiviral vector genome comprises a modified 5' LTR, and wherein the R region of the modified 5' LTR comprises at least one polyadenylation downstream enhancer element (DSE).

In some embodiments, the lentiviral vector genome expression cassette comprises a transgene expression cassette.

In some embodiments, the lentiviral vector genome expression cassette comprises a transgene expression cassette and a vector intron.

In some embodiments, the vector intron comprises one or more transgene mRNA self-destabilization or self-decay element(s) or one or more transgene mRNA nuclear retention signal(s).

In a further aspect, the invention provides a nucleotide sequence comprising a transgene expression cassette wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.

In a further aspect, the invention provides a nucleotide sequence comprising a lentiviral vector genome expression cassette, wherein the lentiviral vector genome expression cassette comprises a transgene expression cassette and a vector intron as described herein.

In some embodiments, the major splice donor site in the lentiviral vector genome expression cassette is inactivated.

In some embodiments, the lentiviral vector genome expression cassette does not comprise a rev-response element (RRE).

In some embodiments, the cryptic splice donor site adjacent to the 3' end of the major splice donor site in the lentiviral vector genome expression cassette is inactivated.

In some embodiments, the transgene expression cassette is in the forward orientation with respect to the lentiviral vector genome expression cassette. Thus, the transgene expression cassette and vector intron may not be transcriptionally opposed.

In some embodiments, the transgene expression cassette is inverted with respect to the lentiviral vector genome expression cassette. Thus, the transgene expression cassette and vector intron are transcriptionally opposed.

In some embodiments:
a) the vector intron is not located between the promoter of the transgene expression cassette and the transgene; and/or
b) the nucleotide sequence comprises a sequence as set forth in any of SEQ ID NOs: 2, 3, 4, 6, 7, and/or 8, and/or the sequences CAGACA, and/or GTGGAGACT; and/or
c) the 3' UTR of the transgene expression cassette comprises the vector intron.

In some embodiments, the vector intron comprises one or more transgene mRNA self-destabilization or self-decay element(s) or one or more transgene mRNA nuclear retention signal(s).

In some embodiments, the nucleotide sequence comprises a lentiviral vector genome expression cassette, wherein:
i) the major splice donor site and cryptic splice donor site adjacent to the 3' end of the major splice donor site in the lentiviral vector genome expression cassette are inactivated;
ii) the lentiviral vector genome expression cassette does not comprise a rev-response element;
iii) the lentiviral vector genome expression cassette comprises a transgene expression cassette and a vector intron; and
iv)
   a) When the transgene expression cassette is inverted with respect to the lentiviral vector genome expression cassette:
      i. the vector intron is not located between the promoter of the transgene expression cassette and the transgene; and
      ii. the nucleotide sequence comprises a sequence as set forth in any of SEQ ID NOs: 2, 3, 4, 6, 7, and/or 8, and/or the sequence CAGACA, and/or GTGGAGACT; and
      iii. the 3' UTR of the transgene expression cassette comprises the vector intron; and
   b) the vector intron comprises one or more transgene mRNA self-destabilization or self-decay element(s) or one or more transgene mRNA nuclear retention signal(s).

As mentioned above, any one or more of the aspects of the invention described herein may be combined. This provides the advantage that the surprising and beneficial effects of each aspect can be achieved in combination, i.e. the inclusion of each aspect has an additive and/or synergistic effect.

Suitably, any two of the aspects of the invention described herein may be combined. Suitably any three of the aspects of the invention described herein may be combined. Suitably, any four of the aspects of the invention may be combined. Suitably, all aspects of the invention described herein may be combined. Therefore, all of the embodiments of the invention described herein with respect to one aspect of the invention also relate to any and all other aspect(s) of the invention.

Accordingly, in one aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette, and wherein the 3' UTR of the transgene expression cassette comprises at least one cis-acting sequence as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome expression cassette comprises a transgene expression cassette and a vector intron, and wherein the 3' UTR of the transgene expression cassette comprises at least one cis-acting sequence as described herein.

In a further aspect, the invention provides a nucleotide sequence comprising a lentiviral vector genome expression cassette, wherein the lentiviral vector genome expression cassette comprises a transgene expression cassette and a vector intron, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome expression cassette comprises a transgene expression cassette and a vector intron as described herein, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the lentiviral vector genome comprises a modified 5' LTR as described herein, wherein the lentiviral vector genome expression cassette comprises a transgene expression cassette and a vector intron as described herein, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, and wherein the lentiviral vector genome comprises a modified 5' LTR as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette, and wherein the 3' UTR of the transgene expression cassette comprises at least one cis-acting sequence as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, and wherein the lentiviral vector genome expression cassette comprises a transgene expression cassette and a vector intron as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette and a vector intron as described herein, and wherein the vector intron comprises one or more transgene mRNA self-destabilization or self-decay element(s) as described herein or one or more transgene mRNA nuclear retention signal(s) as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette and a vector intron as described herein, and wherein the 3' UTR of the transgene expression cassette comprises at least one cis-acting sequence as described herein.

In a further aspect, the invention provides a nucleotide sequence comprising a lentiviral vector genome expression cassette, wherein the 3' LTR of the lentiviral vector genome comprises a modified polyadenylation sequence as described herein, wherein the lentiviral vector genome comprises a modified 5' LTR as described herein, wherein the lentiviral vector genome expression cassette comprises a transgene expression cassette and a vector intron as described herein, wherein the 3' UTR of the transgene expression cassette comprises at least one cis-acting sequence as described herein, and wherein the vector intron comprises one or more transgene mRNA self-destabilization or self-decay element(s) as described herein or one or more transgene mRNA nuclear retention signal(s) as described herein.

In some embodiments, the lentiviral vector genome expression cassette does not comprise a rev-response element (RRE).

In some embodiments, the major splice donor site in the lentiviral vector genome is inactivated, and optionally wherein the cryptic splice donor site 3' to the major splice donor site is inactivated. The inactivated major splice donor site may have the sequence set forth in SEQ ID NO: 4.

In some embodiments, the lentiviral vector genome further comprises a tryptophan RNA-binding attenuation protein (TRAP) binding site.

In some embodiments, the nucleotide sequence further comprises a nucleotide sequence encoding a modified U1 snRNA, wherein said modified U1 snRNA has been modified to bind to a nucleotide sequence within the packaging region of the lentiviral vector genome.

In some embodiments, the nucleotide sequence encoding the lentiviral vector genome is operably linked to the nucleotide sequence encoding the modified U1 snRNA.

In some embodiments, the lentiviral vector genome comprises at least one modified viral *cis-*acting sequence, wherein at least one internal open reading frame (ORF) in the viral cis-acting sequence is disrupted.

In some embodiments, the at least one viral cis-acting sequence is a Woodchuck hepatitis virus (WHV) post-transcriptional regulatory element (WPRE) and/or a Rev response element (RRE).

In some embodiments, the lentiviral vector genome comprises a modified nucleotide sequence encoding gag, and wherein at least one internal open reading frame (ORF) in the modified nucleotide sequence encoding gag is disrupted.

In some embodiments, the at least one internal ORF is disrupted by mutating at least one ATG sequence within the nucleotide sequence, preferably wherein the first ATG sequence within the nucleotide sequence is mutated.

In some embodiments, the lentiviral vector genome lacks (i) a nucleotide sequence encoding Gag-p17 or (ii) a fragment of a nucleotide sequence encoding Gag-p17.

In some embodiments, the fragment of a nucleotide sequence encoding Gag-p17 comprises a nucleotide sequence encoding p17 instability element.

In some embodiments, the nucleotide sequence comprising a lentiviral vector genome does not express Gag-p17 or a fragment thereof.

In some embodiments, said fragment of Gag-p17 comprises the p17 instability element.

In some preferred embodiments, the transgene gives rise to a therapeutic effect.

In some embodiments, the lentiviral vector is derived from HIV-1, HIV-2, SIV, FIV, BIV, EIAV, CAEV or Visna lentivirus.

The present inventors have surprisingly found that RNAi can be employed in lentiviral vector production cells to suppress the expression of the NOI (i.e. transgene) during lentiviral vector production in order to minimize unwanted effects of the transgene protein during vector production and/or to rescue titres of lentiviral vectors harbouring an actively transcribed inverted transgene cassette. The use of interfering RNA(s) specific for the transgene mRNA provides a mechanism for avoiding *de novo* protein synthesis inhibition and/or the consequences of other dsRNA sensing pathway and enables rescue of inverted transgene lentiviral vector titres. The interfering RNA is an interfering RNA as described herein. As such, the interfering RNA is targeted to the transgene mRNA so that any mRNA that does locate to the cytoplasm is a target for RNAi-mediated degradation and/or cleavage, preferably cleavage.

The interfering RNA(s) can be provided *in trans* or *in cis* during lentiviral vector production. Thus, an interfering RNA expression cassette may be co-expressed with lentiviral vector components during lentiviral vector production (such that the interfering RNA(s) are provided *in trans*)*.* Alternatively, the lentiviral vector genome expression cassette further comprises a vector intron and the vector intron comprises the nucleic acid sequence encoding the interfering RNA (such that the interfering RNA(s) are provided *in cis*)*.*

Accordingly, in one aspect, the invention provides a set of nucleotide sequences comprising nucleotide sequences encoding lentiviral vector components and a nucleic acid sequence encoding an interfering RNA of the invention.

In a further aspect, the invention provides a set of nucleotide sequences comprising nucleotide sequences encoding lentiviral vector components and a nucleotide sequence comprising a lentiviral vector genome expression cassette of the invention.

In a further aspect, the invention provides a set of nucleotide sequences comprising nucleotide sequences encoding lentiviral vector components, a nucleotide sequence comprising a lentiviral vector genome expression cassette of the invention and a nucleic acid sequence encoding an interfering RNA of the invention.

In some embodiments, the set of nucleic acid sequences comprises a first nucleic acid sequence encoding the lentiviral vector genome and at least a second nucleic acid sequence encoding the interfering RNA. Preferably, the first and second nucleic acid sequences are separate nucleic acid sequences. Suitably, the nucleic acid encoding the lentiviral vector genome may not comprise the nucleic acid sequence encoding the interfering RNA.

In some embodiments, the nucleic acid encoding the lentiviral vector genome comprises the nucleic acid sequence encoding the interfering RNA.

In some embodiments, the lentiviral vector components include gag-pol, env, and optionally rev.

### Sequence-upgraded polyadenylation LTRs

In eukaryotes, polyadenylation is part of the maturation of mRNA for translation and involves the addition of a polyadenine (poly(A)) tail to an mRNA transcript. The poly(A) tail comprises multiple adenosine monophosphates and is important for the nuclear export, translation and stability of mRNA. The process of polyadenylation begins as the transcription of a gene terminates. A set of cellular proteins binds to the polyA sequence elements such that the 3' segment of the transcribed pre-mRNA is first cleaved followed by synthesis of the poly(A) tail at the 3' end of the mRNA. In alternative polyadenylation, a poly(A) tail is added at one of several possible sites, producing multiple transcripts from a single gene.

Native retroviral vector genomes are typically flanked by 3' and 5' long terminal repeats (LTRs). Native retrovirus LTRs comprise a U3 region (containing the enhancer/promoter activities necessary for transcription), and an R-U5 region that comprises important cis-acting sequences regulating a number of functions, including packaging, splicing, polyadenylation and translation. Retrovirus polyadenylation (polyA) sequences required for efficient transcriptional termination also reside within native retrovirus LTRs (see Figure 1 and Figure 6).

The typical structure and spacing of functional elements of polyadenylation sequences for terminating pol-II transcription have been well characterized (Proudfoot (2011), Genes & Dev. 25: 1770-1782), and can be simply summarized as having: [1] a core polyadenylation signal (PAS; canonical sequence AAUAAA), [2] a cleavage site typically 15-30 nucleotides downstream of the PAS (often a 'CA' motif), [3] a downstream GU-rich downstream enhancer (DSE), broadly within ~100 nucleotides of the PAS (typically with 20 nucleotides for strong polyadenylation sequences), and [4] an upstream enhancer (USE), broadly within ~60 nucleotides of the PAS (see Figure 1).

Host cell and viral gene expression levels can be regulated by the presence/absence or strength of an USE and/or DSE, and so it is recognized that there is great diversity in examples of polyadenylation sequences. Very strong viral polyadenylation sequences such as Simian Virus 40 (SV40) late polyA contain all four of these elements within a sequence less than 130 nucleotides, and strong synthetic polyA sequences based on the rabbit beta-globin polyadenylation sequence that lack a USE entirely, and is less than 50 nucleotides in total have been described (Proudfoot (2011), Genes & Dev. 25: 1770-1782). Nevertheless, these four common elements are widely accepted to contribute to transcription termination efficiency, and have been shown to be employed in retroviral LTRs, including HIV-1.

As summarized above, the PAS, cleavage site and DSE for HIV-1 polyadenylation are all located across the R-U5 region of the LTR, which also forms part of the broader packaging signal for assembly of genomic vRNA in to virions (see Figure 6). Retroviruses typically do not utilize very strong polyadenylation sequences due to the need to balance transcriptional activity driven from the 5' LTR and efficient polyadenylation at the 3' LTR, despite the LTRs being identical in sequence. This may be partly addressed by the fact that the 5' LTR vRNA sequence may adopt a subtly different structure compared to the 3' LTR due to the presence of RNA immediately downstream (which would not be present downstream of the 3' LTR due to termination), and the lack of U3-encoded RNA at the 5' LTR, which is present in 3' LTR transcribed RNA (Das et al. (1999), Journal of virology 73: 81-91; and Klasens et al. (1999), Nucleic Acids Res. 27: 446-54). Indeed, it has been shown that the HIV-1 U3 also contains polyA enhancer sequences that overlay the promoter sequences (DeZazzo et al. (1991), Mol. Cell. Biol. 11:1624-30; and Gilmartin et al. (1995) Genes Dev. 9: 72-83).

The self-inactivating LTR (SIN-LTR) feature essentially introduces a deletion within the U3 region such that enhancer/promoter activity is abolished; due to the LTR copying mechanism during reverse transcription, this results in an integrated LV genome expression cassette with no or very minimal transcriptional activity at either the 5' or 3' LTRs (since they are identical in sequence). This means that the only transcriptionally active component of a SIN-LTR containing LV once integrated, is from the transgene cassette. U3-deleted LTRs have been shown to have less polyadenylation activity compared to wild type, non-U3 deleted LTRs (Yang et al. (2007), Retrovirology 4:4), indicating that SIN-LTRs within LVs would be limited in the same fashion.

There are several consequences of weak PAS within LVs, and in particular SIN-LTR-containing LVs, as described below and presented in Figure 2A:
1) Transcriptional read-through the 3'-LTR (such as a 3' SIN-LTR) of the LV expression cassette during vector production. This will lead to an elongated vector genome RNA (vRNA) that may be too long to package or result in low steady state abundance of vRNA. The current solution to this problem is to employ a strong heterologous polyadenylation sequence (such as the late SV40 polyA) within a few hundred nucleotides downstream of the 3' LTR as a back-up, thus reducing the size of the vRNA in the event of transcriptional read-through the 3'-LTR.
2) Transcriptional read-through of the transgene cassette in transduced cells. This will lead to an elongated 3' UTR for the transgene mRNA, which may lead to lower steady-state levels of the transgene mRNA.
3) Transcription of cellular genes downstream of the 3' LTR (such as a 3' SIN-LTR) in transduced cells due to transcriptional read-through. This will result in inappropriate expression of functional RNA or translation of the ('dark') proteome. Typically, by 'dark' proteome it is meant either 'junk' open-reading frames (which have no function, and will likely trigger mis-folding responses) or an ORF that was no longer transcribed into RNA (due to mutation e.g. of its upstream promoter). Unwanted transcription read-through might therefore result in expression of a gain-of-function of such 'junk' ORFs.
4) Transcriptional read-in through the 5' LTR (such as a 5' SIN-LTR) from cellular genes in transduced cells. This will lead to production of RNA encoding the LV packaging signal and RRE sequences, as well as to potential interference of the internal transgene promoter. The same mechanism may result in inappropriate expression of transgene protein in transduced cells, for example in transduced cells where the transgene expression would otherwise be restricted by a tissue specific promoter.
5) Transcriptional read-in through the 5' LTR (such as a 5' SIN-LTR) to the major splice donor site within the LV genome in transduced cells (this might also be possible during LV production when using circular (i.e. plasmid) DNA, as transcriptional 'read-around' may occur). This may allow inappropriate splicing to downstream transgene RNA and/or trans-splicing to other cellular pre-mRNA transcripts.

The present inventors surprisingly found that modified polyA sequences can be designed to reduce (e.g. greatly minimise) and/or eliminate transcriptional read-out and/or read-in through the LV LTRs. Thus, the invention provides modified polyA sequences. Figure 2B contrasts SIN-LTRs with a simple summary of the invention. In brief, to overcome the stated problems, the invention can be defined by four major facets resulting in the new modified LTRs (termed 'sequence-upgraded polyA' LTRs or 'supA-LTRs'):
1. Introducing a new PAS into the SIN/U3 region such that it becomes the primary functional PAS for polyadenylation. Another way of stating this is to 'move' the primary functional PAS across the transcriptional start site (TSS) boundary (the TSS is also defined as the U3/R boundary). Yet another way of describing the modification is that in the modified 3' SIN-LTR (unlike current HIV-1 based LVs) all retained R region sequence is located downstream of the primary functional PAS.
2. Encoding a minimal but 'sufficient' length of R region sequence between the primary functional PAS (positioned according to 1) and a polyadenylation cleavage site, such that [1] the polyadenylation activity is high (or transcriptional read-through is demonstrated to be very low), and [2] the process of first strand transfer is efficiently retained (see Figure 3), leading to (i.e. maintaining) high titre vector production.
3. Insertion of a sequence comprising a USE within the SIN/U3 region, thus positioning a USE close to the new, primary PAS.
4. Engineering of the 5' R region - preferably the first stem loop (i.e. the TAR loop) - to encode a cleavage region and GU-rich DSE such that the DSE will be positioned close to the new, primary PAS within both 5' and 3' LTRs (i.e. they will both be supA-LTRs) after transduction/integration of the LV. The new DSE sequence must be positioned within the first stem loop such that at least the same 'minimal but sufficient' length of R region sequence is retained such that first strand transfer can occur efficiently, and ideally the engineered 5' R region is predicted to retain a stem loop structure.

Figure 7 displays the general features of a novel LV genome expression cassette employing the four main facets of the invention. The invention could be used to generate any number of 'supA-LTRs' by using known or synthetic USEs/DSEs according to the above four facets.

Accordingly, in one aspect, the invention provides modified 3' LTRs comprising a modified polyA sequence as described herein. The modified polyA sequences have essentially been modified to re-position a PAS across the 3' U3/R boundary (i.e. to re-position a PAS from the 3' R region to the 3' U3 region) such that the PAS is copied from the 3' LTR to the 5' LTR in integrated LVs. By way of an illustrative example, this is most easily achieved by deleting the entire 3' R-U5 region from the 3' LTR of the LV vRNA expression cassette and replacing this sequence with the Simian Virus 40 (SV40) late polyA sequence (i.e. the SV40 USE, PAS and GU-rich DSE sequence), wherein nucleotides identical to nucleotides 1-20 from the 5' R region of the LV vRNA are inserted immediately downstream (within 6 nucleotides) of the SV40 PAS, thus placing this R region sequence between the PAS and the cleavage site/GU-rich DSE sequence. Some of the heterologous sequence between the PAS and the cleavage site/GU-rich DSE may optionally be deleted and replaced with said R region sequence. The modified polyA sequences are herein referred to as "R-embedded heterologous polyadenylation sequences". As a result of these modifications, efficient polyadenylation will occur at the LV vRNA 3' cleavage site, which will typically be located at the 3' end of the embedded R sequence, and resulting in ~20 nucleotides of homology at both 5' and 3' ends of the vRNA (in the R region), which will allow for efficient first strand transfer during reverse transcription.

The inventors surprisingly found that this modified polyA sequence configuration can be employed to improve transcription termination at the 3' LTR whilst simultaneously ensuring that vRNA cleavage (prior to polyadenylation) allows sufficient 3' R region homology with the 5' R region to retain first strand synthesis. Advantageously, the inventors found that no back-up heterologous polyadenylation sequence is required downstream of the vRNA expression cassette when the modified polyA sequences are used. Transcriptional read-in and/or read-out of a lentiviral vector genome expression cassette comprising a modified polyA sequence as described herein may be reduced compared to the corresponding lentiviral vector genome expression cassette which does not comprise a modified polyA sequence as described herein.

In one aspect, the invention provides a nucleotide sequence encoding a lentiviral vector genome, wherein the 3' LTR of the lentiviral vector genome comprises a modified polyadenylation sequence, and wherein the modified polyadenylation sequence comprises a polyadenylation signal which is 5' of the 3' LTR R region.

In one embodiment, the nucleotide sequence encoding a lentiviral vector is a nucleotide sequence comprising a lentiviral vector genome expression cassette.

Accordingly, in some embodiments of the nucleotide sequence comprising a lentiviral vector genome expression cassette of the invention, the 3' LTR of the lentiviral vector genome comprises a modified polyadenylation sequence, wherein the modified polyadenylation sequence comprises a polyadenylation signal which is 5' of the 3' LTR R region.

Preferably, the lentiviral vector is a SIN lentiviral vector. Thus, preferably, the lentiviral vector genome (e.g. the integrated lentiviral vector genome) comprises 5' and 3' SIN-LTRs.

In some embodiments, the 3' LTR comprises a polyA sequence (e.g. a modified polyadenylation sequence as described herein) in the sense orientation with respect to the lentiviral vector genome expression cassette.

In some embodiments, the 3' LTR further comprises a polyA sequence in the antisense orientation with respect to the lentiviral vector genome expression cassette. Thus, the 3' LTR may comprise a polyA sequence in the sense orientation and a polyA sequence in the antisense orientation.

As used herein, the term "polyadenylation sequence" or "polyA sequence" refers to the sequence required for cleavage and polyadenylation of an mRNA. Thus, the polyA sequence effectively acts as a transcriptional termination signal. A polyA sequence typically comprises a polyadenylation signal (PAS), a polyA downstream enhancer element (DSE) and a polyadenylation cleavage site. Suitably, the hexameric PAS is correctly positioned relative to the PAS in order to ensure that the polyA sequence is functional (i.e. facilitates polyadenylation/termination). Suitably, a functional polyA sequence may also comprise a polyA upstream enhancer element (USE).

As used herein, the term "polyadenylation signal" or "PAS" means the central hexamer sequence motif within a native polyA sequence, which is required for polyadenylation/termination of an mRNA. The PAS is the sequence motif recognised by cleavage and polyadenylation specificity factor (CPSF) within the RNA cleavage complex. This sequence motif varies between eukaryotes but is primarily AAUAAA. CPSF is the central component of the 3' processing machinery for polyadenylated mRNAs and recognizes the PAS, thereby providing sequence specificity in both pre-mRNA cleavage and polyadenylation, and catalyses pre-mRNA cleavage

In some embodiments, the modified polyadenylation sequence is a heterologous polyadenylation sequence.

In some embodiments, the modified polyadenylation sequence comprises a heterologous polyadenylation signal.

Heterologous polyA sequences or PAS may be derived from any suitable source. Such sources may be natural, i.e. directly derived from an organism, or synthetic, i.e. partially or wholly non-natural. For example, a synthetic sequence may be a variant of a natural sequence (e.g. a chimeric sequence, modified sequence or the like) or a sequence not based on a natural sequence. Suitable heterologous polyadenylation sequences and polyadenylation signals for use according to the invention can be found in SV40, rabbit beta globin genes, and human or bovine growth hormone genes.

An illustrative heterologous polyA sequence is provided below:
SV40 late polyadenylation sequence (USE in italics, PAS in bold, GU-rich DSE underlined) 
TTCGAGCAGACATGATAAGATACATTGATGAGTTTGGACAAACCACAACTAGAATGCAGTGAAAAAAA
TGCTTTATTTGTGAAATTTGTGATGCTATTGCTTTATTTGTAACCATTATAAGCTGCAATAAACAAGT
TAACAACAACAATTGCATTCATTTTATGTTTCAGGTTCAGGGGGAGATGTGGGAGGTTTTTTAAAGCA
AGT (SEQ ID NO: 86)
Rabbit beta-globin polyadenylation sequence (PAS in bold, GU-rich DSE underlined) 
GAATTCACTCCTCAGGTGCAGGCTGCCTATCAGAAGGTGGTGGCTGGTGTGGCCAATGCCCTGGCTCA
CAAATACCACTGAGATCTTTTTCCCTCTGCCAAAAATTATGGGGACATCATGAAGCCCCTTGAGCATC
TGACTTCTGGCTAATAAAGGAAATTTATTTTCATTGCAATAGTGTGTTGGAATTTTTTGTGTCTCTCA
CTCGGAAGGACATATGGGAGGGCAAATCATTTAAAACATCAGAATGAGTATTTGGTTTAGAGTTTGGC
AACATATGCCCATATGCTGGCTGCCATGAACAAAGGTTGGCTATAAAGAGGTCATCAGTATATGAAAC
 
AGCCCCCTGCTGTCCATTCCTTATTCCATAGAAAAGCCTTGACTTGAGGTTAGATTTTTTTTATATTT

TGTTTTGTGTTATTTTTTTCTTTAACATCCCTAAAATTTTCCTTACATGTTTTACTAGCCAGATTTTT

CCTCCTCTCCTGACTACTCCCAGTCATAGCTGTCCCTCTTCTCTTAT (SEQ ID NO: 87)
Bovine growth hormone polyadenylation sequence (PAS in bold, GU-rich DSE underlined) 
CTGTGCCTTCTAGTTGCCAGCCATCTGTTGTTTGCCCCTCCCCCGTGCCTTCCTTGACCCTGGAAGGT
GCCACTCCCACTGTCCTTTCCTAATAAAATGAGGAAATTGCATCGCATTGTCTGAGTAGGTGTCATTC
TATTCTGGGGGGTGGGGTGGGGCAGGACAGCAAGGGGGAGGATTGGGAAGACAATAGCAGGCATGCTG
GGGATGCGGTGGGCTCTATGG (SEQ ID NO: 88)
Human growth hormone polyadenylation sequence (PAS in bold, GU-rich DSE underlined)

CTAGCTGCCCGGGTGGCATCCCTGTGACCCCTCCCCAGTGCCTCTCCTGGCCCTGGAAGTTGCCACTC
CAGTGCCCACCAGCCTTGTCCTAATAAAATTAAGTTGCATCATTTTGTCTGACTAGGTGTCCTTCTAT
AATATTATGGGGTGGAGGGGGGTGGTATGGAGCAAGGGGCAAGTTGGGAAGACAACCTGTAGGGCCTG
CGGGGTCTATTGGGAACCAAG (SEQ ID NO: 89)

In some embodiments, the heterologous polyA sequence is as set forth in any one of SEQ ID NOs: 86-89.

In some embodiments, the modified polyadenylation sequence is derived from Simian Virus 40 (SV40), a rabbit beta-globin gene, a human or bovine growth hormone gene or is a variant thereof. Suitably, the heterologous polyA sequence has been modified as described herein (for example, to embed the R region within the polyA sequence) to arrive at a modified polyA sequence in accordance with the invention.

In some embodiments, the modified polyadenylation sequence is a synthetic sequence.

In some embodiments, the polyadenylation signal has the sequence AATAAA encoded in DNA expression cassettes.

In some embodiments, the PAS has the sequence AAUAAA. Other PAS are known, for example, AUUAAA, AGUAAA, UAUAAA, CAUAAA, GAUAAA, AAUAUA, AAUACA, AAUAGA, AAAAAG, and ACUAAA (Beaudoing et al. (2000), Genome Res. 10: 1001-1010).

As used herein, the term "polyadenylation cleavage site" or "polyA cleavage site" means the nucleotides at the site of 3'-end cleavage of the mRNA during polyadenylation and the site to which polyadenines are added. Typically, the polyA cleavage site is positioned between the PAS and DSE.

As used herein, the term "upstream enhancer element" or "USE" is used interchangeably with the term "upstream sequence element" to mean a nucleotide sequence which acts as an enhancing element for 3'-end processing efficiency. Typically, in a native polyA sequence, the USE is in the immediate upstream vicinity of the PAS.

As used herein, the term "downstream enhancer element" or "DSE" is used interchangeably with the term "downstream sequence element" to mean a GT/U-rich nucleotide sequence (or "GU-box") which enhances 3'-end formation. By "GT/U-rich nucleotide sequence" is meant a GT-rich DNA sequence or a GU-rich RNA sequence. Typically, in a native polyA sequence, the DSE is located in the downstream vicinity of the 3' end of the mRNA in the 3' LTR.

As a result of the use of the modified polyA sequence, which is copied during reverse transcription such that it is present within both the 5' and 3' LTR, the native retrovirus polyA signals within the 5' and 3' R regions can be functionally mutated or deleted. Suitably, the endogenous (i.e. native) polyA signals may be mutated such that they no longer function to facilitate polyadenylation.

In some embodiments, the native 3' polyadenylation sequence has been mutated or deleted.

In some embodiments, the native 3' polyadenylation signal or native 5' polyadenylation signal has been mutated or deleted.

A strong polyA sequence mediates efficient polyadenylation of the mRNA, i.e. reduces or minimises transcriptional read-through of the polyA sequence relative to a polyA sequence which does not mediate efficient polyadenylation. Typically, a strong polyA sequence possesses both a canonical AAUAAA PAS and clearly defined USE and/or DSE positioned appropriately (for the DSE, this can be within ~100 nucleotides of the cleavage site, and is typically with 20 nucleotides for strong polyadenylation sequences) to enhance polyadenylation as described herein.

In some embodiments, the modified polyadenylation sequence is a strong polyadenylation sequence.

In some embodiments, the polyadenylation signal which is 5' of the 3' LTR R region is in the sense strand. Suitably, the polyadenylation signal which is 5' of the 3' LTR R region is in the sense orientation with respect to the lentiviral vector genome.

In some embodiments, the modified polyadenylation sequence further comprises a polyadenylation upstream enhancer element (USE).

In some embodiments, the USE is 5' of the polyadenylation signal.

In some embodiments, the USE comprises the sequence as set forth in SEQ ID NO: 83.

In some embodiments, the USE has the sequence as set forth in SEQ ID NO: 83.

In some embodiments, the modified polyadenylation sequence comprises a GT/U rich downstream enhancer element.

In some embodiments, the modified polyadenylation sequence comprises a downstream enhancer element that is bound by CFIm25/68.

In some embodiments, the GT/U rich downstream enhancer element is 3' of the polyadenylation signal.

In some embodiments, the enhancer element is a strong enhancer element. For USEs, these may be native or synthetic RNA sequences known to impart a strong enhancement to polyadenylation/termination at a PAS when positioned in the upstream vicinity of the PAS, and may be derived from an RNA sequence known to bind to the CFIm complex (Yang et al. (2011), Structure 19: 368-377). For DSEs, these may be native or synthetic RNA sequences known to impart a strong enhancement to polyadenylation/termination at a PAS when positioned in the upstream vicinity of the PAS, and may be derived from an RNA sequence known to bind CSTF-64 (Takagaki and Manley (1997), Molecular and Cellular Biology 17: 3907-3914).

In some embodiments, the 3' LTR R region of the modified polyadenylation sequence is a minimal R region.

As used herein, the terms "R region" and "embedded R region" in the context of the modified 3' LTR and/or modified 5' LTR of the invention mean a sequence between the PAS and polyA cleavage site. Preferably, the R region has suitable homology within the terminal nucleotides of the 5' vector genome. Suitably, the R region may be an endogenous sequence present between the PAS and polyA cleavage site or may be a heterologous sequence. Suitably, the R region within a modified 3' LTR or modified 5' LTR of the invention may be the native R region or a portion thereof. The portion of a native R region may be a minimal R region. The R region may be a sequence within 50 nt of the transcription start site within the lentiviral vector genome expression cassette.

As used herein, the term "minimal functional R region" or "minimal R region" is meant a truncated 3' R region sequence which retains the function of the full-length R region sequence. Thus, the minimal functional R region retains sufficient homology (i.e. sufficient length) between the 3' and 5' LTR R regions for first strand transfer to occur when employing either a native 5' R region or a modified 5' R region of the invention. For example, a lentiviral vector genome expression cassette may employ a full length native 5' R region in combination with a modified 3' supA-LTR containing a minimal (embedded) R region of 10, 12, 14, 16, 18, or 20 nucleotides. For example, a lentiviral vector genome expression cassette may employ a modified 5' R region containing a GU-rich DSE in combination with a modified 3' supA-LTR containing a minimal (embedded) R region of 10, 12, 14, 16, 18, or 20 nucleotides.

During reverse transcription, a tRNA primer hybridises to a complementary sequence within the viral genome called the primer binding site (PBS) located downstream of the 5' R-U5 region. Reverse transcriptase then synthesises complementary DNA (cDNA; first minus strand DNA) to the 5' R-U5 region of the vRNA, followed by degradation of the 5' R-U5 region on the vRNA by the RNaseH domain of the reverse transcriptase enzyme. The first minus strand DNA then transfers to the 3' end of the vRNA and hybridises to the complementary R region within the 3' R-U5 region of the vRNA. Reverse transcriptase then synthesises complementary DNA (cDNA) from the 3' R region of the vRNA towards the 5' end of the vRNA. The majority of the vRNA is degraded by the RNaseH domain, leaving only the 3' PPT and cPPT sequences. The remaining PPT fragments functions as primers for second strand synthesis, beginning from the PPT fragments and ending at the 3' end of the vRNA. A second transfer then occurs, in which the PBS from the newly synthesised second strand hybridises with the complementary PBS on the first strand, followed by extension of both strands to form the cDNA (i.e. dsDNA).

As a result of the first transfer during reverse transcription of the vRNA, the 5' R-U5 region (including the 5' PAS) is copied from the 5' LTR of the vRNA to the 3' LTR of the cDNA. As a result of the second transfer during reverse transcription of the vRNA, the 3' U3 region is copied from the 3' LTR of the vRNA to the 5' LTR of the cDNA. Therefore, the cDNA comprises identical LTRs at the 5' and 3' ends, each comprising the 3' U3 region and 5' R-U5 region of the vRNA.

In order to maintain the correct process of reverse transcription, the R regions within the 3' and 5' LTRs of the lentiviral vector are of sufficient length to permit the terminal nucleotide sequences of the mRNA to anneal to the first strand of cDNA produced from the 5'end of the vRNA. Since it is known that cleavage of an mRNA prior to addition of polyadenines occurs at the polyA cleavage site which is within 15-30 nucleotides of the PAS for Rous sarcoma virus (RSV), mouse mammary tumour virus (MMTV) and similar retroviruses, the length of 5' and 3' R region homology required for first strand transfer (as part of the reverse transcription process, and copying of LTRs) must be limited to <20 nucleotides. To date, the shortest length of homology shown to allow efficient first strand transfer has only been demonstrated in murine leukaemia virus (MLV), being 12 nucleotides of R region at both 5' and 3' ends of the vRNA (Dang and Hu (2001), J. Virol. 75: 809-20). For wild type HIV-1, a study was performed assessing the wild-type 3' R region length (97 nt) and truncated versions (37 and 15 nt), with both truncated versions resulting in progressively greater attenuated virus growth kinetics of 50% and 5% respectively compared to wild type virus (Berkhaut et al (1995), J. Mol. Biol. 252: 59-69). Others reduced the size of R region to 47 nucleotides within lentiviral vectors when employing heterologous polyA sequences downstream of the 3' R region without apparent negative impact on titres (Koldej and Anson (2009), BMC Biotechnol 9:86). Prior to the present invention therefore, it was not known whether truncation of the 3' R region between a PAS and the cleavage site (i.e. ~20 nucleotides or fewer) would be sufficient to allow for first strand transfer or negatively impact some other aspect of reverse transcription. The present inventors surprisingly found that truncation of the R region to 20 nucleotides or even as few as 14 nucleotides does not result in attenuation of reverse transcription. In particular, the present inventors unexpectedly found that truncation of the R region even further to 10 nucleotides still allowed for production of practicable levels of LVs, albeit 2-to-10 fold lower titres compared to standard LVs.

In some embodiments, the minimal R region is:
(a) at least 10 nucleotides;
(b) at least 12 nucleotides;
(c) at least 14 nucleotides;
(d) at least 16 nucleotides; -
(e) at least 18 nucleotides, or
(f) at least 20 nucleotides
in length.

In one embodiment, the minimal R region is at least 10 nucleotides in length.

In one embodiment, the minimal R region is at least 12 nucleotides in length.

In one embodiment, the minimal R region is at least 14 nucleotides in length.

In one embodiment, the minimal R region is at least 16 nucleotides in length.

In one embodiment, the minimal R region is at least 18 nucleotides in length.

In one embodiment, the minimal R region is at least 20 nucleotides in length.

In some embodiments, the minimal R region comprises a sequence derived from a native R region. Suitably, the minimal R region is a portion of a native R region.

In some embodiments, the R region comprises a sequence as set forth in any one of SEQ ID NOs: 25-36 or the sequence GTCTCTCT.

In one embodiment, the R region comprises a sequence as set forth in SEQ ID NO: 25.

In one embodiment, the R region comprises a sequence as set forth in SEQ ID NO: 26.

In one embodiment, the R region comprises a sequence as set forth in SEQ ID NO: 27.

In one embodiment, the R region comprises a sequence as set forth in SEQ ID NO: 28.

In one embodiment, the R region comprises a sequence as set forth in SEQ ID NO: 29.

In one embodiment, the R region comprises a sequence as set forth in SEQ ID NO: 30.

In one embodiment, the R region comprises a sequence as set forth in SEQ ID NO: 31.

In one embodiment, the R region comprises a sequence as set forth in SEQ ID NO: 32.

In one embodiment, the R region comprises a sequence as set forth in SEQ ID NO: 33.

In one embodiment, the R region comprises a sequence as set forth in SEQ ID NO: 34.

In one embodiment, the R region comprises a sequence as set forth in SEQ ID NO: 35.

In one embodiment, the R region comprises a sequence as set forth in SEQ ID NO: 36.

In one embodiment, the R region comprises the sequence GTCTCTCT.

In some embodiments, the R region has the sequence as set forth in any of SEQ ID NOs: 25-36 or the sequence GTCTCTCT.

In some embodiments, the 3' LTR R region of the modified polyadenylation sequence is homologous to the 5' LTR R region of the lentiviral vector genome. The 3' LTR R region of the modified polyadenylation sequence may have at least 60% (suitably, at least 70%, at least 75%, at least 80%, at least 85%, at least 90%, at least 95%, at least 96%, at least 97%, at least 98% or at least 99%) sequence identity to the 5' LTR R region. The 3' LTR R region of the modified polyadenylation sequence may have less than five (suitably, less than four, less than three, less than two or no) mismatches with the native 5' LTR R region. The 3' LTR R region of the modified polyadenylation sequence may be identical to the 5' LTR R region. The 3' LTR R region of the modified polyadenylation sequence may be identical to the 5' LTR R region.

In some embodiments, the 3' LTR R region of the modified polyadenylation sequence is homologous to the native 5' LTR R region of the lentiviral vector genome. The 3' LTR R region of the modified polyadenylation sequence may have at least 60% (suitably, at least 70%, at least 75%, at least 80%, at least 85%, at least 90%, at least 95%, at least 96%, at least 97%, at least 98% or at least 99%) sequence identity to the native 5' LTR R region. The 3' LTR R region of the modified polyadenylation sequence may have less than five (suitably, less than four, less than three, less than two or no) mismatches with the native 5' LTR R region. The 3' LTR R region of the modified polyadenylation sequence may be identical to the native 5' LTR R region. The 3' LTR R region of the modified polyadenylation sequence may be identical to the native 5' LTR R region.

As used herein, the term "mismatch" refers to the presence of an uncomplimentary base. Thus, a "mismatch" refers to an uncomplimentary base in the 3' R region which is not capable of Watson-Crick base pairing with the complementary sequence within the 5' R region or *vice versa.*

As described herein, the R region may be embedded within the polyA sequence.

In some embodiments, the 3' modified polyA sequence comprises a sequence as set forth in any one of SEQ ID NOs: 37-54.

In one embodiment, the 3' modified polyA sequence comprises a sequence as set forth in SEQ ID NO: 37.

In one embodiment, the 3' modified polyA sequence comprises a sequence as set forth in SEQ ID NO: 38.

In one embodiment, the 3' modified polyA sequence comprises a sequence as set forth in SEQ ID NO: 39.

In one embodiment, the 3' modified polyA sequence comprises a sequence as set forth in SEQ ID NO: 40.

In one embodiment, the 3' modified polyA sequence comprises a sequence as set forth in SEQ ID NO: 41.

In one embodiment, the 3' modified polyA sequence comprises a sequence as set forth in SEQ ID NO: 42.

In one embodiment, the 3' modified polyA sequence comprises a sequence as set forth in SEQ ID NO: 43.

In one embodiment, the 3' modified polyA sequence comprises a sequence as set forth in SEQ ID NO: 44.

In one embodiment, the 3' modified polyA sequence comprises a sequence as set forth in SEQ ID NO: 45.

In one embodiment, the 3' modified polyA sequence comprises a sequence as set forth in SEQ ID NO: 46.

In one embodiment, the 3' modified polyA sequence comprises a sequence as set forth in SEQ ID NO: 47.

In one embodiment, the 3' modified polyA sequence comprises a sequence as set forth in SEQ ID NO: 48.

In one embodiment, the 3' modified polyA sequence comprises a sequence as set forth in SEQ ID NO: 49.

In one embodiment, the 3' modified polyA sequence comprises a sequence as set forth in SEQ ID NO: 50.

In one embodiment, the 3' modified polyA sequence comprises a sequence as set forth in SEQ ID NO: 51.

In one embodiment, the 3' modified polyA sequence comprises a sequence as set forth in SEQ ID NO: 52.

In one embodiment, the 3' modified polyA sequence comprises a sequence as set forth in SEQ ID NO: 53.

In one embodiment, the 3' modified polyA sequence comprises a sequence as set forth in SEQ ID NO: 54.

In some embodiments, the 3' LTR R region of the modified polyadenylation sequence is 0-6 nucleotides downstream of the polyadenylation signal.

In some embodiments, the 3' LTR R region of the modified polyadenylation sequence is immediately downstream of the polyadenylation signal.

In some embodiments, the modified polyadenylation sequence further comprises a polyadenylation cleavage site.

In some embodiments, the polyadenylation cleavage site comprises at least one CA dinucleotide motif. Suitably, the polyA cleavage site comprises two CA dinucleotide motifs, i.e. has the sequence CACA.

In some embodiments, the polyadenylation cleavage site is 3' of the polyadenylation signal.

In some embodiments, the 3' modified polyA sequence comprises a sequence as set forth in any one of SEQ ID NOs: 55-60, or 200-208.

In one embodiment, the 3' modified polyA sequence comprises a sequence as set forth in SEQ ID NO: 55.

In one embodiment, the 3' modified polyA sequence comprises a sequence as set forth in SEQ ID NO: 56.

In one embodiment, the 3' modified polyA sequence comprises a sequence as set forth in SEQ ID NO: 57.

In one embodiment, the 3' modified polyA sequence comprises a sequence as set forth in SEQ ID NO: 58.

In one embodiment, the 3' modified polyA sequence comprises a sequence as set forth in SEQ ID NO: 59.

In one embodiment, the 3' modified polyA sequence comprises a sequence as set forth in SEQ ID NO: 60.

In one embodiment, the 3' modified polyA sequence comprises a sequence as set forth in SEQ ID NO: 200.

In one embodiment, the 3' modified polyA sequence comprises a sequence as set forth in SEQ ID NO: 201.

In one embodiment, the 3' modified polyA sequence comprises a sequence as set forth in SEQ ID NO: 202.

In one embodiment, the 3' modified polyA sequence comprises a sequence as set forth in SEQ ID NO: 203.

In one embodiment, the 3' modified polyA sequence comprises a sequence as set forth in SEQ ID NO: 204.

In one embodiment, the 3' modified polyA sequence comprises a sequence as set forth in SEQ ID NO: 205.

In one embodiment, the 3' modified polyA sequence comprises a sequence as set forth in SEQ ID NO: 206.

In one embodiment, the 3' modified polyA sequence comprises a sequence as set forth in SEQ ID NO: 207.

In one embodiment, the 3' modified polyA sequence comprises a sequence as set forth in SEQ ID NO: 208.

In some embodiments, the 3' modified polyA sequence has a sequence as set forth in any one of SEQ ID NOs: 55-60, 200 or 201.

In one embodiment, the modified 3' LTR further comprises a polyadenylation sequence in anti-sense orientation in the modified 3' LTR between the U3 (e.g. SIN-U3 or ΔU3) and R-embedded heterologous polyadenylation sequence. Suitably, the polyadenylation sequence in anti-sense orientation is a functional polyadenylation sequence. The further polyadenylation sequence is in anti-sense orientation with respect to the lentiviral vector genome. Suitably, the further polyadenylation sequence is 5' of the polyadenylation sequence in the sense strand (i.e. 5' of the embedded R region comprising the heterologous polyadenylation sequence in the sense strand) and 3' of the attachment sequence of the 3' LTR (e.g. of the SIN-LTR), i.e. 3' of the U3, SIN-U3 or ΔU3 (see Figure 53). Thus, the modified 3' LTR may comprise a first polyadenylation signal which is 5' of the 3' LTR R region in the sense strand and a second polyadenylation sequence in anti-sense strand between the U3 (e.g. ΔU3) and R-embedded heterologous polyadenylation sequence of the 3' LTR.

In one embodiment, the modified 3' LTR further comprises a polyadenylation sequence in anti-sense orientation in the modified 3' LTR between the ΔU3 and R-embedded heterologous polyadenylation sequence. Suitably, the polyadenylation sequence in anti-sense orientation is a functional polyadenylation sequence.

In another aspect, the invention provides modified 5' LTRs which have been engineered to contain a GU-rich DSE as described herein. The inventors surprisingly found that the 5' R region of the vRNA may be engineered to contain a GU-rich sequence that functions as a DSE in the recapitulated LTRs (i.e. in the LTRs following reverse transcription) to provide the PAS with an efficient DSE in a close position within the LTRs of the integrated LV genome cassette. Following reverse transcription and the LTR-copying process, the USE-PAS sequence residing within the U3 region in both 5' and 3' LTRs will be 'serviced' by this new DSE (i.e. the DSE will act upon the USE-PAS sequence). By way of illustrative example, the 5' R region of the modified 5' LTR has been designed in order to retain the first 20 nucleotides of native HIV-1 5' R region (so that it can partake in first strand transfer with the minimal 14-20 nucleotides present at the 3' end of the vRNA resulting from polyadenylation using an R-embedded heterologous polyadenylation sequence described herein) as well as retaining a stem-loop structure, which may be important for vRNA packaging and/or stability.

Optionally, the DSE-modified R sequence can also be employed at the 3' LTR as part of a synthetic R-embedded heterologous polyA sequence, functioning as the DSE for the 3' polyA sequence as well.

Accordingly, in a further aspect, the invention provides a nucleotide sequence encoding a lentiviral vector genome, wherein the lentiviral vector genome comprises a modified 5' LTR, and wherein the R region of the modified 5' LTR comprises at least one polyadenylation downstream enhancer element (DSE). Suitably, the modified 5' LTR is engineered to introduce two or three DSEs within the R region.

In one embodiment, the nucleotide sequence encoding a lentiviral vector is a nucleotide sequence comprising a lentiviral vector genome expression cassette.

Accordingly, in some embodiments of the nucleotide sequence comprising a lentiviral vector genome expression cassette of the invention, the lentiviral vector genome comprises a modified 5' LTR, wherein the R region of the modified 5' LTR comprises at least one polyadenylation downstream enhancer element (DSE).

The R region may be an R region or a minimal R region as described herein.

In some embodiments, the first 55 nucleotides of the modified 5' LTR comprises the at least one polyadenylation DSE. Preferably, the first 40 nucleotides of the modified 5' LTR comprises the at least one polyadenylation DSE.

In some embodiments, the at least one polyadenylation DSE is comprised within a stem loop structure of the modified 5' LTR.

In some embodiments, the at least one polyadenylation DSE is comprised within loop 1 loop 1 (i.e. the TAR loop) of the modified 5' LTR.

In some embodiments, the polyadenylation DSE is a GT/U-rich sequence.

In some embodiments, the polyadenylation DSE comprises a sequence as set forth in any one of SEQ ID NOs: 75-82.

In one embodiment, the polyadenylation DSE comprises a sequence as set forth in SEQ ID NO: 75.

In one embodiment, the polyadenylation DSE comprises a sequence as set forth in SEQ ID NO: 76.

In one embodiment, the polyadenylation DSE comprises a sequence as set forth in SEQ ID NO: 77.

In one embodiment, the polyadenylation DSE comprises a sequence as set forth in SEQ ID NO: 78.

In one embodiment, the polyadenylation DSE comprises a sequence as set forth in SEQ ID NO: 79.

In one embodiment, the polyadenylation DSE comprises a sequence as set forth in SEQ ID NO: 80.

In one embodiment, the polyadenylation DSE comprises a sequence as set forth in SEQ ID NO: 81.

In one embodiment, the polyadenylation DSE comprises a sequence as set forth in SEQ ID NO: 82.

In some embodiments, the polyadenylation DSE has a sequence as set forth in any one of SEQ ID NOs: 75-82.

In some embodiments, the GT/U-rich sequence is derived from RSV or MMTV, or wherein the GU-rich sequence is synthetic. Preferably, the GT/U-rich sequence is derived from MMTV.

In some embodiments, the GT/U-rich sequence is bound by CSTF-64.

In some embodiments, the native polyadenylation signal of the modified 5' LTR has been mutated or deleted.

In some embodiments, the modified 5' LTR does not comprise a native polyadenylation signal.

In some embodiments, the 5' R region of the modified 5' LTR further comprises a polyadenylation cleavage site.

In some embodiments, the polyadenylation cleavage site comprises at least one CA dinucleotide motif. Suitably, the polyA cleavage site comprises two CA dinucleotide motifs, i.e. has the sequence CACA.

In some embodiments, the modified 5' LTR comprises a sequence as set forth in any one of SEQ ID NOs: 61-74 or 186-199.

In one embodiment, the modified 5' LTR comprises a sequence as set forth in SEQ ID NO: 61.

In one embodiment, the modified 5' LTR comprises a sequence as set forth in SEQ ID NO: 62.

In one embodiment, the modified 5' LTR comprises a sequence as set forth in SEQ ID NO: 63.

In one embodiment, the modified 5' LTR comprises a sequence as set forth in SEQ ID NO: 64.

In one embodiment, the modified 5' LTR comprises a sequence as set forth in SEQ ID NO: 65.

In one embodiment, the modified 5' LTR comprises a sequence as set forth in SEQ ID NO: 66.

In one embodiment, the modified 5' LTR comprises a sequence as set forth in SEQ ID NO: 67.

In one embodiment, the modified 5' LTR comprises a sequence as set forth in SEQ ID NO: 68.

In one embodiment, the modified 5' LTR comprises a sequence as set forth in SEQ ID NO: 69.

In one embodiment, the modified 5' LTR comprises a sequence as set forth in SEQ ID NO: 70.

In one embodiment, the modified 5' LTR comprises a sequence as set forth in SEQ ID NO: 71.

In one embodiment, the modified 5' LTR comprises a sequence as set forth in SEQ ID NO: 72.

In one embodiment, the modified 5' LTR comprises a sequence as set forth in SEQ ID NO: 73.

In one embodiment, the modified 5' LTR comprises a sequence as set forth in SEQ ID NO: 74.

In one embodiment, the modified 5' LTR comprises a sequence as set forth in SEQ ID NO: 186.

In one embodiment, the modified 5' LTR comprises a sequence as set forth in SEQ ID NO: 187.

In one embodiment, the modified 5' LTR comprises a sequence as set forth in SEQ ID NO: 188.

In one embodiment, the modified 5' LTR comprises a sequence as set forth in SEQ ID NO: 189.

In one embodiment, the modified 5' LTR comprises a sequence as set forth in SEQ ID NO: 190.

In one embodiment, the modified 5' LTR comprises a sequence as set forth in SEQ ID NO: 191.

In one embodiment, the modified 5' LTR comprises a sequence as set forth in SEQ ID NO: 192.

In one embodiment, the modified 5' LTR comprises a sequence as set forth in SEQ ID NO: 193.

In one embodiment, the modified 5' LTR comprises a sequence as set forth in SEQ ID NO: 194.

In one embodiment, the modified 5' LTR comprises a sequence as set forth in SEQ ID NO: 195.

In one embodiment, the modified 5' LTR comprises a sequence as set forth in SEQ ID NO: 196.

In one embodiment, the modified 5' LTR comprises a sequence as set forth in SEQ ID NO: 197.

In one embodiment, the modified 5' LTR comprises a sequence as set forth in SEQ ID NO: 198.

In one embodiment, the modified 5' LTR comprises a sequence as set forth in SEQ ID NO: 199.

In some embodiments, the modified 5' LTR has a sequence as set forth in any one of SEQ ID NOs: 61-74, or 186-199.

For HIV-1, vRNA transcription initiation at the specific transcription start site (TSS) can vary across the first three 'G' nucleotides (i.e. nucleotides 1-3) of the 5' R region, resulting in '3G', '2G' or '1G' vRNA species (see Figure 17). It is believed that for wild type HIV-1 infection the specific TSS shifts from 3G/2G to 1G affecting the fate of vRNA produced, altering the requirements of component expression from just gagpol translation (3G/2G vRNA) to include vRNA packaging (1G vRNA) at later times in infection (Kharytonchyk et al. (2016) Proc. Natl. Acad. Sci. U S A. 113: 13378-13383; and Brown et al (2020) Science 368: 413-417). The production of only 1G vRNA would provide the option of employing a shorter embedded R region in the 3' supA-LTR (i.e. the modified 3' LTR described herein) and may provide some benefit in general to being able to synthesise 1G vRNA from every transcription event during LV production since, for wild type HIV-1, 1G vRNA has been shown to preferentially dimerise compared to 3G/2G vRNA.

Accordingly, the invention also provides a promoter that enables production of only 1G vRNA; the TSS only encodes a single 'G'. Wild type RSV harbours a single 'G' at its TSS - and consequently 5' terminus - on the vRNA genome, indicating that this promoter is able to position the transcription initiation complex on a precise 'G' nucleotide. The CMV promoter is stronger than RSV, and so the inventors sought to generate a promoter that retained the power of the CMV enhancer/promoter sequence but also contained core sequence from RSV that allowed precise transcription initiation at the single 'G' of an HIV-1 based LV. Accordingly, two CMV-RSV hybrid promoters were designed: one, wherein the sequence between the CMV TATA box and the TSS was replaced with the analogous sequence from RSV U3 ('CMV-RSV1-1G'), and the second wherein the RSV TATA box (including 6 nts upstream) was additionally swapped in ('CMV-RSV2-1G') - see Figure 18A.

An illustrative example of a promoter ('CMV-RSV1-1G') that enables production of only 1G vRNA is as follows (no underline = CMV, underlined = RSV U3, bold = 1G TSS, italics = example 5' R [nts 3-22 of HIV-1] - non-promoter sequence):

CGTTACATAACTTACGGTAAATGGCCCGCCTGGCTGACCGCCCAACGACCCCCGCCCATTGACGTCAA
TAATGACGTATGTTCCCATAGTAACGCCAATAGGGACTTTCCATTGACGTCAATGGGTGGAGTATTTA
CGGTAAACTGCCCACTTGGCAGTACATCAAGTGTATCATATGCCAAGTACGCCCCCTATTGACGTCAA
TGACGGTAAATGGCCCGCCTGGCATTATGCCCAGTACATGACCTTATGGGACTTTCCTACTTGGCAGT
ACATCTACGTATTAGTCATCGCTATTACCATGGTGATGCGGTTTTGGCAGTACATCAATGGGCGTGGA
TAGCGGTTTGACTCACGGGGATTTCCAAGTCTCCACCCCATTGACGTCAATGGGAGTTTGTTTTGGCA
CCAAAATCAACGGGACTTCCGCCCCATTGACGCAAATGGGCGGTAGGCGTGTACGGTGGGAGGTCTAT
ATAAGTGCCTAGCTCGATACAATAAACGTCTCTCTGGTTAGACCACA (SEQ ID NO: 84)

A further illustrative example of a promoter ('CMV-RSV2-1G') that enables production of only 1G vRNA is as follows (no underline = CMV, underlined = RSV U3, bold = 1G TSS, italics = example 5' R [nts 3-22 of HIV-1] - non-promoter sequence):

CGTTACATAACTTACGGTAAATGGCCCGCCTGGCTGACCGCCCAACGACCCCCGCCCATTGACGTCAA
TAATGACGTATGTTCCCATAGTAACGCCAATAGGGACTTTCCATTGACGTCAATGGGTGGAGTATTTA
CGGTAAACTGCCCACTTGGCAGTACATCAAGTGTATCATATGCCAAGTACGCCCCCTATTGACGTCAA
TGACGGTAAATGGCCCGCCTGGCATTATGCCCAGTACATGACCTTATGGGACTTTCCTACTTGGCAGT
ACATCTACGTATTAGTCATCGCTATTACCATGGTGATGCGGTTTTGGCAGTACATCAATGGGCGTGGA
TAGCGGTTTGACTCACGGGGATTTCCAAGTCTCCACCCCATTGACGTCAATGGGAGTTTGTTTTGGCA

CCAAAATCAACGGGACTTCCGCCCCATTGACGCAAATGGGCGGTAGGCGTGTACGGTGGATATTGTAT
TTAAGTGCCTAGCTCGATACAATAAACGTCTCTCTGGTTAGACCACA (SEQ ID NO: 85)

The promoter sequences may be positioned immediately upstream of the R region or minimal R region as described herein.

The modified 3' LTR and/or modified 5' LTRs of the invention may be used in combination with a promoter that enables production of only 1G vRNA as described herein.

In some embodiments, the modified 5' LTR further comprises a promoter comprising the sequence as set forth in SEQ ID NO: 84 or SEQ ID NO: 85.

Further synthetic versions of the improved R-embedded heterologous polyA sequences of the invention can be made by pairing different USEs inserted upstream of the PAS and embedded R sequence with different GT/U-rich DSE elements inserted downstream of the embedded R sequence. This is because, whilst the USE-PAS sequence residing within the 3' U3 region will be copied to the 5' LTR upon integration, the heterologous 3' GU-rich DSE will not be copied. Therefore, combining the modified 3' LTR and modified 5' LTR described herein is advantageous in that the improved DSE in the modified 5' LTR is used in both LTRs following reverse transcription.

Accordingly, in a further aspect, the invention provides a nucleotide sequence encoding a lentiviral vector genome, wherein the 3' LTR of the lentiviral vector genome is a modified 3' LTR as described herein and the 5' LTR of the lentiviral vector genome is a modified 5' LTR as described herein.

Thus, in some embodiments of the nucleotide sequence comprising a lentiviral vector genome expression cassette of the invention, the 3' LTR of the lentiviral vector genome is a modified 3' LTR as described herein and the 5' LTR of the lentiviral vector genome is a modified 5' LTR as described herein.

In some embodiments, the R region of the modified 5' LTR is homologous to the R region of the modified 3' LTR and wherein the R region of the modified 3' LTR is immediately downstream of the 3' polyadenylation signal within the modified 3' LTR.

In some embodiments, the R region of the modified 5' LTR is identical to the R region of the modified 3' LTR and wherein the R region of the modified 3' LTR is immediately downstream of the 3' polyadenylation signal within the modified 3' LTR.

As described herein, during reverse transcription of the vRNA, the 5' R-U5 region (including the 5' PAS) is copied from the 5' LTR of the vRNA to the 3' LTR of the cDNA and the 3' U3 region (e.g. 3' SIN-U3 region) is copied from the 3' LTR of the vRNA to the 5' LTR of the cDNA. Therefore, the integrated LV genome expression cassette in a transduced cell comprises identical LTRs at the 5' and 3' ends, each comprising the 3' U3 region (e.g. 3' SIN-U3 region) and 5' R-U5 region of the vRNA. Thus, the invention encompasses a nucleotide sequence comprising a lentiviral vector genome following reverse transcription.

Accordingly, in a further aspect, the invention provides a nucleotide sequence comprising a lentiviral vector genome, wherein the lentiviral vector genome comprises a modified 3' LTR and a modified 5' LTR, wherein the modified 3' LTR comprises a modified polyadenylation sequence comprising a polyadenylation signal which is 5' of the R region within the modified 3' LTR, and wherein the modified 5' LTR comprises a modified polyadenylation sequence comprising a polyadenylation signal which is 5' of the R region within the modified 5' LTR.

In a further aspect, the invention provides a nucleotide sequence comprising a lentiviral vector genome, wherein the lentiviral vector genome comprises a modified 3' LTR and a modified 5' LTR, wherein the R region within the modified 3' LTR comprises at least one polyadenylation DSE wherein the R region within the modified 5' LTR comprises at least one polyadenylation DSE.

In a further aspect, the invention provides a nucleotide sequence comprising a lentiviral vector genome, wherein the lentiviral vector genome comprises a modified 3' LTR and a modified 5' LTR, wherein the modified 3' LTR comprises a modified polyadenylation sequence comprising a polyadenylation signal which is 5' of the R region within the modified 3' LTR and wherein the R region within the 3' LTR comprises at least one polyadenylation DSE, and wherein the modified 5' LTR comprises a modified polyadenylation sequence comprising a polyadenylation signal which is 5' of the R region within the 5' LTR and wherein the R region within the modified 5' LTR comprises at least one polyadenylation DSE.

In some embodiments, the native 3' LTR polyadenylation sequence has been mutated or deleted.

In some embodiments, the native 3' LTR polyadenylation signal and/or the native 5' LTR polyadenylation signal has been mutated or deleted.

In some embodiments, the modified polyadenylation sequence is a heterologous polyadenylation sequence.

In some embodiments, the modified 3' LTR and the modified 5' LTR are identical.

The supA-LTR approach described herein has been developed further by inserting a functional polyadenylation sequence in anti-sense orientation in the 3'supA-LTR between the ΔU3 and R-embedded heterologous polyadenylation sequence. The consequence and purpose of this feature is to reduce transcriptional read-in from cellular promoters downstream of the integration site, and additionally would provide a 'back-up' polyadenylation sequence to an inverted transgene cassette, should it be employed alone or as part of a bi-directional transgene cassette. The modified LTRS are termed 'sequence-upgraded polyA-2 polyA' LTRs or 'supA-2pA-LTRs'. Thus, the result of this approach is to generate 5' and 3' 'supA-2pA-LTRs' after integration wherein effectively the LTRs contain strong, bi-directional polyadenylation sequences. This results in insulation from transcriptional read-in and read-out from both upstream and/or downstream cellular promoters, i.e. on both flanks of the integrated LV.

Hence, in some embodiments, the modified 3' LTR may further comprise a second polyadenylation sequence in anti-sense orientation in the 3' supA-LTR between the ΔU3 and R-embedded heterologous polyadenylation sequence.

In some embodiments, the inverted DSE/GU rich sequence servicing the inverted polyA sequence comprises a sequence as set forth in any one of SEQ ID NOs: 202-208.

In some embodiments, the inverted DSE/GU rich sequence servicing the inverted polyA sequence has a sequence as set forth in any one of SEQ ID NOs: 202-208.

In a further aspect, the invention provides a lentiviral vector genome encoded by the nucleotide sequence of the invention.

In a further aspect, the invention provides a lentiviral vector genome as described herein. Suitably, the lentiviral vector genome comprises a modified 3' LTR and/or a modified 5' LTR as described herein. Preferably, the lentiviral vector genome comprises a supA-LTR as described herein.

In a further aspect, the invention provides an expression cassette comprising a nucleotide sequence of the invention.

In a further aspect, the invention provides a lentiviral vector comprising the nucleotide sequence comprising a lentiviral vector genome expression cassette as described herein. Suitably, the lentiviral vector genome comprises a modified 3' LTR and/or a modified 5' LTR as described herein. Preferably, the lentiviral vector genome comprises a supA-LTR as described herein.

In a further aspect, the invention provides a lentiviral vector comprising the lentiviral vector genome as described herein. Suitably, the lentiviral vector genome comprises a modified 3' LTR and/or a modified 5' LTR as described herein. Preferably, the lentiviral vector genome comprises a supA-LTR as described herein. Preferably, the lentiviral vector genome comprises a supA-2pA-LTR as described herein.

### Illustrative supA-LTR sequences

Illustrative sequences for use according to the invention are provided in Table 2 below:

### Key for the following sequences:

lower case = 3'ppt
upper case only = ΔU3 region
underlined = heterologous polyA sequence
dark grey highlighted = USE
italics = PAS
bold = R Region
black/red highlighted (i.e. white text) = presumed cleavage site(s)
light grey highlighted = DSE
boxed = anti-sense PAS-DSE

**Table 2**

| **Description** | **Sequence** | **SEQ ID NO.** |
|---|---|---|
| Native HIV-1 R region [TAR/SL1] | [GGGTCTCTCTGGTTAGACCAGATCTGAGCCTGGGAGCTCTCTGGC TAACTAGGGAACCC]ACTGCTTAAGCCTCAATAAAGCTTGCCTTGA GTGCTTC | 24 |
| R. 1-60 | GGGTCTCTCTGGTTAGACCAGATCTGAGCCTGGGAGCTCTCTGGCT AACTAGGGAACCCA | 25 |
| R. 1-24 | GGGTCTCTCTGGTTAGACCAGATCA | 26 |
| R.1-20 [3G-R20] and R. 1-20b | GGGTCTCTCTGGTTAGACCA | 27 |
| R.1-20c | GGGTCTCTCTGGTTAGACCACACACATGGTCTAACCAGAGAGACCC | 28 |
| R.3-20 [R.1-18] [1G-R18] | GTCTCTCTGGTTAGACCA | 29 |
| R.1-16 [3G-R16] | GGGTCTCTCTGGTTAG | 30 |
| R.3-16 [R.1-14] [1G-R14] | GTCTCTCTGGTTAG | 31 |
| R.1-14 [3G-R14] | GGGTCTCTCTGGTT | 32 |
| R.3-14 [R.1-12] [1G-R12] | GTCTCTCTGGTT | 33 |
| R.1-12 [3G-R12] | GGGTCTCTCTGG | 34 |
| R.3-12 [R.1-10] [1G-R10] | GTCTCTCTGG | 35 |
| R.1-10 [3G-R10] | GGGTCTCTCT | 36 |
| R.3-10 [R.1-8] [1G-R8] | GTCTCTCT | - |
| SV40 polyA embedded R.1-20 sequence | AATAAAGGGTCTCTCTGGTTAGACCACAAGTTAACAA | 37 |
| SV40 polyA embedded R.1-20b sequence | AATAAAGGGTCTCTCTGGTTAGACCAAGTTAACAA | 38 |
| SV40 polyA embedded R.1-20c sequence | AATAAAGGGTCTCTCTGGTTAGACCACACACATGGTCTAACCAGAG AGACCCCAAGTTAACAA | 39 |
| SV40 polyA embedded R.3-20 [R.1-18] sequence | AATAAAGTCTCTCTGGTTAGACCACAAGTTAACAA | 40 |
| SV40 polyA embedded R.1-16 sequence | AATAAAGGGTCTCTCTGGTTAGCAAGTTAACAA | 41 |
| SV40 polyA embedded R.3-16 [R.1-14] sequence | AATAAAGTCTCTCTGGTTAGCAAGTTAACAA | 42 |
| SV40 polyA embedded R.1-14 sequence | AATAAAGGGTCTCTCTGGTTCAAGTTAACAA | 43 |
| SV40 polyA embedded R.3-14 [R.1-12] sequence | AATAAAGTCTCTCTGGTTCAAGTTAACAA | 44 |
| SV40 polyA embedded R.1-12 sequence | AATAAAGGGTCTCTCTGGCAAGTTAACAA | 45 |
| SV40 polyA embedded R.3-12 [R.1-10] sequence | AATAAAGTCTCTCTGGCAAGTTAACAA | 46 |
| SV40 polyA embedded R.1-10 sequence | AATAAAGGGTCTCTCTCAAGTTAACAA | 47 |
| SV40 polyA embedded R.3-10 [R.1-8] sequence | AATAAAGTCTCTCTCAAGTTAACAA | 48 |
| Rabbit beta-globin polyA embedded R. 1-24 sequence | AATAAAGGGTCTCTCTGGTTAGACCAGATCATTGCAA | 49 |
| Rabbit beta-globin polyA embedded R. 1-20 sequence | AATAAAGGGTCTCTCTGGTTAGACCACATTGCAA | 50 |
| Rabbit beta-globin polyA embedded R. 1-20b sequence | AATAAAGGGTCTCTCTGGTTAGACCATTGCAA | 51 |
| Rabbit beta-globin polyA embedded R. 1-16 sequence | AATAAAGGGTCTCTCTGGTTAGCATTGCAA | 52 |
| Rabbit beta-globin polyA embedded R. 1-14 sequence | AATAAAGGGTCTCTCTGGTTCATTGCAA | 53 |
| Rabbit beta-globin polyA embedded R. 1-12 sequence | AATAAAGGGTCTCTCTGGCATTGCAA | 54 |
| 3' supA-LTR containing 'R.1-60' embedded SV40 late polyA sequence | aaaagaaaaggggggACTGGAAGGGCTAATTCACTCCCAACGAAGA CAAGATACTAGTAAAAAAATGCTTTATTTGTGAAATTTGTGATGCT ATTGCTTTATTTCTAACCATTATAAGCTGCAATAAAGGGTCTCTCT GGTTAGACCAGATCTGAGCCTGGGAGCTCTCTGGCTAACTAGGGAA CCCACAAGTTAACAACAACAATTGCATTCATTTTATGTTTCAGGTT CAGGGGGAGGTGTGGGAGGTTTTTTAAA | 55 |
| 3' supA-LTR containing 'R.1-20c' embedded SV40 late polyA sequence | aaaagaaaaggggggACTGGAAGGGCTAATTCACTCCCAACGAAGA CAAGATACTAGTAAAAAAATGCTTTATTTGTGAAATTTGTGATGCT ATTGCTTTATTTGTAACCATTATAAGCTGCAATAAAGGGTCTCTCT GGTTAGACCACACACATGGTCTAACCAGAGAGACCCCAAGTTAACA ACAACAATTGCATTCATTTTATGTTTCAGGTTCAGGGGGAGGTGTG GGAGGTTTTTTAAA | 56 |
| 3' supA-LTR containing 'R.1-20' embedded SV40 late polyA sequence | aaaagaaaaggggggACTGGAAGGGCTAATTCACTCCCAACGAAGA CAAGATACTAGTAAAAAAATGCTTTATTTGTGAAATTTGTGATGCT ATTGCTTTATTTCTAACCATTATAAGCTGCAATAAAGGGTCTCTCT GGTTAGACCACAAGTTAACAACAACAATTGCATTCATTTTATGTTT CAGGTTCAGGGGGAGGTGTGGGAGGTTTTTTAAA | 57 |
| 3' supA-LTR containing 'R.1-20' embedded rabbit beta-globin polyA sequence | aaaagaaaaggggggACTGGAAGGGCTAATTCACTCCCAACGAAGA CAAGATACTAGTGAATTCACTCCTCAGGTGCAGGCTGCCTATCAGA | 58 |
| | AGGTGGTGGCTGGTGTGGCCAATGCCCTGGCTCACAAATACCACTG AGATCTTTTTCCCTCTGCCAAAAATTATGGGGACATCATGAAGCCC CTTGAGCATCTGACTTCTGGCTAATAAAGGGTCTCTCTGGTTAGAC CAGATCATTGCAATACTGTGTTGGAATTTTTTGTGTCTCTCACTCG GAAGGACATATGGGAGGGCAAATCATTTAAAACATCAGAATGAGTA TTTGGTTTAGAGTTTGGCAACATATGCCCATATGCTGGCTGC | |
| 3' supA-2pA-LTR containing 'R.1-20' embedded SV40 late polyA sequence with SV40 early polyA sequence in anti-sense | aaaagaaaaggggggACTGGAAGGGCTAATTCACTCCCAACGAAGA CAAGATACTAGTCAGACATGATAAGATACATTGATGAGTTTGGACA AACCACAACTAGAATGCAGTGAAAAAAATGCTTTATTTGTGAAATT TGTGATGCTATTGCTTTATTTGTAACCATTATAAGCTGCAATAAAG GGTCTCTCTGGTTAGACCACAAGTTAACAACAACAATTGCATTCAT TTTATGTTTCAGGTTCAGGGGGAGGTGTGGGAGGTTTTTTAAA | 59 |
| 3' supA-2pA-LTR containing 'R.1-20' embedded SV40 late polyA sequence with synthetic polyA sequence in anti-sense | aaaagaaaaggggggACTGGAAGGGCTAATTCACTCCCAACGAAGA CAAGATACTAGTCACACAAAAAACCAACACACAGATGTAATGAAAA TAAAGATCTTTATTTGTGAAATTTGTGATGCTATTGCTTTATTTGT AACCATTATAAGCTGCAATAAAGGGTCTCTCTGGTTAGACCACAAG TTAACAACAACAATTGCATTCATTTTATGTTTCAGGTTCAGGGGGA GGTGTGGGAGGTTTTTTAAA | 60 |
| 5' DSE modified R region 3GR-GU1 | GGGTCTCTCTGGTTAGACCACACATTGGTGTGCACCTGGCTAACTA GGGAACCC | 61 |
| 5' DSE modified R region 3GR-GU2 | GGGTCTCTCTGGTTAGACCACACATTCGTGTGTTTGTGTCTGTTCT AACTAGGGAACCC | 62 |
| 5' DSE modified R region 3GR-GU2.1 | GGGTCTCTCTGGTTAGACCACACACTTCGTGTGTTTGTGTCTGTTC TAACTAGGGAACCC | 63 |
| 5' DSE modified R region 3GR-GU2.2 | GGGTCTCTCTGGTTAGACCACACACATTCGTGTGTTTGTGTCTGTT CTAACTAGGGAACCC | 64 |
| 5' DSE modified R region 3GR-GU2.3 | GGGTCTCTCTGGTTAGACCACACACAGTTCGTGTGTTTGTGTCTGT TCTAACTAGGGAACCC | 65 |
| 5' DSE modified R region 3GR-GU2.4 | GGGTCTCTCTGGTTAGACCACACACAATTCGTGTGTTTGTGTCTGT TCTAACTAGGGAACCC | 66 |
| 5' DSE modified R region 3GR-GU2.5 | GGGTCTCTCTGGTTAGACCACACACAGATTCGTGTGTTTGTGTCTG TTCTAACTAGGGAACCC | 67 |
| 5' DSE modified R region 3GR-GU2.6 | GGGTCTCTCTGGTTAGACCACACATTCCAGATTCGTGTGTTTGTGT CTGTTCTAACTAGGGAACCC | 68 |
| 5' DSE modified R region 3GR-GU3 | GGGTCTCTCTGGTTAGACCACACATGGTGTGCACCTGGGTTCTAAC TAGGGAACCC | 69 |
| 5' DSE modified R region 3GR-GU4 | GGGTCTCTCTGGTTAGACCACACATTGGTGTGCACCTGTGGTTCTA ACTAGGGAACCC | 70 |
| 5' DSE modified R region 3GR-GU5 | GGGTCTCTCTGGTTAGACCACACATGTCTCGTGTGTTTGTGTCTGT TCGCTAACTAGGGAACCC | 71 |
| 5' DSE modified R region 3GR-GU6 | GGGTCTCTCTGGTTACACCACAATTTGGTGTGCACCTGGGTTTAAC TAGGGAACCC | 72 |
| 5' DSE modified R region 3GR-GU7 | GGGTCTCTCTGGTTAGACCGGCGCAGCGTCATTGGTGTGCACCTGG GTTCTAACTAGGGAACCC | 73 |
| 5' DSE modified R region 3GR-GU8 | GGGTCTCTCTGGTTAGACGCACAACACAAAACCGTGTGTTGGTTTT TTGTGTGCTAACTAGGGAACCC | 74 |
| 5' DSE modified R region 1GR-GU1 | GTCTCTCTGGTTAGACCACACATTGGTGTGCACCTGGCTAACTAGG GAAC | 186 |
| 5' DSE modified R region 1GR-GU2 | GTCTCTCTGGTTAGACCACACATTCGTGTGTTTGTGTCTGTTCTAA CTAGGGAAC | 187 |
| 5' DSE modified R region 1GR-GU2.1 | GTCTCTCTGGTTAGACCACACACTTCGTGTGTTTGTGTCTGTTCTA ACTAGGGAAC | 188 |
| 5' DSE modified R region 1GR-GU2.2 | GTCTCTCTGGTTAGACCACACACATTCGTGTGTTTGTGTCTGTTCT AACTAGGGAAC | 189 |
| 5' DSE modified R region 1GR-GU2.3 | GTCTCTCTGGTTAGACCACACACAGTTCGTGTGTTTGTGTCTGTTC TAACTAGGGAAC | 190 |
| 5' DSE modified R region 1GR-GU2.4 | GTCTCTCTGGTTAGACCACACACAATTCGTGTGTTTGTGTCTGTTC TAACTAGGGAAC | 191 |
| 5' DSE modified R region 1GR-GU2.5 | GTCTCTCTGGTTAGACCACACACAGATTCGTGTGTTTGTGTCTGTT CTAACTAGGGAAC | 192 |
| 5' DSE modified R region 1GR-GU2.6 | GTCTCTCTGGTTAGACCACACATTCCAGATTCGTGTGTTTGTGTCT GTTCTAACTAGGGAAC | 193 |
| 5' DSE modified R region 1GR-GU3 | GTCTCTCTGGTTAGACCACACATGGTGTGCACCTGGGTTCTAACTA GGGAAC | 194 |
| 5' DSE modified R region 1GR-GU4 | GTCTCTCTGGTTAGACCACACATTGGTGTGCACCTGTGGTTCTAAC TAGGGAAC | 195 |
| 5' DSE modified R region 1GR-GU5 | GTCTCTCTGGTTAGACCACACATGTCTCGTGTGTTTGTGTCTGTTC GCTAACTAGGGAAC | 196 |
| 5' DSE modified R region 1GR-GU6 | GTCTCTCTGGTTACACCACAATTTGGTGTGCACCTGGGTTTAACTA GGGAAC | 197 |
| 5' DSE modified R region 1GR-GU7 | GTCTCTCTGGTTAGACCGGCGCAGCGTCATTGGTGTGCACCTGGGT TCTAACTAGGGAAC | 198 |
| 5' DSE modified R region 1GR-GU8 | GTCTCTCTGGTTAGACGCACAACACAAAACCGTGTGTTGGTTTTTT GTGTGCTAACTAGGGAAC | 199 |
| DSE1 | TTGGTGTGCACCTGG | 75 |
| DSE2 | GTGTGTTTGTGTCTGTT | 76 |
| DSE3 | TGGTGTGCACCTGGGTT | 77 |
| DSE4 | TTGGTGTGCACCTGTGGTT | 78 |
| DSE5 | GTGTGTTTGTGTCTCTGTTCG | 79 |
| DSE6 | TTTGGTGTGCACCTGGGTTT | 80 |
| DSE7 | TTGGTGTGCACCTGGGTT | 81 |
| DSE8 | GTGTGTTGGTTTTTTGTG | 82 |
| USE | ATTTGTGAAATTTGTGATGCTATTGCTTTATTTGTAA | 83 |

### Illustrative supA-2pA-LTR sequences

Illustrative sequences for use according to the invention are provided in Table 9 below:

### Key for the following sequences:

- (n)20-TRANSG-(n)19 = represents LV genome/transgene sequences between the PBS and the 3'ppt
- bold = the inverted DSE/GU rich sequence servicing the inverted polyA sequence
- bold italics = the inverted GU box
- underlined Y = C/T (denotes optional native 5'pA HIV signal; other mutations/deletions of this are not excluded from presenting this example)

**Table 9**

| **Description** | **Sequence** | **SEQ ID NO.** |
|---|---|---|
| supA-2pA-LTR variant 'GU1': | GGGTCTCTCTGGTTAGACCACACATTCGTGTGTTTGTGTCTG TTCTAACTAGGGAACCCACTGCTTAAGCCTCAAYAAAGCTTG CCTTGAGTGCTTCAAGTAGTGTGTGCCCGTCTGTTGTGTGAC TCTGGTAACTAGAGATCCCTCAGACCCTTTTAGTCAGTGTGG AAAATCTCTAGCAGTGGCGCCCGAACAGGGACnnnnnnnnnn nnnnnnnnnnTRANSGnnnnnnnnnnnnnnnnnAAAAGAA AAGGGGGGACTGGAAGGGCTAATTCACTCCCAACGAAGACAA GATACTAGTTAAGATACATTGATGAGTTTGGACAAACCACAA CTAGAATGCAGTGAAAAAAATGCTTTATTTGTGAAATTTGTG ATGCTATTGCTTTATTTGTAACCATTATAAGCTGCAATAAAG GGTCTCTCTGGTTAGACCACAAGTTAACAACAACAATTGCAT TCATTTTATGTTTCAGGTTCAGGGGGAGGTGTGGGAGGTTTT TTAAA | 200 |
| LV-supA-2pA-LTR expression cassette (5'R to 'R-embedded' heterologous polyA sequence) [see Figure 27 upper panel] | | |
| supA-2pA-LTR variant 'GU1': | ACTGGAAGGGCTAATTCACTCCCAACGAAGACAAGATACTAG TTAAGATACATTGATGAGTTTGGACAAACCACAACTAGAATG CAGTGAAAAAAATGCTTTATTTGTGAAATTTGTGATGCTATT GCTTTATTTGTAACCATTATAAGCTGCAATAAAGGGTCTCTC TGGTTAGACCACACATTCGTGTGTTTGTGTCTGTTCTAACTA GGGAACCCACTGCTTAAGCCTCAAYAAAGCTTGCCTTGAGTG CTTCAAGTAGTGTGTGCCCGTCTGTTGTGTGACTCTGGTAAC TAGAGATCCCTCAGACCCTTTTAGTCAGTGTGGAAAATCTCT AGCAGT | 201 |
| LV-supA-2pA-LTR integrated LTR (from 5' to 3' att sites - two LTRs flank the integrated LV) [see Figure 27 upper panel] | | |
| Sequence for inverted DSE/GU box for supA-2pA-LTR variant 'GU2' | ***AGCGAACAGACACAAACACAC*GAGACATGTGTG** | 202 |
| Sequence for inverted DSE/GU box for supA-2pA-LTR variant 'GU3' | ***ACACAAAAAATTCCAACACAC*** | 203 |
| Sequence for inverted DSE/GU box for supA-2pA-LTR variant 'GU4' | ***GAACAAACGACCCAACACCC*** | 204 |
| Sequence for inverted DSE/GU box for supA-2pA-LTR variant 'GU5' | ***CCAACACACCACACAGA*** | 205 |
| Sequence for inverted DSE/GU box for supA-2pA-LTR variant 'GU6' | ***CCAATGCTTATGAATAACA*CAGGCGA** | 206 |
| Sequence for inverted DSE/GU box for supA-2pA-LTR variant 'GU6' | ***AAAAACCAACACAC*GGTTTTGTGT** | 207 |
| Sequence for inverted DSE/GU box for supA-2pA-LTR variant 'GU1' | **TAAGATACATTGATGAGTTTGG*ACAAACCACAAC*TAGAATG** | 208 |

Any one of SEQ ID NOs: 202 - 207 may replace the bold sequence (including the bold italics sequence) indicated in SEQ ID NO: 200 or SEQ ID NO: 201.

See Figure 53 for graphical summary.

### CARe

The present invention provides novel nucleotide sequences, and viral vectors or cells comprising such nucleotide sequences.

A nucleotide sequence is provided, comprising a transgene expression cassette wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.

Accordingly, in some embodiments of the nucleotide sequence comprising a lentiviral vector genome expression cassette of the invention, the lentiviral vector genome expression cassette comprises a transgene expression cassette wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.

The term "nucleotide sequence" is synonymous with the term "polynucleotide" and/or the term "nucleic acid sequence". The "nucleotide sequence" can be a double stranded or single stranded molecule and includes genomic DNA, cDNA, synthetic DNA, RNA and a chimeric DNA/RNA molecule. Polynucleotides may be produced recombinantly, synthetically or by any means available to those of skill in the art. They may also be cloned by standard techniques.

The nucleotide sequence may comprise a transgene expression cassette. An expression cassette is a distinct component of a vector, comprising a gene (in this case a transgene) and regulatory sequence(s) to be expressed by a transfected, transduced or infected cell. As used herein, "transgene" refers to a segment of DNA or RNA that contains a gene sequence that has been isolated from one organism and is introduced into a different organism, is a non-native segment of DNA or RNA, or is a recombinant sequence that has been made using genetic engineering techniques. The terms "transgene", "transgene construct", "GOI" (gene of interest) and "NOI" (nucleotide of interest) are used interchangeably herein.

The transgene expression cassettes described herein are preferably lentiviral vector transgene expression cassettes. Suitable lentiviral vector transgene expression cassettes are described in more detail elsewhere herein.

The 3' UTR of the transgene expression cassettes described herein may comprise at least one of the novel cis-acting sequences described herein. Cis-acting sequences affect the expression of genes that are encoded in the same nucleotide sequence (i.e. the one in which the cis-acting sequence is also present). In the context of viral vectors, cis-acting sequences include the typical post-transcriptional regulatory elements (PREs) such as that from the woodchuck hepatitis virus (wPRE). General examples of cis-acting sequences are provided elsewhere herein. The terms "cis-acting element" and "cis-acting sequence" are used interchangeably herein.

In one embodiment, the 3' UTR of the transgene expression cassette described herein comprises at least one cis-acting sequence selected from (a) a cis-acting Cytoplasmic Accumulation Region (CAR) sequence; and/or (b) a cis-acting ZCCHC14 protein-binding sequence.

As used herein, a "Cytoplasmic Accumulation Region (CAR) sequence" is a nucleotide sequence that is transcribed into mRNA and increases the stability and/or export of the mRNA to the cytoplasm and accumulation of the mRNA in the cytoplasm of a cell by sequence-dependent recruitment of the mRNA export machinery. CAR sequences have been described previously, see for example Lei et al., 2013, which describes that insertion of a CAR sequence upstream (i.e. at the 5' end) of a naturally intronless gene can promote the cytoplasmic accumulation of the mRNA transcript.

The inventors have surprisingly found that insertion of a CAR sequence into the 3' UTR of a transgene expression cassette enhances gene expression. Surprisingly, these CAR sequences are shown to enhance the transgene expression from transgene cassettes utilizing introns as well as from transgene cassettes that are intronless, as well as boosting expression from cassettes already containing a full length wPRE.

Suitable CAR sequences for insertion into the 3' UTR of a transgene expression cassette described herein may be readily identifiable by a person of skill in the art, based on the disclosure provided herein, together with their common general knowledge (see e.g. the disclosure in Lei et al., 2013, which is incorporated herein in its entirety).

The CAR sequences described herein comprise at least one CAR element (CARe) sequence. As would be understood by a person of skill in the art, a CARe sequence is a core sequence that is present within a CAR sequence (and typically, wherein the CARe sequence is repeated a number of times within the CAR sequence). Examples of CARe sequences are shown in Figure 26 and are described in Lei et al., 2013.

For example, the CARe sequence may be a sequence that is represented by BMWGHWSSWS (SEQ ID NO: 92) or BMWRHWSSWS (SEQ ID NO: 243), wherein:

**Table 3: nucleotide symbols**

| **Nucleotide symbol** | **Full name** |
|---|---|
| A | Adenine |
| C | Cytosine |
| G | Guanine |
| T | Thymine |
| U | Uracil |
| R | Guanine / Adenine (purine) |
| Y | Cytosine / Thymine (pyrimidine) |
| K | Guanine / Thymine |
| M | Adenine / Cytosine |
| S | Guanine / Cytosine |
| W | Adenine / Thymine |
| B | Guanine / Thymine / Cytosine |
| D | Guanine / Adenine / Thymine |
| H | Adenine / Cytosine / Thymine |
| V | Guanine / Cytosine / Adenine |
| N | Adenine / Guanine / Cytosine / Thymine |

Exemplary CARe sequences that are encompassed by BMWGHWSSWS (SEQ ID NO: 92) or BMWRHWSSWS (SEQ ID NO: 243), include those with a sequence represented by CMAGHWSSTG (using the nomenclature of Table 3; SEQ ID NO: 96). Such CARe sequences include the CARe sequences identified previously in Lei et al., 2013 (where the CARe sequences were identified in the 5' region of HSPB3, c-Jun, IFNα1 and IFNβ1 genes).

The CARe sequence may be selected from the group consisting of: CCAGTTCCTG (SEQ ID NO: 97), CCAGATCCTG (SEQ ID NO: 98), CCAGTTCCTC (SEQ ID NO: 99), TCAGATCCTG (SEQ ID NO: 100), CCAGATGGTG (SEQ ID NO: 101), CCAGTTCCAG (SEQ ID NO: 102), CCAGCAGCTG (SEQ ID NO: 103), CAAGCTCCTG (SEQ ID NO: 104), CAAGATCCTG (SEQ ID NO: 105), CCTGAACCTG (SEQ ID NO: 106), CAAGAACGTG (SEQ ID NO: 107), TCAGTTCCTG (SEQ ID NO: 227), GCAGTTCCTG (SEQ ID NO: 228), CAAGTTCCTG (SEQ ID NO: 229), CCTGTTCCTG (SEQ ID NO: 230), CCTGCTCCTG (SEQ ID NO: 231), CCTGTACCTG (SEQ ID NO:232), CCTGTTGCTG (SEQ ID NO: 233), CCTGTTCGTG (SEQ ID NO: 234), CCTGTTCCAG (SEQ ID NO: 235), CCTGTTCCTC (SEQ ID NO: 236), CCAATTCCTG (SEQ ID NO: 237) and GAAGCTCCTG (SEQ ID NO: 238).

In a particular example, the CARe nucleotide sequence is selected from the group consisting of: CCAGTTCCTG (SEQ ID NO: 97), CCTGTTCCTG (SEQ ID NO: 230), CCTGTACCTG (SEQ ID NO: 232), CCTGTTCCAG (SEQ ID NO: 235), CCAATTCCTG (SEQ ID NO: 237), CCTGAACCTG (SEQ ID NO: 106), CCAGTTCCTC (SEQ ID NO: 99) and CCAGTTCCAG (SEQ ID NO: 102).

Suitably, the CARe nucleotide sequence may be CCAGTTCCTG (SEQ ID NO: 97). This sequence is also referred to as a "consensus" tile herein. It is the CARe sequence that is used to exemplify the invention in examples 10 to 13 below.

In a particular example, the CARe sequence may be CCAGTTCCTG (SEQ ID NO: 97). This is the sequence that is used to exemplify the invention in examples 10 to 13 below.

In one example, the CARe sequence may be CCAGATCCTG (SEQ ID NO: 98). This is the consensus sequence identified in Figure 26A.

In one example, the CARe sequence may be CCAGTTCCTC (SEQ ID NO: 99). This sequence is also referred to as HSPB3 v2 herein (see e.g. Figure 67).

For example, the CARe sequence may be TCAGATCCTG (SEQ ID NO: 100).

In one example, the CARe sequence may be CCAGATGGTG (SEQ ID NO: 101). This sequence is also referred to as HSPB3 v3 herein (see e.g. Figure 67).

In one example, the CARe sequence may be CCAGTTCCAG (SEQ ID NO: 102). This sequence is also referred to as IFNa1 v1 herein (see e.g. Figure 67).

For example, the CARe sequence may be CCAGCAGCTG (SEQ ID NO: 103).

In one example, the CARe sequence may be CAAGCTCCTG (SEQ ID NO: 104).

In one example, the CARe sequence may be CAAGATCCTG (SEQ ID NO: 105).

For example, the CARe sequence may be CCTGAACCTG (SEQ ID NO: 106). This sequence is also referred to as c-Jun v2 herein (see e.g. Figure 67).

In one example, the CARe sequence may be CAAGAACGTG (SEQ ID NO: 107). This sequence is also referred to as c-Jun v4 herein (see e.g. Figure 67).

In one example, the CARe sequence may be TCAGTTCCTG (SEQ ID NO: 227). This sequence is also referred to as variant 1 in Figure 66.

In one example, the CARe sequence may be GCAGTTCCTG (SEQ ID NO: 228). This sequence is also referred to as variant 2 in Figure 66.

In one example, the CARe sequence may be CAAGTTCCTG (SEQ ID NO: 229). This sequence is also referred to as variant 3 in Figure 66.

In one example, the CARe sequence may be CCTGTTCCTG (SEQ ID NO: 230). This sequence is also referred to as variant 4 in Figure 66.

In one example, the CARe sequence may be CCTGCTCCTG (SEQ ID NO: 231). This sequence is also referred to as variant 6 in Figure 66.

In one example, the CARe sequence may be CCTGTACCTG (SEQ ID NO: 232). This sequence is also referred to as variant 7 in Figure 66.

In one example, the CARe sequence may be CCTGTTGCTG (SEQ ID NO: 233). This sequence is also referred to as variant 8 in Figure 66.

In one example, the CARe sequence may be CCTGTTCGTG (SEQ ID NO: 234). This sequence is also referred to as variant 9 in Figure 66.

In one example, the CARe sequence may be CCTGTTCCAG (SEQ ID NO: 235). This sequence is also referred to as variant 10 in Figure 66.

In one example, the CARe sequence may be CCTGTTCCTC (SEQ ID NO: 236). This sequence is also referred to as variant 11 in Figure 66.

In one example, the CARe sequence may be CCAATTCCTG (SEQ ID NO: 237). This sequence is also referred to as variant 12 in Figure 66.

In one example, the CARe sequence may be GAAGCTCCTG (SEQ ID NO: 238). This sequence is also referred to as IFNb1 v1 in Figure 67.

In other words, a transgene expression cassette described herein (typically a lentiviral vector transgene expression cassette) may comprise a cis-acting Cytoplasmic Accumulation Region (CAR) sequence, comprising at least one of the CAR element (CARe) sequences described herein.

CAR sequences typically comprise a plurality of CARe sequences. For example, the CAR sequences described herein may include a plurality of CARe sequences, e.g. at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen, or at least twenty CARe sequences.

In one example, the CAR sequence described herein comprises at least two CARe sequences.

In one example, the CAR sequence described herein comprises at least four CARe sequences.

In one example, the CAR sequence described herein comprises at least six CARe sequences.

In one example, the CAR sequence described herein comprises at least eight CARe sequences. In this context particularly, the transgene expression cassette may also comprise at least one cis-acting ZCCHC14 protein-binding sequence provided herein.

In one example, the CAR sequence described herein comprises at least ten CARe sequences.

In one example, the CAR sequence described herein comprises at least twelve CARe sequences.

In one example, the CAR sequence described herein comprises at least fourteen CARe sequences.

In one example, the CAR sequence described herein comprises at least sixteen CARe sequences. In this context particularly, the transgene expression cassette may also comprise at least one cis-acting ZCCHC14 protein-binding sequence provided herein.

In one example, the CAR sequence described herein comprises at least eighteen CARe sequences.

In one example, the CAR sequence described herein comprises at least twenty CARe sequences.

There may be a desire to use a CAR sequence that is as short as possible. Accordingly, in one example, the CAR sequences described herein may include a plurality of CARe sequences, e.g. no more than two, no more than three, no more than four, no more than five, no more than six, no more than seven, no more than eight, no more than nine, no more than ten, no more than eleven, no more than twelve, no more than thirteen, no more than fourteen, no more than fifteen, no more than sixteen, no more than seventeen, no more than eighteen, no more than nineteen, or no more than twenty CARe sequences.

In one example, the CAR sequence described herein has no more than two CARe sequences.

In one example, the CAR sequence described herein has no more than four CARe sequences. In this context particularly, the transgene expression cassette may also comprise at least one cis-acting ZCCHC14 protein-binding sequence provided herein.

In one example, the CAR sequence described herein has no more than six CARe sequences.

In one example, the CAR sequence described herein has no more than eight CARe sequences. In this context particularly, the transgene expression cassette may also comprise at least one cis-acting ZCCHC14 protein-binding sequence provided herein.

In one example, the CAR sequence described herein has no more than ten CARe sequences.

In one example, the CAR sequence described herein has no more than twelve CARe sequences.

In one example, the CAR sequence described herein has no more than fourteen CARe sequences.

In one example, the CAR sequence described herein has no more than sixteen CARe sequences. In this context particularly, the transgene expression cassette may also comprise at least one cis-acting ZCCHC14 protein-binding sequence provided herein.

In one example, the CAR sequence described herein has no more than eighteen CARe sequences.

In one example, the CAR sequence described herein has no more than twenty CARe sequences.

In one example, the CAR sequence described herein has at least four, but no more than twenty, CARe sequences.

In one example, the CAR sequence described herein has at least eight, but no more than twenty, CARe sequences.

In one example, the CAR sequence described herein has at least twelve, but no more than twenty, CARe sequences.

In one example, the CAR sequence described herein has at least sixteen, but no more than twenty, CARe sequences.

In one example, the CAR sequence described herein has at least four, but no more than sixteen, CARe sequences.

In one example, the CAR sequence described herein has at least eight, but no more than sixteen, CARe sequences.

In one example, the CAR sequence described herein has at least twelve, but no more than sixteen, CARe sequences.

Suitably, a CAR sequence described herein may consist of two CARe sequences, or consist of four CARe sequences, or consist of six CARe sequences, or consist of eight CARe sequences, or consist of ten CARe sequences, or consist of twelve CARe sequences, or consist of fourteen CARe sequences, or consist of sixteen CARe sequences, or consist of eighteen CARe sequences, or consist of twenty CARe sequences.

The inventors have identified herein that inserting a CAR sequence comprising sixteen CARe sequences into the 3' UTR of a transgene expression cassette (typically lentiviral vector transgene expression cassettes herein) enhances transgene expression. A CAR sequence comprising at least sixteen CARe sequences (e.g. with at least one (or all) CARe sequence(s) being CCAGTTCCTG (SEQ ID NO: 97)) is therefore particularly contemplated herein.

A CAR sequence having no more than sixteen CARe sequences (e.g. with at least one (or all) CARe sequence(s) being CCAGTTCCTG (SEQ ID NO: 97)) is therefore also particularly contemplated herein.

From the foregoing, it will be appreciated that a CAR sequence having a total of sixteen CARe sequences (e.g. with at least one (or all) of the CARe sequence(s) being CCAGTTCCTG (SEQ ID NO: 97)) is contemplated herein.

An illustrative example of a CAR sequence having a total of sixteen CARe sequences, wherein each CARe sequence is a repeat of CCAGTTCCTG (SEQ ID NO: 97) inverted is as follows:

CAGGAACTGGCAGGAACTGGCAGGAACTGGCAGGAACTGGCAGGAACTGGCAGGAACTGGCAGGAACT
GGCAGGAACTGGCAGGAACTGGCAGGAACTGGCAGGAACTGGCAGGAACTGGCAGGAACTGGCAGGAA
CTGGCAGGAACTGGCAGGAACTGG (SEQ ID NO: 112; Inverted [CAR-E]16)

In one embodiment, the CARe for use according to the invention has a sequence as set forth in SEQ ID NO: 112.

In addition, the inventors have shown that enhanced expression may also be achieved with less than sixteen CARe sequences (e.g. with one or more CARe sequences - see Figure 32). This is advantageous as it provides greater transgene capacity in the expression cassette.

A CAR sequence comprising at least eight CARe sequences (e.g. with at least one (or all) CARe sequence(s) being CCAGTTCCTG (SEQ ID NO: 97)) is particularly contemplated herein.

Accordingly, a CAR sequence having a total of eight CARe sequences (e.g. with at least one (or all) of the CARe sequence(s) being CCAGTTCCTG (SEQ ID NO: 97)) is contemplated herein.

A CAR sequence having no more than eight CARe sequences (e.g. with at least one (or all) CARe sequence(s) being CCAGTTCCTG (SEQ ID NO: 97)) is therefore also particularly contemplated herein.

Furthermore, in the context of inserting CARe sequences at the 5' end of intronless mRNA, CAR sequence comprising six or ten CARe sequences have previously been shown to be functional. Accordingly, in the context of the invention, wherein the CAR sequence is inserted in the 3'UTR of a transgene expression cassette, a CAR sequence comprising at least six CARe sequences (e.g. with at least one (or all) CARe sequence(s) being CCAGTTCCTG (SEQ ID NO: 97)) is therefore particularly contemplated herein.

Similarly, in the context of the invention, wherein the CAR sequence is inserted in the 3'UTR of a transgene expression cassette, a CAR sequence comprising at least ten CARe sequences (e.g. with at least one (or all) CARe sequence(s) being CCAGTTCCTG (SEQ ID NO: 97)) is therefore also particularly contemplated herein.

A CAR sequence having no more than six CARe sequences (e.g. with at least one (or all) CARe sequence(s) being CCAGTTCCTG (SEQ ID NO: 97)) is therefore particularly contemplated herein. Such sequences are particularly contemplated in the case that the CAR sequence is inserted in the 3'UTR of a transgene expression cassette.

A CAR sequence having no more than ten CARe sequences (e.g. with at least one (or all) CARe sequence(s) being CCAGTTCCTG (SEQ ID NO: 97)) is therefore also particularly contemplated herein. As above, such sequences are particularly contemplated in the case that the CAR sequence is inserted in the 3'UTR of a transgene expression cassette.

The CAR sequence may comprise a plurality of CARe sequences that are the same (i.e. the CAR sequence may comprise a number of repeats of the same CARe sequence). For example, the CAR sequence may comprise at least two, at least four, at least six, at least eight, at least ten, at least twelve, at least fourteen, at least sixteen, at least eighteen, or at least twenty of the same CARe sequence.

In one example, the CAR sequence may have no more than two, no more than four, no more than six, no more than eight, no more than ten, no more than twelve, no more than fourteen, no more than sixteen, no more than eighteen, or no more than twenty of the same CARe sequence.

Alternatively, the CAR sequence may comprise at least two, at least four, at least six, at least eight, at least ten, at least twelve, at least fourteen, at least sixteen, at least eighteen, or at least twenty CARe sequences, wherein at least two of the CARe sequences are different.

In one example, the CAR sequence may have no more than two, no more than four, no more than six, no more than eight, no more than ten, no more than twelve, no more than fourteen, no more than sixteen, no more than eighteen, or no more than twenty CARe sequences, wherein at least two of the CARe sequences are different.

Indeed, in each of the embodiments described herein where a CAR sequence comprises a plurality of CARe sequences, the CARe sequences each may be selected independently from the group consisting of: SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107 SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 233, SEQ ID NO: 234, SEQ ID NO: 235, SEQ ID NO: 236, SEQ ID NO: 237, SEQ ID NO: 238. In a specific example, where a CAR sequence comprises a plurality of CARe sequences, the CARe sequences each may be selected independently from the group consisting of: SEQ ID NO: 97, SEQ ID NO: 230, SEQ ID NO: 232, SEQ ID NO: 235, SEQ ID NO: 237, SEQ ID NO: 106, SEQ ID NO: 99, and SEQ ID NO: 102.

Appropriate combinations of CARe sequences may be identified by a person of skill in the art.

The plurality of CARe sequences within the CAR sequence may be in tandem (i.e. they may be referred to as "tandem CARe sequences").

For example, the at least two, at least four, at least six, at least eight, at least ten, at least twelve, at least fourteen, at least sixteen, at least eighteen, or at least twenty CARe sequences within the CAR sequence may be in tandem.

Suitably, the CAR sequence may comprise at least six CARe sequences in tandem, or at least ten CARe sequences in tandem.

Suitably, the CAR sequence may comprise at least eight CARe sequences in tandem, at least twelve CARe sequences in tandem, or at least sixteen CARe sequences in tandem.

In one example, the no more than two, no more than four, no more than six, no more than eight, no more than ten, no more than twelve, no more than fourteen, no more than sixteen, no more than eighteen, or no more than twenty CARe sequences within the CAR sequence may be in tandem.

Tandem CARe sequences are located directly adjacent to each other in the nucleotide sequence. By way of an example, if the CAR sequence comprises two CARe sequences (each having the sequence CCAGTTCCTG (SEQ ID NO: 97)) in tandem, it would comprise the sequence CCAGTTCCTGCCAGTTCCTG (SEQ ID NO: 116). Similarly, if the CAR sequence comprises three CARe sequences (each having the sequence CCAGTTCCTG (SEQ ID NO: 97)) in tandem, it would comprise the sequence CCAGTTCCTGCCAGTTCCTGCCAGTTCCTG (SEQ ID NO: 117) etc.

In embodiments in which the CAR sequence comprises tandem CARe sequences, the tandem sequences may each have the same CARe sequence.

The CAR sequence may be described by its size. For example, where a CAR sequence comprises a plurality of CARe sequences in tandem, its size will be reflective of the number of CARe sequences that are present. For example, using the CARe sequences specifically recited herein (which all have a sequence of 10 nucleotides) the CAR sequence may be at least 20 nucleotides when it comprises two CARe sequences in tandem, at least 30 nucleotides when it comprises three CARe sequences in tandem, at least 40 nucleotides when it comprises four CARe sequences in tandem etc.

Accordingly, the CAR sequence may be at least 10 nucleotides, at least 20 nucleotides, at least 30 nucleotides, at least 40 nucleotides, at least 50 nucleotides etc.

It may be at least 60 nucleotides (e.g. with at least six CARe sequences in tandem).

It may be at least 80 nucleotides (e.g. with at least eight CARe sequences in tandem).

It may be at least 100 nucleotides (e.g. with at least ten CARe sequences in tandem).

It may be at least 160 nucleotides (e.g. with at least sixteen CARe sequences in tandem).

The CAR sequence may be no more than 10 nucleotides, no more than 20 nucleotides, no more than 30 nucleotides, no more than 40 nucleotides, no more than 50 nucleotides etc.

It may be no more than 60 nucleotides (e.g. with up to six CARe sequences in tandem).

It may be no more than 80 nucleotides (e.g. with up to eight CARe sequences in tandem).

It may be no more than 100 nucleotides (e.g. with up to ten CARe sequences in tandem).

It may be no more than 160 nucleotides (e.g. with up to sixteen CARe sequences in tandem).

It may be no more than 200 nucleotides (e.g. with up to twenty CARe sequences in tandem).

It may be no more than 240 nucleotides (e.g. with up to twenty four CARe sequences in tandem).

It may be no more than 290 nucleotides (e.g. with up to twenty nine CARe sequences in tandem).

It may be no more than 300 nucleotides (e.g. with up to thirty CARe sequences in tandem).

It may be no more than 350 nucleotides (e.g. with up to thirty five CARe sequences in tandem).

It may be no more than 400 nucleotides (e.g. with up to forty CARe sequences in tandem).

It may be no more than 410 nucleotides (e.g. with up to forty one CARe sequences in tandem).

Alternatively, the plurality of CARe sequences within the CAR sequence may be spatially separated by intervening sequences (i.e. one or more nucleotides may be present between neighbouring CARe sequences within the CAR sequence). In one example, the intervening sequence between neighbouring CARe sequences within the CAR sequence is no more than twenty nucleotides e.g. there are no more than two, no more than three, no more than four, no more than five, no more than six, no more than seven, no more than eight, no more than nine, no more than ten, no more than eleven, no more than twelve, no more than thirteen, no more than fourteen, no more than fifteen, no more than sixteen, no more than seventeen, no more than eighteen, no more than nineteen, or no more than twenty intervening nucleotides between neighbouring CARe sequences within the CAR sequence.

In one example, the intervening sequence between neighbouring CARe sequences within the CAR sequence is no more than two nucleotides.

In one example, the intervening sequence between neighbouring CARe sequences within the CAR sequence is no more than four nucleotides.

In one example, the intervening sequence between neighbouring CARe sequences within the CAR sequence is no more than six nucleotides.

In one example, the intervening sequence between neighbouring CARe sequences within the CAR sequence is no more than eight nucleotides.

In one example, the intervening sequence between neighbouring CARe sequences within the CAR sequence is no more than ten nucleotides.

In one example, the intervening sequence between neighbouring CARe sequences within the CAR sequence is no more than twelve nucleotides.

In one example, the intervening sequence between neighbouring CARe sequences within the CAR sequence is no more than fourteen nucleotides.

In one example, the intervening sequence between neighbouring CARe sequences within the CAR sequence is no more than sixteen nucleotides.

In one example, the intervening sequence between neighbouring CARe sequences within the CAR sequence is no more than eighteen nucleotides.

In one example, the intervening sequence between neighbouring CARe sequences within the CAR sequence is no more than twenty nucleotides.

The transgene expression cassette may comprise at least one a cis-acting ZCCHC14 protein-binding sequence (as an alternative to the CARe sequence(s) described herein, or in addition to the CARe sequence(s) described herein).

As used herein, a ZCCHC14 protein-binding sequence is a nucleotide sequence that is capable of interacting with/being bound by a ZCCHC14 protein. "ZCCHC14" refers to human Zinc finger CCHC domain-containing protein 14 (also referred to as BDG-29), with UniProtKB identifier: Q8WYQ9; and NCBI Gene ID: 23174, updated on 4-Jul-2021). Recruitment of ZCCHC14 to the 3' region of the transgene mRNA results in a complex of ZCCHC14-Tent4, which enables mixed tailing within polyA tails of the polyadenylated mRNA. Mixed tailing increases the stability of the transgene mRNA in target cells.

Methods for determining whether a specific nucleotide sequence is capable of being bound by a ZCCHC14 protein are well known in the art; see for example the method of 'Systematic evolution of ligands by exponential enrichment' (SELEX) or the method of RNA electrophoretic mobility shift assay or the method RNA pull-down (cross-linking/immunoprecipitation) or a combination of these methods (Mol Biol (Mosk). May-Jun 2015;49(3):472-81). Routine methods for detecting nucleotide: protein interactions may also be used e.g. nucleotide pull down assays, ELISA assays, reporter assays etc. Appropriate ZCCHC14 protein-binding sequences can therefore readily be identified by a person of skill in the art.

The cis-acting ZCCHC14 protein-binding sequences described herein comprise at least one CNGGN-type pentaloop sequence. Several ZCCHC14 protein-binding sequences comprising a CNGGN-type pentaloop sequence are known in the art. For example, the PRE of HBV and HCMV RNA 2.7, as well as wPRE, are known to comprise a ZCCHC14 protein-binding sequence with a CNGGN-type pentaloop sequence. The pentaloop adopts the GNGG(N) family loop conformation with a single bulged G residue, flanked by A-helical regions (see for example Kim et al., 2020), where N can be any nucleotide.

The cis-acting ZCCHC14 protein-binding sequences described herein may comprise any appropriate CNGGN-type pentaloop sequence.

For example, they may comprise a CTGGT pentaloop sequence (as is seen in the stem loop found HCMV RNA2.7; also known as the SLα of HCMV RNA2.7).

Alternatively, they may comprise a CTGGA pentaloop sequence (as is seen in the stem loop found in the α element of wPRE; also known as the SLα of wPRE).

Further alternatively, they may comprise a CAGGT pentaloop sequence (as is seen in the stem loop found in the PRE α element of HBV; also known as the SLα of HBV).

The CNGGN-type pentaloop sequences found within the SLα of HCMV RNA 2.7, wPRE and HBV are typically part of a stem-loop structure, which facilitates TENT4-dependent tail regulation.

Accordingly, the cis-acting ZCCHC14 protein-binding sequences described herein may also comprise the CNGGN-type pentaloop sequence as part of a stem loop sequence. As would be clear to a person of skill in the art, any appropriate stem loop sequence may be used. Suitable stem loop sequences can readily be identified by a person of skill in the art, as the required level of complementarity needed for a stem loop sequence is known.

Differing stem lengths may also be used. In the examples herein, stems of 7 or 8 nucleotides have been used, however, longer stems with up to an additional 9 nucleotides (18 nt added in total, 9 on each side) have also been shown to work (data not shown).

Non-limiting examples of stem loop sequences are provided below.

For example, a cis-acting ZCCHC14 protein-binding sequence described herein may comprise the stem loop sequence TCCTCGTAGGCTGGTCCTGGGGA (SEQ ID NO: 108; which includes the pentaloop sequence CTGGT, and corresponds to the sequence of SLα of HCMV RNA2.7).

Alternatively, a cis-acting ZCCHC14 protein-binding sequence described herein may comprise the stem loop sequence GCCCGCTGCTGGACAGGGGC (SEQ ID NO: 109; which includes the pentaloop sequence CTGGA, and corresponds to the sequence of SLα of wPRE).

Further alternatively, they may comprise the stem loop sequence TTGCTCGCAGCAGGTCTGGAGCAA (SEQ ID NO: 118; which includes the pentaloop sequence CAGGT, and corresponds to the sequence of SLα of HBV).

Alternatively, the cis-acting ZCCHC14 protein-binding sequences described herein may comprise the CNGGN-type pentaloop sequence as part of a heterologous stem loop sequence. Appropriate heterologous stem loop sequences may readily be identified by a person of skill in the art.

The cis-acting ZCCHC14 protein-binding sequences described herein may comprise the CNGGN-type pentaloop sequence as part of a stem loop sequence, within a longer sequence (i.e. wherein the cis-acting ZCCHC14 protein-binding sequence comprises additional sequences that flank the stem loop sequence itself).

Examples of flanking sequences are given in SEQ ID NO: 110 (which shows the sequence of a stem-loop structure as set forth in SEQ ID NO: 108, with additional flanking sequences) and SEQ ID NO: 111 (which shows the sequence of a stem-loop structure as set forth in SEQ ID NO: 109, with additional flanking sequences):
TGCCGTCGCCACCGCGTTATCCGT**TCCTCGTAGGCTGGTCCTGGGGA**ACGGGTCGGCGGCCGGTCGGC TTCT (SEQ ID NO: 110: ZCCHC14 stem loop (from the post-transcriptional regulatory element (PRE) of the HCMV RNA2.7) (flanking sequence underlined; ZCCHC14 interacting loop in bold)
CTATTGCCACGGCGGAACTCATCGCCGCCTGCCTT**GCCCGCTGCTGGACAGGGGC**TCGGCTGTTGGGC ACTGACAATTCCGTGGTGTTGT (SEQ ID NO: 111 - ZCCHC14 stem loop (from PRE of the woodchuck hepatitis virus (WPRE) - (flanking sequence underlined; ZCCHC14 interacting loop in bold)

Further examples of flanking sequences are given in SEQ ID NO: 142 (which shows the sequence of a stem-loop structure as set forth in SEQ ID NO: 118, with additional flanking sequences):

CGATCCATACTGCGGAACTCCTAGCCGCTTGTTTTGCTCGCAGCAGGTCTGGAGCAAACCTCATCGGG
ACCGACAATTCTGTCGTACTCT (HBV stem loop + flanking sequences: where
the stem loop is underlined) (SEQ ID NO: 142)

The flanking sequences may be nucleotides that are naturally present at these positions in the corresponding PRE (e.g. for SEQ ID NO: 111, the flanking sequences are those that are naturally present around the SLα sequence of wPRE). Alternatively, heterologous flanking sequences may be used. The flanking sequences provided herein are merely by way of example and alternative flanking sequences and flanking sequences with different lengths may also be used.

In one example, a ZCCHC14 protein-binding sequence as described herein may comprise at least one CNGGN-type pentaloop sequence and a stem loop sequence, but does not comprise a flanking sequence.

In one example, a ZCCHC14 protein-binding sequence as described herein comprises at least one CNGGN-type pentaloop sequence, but does not comprise a stem loop sequence or a flanking sequence.

The cis-acting ZCCHC14 protein-binding sequences described herein do not comprise a full length post-transcriptional regulatory element (PRE) α element. In addition, they do not comprise a full length PRE γ element (for example that found in wPRE or wPRE3). As such, the cis-acting ZCCHC14 protein-binding sequences described herein have neither a full length post-transcriptional regulatory element (PRE) α element nor a full length PRE γ element. The reason for this is that the invention aims to minimise the size of the cis-acting sequences in the 3' UTR of the transgene expression cassette as much as possible, to provide more transgene capacity. The inventors have surprisingly found that the full length sequence of a PRE α element and the full length sequence of a PRE γ element are not needed in order to obtain the effects observed herein.

The ZCCHC14 protein-binding sequence described herein therefore is not wPRE or wPRE3.

Full length PRE α element, β element and γ element sequences are readily identifiable by a person of skill in the art. By way of example, full length PRE α element, β element and γ element sequences for wPRE are provided herein as SEQ ID NO: 114, SEQ ID NO: 115 and SEQ ID NO: 113, respectively:

AATCAACCTCTGGATTACAAAATTTGTGAAAGATTGACTGGTATTCTTAACTATGTTGCTCCTTTTAC
GCTATGTGGATACGCTGCTTTAATGCCTTTGTATCATGCTATTGCTTCCCGTATGGCTTTCATTTTCT
CCTCCTTGTATAAATCCTGGTT (SEQ ID NO: 113; full length sequence of wPRE
γ element)

GCTGTCTCTTTATGAGGAGTTGTGGCCCGTTGTCAGGCAACGTGGCGTGGTGTGCACTGTGTTTGCTG
ACGCAACCCCCACTGGTTGGGGCATTGCCACCACCTGTCAGCTCCTTTCCGGGACTTTCGCTTTCCCC
CTCCCTATTGCCACGGCGGAACTCATCGCCGCCTGCCTTGCCCGCTGCTGGACAGGGGCTCGGCTGTT
GGGCACTGACAATTCCGTGGTGTTGTCGGGGAAGCTGACGTCCTTTCCATGGC (SEQ ID NO:
114; full length sequence for wPRE α element)

TGCTCGCCTGTGTTGCCACCTGGATTCTGCGCGGGACGTCCTTCTGCTACGTCCCTTCGGCCCTCAAT
CCAGCGGACCTTCCTTCCCGCGGCCTGCTGCCGGCTCTGCGGCCTCTTCCGCGTCTTCGCCTTCGCCC
TCAGACGAGTCGGATCTCCCTTTGGGCCGCCTCCCCGCCTG (SEQ ID NO: 115; full
length sequence for wPRE β element)

Although a PRE α element as such has not been identified within HCMV RNA 2.7, a "minimal element" having equivalent function has been found:

ACCGCGTTATCCGTTCCTCGTAGGCTGGTCCTGGGGAACGGGTCGGCGGCCGGTCGGCTTCTGTTTTA
TTTTTTTTTTTATTTTTTATCTTCTCCTTTC (SEQ ID NO: 141) (where the stem
loop is underlined) (HCMV-RNA2.7 PRE α "like" element (fragment 1E
in Kim et al., 2020).

Furthermore, a full length PRE α element sequence for HBV is also provided as SEQ ID NO: 95 (HBV does not comprise a γ element):

CTCGGCAACGGCCTGGTCTGTGCCAAGTGTTTGCTGACGCAACCCCCACTGGTTGGGGCTTGGCCATA
GGCCATCAGCGCATGCGTGGAACCTTTGTGTCTCCTCTGCCGATCCATACTGCGGAACTCCTAGCCGC
TTGTTTTGCTCGCAGCAGGTCTGGAGCAAACCTCATCGGGACCGACAATTCTGTCGTACTCT (SEQ
ID NO: 95)

The cis-acting ZCCHC14 protein-binding sequences described herein therefore do not comprise any of the following sequences: SEQ ID NO: 114, SEQ ID NO: 113, SEQ ID NO: 95 or SEQ ID NO: 141.

In one example, a cis-acting ZCCHC14 protein-binding sequence provided herein may have a sequence that corresponds to a PRE α element fragment (in other words, it may have a sequence that is identical to a portion of a PRE α element, but does not include all of (i.e. is shorter than) the full length PRE α element sequence). In other words, the cis-acting ZCCHC14 protein-binding sequence provided herein may be a truncated nucleotide sequence that constitutes a part of a PRE α element.

For example, the cis-acting ZCCHC14 protein-binding sequence may be a fragment (a portion of) a HBV PRE α element. In this context, it may be described as a HBV PRE α element fragment. It may also be described as a truncated nucleotide sequence that constitutes a part of a HBV PRE α element. In this context, the cis-acting ZCCHC14 protein-binding sequence may be a fragment (a portion of) SEQ ID NO: 95. In other words, it may be a truncated nucleotide sequence that constitutes a part of SEQ ID NO: 95. In this example, the ZCCHC14 protein-binding sequence may comprise the sequence of SEQ ID NO: 118, or SEQ ID NO: 142.

For example, the cis-acting ZCCHC14 protein-binding sequence may be a fragment (a portion of) a HCMV RNA 2.7 sequence. For example, it may be a fragment (a portion of) the sequence shown in SEQ ID NO: 141. It may be described as a HCMV RNA 2.7 fragment (for example, a fragment of the sequence shown in SEQ ID NO: 141). It may also be described as a truncated nucleotide sequence that constitutes a part of a HCMV RNA 2.7 sequence. In this example, the ZCCHC14 protein-binding sequence may comprise the sequence of SEQ ID NO: 108 or 110. For example, the ZCCHC14 protein-binding sequence may be a fragment of the sequence shown in SEQ ID NO: 141 and comprise the sequence of SEQ ID NO: 108 or 110.

For example, the cis-acting ZCCHC14 protein-binding sequence may be a fragment (a portion of) a wPRE PRE α element. In this context, it may be described as a wPRE α element fragment. It may also be described as a truncated nucleotide sequence that constitutes a part of a wPRE α element. In this context, the cis-acting ZCCHC14 protein-binding sequence may be a fragment (a portion of) SEQ ID NO: 114. In other words, it may be a truncated nucleotide sequence that constitutes a part of SEQ ID NO: 114. In this example, the ZCCHC14 protein-binding sequence may comprise the sequence of SEQ ID NO: 109 or 111.

The inventors have exemplified the invention by using ZCCHC14 protein-binding sequences that are derived from known PREs (specifically, the ZCCHC14 protein-binding sequence that is present in the HCMV RNA2.7; and/or the ZCCHC14 protein-binding sequence that is present in the PRE of the woodchuck hepatitis virus (wPRE)). Although these ZCCHC14 protein-binding sequences are particularly contemplated herein, other appropriate ZCCHC14 protein-binding sequences (e.g. from other PREs) may alternatively (or additionally) be used.

The ZCCHC14 protein-binding sequences that are described herein are used to enhance transgene expression, whilst minimising the 'backbone' sequences of viral vectors such that titres of vectors containing larger payloads can be maintained or increased. The ZCCHC14 protein-binding sequence is therefore typically small.

For example, the ZCCHC14 protein-binding sequence that is inserted into the 3' UTR of the transgene expression cassette described herein may be up to 240 nucleotides. In this example, the ZCCHC14 protein-binding sequence may comprise the sequence of SEQ ID NO: 108, 109, 110, 111, 118 or 142.

In one example, the ZCCHC14 protein-binding sequence that is inserted into the 3' UTR of the transgene expression cassette described herein may be up to 200 nucleotides (i.e. no more than 200 nucleotides in length). In this example, the ZCCHC14 protein-binding sequence may comprise the sequence of SEQ ID NO: 108, 109, 110, 111, 118 or 142.

In a further example, the ZCCHC14 protein-binding sequence that is inserted into the 3' UTR of the transgene expression cassette described herein may be up to 150 nucleotides (i.e. no more than 150 nucleotides in length). In this example, the ZCCHC14 protein-binding sequence may comprise the sequence of SEQ ID NO: 108, 109, 110, 111, 118 or 142.

In one example, the ZCCHC14 protein-binding sequence that is inserted into the 3' UTR of the transgene expression cassette described herein may be up to 100 nucleotides (i.e. no more than 100 nucleotides in length). In this example, the ZCCHC14 protein-binding sequence may comprise the sequence of SEQ ID NO: 108, 109, 110, 111, 118 or 142.

For example, the ZCCHC14 protein-binding sequence that is inserted into the 3' UTR of the transgene expression cassette described herein may be up to 90 nucleotides (i.e. no more than 90 nucleotides in length). In this example, the ZCCHC14 protein-binding sequence may comprise the sequence of SEQ ID NO: 108, 109, 110, 111, 118 or 142.

In an example where the cis-acting ZCCHC14 protein-binding sequence is a fragment (a portion of) an HBV PRE α element (i.e. a truncated nucleotide sequence that constitutes a part of SEQ ID NO: 95), the cis-acting ZCCHC14 protein-binding sequence may be up to 240 nucleotides of SEQ ID NO: 95. In this example, the ZCCHC14 protein-binding sequence may comprise the sequence of SEQ ID NO: 118, or 142.

In one example it may be up to 200 nucleotides of SEQ ID NO: 95. In this example, the ZCCHC14 protein-binding sequence may comprise the sequence of SEQ ID NO: 118, or 142.

For example, it may be up to 150 nucleotides of SEQ ID NO: 95. In this example, the ZCCHC14 protein-binding sequence may comprise the sequence of SEQ ID NO: 118, or 142.

In a further example it may be up to 100 nucleotides of SEQ ID NO: 95. In this example, the ZCCHC14 protein-binding sequence may comprise the sequence of SEQ ID NO: 118, or 142.

For example, it may be up to 90 nucleotides of SEQ ID NO: 95. In this example, the ZCCHC14 protein-binding sequence may comprise the sequence of SEQ ID NO: 118, or 142.

In an example where the cis-acting ZCCHC14 protein-binding sequence is a fragment (a portion of) a HCMV RNA 2.7 sequence (e.g. a truncated nucleotide sequence that constitutes a part of SEQ ID NO: 141), the cis-acting ZCCHC14 protein-binding sequence may be up to 240 nucleotides of SEQ ID NO: 141. In this example, the ZCCHC14 protein-binding sequence may comprise the sequence of SEQ ID NO: 108 or 110.

In one example it may be up to 200 nucleotides of SEQ ID NO: 141. In this example, the ZCCHC14 protein-binding sequence may comprise the sequence of SEQ ID NO: 108 or 110.

For example, it may be up to 150 nucleotides of SEQ ID NO: 141. In this example, the ZCCHC14 protein-binding sequence may comprise the sequence of SEQ ID NO: 108 or 110.

In a further example it may be up to 100 nucleotides of SEQ ID NO: 141. In this example, the ZCCHC14 protein-binding sequence may comprise the sequence of SEQ ID NO: 108 or 110.

In a further example it may be up to 90 nucleotides of SEQ ID NO: 141. In this example, the ZCCHC14 protein-binding sequence may comprise the sequence of SEQ ID NO: 108 or 110.

In an example where the cis-acting ZCCHC14 protein-binding sequence is a fragment (a portion of) a wPRE α element (i.e. a truncated nucleotide sequence that constitutes a part of SEQ ID NO: 114), the cis-acting ZCCHC14 protein-binding sequence may be up to 240 nucleotides of SEQ ID NO: 114. In this example, the ZCCHC14 protein-binding sequence may comprise the sequence of SEQ ID NO: 109 or 111.

In one example it may be up to 200 nucleotides of SEQ ID NO: 114. In this example, the ZCCHC14 protein-binding sequence may comprise the sequence of SEQ ID NO: 109 or 111.

For example, it may be up to 150 nucleotides of SEQ ID NO: 114. In this example, the ZCCHC14 protein-binding sequence may comprise the sequence of SEQ ID NO: 109 or 111.

In a further example it may be up to 100 nucleotides of SEQ ID NO: 114. In this example, the ZCCHC14 protein-binding sequence may comprise the sequence of SEQ ID NO: 109 or 111.

In a further example it may be up to 90 nucleotides of SEQ ID NO: 114. In this example, the ZCCHC14 protein-binding sequence may comprise the sequence of SEQ ID NO: 109 or 111.

In one example provided herein, the ZCCHC14 protein-binding sequence that is inserted into the 3' UTR of the transgene expression cassette described herein is up to 90 nucleotides (i.e. a maximum of 90 nucleotides long). See for example SEQ ID NO: 111, which provides the sequence for the ZCCHC14 stem loop from wPRE and is 90 nucleotides in length).

In one example provided herein, ZCCHC14 protein-binding sequence that is inserted into the 3' UTR of the transgene expression cassette described herein is up to 72 nucleotides (i.e. a maximum of 72 nucleotides long). See for example SEQ ID NO: 112, which provides the sequence for the ZCCHC14 stem loop from HCMV RNA2.7 and is 72 nucleotides in length). These examples demonstrate that relatively short sequences can be functional.

The ZCCHC14 protein-binding sequence that is inserted into the 3' UTR of the transgene expression cassette described herein may be the minimal sequence that is capable of being bound by a ZCCHC14 protein (i.e. it may be a minimal ZCCHC14 protein-binding sequence). In other words, it may be the smallest sequence that still provides the desired functionality. In the context of ZCCHC14 protein-binding sequences that are naturally found in PREs, the ZCCHC14 protein-binding sequence may therefore be the minimal PRE sequence that is capable of being bound by a ZCCHC14 protein. Methods for determining such minimal sequences are known in the art (e.g. nucleotide pull down assays, ELISA assays, reporter assays etc. may be used).

In an example, the ZCCHC14 protein-binding sequence may comprise or consist of the sequence of SEQ ID NO: 108 or 109. The ZCCHC14 protein-binding sequence may comprise or consist of a fragment of SEQ ID NO: 108 or 109 that is capable of binding ZCCHC14.

In one example, the ZCCHC14 protein-binding sequence may comprise or consist of the sequence of SEQ ID NO: 110 or 111. The ZCCHC14 protein-binding sequence may comprise or consist of a fragment of SEQ ID NO: 110 or 111 that is capable of binding ZCCHC14.

In one example, the ZCCHC14 protein-binding sequence may comprise or consist of the sequence of SEQ ID NO: 118 or 142.The ZCCHC14 protein-binding sequence may comprise or consist of a fragment of SEQ ID NO: 118 or 142 that is capable of binding ZCCHC14.

In the case of embodiments directed to a fragment of a specified sequence that is capable of binding ZCCHC14, the skilled person will readily be able to determine whether or not a given fragment of interest retains this protein-binding activity (for example by means of the assays described elsewhere in this disclosure) and, so will be able to assess whether or not this requirement is met without undue burden or need for excessive experimentation.

Suitably, a ZCCHC14 protein-binding sequence as described herein does not comprise a PRE β element. In some examples, the ZCCHC14 protein-binding sequence does not include any sequences that are specific to the PRE β element of SEQ ID NO: 115. In other words, it does not include any of the PRE β element of wPRE. In addition, it may not include any sequences that are specific to the PRE γ element of SEQ ID NO: 113. In other words, it may not include any of the PRE γ element of wPRE and also may not include any of the PRE β element of wPRE.

Thus far, the cis-acting ZCCHC14 protein-binding sequences and cis-acting CAR sequences provided herein have been discussed separately. However, as is shown in the examples section below (see also Figure 25), both of these sequences may be present within a 3' UTR of a transgene expression cassette. Accordingly, any aspect of the cis-acting ZCCHC14 protein-binding sequences described herein may be combined with any aspect of the cis-acting CAR sequences described herein to provide a transgene expression cassette wherein the 3' UTR comprises both a cis-acting CAR sequence and a cis-acting ZCCHC14 protein-binding sequence.

In such examples, the ZCCHC14 protein-binding sequence may be located 3' to the CAR sequence.

Alternatively, the ZCCHC14 protein-binding sequence may be located 5' to the CAR sequence within the 3'UTR of the transgene expression cassette.

Suitable positions for the novel cis-acting sequences described herein may be identified by a person of skill in the art, taking into account Figure 25, for example.

In some examples, the 3' UTR of the transgene expression cassette comprises at least two spatially distinct cis-acting CAR sequences and/or at least two spatially distinct cis-acting ZCCHC14 protein-binding sequences.

In some examples, the cis-acting sequences in the 3' UTR of a transgene expression cassette may comprise the sequence of SEQ ID NO: 240, SEQ ID NO: 241 or SEQ ID NO: 242.

In some examples, the 3' UTR of the transgene expression cassette further comprises a polyA sequence located 3' to the cis-acting CAR sequence and/or cis-acting ZCCHC14 protein-binding sequence. Details of polyA sequences are provided elsewhere herein.

The 3'UTR of the transgene expression cassette may comprise additional PRE sequences (in addition to the novel cis-acting CAR sequence and/or cis-acting ZCCHC14 protein-binding sequences described herein). For example, the 3'UTR may have a full length PRE sequence, such as wPRE.

Woodchuck Hepatitis Virus (WHV) Posttranscriptional Regulatory Element (wPRE) is a nucleotide sequence that, when transcribed, creates a tertiary structure enhancing expression. The sequence is commonly used in molecular biology to increase expression of genes delivered by viral vectors. wPRE is a tripartite regulatory element with y, α, and β components (also referred to as elements herein), in the given order. wPRE facilitates nucleocytoplasmic transport of RNA mediated by several alternative pathways that may be cooperative. In addition, the wPRE has been shown to act on additional posttranscriptional mechanisms to stimulate expression of heterologous cDNAs. Further details relating to wPRE are provided elsewhere herein.

The inventors have demonstrated that transgene expression cassettes the 3' UTR of which comprises both a novel cis-acting CAR sequence and/or cis-acting ZCCHC14 protein-binding sequence and an additional PRE, such as wPRE, are able to achieve markedly elevated transgene expression in cells.

In other examples, the 3'UTR of the transgene expression cassette does not comprise additional PRE sequences (in such examples, the novel cis-acting CAR sequence and/or *cis-*acting ZCCHC14 protein-binding sequences described herein are considered enhance transgene expression sufficiently, and, for example, the additional transgene capacity achieved by omitting additional PRE sequences (such as wPRE sequences) is desirable).

The transgene expression cassettes described herein may further comprise a promoter operably linked to the transgene. Several appropriate promoters are discussed in detail elsewhere herein.

For example, the promoter may be one that lacks its native intron (such as a promoter selected from the group consisting of: an EFS promoter, a PGK promoter, and a UBCs promoter).

Alternatively, the promoter may comprise an intron (for example, the promoter may be selected from the group consisting of: an EF1a promoter and a UBC promoter). These promoters are discussed in detail elsewhere herein.

The nucleotide sequences described herein may comprise a transgene expression cassette (also referred to as transgene cassettes herein). The invention has particular utility when the novel cis-acting sequences described herein are present within the 3'UTR of a lentiviral vector transgene expression cassette. Accordingly, any discussion of a transgene expression cassette is particularly relevant to lentiviral vector transgene expression cassettes.

The lentiviral vector transgene expression cassette may be any suitable lentiviral vector transgene expression cassette. Appropriate lentiviral vectors are discussed in detail elsewhere herein.

The nucleotide sequences provided herein may be part of a viral vector genome. In other words, the transgene expression cassette provided herein may be part of a larger nucleotide sequence, which further comprises additional elements that are required to make up a viral vector genome. This may include, for example in the context of lentiviral vector genomes, a typical packaging sequence and rev-response element (RRE). In suitable embodiments, these may further comprise additional post-transcriptional regulatory elements (PREs) such as that from the woodchuck hepatitis virus (wPRE), as considered above. Each of these elements are discussed in more detail elsewhere herein.

Accordingly, a nucleotide sequence comprising a lentiviral vector genome expression cassette is also provided herein, wherein the lentiviral vector genome expression cassette comprises the transgene expression cassette described elsewhere herein.

In one example, the transgene expression cassette is in the forward orientation with respect to the lentiviral vector genome expression cassette (such that the transgene expression cassette is encoded in the sense orientation).

Alternatively, the transgene expression cassette may be inverted with respect to the vector genome expression cassette, i.e. the internal transgene promoter and gene sequences oppose the vector genome cassette promoter.

As would be known by a person of skill in the art, the 3' UTR of the lentiviral vector genome expression cassette typically further comprises a 3' polypurine tract (3'ppt) and a DNA attachment (att) site. Typically, the 3'ppt is located 5' to the att site within the 3'UTR of the retroviral vector genome expression cassette. When the invention is contemplated in the context of a transgene expression cassette that is the forward orientation (sense orientation) with respect to the genome expression cassette, the positioning of the novel cis-acting sequences provided herein relative to the 3'ppt and att site may need to be considered. Further details of this are provided in Example 10.

For example the core sequence that comprises both the 3'ppt and the att site (e.g. of a lentiviral vector genome expression cassette as described herein) may have a sequence of SEQ ID NO: 93 (wherein 3'ppt is in bold, and *att* is underlined):
5'-**AAAAGAAAAGGGGGG**ACTGGAAGGGCTAATTCAC-3' (SEQ ID NO: 93)

Accordingly, where the transgene cassette is in a forward orientation with respect to the lentiviral vector genome expression cassette, it is preferable if the sequence above (of SEQ ID NO: 93) is not disrupted by the novel cis-acting sequences described herein.

In one example, the sequence of SEQ ID NO: 94 may be used to provide the 3'ppt and att site (e.g. of a lentiviral vector genome expression cassette as described herein), (wherein 3'ppt is in bold, and *att* is underlined):

5’-
CCAATGACTTACAAGGCAGCTGTAGATCTTAGCCACTTTTTAAAAGAAAAGGGGGGAC
TGGAAGGGCTAATTCACTCCCAA-3’ (SEQ ID NO: 94)

Accordingly, where the transgene cassette is in a forward orientation with respect to the lentiviral vector genome expression cassette, it is preferable if the sequence above (of SEQ ID NO: 94) is not disrupted by the novel cis-acting sequences described herein.

Preferably, where the transgene cassette is in a forward orientation with respect to the lentiviral vector genome expression cassette cis-acting elements within the 3'UTR of the transgene cassette may be positioned upstream and/or downstream of the above uninterrupted sequences. Figure 25 also indicates how the CARe and/or ZCCHC14 binding loop(s) may be variably positioned within LV genome expression cassettes with or without a PRE, with transgene sequences in forward or reverse orientation.

For example, when the transgene expression cassette is in the forward orientation with respect to the genome expression cassette, the cis-acting sequence(s) described herein may be located 5' to the 3'ppt and/or 3' to the att site. Preferably, in this example, the cis-acting sequence(s) described herein are located 5' to the sequence of SEQ ID NO: 93 or SEQ ID NO: 94.

Alternatively, the cis-acting sequence(s) described herein may be located 3' to the sequence of SEQ ID NO: 93 or SEQ ID NO: 94.

In either example, the sequence of SEQ ID NO: 93 or SEQ ID NO: 94 is not disrupted.

The nucleotide sequences described herein may include additional features that are described in more detail elsewhere herein. For example, when the nucleotide sequence comprises a lentiviral vector genome expression cassette, the major splice donor site in the lentiviral vector genome expression cassette may be inactivated. Furthermore, the cryptic splice donor site 3' to the major splice donor site may also be inactivated. In one example that is described in more detail elsewhere herein, the inactivated major splice donor site may have the sequence of GGGGAAGGCAACAGATAAATATGCCTTAAAAT (SEQ ID NO: 4; MSD-2KOm5).

When the nucleotide sequence comprises a lentiviral vector genome expression cassette, the nucleotide sequence may further comprise a nucleotide sequence encoding a modified U1 snRNA, wherein the modified U1 snRNA has been modified to bind to a nucleotide sequence within the packaging region of the lentiviral vector genome. For example, the viral vector genome expression cassette may be operably linked to the nucleotide sequence encoding the modified U1 snRNA. This feature is described in more detail elsewhere herein.

A method for identifying one or more cis-acting sequence(s) that improve transgene expression in a target cell is also provided, the method comprising the steps of:
(a) transducing target cells with a viral vector described herein;
(b) identifying target cells with a high level transgene expression; and
(c) optionally identifying the one or more cis-acting sequence(s) located within the 3' UTR of the transgene mRNA present within these target cells.

This method may be used to determine which one or more of the specific cis-acting sequences described herein improves transgene expression in a specific target cell. The method therefore provides a mechanism for tailoring the selection of specific cis-acting sequences described herein for a specific combination of viral vector, transgene, and target cell.

The method can therefore advantageously be performed using a plurality of viral vectors with:
(i) different cis-acting sequences in the 3'UTR of the transgene expression cassette; and
(ii) different cis-acting sequence locations within the 3'UTR of the transgene expression cassette; and/or
(iii) different cis-acting sequence combinations in the 3'UTR of the transgene expression cassette;
to identify one or more *cis*-acting sequence(s) that improve transgene expression in the target cell.

Any appropriate means for identifying the one or more cis-acting sequence(s) located within the 3' UTR of the transgene mRNA present within these target cells may be used within the method. In one example, step (c) of the method comprises performing RT-PCR and optionally sequencing the transgene mRNA.

### Vector intron

In one aspect, the present invention provides a nucleotide sequence comprising a lentiviral vector genome expression cassette, wherein the lentiviral vector genome expression cassette comprises a transgene expression cassette and a vector intron (VI).

Accordingly, in some embodiments of the nucleotide sequence comprising a lentiviral vector genome expression cassette of the invention:
i) the major splice donor site in the lentiviral vector genome expression cassette is inactivated;
ii) the lentiviral vector genome expression cassette does not comprise a rev-response element;
iii) the lentiviral vector genome expression cassette comprises a transgene expression cassette and a vector intron.

In some embodiments, the cryptic splice donor site adjacent to the 3' end of the major splice donor site in the lentiviral vector genome expression cassette is inactivated.

In some embodiments of the nucleotide sequence comprising a lentiviral vector genome expression cassette of the invention:
i) the major splice donor site and cryptic splice donor site adjacent to the 3' end of the major splice donor site in the lentiviral vector genome expression cassette are inactivated;
ii) the lentiviral vector genome expression cassette does not comprise a rev-response element;
iii) the lentiviral vector genome expression cassette comprises a transgene expression cassette and a vector intron.

The vector intron is in the sense orientation (i.e. forward orientation) with respect to the lentiviral vector genome expression cassette.

In some embodiments, the vector intron comprises one or more transgene mRNA self-destabilization or self-decay element(s) or one or more transgene mRNA nuclear retention signal(s).

In one embodiment, the transgene expression cassette is in the forward orientation with respect to the lentiviral vector genome expression cassette, i.e. such that the transgene expression cassette is encoded in the sense orientation.

In one embodiment, the transgene expression cassette is inverted with respect to the lentiviral vector genome expression cassette.

In some embodiments:
a) the vector intron is not located between the promoter of the transgene expression cassette and the transgene; and/or
b) the nucleotide sequence comprises a sequence as set forth in any of SEQ ID NOs: 2, 3, 4, 6, 7, and/or 8, and/or the sequences CAGACA, and/or GTGGAGACT; and/or
c) the 3' UTR of the transgene expression cassette comprises the vector intron.

In some embodiments:
a) the vector intron is not located between the promoter of the transgene expression cassette and the transgene; and
b) the nucleotide sequence comprises a sequence as set forth in any of SEQ ID NOs: 2, 3, 4, 6, 7, and/or 8, and/or the sequences CAGACA, and/or GTGGAGACT; and
c) the 3' UTR of the transgene expression cassette comprises the vector intron.

In some embodiments, the transgene expression cassette is inverted with respect to the lentiviral vector genome expression cassette and:
a) the vector intron is not located between the promoter of the transgene expression cassette and the transgene; and/or
b) the nucleotide sequence comprises a sequence as set forth in any of SEQ ID NOs: 2, 3, 4, 6, 7, and/or 8, and/or the sequences CAGACA, and/or GTGGAGACT; and/or
c) the 3' UTR of the transgene expression cassette comprises the vector intron.

In some embodiments, the transgene expression cassette is inverted with respect to the lentiviral vector genome expression cassette and:
a) the vector intron is not located between the promoter of the transgene expression cassette and the transgene; and
b) the nucleotide sequence comprises a sequence as set forth in any of SEQ ID NOs: 2, 3, 4, 6, 7, and/or 8, and/or the sequences CAGACA, and/or GTGGAGACT; and
c) the 3' UTR of the transgene expression cassette comprises the vector intron.

In some embodiments, the 3' UTR of the transgene expression cassette comprises the vector intron encoded in antisense with respect to the transgene expression cassette. Thus, when the transgene expression cassette is inverted with respect to the lentiviral vector genome expression cassette and the 3' UTR of the transgene expression cassette comprises the vector intron; the vector intron is in an antisense orientation with respect to the lentiviral vector genome expression cassette.

In some embodiments of the nucleotide sequence comprising a lentiviral vector genome expression cassette:
i) the major splice donor site and cryptic splice donor site adjacent to the 3' end of the major splice donor site in the lentiviral vector genome expression cassette are inactivated;
ii) the lentiviral vector genome expression cassette does not comprise a rev-response element;
iii) the lentiviral vector genome expression cassette comprises a transgene expression cassette and a vector intron; and
iv)
   a) When the transgene expression cassette is inverted with respect to the lentiviral vector genome expression cassette:
      i. the vector intron is not located between the promoter of the transgene expression cassette and the transgene; and
      ii. the nucleotide sequence comprises a sequence as set forth in any of SEQ ID NOs: 2, 3, 4, 6, 7, and/or 8, and/or the sequence CAGACA, and/or GTGGAGACT; and
      iii. the 3' UTR of the transgene expression cassette comprises the vector intron; and
   b) the vector intron comprises one or more transgene mRNA self-destabilization or self-decay element(s) or one or more transgene mRNA nuclear retention signal(s).

As described herein, the present inventors surprisingly found that the VI enabled deletion of the RRE and resulted in a rev/RRE-independent LV genome (see Example 14). Since the VI sequence is removed from the LV vRNA prior to appearance in the cytoplasm, this allows for increased transgene capacity of LVs by ~780nts. In other Examples herein, the inventors show that at least a further ~260nts can be liberated by deletion of the gag-p17 instability (p17-INS) element from the gag sequence typically retained as part of the packaging sequence.

Even more surprising was that to achieve the highest LV titres using the VI in a RRE-deleted LV genome, the MSD-2KO feature appeared to be beneficial (see Example 14). Therefore, the MSD-2KO and VI features of these new class of rev/RRE-independent LV genomes may be mutually 'symbiotic', i.e. mutually beneficial, at the molecular level. The VI may rescue the negative impact of the MSD-2KO mutation on LV vRNA production/titres, and the MSD-2KO mutation may stop aberrant splicing to internal splice acceptors (including that of the VI) and to allow for maximal titres of VI-containing, RRE-deleted LV genomes.

The VI according to the present invention may be any suitable functional intron.

As such, VI may comprise any nucleotide sequence recognizable as an intron. Introns are well known in the art. Typically, an intron is a nucleotide sequence within a gene that is spliced-out, i.e. removed by RNA splicing, before the RNA molecule is translated into protein. Introns may be identified by a number of features, e.g. the presence of splice sites and/or a branch point.

Thus, the VI according to the present invention may comprise a splice donor site, a splice acceptor site and a branch point.

As used herein, the term "branch point" refers to a nucleotide, which initiates a nucleophilic attack on the splice donor site during RNA splicing. The resulting free 3' end of the upstream exon may then initiate a second nucleophilic attack on the splice acceptor site, releasing the intron as an RNA lariat and covalently combining the two sequences flanking the intron (e.g. the upstream and downstream exons).

Illustrative splice donor and splice acceptor sequences suitable for use according to the invention are provided in Table 4 below.

**Table 4. Illustrative splice donor and splice acceptor sequences.**

| **Description** | **Sequence** | **SE Q ID NO** |
|---|---|---|
| Core splice donor consensus | MAGGURR; wherein M is A or C and R is A or G | - |
| Splice donor consensus with further complement arity to U1 snRNA | MAGGUAAGU; wherein M is A or C. | - |
| Splice donor consensus with maximum complement arity to U1 snRNA | MAGGUAAGUAU; wherein M is A or C. | 119 |
| EF1a splice donor (underlined =exon/intron) | GAACACAG/GTAAGTGCCGTGTGTGG | 120 |
| Ubiquitin splice donor (underlined= exon/intron) | GTCACTTG/GTGAGTAGCGGGCTGCT | 121 |
| Chicken Actin splice donor (underlined= exon/intron) | GGGCGGGA/GTCGCTGCGTTGCCTTC | 122 |
| Rabbit β-globin splice donor (underlined exon/intron) | CTGGGCAG/GTAAGTATCAAGGTTAC | 123 |
| HIV-1 major splice donor (underlined= exon/intron) | GGCGACTG/GTGAGTACGCC | 124 |
| HIV-1 splice donor 1a (underlined= exon/intron) | TAGGACAG/GTAAGAGATCAGGCTGA | 125 |
| HIV-1 splice donor 4 (underlined= exon/intron) | TCAAAGCA/GTAAGTAGTACATGTAA | 126 |
| Human β-globin splice donor (underlined= exon/intron) | CTGGGCAG/GTGAGTCTATGGGACCC | 127 |
| Core splice acceptor consensus | YYYYYYYNCAGR; wherein Y is C or T/U, N is any nucleotide (i.e. G, C, A or T/U) and R is G or A | 128 |
| EF1a splice acceptor (underlined= intron/exon; bold = branch site zone) | GGTTCATTCTCAAGCCTCAGACAGTGGTTCAAAGTTTTTTTCTTCCATTT CAG/GTGTCGTGA | 129 |
| Ubiquitin splice acceptor (underlined= intron/exon; bold = branch site zone) | CAGGGTGGGGCCACCTGCCGGTAGGTGTGCGGTAGGCTTTTCTCCGTCGC AG/GACGCAG | 130 |
| IgG heavy chain splice acceptor (underlined intron/exon; bold = branch site zone) | TGATAGGCACCTATTGGTCTTACTGACATCCACTTTGCCTTTCTCTCCAC AG/GTGTCCACTC | 131 |
| Rabbit β-globin splice acceptor (underlined intron/exon; bold = branch site zone) | GCGGCTTTAGAGCCTCTGCTAACCATGTTCATGCCTTCTTCTTTTTCCTA CAG/CTCCTGGGC | 132 |
| Human β-globin splice acceptor (underlined intron/exon; bold = branch site zone) | CCAAGCTAGGCCCTTTTGCTAATCATGTTCATACCTCTTATCTTCCTCCC ACAG/GTGTCCACT | 133 |

In some embodiments, the vector intron according to the invention comprises a sequence selected from MAGGURR, MAGGUAAGU and SEQ ID NOs: 119-127.

In some embodiments, the vector intron according to the invention comprises a sequence selected from SEQ ID NOs: 128-133.

In some embodiments, the vector intron according to the invention comprises a sequence selected from MAGGURR, MAGGUAAGU and SEQ ID NOs: 119-127 and a sequence selected from SEQ ID NOs: 128-133.

In one preferred embodiment, the vector intron according to the invention comprises the sequence as set forth in SEQ ID NO: 126 and the sequence as set forth in SEQ ID NO: 132.

Native splice donor sequences may not adhere fully to the core splice donor consensus sequence described herein (i.e. to MAGGURR; wherein M is A or C and R is A or G). For example, the splice donor sequence of SEQ ID NO: 126 does not fully adhere to the core splice donor consensus sequence. Such native splice donor sequences may be modified to increase the conformity, or to fully conform with, with the core splice donor consensus sequence without deleterious effect on the function of the resulting modified splice donor sequence and/or vector intron comprising the resulting modified splice donor sequence. Methods to modify a nucleotide sequence are known in the art. Modifying a splice donor sequence as described above to increase its conformity with the core splice donor consensus sequence is within the ambit of the skilled person.

The VI according to the present invention may be a naturally occurring intron.

The VI according to the present invention may be synthetic, or derived wholly or partially from any suitable organism.

In one embodiment the vector intron is from EF1α. In one embodiment, the vector intron is the intron of EF1α.

In one embodiment the vector intron is from human β-globin intron-2. In one embodiment the vector intron is human β-globin intron-2.

Further, the VI may be optimized to improve vector titre by use of the following sequences: [1] short exonic splicing enhancers (ESEs) upstream of the VI splice donor site, [2] optimal splice donor sites (typically with maximal annealing potential to U1 snRNA), [3] use of optimal branch and splice acceptor sites, and [4] the use of short exonic splicing enhancers (ESEs) downstream of the VI splice acceptor site. Examples of the most optimal VI variants were composite sequences from HIV-1 and cellular introns such as human β-globin intron-2 (Example 18).

The VI according to the present invention may be a chimeric or modular intron comprising sequences, such as functional sequences, from different introns. Thus, the VI of the invention may be designed to comprise a composite of different sequences, such as functional sequences, from more than one intron, such sequences may comprise, for example, a splice donor site sequence, a splice acceptor site sequence, a branch point sequence (see Table 7). The branch point and splice acceptor site sequence may together be referred to as the "branch-splice acceptor sequence" herein.

The VI according to the invention may be furnished with upstream exonic splicing enhancer (ESE) elements, for example hESE, hESE2 and hGAR from HIV-1 (see Table 7). The sequences of hESE, hESE2 and hGAR are provided below.
hESE (underlined = hESE):
   GTCGACTGATCTTCAGACCTGGAGGAGGAGATATGAGGGACAATTGATGCATCTCGAGC (SEQ ID NO: 134)
hESE2, shown downstream of cppt/CTS (underlined = hESE2; bold = cppt/CTS): 
AAATTGGCAGTATTCATCCACAATTTTAAAAGAAAAGGGGGGATTGGGGGGTACAGTGCAGG
GGAAAGAATAGTAGACATAATAGCAACAGACATACAAACTAAAGAATTACAAAAACAAATTA
CAAAAATTCAAAATTTTCGGGTTTCACGTGACGCGTCTGCAGCCGAAGGAATAGAAGAAGAA
GGTGGAGAGAGAGACAGAGACCTGCAGACG (SEQ ID NO: 135)
GAR (underlined = GAR):
   TGGCAGGAAGAAGCGGAGACAGCGACGAAGAGCTCATCAGAA (SEQ ID NO: 136)

In one embodiment, the VI of the invention comprises a sequence as set forth in SEQ ID NO: 134, 135 or 136.

In some embodiments, the vector intron according to the invention comprises a sequence selected from MAGGURR, MAGGUAAGU and SEQ ID NOs: 119-127 and a sequence selected from SEQ ID NOs: 134-136.

In some embodiments, the vector intron according to the invention comprises a sequence selected from SEQ ID NOs: 128-133 and a sequence selected from SEQ ID NOs: 134-136.

In some embodiments, the vector intron according to the invention comprises a sequence selected from MAGGURR, MAGGUAAGU and SEQ ID NOs: 119-127, and a sequence selected from SEQ ID NOs: 128-133 and a sequence selected from SEQ ID NOs: 134-136.

In one preferred embodiment, the vector intron according to the invention comprises the sequences as set forth in SEQ ID NO: 126, SEQ ID NO: 132 and SEQ ID NO: 135.

In one preferred embodiment, the vector intron according to the invention comprises the sequences as set forth in SEQ ID NO: 126, SEQ ID NO: 132 and SEQ ID NO: 136.

In one preferred embodiment, the vector intron according to the invention comprises the sequences as set forth in SEQ ID NO: 126, SEQ ID NO: 132, SEQ ID NO: 135 and SEQ ID NO: 136.

In one embodiment, the VI of the invention is operably linked to an upstream exonic splicing enhancer (ESE) element, such as hESE, hESE2 or hGAR.

In one embodiment, the VI of the invention is a synthetic vector intron comprising the HIV-1 guanosine-adenosine rich (GAR) splicing element

In one embodiment, the VI of the invention is a synthetic vector intron comprising the HIV-1 guanosine-adenosine rich (GAR) splicing element upstream of the splice donor sequence.

In one embodiment, the VI of the invention is a synthetic vector intron comprising the hESE2 downstream of the splice acceptor.

In one embodiment, the VI of the invention is a synthetic vector intron comprising the hESE2 downstream of the splice acceptor and the cppt/CTS sequence of the vector genome.

In one embodiment, the lentiviral vector genome expression cassette comprises an hGAR upstream enhancer element and a VI comprising or consisting of a HIV SD4 splice donor sequence, and a human β-globin intron-2 derived sequence containing a branch-splice acceptor sequence.

In one embodiment, the lentiviral vector genome expression cassette comprises an hGAR upstream enhancer element and a VI comprising or consisting of a HIV SD4 splice donor sequence, a human β-globin intron-2 derived sequence containing a branch-splice acceptor sequence, and hESE2 downstream of the cppt/CTS.

In one embodiment, the VI of the invention comprises any of the features disclosed in Table 7. In another embodiment, the VI of the invention comprises an intron and (where applicable) an upstream enhancer element with the combination of features described for any one of VI_v1.1, VI_v1.2, VI_v2.1, VI_v2.2, VI_v3.1, VI_v4.1, VI_v4.2, VI_v4.3, VI_v4.4, VI_v4.5, VI_v4.6, VI_v4.7, VI_v4.8, VI_v4.9, VI_v4.10, VI_v4.11, VI_v4.12, VI_v5.1, VI_v5.2, VI_v5.3, VI_v5.4, VI_v5.5, or VI_5.7. In a preferred embodiment the VI of the invention comprises the features of VI_v5.5.

Illustrative examples of vector intron sequences are provided below.
VI_4.12 (bold = GAR; underlined = HIV-1 splice donor 4; italics = chimeric intron-branch-splice acceptor sequence; plain text = exon/intron/exon): 
TTTTGGTCGTGAGGCACTGGGCAGGAAGAAGCGGAGACAGCGACGAAGAGCTCATCAGAACT
AGTCAGACTCATCAAGCTTCTCTATCAAAGCA/GTAAGTAGTACATGTAACAAGGTTACAAG
ACAGGTTTAAGGAGACCAATAGAAACTGGGCTTGTCGAGACAGAGAAGACTCTTGCGTTTCT
GATAGGCACCTATTGGTCTTACTGACATCCACTTTGCCTTTCTCTCCACAG/GTGTCCACTC
CCAGTTC (SEQ ID NO: 137)
VI_5.5 (bold = GAR; underlined = HIV-1 splice donor 4; italics = human β-globin [intron 2] splice acceptor; plain text = exon/intron/exon) 
TTTTGGTCGTGAGGCACTGGGCAGGAAGAAGCGGAGACAGCGACGAAGAGCTCATCAGAACT
AGTCAGACTCATCAAGCTTCTCTATCAAAGCA/GTAAGTAGTACATGTAAGAGTCTATGGGA
CCCTTGATGTTTTCTTTCCCCTTCTTTTCTATGGTTAAGTTCATGTCATAGGAAGGGGAGAA
GTAACAGGGTACACATATTGACCAAATCAGGGTAATTTTGCATTTGTAATTTTAAAAAATGC
TTTCTTCTTTTAATATACTTTTTTGTTTATCTTATTTCTAATACTTTCCCTAATCTCTTTCT
 
TTCAGGGCAATAATGATACAATGTATCATGCCTCTTTGCACCATTCTAAAGAATAACAGTGA
TAATTTCTGGGTTAAGGCAATAGCAATATTTCTGCATATAAATATTTCTGCATATAAATTGT
AACTGATGTAAGAGGTTTCATATTGCTAATAGCAGCTACAATCCAGCTACCATTCTGCTTTT
ATTTTATGGTTGGGATAAGGCTGGATTATTCTGAGTCCAAGCTAGGCCCTTTTGCTAATCAT
GTTCATACCTCTTATCTTCCTCCCACAG/GTGTCCACTCCCAGTTC (SEQ ID NO:
138)
VI_5.6 (bold = GAR; underlined = HIV-1 splice donor 4; italics = human β-globin [intron 2] splice acceptor; plain text = exon/intron/exon; boxed = cppt/CTS; bold italics = hESE2)

TTTTGGTCGTGAGGCACTGGGCAGGAAGAAGCGGAGACAGCGACGAAGAGCTCATCAGAACT
AGTCAGACTCATCAAGCTTCTCTATCAAAGCA/GTAAGTAGTACATGTAAGAGTCTATGGGA
CCCTTGATGTTTTCTTTCCCCTTCTTTTCTATGGTTAAGTTCATGTCATAGGAAGGGGAGAA
GTAACAGGGTACACATATTGACCAAATCAGGGTAATTTTGCATTTGTAATTTTAAAAAATGC
TTTCTTCTTTTAATATACTTTTTTGTTTATCTTATTTCTAATACTTTCCCTAATCTCTTTCT
TTCAGGGCAATAATGATACAATGTATCATGCCTCTTTGCACCATTCTAAAGAATAACAGTGA
TAATTTCTGGGTTAAGGCAATAGCAATATTTCTGCATATAAATATTTCTGCATATAAATTGT
AACTGATGTAAGAGGTTTCATATTGCTAATAGCAGCTACAATCCAGCTACCATTCTGCTTTT
ATTTTATGGTTGGGATAAGGCTGGATTATTCTGAGTCCAAGCTAGGCCCTTTTGCTAATCAT
GTTCATACCTCTTATCTTCCTCCCACAG/GTGTCCACTCCCAGTTCCTGCAGGCACGTGAAA
TTGGCAGTATTCATCCACAATTTTAAAAGAAAAGGGGGGATTGGGGGGTACAGTGCAGGGGA
AAGAATAGTAGACATAATAGCAACAGACATACAAACTAAAGAATTACAAAAACAAATTACAA
AAATTCAAAATTTTCGGGTTTCACGTGACGCGTCTGCAGCCGAAGGAATAGAAGAAGAAGGT
GGAGAGAGAGACAGAGACCTGCAGACGCGT (SEQ ID NO: 139)

In one embodiment, the vector intron of the invention comprises the sequence as set forth in SEQ ID NO: 137.

In a preferred embodiment, the vector intron of the invention comprises the sequence as set forth in SEQ ID NO: 138.

In one embodiment, the vector intron of the invention comprises the sequence as set forth in SEQ ID NO: 139.

As illustrative example of a nucleotide sequence encoding a lentiviral vector genome sequence comprising a vector intron of the invention is provided below:
LV-MSD2KOmM5-Δp17INS-ΔRRE-VI_5.6 (shown is the 5' R to internal transgene cassette: bold italics = MSD2KOm5; underlined = gagΔp17INS; bold = VI_5.6; boxed = cppt/CTS):

GGGTCTCTCTGGTTAGACCAGATCTGAGCCTGGGAGCTCTCTGGCTAACTAGGGAACCCACT
GCTTAAGCCTCAATAAAGCTTGCCTTGAGTGCTTCAAGTAGTGTGTGCCCGTCTGTTGTGTG
ACTCTGGTAACTAGAGATCCCTCAGACCCTTTTAGTCAGTGTGGAAAATCTCTAGCAGTGGC
GCCCGAACAGGGACTTGAAAGCGAAAGGGAAACCAGAGGAGCTCTCTCGACGCAGGACTCGG
CTTGCTGAAGCGCGCACGGCAAGAGGCGAGGGGAAGGCAACAGATAAATATGCCTTAAAATT
TTGACTAGCGGAGGCTAGAAGGAGAGAGACGGGTGCGAGAGCGTCAGTATTAAGCGGGGGAG
AATTAGATCGCGATTGGGAAAAAATTCGGTTAAGGCCAGGGGGAAAGACTCGAGCAATTTTG

GTCGTGAGGCACTGGGCAGGAAGAAGCGGAGACAGCGACGAAGAGCTCATCAGAACTAGTCA
GACTCATCAAGCTTCTCTATCAAAGCAGTAAGTAGTACATGTAAGAGTCTATGGGACCCTTG
ATGTTTTCTTTCCCCTTCTTTTCTATGGTTAAGTTCATGTCATAGGAAGGGGAGAAGTAACA
GGGTACACATATTGACCAAATCAGGGTAATTTTGCATTTGTAATTTTAAAAAATGCTTTCTT
CTTTTAATATACTTTTTTGTTTATCTTATTTCTAATACTTTCCCTAATCTCTTTCTTTCAGG
GCAATAATGATACAATGTATCATGCCTCTTTGCACCATTCTAAAGAATAACAGTGATAATTT
CTGGGTTAAGGCAATAGCAATATTTCTGCATATAAATATTTCTGCATATAAATTGTAACTGA
TGTAAGAGGTTTCATATTGCTAATAGCAGCTACAATCCAGCTACCATTCTGCTTTTATTTTA
TGGTTGGGATAAGGCTGGATTATTCTGAGTCCAAGCTAGGCCCTTTTGCTAATCATGTTCAT
ACCTCTTATCTTCCTCCCACAGGTGTCCACTCCCAGTTCCTGCAGGCACGTGAAATTGGCAG
TATTCATCCACAATTTTAAAAGAAAAGGGGGGATTGGGGGGTACAGTGCAGGGGAAAGAATA
GTAGACATAATAGCAACAGACATACAAACTAAAGAATTACAAAAACAAATTACAAAAATTCA
AAATTTTCGGGTTTCACGTGACGCGTCTGCAGCCGAAGGAATAGAAGAAGGTGGAGAGA
GAGACAGAGACCTGCAGACGCGT (SEQ ID NO: 140)

Transgene sequences followed by the 3' SIN-LTR may be located at the 3' end of SEQ ID NO: 140.

In one embodiment, the nucleotide sequence encoding the lentiviral vector genome of the invention comprises the sequence as set forth in SEQ ID NO: 140.

As out-splicing of other introns is stimulated by the presence of VI (and absence of rev/RRE system), transgene cassettes encoding their own intron(s) (e.g. the EF1a promoter) will lose these introns from the vRNA. To avoid this, the transgene cassette may be inverted. Inverting the transgene cassette allows the embedded introns to not be lost from the resultant vRNA since they will not function as introns within the LV genome expression cassette. A drawback of this configuration is a reduction in LV titres due to production of double-stranded RNA (dsRNA) species that are formed as a result of annealing between the vRNA and transgene mRNA. Sensing of dsRNA can trigger cellular responses such as the PKR response, cleavage by Dicer, or deamination by ADAR. This may not be the case for transgene cassettes driven by tissue specific promoters that are minimally active in LV production cells, such as HEK293T cells.

Hence, in one aspect, the lentiviral vector genome expression cassette of the invention may utilise tissue specific promoters to reduce the negative effects, e.g. titre reduction, incurred as a result of the formation of dsRNA species.

The VI intron of the present invention facilitates the use of LV genomes comprising additional desirable features, e.g. MSD-2KO and RRE-deletions. Hence, use of MSD-2KO, RRE-deleted LV genomes containing VI advantageously enables: [1] increased transgene capacity, [2] ablation of aberrant splicing from the packaging sequence, [3] removal of rev from the production system, [4] reduced transcriptional read-in in target cells, and [5] use of inverted transgene cassettes with 3' UTR *cis*-elements to repress transgene expression during LV production. All of the aforementioned contribute to improved safety of the vector genome expression cassettes of the invention.

In an aspect, the invention provides a nucleotide sequence comprising a lentiviral vector genome expression cassette, wherein the major splice donor site in the lentiviral vector genome expression cassette is inactivated; the lentiviral vector genome expression cassette does not comprise a rev-response element; and the lentiviral vector genome expression cassette comprises a transgene expression cassette and a vector intron.

In an aspect, the vector intron is a synthetic vector intron.

In an aspect, the synthetic vector intron comprises the HIV-1 GAR splicing element.

In an aspect, the vector intron comprises a splice donor sequence, wherein said splice donor sequence comprises from 7 to 11 nucleotides which are complementary to a portion of the U1 snRNA sequence.

In a further aspect, the synthetic vector intron comprises the HIV-1 guanosine-adenosine rich splicing element upstream of the splice donor sequence.

In an aspect, the synthetic vector intron comprises the branch site and splice acceptor of the human beta-globin intron-2.

Since introns, such as a the VI according to the invention, may be spliced out of a given nucleotide sequence, such as the nucleotide sequences comprising the lentiviral vector genome expression cassettes according to the invention, it is to be understood that products of such nucleotides, e.g. nucleotide products and cells or particles comprising the same, may not comprise the VI. Such nucleotides, cells, and/or particles may comprise a residual sequence, such as a splice junction sequence, by virtue of splicing of the VI. The junctional sequence may correspond to SEQ ID NO: 19.

The terms "EF1α" and "EF1a" are used interchangeably herein.

### Transgene mRNA self-destabilization or self-decay elements

The formation of dsRNA species has undesirable titre-reducing effects. As described herein, dsRNA species may be formed during production of viral vectors comprising an inverted transgene expression cassette. The present invention solves this problem by providing LV genome expression cassettes that comprise transgene mRNA self-destabilization or self-decay elements, or transgene mRNA nuclear retention signals, that function to reduce the amount of dsRNA formed when the LV genome expression cassette comprises an inverted transgene expression cassette. Preferably, such functional cis-acting transgene mRNA self-destabilization or self-decay elements, or functional cis-acting transgene mRNA nuclear retention signals, are positioned within the 3'UTR of the transgene cassette and within the VI sequence, and are used to achieve transgene repression in order to avoid dsRNA responses during LV production, which have deleterious effects on titre.

Preferably, the vector intron is in the antisense orientation with respect to the transgene expression cassette. Suitably, the VI may comprise the transgene mRNA self-destabilisation or self-decay element in or reverse (i.e. antisense) orientation with respect to the lentiviral vector genome expression cassette. Suitably, when the transgene mRNA self-destabilisation or self-decay element is a miRNA, the VI may comprise the miRNA in the forward (i.e. sense) orientation or reverse (i.e. antisense) with respect to the lentiviral vector genome expression cassette. The reverse orientation of the miRNA provides the advantage that use is made of the miRNA processing step, resulting in transgene mRNA cleavage in generation of the pre-miRNA in addition to subsequent miRNA-mediated transgene mRNA cleavage. Use of a transgene mRNA-targeting miRNA cassette in the forward orientation within the VI with respect to the lentiviral vector genome cassette would result in the pre-miRNA being processed within the VI RNA (i.e. derived from (pre-) vRNA) and not the transgene mRNA. Thus, the miRNA may be provided in either the forward or the reverse orientation within the VI.

Thus, the inventors herein provide a solution to the problems associated with the formation of dsRNA, namely modifying the configuration or orientation of the VI with respect to the transgene expression cassette. By positioning the 3' UTR of the inverted transgene cassette such that the VI sits within it, cis-elements can be inserted within the 3' UTR sequence encompassed by the VI (preferably in antisense orientation with respect to the vector genome expression cassette unless the *cis*-element is a miRNA, which can be in either orientation), which destabilize the transgene mRNA or induce its decay or degradation. These can be known destabilization elements or self-destabilization or self-decay elements, and comprise elements such as AU-rich element (AREs), self-cleaving ribozymes, microRNAs, microRNA target sequences and pre-miR, which can be processed and 'self-target' the transgene mRNA.

An alternative or additional type of element that can be used are target sequences for miRNAs expressed during lentiviral vector production; these miRNAs expressed during lentiviral vector production can either be endogenously expressed miRNAs by the host cell or by exogenously expressed miRNAs (e.g. by co-transfection of a U6-driven mi/shRNA cassette). This concept is described in Figure 62.

miRNAs are transcribed as primary miRNA (pri-miRNA) that can be several kilobases long. These transcripts are processed in the nucleus to 60-90 nt long precursor-miRNA hairpins (pre-miRs or pre-miRNAs, used interchangeably herein) by the Microprocessor complex. Thus, pre-miRs refer to the hairpin precursors of miRNAs formed by the cleavage of primary miRNAs by DCGR8 and Drosha.

A further example of the use of miRNA to degrade transgene mRNA when using Vector-Intron lentiviral vectors harbouring an inverted transgene cassette, is the use of the 3'UTR sequence encompassed by the VI on the top strand to contain one or more pre-miRs, such that processing of such pre-miRs leads to cleavage of the 3'UTR by Drosha/Pasha. Optionally, the miRNA generated by such pre-miR cassettes could then target other sequences across the transgene mRNA. Again, critically these self-cleaving sequences are removed by splicing out of the VI to generate packaged vRNA, and are therefore not present in the target cell. This concept is presented in Figure 64 and expands on the concept presented in Figures 44 and 45.

Alternatively, the cis-elements may be functional nuclear retention signals such as those in long non-coding RNAs (IncRNAs) such as Metastasis Associated Lung Adenocarcinoma Transcript 1 (MALAT1) (Miyagwa et al. (2012), RNA 18: 738-751), maternally expressed gene 3 (MEG3) (Azam et al. (2019), RNA Biol. 16: 1001-1009), and the SINE-derived nuclear RNA LOcalizatIoN (SIRLION) element (Lubelsky and Ulitsky (2018), Nature 555: 107-111; Lubelsky et al. (2021), EMBO J. 40: e106357).

By utilizing such elements in the LV genome expression cassettes of the invention, the amount of transgene mRNA produced to be available for forming dsRNA with the vRNA can be reduced, minimizing the impact on LV titres. Critically, these elements are only active during LV production because they are lost from the packaged vRNA by splicing-out of the VI.

Hence, these cis-acting element(s) will not be present within the final integrated transgene cassette due to out-splicing of the VI, and therefore transgene mRNA stability and/or localisation in the transduced cell will be unaffected.

The terms "self-destabilisation" and "self-decay" as used in relation to the aforementioned *cis-*acting element(s) refers to the ability of said elements to act upon nucleotides, i.e. transgene mRNA, that comprise sequences encoding said elements. For example, an intron which is inverted with respect to the lentiviral vector genome expression cassette may encode a *cis-*acting element that facilitates the destabilisation or decay of transgene mRNA encoding the intron.

AU-rich elements (AREs) may facilitate the recruitment of RNA-binding proteins that destabilise the mRNA in which they are present, either directly or via the recruitment of additional factors.

Self-cleaving ribozymes are well known in the art (Jimenez, R., M., et al. "Chemistry and Biology of Self-Cleaving Ribozymes." Trends in biochemical sciences vol. 40,11 (2015): 648-661. doi:10.1016/j.tibs.2015.09.001). Ribozymes can be found in diverse genomic contexts in a vast array of organisms wherein they belong to families that are defined by structure and active site. Ribozymes catalyse trans-esterification reactions and typically mediated self-cleavage via general acid-base catalysis. Such self-cleavage reactions result in scission of the nucleotide comprising the ribozyme.

Interfering RNAs are also well known in the art. Interfering RNAs (e.g. miRNAs) may promote cleavage of the target RNA (van den Berg et al. (2008), Biochim Biophys Acta 1779: 668-677).

The terms "self-destabilisation" and "self-decay" as used herein encompasses mechanisms that contribute to the overall quantitative reduction or functional attenuation of target RNA, e.g. through processes such as ribozyme-mediated or enzyme-mediated cleavage.

Nuclear retention signals are RNA sequences that lead to the reduction in transport of the target RNA from the nucleus to the cytoplasm after initial transcription. Target RNAs (such as the transgene mRNA) may further be located to nuclear speckles. Thus, the one or more transgene mRNA nuclear retention signals for use according to the invention facilitates the nuclear retention of mRNA encoding the transgene.

A further advantage of this novel feature is that it also provides a mechanism for transgene repression during LV production, which has previously shown to be an effective way of rescuing LV titres for genomes encoding toxic or 'problematic' transgene proteins.

In some embodiments, the 3' UTR of the inverted transgene expression cassette comprises the vector intron. Thus, one or more transgene mRNA self-destabilisation or self-decay element(s), or one or more transgene mRNA nuclear retentions signal(s), may be present within the 3'UTR sequence, wherein the 3' UTR sequence encompasses the vector intron encoded in antisense orientation with respect to the transgene expression cassette.

In an aspect, the synthetic vector intron comprises one or more transgene mRNA self-destabilizing or self-decay element(s) or one or more transgene mRNA nuclear retention signals. The one or more transgene mRNA self-destabilizing elements(s), self-decay element(s) or nuclear retention signal(s) may be of the same type or a different type.

In one aspect, the one or more transgene mRNA self-destabilization or self-decay element(s) of the invention promotes cleavage of target nucleotides. In another aspect, said cleavage is performed by cellular mediators, preferably the RISC complex.

In a further aspect, the vector intron comprises one or more transgene mRNA self-destabilization or self-decay element(s) selected from a list comprising:
(a) a self-cleaving ribozyme; and/or
(b) an AU-rich element; and/or
(c) an interfering RNA, preferably a miRNA, siRNA or shRNA which targets the transgene mRNA; and/or
(d) a target sequence for an interfering RNA, preferably for a miRNA; and/or
(e) a pre-miR.

In one embodiment, the vector intron comprises one or more transgene mRNA self-destabilization or self-decay element(s) selected from a list comprising:
(a) a self-cleaving ribozyme; and/or
(b) an interfering RNA, preferably a miRNA, siRNA or shRNA which targets the transgene mRNA.

Thus, in one aspect, the one or more transgene mRNA self-destabilization or self-decay element(s) is an interfering RNA as described elsewhere herein.

In some preferred embodiments, the interfering RNA is specific for mRNA encoding the transgene.

One or more pre-miRs can be inserted within the antisense VI sequence, i.e. the inverted VI. miRNAs can be targeted to the transgene mRNA so that any mRNA that does locate to the cytoplasm is a target for miRNA-mediated degradation/cleavage. In this instance, the guide strand is preferably 100% matched, i.e. 100% complementary, to its target. The vRNA will not be targeted by the guide strand. The passenger strand should be mis-matched, i.e. not 100% complementary, to the vRNA sequence, in order to avoid cleavage of the vRNA, should the passenger strand become a legitimate microRNA effector.

In one aspect, the one or more transgene mRNA self-destabilization or self-decay element(s) comprises an interfering RNA, preferably a miRNA, wherein the guide strand of the miRNA does not comprise a mismatch with the target transgene mRNA sequence.

In another aspect, the passenger strand of the miRNA according to the invention imperfectly matches its target vector genome sequence resulting in a central bulge.

In another aspect, the one or more transgene mRNA self-destabilization or self-decay element(s) comprises an interfering RNA, preferably a miRNA, wherein the passenger strand comprises at least one mismatch (suitably, at least two or at least three) - preferably at position 2, 9, 10 or 11 - with its target vector genome sequence.

In an aspect, the invention provides a nucleotide sequence comprising a nucleotide expression cassette for use in a lentiviral vector production cell, wherein the expression cassette comprises a sequence encoding a an intron (or VI) which encodes a miRNA; wherein the miRNA has no mis-match between the guide strand and its target mRNA; and wherein the miRNA targets mRNA corresponding to an inverted transgene according to the invention, but not the vector genome RNA.

The aforementioned transgene mRNA self-destabilization or self-decay element(s) (e.g. miRNA) may comprise a plurality of RNA self-destabilization or self-decay element(s) that may also target a plurality of nucleic acid sequences. Conversely, the aforementioned RNA self-destabilization or self-decay element(s) (e.g. miRNA) may comprise a single RNA self-destabilization or self-decay element that targets a single nucleic acid sequence.

The terms "microRNA" and "miRNA" are used interchangeably herein.

In one embodiment, the VI of the invention comprises any of the features disclosed in Table 11.

In one embodiment, the VI of the invention comprises a sequence as set forth in any of SEQ ID NOs: 209-226.

*Illustrative Vector-Intron sequences, and in combination with embedded 'self-cleaving*/*targeting' element (shown in antisense) for inverted transgene cassettes.*

Illustrative sequences for use according to the invention are provided in Table 11 below:

### Key for the following sequences:

- Sequences are all presented 5' to 3' in vector RNA sense (top strand) configuration, including the encompassed elements that are present within the 3' UTR of the inverted transgene cassette (i.e. 'functionalised', 'self-cleaving', 'self-targeting' elements are shown in anti-sense).
- The Vector-Intron splice donor and splice acceptor core sequences are boxed.
- The Guanosine-Adenosine Rich upstream splicing enhancer (GAR) is indicated in bold/underline.
- The HDV_AG ribozyme is indicated in italics.
- The T3H38 ribozyme is underlined and italicised.
- cPPT sequences are indicated in bold italics.
- The downstream splice enhancer sequence is indicated in bold, underlined italics.
- The sequences highlighted in bold only were replaced with the miRNA target sites.
- The pre-miRNA cassette was inserted in the underlined bracketed region.

**TABLE 11**

| **Description** | **Sequence** | **SEQ ID NO.** |
|---|---|---|
| Parent VI_5.7 | GAAGAAGCGGAGACAGCGACGAAGAGCTCAATCAGAACTAGTCAGA CTCAATCAAGCTTCTCTATCAACCAGGTAAGTATTACATGTAAGAG TCTATGGGACCCTTGATGTTTTCTTTCCCCTTCTTTTTCTAGATAT GGTTAAGTTCAATCGATTGTCATAGGAAGGGGAGAAGTAACAGGGT ACACATATTGACCAAATCAGGGTAATTTTGCATTTGTAATTTTAAA AAATGCTTTCTTCTTTTAATATACTTTTTTGTTTATCTTATTTCTA ATACTTTCCCTAATCTCTTTCTTTCAGGGCAATAATGAGCTAAGCT ACAATGTATCATGCCTAGGCCTCTTTGCACCATTCTAAAGAATAAC | 209 |
| | AGTGATAATTTCTGGGTTAAGGCAATAGCAATATTTCTGCATATAA ATATTTCTGCATATAAATTGTAACTGATGTAAGAGGTTTCATATTG CTAATAGCAGCTACAATCCAGCTACCATTCTGCTTATGCAT[pre- miRNAcassette]TTATTTTATGGTTGGATCCGGGATAAGGCTGG ATTATTCTGAGTCCAAGCTAGGCCCTTTTGCTAATCATGTTCATAC CTCTTATCTTCCTCCCACAGGTGTCCACTCCCAGTTCCTGCAGGCA CGTGAAATTGGCAGTATTCAATCCACAATTTTAAAAGAAAAGGGGG GATTGGGGGGTACAGTGCAGGGGAAAGAATAGTAGACAATAATAGC AACAGACAATACAAACTAAAGAATTACAAAAACAAATTACAAAAAT TCAAAATTTTCGGGTTTCACGTGACGCGTGCCGAAGGAATAGAAGA AGAAGGTGGAGAGAGAGACAGAGAC | |
| VI_5.7 with Ribozymes | GAAGAAGCGGAGACAGCGACGAAGAGCTCAATCAGAACTAGTCAGA CTCAATCAAGCTTCTCTATCAACCAGGTAAGTATTACATGTAAGAG TCTATGGGACCCTTGATGTTTTCTTTCCCCTTCTTTTTCTAGATAT GGTTAAGTTCAATCGATTGTCGTGGCTCTCCCTTAGCCATCCGAGT GGACGTGCGTCCTCCTTCGGATGCCCAGGTCGGACCGCGAGGAGGT GGAGATGCCATGCCGACCCATAGGAAGGGGAGAAGTAACAGGGTAC ACATATTGACCAAATCAGGGTAATTTTGCATTTGTAATTTTAAAAA ATGCTTTCTTCTTTTAATATACTTTTTTGTTTATCTTATTTCTAAT ACTTTCCCTAATCTCTTTCTTTCAGGGCAATAATGAGCTAAGCTAC AATGTATCATGCCTAGGCCTCTTTGCACCATTCTAAAGAATAACAG TGATAATTTCTGGGTTAAGGCAATAGCAATATTTCTGCATATAAAT ATTTCTGCATATAAATTGTAACTGATGTAAGAGGTTTCATATTGCT AATAGCAGCTACAATCCAGCTACCATTCTGCTTATGCAT[pre- miRNAcassette]TTATTTTATGGTTGCGCGTTTCGTCCTATTTG GGACTCGTCAGCTGGATGTACCCGAAGTGGAATCCAGGACGCGCGG ATCCGGGATAAGGCTGGATTATTCTGAGTCCAAGCTAGGCCCTTTT GCTAATCATGTTCATACCTCTTATCTTCCTCCCACAGGTGTCCACT CCCAGTTCCTGCAGGCACGTGAAATTGGCAGTATTCAATCCACAAT TTTAAAAGAAAAGGGGGGATTGGGGGGTACAGTGCAGGGGAAAGAA TAGTAGACAATAATAGCAACAGACAATACAAACTAAAGAATTACAA AAACAAATTACAAAAATTCAAAATTTTCGGGTTTCACGTGACGCGT GCCGAAGGAATAGAAGAAGAAGGTGGAGAGAGAGACAGAGAC | 210 |
| GAR (guanine-adenine rich region) | GAAGAAGCGGAGACAGCGACGAAGA | 211 |
| cPPT variant 1 | AAATTGGCAGTATTCAATCCACAATTTTAAAAGAAAAGGGGGGATT GGGGGGTACAGTGCAGGGGAAAGAATAGTAGACAATAATAGCAACA GACAATACAAACTAAAGAATTACAAAAACAAATTACAAAAATTCAA AATTTTCGGGTTT | 212 |
| cPPT variant 2 | AAATTGGCAGTATTCATCCACAATTTTAAAAGAAAAGGGGGGATTG GGGGGTACAGTGCAGGGGAAAGAATAGTAGACATAATAGCAACAGA CATACAAACTAAAGAATTACAAAAACAAATTACAAAAATTCAAAAT TTTCGGGTTT | 213 |
| HDV AG RZ (antisense) | GGCTCTCCCTTAGCCATCCGAGTGGACGTGCGTCCTCCTTCGGATG CCCAGGTCGGACCGCGAGGAGGTGGAGATGCC | 214 |
| T3H38 RZ (antisense) | GCGCGTTTCGTCCTATTTGGGACTCGTCAGCTGGATGTACCCGAAG TGGAATCCAGGACGCGC | 215 |
| miRNA target insertion site (sequence replaced) | TAAATTGTAACTGATGTAA | 216 |
| miR-17-5p target site (1x; antisense) | CAAAGTGCTTACAGTGCAGGTAGAATT | 217 |
| miR-17-5p target site (3x; antisense) | CAAAGTGCTTACAGTGCAGGTAGAATTTAAATTGTAACAAAGTGCT TACAGTGCAGGTAGAATTCTGATGTAACAAAGTGCTTACAGTGCAG GTAGAATT | 218 |
| miR-20a target site (1x; antisense) | TAAAGTGCTTATAGTGCAGGTAGAATT | 219 |
| miR-20a target site (3x; antisense) | TAAAGTGCTTATAGTGCAGGTAGAATTTAAATTGTAATAAAGTGCT TATAGTGCAGGTAGAATTCTGATGTAATAAAGTGCTTATAGTGCAG GTAGAATT | 220 |
| miR-106a target site (1x; antisense) | AAAAGTGCTTACAGTGCAGGTAGAATT | 221 |
| miR-106a target site (3x; antisense) | AAAAGTGCTTACAGTGCAGGTAGAATTTAAATTGTAAAAAAGTGCT TACAGTGCAGGTAGAATTCTGATGTAAAAAAGTGCTTACAGTGCAG GTAGAATT | 222 |
| miR-GE insert (antisense) [target sequence] | GATATC [TCATAGATTGGACTTGACACTT] GATATCGTAGAGTATG GTCAACCTTACTTGCATAGATTGGGCTTGACACTTCATCTGTGGCT TCACAAGTGTCAAGTCCAATCTATGAAGGCACTGCTGACATTGCTA TCACCATGCAT | 223 |
| miR-GEΔtarget insert (antisense) | GATATCGTAGAGTATGGTCAACCTTACTTGCATAGATTGGGCTTGA CACTTCATCTGTGGCTTCACAAGTGTCAAGTCCAATCTATGAAGGC ACTGCTGACATTGCTATCACCATGCAT | 224 |
| CCR5 miRNA target site | [TCATAGATTGGACTTGACACTT] | 225 |
| miR-GE | GTCAACCTTACTTGCATAGATTGGGCTTGACACTTCATCTGTGGCT TCACAAGTGTCAAGTCCAATCTATGAAGGCACTGCTGAC | 226 |

### RNA interference (RNAi)

Post-transcriptional gene silencing (PTGS) mediated by double-stranded RNA (dsRNA) is a conserved cellular defence mechanism for controlling the expression of foreign genes. It is thought that the random integration of elements such as transposons or viruses causes the expression of dsRNA which activates sequence-specific degradation of homologous single-stranded mRNA or viral genomic RNA. The silencing effect is known as RNA interference (RNAi). RNAi has also been exploited to modulate the expression of a target nucleotide sequence.

RNAi is a biological process in which RNA molecules inhibit gene expression or translation, by inhibiting targeted mRNA molecules (Ralph et al. 2005, Nat. Medicine 11: 429-433). The mechanism of RNAi involves the processing of long dsRNAs into duplexes of about 21-25 nucleotide (nt) RNAs. These products are called small interfering or silencing RNAs (siRNAs) which are the sequence-specific mediators of mRNA degradation.

shRNAs consist of short inverted RNA repeats separated by a small loop sequence. These are rapidly processed by the cellular machinery into 19-22 nt siRNAs, thereby suppressing the target gene expression.

Micro-RNAs (miRNAs) are small (22-25 nucleotides in length) noncoding RNAs that can effectively reduce the translation of target mRNAs by binding to their 3' untranslated region (UTR). Micro-RNAs are a very large group of small RNAs produced naturally in organisms, at least some of which regulate the expression of target genes. Founding members of the microRNA family are let-7 and lin-4. The let-7 gene encodes a small, highly conserved RNA species that regulates the expression of endogenous protein-coding genes during worm development. The active RNA species is transcribed initially as a ~70 nt precursor, which is post-transcriptionally processed into a mature ~21 nt form. Both let-7 and lin-4 are transcribed as hairpin RNA precursors which are processed to their mature forms by Dicer enzyme. Subsequently, the mature forms may be loaded into the RISC complex such that further cleavage of the target mRNAs can occur.

Methods for the design of interfering RNA to modulate the expression of a target nucleotide sequence are well known in the art.

Interfering RNAs may be, for example, a siRNA; a sisiRNA; a tsiRNA; a RNA-DNA chimeric duplex; a tkRNA; a Dicer-substrate dsRNA; a shRNA; a tRNA-shRNA; an aiRNA; a pre-miRNA; a pri-miRNA mimic; a pri-miRNA mimic cluster; a transcriptional gene silencing (TGS); and combinations thereof. Suitably, the interfering RNA for use according to the invention is a siRNA, shRNA or miRNA. Preferably, the interfering RNA for use according to the invention is a miRNA.

### Rev response element (RRE)

The rev-response element (RRE) in HIV-1 is an approximately 350 nucleotide, highly structured, cis-acting RNA element usually essential for viral replication. It is located in the env coding region of the viral genome and is extremely well conserved across different HIV-1 isolates. It is present on all partially spliced and unspliced viral mRNA transcripts, and serves as an RNA framework onto which multiple molecules of the viral protein Rev assemble. The Rev-RRE oligomeric complex mediates the export of these messages from the nucleus to the cytoplasm, where they are translated to produce essential viral proteins and/or packaged as genomes for new virions. The RRE serves as a specific RNA scaffold that coordinates the assembly of a unique homo-oligomeric ribonucleoprotein (RNP) complex to mediate the nuclear export of essential, intron-containing, viral messages. The Rev protein is a transactivating protein that is essential to the regulation of HIV-1 (and other lentiviral) protein expression. A nuclear localization signal is encoded in the *rev* gene, which allows the Rev protein to be localized to the nucleus, where it is involved in the export of unspliced and incompletely spliced mRNAs.

As described herein, the present inventors surprisingly found that inclusion of a Vector-Intron negated the need for the RRE to be present in the lentiviral vector genome expression cassette.

As such, in an aspect, the nucleotide sequence comprising a lentiviral vector genome expression cassette according to the invention does not comprise a rev-response element.

In one aspect the rev-response element may have been removed, or deleted, or otherwise inactivated in the nucleotide sequence. Suitable methods for such removal, deletion or inactivation will be known to those of skill in the art.

### Interfering RNA

Introns within the transgene expression cassette (e.g. the intron within the EF1α promoter if employed as the internal transgene promoter) provide a boost to expression in certain target cells. However, such additional introns within the transgene expression cassette may be spliced-out during transcription. A way of ensuring transgene intron retention within vRNA is to invert the transgene cassette so that the intron within the transgene expression cassette is not recognised as such because it is in the anti-sense direction with respect to the retroviral vector genome expression cassette.

Accordingly, the transgene expression cassette may be inverted with respect to the retroviral vector genome expression cassette. In other words, the internal transgene promoter and transgene sequences oppose the retroviral vector genome cassette promoter such that the retroviral vector genome and transgene are in opposed transcriptional orientations. Thus, the transgene expression cassette may be in the antisense orientation (i.e. encoded on the antisense strand / the bottom strand) with respect to the retroviral vector genome expression cassette.

Alternatively, the transgene expression cassette may be in the forward orientation with respect to the retroviral vector genome expression cassette. In other words, the internal transgene promoter and transgene sequences are in the same orientation as the retroviral vector genome cassette promoter such that the retroviral vector genome and transgene are in the same transcriptional orientation. Thus, the transgene expression cassette may be in the sense orientation (i.e. encoded on the sense strand / on the top strand) with respect to the retroviral vector genome expression cassette.

Hence, the transgene expression cassette can be inverted or non-inverted (i.e. in the forward orientation) with respect to the retroviral vector genome cassette.

If a tissue specific promoter is utilized as the internal transgene promoter that is not/minimally active during retroviral vector production then the inverted transgene approach requires no further considerations. However, should the transgene promoter generate sufficient levels of transgene mRNA during retroviral vector production then the possibility of generating long dsRNA products via vRNA:mRNA annealing increases, and this will trigger innate dsRNA sensing pathways, such as those involving oligoadenylate synthetase-ribonuclease L (OAS-RNase L), protein kinase R (PKR), and interferon (IFN)/ melanoma differentiation-associated protein 5 (MDA-5). Whilst a number of these pathways are likely to be (partly) defective in HEK293(T) cells (Ferreira, C., B., et. al., Mol Ther Methods Clin Dev. (2019);17:209-219.), here the inventors provide evidence that generation of cytoplasmic dsRNA results in suppression of *de novo* protein synthesis (see Figure 2).

The present inventors have surprisingly found that RNAi can be employed in retroviral vector production cells to suppress the expression of the NOI (i.e. transgene) during retroviral vector production in order to minimize unwanted effects of the transgene protein during vector production and/or to rescue titres of retroviral vectors harbouring an actively transcribed inverted transgene cassette. The inventors surprisingly found that RNAi can be employed during vector production to minimize and/or eliminate mRNA encoding the transgene but not vector genome RNA (vRNA) required for packaging. Thus, the amount of transgene mRNA produced to be available for forming dsRNA with the vRNA is reduced, minimizing the impact on titres of retroviral vectors harbouring an actively transcribed inverted transgene cassette. This feature also provides a mechanism for transgene repression during LV production, which has previously shown to be an effective way of rescuing titres for genomes encoding toxic or 'problematic' transgene proteins.

In other words, the use of interfering RNA(s) specific for the transgene mRNA provides a mechanism for avoiding *de novo* protein synthesis inhibition and/or the consequences of other dsRNA sensing pathway and enables rescue of inverted transgene retroviral vector titres. The interfering RNA is targeted to the transgene mRNA so that any mRNA that does locate to the cytoplasm is a target for RNAi-mediated degradation and/or cleavage, preferably cleavage.

Preferably, the interfering RNA(s) employed result in cleavage of mRNA encoding the transgene in order to minimize and/or eliminate the formation of dsRNA. Suitably, the interfering RNA(s) target the mRNA encoding the transgene for cleavage. The interfering RNA(s) may target the mRNA encoding the transgene for cleavage by the RNA-induced silencing complex (RISC).

As used herein, the term "is specific for" means that the interfering RNA preferentially binds to mRNA encoding the transgene over an mRNA molecule which does not encode the transgene. Thus, the interfering RNA targets the mRNA encoding the transgene.

As used herein, the term "interfering RNA" means an RNA which is capable of mediating RNA interference (RNAi).

Interfering RNAs may be, for example, a siRNA; a sisiRNA; a tsiRNA; a RNA-DNA chimeric duplex; a tkRNA; a Dicer-substrate dsRNA; a shRNA; a tRNA-shRNA; an aiRNA; a pre-miRNA; a pri-miRNA mimic; a pri-miRNA mimic cluster; and combinations thereof.

The interfering may be synthetic. Synthetic interfering RNAs are suitable for use by transient co-transfection during lentiviral vector production, i.e. may be provided *in trans* to the viral vector genome expression cassette in a retroviral vector production cell.

Suitably, the interfering RNA may be provided *in cis* to the viral vector genome expression cassette in a lentiviral vector production cell, i.e. the vector intron may comprise the interfering RNA. Examples of interfering RNAs which maybe provided *in cis* include a siRNA; a shRNA; a tRNA-shRNA; a pre-miRNA; a pri-miRNA mimic; a pri-miRNA mimic cluster; and combinations thereof.

Preferably, the interfering RNA is siRNA, shRNA or miRNA. Preferably, the interfering RNA is shRNA or miRNA. Preferably, the interfering RNA for use according to the invention is a miRNA.

In some embodiments, the guide strand of the interfering RNA (preferably, miRNA) is fully complementary to the target sequence of the transgene mRNA (Figure 6). In this instance, the guide strand is 100% matched, i.e. 100% complementary, to its target. Thus, the guide strand may target the RISC complex to the target sequence of the mRNA encoding the transgene (i.e. transgene mRNA) Watson-Crick base pairing. The vRNA is not targeted by the guide strand in order to avoid degradation and/or cleavage of vRNA required for packaging.

In one embodiment, the guide strand of the interfering RNA, preferably miRNA, does not comprise a mismatch with the target sequence within the mRNA encoding the transgene.

As used herein, the term "mismatch" refers to the presence of an uncomplimentary base. Thus, a "mismatch" refers to an uncomplimentary base in the guide strand or passenger strand which is not capable of Watson-Crick base pairing with the complementary sequence within the mRNA encoding the transgene or vRNA, respectively.

Preferably, when employing the interfering RNA to target mRNA encoding the transgene derived from an inverted transgene cassette, the interfering RNA (preferably miRNA) is designed such that the passenger strand is mismatched (i.e. not 100% complementary) to its complementary sequence in the vRNA (Figure 6). In this way, should the passenger strand be used as the guide strand in a RISC, it will not lead to cleavage of the vRNA, which would otherwise lead to reduction in vRNA available for packaging.

In some embodiments, the interfering RNA (preferably miRNA) comprises a passenger strand which comprises at least one mismatch (suitably, at least two, or at least three) - preferably at position 2, 9, 10, or 11 of the passenger stand - with its complimentary sequence within the RNA genome of the retroviral vector.

In another aspect, the passenger strand of the interfering RNA (preferably miRNA) imperfectly matches its target vector genome sequence resulting in a central bulge.

In some embodiments, the set of nucleic acid sequences comprises multiple nucleic acid sequences encoding a plurality of interfering RNAs specific for multiple target nucleotide sequences. Preferably, each interfering RNA is specific for a different target nucleotide sequence.

The interfering RNA(s) can be provided *in trans* or *in cis* during lentiviral vector production. Cis elements are present on the same molecule of DNA as the gene they affect whereas *trans* elements can affect genes distant from the gene from which they were transcribed.

Thus, an interfering RNA expression cassette may be co-expressed with lentiviral vector components during lentiviral vector production. The interfering RNA is an interfering RNA as described herein. Thus, the interfering RNA targets the mRNA encoding the transgene, leading to mRNA degradation and concomitant reduction in dsRNA and transgene expression. Such an interfering RNA expression cassette may be easily constructed by those skilled in the art, for example driven by a U6 pol-Ill promoter or tRNA promoter. The target of the interfering RNA may be the sequence of the 3' UTR of the transgene mRNA encompassed by the VI.

In some alternative embodiments, the lentiviral vector genome expression cassette further comprises a vector intron. Suitably, the vector intron comprises the nucleic acid sequence encoding the interfering RNA. Thus, the interfering RNA may be provided in *cis* during lentiviral vector production.

Inverted transgene expression cassettes are able to retain an intron (herein termed "vector intron" (VI)) within the transgene cassette. By contrast, in non-inverted cassettes the transgene cassette would be spliced out together with the VI, which is undesirable. Thus, the VI is provided in the forward orientation (i.e. on the sense strand) with respect to the retroviral vector genome expression cassette. This permits out-splicing of the VI during transcription of the lentiviral vector genome. The inverted transgene expression cassettes of the invention may make use of the VI to reduce transgene mRNA expression during vector production, since an interfering RNA which is specific for the mRNA encoding the transgene can be inserted within the 3' UTR of the inverted transgene expression cassette, such that the interfering RNA is also encompassed by the VI on the sense strand. Thus, transgene mRNA is destabilized and/or degraded within vector production cells. By contrast, upon splicing-out of the VI, the interfering RNA is also removed from the packaged genome of the lentiviral vector (e.g. vRNA) such that the transgene mRNA will lack the interfering RNA in transduced cells (see Figure 44). As such, critically, the interfering RNA are only active during lentiviral vector production because they are lost from the packaged vRNA by splicing-out of the VI.

One or more interfering RNAs can be inserted within the antisense VI sequence, i.e. the inverted VI.

By way of illustrative example, the interfering RNA may be one or more 'self-cleaving' miRNAs that are located within the 3' UTR of the transgene expression cassette. The one or more miRNAs (i.e. pre-miRNAs) are therefore cleaved from the 3' UTR of the mRNA encoding the transgene (thus removing the polyA tail of the mRNA, leading to destabilisation of the mRNA encoding the transgene), and then are processed by DROSHA/Dicer into mature miRNAs and loaded into the RISC to target sequences within the transgene mRNA such that further cleavage can occur. dsRNA fragments are loaded into RISC with each strand having a different fate based on the asymmetry rule phenomenon, the selection of one strand as the guide strand over the other based on thermodynamic stability. The newly generated miRNA or siRNA act as single-stranded guide sequences for RISC to target mRNA for degradation. The strand with the less thermodynamically stable 5' end is selected by the protein Argonaute and integrated into RISC. This strand is known as the guide strand and targets mRNA for degradation. The other strand, known as the passenger strand, is degraded by RISC.

By 'self-cleaving' it is meant that, since the miRNA(s) are located within the 3' UTR of the mRNA encoding the transgene and target the mRNA encoding the transgene, the miRNA(s) are self-targeting for cleavage.

The interfering RNA may be specific for a sequence within the 5' UTR and/or coding-region and/or 3'UTR of the transgene expression cassette (i.e. the 5' UTR and/or coding-region and/or 3'UTR of the transgene expression cassette comprises a target nucleotide sequence). Suitably, the target nucleotide sequence may be within the 5' UTR of the transgene expression cassette. Alternatively, the target nucleotide sequence may be within the 3' UTR. If a plurality of interfering RNAs are employed, the target nucleotide sequences may be within the 3' UTR and the 5' UTR of the transgene expression cassette.

In some embodiments, the 3' UTR or the 5' UTR of the transgene expression cassette comprises at least one target nucleotide sequence.

In some embodiments, the transgene expression cassette is genetically engineered to comprise at least one target nucleotide sequence within the 3' UTR or 5' UTR. The at least one target nucleotide sequence may be at least one predetermined heterologous target nucleotide sequence for which efficient interfering RNAs are already available.

Molecular cloning methods to introduce a nucleotide sequence into a target sequence are known in the art. For example, conventional techniques of molecular biology (described elsewhere herein) may be employed.

In some embodiments, the interfering RNA is specific for the at least one target nucleotide sequence.

As used herein, the term "target nucleotide sequence" means a sequence within the transgene expression cassette to which the interfering RNA binds. Suitably, the target nucleotide sequence is 100% complementary to the guide strand of the interfering RNA, which is preferably a miRNA.

As described herein, the interfering RNA may be provided in *cis* during retroviral vector production.

Accordingly, in a further aspect, the invention provides an expression cassette encoding a lentiviral vector genome comprising:
(i) a transgene expression cassette; and
(ii) a vector intron comprising at least one interfering RNA as described herein.

In a further aspect, the invention provides a retroviral vector genome comprising a transgene expression cassette and a vector intron, wherein the vector intron comprises at least one interfering RNA as described herein.

In some embodiments, the transgene expression cassette is inverted with respect to the retroviral vector genome expression cassette.

In a further aspect, the invention provides a nucleotide sequence comprising the expression cassette of the invention.

An illustrative example of the use of one or more interfering RNAs to target transgene mRNA for cleavage by the RISC complex in order to [1] reduce transgene expression during retroviral vector production and [2] avoid the production of dsRNA when employing transgene cassettes containing active transcription units that are inverted with respect to the vector genome cassette, and thus would otherwise lead to dsRNA sensing pathways detrimental to vector production (see Figure 50) is provided below.

### Illustrative example

The illustrative example of a process flow of implementing this approach can be summarized as follows:
*1. Identify one or more target sites within the transgene mRNA sequence to enable efficient mRNA cleavage(s), leading to reduced steady-state levels of mRNA, reduction of transgene protein expression and rescue of titres (for inverted transgenes).*

This can be achieved in a number of ways. Firstly, the transgene mRNA sequence can be *in silico* screened for potential sites that are predicted to be good targets for microRNA (such services are commercially available, for example:
- iScore (http://www.med.nagoya-u.ac.jp/neurogenetics/i_Score/i_score.html);
- Invitrogen (http://rnaidesigner.invitrogen.com/rnaiexpress/design.do);
- Thermo Fisher (http://www.thermoscientificbio.com/design-center/);
- siSPOTR (https://sispotr.icts.uiowa.edu/sispotr/tools/sispotr).

This process involves:
I. Target sequence analysis
II. Thermodynamic profiling of guide strand
III. Addition of passenger strand mis-matches if required
IV. Assessment of microRNA secondary structure (e.g. via m-fold)
V. Selected siRNA candidates tested for off target effects by blasting (e.g. NCBI BLAST) against human transcriptome using the following settings: word size - 7; threshold 1000; mismatch penalty -1 or 0; gap open 3; extension 2. (Such loose criteria ensure an effective search for siRNA off-targets, unlike using the default BLAST settings.)

Several (for example, up to 10) siRNAs or shRNAs can be generated and screened against a simple transgene expression cassette within the production cells, thus identifying one or more siRNA/shRNAs that can be used in the invention.

Alternatively, a predetermined heterologous target sequence for which there is already available efficient siRNA/shRNAs can be cloned within the 5' or 3' UTRs of the transgene cassette and empirically tested for mRNA cleavage and transgene protein knock-down by supplying these siRNA/shRNAs in co-transfection experiments.

Alternatively, the production cell may be characterized for endogenous microRNAs that are highly expressed constitutively, and/or under vector production conditions (for example microRNAs upregulated by sodium butyrate induction). The target sequence(s) of these endogenous microRNAs can be cloned into the 5' or 3' UTRs of the transgene cassette and empirically tested for mRNA cleavage and transgene protein knock-down.

Multiple target sites of one or more microRNAs can optionally be cloned into 5' and/or 3' UTRs of the transgene cassette.

It is desirable for the guide strand of the microRNA to be designed such that the resultant active RISC mediates transgene mRNA cleavage i.e. the guide strand is fully complementary to the target sequence. Optionally, microRNAs can be designed for use with inverted transgene cassettes such that the passenger strand is mis-matched in order to minimize any possible cleavage of vRNA should the passenger strand be loaded into a RISC.

### 2. Choice of microRNA modality (see Figure 51)

The siRNA/shRNA/miRNA identified to induce the desired levels of transgene repression and/or mRNA cleavage can be used directly in co-transfection production of retroviral vectors. Alternatively, the interfering RNA can be designed as part of an expression cassette in order to be *de novo* transcribed during vector production, for example, by a polymerase-Ill promoter such as U6 or a tRNA promoter. Thus, a plasmid encoding the miRNA cassette can be co-transfected into the production cell together with vector component plasmids. The miRNA plasmid may contain multiple single miRNA expression cassettes, or a single expression cassette encoding multiple tandem miRNAs processed from a single transcript. Alternatively, the miRNA expression cassette(s) may be stably integrated into the host cell DNA or stably maintained as an episome.

Alternatively, such miRNA expression cassette(s) may be cloned into the vector genome or packaging plasmids in *cis.*

### 3. Optimisation of vector production

Depending on the mode of microRNA being employed for the implementation of transgene mRNA knock-down during vector production, the process can be optimized to achieve the maximal effect i.e. efficient transgene repression and/or recovery in titres of vectors containing an inverted transgene cassette.

For transient production utilizing siRNA/shRNA/miRNA, this will involve empirically testing different amounts and ratios of interfering RNA effectors relative to plasmids encoding vector components and transfection reagent, as well as harvest times and/or sodium butyrate induction levels/timings.

For the approach where the miRNA cassette(s) is inserted in *cis* on one or more plasmids encoding vector components, the number, position and orientation of the miRNA(s) should be empirically tested.

For development of stable a cell line expressing miRNA(s), the screening process will empirically test and identify clones that have low transgene levels and high vector titres.

### Use of an interfering RNA

In a further aspect, the invention provides the use of a nucleic acid sequence encoding an interfering RNA as described herein for repressing expression of a transgene and/or increasing retroviral titre during retroviral vector production.

In a further aspect, the invention provides the use of a nucleic acid sequence encoding an interfering RNA as described herein for repressing expression of a transgene and/or increasing retroviral titre in a retroviral vector production cell.

Suitably, the nucleic acid sequence encoding an interfering RNA as described herein is used in conjunction with nucleotide sequences encoding retroviral vector components. In other words, the nucleic acid sequence encoding an interfering RNA may be used as part of a set of nucleic acid sequences as described herein.

Titres of vectors containing actively expressed inverted transgene cassettes may be negatively impacted due to the triggering of innate dsRNA sensing pathways within the cell leading to loss of *de novo* protein synthesis as described above. Thus, titres of vectors containing actively expressed inverted transgene cassettes may be enhanced by the use of an interfering RNA as described herein to target the transgene mRNA during retroviral vector production, thereby preventing the triggering of innate dsRNA sensing pathways and the loss of *de novo* protein synthesis.

In addition, certain transgenes may be toxic to the cell or have other deleterious properties when expressed in a cell. Thus, the use of an interfering RNA as described herein to target the transgene mRNA during retroviral vector production may further boost titres of vectors harbouring such a transgene.

Suitably, titres of vectors containing actively expressed inverted transgene cassettes may be restored to the titre levels seen during production of a retroviral vector harbouring a reporter gene construct (e.g. a GFP transgene) by the use of an interfering RNA as described herein to target the transgene mRNA during retroviral vector production.

Accordingly, the use of an interfering RNA as described herein may enhance the titre of a retroviral vector containing an actively expressed inverted transgene cassette during retroviral vector production relative to retroviral vector production of the corresponding retroviral vector in the absence of an interfering RNA as described herein. Thus, production of a retroviral vector containing an actively expressed inverted transgene cassette in the presence of an interfering RNA as described herein enhances retroviral vector titre relative to retroviral vector production of the corresponding retroviral vector in the absence of an interfering RNA as described herein. The present invention is particularly advantageous for enhancing the titre of retroviral vectors harbouring an actively transcribed inverted transgene cassette and a transgene which is, for example, toxic to the cell.

A suitable assay for the measurement of retroviral vector titre is as described herein. Suitably, the retroviral vector production involves co-expression of said interfering RNA with vector components including gag, env, rev and the genome of the retroviral vector. Alternatively, the retroviral vector production involves provision of said interfering RNA in *cis.*

In some embodiments, the use of an interfering RNA as described herein may increase retroviral vector titre of a retroviral vector containing an actively expressed inverted transgene cassette during retroviral vector production by at least 30% relative to retroviral vector production of the corresponding retroviral vector in the absence of an interfering RNA as described herein. Suitably, the use of an interfering RNA as described herein may increase retroviral vector titre of a retroviral vector containing an actively expressed inverted transgene cassette during retroviral vector production by at least 35% (suitably at least 40%, 45%, 50%, 60%, 70%, 100%, 150%, 200%, 250%, 300%, 350%, 400%, 450%, 500%, 550%, 600%, 650%, 700%, 750%, 800%, 850%, 900%, 950% or 1000%) relative to retroviral vector production of the corresponding retroviral vector in the absence of an interfering RNA as described herein.

As described above, expression of the transgene protein during retroviral vector production may have unwanted effects on vector virion assembly, vector virion activity, process yields and/or final product quality. Therefore, it is desirable to repress expression of the transgene in viral vector production cells. Suitably, the translation of the mRNA encoding the transgene may be repressed.

Repression or prevention of the translation of the NOI (i.e. transgene) is to be understood as alteration of the amount of the product (e.g. transgene protein) encoded by the NOI that is translated during viral vector production in comparison to the amount translated in the absence of the interfering RNA as described herein at the equivalent time point.

In one embodiment, expression of the transgene is repressed or prevented in a retroviral vector production cell.

The expression of the protein from the transgene at any given time during vector production may be reduced to 90% (suitably, to 80%, 70%, 60%, 50%, 40%, 30%, 20%, 10%, 5%, 4%, 3%, 2%, 1%, 0.5% or 0.1%) of the amount expressed in the absence of the interfering RNA as described herein at the same time-point in vector production.

The expression of the protein from the transgene at any given time during vector production may be reduced to less than 90% (suitably, to less than 80%, 70%, 60%, 50%, 40%, 30%, 20%, 10%, 5%, 4%, 3%, 2%, 1%, 0.5% or 0.1%) of the amount expressed in the absence of the interfering RNA as described herein at the same time-point in vector production.

In one embodiment, translation of the transgene is repressed or prevented in a retroviral vector production cell.

Preventing the expression of the protein from the transgene is to be understood as reducing the amount of the protein that is expressed to substantially zero (suitably, to zero).

The translation of the transgene at any given time during vector production may be reduced to 90% (suitably, to 80%, 70%, 60%, 50%, 40%, 30%, 20%, 10%, 5%, 4%, 3%, 2%, 1%, 0.5% or 0.1%) of the amount translated in the absence of the interfering RNA as described herein at the same time-point in vector production.

The translation of the NOI at any given time during vector production may be reduced to less than 90% (suitably, to less than 80%, 70%, 60%, 50%, 40%, 30%, 20%, 10%, 5%, 4%, 3%, 2%, 1%, 0.5% or 0.1%) of the amount translated in the absence of the interfering RNA as described herein at the same time-point in vector production.

Preventing the translation of the NOI is to be understood as reducing the amount of translation to substantially zero (suitably, to zero).

Methods for the analysis and/or quantification of the translation of a transgene are well known in the art.

A protein product from lysed cells may be analysed using methods such as SDS-PAGE analysis with visualisation by Coomassie or silver staining. Alternatively a protein product may be analysed using Western blotting or enzyme-linked immunosorbent assays (ELISA) with antibody probes which bind the protein product. A protein product in intact cells may be analysed by immunofluorescence.

### RNA Splicing

The present invention, as disclosed herein, may be combined with major splice donor (MSD) site knock out lentiviral vector genomes. The invention may employ lentiviral vector genomes in which the major splice donor site, and optionally the cryptic splice donor site 3' to the major splice donor site, are inactivated.

Thus, in some embodiments, the major splice donor site in the genome of the lentiviral vector, and optionally the cryptic splice donor site 3' to the major splice donor site in the genome of the lentiviral vector are inactivated.

In some embodiments, the inactivated major splice donor site has the sequence set forth in SEQ ID NO: 4.

Suitable inactivated splice sites for use according to the present invention are described in WO 2021/160993 and incorporated herein by reference.

RNA splicing is catalysed by a large RNA-protein complex called the spliceosome, which is comprised of five small nuclear ribonucleoproteins (snRNPs). The borders between introns and exons are marked by specific nucleotide sequences within a pre-mRNA, which delineate where splicing will occur. Such boundaries are referred to as "splice sites". The term "splice site" refers to polynucleotides that are capable of being recognized by the splicing machinery of a eukaryotic cell as suitable for being cut and/or ligated to another splice site.

Splice sites allow for the excision of introns present in a pre-mRNA transcript. Typically, the 5' splice boundary is referred to as the "splice donor site" or the "5' splice site," and the 3' splice boundary is referred to as the "splice acceptor site" or the "3' splice site". Splice sites include, for example, naturally occurring splice sites, engineered or synthetic splice sites, canonical or consensus splice sites, and/or non-canonical splice sites, for example, cryptic splice sites.

Splice acceptor sites generally consist of three separate sequence elements: the branch point or branch site, a polypyrimidine tract and the acceptor consensus sequence. The branch point consensus sequence in eukaryotes is YNYTRAC (where Y is a pyrimidine, N is any nucleotide, and R is a purine). The 3' acceptor splice site consensus sequence is YAG (where Y is a pyrimidine) (see, e.g., Griffiths et al., eds., Modern Genetic Analysis, 2nd edition, W.H. Freeman and Company, New York (2002)). The 3' splice acceptor site typically is located at the 3' end of an intron.

The terms "canonical splice site" or "consensus splice site" may be used interchangeably and refer to splice sites that are conserved across species.

Consensus sequences for the 5' donor splice site and the 3' acceptor splice site used in eukaryotic RNA splicing are well known in the art. These consensus sequences include nearly invariant dinucleotides at each end of the intron: GT at the 5' end of the intron, and AG at the 3' end of an intron.

The canonical splice donor site consensus sequence may be (for DNA) AG/GTRAGT (where A is adenosine, T is thymine, G is guanine, C is cytosine, R is a purine and "/" indicates the cleavage site). This conforms to the more general splice donor consensus sequence MAGGURR described herein. It is well known in the art that a splice donor may deviate from this consensus, especially in viral genomes where other constraints bear on the same sequence, such as secondary structure for example within a vRNA packaging region. Non-canonical splice sites are also well known in the art, albeit they occur rarely compared to the canonical splice donor consensus sequence.

By "major splice donor site" is meant the first (dominant) splice donor site in the viral vector genome, encoded and embedded within the native viral RNA packaging sequence typically located in the 5' region of the viral vector nucleotide sequence.

In one embodiment, the lentiviral vector genome does not contain an active major splice donor site, that is splicing does not occur from the major splice donor site, and splicing activity from the major splice donor site is ablated.

The major splice donor site is located in the 5' packaging region of a lentiviral genome.

In the case of the HIV-1 virus, the major splice donor consensus sequence is (for DNA) TG/GTRAGT (where A is adenosine, T is thymine, G is guanine, C is cytosine, R is a purine and "/" indicates the cleavage site).

In one embodiment, the splice donor region, i.e. the region of the vector genome which comprises the major splice donor site prior to mutation, may have the following sequence:
GGGGCGGCGACTGGTGAGTACGCCAAAAAT (SEQ ID NO: 1)

In one embodiment, the mutated splice donor region may comprise the sequence:
GGGGCGGCGACTGCAGACAACGCCAAAAAT (SEQ ID NO: 2 - MSD-2KO)

In one embodiment, the mutated splice donor region may comprise the sequence:
GGGGCGGCGAGTGGAGACTACGCCAAAAAT (SEQ ID NO: 3 - MSD-2KOv2)

In one embodiment, the mutated splice donor region may comprise the sequence:
GGGGAAGGCAACAGATAAATATGCCTTAAAAT (SEQ ID NO: 4 - MSD-2KOm5)

In one embodiment, prior to modification the splice donor region may comprise the sequence:
GGCGACTGGTGAGTACGCC (SEQ ID NO: 5)

This sequence is also referred to herein as the "stem loop 2" region (SL2). This sequence may form a stem loop structure in the splice donor region of the vector genome. In one aspect of the invention this sequence (SL2) may have been deleted from the nucleotide sequence according to the invention as described herein.

As such, the invention encompasses the use of a lentiviral vector genome that does not comprise SL2. The invention encompasses the use of a lentiviral vector genome that does not comprise a sequence according to SEQ ID NO: 5.

In one aspect of the invention the major splice donor site may have the following consensus sequence, wherein R is a purine and "/" is the cleavage site:
TG/GTRAGT

In one aspect, R may be guanine (G).

In one aspect of the invention, the major splice donor and cryptic splice donor region may have the following core sequence, wherein "/" are the cleavage sites at the major splice donor and cryptic splice donor sites:
/GTGA/GTA.

In one aspect of the invention the MSD-mutated vector genome may have at least two mutations in the major splice donor and cryptic splice donor 'region' (/GTGA/GTA), wherein the first and second 'GT' nucleotides are the immediately 3' of the major splice donor and cryptic splice donor nucleotides respectively

In one aspect of the invention the major splice donor consensus sequence is CTGGT. The major splice donor site may contain the sequence CTGGT.

In one aspect the nucleotide sequence encoding the lentiviral vector genome, prior to inactivation of the splice sites, comprises the sequence as set forth in any of SEQ ID NOs: 1, 5 and/or the sequence TG/GTRAGT, CTGGT, TGAGT and/or /GTGA/GTA.

In one aspect the nucleotide sequence comprises an inactivated major splice donor site which would otherwise have a cleavage site between nucleotides corresponding to nucleotides 13 and 14 of SEQ ID NO: 1.

According to the invention as described herein, the nucleotide sequence encoding the lentiviral vector genome also contains an inactive cryptic splice donor site. In one aspect the nucleotide sequence does not contain an active cryptic splice donor site adjacent to (3' of) the major splice donor site, that is to say that splicing does not occur from the adjacent cryptic splice donor site, and splicing from the cryptic splice donor site is ablated.

The term "cryptic splice donor site" refers to a nucleic acid sequence which does not normally function as a splice donor site or is utilised less efficiently as a splice donor site due to the adjacent sequence context (e.g. the presence of a nearby 'preferred' splice donor), but can be activated to become a more efficient functioning splice donor site by mutation of the adjacent sequence (e.g. mutation of the nearby 'preferred' splice donor).

In one aspect the cryptic splice donor site is the first cryptic splice donor site 3' of the major splice donor.

In one aspect the cryptic splice donor site is within 6 nucleotides of the major splice donor site on the 3' side of the major splice donor site. Preferably the cryptic splice donor site is within 4 or 5, preferably 4, nucleotides of the major splice donor cleavage site.

In one aspect of the invention the cryptic splice donor site has the consensus sequence TGAGT.

In one aspect the inactivated cryptic splice donor site which would otherwise have a cleavage site between nucleotides corresponding to nucleotides 17 and 18 of SEQ ID NO: 1.

In one aspect of the invention the major splice donor site and/or adjacent cryptic splice donor site contain a "GT" motif. In one aspect of the invention both the major splice donor site and adjacent cryptic splice donor site contain a "GT" motif which is mutated. The mutated GT motifs may inactivate splice activity from both the major splice donor site and adjacent cryptic splice donor site. An example of such a mutation is referred to herein as "MSD-2KO".

In one aspect the splice donor region may comprise the following sequence:
CAGACA

For example, in one aspect the mutated splice donor region may comprise the following sequence:
GGCGACTGCAGACAACGCC (SEQ ID NO: 6)

A further example of an inactivating mutation is referred to herein as "MSD-2KOv2".

In one aspect the mutated splice donor region may comprise the following sequence:
GTGGAGACT

For example, in one aspect the mutated splice donor region may comprise the following sequence:
GGCGAGTGGAGACTACGCC (SEQ ID NO: 7)

For example, in one aspect the mutated splice donor region may comprise the following sequence:
AAGGCAACAGATAAATATGCCTT (SEQ ID NO: 8)

In one aspect the stem loop 2 region as described above may be deleted from the splice donor region, resulting in inactivation of both the major splice donor site and the adjacent cryptic splice donor site. Such a deletion is referred to herein as "ΔSL2".

A variety of different types of mutations can be introduced into the nucleic acid sequence in order to inactivate the major and adjacent cryptic splice donor sites.

In one aspect the mutation is a functional mutation to ablate or suppress splicing activity in the splice region. The nucleotide sequence may contain a mutation or deletion in any of the nucleotides in any of SEQ ID NOs: 1, 5 and/or the sequence TG/GTRAGT, CTGGT, TGAGT and/or /GTGA/GTA. Suitable mutations will be known to one skilled in the art, and are described herein.

For example, a point mutation can be introduced into the nucleic acid sequence. The term "point mutation," as used herein, refers to any change to a single nucleotide. Point mutations include, for example, deletions, transitions, and transversions; these can be classified as nonsense mutations, missense mutations, or silent mutations when present within protein coding sequence. A "nonsense" mutation produces a stop codon. A "missense" mutation produces a codon that encodes a different amino acid. A "silent" mutation produces a codon that encodes either the same amino acid or a different amino acid that does not alter the function of the protein. One or more point mutations can be introduced into the nucleic acid sequence comprising the cryptic splice donor site. For example, the nucleic acid sequence comprising the cryptic splice site can be mutated by introducing two or more point mutations therein.

At least two point mutations can be introduced in several locations within the nucleic acid sequence comprising the major splice donor and cryptic splice donor sites to achieve attenuation of splicing from the splice donor region. In one aspect the mutations may be within the four nucleotides at the splice donor cleavage site; in the canonical splice donor consensus sequence this is A¹G²/G³T⁴, wherein "/" is the cleavage site. It is well known in the art that a splice donor cleavage site may deviate from this consensus, especially in viral genomes where other constraints bear on the same sequence, such as secondary structure for example within a vRNA packaging region. It is well known that the G³T⁴ dinucleotide is generally the least variable sequence within the canonical splice donor consensus sequence, and mutations to the G³ and or T⁴ will most likely achieve the greatest attenuating effect. For example, for the major splice donor site in HIV-1 viral vector genomes this can be T¹G²/G³T⁴, wherein "/" is the cleavage site. For example, for the cryptic splice donor site in HIV-1 viral vector genomes this can be G¹A²/G³T⁴, wherein "/" is the cleavage site. Additionally, the point mutation(s) can be introduced adjacent to a splice donor site. For example, the point mutation can be introduced upstream or downstream of a splice donor site. In aspects where the nucleic acid sequence comprising a major and/or cryptic splice donor site is mutated by introducing multiple point mutations therein, the point mutations can be introduced upstream and/or downstream of the cryptic splice donor site.

As described herein, the nucleotide sequence encoding the RNA genome of the lentiviral vector for use according to the invention may optionally further comprise a mutation in a cryptic splice donor site within the SL4 loop of the packaging sequence. In one aspect a GT dinucleotide in said cryptic splice donor site within the SL4 loop of the packaging sequence is mutated to GC.

In one aspect, the nucleotide sequence encoding the lentiviral vector genome may be suitable for use in a lentiviral vector in a U3 or tat-independent system for vector production. As described herein, 3^{rd} generation lentiviral vectors are U3/tat-independent, and the nucleotide sequences according to the present invention may be used in the context of a 3^{rd} generation lentiviral vector. In one aspect of the invention tat is not provided in the lentiviral vector production system, for example tat is not provided in trans. In one aspect the cell or vector or vector production system as described herein does not comprise the tat protein. In one aspect of the invention HIV-1 U3 is not present in the lentiviral vector production system, for example HIV-1 U3 is not provided in cis to drive transcription of vector genome expression cassette.

In one aspect the major splice donor site in the lentiviral vector genome is inactivated and the cryptic splice donor site 3' to the major splice donor site is inactivated, and said nucleotide sequence is for use in a tat-independent lentiviral vector.

In one aspect the major splice donor site in the RNA genome of the lentiviral vector is inactivated and the cryptic splice donor site 3' to the major splice donor site is inactivated, and said nucleotide sequence is produced in the absence of tat.

In one aspect the major splice donor site in the RNA genome of the lentiviral vector is inactivated and the cryptic splice donor site 3' to the major splice donor site is inactivated, and said nucleotide sequence has been transcribed independently of tat.

In one aspect the major splice donor site in the RNA genome of the lentiviral vector is inactivated and the cryptic splice donor site 3' to the major splice donor site is inactivated, and said nucleotide sequence is for use in a U3-independent lentiviral vector.

In one aspect the major splice donor site in the RNA genome of the lentiviral vector is inactivated and the cryptic splice donor site 3' to the major splice donor site is inactivated, and said nucleotide sequence has been transcribed independently of the U3 promoter.

In one aspect the major splice donor site in the RNA genome of the lentiviral vector is inactivated and the cryptic splice donor site 3' to the major splice donor site is inactivated, and said nucleotide sequence has been transcribed by a heterologous promoter.

In one aspect, transcription of the nucleotide sequence as described herein is not dependent on the presence of U3. The nucleotide sequence may be derived from a U3-independent transcription event. The nucleotide sequence may be derived from a heterologous promoter. A nucleotide sequence as described herein may not comprise a native U3 promoter.

### Construction of Splice Site Mutants

Splice site mutants of the present invention may be constructed using a variety of techniques. For example, mutations may be introduced at particular loci by synthesising oligonucleotides containing a mutant sequence, flanked by restriction sites enabling ligation to fragments of the native sequence. Following ligation, the resulting reconstructed sequence comprises a derivative having the desired nucleotide insertion, substitution, or deletion. By way of further example, splice site mutants may be constructed as described in WO 2021/160993 (which is incorporated herein by reference in its entirety).

Other known techniques allowing alterations of DNA sequence include recombination approaches such as Gibson assembly, Golden-gate cloning and In-fusion.

Alternatively, oligonucleotide-directed site-specific (or segment specific) mutagenesis procedures may be employed to provide an altered sequence according to the substitution, deletion, or insertion required. Deletion or truncation derivatives of splice site mutants may also be constructed by utilising convenient restriction endonuclease sites adjacent to the desired deletion. Subsequent to restriction, overhangs may be filled in, and the DNA religated. Exemplary methods of making the alterations set forth above are disclosed by Sambrook et al. (Molecular cloning: A Laboratory Manual, 2d Ed., Cold Spring Harbor Laboratory Press, 1989).

Splice site mutants may also be constructed utilising techniques of PCR mutagenesis, chemical mutagenesis, chemical mutagenesis (Drinkwater and Klinedinst, 1986) by forced nucleotide misincorporation (e.g., Liao and Wise, 1990), or by use of randomly mutagenised oligonucleotides (Horwitz et al., 1989).

The present invention also provides a method for producing a lentiviral vector nucleotide sequence, comprising the steps of:
providing a nucleotide sequence encoding the RNA genome of a lentiviral vector as described herein; and mutating the major splice donor site and cryptic splice donor site as described herein in said nucleotide sequence.

### Combination with modified U1 snRNA to improve vector titre

MSD-mutated lentiviral vectors are preferable to current standard lentiviral vectors for use as gene therapy vectors due to their reduced capacity to partake in aberrant splicing events both during LV production and in target cells. However, the production of MSD mutated vectors has either relied upon supply of the HIV-1 tat protein (1^{st} and 2^{nd} generation U3-dependent lentiviral vectors), has been of lower efficiency due to the destabilising effect of mutating the MSD on vector RNA levels (in 3^{rd} generation vectors), or, as discovered by the present inventors, is improved by co-expression of modified U1 snRNA. The present inventors have previously found that MSD-mutated, 3^{rd} generation (i.e. U3/tat-independent) LVs could be produced to high titre by co-expression of a modified U1 snRNA directed to bind to the 5'packaging region of the vector genome RNA during production (see WO 2021/014157 and WO 2021/160993, incorporated herein by reference).

The amount of vRNA produced from so-called MSD-mutated (or MSD-2KO) lentiviral vector genomes is typically substantially reduced, leading to lower vector titres. It is theorized that an 'early' interaction with the MSD and U1 snRNA (prior to splicing decisions) is important for transcription elongation from the external promoter. The inventors previously found that one solution to this problem was to provide a modified U1 snRNA *in trans* during LV production to stabilize the vRNA (see WO 2021/014157 and WO 2021/160993).

These modified U1 snRNAs can enhance the production titres of MSD-mutated LVs in a manner that is independent of the presence of the 5'polyA signal within the 5'R region, indicating a novel mechanism over others' use of modified U1 snRNAs to suppress polyadenylation (so called U1-interference, [Ui]). Targeting the modified U1 snRNAs to critical sequences of the packaging region produced the greatest enhancement in MSD-mutated LV titres.

The present inventors previously showed that the output titres of lentiviral vectors can be enhanced by co-expressing non-coding RNAs based on U1 snRNAs, which have been modified so that they no longer target the endogenous sequence (a splice donor site) but now target a sequence within the vRNA molecule. As demonstrated in WO 2021/014157 and WO 2021/160993, the relative enhancement in output titres of lentiviral vectors harbouring attenuating mutations within the major splice donor region (containing the major splice donor and cryptic splice donor sites) by said modified U1 snRNAs are greater than standard lentiviral vectors containing a non-mutated major splice donor region.

Vector genomes harbouring a broad range of mutation types within the major splice donor region (point mutations, region deletion, and sequence replacement) that lead to reduced titres may be used in combination with a modified U1 snRNA. The approach may comprise co-expression of modified U1 snRNAs together with the other vector components during vector production. The modified U1 snRNAs are designed such that binding to the consensus splice donor site has been ablated by replacing it with a heterologous sequence that is complementary to a target sequence within the vector genome vRNA.

In some embodiments, the nucleotide sequence of the invention is used in combination with a modified U1 snRNA.

In some embodiments, the nucleotide sequence of the invention further comprises a nucleotide sequence encoding a modified U1 snRNA.

In some embodiments, the nucleotide sequence encoding the lentiviral vector genome further encodes a modified U1 snRNA.

In some embodiments, the nucleotide sequence encoding the lentiviral vector genome is operably linked to the nucleotide sequence encoding the modified U1 snRNA.

In some embodiments, wherein said modified U1 snRNA has been modified to bind to a nucleotide sequence within the packaging region of the lentiviral vector genome.

In one embodiment, the nucleotide sequence encoding a modified U1 snRNA may be provided on a different nucleotide sequence, for example on a different plasmid. In other words, the nucleotide sequence encoding a modified U1 snRNA may be provided *in trans* during production of a lentiviral vector as described herein.

Splicing and polyadenylation are key processes for mRNA maturation, particularly in higher eukaryotes where most protein-coding transcripts contain multiple introns. The elements within a pre-mRNA that are required for splicing include the 5' splice donor signal, the sequence surrounding the branch point and the 3' splice acceptor signal. Interacting with these three elements is the spliceosome, which is formed by five small nuclear RNAs (snRNAs), including U1 snRNA, and associated nuclear proteins (snRNP). U1 snRNA is expressed by a polymerase II promoter and is present in most eukaryotic cells (Lund et al., 1984, J. Biol. Chem., 259:2013-2021). Human U1 snRNA (small nuclear RNA) is 164 nt long with a well-defined structure consisting of four stem-loops (West, S., 2012, Biochemical Society Transactions, 40:846-849). U1 snRNA contains a short sequence at its 5'-end that is broadly complementary to the 5' splice donor sites at exon-intron junctions. U1 snRNA participates in splice-site selection and spliceosome assembly by base pairing to the 5' splice donor site. A known function for U1 snRNA outside splicing is in the regulation of 3'-end mRNA processing: it suppresses premature polyadenylation (polyA) at early polyA signals (particularly within introns).

Human U1 snRNA (small nuclear RNA) is 164 nt long with a well-defined structure consisting of four stem-loops. The endogenous non-coding RNA, U1 snRNA, binds to the consensus 5' splice donor site (e.g. 5'-MAGGURR-3', wherein M is A or C and R is A or G) via the native splice donor annealing sequence (e.g. 5'-ACUUACCUG-3') during early steps of intron splicing. Stem loop I binds to U1A-70K protein that has been shown to be important for polyA suppression. Stem loop II binds to U1A protein, and the 5'-AUUUGUGG-3' sequence binds to Sm proteins, which together with Stem loop IV, is important for U1 snRNA processing. As defined herein, the modified U1 snRNA for use according to the present invention is modified to introduce a heterologous sequence that is complementary to a target sequence within the vector genome vRNA molecule at the site of the native splice donor targeting/annealing sequence.

Suitable modified U1 snRNAs for use according to the present invention are described in WO 2021/014157 and WO 2021/160993 and are incorporated herein by reference.

The modified U1 snRNAs can be manufactured according to methods generally known in the art. For example, the modified U1 snRNAs can be manufactured by chemical synthesis or recombinant DNA/RNA technology. By way of further example, the modified U1 snRNAs as described herein can be manufactured as described in WO 2021/014157 and WO 2021/160993.

The introduction of a nucleotide sequence encoding a modified U1 snRNA as described herein into a cell using conventional molecular and cell biology techniques is within the capabilities of a person of ordinary skill in the art.

### TRIP System

The present invention, as disclosed herein, may be combined with the 'TRIP' system.

The expression of transgenes during viral vector production can be detrimental to vector yields and/or quality by negatively affecting one or more aspects of the production phase. For example, a transgene protein may be cytotoxic or may, directly or indirectly, impair vector virion assembly and/or infectivity, or its presence during downstream processing or in the final product may be problematic. Thus, expression of the protein encoded by the NOI within viral vector production cells can adversely affect therapeutic vector titres (as shown in WO2015/092440). In addition, an NOI encoding a transmembrane POI may, for example, lead to high surface expression of the transmembrane protein in the viral vector virion, potentially altering the physical properties of the virions. Furthermore, this incorporation may present the POI to the patient's immune system at the site of delivery, which may negatively affect transduction and/or the long-term expression of the therapeutic gene *in vivo.*

WO2015/092440 and WO2021/094752, which are incorporated in their entirety herein by reference, disclose the use of a heterologous translation control system in eukaryotic cell cultures to repress the translation of the NOI (repress transgene expression) during viral vector production and thus repress or prevent expression of the protein encoded by the NOI. This system is referred to as the Transgene Repression In vector Production cell system or TRIP system.

In one form, the TRIP system utilises the bacterial *trp* operon regulation protein, tryptophan RNA-binding attenuation protein (TRAP), and the TRAP binding site/sequence (tbs) to mediate transgene repression. The use of this system does not impede the production of packageable vector genome molecules nor the activity of vector virions, and does not interfere with the long-term expression of the NOI in the target cell.

The term "binding site" is to be understood as a nucleic acid sequence that is capable of interacting with a certain protein.

By "capable of interacting" it is to be understood that the nucleic acid binding site (e.g. tbs or portion thereof) is capable of binding to a protein, for example TRAP, under the conditions that are encountered in a cell, for example a eukaryotic viral vector production cell. Such an interaction with an RNA-binding protein such as TRAP results in the repression or prevention of translation of a NOI to which the nucleic acid binding site (e.g. the tbs or portion thereof) is operably linked.

A consensus TRAP binding site sequence that is capable of binding TRAP is [KAGNN] repeated multiple times (e.g. 6, 7, 8, 9, 10, 11, 12 or more times); such sequence is found in the native *trp* operon. In the native context, occasionally AAGNN is tolerated and occasionally additional "spacing" N nucleotides result in a functional sequence. *In vitro* experiments have demonstrated that at least 6 or more consensus repeats are required for TRAP-RNA binding (Babitzke P, Y. J., Campanelli D. (1996) Journal of Bacteriology 178(17): 5159-5163). Therefore, preferably in one aspect there are 6 or more continuous [KAGN_{≥2}] sequences present within the tbs, wherein "K" may be T or G in DNA and U or G in RNA and "N" is to be understood as specifying any nucleotide at that position in the sequence (for example, "N" could be G, A, T, C or U).

In some embodiments, the lentiviral vector genome further comprises a tbs.

In some embodiments, the nucleotide sequence of the invention further comprises a TRAP binding site (tbs).

In some embodiments, a nucleotide sequence encoding TRAP is present during production of the lentiviral vector as described herein.

Suitable tbs are described in WO2015/092440 and WO2021/094752 and are incorporated herein by reference. Suitably, the nucleotide sequence may further comprise a tbs, and also may comprise a Kozak sequence, wherein said tbs overlaps the Kozak sequence, or wherein said Kozak sequence comprises a portion of a tbs. Suitably, the nucleotide sequence may further comprise a multiple cloning site and a Kozak sequence, wherein said multiple cloning site is overlapping with or located downstream to the 3' KAGN2-3 repeat of the tbs and upstream of the Kozak sequence.

As used herein, a "multiple cloning site" is to be understood as a DNA region which contains several restriction enzyme recognition sites (restriction enzyme sites) very close to each other. In one aspect, the RE sites may be overlapping in the MCS for use in the invention.

As used herein, a "restriction enzyme site" or "restriction enzyme recognition site" is a location on a DNA molecule containing specific sequences of nucleotides, 4-8 nucleotides in length, which are recognised by restriction enzymes. A restriction enzyme recognises a specific RE site (i.e. a specific sequence) and cleaves the DNA molecule within, or nearby, the RE site.

In some aspects of the present invention, the nucleotide of interest (i.e. transgene) is operably linked to the tbs. In some aspects, the nucleotide of interest is translated in a target cell which expresses TRAP. In some aspects, the nucleotide of interest is translated in a target cell which lacks TRAP.

By "operably linked" it is to be understood that the components described are in a relationship permitting them to function in their intended manner. Therefore a tbs or portion thereof for use in the invention operably linked to a NOI is positioned in such a way that translation of the NOI is modified when as TRAP binds to the tbs or portion thereof.

The tbs may be capable of interacting with TRAP such that translation of the nucleotide of interest is repressed or prevented in a viral vector production cell.

### Viral cis-acting sequences

ORFs present in the vector backbone delivered in transduced (e.g. patient) cells could be transcribed, for example, when read-through transcription from upstream cellular promoters occurs (lentiviral vectors target active transcription sites), leading to potential aberrant transcription of genetic material located in the vector backbone in patient cells. This potential aberrant transcription of genetic material located in the vector backbone following read-through transcription could also occur during lentiviral vector production in production cells.

The viral cis-acting sequence present within lentiviral vector genomes may contain multiple internal ORFs. These internal ORFs may be found between an internal ATG sequence of the viral cis-acting sequence and the stop codon immediately 3' to the ATG sequence.

Modifications in a viral cis-acting sequence to disrupt at least one internal ORF, for example by mutating the ATG sequence which denotes the start of the at least one internal ORF, are tolerated. Thus, the modified viral cis-acting sequence described herein retains its function.

Accordingly, in some embodiments of the present invention, the lentiviral vector genome comprises at least one modified viral cis-acting sequence, wherein at least one internal open reading frame (ORF) in the viral cis-acting sequence is disrupted (see WO 2021/181108 A1, incorporated herein by reference in its entirety). The at least one internal ORF may be disrupted by mutating at least one ATG sequence (ATG sequences may function as translation initiation codons).

In some embodiments of the present invention, the lentiviral vector genome comprises a modified nucleotide sequence encoding gag, wherein at least one internal ORF in the modified nucleotide sequence encoding gag is disrupted (see WO 2021/181108 A1, incorporated herein by reference in its entirety). The at least one internal ORF in the modified nucleotide sequence encoding gag may be disrupted by mutating at least one ATG sequence as described herein.

Suitable modified viral cis-acting sequences and modified nucleotide sequences encoding gag for use according to the present invention are described in WO 2021/181108 A1 and are incorporated herein by reference.

In some embodiments, the lentiviral vector genome comprises at least two (suitably at least three, at least four, at least five, at least six, at least seven) modified viral cis-acting sequences.

In some embodiments, at least two (suitably at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen or at least twenty) internal ORFs in the at least one viral cis-acting sequence and/or in the nucleotide sequence encoding gag may be disrupted. In some embodiments, at least three internal ORFs in the at least one viral cis-acting sequence and/or in the nucleotide sequence encoding gag may be disrupted.

In some embodiments, one (suitably, two, three, four, five, six, seven, eight, nine, ten, eleven, twelve, thirteen, fourteen, fifteen, sixteen, seventeen, eighteen, nineteen or twenty) internal ORFs in the at least one viral cis-acting sequence and/or the nucleotide sequence encoding gag may be disrupted.

In some embodiments, the at least one internal ORF may be disrupted such that the internal ORF is not expressed. In some embodiments, the at least one internal ORF may be disrupted such that the internal ORF is not translated. In some embodiments, the at least one internal ORF may be disrupted such that no protein is expressed from the internal ORF. In some embodiments, the at least one internal ORF may be disrupted such that no protein is translated from the internal ORF. Thus, the at least one internal ORF present in the modified viral *cis-*acting sequence and/or in the modified nucleotide sequence encoding gag in the vector backbone delivered in transduced cells may be disrupted such that aberrant transcription of the internal ORF is prevented when there is read-through transcription from upstream cellular promoters.

In one embodiment, the at least one internal ORF may be disrupted by mutating at least one ATG sequence. A "mutation" of an ATG sequence may comprise one or more nucleotide deletions, additions, or substitutions.

In one embodiment, the at least one ATG sequence may be mutated in the modified viral *cis-*acting sequence and/or in the modified nucleotide sequence encoding gag to a sequence selected from the group consisting of:
a) an ATTG sequence;
b) an ACG sequence;
c) an A-G sequence;
d) an AAG sequence;
e) a TTG sequence; and/or
f) an ATT sequence.

The at least one ATG sequence may be mutated to an ATTG sequence in the modified viral cis-acting sequence and/or the modified nucleotide sequence encoding gag. The at least one ATG sequence may be mutated to an ACG sequence in the modified viral cis-acting sequence and/or the modified nucleotide sequence encoding gag. The at least one ATG sequence may be mutated to an A-G sequence in the modified viral cis-acting sequence and/or the modified nucleotide sequence encoding gag. The at least one ATG sequence may be mutated to an AAG sequence in the modified viral cis-acting sequence and/or the modified nucleotide sequence encoding gag. The at least one ATG sequence may be mutated to a TTG sequence in the modified viral cis-acting sequence and/or the modified nucleotide sequence encoding gag. The at least one ATG sequence may be mutated to an ATT sequence in the modified viral cis-acting sequence and/or the modified nucleotide sequence encoding gag.

In one embodiment, the at least one modified viral cis-acting element and/or the modified nucleotide sequence encoding gag may lack ATG sequences.

In some embodiments, all ATG sequences within viral cis-acting sequences and/or the nucleotide sequence encoding gag in the lentiviral vector genome are mutated.

Lentiviral vectors typically comprise multiple viral *cis*-acting sequences. Example viral *cis-*acting sequences include gag-p17, Rev response element (RRE), central polypurine tract (cppt) and Woodchuck hepatitis virus (WHV) post-transcriptional regulatory element (WPRE).

In some embodiments, the at least one viral *cis*-acting sequence may be at least one lentiviral *cis*-acting sequence. Example lentiviral *cis*-acting sequences include the RRE and cppt.

In some embodiments, the at least one viral *cis*-acting sequence may be at least one non-lentiviral cis-acting sequence.

In some embodiments, the at least one viral *cis*-acting sequence may be at least one lentiviral *cis*-acting sequence and at least one non-lentiviral *cis*-acting sequence.

In some embodiments, the at least one viral *cis*-acting sequence is:
a) gag-p17; and/or
b) a Rev response element (RRE); and/or
c) a Woodchuck hepatitis virus (WHV) post-transcriptional regulatory element (WPRE).

In some embodiments, the at least one viral *cis*-acting sequence is a RRE.

In some embodiments, the at least one viral *cis*-acting sequence is a WPRE.

In some embodiments, the lentiviral vector genome comprises at least two (suitably, at least 3, at least 4, at least 5) modified viral cis-acting sequences.

In some embodiments, the lentiviral vector genome comprises a modified RRE as described herein and a modified WPRE as described herein.

In some embodiments, the lentiviral vector genome comprises a modified RRE as described herein, a modified WPRE as described herein and a modified nucleotide sequence encoding gag as described herein.

In one embodiment, the lentiviral vector genome as described herein lacks ATG sequences in the backbone of the vector genome. In one embodiment, the lentiviral vector genome as described herein lacks ATG sequences except in the NOI (i.e. transgene).

In one embodiment, the lentiviral vector genome comprises at least one modified viral *cis-*acting sequence and/or a modified nucleotide sequence encoding gag, wherein at least one internal open reading frame (ORF) in the viral cis-acting sequence or in the nucleotide sequence encoding gag is ablated.

In one embodiment, the lentiviral vector genome comprises at least one modified viral *cis-*acting sequence and/or a modified nucleotide sequence encoding gag, wherein at least one internal open reading frame (ORF) in the viral cis-acting sequence or in the nucleotide sequence encoding gag is silenced.

### Modified gag and viral Gag-p17 protein

A further preferred but optional feature of the invention is the minimization of gag sequences included within the packaging sequences used in combination with the aforementioned features. The amount of gag typically included within HIV-1 lentiviral vector packaging sequences can be reduced by at least 270 nucleotides, but may be reduced by up to the entire gag sequence. The deleted gag nucleotide sequence may be that of the gag-p17 instability sequence. Deletion of the gag-p17 instability sequence typically results in reduced vector titres unless the first ATG codon of the remaining gag sequence is mutated.

In some aspects the reduced packaging sequences comprise deleted gag sequences wherein only the first 80 nucleotides of gag remain.

In some aspects the reduced packaging sequences comprise deleted gag sequences wherein only the first 70 nucleotides of gag remain.

In some aspects the reduced packaging sequences comprise deleted gag sequences wherein only the first 60 nucleotides of gag remain.

In some aspects the reduced packaging sequences comprise deleted gag sequences wherein only the first 50 nucleotides of gag remain.

In some aspects the reduced packaging sequences comprise deleted gag sequences wherein only the first 40 nucleotides of gag remain.

In some aspects the reduced packaging sequences comprise deleted gag sequences wherein only the first 30 nucleotides of gag remain.

In some aspects the reduced packaging sequences comprise deleted gag sequences wherein only the first 20 nucleotides of gag remain.

In some aspects the reduced packaging sequences comprise deleted gag sequences wherein only the first 10 nucleotides of gag remain.

In some aspects the reduced packaging sequences comprise deleted gag sequences wherein no nucleotides of gag remain.

In some aspects, nucleotide sequences of the invention comprise ablated gag sequences wherein the gag sequences comprise only up to the first 10, up to the first 20, up to the first 30, up to the first 40, up to the first 50, up to the first 60, up to the first 70, or up to the first 80 nucleotides of gag.

The nucleotide sequence encoding gag may be a truncated nucleotide sequence encoding a part of gag. The nucleotide sequence encoding gag may be a minimal truncated nucleotide sequence encoding a part of gag. The part of gag may be a contiguous sequence. The truncated nucleotide sequence or minimal truncated nucleotide sequence encoding a part of gag may also contain at least one frameshift mutation.

An example truncated nucleotide sequence encoding a part of gag and which contains a frameshift mutation at position 45-46 is as follows:

ATGGGTGCGAGAGCGTCAGTATTAAGCGGGGGAGAATTAGATCGCGATGGGAAAAAATTCGGTTAAGG
CCAGGGGGAAAGAAAAAATATAAATTAAAACATATAGTATGGGCAAGCAGGGAGCTAGAACGATTCGC
AGTTAATCCTGGCCTGTTAGAAACATCAGAAGGCTGTAGACAAATACTGGGACAGCTACAACCATCCC
TTCAGACAGGATCAGAAGAACTTAGATCATTATATAATACAGTAGCAACCCTCTATTGTGTGCATCAA
AGGATAGAGATAAAAGACACCAAGGAAGCTTTAGACAAGATAGAGGGAGAGCAAAACAAAAGTAAGA
(SEQ ID NO: 9).

An example minimal truncated nucleotide sequence encoding a part of gag and which contains a frameshift mutation at position 45-46 is as follows:

ATGGGTGCGAGAGCGTCAGTATTAAGCGGGGGAGAATTAGATCGCGATGGGAAAAAATTCGGTTAAGG
CCAGGGGGAAAGA (SED ID NO: 10).

The nucleotide sequence encoding gag may, for example, comprise:
a) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 9; or
b) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 10.

The modified nucleotide sequence encoding gag may comprise:
a) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 9; or
b) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 10.

The modified nucleotide sequence encoding gag may comprise the sequence as set forth in SEQ ID NO: 9 or SEQ ID NO: 10, or a sequence having at least 80% identity (suitably at least 85%, at least 90%, at least 91%, at least 92%, at least 93%, at least 94%, at least 95%, at least 96%, at least 97%, at least 98%, at least 99%) thereto, wherein at least one ATG sequence selected from (a) to (c) is mutated:
a) ATG corresponding to positions 1-3 of SEQ ID NO: 9;
b) ATG corresponding to positions 47-49 of SEQ ID NO: 9; and/or
c) ATG corresponding to positions 107-109 of SEQ ID NO: 9.

An example modified truncated nucleotide sequence encoding part of gag and which contains a frameshift mutation is as follows:

ACGGGTGCGAGAGCGTCAGTATTAAGCGGGGGAGAATTAGATCGCGATTGGGAAAAAATTCGGTTAAG
GCCAGGGGGAAAGAAAAAATATAAATTAAAACATATAGTATTGGGCAAGCAGGGAGCTAGAACGATTC
GCAGTTAATCCTGGCCTGTTAGAAACATCAGAAGGCTGTAGACAAATACTGGGACAGCTACAACCATC
CCTTCAGACAGGATCAGAAGAACTTAGATCATTATATAATACAGTAGCAACCCTCTATTGTGTGCATC
AAAGGATAGAGATAAAAGACACCAAGGAAGCTTTAGACAAGATAGAGGGAGAGCAAAACAAAAGTAAG
A (SEQ ID NO: 11).

An example modified minimal truncated nucleotide sequence encoding a part of gag and which contains a frameshift mutation is as follows:

ACGGGTGCGAGAGCGTCAGTATTAAGCGGGGGAGAATTAGATCGCGATTGGGAAAAAATTCGGTTAAG
GCCAGGGGGAAAGA (SED ID NO: 12).

The modified nucleotide sequence encoding gag may comprise the sequence as set forth in SEQ ID NO: 11, or a sequence having at least 80% (suitably at least 85%, at least 90%, at least 91%, at least 92%, at least 93%, at least 94%, at least 95%, at least 96%, at least 97%, at least 98%, at least 99%) identity thereto. The sequence may comprise less than three (suitably less than two or less than one) ATG sequences.

The modified nucleotide sequence encoding gag may comprise the sequence as set forth in SEQ ID NO: 12, or a sequence having at least 80% (suitably at least 85%, at least 90%, at least 91%, at least 92%, at least 93%, at least 94%, at least 95%, at least 96%, at least 97%, at least 98%, at least 99%) identity thereto. The sequence may comprise less than two (suitably less than one) ATG sequences.

The modified nucleotide sequence encoding gag may comprise less than three ATG sequences. Suitably, the modified nucleotide sequence encoding gag may comprise less than two or less than one ATG sequence(s). The modified nucleotide sequence encoding gag may lack an ATG sequence.

Lentiviral vector genomes lacking a nucleotide sequence encoding Gag-p17 or a fragment thereof are described in WO 2021/181108 A1, incorporated herein by reference in its entirety. Such lentiviral vector genomes as described in WO 2021/181108 are suitable for use according to the present invention.

The lentiviral vector genome as described herein may lack a nucleotide sequence encoding Gag-p17 or a fragment thereof. The lentiviral vector genome may, for example, not express Gag-p17 or a fragment thereof. In one embodiment, the lentiviral vector genome may lack the sequence as set forth in SEQ ID NO: 13.

The viral protein Gag-p17 surrounds the capsid of the lentiviral vector particle, and is in turn surrounded by the envelope protein. A nucleotide sequence encoding Gag-p17 has historically been included in lentiviral vector genomes for the production of therapeutic lentiviral vectors. The nucleotide sequence encoding Gag-p17 present within lentiviral vector genomes is typically embedded within the packaging region containing highly structured RNA towards the 5' region of the RNA (the 5'UTR). The nucleotide sequence encoding Gag-p17 typically comprises an RNA instability sequence (INS), herein referred to as p17-INS.

Deletion of p17-INS from the backbone of the lentiviral vector genome does not significantly impact vector titres during lentiviral vector production.

The lentiviral vector genome lacking a nucleotide sequence encoding Gag-p17 or p17-INS is of a smaller size compared to a lentiviral vector genome comprising a nucleotide sequence encoding Gag-p17 or p17-INS. Thus, the amount of viral DNA contained within the viral vector backbone delivered in transduced cells is reduced when a lentiviral vector genome lacking a nucleotide sequence encoding Gag-p17 or p17-INS is used. Further, the lentiviral vector genome lacking a nucleotide sequence encoding Gag-p17 or p17-INS may be used to deliver a transgene of larger size than the transgenes which can be delivered using a lentiviral vector genome containing a nucleotide sequence encoding Gag-p17 or p17-INS. Therefore, there are several reasons why it may be desirable to delete nucleotide sequence encoding Gag-p17 or p17-INS within the vector backbone. Deletion of gag sequences in order to reduce the size of lentiviral vector genome sequences has been reported (Sertkaya, H., et al., Sci Rep 11:12067 (2021)).

In some embodiments, the lentiviral vector genome lacks either (i) a nucleotide sequence encoding Gag-p17 or (ii) a fragment of a nucleotide sequence encoding Gag-p17.

In some embodiments, the lentiviral vector genome lacks a nucleotide sequence encoding p17-INS or a fragment thereof.

An example p17-INS is as follows:

AAAAATATAAATTAAAACATATAGTATGGGCAAGCAGGGAGCTAGAACGATTCGCAGTTAATCCTGGC
CTGTTAGAAACATCAGAAGGCTGTAGACAAATACTGGGACAGCTACAACCATCCCTTCAGACAGGATC
AGAAGAACTTAGATCATTATATAATACAGTAGCAACCCTCTATTGTGTGCATCAAAGGATAGAGATAA
AAGACACCAAGGAAGCTTTAGACAAGATAGAGGGAGAGCAAAACAAAAGTA (SEQ ID NO: 13).

In one embodiment, the lentiviral vector genome may lack the sequence as set forth in SEQ ID NO: 13.

In one embodiment, the fragment of a nucleotide sequence encoding Gag-p17 is a part of a full-length nucleotide sequence encoding Gag-p17. In one embodiment, the fragment comprises or consists of at least about 10 nucleotides (suitably at least about 20, at least about 30, at least about 40, at least about 50, at least about 100, at least about 150, at least about 200, at least about 250, at least about 300, at least about 350 nucleotides).

In one embodiment, the fragment of a nucleotide sequence encoding Gag-p17 may have a length which is between 1% and 99% of full-length nucleotide sequence encoding Gag-p17. Suitably, the fragment of a nucleotide sequence encoding Gag-p17 may have a length which is at least about 10% (suitably at least about 20%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%) of a full-length nucleotide sequence encoding Gag-p17, such as a native nucleotide sequence encoding Gag-p17. The fragment may be a contiguous region of a full-length nucleotide sequence encoding Gag-p17, such as a native nucleotide sequence encoding Gag-p17.

In one embodiment, the fragment of a nucleotide sequence encoding Gag-p17 may have a length which is between 1% and 99% of full-length nucleotide sequence encoding p17-INS. Suitably, the fragment of a nucleotide sequence encoding Gag-p17 may have a length which is at least about 10% (suitably at least about 20%, at least about 30%, at least about 40%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%) of a full-length nucleotide sequence encoding p17-INS, such as a native nucleotide sequence encoding p17-INS (e.g. SEQ ID NO: 13). The fragment may be a contiguous region of a full-length nucleotide sequence encoding p17-INS, such as a native nucleotide sequence encoding p17-INS (e.g. SEQ ID NO: 13).

In one embodiment, the fragment of a nucleotide sequence encoding Gag-p17 comprises or consists of the INS located in the nucleotide sequence encoding Gag-p17.

In one embodiment, the lentiviral vector genome lacking either (i) a nucleotide sequence encoding Gag-p17 or (ii) a fragment of a nucleotide sequence encoding Gag-p17 comprises at least one modified viral cis-acting sequence as described herein.

In one embodiment, the lentiviral vector genome lacking either (i) a nucleotide sequence encoding Gag-p17 or (ii) a fragment of a nucleotide sequence encoding Gag-p17 may comprise a modified RRE, a modified WPRE and/or a modified nucleotide sequence encoding gag as described herein.

In one embodiment, the lentiviral vector genome lacking either (i) a nucleotide sequence encoding Gag-p17 or (ii) a fragment of a nucleotide sequence encoding Gag-p17 may comprise a modified RRE as described herein, a modified WPRE as described herein and a modified nucleotide sequence encoding gag as described herein.

### Modified Rev response element (RRE)

In some embodiments, the lentiviral vector genome comprises a modified Rev response element (RRE), wherein at least one internal open reading frame (ORF) in the RRE is disrupted as described herein.

The RRE is an essential viral RNA element that is well conserved across lentiviral vectors and across different wild-type HIV-1 isolates. The RRE present within lentiviral vector genomes may contain multiple internal ORFs. These internal ORFs may be found between an internal ATG sequence of the RRE and the stop codon immediately 3' to the ATG sequence.

The RRE present within lentiviral vector genomes is typically embedded within the packaging region containing highly structured RNA towards the 5' region of the RNA (the 5'UTR). The 5' UTR structure consists of series of stem-loop structures connected by small linkers. These stem-loops include the RRE. Thus, the RRE itself has a complex secondary structure, involving complementary base-pairing, to which Rev binds.

Modifications in the RRE to disrupt at least one internal ORF, for example by mutating the ATG sequence which denotes the start of the at least one internal ORF, are tolerated. Thus, the modified RREs described herein retain Rev binding capacity.

The modified RRE may comprise less than eight ATG sequences.

Accordingly, in some embodiments, the lentiviral vector genome comprises a modified Rev response element (RRE), wherein the modified RRE comprises less than eight ATG sequences.

Suitably, the modified RRE may comprise less than seven, less than six, less than five, less than four, less than three, less than two or less than one ATG sequence(s). The modified RRE may lack an ATG sequence.

The RRE may be a minimal functional RRE. An example minimal functional RRE is as follows:

AGGAGCTTTGTTCCTTGGGTTCTTGGGAGCAGCAGGAAGCACTATGGGCGCAGCGTCAATGACGCTGA
CGGTACAGGCCAGACAATTATTGTCTGGTATAGTGCAGCAGCAGAACAATTTGCTGAGGGCTATTGAG
GCGCAACAGCATCTGTTGCAACTCACAGTCTGGGGCATCAAGCAGCTCCAGGCAAGAATCCTGGCTGT
GGAAAGATACCTAAAGGATCAACAGCTCCT (SEQ ID NO: 14).

By "minimal functional RRE" or "minimal RRE" is meant a truncated RRE sequence which retains the function of the full-length RRE. Thus, the minimal functional RRE retains Rev binding capacity.

The RRE may be the core RRE. An example core RRE is as follows:

GGAGCTTTGTTCCTTGGGTTCTTGGGAGCAGCAGGAAGCACTATGGGCGCAGCGTCAATGACGCTGAC
GGTACAGGCCAGACAATTATTGTCTGGTATAGTGCAGCAGCAGAACAATTTGCTGAGGGCTATTGAGG
CGCAACAGCATCTGTTGCAACTCACAGTCTGGGGCATCAAGCAGCTCCAGGCAAGAATCCTGGCTGTG
GAAAGATACCTAAAGGATCAACAGCTCCTGGGGATTTGGGGTTGCTCTGGAAAACTCATTTGCACCAC
TGCTGTGCCTTGGAATGCTAGTTGGAGTAATAAATCTCTGGAACAGATTTGGAATCACACGACCTGGA
TGGAGTGGGAC (SEQ ID NO: 90)

The RRE may be a full-length RRE. An example full-length RRE is as follows:

TGATCTTCAGACCTGGAGGAGGAGATATGAGGGACAATTGGAGAAGTGAATTATATAAATATAAAGTA
GTAAAAATTGAACCATTAGGAGTAGCACCCACCAAGGCAAAGAGAAGAGTGGTGCAGAGAGAAAAAAG
AGCAGTGGGAATAGGAGCTTTGTTCCTTGGGTTCTTGGGAGCAGCAGGAAGCACTATGGGCGCAGCGT
CAATGACGCTGACGGTACAGGCCAGACAATTATTGTCTGGTATAGTGCAGCAGCAGAACAATTTGCTG
AGGGCTATTGAGGCGCAACAGCATCTGTTGCAACTCACAGTCTGGGGCATCAAGCAGCTCCAGGCAAG
AATCCTGGCTGTGGAAAGATACCTAAAGGATCAACAGCTCCTGGGGATTTGGGGTTGCTCTGGAAAAC
TCATTTGCACCACTGCTGTGCCTTGGAATGCTAGTTGGAGTAATAAATCTCTGGAACAGATTTGGAAT
CACACGACCTGGATGGAGTGGGACAGAGAAATTAACAATTACACAAGCTTAATACACTCCTTAATTGA
AGAATCGCAAAACCAGCAAGAAAAGAATGAACAAGAATTATTGGAATTAGATAAATGGGCAAGTTTGT
GGAATTGGTTTAACATAACAAATTGGCTGTGGTATATAAAATTATTCATAATGATAGTAGGAGGCTTG
GTAGGTTTAAGAATAGTTTTTGCTGTACTTTCTATAGTGAATAGAGTTAGGCAGGGATATTCACCATT
ATCGTTTCAGACCCACCTCCCAACCCCGAGGGGAC (SEQ ID NO: 15).

The RRE may comprise:
a) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 14;
b) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 90; and/or
c) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 15.

The modified RRE may comprise:
a) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 14;
b) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 90; and/or
c) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 15.

The modified RRE may comprise the sequence as set forth in SEQ ID NO: 14, SEQ ID NO: 90 or SEQ ID NO: 15, or a sequence having at least 80% identity (suitably at least 85%, at least 90%, at least 91%, at least 92%, at least 93%, at least 94%, at least 95%, at least 96%, at least 97%, at least 98%, at least 99%) thereto, wherein at least one ATG sequence selected from the group (a)-(h) is mutated:
a) ATG corresponding to positions 27-29 of SEQ ID NO: 15;
b) ATG corresponding to positions 192-194 of SEQ ID NO: 15;
c) ATG corresponding to positions 207-209 of SEQ ID NO: 15;
d) ATG corresponding to positions 436-438 of SEQ ID NO: 15;
e) ATG corresponding to positions 489-491 of SEQ ID NO: 15;
f) ATG corresponding to positions 571-573 of SEQ ID NO: 15;
g) ATG corresponding to positions 599-601 of SEQ ID NO: 15; and/or
h) ATG corresponding to positions 663-665 of SEQ ID NO: 15.

An example modified RRE sequence is as follows:

AGGAGCTTTGTTCCTTGGGTTCTTGGGAGCAGCAGGAAGCACTATTGGGCGCAGCGTCAATTGACGCT
GACGGTACAGGCCAGACAATTATTGTCTGGTATAGTGCAGCAGCAGAACAATTTGCTGAGGGCTATTG
AGGCGCAACAGCATCTGTTGCAACTCACAGTCTGGGGCATCAAGCAGCTCCAGGCAAGAATCCTGGCT
GTGGAAAGATACCTAAAGGATCAACAGCTCCT (SEQ ID NO: 16).

A further example modified RRE sequence is as follows:

GGAGCTTTGTTCCTTGGGTTCTTGGGAGCAGCAGGAAGCACTATTGGGCGCAGCGTCAATTGACGCTG
ACGGTACAGGCCAGACAATTATTGTCTGGTATAGTGCAGCAGCAGAACAATTTGCTGAGGGCTATTGA
GGCGCAACAGCATCTGTTGCAACTCACAGTCTGGGGCATCAAGCAGCTCCAGGCAAGAATCCTGGCTG
TGGAAAGATACCTAAAGGATCAACAGCTCCTGGGGATTTGGGGTTGCTCTGGAAAACTCATTTGCACC
ACTGCTGTGCCTTGGAATTGCTAGTTGGAGTAATAAATCTCTGGAACAGATTTGGAATCACACGACCT
GGATTGGAGTGGGAC (SEQ ID NO: 91)

A further example modified RRE sequence is as follows:

TGATCTTCAGACCTGGAGGAGGAGATATTGAGGGACAATTGGAGAAGTGAATTATATAAATATAAAGT
AGTAAAAATTGAACCATTAGGAGTAGCACCCACCAAGGCAAAGAGAAGAGTGGTGCAGAGAGAAAAAA
GAGCAGTGGGAATAGGAGCTTTGTTCCTTGGGTTCTTGGGAGCAGCAGGAAGCACTATTGGGCGCAGC
GTCAATTGACGCTGACGGTACAGGCCAGACAATTATTGTCTGGTATAGTGCAGCAGCAGAACAATTTG
CTGAGGGCTATTGAGGCGCAACAGCATCTGTTGCAACTCACAGTCTGGGGCATCAAGCAGCTCCAGGC
AAGAATCCTGGCTGTGGAAAGATACCTAAAGGATCAACAGCTCCTGGGGATTTGGGGTTGCTCTGGAA
AACTCATTTGCACCACTGCTGTGCCTTGGAATTGCTAGTTGGAGTAATAAATCTCTGGAACAGATTTG
GAATCACACGACCTGGATTGGAGTGGGACAGAGAAATTAACAATTACACAAGCTTAATACACTCCTTA
ATTGAAGAATCGCAAAACCAGCAAGAAAAGAATGAACAAGAATTATTGGAATTAGATAAATGGGCAAG
TTTGTGGAATTGGTTTAACATAACAAATTGGCTGTGGTATATAAAATTATTCATAATTGATAGTAGGA
GGCTTGGTAGGTTTAAGAATAGTTTTTGCTGTACTTTCTATAGTGAATAGAGTTAGGCAGGGATATTC
ACCATTATCGTTTCAGACCCACCTCCCAACCCCGAGGGGAC (SEQ ID NO: 17).

An example of a modified RRE sequence lacking an ATG sequence is as follows:

TGATCTTCAGACCTGGAGGAGGAGATATTGAGGGACAATTGGAGAAGTGAATTATATAAATATAAAGT
AGTAAAAATTGAACCATTAGGAGTAGCACCCACCAAGGCAAAGAGAAGAGTGGTGCAGAGAGAAAAAA
GAGCAGTGGGAATAGGAGCTTTGTTCCTTGGGTTCTTGGGAGCAGCAGGAAGCACTATTGGGCGCAGC
GTCAATTGACGCTGACGGTACAGGCCAGACAATTATTGTCTGGTATAGTGCAGCAGCAGAACAATTTG
CTGAGGGCTATTGAGGCGCAACAGCATCTGTTGCAACTCACAGTCTGGGGCATCAAGCAGCTCCAGGC
AAGAATCCTGGCTGTGGAAAGATACCTAAAGGATCAACAGCTCCTGGGGATTTGGGGTTGCTCTGGAA
AACTCATTTGCACCACTGCTGTGCCTTGGAATTGCTAGTTGGAGTAATAAATCTCTGGAACAGATTTG
GAATCACACGACCTGGATTGGAGTGGGACAGAGAAATTAACAATTACACAAGCTTAATACACTCCTTA
ATTGAAGAATCGCAAAACCAGCAAGAAAAGAATtGAACAAGAATTATTGGAATTAGATAAATtGGGCA
AGTTTGTGGAATTGGTTTAACATAACAAATTGGCTGTGGTATATAAAATTATTCATAATTGATAGTAG
GAGGCTTGGTAGGTTTAAGAATAGTTTTTGCTGTACTTTCTATAGTGAATAGAGTTAGGCAGGGATAT
TCACCATTATCGTTTCAGACCCACCTCCCAACCCCGAGGGGAC (SEQ ID NO: 18).

The modified RRE may comprise the sequence as set forth in SEQ ID NO: 16, SEQ ID NO: 91, SEQ ID NO: 17 or SEQ ID NO: 18, or a sequence having at least 80% (suitably at least 85%, at least 90%, at least 91%, at least 92%, at least 93%, at least 94%, at least 95%, at least 96%, at least 97%, at least 98%, at least 99%) identity thereto. The sequence may comprise less than eight (suitably less than seven, less than six, less than five, less than four, less than three, less than two or less than one) ATG sequences.

### Modified Woodchuck hepatitis virus (WHV) post-transcriptional regulatory element (WPRE)

In some embodiments, the lentiviral vector genome comprises a modified Woodchuck hepatitis virus (WHV) post-transcriptional regulatory element (WPRE), wherein at least one internal open reading frame (ORF) in the WPRE is disrupted as described herein.

The WPRE can enhance expression from a number of different vector types including lentiviral vectors (U.S. Patent Nos. 6,136,597; 6,287,814; Zufferey, R., et al. (1999) J. Virol. 73: 2886-92). Without wanting to be bound by theory, this enhancement is thought to be due to improved RNA processing at the post-transcriptional level, resulting in increased levels of nuclear transcripts. A two-fold increase in mRNA stability also contributes to this enhancement (Zufferey, R., et al. *ibid*)*.* The level of enhancement of protein expression from transcripts containing the WPRE versus those without the WPRE has been reported to be around 2-to-5 fold, and correlates well with the increase in transcript levels. This has been demonstrated with a number of different transgenes (Zufferey, R., et al. *ibid*)*.*

The WPRE contains three *cis*-acting sequences important for its function in enhancing expression levels. In addition, it contains a fragment of approximately 180 bp comprising the 5'-end of the WHV X protein ORF (full length ORF is 425bp), together with its associated promoter. The full-length X protein has been implicated in tumorigenesis (Flajolet, M. et al, (1998) J. Virol. 72: 6175-6180). Translation from transcripts initiated from the X promoter results in formation of a protein representing the NH₂-terminal 60 amino acids of the X protein. This truncated X protein can promote tumorigenesis, particularly if the truncated X protein sequence is integrated into the host cell genome at specific loci (Balsano, C. et al, (1991) Biochem. Biophys Res. Commun. 176: 985-92; Flajolet, M. et al, (1998) J. Virol. 72: 6175-80; Zheng, Y.W., et al, (1994) J. Biol. Chem. 269: 22593-8; Runkel, L., et al, (1993) Virology 197: 529-36). Therefore, expression of the truncated X protein could promote tumorigenesis if delivered to cells of interest, precluding safe use of wild-type WPRE sequences.

US 2005/0002907 discloses that mutation of a region of the WPRE corresponding to the X protein ORF ablates the tumorigenic activity of the X protein, thereby allowing the WPRE to be used safely in retroviral and lentiviral expression vectors to enhance expression levels of heterologous genes or nucleotides of interest.

As used herein, the "X region" of the WPRE is defined as comprising at least the first 60-amino acids of the X protein ORF, including the translation initiation codon, and its associated promoter. A "functional" X protein is defined herein as a truncated X protein that is capable of promoting tumorigenesis, or a transformed phenotype, when expressed in cells of interest. A "non-functional" X protein in the context of this application is defined as an X protein that is incapable of promoting tumorigenesis in cells of interest.

The modified WPREs described herein retain the capacity to enhance expression from the lentiviral vector.

The modified WPRE may comprise less than seven ATG sequences. The modified WPRE may comprise less than six ATG sequences.

Accordingly, in some embodiments, the lentiviral vector genome comprises a modified Woodchuck hepatitis virus (WHV) post-transcriptional regulatory element (WPRE), wherein the modified WPRE comprises less than seven ATG sequences, preferably less than six ATG sequences.

Suitably, the modified WPRE may comprise less than seven, less than six, less than five, less than four, less than three, less than two or less than one ATG sequence(s). The modified WPRE may lack ATG sequences.

In some embodiments, at least one ATG sequence in the X region of the WPRE is mutated, whereby expression of a functional X protein is prevented. In preferred embodiments, the mutation is in the translation initiation codon of the X region. As a result of the mutation of the at least one ATG sequence, the X protein may not be expressed.

In some embodiments, the modified WPRE does not comprise a mutation in an ATG sequence in the X region of the WPRE.

An example WPRE sequence is as follows:

AATCAACCTCTGGATTACAAAATTTGTGAAAGATTGACTGGTATTCTTAACTATGTTGCTCCTTTTAC
GCTATGTGGATACGCTGCTTTAATGCCTTTGTATCATGCTATTGCTTCCCGTATGGCTTTCATTTTCT
CCTCCTTGTATAAATCCTGGTTGCTGTCTCTTTATGAGGAGTTGTGGCCCGTTGTCAGGCAACGTGGC
GTGGTGTGCACTGTGTTTGCTGACGCAACCCCCACTGGTTGGGGCATTGCCACCACCTGTCAGCTCCT
TTCCGGGACTTTCGCTTTCCCCCTCCCTATTGCCACGGCGGAACTCATCGCCGCCTGCCTTGCCCGCT
GCTGGACAGGGGCTCGGCTGTTGGGCACTGACAATTCCGTGGTGTTGTCGGGGAAGCTGACGTCCTTT
CCATGGCTGCTCGCCTGTGTTGCCACCTGGATTCTGCGCGGGACGTCCTTCTGCTACGTCCCTTCGGC
CCTCAATCCAGCGGACCTTCCTTCCCGCGGCCTGCTGCCGGCTCTGCGGCCTCTTCCGCGTCTTCGCC
TTCGCCCTCAGACGAGTCGGATCTCCCTTTGGGCCGCCTCC (SEQ ID NO: 19).

An example WPRE sequence which contains a disrupted X-protein ORF is as follows:

AATCAACCTCTGGATTACAAAATTTGTGAAAGATTGACTGGTATTCTTAACTATGTTGCTCCTTTTAC
GCTATGTGGATACGCTGCTTTAATGCCTTTGTATCATGCTATTGCTTCCCGTATGGCTTTCATTTTCT
CCTCCTTGTATAAATCCTGGTTGCTGTCTCTTTATGAGGAGTTGTGGCCCGTTGTCAGGCAACGTGGC
GTGGTGTGCACTGTGTTTGCTGACGCAACCCCCACTGGTTGGGGCATTGCCACCACCTGTCAGCTCCT
TTCCGGGACTTTCGCTTTCCCCCTCCCTATTGCCACGGCGGAACTCATCGCCGCCTGCCTTGCCCGCT
GCTGGACAGGGGCTCGGCTGTTGGGCACTGACAATTCCGTGGTGTTGTCGGGGAAATCATCGTCCTTT
CCTTGGCTGCTCGCCTGTGTTGCCACCTGGATTCTGCGCGGGACGTCCTTCTGCTACGTCCCTTCGGC
CCTCAATCCAGCGGACCTTCCTTCCCGCGGCCTGCTGCCGGCTCTGCGGCCTCTTCCGCGTCTTCGCC
TTCGCCCTCAGACGAGTCGGATCTCCCTTTGGGCCGCCTCC (SEQ ID NO: 20).

The WPRE may comprise:
a) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 19; and/or
b) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 20.

The modified WPRE may comprise:
a) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 19; and/or
b) a sequence having at least 80% (suitably at least 85%, at least 90%, at least 91%, at least 92%, at least 93%, at least 94%, at least 95%, at least 96%, at least 97%, at least 98%, at least 99%) identity to SEQ ID NO: 20.

The modified WPRE may comprise the sequence as set forth in SEQ ID NO: 19 or SEQ ID NO: 20, or a sequence having at least 80% identity (suitably at least 85%, at least 90%, at least 91%, at least 92%, at least 93%, at least 94%, at least 95%, at least 96%, at least 97%, at least 98%, at least 99%) thereto, wherein at least one ATG sequence selected from the group (a)-(g) is mutated:
a) ATG corresponding to positions 53-55 of SEQ ID NO: 19;
b) ATG corresponding to positions 72-74 of SEQ ID NO: 19;
c) ATG corresponding to positions 91-93 of SEQ ID NO: 19;
d) ATG corresponding to positions 104-106 of SEQ ID NO: 19;
e) ATG corresponding to positions 121-123 of SEQ ID NO: 19;
f) ATG corresponding to positions 170-172 of SEQ ID NO: 19; and/or
g) ATG corresponding to positions 411-413 of SEQ ID NO: 19.

The WRPE typically contains a retained Pol ORF. An example retained Pol ORF sequence is as follows:

ATGCTATTGCTTCCCGTATGGCTTTCATTTTCTCCTCCTTGTATAAATCCTGGTTGCTGTCTCTTTAT
GAGGAGTTGTGGCCCGTTGTCAGGCAACGTGGCGTGGTGTGCACTGTGTTTGCTGACGCAACCCCCAC
TGGTTGGGGCATTGCCACCACCTGTCAGCTCCTTTCCGGGACTTTCGCTTTCCCCCTCCCTATTGCCA
CGGCGGAACTCATCGCCGCCTGCCTTGCCCGCTGCTGGACAGGGGCTCGGCTGTTGGGCACTGACAAT
TCCGTGGTGTTGTCGGGGAAATCATCGTCCTTTCCTTGGCTGCTCGCCTGTGTTGCCACCTGGATTCT
GCGCGGGACGTCCTTCTGCTACGTCCCTTCGGCCCTCAATCCAGCGGACCTTCCTTCCCGCGGCCTGC
TGCCGGCTCTGCGGCCTCTTCCGCGTCTTCGCCTTCGCCCTCAGACGAGTCGGATCTCCCTTTGGGCC
GCCTCC (SEQ ID NO: 21).

In one embodiment, at least one (suitably at least two or at least three) ATG sequence within the retained Pol ORF sequence in the WPRE is mutated. In one embodiment, all ATG sequences within the retained Pol ORF sequence in the WPRE are mutated.

In one embodiment, the modified WPRE comprises less than three (suitably less than two or less than one) ATG sequences in the retained Pol ORF sequence in the WPRE. In one embodiment, the modified WPRE lacks an ATG sequence in the retained Pol ORF sequence in the WPRE.

An example modified WPRE sequence in which all ATG codons within the retained Pol ORF are mutated is as follows:

AATCAACCTCTGGATTACAAAATTTGTGAAAGATTGACTGGTATTCTTAACTATGTTGCTCCTTTTAC
GCTATGTGGATACGCTGCTTTAATGCCTTTGTATCATTGCTATTGCTTCCCGTATGGCTTTCATTTTC
TCCTCCTTGTATAAATCCTGGTTGCTGTCTCTTTATTGAGGAGTTGTGGCCCGTTGTCAGGCAACGTG
GCGTGGTGTGCACTGTGTTTGCTGACGCAACCCCCACTGGTTGGGGCATTGCCACCACCTGTCAGCTC
CTTTCCGGGACTTTCGCTTTCCCCCTCCCTATTGCCACGGCGGAACTCATCGCCGCCTGCCTTGCCCG
CTGCTGGACAGGGGCTCGGCTGTTGGGCACTGACAATTCCGTGGTGTTGTCGGGGAAATCATCGTCCT
TTCCTTGGCTGCTCGCCTGTGTTGCCACCTGGATTCTGCGCGGGACGTCCTTCTGCTACGTCCCTTCG
GCCCTCAATCCAGCGGACCTTCCTTCCCGCGGCCTGCTGCCGGCTCTGCGGCCTCTTCCGCGTCTTCG
CCTTCGCCCTCAGACGAGTCGGATCTCCCTTTGGGCCGCCTCC (SEQ ID NO: 22).

An example of a modified WPRE sequence lacking an ATG sequence is as follows:

AATCAACCTCTGGATTACAAAATTTGTGAAAGATTGACTGGTATTCTTAACTATtGTTGCTCCTTTTA
CGCTATtGTGGATACGCTGCTTTAATtGCCTTTGTATCATtGCTATTGCTTCCCGTATtGGCTTTCAT
TTTCTCCTCCTTGTATAAATCCTGGTTGCTGTCTCTTTATtGAGGAGTTGTGGCCCGTTGTCAGGCAA
CGTGGCGTGGTGTGCACTGTGTTTGCTGACGCAACCCCCACTGGTTGGGGCATTGCCACCACCTGTCA
GCTCCTTTCCGGGACTTTCGCTTTCCCCCTCCCTATTGCCACGGCGGAACTCATCGCCGCCTGCCTTG
CCCGCTGCTGGACAGGGGCTCGGCTGTTGGGCACTGACAATTCCGTGGTGTTGTCGGGGAAATCATCG
TCCTTTCCTTGGCTGCTCGCCTGTGTTGCCACCTGGATTCTGCGCGGGACGTCCTTCTGCTACGTCCC
TTCGGCCCTCAATCCAGCGGACCTTCCTTCCCGCGGCCTGCTGCCGGCTCTGCGGCCTCTTCCGCGTC
TTCGCCTTCGCCCTCAGACGAGTCGGATCTCCCTTTGGGCCGCCTCC (SEQ ID NO: 23).

The modified WPRE may comprise the sequence as set forth in SEQ ID NO: 22 or SEQ ID NO: 23, or a sequence having at least 80% (suitably at least 85%, at least 90%, at least 91%, at least 92%, at least 93%, at least 94%, at least 95%, at least 96%, at least 97%, at least 98%, at least 99%) identity thereto. The sequence may comprise less than six (suitably less than five, less than four, less than three, less than two or less than one) ATG sequences.

### Vector / Expression Cassette

A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous cDNA segment), to be transferred into and expressed by a target cell. The vector may facilitate the integration of the nucleotide sequence encoding a viral vector component to maintain the nucleotide sequence encoding the viral vector component and its expression within the target cell.

The vector may be or may include an expression cassette (also termed an expression construct). Expression cassettes as described herein comprise regions of nucleic acid containing sequences capable of being transcribed. Thus, sequences encoding mRNA, tRNA and rRNA are included within this definition.

The vector may contain one or more selectable marker genes (e.g. a neomycin resistance gene) and/or traceable marker gene(s) (e.g. a gene encoding green fluorescent protein (GFP)). Vectors may be used, for example, to infect and/or transduce a target cell. The vector may further comprise a nucleotide sequence enabling the vector to replicate in the host cell in question, such as a conditionally replicating oncolytic vector.

The term "cassette" - which is synonymous with terms such as "conjugate", "construct" and "hybrid" - includes a polynucleotide sequence directly or indirectly attached to a promoter. Preferably the cassette comprises at least a polynucleotide sequence operably linked to a promoter. For example, expression cassettes for use in the invention may comprise a promoter for the expression of the nucleotide sequence encoding a viral vector component and optionally a regulator of the nucleotide sequence encoding the viral vector component.

The choice of expression cassette, e.g. plasmid, cosmid, virus or phage vector, will often depend on the host cell into which it is to be introduced. The expression cassette can be a DNA plasmid (supercoiled, nicked or linearised), minicircle DNA (linear or supercoiled), plasmid DNA containing just the regions of interest by removal of the plasmid backbone by restriction enzyme digestion and purification, DNA generated using an enzymatic DNA amplification platform e.g. doggybone DNA (dbDNA^{™}) where the final DNA used is in a closed ligated form or where it has been prepared (e.g. restriction enzyme digestion) to have open cut ends.

### Set of nucleotide sequences

In one aspect, the invention provides a set of nucleotide sequences for producing a lentiviral vector comprising:
(i) a nucleic acid sequence encoding the lentiviral vector genome, wherein the lentiviral vector genome comprises a transgene expression cassette; and
(ii) at least one nucleic acid sequence encoding an interfering RNA which is specific for mRNA encoding the transgene.

In one aspect, the invention provides a set of nucleotide sequences for producing a lentiviral vector comprising:
(i) a nucleic acid sequence comprising a lentiviral vector genome expression cassette, wherein the lentiviral vector genome expression cassette comprises a transgene expression cassette; and
(ii) at least one nucleic acid sequence encoding an interfering RNA which is specific for mRNA encoding the transgene.

In a further aspect, the invention provides a set of nucleotide sequences comprising nucleotide sequences encoding lentiviral vector components and a nucleotide sequence of the invention.

In a further aspect, the invention provides a set of nucleotide sequences comprising nucleotide sequences encoding lentiviral vector components and a nucleic acid sequence encoding an interfering RNA of the invention.

In a further aspect, the invention provides a set of nucleotide sequences comprising nucleotide sequences encoding lentiviral vector components, a nucleotide sequence of the invention and a nucleic acid sequence encoding an interfering RNA of the invention.

In one embodiment, the set of nucleotide sequences for producing a lentiviral vector encodes the components required for production of the lentiviral vector. Accordingly, the set of nucleotide sequences may encode the lentiviral vector components necessary to generate viral vector particles. Suitably, lentiviral vector components necessary to generate viral vector particles include gag, env, the genome of the lentiviral vector (i.e. the RNA genome of the lentiviral vector) and/or optionally rev.

In some embodiments, the set of nucleic acid sequences further comprises nucleic acid sequences encoding Gag/pol and env or a functional substitute thereof.

In some embodiments, the set of nucleic acid sequences further comprises a nucleic acid sequence encoding rev or a functional substitute thereof. As described elsewhere herein, the use of the vector intron of the invention provides the advantage that the RRE can be omitted from the lentiviral vector genome (i.e. that lentiviral vector production can proceed in the absence of rev). Therefore, the set of nucleotide sequences may lack a nucleotide sequence encoding the Rev protein, or functional substitute thereof, particularly when the lentiviral vector genome comprises a vector intron as described herein.

The interfering RNA(s) can be provided *in trans* or *in cis* during lentiviral vector production. Cis elements are present on the same molecule of DNA as the gene they affect whereas *trans* elements can affect genes distant from the gene from which they were transcribed.

Thus, an interfering RNA expression cassette may be co-expressed with lentiviral vector components during lentiviral vector production. The interfering RNA is an interfering RNA as described herein. Thus, the interfering RNA targets the mRNA encoding the transgene, leading to mRNA degradation and concomitant reduction in dsRNA and transgene expression. Such an interfering RNA expression cassette may be easily constructed by those skilled in the art, for example driven by a U6 pol-Ill promoter or tRNA promoter. The target of the interfering RNA may be the sequence of the 3' UTR of the transgene mRNA encompassed by the VI.

Accordingly, in some embodiments, the set of nucleic acid sequences comprises a first nucleic acid sequence encoding the lentiviral vector genome and at least a second nucleic acid sequence encoding the interfering RNA. Preferably, the first and second nucleic acid sequences are separate nucleic acid sequences. Suitably, the nucleic acid encoding the lentiviral vector genome may not comprise the nucleic acid sequence encoding the interfering RNA.

In some embodiments, the nucleic acid encoding the lentiviral vector genome comprises the nucleic acid sequence encoding the interfering RNA.

### Lentiviral Vector Production Systems and Cells

In a further aspect, the invention provides a viral vector production system comprising a set of nucleotide sequences of the invention.

In a further aspect, the invention provides a viral vector production system comprising a set of nucleotide sequences, wherein the nucleotide sequences comprise nucleotide sequences encoding vector components including gag-pol, env, optionally rev, and a nucleotide sequence of the invention.

In a further aspect, the invention provides a cell comprising the nucleotide sequence of the invention, the expression cassette of the invention, the set of nucleotide sequences of the invention, or the vector production system of the invention.

In a further aspect, the invention provides a cell for producing lentiviral vectors comprising:
(a)
   (i) nucleotide sequences encoding vector components including gag-pol and env, and optionally rev, and the nucleotide sequence of the invention or the expression cassette of the invention, or the set of nucleotide sequences of the invention; or
   (ii) the viral vector production system of the invention; and
(b) optionally, a nucleotide sequence encoding a modified U1 snRNA and/or optionally a nucleotide sequence encoding TRAP.

In some embodiments, the splicing activity from the major splice donor site and/or splice donor region of the RNA genome of the lentiviral vector is suppressed or ablated.

In some embodiments, the splicing activity from the major splice donor site and/or splice donor region of the RNA genome of the lentiviral vector is suppressed or ablated during lentiviral vector production.

In a further aspect, the invention provides a method for producing a lentiviral vector, comprising the steps of:
(a) introducing:
   (i) nucleotide sequences encoding vector components including gag-pol and env, and optionally rev, and the nucleotide sequence of the invention or the expression cassette of the invention, or the set of nucleotide sequences of the invention; or
   (ii) the viral vector production system of the invention,
      into a cell; and
(b) optionally selecting for a cell that comprises nucleotide sequences encoding vector components and the RNA genome of the lentiviral vector; and
(c) culturing the cell under conditions suitable for the production of the lentiviral vector.

In a further aspect, the invention provides a lentiviral vector produced by the method of the invention.

In some embodiments, the lentiviral vector comprises the RNA genome of the lentiviral vector as described herein. Suitably, the lentiviral vector genome comprises a modified 3' LTR and/or a modified 5' LTR as described herein. Preferably, the lentiviral vector genome comprises a supA-LTR as described herein. Suitably, the lentiviral vector genome comprises a transgene expression cassette and a vector intron as described herein.

In a further aspect, the invention provides the use of the nucleotide sequence of the invention, the expression cassette of the invention, the set of nucleic acid sequences of the invention, the viral vector production system of the invention, or the cell of the invention, for producing a lentiviral vector.

A lentiviral vector production system comprises a set of nucleotide sequences encoding the components required for production of the lentiviral vector. Accordingly, a vector production system comprises a set of nucleotide sequences which encode the viral vector components necessary to generate lentiviral vector particles.

"Viral vector production system" or "vector production system" or "production system" is to be understood as a system comprising the necessary components for lentiviral vector production.

In an aspect, the viral vector production system comprises nucleotide sequences encoding Gag and Gag/Pol proteins, and Env protein and the vector genome sequence. The production system may optionally comprise a nucleotide sequence encoding the Rev protein, or functional substitute thereof. As described elsewhere herein, the use of the vector intron of the invention provides the advantage that the RRE can be omitted from the lentiviral vector genome (i.e. that lentiviral vector production can proceed in the absence of rev). Therefore, the production system may lack a nucleotide sequence encoding the Rev protein, or functional substitute thereof, particularly when the lentiviral vector genome comprises a vector intron as described herein.

In an aspect, the viral vector production system comprises modular nucleic acid constructs (modular constructs). A modular construct is a DNA expression construct comprising two or more nucleic acids used in the production of lentiviral vectors. A modular construct can be a DNA plasmid comprising two or more nucleic acids used in the production of lentiviral vectors. The plasmid may be a bacterial plasmid. The nucleic acids can encode for example, gag-pol, rev, env, vector genome. In addition, modular constructs designed for generation of packaging and producer cell lines may additionally need to encode transcriptional regulatory proteins (e.g. TetR, CymR) and/or translational repression proteins (e.g. TRAP) and selectable markers (e.g. Zeocin^{™}, hygromycin, blasticidin, puromycin, neomycin resistance genes). Suitable modular constructs for use in the present invention are described in EP 3502260, which is hereby incorporated by reference in its entirety.

As the modular constructs for use in accordance with the present invention contain nucleic acid sequences encoding two or more of the retroviral components on one construct, the safety profile of these modular constructs has been considered and additional safety features directly engineered into the constructs. These features include the use of insulators for multiple open reading frames of retroviral vector components and/or the specific orientation and arrangement of the retroviral genes in the modular constructs. It is believed that by using these features the direct read-through to generate replication-competent viral particles will be prevented.

The nucleic acid sequences encoding the viral vector components may be in reverse and/or alternating transcriptional orientations in the modular construct. Thus, the nucleic acid sequences encoding the viral vector components are not presented in the same 5' to 3' orientation, such that the viral vector components cannot be produced from the same mRNA molecule. The reverse orientation may mean that at least two coding sequences for different vector components are presented in the 'head-to-head' and 'tail-to-tail' transcriptional orientations. This may be achieved by providing the coding sequence for one vector component, e.g. env, on one strand and the coding sequence for another vector component, e.g. rev, on the opposing strand of the modular construct. Preferably, when coding sequences for more than two vector components are present in the modular construct, at least two of the coding sequences are present in the reverse transcriptional orientation. Accordingly, when coding sequences for more than two vector components are present in the modular construct, each component may be orientated such that it is present in the opposite 5' to 3' orientation to all of the adjacent coding sequence(s) for other vector components to which it is adjacent, i.e. alternating 5' to 3' (or transcriptional) orientations for each coding sequence may be employed.

The modular construct for use according to the present invention may comprise nucleic acid sequences encoding two or more of the following vector components: gag-pol, rev, an env, vector genome. The modular construct may comprise nucleic acid sequences encoding any combination of the vector components. In an aspect, the modular construct may comprise nucleic acid sequences encoding:
i) the RNA genome of the retroviral vector and rev, or a functional substitute thereof;
ii) the RNA genome of the retroviral vector and gag-pol;
iii) the RNA genome of the retroviral vector and env;
iv) gag-pol and rev, or a functional substitute thereof;
v) gag-pol and env;
vi) env and rev, or a functional substitute thereof;
vii) the RNA genome of the retroviral vector, rev, or a functional substitute thereof, and gag-pol;
viii) the RNA genome of the retroviral vector, rev, or a functional substitute thereof, and env;
ix) the RNA genome of the retroviral vector, gag-pol and env; or
x) gag-pol, rev, or a functional substitute thereof, and env,
wherein the nucleic acid sequences are in reverse and/or alternating orientations.

In one aspect, a cell for producing lentiviral vectors may comprise nucleic acid sequences encoding any one of the combinations i) to x) above, wherein the nucleic acid sequences are located at the same genetic locus and are in reverse and/or alternating orientations. The same genetic locus may refer to a single extrachromosomal locus in the cell, e.g. a single plasmid, or a single locus (i.e. a single insertion site) in the genome of the cell. The cell may be a stable or transient cell for producing lentiviral vectors. In one aspect the cell does not comprise tat.

The DNA expression construct can be a DNA plasmid (supercoiled, nicked or linearised), minicircle DNA (linear or supercoiled), plasmid DNA containing just the regions of interest by removal of the plasmid backbone by restriction enzyme digestion and purification, DNA generated using an enzymatic DNA amplification platform e.g. doggybone DNA (dbDNA^{™}) where the final DNA used is in a closed ligated form or where it has been prepared (e.g. restriction enzyme digestion) to have open cut ends.

In one aspect, the lentiviral vector is derived from HIV-1, HIV-2, SIV, FIV, BIV, EIAV, CAEV or Visna lentivirus.

A "viral vector production cell", "vector production cell", or "production cell" is to be understood as a cell that is capable of producing a lentiviral vector or lentiviral vector particle. Lentiviral vector production cells may be "producer cells" or "packaging cells". One or more DNA constructs of the viral vector system may be either stably integrated or episomally maintained within the viral vector production cell. Alternatively, all the DNA components of the viral vector system may be transiently transfected into the viral vector production cell. In yet another alternative, a production cell stably expressing some of the components may be transiently transfected with the remaining components required for vector production.

As used herein, the term "packaging cell" refers to a cell which contains the elements necessary for production of lentiviral vector particles but which lacks the vector genome. Optionally, such packaging cells contain one or more expression cassettes which are capable of expressing viral structural proteins (such as *gag, gag*/*pol* and *env*)*.*

Producer cells/packaging cells can be of any suitable cell type. Producer cells are generally mammalian cells but can be derived from other organisms, e.g. insect cells.

As used herein, the term "producer cell" or "vector producing/producer cell" refers to a cell which contains all the elements necessary for production of lentiviral vector particles. The producer cell may be either a stable producer cell line or derived transiently or may be a stable packaging cell wherein the lentiviral genome is transiently expressed.

In the methods of the invention, the vector components may include gag, env, rev and/or the RNA genome of the lentiviral vector when the viral vector is a lentiviral vector. The nucleotide sequences encoding vector components may be introduced into the cell either simultaneously or sequentially in any order.

The vector production cells may be cells cultured *in vitro* such as a tissue culture cell line. In some aspects of the methods and uses of the invention, suitable production cells or cells for producing a lentiviral vector are those cells which are capable of producing viral vectors or viral vector particles when cultured under appropriate conditions. Thus, the cells typically comprise nucleotide sequences encoding vector components, which may include gag, env, rev and the RNA genome of the lentiviral vector. Suitable cell lines include, but are not limited to, mammalian cells such as murine fibroblast derived cell lines or human cell lines. They are generally mammalian, including human cells, for example HEK293T, HEK293, CAP, CAP-T or CHO cells, but can be, for example, insect cells such as SF9 cells. Preferably, the vector production cells are derived from a human cell line. Accordingly, such suitable production cells may be employed in any of the methods or uses of the present invention.

Methods for introducing nucleotide sequences into cells are well known in the art and have been described previously. Thus, the introduction into a cell of nucleotide sequences encoding vector components including gag, env, rev and the RNA genome of the lentiviral vector, using conventional techniques in molecular and cell biology is within the capabilities of a person skilled in the art.

Stable production cells may be packaging or producer cells. To generate producer cells from packaging cells the vector genome DNA construct may be introduced stably or transiently. Packaging/producer cells can be generated by transducing a suitable cell line with a retroviral vector which expresses one of the components of the vector, i.e. a genome, the *gag-pol* components and an envelope as described in WO 2004/022761.

Alternatively, the nucleotide sequence can be transfected into cells and then integration into the production cell genome occurs infrequently and randomly. The transfection methods may be performed using methods well known in the art. For example, a stable transfection process may employ constructs which have been engineered to aid concatemerisation. In another example, the transfection process may be performed using calcium phosphate or commercially available formulations such as Lipofectamine^{™} 2000CD (Invitrogen, CA), FuGENE^{®} HD or polyethylenimine (PEI). Alternatively nucleotide sequences may be introduced into the production cell via electroporation. The skilled person will be aware of methods to encourage integration of the nucleotide sequences into production cells. For example, linearising a nucleic acid construct can help if it is naturally circular. Less random integration methodologies may involve the nucleic acid construct comprising of areas of shared homology with the endogenous chromosomes of the mammalian host cell to guide integration to a selected site within the endogenous genome. Furthermore, if recombination sites are present on the construct then these can be used for targeted recombination. For example, the nucleic acid construct may contain a *loxP* site which allows for targeted integration when combined with Cre recombinase (i.e. using the Cre/*lox* system derived from P1 bacteriophage). Alternatively or additionally, the recombination site is an *att* site (e.g. from λ phage), wherein the *att* site permits site-directed integration in the presence of a lambda integrase. This would allow the lentiviral genes to be targeted to a locus within the host cellular genome which allows for high and/or stable expression.

Other methods of targeted integration are well known in the art. For example, methods of inducing targeted cleavage of genomic DNA can be used to encourage targeted recombination at a selected chromosomal locus. These methods often involve the use of methods or systems to induce a double strand break (DSB) e.g. a nick in the endogenous genome to induce repair of the break by physiological mechanisms such as non-homologous end joining (NHEJ). Cleavage can occur through the use of specific nucleases such as engineered zinc finger nucleases (ZFN), transcription-activator like effector nucleases (TALENs), using CRISPR/Cas9 systems with an engineered crRNA/tracr RNA ('single guide RNA') to guide specific cleavage, and/or using nucleases based on the Argonaute system (e.g., from T. *thermophilus*)*.*

Packaging/producer cell lines can be generated by integration of nucleotide sequences using methods of just lentiviral transduction or just nucleic acid transfection, or a combination of both can be used.

Methods for generating retroviral vectors from production cells and in particular the processing of retroviral vectors are described in WO 2009/153563.

In one aspect, the production cell may comprise the RNA-binding protein (e.g. tryptophan RNA-binding attenuation protein, TRAP) and/or the Tet Repressor (TetR) protein or alternative regulatory proteins (e.g. CymR).

Production of lentiviral vector from production cells can be via transfection methods, from production from stable cell lines which can include induction steps (e.g. doxycycline induction) or via a combination of both. The transfection methods may be performed using methods well known in the art, and examples have been described previously.

Production cells, either packaging or producer cell lines or those transiently transfected with the lentiviral vector encoding components are cultured to increase cell and virus numbers and/or virus titres. Culturing a cell is performed to enable it to metabolize, and/or grow and/or divide and/or produce viral vectors of interest according to the invention. This can be accomplished by methods well known to persons skilled in the art, and includes but is not limited to providing nutrients for the cell, for instance in the appropriate culture media. The methods may comprise growth adhering to surfaces, growth in suspension, or combinations thereof. Culturing can be done for instance in tissue culture flasks, tissue culture multiwell plates, dishes, roller bottles, wave bags or in bioreactors, using batch, fed-batch, continuous systems and the like. In order to achieve large scale production of viral vector through cell culture it is preferred in the art to have cells capable of growing in suspension. Suitable conditions for culturing cells are known (see e.g. Tissue Culture, Academic Press, Kruse and Paterson, editors (1973), and R.I. Freshney, Culture of animal cells: A manual of basic technique, fourth edition (Wiley- Liss Inc., 2000, ISBN 0-471-34889-9).

Preferably cells are initially 'bulked up' in tissue culture flasks or bioreactors and subsequently grown in multi-layered culture vessels or large bioreactors (greater than 50L) to generate the vector producing cells for use in the present invention.

Preferably cells are grown in a suspension mode to generate the vector producing cells for use in the present invention.

### Lentiviral Vectors

Lentiviruses are part of a larger group of retroviruses. A detailed list of lentiviruses may be found in Coffin et al (1997) "Retroviruses" Cold Spring Harbour Laboratory Press Eds: JM Coffin, SM Hughes, HE Varmus pp 758-763). In brief, lentiviruses can be divided into primate and non-primate groups. Examples of primate lentiviruses include but are not limited to: the human immunodeficiency virus (HIV), the causative agent of human auto-immunodeficiency syndrome (AIDS), and the simian immunodeficiency virus (SIV). The non-primate lentiviral group includes the prototype "slow virus" Visna/maedi virus (VMV), as well as the related caprine arthritis-encephalitis virus (CAEV), equine infectious anaemia virus (EIAV), feline immunodeficiency virus (FIV), Maedi visna virus (MVV) and bovine immunodeficiency virus (BIV). In one aspect, the lentiviral vector is derived from HIV-1, HIV-2, SIV, FIV, BIV, EIAV, CAEV or Visna lentivirus.

The lentivirus family differs from retroviruses in that lentiviruses have the capability to infect both dividing and non-dividing cells (Lewis et al (1992) EMBO J 11(8):3053-3058 and Lewis and Emerman (1994) J Virol 68 (1):510-516). In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects or transduces target cells and expresses a nucleotide of interest (NOI), or nucleotides of interest.

The lentiviral vector may be used to replicate the NOI in a compatible target cell *in vitro.* Thus, described herein is a method of making proteins *in vitro* by introducing a vector of the invention into a compatible target cell *in vitro* and growing the target cell under conditions which result in expression of the NOI. Protein and NOI may be recovered from the target cell by methods well known in the art. Suitable target cells include mammalian cell lines and other eukaryotic cell lines.

In some aspects the vectors may have "insulators" - genetic sequences that block the interaction between promoters and enhancers, and act as a barrier reducing read-through from an adjacent gene.

In one aspect the insulator is present between one or more of the lentiviral nucleic acid sequences to prevent promoter interference and read-thorough from adjacent genes. If the insulators are present in the vector between one or more of the lentiviral nucleic acid sequences, then each of these insulated genes may be arranged as individual expression units.

The basic structure of retroviral and lentiviral genomes share many common features such as a 5' LTR and a 3' LTR, between or within which are located a packaging signal to enable the genome to be packaged, a primer binding site, integration sites to enable integration into a target cell genome and *gag*/*pol* and *env* genes encoding the packaging components - these are polypeptides required for the assembly of viral particles. Lentiviruses have additional features, such as the *rev* gene and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration, and transcription. LTRs also serve as enhancer-promoter sequences and can control the expression of the viral genes.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

In a typical retroviral vector as described herein, at least part of one or more protein coding regions essential for replication may be removed from the virus; for example, *gag*/*pol* and *env* may be absent or not functional. This makes the viral vector replication-defective.

The lentiviral vector may be derived from either a primate lentivirus (e.g. HIV-1) or a non-primate lentivirus (e.g. EIAV).

In general terms, a typical retroviral vector production system involves the separation of the viral genome from the essential viral packaging functions. These viral vector components are normally provided to the production cells on separate DNA expression cassettes (alternatively known as plasmids, expression plasmids, DNA constructs or expression constructs).

The vector genome comprises the NOI. Vector genomes typically require a packaging signal (ψ), the internal expression cassette harbouring the NOI, (optionally) a post-transcriptional element (PRE), typically a central polypurine tract (cppt), the 3'-ppu and a self-inactivating (SIN) LTR. The R-U5 regions are required for correct polyadenylation of both the vector genome RNA and NOI mRNA, as well as the process of reverse transcription. The vector genome may optionally include an open reading frame, as described in WO 2003/064665, which allows for vector production in the absence of rev.

The packaging functions include the *gag*/*pol* and *env* genes. These are required for the production of vector particles by the production cell. Providing these functions *in trans* to the genome facilitates the production of replication-defective viral vectors.

Production systems for gamma-retroviral vectors are typically 3-component systems requiring genome, *gag*/*pol* and *env* expression constructs. Production systems for HIV-1-based lentiviral vectors may additionally require the accessory gene *rev* to be provided and for the vector genome to include the rev-responsive element (RRE). EIAV-based lentiviral vectors do not require *rev* to be provided in *trans* if an open-reading frame (ORF) is present within the genome (see WO 2003/064665).

Usually both the "external" promoter (which drives the vector genome cassette) and "internal" promoter (which drives the NOI cassette) encoded within the vector genome cassette are strong eukaryotic or virus promoters, as are those driving the other vector system components. Examples of such promoters include CMV, EF1α, PGK, CAG, TK, SV40 and Ubiquitin promoters. Strong 'synthetic' promoters, such as those generated by DNA libraries (e.g. JeT promoter) may also be used to drive transcription. Alternatively, tissue-specific promoters such as rhodopsin (Rho), rhodopsin kinase (RhoK), cone-rod homeobox containing gene (CRX), neural retina-specific leucine zipper protein (NRL), Vitelliform Macular Dystrophy 2 (VMD2), Tyrosine hydroxylase, neuronal-specific neuronal-specific enolase (NSE) promoter, astrocyte-specific glial fibrillary acidic protein (GFAP) promoter, human α1-antitrypsin (hAAT) promoter, phosphoenolpyruvate carboxykinase (PEPCK), liver fatty acid binding protein promoter, Flt-1 promoter, INF-β promoter, Mb promoter, SP-B promoter, SYN1 promoter, WASP promoter, SV40 / hAlb promoter, SV40 / CD43, SV40 / CD45, NSE / RU5' promoter, ICAM-2 promoter, GPllb promoter, GFAP promoter, Fibronectin promoter, Endoglin promoter, Elastase-1 promoter, Desmin promoter, CD68 promoter, CD14 promoter and B29 promoter may be used to drive transcription.

Production of retroviral vectors involves either the transient co-transfection of the production cells with these DNA components or use of stable production cell lines wherein all the components are stably integrated within the production cell genome (e.g. Stewart HJ, Fong-Wong L, Strickland I, Chipchase D, Kelleher M, Stevenson L, Thoree V, McCarthy J, Ralph GS, Mitrophanous KA and Radcliffe PA. (2011). Hum Gene Ther. Mar; 22 (3):357-69). An alternative approach is to use a stable packaging cell (into which the packaging components are stably integrated) and then transiently transfect in the vector genome plasmid as required (e.g. Stewart, H. J., M. A. Leroux-Carlucci, C. J. Sion, K. A. Mitrophanous and P. A. Radcliffe (2009). Gene Ther. Jun; 16 (6):805-14). It is also feasible that alternative, not complete, packaging cell lines could be generated (just one or two packaging components are stably integrated into the cell lines) and to generate vector the missing components are transiently transfected. The production cell may also express regulatory proteins such as a member of the tet repressor (TetR) protein group of transcription regulators (e.g.T-Rex, Tet-On, and Tet-Off), a member of the cumate inducible switch system group of transcription regulators (e.g. cumate repressor (CymR) protein), or an RNA-binding protein (e.g. TRAP - tryptophan-activated RNA-binding protein).

In one aspect of the present invention, the viral vector is derived from EIAV. EIAV has the simplest genomic structure of the lentiviruses and is particularly preferred for use in the present invention. In addition to the *gag*/*pol* and *env* genes, EIAV encodes three other genes: *tat, rev,* and *S2. Tat* acts as a transcriptional activator of the viral LTR (Derse and Newbold (1993) Virology 194(2):530-536 and Maury et al (1994) Virology 200(2):632-642) and *rev* regulates and coordinates the expression of viral genes through rev-response elements (RRE) (Martarano et al. (1994) J Virol 68(5):3102-3111). The mechanisms of action of these two proteins are thought to be broadly similar to the analogous mechanisms in the primate viruses (Martarano et al. (1994) J Virol 68(5):3102-3111). The function of S2 is unknown. In addition, an EIAV protein, Ttm, has been identified that is encoded by the first exon of *tat* spliced to the *env* coding sequence at the start of the transmembrane protein. In an alternative aspect of the present invention the viral vector is derived from HIV: HIV differs from EIAV in that it does not encode S2 but unlike EIAV it encodes vif, vpr, vpu and nef.

The term "recombinant retroviral or lentiviral vector" (RRV) refers to a vector with sufficient retroviral genetic information to allow packaging of an RNA genome, in the presence of packaging components, into a viral particle capable of transducing a target cell. Transduction of the target cell may include reverse transcription and integration into the target cell genome. The RRV carries non-viral coding sequences which are to be delivered by the vector to the target cell. A RRV is incapable of independent replication to produce infectious retroviral particles within the target cell. Usually the RRV lacks a functional *gag*/*pol* and/or *env* gene, and/or other genes essential for replication.

Preferably the RRV vector of the present invention has a minimal viral genome.

As used herein, the term "minimal viral genome" means that the viral vector has been manipulated so as to remove the non-essential elements whilst retaining the elements essential to provide the required functionality to infect, transduce and deliver a NOI to a target cell. Further details of this strategy can be found in WO 1998/17815 and WO 99/32646. A minimal EIAV vector lacks *tat, rev* and S2 genes and neither are these genes provided in *trans* in the production system. A minimal HIV vector lacks vif, vpr, vpu, tat and nef.

The expression plasmid used to produce the vector genome within a production cell may include transcriptional regulatory control sequences operably linked to the retroviral genome to direct transcription of the genome in a production cell/packaging cell. All 3rd generation lentiviral vectors are deleted in the 5' U3 enhancer-promoter region, and transcription of the vector genome RNA is driven by heterologous promoter such as another viral promoter, for example the CMV promoter, as discussed below. This feature enables vector production independently of tat. Some lentiviral vector genomes require additional sequences for efficient virus production. For example, particularly in the case of HIV, RRE sequences may be included. However the requirement for RRE on the (separate) GagPol cassette (and dependence on rev which is provided *in trans*) may be reduced or eliminated by codon optimisation of the GagPol ORF. Further details of this strategy can be found in WO 2001/79518.

Alternative sequences which perform the same function as the rev/RRE system are also known. For example, a functional analogue of the rev/RRE system is found in the Mason Pfizer monkey virus. This is known as the constitutive transport element (CTE) and comprises an RRE-type sequence in the genome which is believed to interact with a factor in the infected cell. The cellular factor can be thought of as a *rev* analogue. Thus, CTE may be used as an alternative to the *rev*/RRE system. Any other functional equivalents of the Rev protein which are known or become available may be relevant to the invention. For example, it is also known that the Rex protein of HTLV-I can functionally replace the Rev protein of HIV-1. *Rev* and RRE may be absent or non-functional in the vector for use in the methods of the present invention; in the alternative *rev* and RRE, or functionally equivalent system, may be present.

It is therefore understood that 'rev' may refer to a sequence encoding the HIV-1 Rev protein or a sequence encoding any functional equivalent thereof. Thus, in an aspect, the invention provides a viral vector production system and/or a cell comprising a set of nucleotide sequences, wherein the nucleotide sequences encode vector components including gag-pol, env, optionally rev, and the nucleotide sequences of the invention.

As used herein, the term "functional substitute" means a protein or sequence having an alternative sequence which performs the same function as another protein or sequence. The term "functional substitute" is used interchangeably with "functional equivalent" and "functional analogue" herein with the same meaning.

### SIN Vectors

The lentiviral vectors as described herein may be used in a self-inactivating (SIN) configuration in which the viral enhancer and promoter sequences have been deleted. SIN vectors can be generated and transduce non-dividing target cells *in vivo, ex vivo* or *in vitro* with an efficacy similar to that of non-SIN vectors. The transcriptional inactivation of the long terminal repeat (LTR) in the SIN provirus should prevent mobilisation of vRNA, and is a feature that further diminishes the likelihood of formation of replication-competent virus. This should also enable the regulated expression of genes from internal promoters by eliminating any cis-acting effects of the LTR.

By way of example, self-inactivating retroviral vector systems have been constructed by deleting the transcriptional enhancers or the enhancers and promoter in the U3 region of the 3' LTR. After a round of vector reverse transcription and integration, these changes are copied into both the 5' and the 3' LTRs producing a transcriptionally inactive provirus. However, any promoter(s) internal to the LTRs in such vectors will still be transcriptionally active. This strategy has been employed to eliminate effects of the enhancers and promoters in the viral LTRs on transcription from internally placed genes. Such effects include increased transcription or suppression of transcription. This strategy can also be used to eliminate downstream transcription from the 3' LTR into genomic DNA. This is of particular concern in human gene therapy where it is important to prevent the adventitious activation of any endogenous oncogene. Yu et al., (1986) PNAS 83: 3194-98; Marty et al., (1990) Biochimie 72: 885-7; Naviaux et al., (1996) J. Virol. 70: 5701-5; Iwakuma et al., (1999) Virol. 261: 120-32; Deglon et al., (2000) Human Gene Therapy 11: 179-90. SIN lentiviral vectors are described in US 6,924,123 and US 7,056,699.

### Replication-Defective Lentiviral Vectors

In the genome of a replication-defective lentiviral vector the sequences of *gag*/*pol* and/or *env* may be mutated and/or not functional.

In a typical lentiviral vector as described herein, at least part of one or more coding regions for proteins essential for virus replication may be removed from the vector. This makes the viral vector replication-defective. Portions of the viral genome may also be replaced by a NOI in order to generate a vector comprising an NOI which is capable of transducing a non-dividing target cell and/or integrating its genome into the target cell genome.

In one aspect the lentiviral vectors are non-integrating vectors as described in WO 2006/010834 and WO 2007/071994.

In a further aspect the vectors have the ability to deliver a sequence which is devoid of or lacking viral RNA. In a further aspect a heterologous binding domain (heterologous to *gag*) located on the RNA to be delivered and a cognate binding domain on Gag or GagPol can be used to ensure packaging of the RNA to be delivered. Both of these vectors are described in WO 2007/072056.

### Adenoviral and Adeno-Associated Viral Vectors

An adenovirus is a double-stranded, linear DNA virus that does not replicate through an RNA intermediate. There are over 50 different human serotypes of adenovirus divided into 6 subgroups based on their genetic sequence.

Adenoviruses are double-stranded DNA non-enveloped viruses that are capable of in vivo, ex vivo and in vitro transduction of a broad range of cell types of human and non-human origin. These cells include respiratory airway epithelial cells, hepatocytes, muscle cells, cardiac myocytes, synoviocytes, primary mammary epithelial cells and post-mitotically terminally differentiated cells such as neurons.

Adenoviral vectors are also capable of transducing non-dividing cells. This is very important for diseases, such as cystic fibrosis, in which the affected cells in the lung epithelium have a slow turnover rate. In fact, several trials are underway utilising adenovirus-mediated transfer of cystic fibrosis transporter (CFTR) into the lungs of afflicted adult cystic fibrosis patients.

Adenoviruses have been used as vectors for gene therapy and for expression of heterologous genes. The large (36 kb) genome can accommodate up to 8 kb of foreign insert DNA and is able to replicate efficiently in complementing cell lines to produce very high titres of up to 1012 transducing units per ml. Adenovirus is thus one of the best systems to study the expression of genes in primary non-replicative cells.

The expression of viral or foreign genes from the adenovirus genome does not require a replicating cell. Adenoviral vectors enter cells by receptor-mediated endocytosis. Once inside the cell, adenovirus vectors rarely integrate into the host chromosome. Instead, they function episomally (independently from the host genome) as a linear genome in the host nucleus.

The use of recombinant adeno-associated viral (AAV) and Adenovirus based viral vectors for gene therapy is widespread, and manufacture of the same has been well documented. Typically, AAV-based vectors are produced in mammalian cell lines (e.g. HEK293-based) or through use of the baculovirus/Sf9 insect cell system. AAV vectors can be produced by transient transfection of vector component encoding DNAs, typically together with helper functions from Adenovirus or Herpes Simplex virus (HSV), or by use of cell lines stably expressing AAV vector components. Adenoviral vectors are typically produced in mammalian cell lines that stably express Adenovirus E1 functions (e.g. HEK293-based).

Adenoviral vectors are also typically 'amplified' via helper-function-dependent replication through serial rounds of 'infection' using the production cell line. An adenoviral vector and production system thereof comprises a polynucleotide comprising all or a portion of an adenovirus genome. It is well known that an adenovirus is, without limitation, an adenovirus derived from Ad2, Ad5, Ad12, and Ad40. An adenoviral vector is typically in the form of DNA encapsulated in an adenovirus coat or adenoviral DNA packaged in another viral or viral-like form (such as herpes simplex, and AAV).

An AAV vector it is commonly understood to be a vector derived from an adeno-associated virus serotype, including without limitation, AAV-1, AAV-2, AAV-3, AAV-4, AAV-5, AAV-6, AAV-7 and AAV-8. AAV vectors can have one or more of the AAV wild-type genes deleted in whole or part, preferably the rep and/or cap genes, but retain functional flanking ITR sequences. Functional ITR sequences are necessary for the rescue, replication and packaging of the AAV virion. Thus, an AAV vector is defined herein to include at least those sequences required in cis for replication and packaging (e.g., functional ITRs) of the virus. The ITRs need not be the wild-type nucleotide sequences, and may be altered, e.g., by the insertion, deletion or substitution of nucleotides, as long as the sequences provide for functional rescue, replication and packaging. An 'AAV vector' also refers to its protein shell or capsid, which provides an efficient vehicle for delivery of vector nucleic acid to the nucleus of target cells. AAV production systems require helper functions, which typically refers to AAV-derived coding sequences which can be expressed to provide AAV gene products that, in turn, function in trans for productive AAV replication. As such, AAV helper functions include both of the major AAV open reading frames (ORFs), rep and cap. The Rep expression products have been shown to possess many functions, including, among others: recognition, binding and nicking of the AAV origin of DNA replication; DNA helicase activity; and modulation of transcription from AAV (or other heterologous) promoters. The Cap expression products supply necessary packaging functions. AAV helper functions are used herein to complement AAV functions in trans that are missing from AAV vectors. It is understood that an AAV helper construct refers generally to a nucleic acid molecule that includes nucleotide sequences providing AAV functions deleted from an AAV vector which is to be used to produce a transducing vector for delivery of a nucleotide sequence of interest. AAV helper constructs are commonly used to provide transient expression of AAV rep and/or cap genes to complement missing AAV functions that are necessary for AAV replication; however, helper constructs lack AAV ITRs and can neither replicate nor package themselves. AAV helper constructs can be in the form of a plasmid, phage, transposon, cosmid, virus, or virion. A number of AAV helper constructs have been described, such as the commonly used plasmids pAAV/Ad and pIM29+45 which encode both Rep and Cap expression products. See, e.g., Samulski et al. (1989) J. Virol. 63:3822-3828; and McCarty et al. (1991) J. Virol. 65:2936-2945. A number of other vectors have been described which encode Rep and/or Cap expression products. See, e.g., U.S. Pat. Nos. 5,139,941 and 6,376,237. In addition, it is common knowledge that the term "accessory functions" refers to non-AAV derived viral and/or cellular functions upon which AAV is dependent for its replication. Thus, the term captures proteins and RNAs that are required in AAV replication, including those moieties involved in activation of AAV gene transcription, stage specific AAV mRNA splicing, AAV DNA replication, synthesis of Cap expression products and AAV capsid assembly. Viral-based accessory functions can be derived from any of the known helper viruses such as adenovirus, herpesvirus (other than herpes simplex virus type-1) and vaccinia virus.

### NOI and Polynucleotides

Polynucleotides of the invention may comprise DNA or RNA. They may be single-stranded or double-stranded. A nucleotide, or nucleotides, of interest is/are commonly referred to as NOI. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

The polynucleotides may be modified by any method available in the art. Such modifications may be carried out in order to enhance the *in vivo* activity or lifespan of the polynucleotides of the invention.

Polynucleotides such as DNA polynucleotides may be produced recombinantly, synthetically or by any means available to those of skill in the art. They may also be cloned by standard techniques.

Longer polynucleotides will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing PCR under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

### Common Retroviral Vector Elements

### Promoters and Enhancers

Expression of a NOI and polynucleotide may be controlled using control sequences for example transcription regulation elements or translation repression elements, which include promoters, enhancers and other expression regulation signals (e.g. tet repressor (TetR) system) or the Transgene Repression In vector Production cell system (TRiP) or other regulators of NOls described herein.

Prokaryotic promoters and promoters functional in eukaryotic cells may be used. Tissue-specific or stimuli-specific promoters may be used. Chimeric promoters may also be used comprising sequence elements from two or more different promoters.

Suitable promoting sequences are strong promoters including those derived from the genomes of viruses, such as polyoma virus, adenovirus, fowlpox virus, bovine papilloma virus, avian sarcoma virus, cytomegalovirus (CMV), retrovirus and Simian Virus 40 (SV40), or from heterologous mammalian promoters, such as the actin promoter, EF1α, CAG, TK, SV40, ubiquitin, PGK or ribosomal protein promoter. Alternatively, tissue-specific promoters such as rhodopsin (Rho), rhodopsin kinase (RhoK), cone-rod homeobox containing gene (CRX), neural retina-specific leucine zipper protein (NRL), Vitelliform Macular Dystrophy 2 (VMD2), Tyrosine hydroxylase, neuronal-specific neuronal-specific enolase (NSE) promoter, astrocyte-specific glial fibrillary acidic protein (GFAP) promoter, human α1-antitrypsin (hAAT) promoter, phosphoenolpyruvate carboxykinase (PEPCK), liver fatty acid binding protein promoter, Flt-1 promoter, INF-β promoter, Mb promoter, SP-B promoter, SYN1 promoter, WASP promoter, SV40 / hAlb promoter, SV40 / CD43, SV40 / CD45, NSE / RU5' promoter, ICAM-2 promoter, GPllb promoter, GFAP promoter, Fibronectin promoter, Endoglin promoter, Elastase-1 promoter, Desmin promoter, CD68 promoter, CD14 promoter and B29 promoter may be used to drive transcription.

Transcription of a NOI may be increased further by inserting an enhancer sequence into the vector. Enhancers are relatively orientation- and position-independent; however, one may employ an enhancer from a eukaryotic cell virus, such as the SV40 enhancer and the CMV early promoter enhancer. The enhancer may be spliced into the vector at a position 5' or 3' to the promoter, but is preferably located at a site 5' from the promoter.

The promoter can additionally include features to ensure or to increase expression in a suitable target cell. For example, the features can be conserved regions e.g. a Pribnow Box or a TATA box. The promoter may contain other sequences to affect (such as to maintain, enhance or decrease) the levels of expression of a nucleotide sequence. Suitable other sequences include the Sh1-intron or an ADH intron. Other sequences include inducible elements, such as temperature, chemical, light or stress inducible elements. Also, suitable elements to enhance transcription or translation may be present.

### Regulators of NOIs

A complicating factor in the generation of retroviral packaging/producer cell lines and retroviral vector production is that constitutive expression of certain retroviral vector components and NOls are cytotoxic leading to death of cells expressing these components and therefore inability to produce vector. Therefore, the expression of these components (e.g. gag-pol and envelope proteins such as VSV-G) can be regulated. The expression of other non-cytotoxic vector components, e.g. rev, can also be regulated to minimise the metabolic burden on the cell. The modular constructs and/or cells as described herein may comprise cytotoxic and/or non-cytotoxic vector components associated with at least one regulatory element. As used herein, the term "regulatory element" refers to any element capable of affecting, either increasing or decreasing, the expression of an associated gene or protein. A regulatory element includes a gene switch system, transcription regulation element and translation repression element.

A number of prokaryotic regulator systems have been adapted to generate gene switches in mammalian cells. Many retroviral packaging and producer cell lines have been controlled using gene switch systems (e.g. tetracycline and cumate inducible switch systems) thus enabling expression of one or more of the retroviral vector components to be switched on at the time of vector production. Gene switch systems include those of the (TetR) protein group of transcription regulators (e.g.T-Rex, Tet-On, and Tet-Off), those of the cumate inducible switch system group of transcription regulators (e.g. CymR protein) and those involving an RNA-binding protein (e.g. TRAP).

One such tetracycline-inducible system is the tetracycline repressor (TetR) system based on the T-REx^{™} system. By way of example, in such a system tetracycline operators (TetO2) are placed in a position such that the first nucleotide is 10bp from the 3' end of the last nucleotide of the TATATAA element of the human cytomegalovirus major immediate early promoter (hCMVp) then TetR alone is capable of acting as a repressor (Yao F, Svensjo T, Winkler T, Lu M, Eriksson C, Eriksson E. Tetracycline repressor, tetR, rather than the tetR-mammalian cell transcription factor fusion derivatives, regulates inducible gene expression in mammalian cells. 1998. Hum Gene Ther, 9: 1939-1950). In such a system the expression of the NOI can be controlled by a CMV promoter into which two copies of the TetO2 sequence have been inserted in tandem. TetR homodimers, in the absence of an inducing agent (tetracycline or its analogue doxycycline [dox]), bind to the TetO2 sequences and physically block transcription from the upstream CMV promoter. When present, the inducing agent binds to the TetR homodimers, causing allosteric changes such that it can no longer bind to the TetO2 sequences, resulting in gene expression. The TetR gene may be codon optimised as this may improve translation efficiency resulting in tighter control of TetO2 controlled gene expression.

The TRiP system is described in WO 2015/092440 and provides another way of repressing expression of the NOI in the production cells during vector production. The TRAP-binding sequence (e.g. TRAP-tbs) interaction forms the basis for a transgene protein repression system for the production of retroviral vectors, when a constitutive and/or strong promoter, including a tissue-specific promoter, driving the transgene is desirable and particularly when expression of the transgene protein in production cells leads to reduction in vector titres and/or elicits an immune response *in vivo* due to viral vector delivery of transgene-derived protein (Maunder et al, Nat Commun. (2017) Mar 27; 8).

Briefly, the TRAP-tbs interaction forms a translational block, repressing translation of the transgene protein (Maunder et al, Nat Commun. (2017) Mar 27; 8). The translational block is only effective in production cells and as such does not impede the DNA- or RNA- based vector systems. The TRiP system is able to repress translation when the transgene protein is expressed from a constitutive and/or strong promoter, including a tissue-specific promoter from single- or multi cistronic mRNA. It has been demonstrated that unregulated expression of transgene protein can reduce vector titres and affect vector product quality. Repression of transgene protein for both transient and stable PaCL/PCL vector production systems is beneficial for production cells to prevent a reduction in vector titres: where toxicity or molecular burden issues may lead to cellular stress; where transgene protein elicits an immune response *in vivo* due to viral vector delivery of transgene-derived protein; where the use of gene-editing transgenes may result in on/off target affects; where the transgene protein may affect vector and/or envelope glycoprotein exclusion.

### Envelope and Pseudotyping

In one preferred aspect, the lentiviral vector as described herein has been pseudotyped. In this regard, pseudotyping can confer one or more advantages. For example, the *env* gene product of the HIV based vectors would restrict these vectors to infecting only cells that express a protein called CD4. But if the *env* gene in these vectors has been substituted with *env* sequences from other enveloped viruses, then they may have a broader infectious spectrum (Verma and Somia (1997) Nature 389(6648):239-242). By way of example, workers have pseudotyped an HIV based vector with the glycoprotein from VSV (Verma and Somia (1997) Nature 389(6648):239-242). Accordingly, alternative sequences which perform the equivalent function as the *env* gene product of HIV based vectors are also known.

In another alternative, the Env protein may be a modified Env protein such as a mutant or engineered Env protein. Modifications may be made or selected to introduce targeting ability or to reduce toxicity or for another purpose (Valsesia-Wittman et al 1996 J Virol 70: 2056-64; Nilson et al (1996) Gene Ther 3(4):280-286; and Fielding et al (1998) Blood 91(5):1802-1809 and references cited therein).

The vector may be pseudotyped with any molecule of choice.

As used herein, "env" shall mean an endogenous lentiviral envelope or a heterologous envelope, as described herein. Suitably, env may be Env of HIV based vectors or a functional substitute thereof.

### VSV-G

The envelope glycoprotein (G) of Vesicular stomatitis virus (VSV), a rhabdovirus, is an envelope protein that has been shown to be capable of pseudotyping certain enveloped viruses and viral vector virions.

Its ability to pseudotype MoMLV-based retroviral vectors in the absence of any retroviral envelope proteins was first shown by Emi et al. (1991) Journal of Virology 65:1202-1207. WO 1994/294440 teaches that retroviral vectors may be successfully pseudotyped with VSV-G. These pseudotyped VSV-G vectors may be used to transduce a wide range of mammalian cells. More recently, Abe et al. (1998) J Virol 72(8) 6356-6361 teach that non-infectious retroviral particles can be made infectious by the addition of VSV-G.

Burns et al. (1993) Proc. Natl. Acad. Sci. USA 90:8033-7 successfully pseudotyped the retrovirus MLV with VSV-G and this resulted in a vector having an altered host range compared to MLV in its native form. VSV-G pseudotyped vectors have been shown to infect not only mammalian cells, but also cell lines derived from fish, reptiles and insects (Burns *et al.* (1993) ibid). They have also been shown to be more efficient than traditional amphotropic envelopes for a variety of cell lines (Yee et al., (1994) Proc. Natl. Acad. Sci. USA 91:9564-9568, Emi et al. (1991) Journal of Virology 65:1202-1207). VSV-G protein can be used to pseudotype certain retroviruses because its cytoplasmic tail is capable of interacting with the retroviral cores.

The provision of a non-retroviral pseudotyping envelope such as VSV-G protein gives the advantage that vector particles can be concentrated to a high titre without loss of infectivity (Akkina et al. (1996) J. Virol. 70:2581-5). Retrovirus envelope proteins are apparently unable to withstand the shearing forces during ultracentrifugation, probably because they consist of two non-covalently linked subunits. The interaction between the subunits may be disrupted by the centrifugation. In comparison the VSV glycoprotein is composed of a single unit. VSV-G protein pseudotyping can therefore offer potential advantages for both efficient target cell infection/transduction and during manufacturing processes.

WO 2000/52188 describes the generation of pseudotyped retroviral vectors, from stable producer cell lines, having vesicular stomatitis virus-G protein (VSV-G) as the membrane-associated viral envelope protein, and provides a gene sequence for the VSV-G protein.

### Ross River Virus

The Ross River viral envelope has been used to pseudotype a non-primate lentiviral vector (FIV) and following systemic administration predominantly transduced the liver (Kang et al., 2002, J. Virol., 76:9378-9388). Efficiency was reported to be 20-fold greater than obtained with VSV-G pseudotyped vector, and caused less cytotoxicity as measured by serum levels of liver enzymes suggestive of hepatotoxicity.

### Baculovirus GP64

The baculovirus GP64 protein has been shown to be an alternative to VSV-G for viral vectors used in the large-scale production of high-titre virus required for clinical and commercial applications (Kumar M, Bradow BP, Zimmerberg J (2003) Hum Gene Ther. 14(1):67-77). Compared with VSV-G-pseudotyped vectors, GP64-pseudotyped vectors have a similar broad tropism and similar native titres. Because, GP64 expression does not kill cells, HEK293T-based cell lines constitutively expressing GP64 can be generated.

### Alternative Envelopes

Other envelopes which give reasonable titre when used to pseudotype EIAV include Mokola, Rabies, Ebola and LCMV (lymphocytic choriomeningitis virus). Intravenous infusion into mice of lentivirus pseudotyped with 4070A led to maximal gene expression in the liver.

### Packaging Sequence

As utilized within the context of the present invention the term "packaging signal", which is referred to interchangeably as "packaging sequence" or "psi", is used in reference to the noncoding, cis-acting sequence required for encapsidation of retroviral RNA strands during viral particle formation. In HIV-1, this sequence has been mapped to loci extending from upstream of the major splice donor site (SD) to at least the gag start codon (some or all of the 5' sequence of gag to nucleotide 688 may be included). In EIAV the packaging signal comprises the R region into the 5' coding region of Gag.

As used herein, the term "extended packaging signal" or "extended packaging sequence" refers to the use of sequences around the psi sequence with further extension into the *gag* gene. The inclusion of these additional packaging sequences may increase the efficiency of insertion of vector RNA into viral particles.

Feline immunodeficiency virus (FIV) RNA encapsidation determinants have been shown to be discrete and non-continuous, comprising one region at the 5' end of the genomic mRNA (R-U5) and another region that mapped within the proximal 311 nt of gag (Kaye et al., J Virol. Oct;69(10):6588-92 (1995).

### Internal Ribosome Entry Site (IRES)

Insertion of IRES elements allows expression of multiple coding regions from a single promoter (Adam et al (as above); Koo et al (1992) Virology 186:669-675; Chen et al 1993 J. Virol 67:2142-2148). IRES elements were first found in the non-translated 5' ends of picornaviruses where they promote cap-independent translation of viral proteins (Jang et al (1990) Enzyme 44: 292-309). When located between open reading frames in an RNA, IRES elements allow efficient translation of the downstream open reading frame by promoting entry of the ribosome at the IRES element followed by downstream initiation of translation.

A review on IRES is presented by Mountford and Smith (TIG May 1995 vol 11, No 5:179-184). A number of different IRES sequences are known including those from encephalomyocarditis virus (EMCV) (Ghattas, I.R., etal., Mol. Cell. Biol., 11:5848-5859 (1991); BiP protein [Macejak and Sarnow, Nature 353:91 (1991)]; the Antennapedia gene of Drosophila (exons d and e) [Oh, et al., Genes & Development, 6:1643-1653 (1992)] as well as those in polio virus (PV) [Pelletier and Sonenberg, Nature 334: 320-325 (1988); see also Mountford and Smith, TIG 11, 179-184 (1985)].

IRES elements from PV, EMCV and swine vesicular disease virus have previously been used in retroviral vectors (Coffin et al, as above).

The term "IRES" includes any sequence or combination of sequences which work as or improve the function of an IRES. The IRES(s) may be of viral origin (such as EMCV IRES, PV IRES, or FMDV 2A-like sequences) or cellular origin (such as FGF2 IRES, NRF IRES, Notch 2 IRES or EIF4 IRES).

In order for the IRES to be capable of initiating translation of each polynucleotide it should be located between or prior to the polynucleotides in the modular construct.

The nucleotide sequences utilised for development of stable cell lines require the addition of selectable markers for selection of cells where stable integration has occurred. These selectable markers can be expressed as a single transcription unit within the nucleotide sequence or it may be preferable to use IRES elements to initiate translation of the selectable marker in a polycistronic message (Adam et al 1991 J.Virol. 65, 4985).

### Genetic Orientation and Insulators

It is well known that nucleic acids are directional and this ultimately affects mechanisms such as transcription and replication in the cell. Thus genes can have relative orientations with respect to one another when part of the same nucleic acid construct.

In certain aspects of the present invention, at least two nucleic acid sequences present at the same locus in the cell or construct can be in a reverse and/or alternating orientations. In other words, in certain aspects of the invention at this particular locus, the pair of sequential genes will not have the same orientation. This can help prevent both transcriptional and translational read-through when the region is expressed within the same physical location of the host cell.

Having the alternating orientations benefits retroviral vector production when the nucleic acids required for vector production are based at the same genetic locus within the cell. This in turn can also improve the safety of the resulting constructs in preventing the generation of replication-competent retroviral vectors.

When nucleic acid sequences are in reverse and/or alternating orientations the use of insulators can prevent inappropriate expression or silencing of a NOI from its genetic surroundings.

The term "insulator" refers to a class of nucleotide, e.g. DNA, sequence elements that when bound to insulator-binding proteins possess an ability to protect genes from surrounding regulator signals. There are two types of insulators: an enhancer blocking function and a chromatin barrier function. When an insulator is situated between a promoter and an enhancer, the enhancer-blocking function of the insulator shields the promoter from the transcription-enhancing influence of the enhancer (Geyer and Corces 1992; Kellum and Schedl 1992). The chromatin barrier insulators function by preventing the advance of nearby condensed chromatin which would lead to a transcriptionally active chromatin region turning into a transcriptionally inactive chromatin region and resulting in silencing of gene expression. Insulators which inhibit the spread of heterochromatin, and thus gene silencing, recruit enzymes involved in histone modifications to prevent this process (Yang J, Corces VG. 2011;110:43-76; Huang, Li et al. 2007; Dhillon, Raab et al. 2009). An insulator can have one or both of these functions and the chicken β-globin insulator (cHS4) is one such example. This insulator is the most extensively studied vertebrate insulator, is highly rich in G+C and has both enhancer-blocking and heterochromatic barrier functions (Chung J H, Whitely M, Felsenfeld G. Cell. 1993;74:505-514). Other such insulators with enhancer blocking functions are not limited to but include the following: human β-globin insulator 5 (HS5), human β-globin insulator 1 (HS1), and chicken β-globin insulator (cHS3) (Farrell CM1, West AG, Felsenfeld G., Mol Cell Biol. 2002 Jun;22(11):3820-31; J Ellis et al. EMBO J. 1996 Feb 1; 15(3): 562-568). In addition to reducing unwanted distal interactions the insulators also help to prevent promoter interference (i.e. where the promoter from one transcription unit impairs expression of an adjacent transcription unit) between adjacent retroviral nucleic acid sequences. If the insulators are used between each of the retroviral vector nucleic acid sequences, then the reduction of direct read-through will help prevent the formation of replication-competent retroviral vector particles.

The insulator may be present between each of the retroviral nucleic acid sequences. In one aspect, the use of insulators prevents promoter-enhancer interactions from one NOI expression cassette interacting with another NOI expression cassette in a nucleotide sequence encoding vector components.

An insulator may be present between the vector genome and *gag-pol* sequences. This therefore limits the likelihood of the production of a replication-competent retroviral vector and 'wild-type' like RNA transcripts, improving the safety profile of the construct. The use of insulator elements to improve the expression of stably integrated multigene vectors is cited in Moriarity et al, Nucleic Acids Res. 2013 Apr;41(8):e92.

### Vector Titre

The skilled person will understand that there are a number of different methods of determining the titre of lentiviral vectors. Titre is often described as transducing units/mL (TU/mL). Titre may be increased by increasing the number of vector particles and by increasing the specific activity of a vector preparation.

### Therapeutic Use

The lentiviral vector as described herein or a cell or tissue transduced with the lentiviral vector as described herein may be used in medicine.

In addition, the lentiviral vector as described herein, a production cell of the invention or a cell or tissue transduced with the lentiviral vector as described herein may be used for the preparation of a medicament to deliver a nucleotide of interest to a target site in need of the same. Such uses of the lentiviral vector or transduced cell of the invention may be for therapeutic or diagnostic purposes, as described previously.

Accordingly, there is provided a cell transduced by the lentiviral vector as described herein.

A "cell transduced by a viral vector particle" is to be understood as a cell, in particular a target cell, into which the nucleic acid carried by the viral vector particle has been transferred.

### Nucleotide of interest

In one embodiment of the invention, the nucleotide of interest (i.e. transgene) is translated in a target cell which lacks TRAP.

"Target cell" is to be understood as a cell in which it is desired to express the NOI. The NOI may be introduced into the target cell using a viral vector of the present invention. Delivery to the target cell may be performed *in vivo, ex vivo* or *in vitro.*

In a preferred embodiment, the nucleotide of interest gives rise to a therapeutic effect.

The NOI may have a therapeutic or diagnostic application. Suitable NOls include, but are not limited to sequences encoding enzymes, co-factors, cytokines, chemokines, hormones, antibodies, anti-oxidant molecules, engineered immunoglobulin-like molecules, single chain antibodies, fusion proteins, immune co-stimulatory molecules, immunomodulatory molecules, chimeric antigen receptors a transdomain negative mutant of a target protein, toxins, conditional toxins, antigens, transcription factors, structural proteins, reporter proteins, subcellular localization signals, tumour suppressor proteins, growth factors, membrane proteins, receptors, vasoactive proteins and peptides, anti-viral proteins and ribozymes, and derivatives thereof (such as derivatives with an associated reporter group). The NOIs may also encode micro-RNA..

In one embodiment, the NOI may be useful in the treatment of a neurodegenerative disorder.

In another embodiment, the NOI may be useful in the treatment of Parkinson's disease and/or multiple system atrophy.

In another embodiment, the NOI may encode an enzyme or enzymes involved in dopamine synthesis. For example, the enzyme may be one or more of the following: tyrosine hydroxylase, GTP-cyclohydrolase I and/or aromatic amino acid dopa decarboxylase. The sequences of all three genes are available (GenBank^{®} Accession Nos. X05290, U19523 and M76180, respectively).

In another embodiment, the NOI may encode the vesicular monoamine transporter 2 (VMAT2). In an alternative embodiment, the viral genome may comprise a NOI encoding aromatic amino acid dopa decarboxylase and a NOI encoding VMAT2. Such a genome may be used in the treatment of Parkinson's disease, in particular in conjunction with peripheral administration of L-DOPA.

In another embodiment, the NOI may encode a therapeutic protein or combination of therapeutic proteins.

In another embodiment, the NOI may encode a protein or proteins selected from the group consisting of glial cell derived neurotrophic factor (GDNF), brain derived neurotrophic factor (BDNF), ciliary neurotrophic factor (CNTF), neurotrophin-3 (NT-3), acidic fibroblast growth factor (aFGF), basic fibroblast growth factor (bFGF), interleukin-1 beta (IL-1β), tumor necrosis factor alpha (TNF-α), insulin growth factor-2, VEGF-A, VEGF-B, VEGF-C/VEGF-2, VEGF-D, VEGF-E, PDGF-A, PDGF-B, hetero- and homo-dimers of PDFG-A and PDFG-B.

In another embodiment, the NOI may encode an anti-angiogenic protein or anti-angiogenic proteins selected from the group consisting of angiostatin, endostatin, platelet factor 4, pigment epithelium derived factor (PEDF), placental growth factor, restin, interferon-α, interferon-inducible protein, gro-beta and tubedown-1, interleukin(IL)-1, IL-12, retinoic acid, anti-VEGF antibodies or fragments /variants thereof such as aflibercept, thrombospondin, VEGF receptor proteins such as those described in US 5,952,199 and US 6,100,071, and anti-VEGF receptor antibodies.

In another embodiment, the NOI may encode anti-inflammatory proteins, antibodies or fragment/variants of proteins or antibodies selected from the group consisting of NF-kB inhibitors, IL1beta inhibitors, TGFbeta inhibitors, IL-6 inhibitors, IL-23 inhibitors, IL-18 inhibitors, Tumour necrosis factor alpha and Tumour necrosis factor beta, Lymphotoxin alpha and Lymphotoxin beta, LIGHT inhibitors, alpha synuclein inhibitors, Tau inhibitors, beta amyloid inhibitors, IL-17 inhibitors, IL-33 inhibitors, IL-33 receptor inhibitors and TSLP inhibitors.

In another embodiment the NOI may encode cystic fibrosis transmembrane conductance regulator (CFTR).

In another embodiment the NOI may encode a protein normally expressed in an ocular cell.

In another embodiment, the NOI may encode a protein normally expressed in a photoreceptor cell and/or retinal pigment epithelium cell.

In another embodiment, the NOI may encode a protein selected from the group comprising RPE65, arylhydrocarbon-interacting receptor protein like 1 (AIPL1), CRB1, lecithin retinal acetyltransferace (LRAT), photoreceptor-specific homeo box (CRX), retinal guanylate cyclise (GUCY2D), RPGR interacting protein 1 (RPGRIP1), LCA2, LCA3, LCA5, dystrophin, PRPH2, CNTF, ABCR/ABCA4, EMP1, TIMP3, MERTK, ELOVL4, MYO7A, USH2A, VMD2, RLBP1, COX-2, FPR, harmonin, Rab escort protein 1, CNGB2, CNGA3, CEP 290, RPGR, RS1, RP1, PRELP, glutathione pathway enzymes and opticin.

In other embodiments, the NOI may encode the human clotting Factor VIII or Factor IX.

In other embodiments, the NOI may encode protein or proteins involved in metabolism selected from the group comprising phenylalanine hydroxylase (PAH), Methylmalonyl CoA mutase, Propionyl CoA carboxylase, Isovaleryl CoA dehydrogenase, Branched chain ketoacid dehydrogenase complex, Glutaryl CoA dehydrogenase, Acetyl CoA carboxylase, propionyl CoA carboxylase, 3 methyl crotonyl CoA carboxylase, pyruvate carboxylase, carbamoyl-phophate synthase ammonia, ornithine transcarbamylase, glucosylceramidase beta, alpha galactosidase A, glucosylceramidase beta, cystinosin, glucosamine(N-acetyl)-6-sulfatase, N-acetyl-alpha-glucosaminidase, glucose-6-phosphatase, ATP7B, ATP8B1, ABCB11, ABCB4, TJP2, N-sulfoglucosamine sulfohydrolase, Galactosamine-6 sulfatase, arylsulfatase A, cytochrome B-245 beta, ABCD1, ornithine carbamoyltransferase, argininosuccinate synthase, argininosuccinate lysase, arginase 1, alanine glycoxhylate amino transferase, ATP-binding cassette, sub-family B members.

In other embodiments, the NOI may encode a chimeric antigen receptor (CAR) or a T cell receptor (TCR). In one embodiment, the CAR is an anti-5T4 CAR. In other embodiments, the NOI may encode B-cell maturation antigen (BCMA), CD19, CD22, CD20, CD138, CD30, CD33, CD123, CD70, prostate specific membrane antigen (PSMA), Lewis Y antigen (LeY), Tyrosine-protein kinase transmembrane receptor (ROR1), Mucin 1, cell surface associated (Muc1), Epithelial cell adhesion molecule (EpCAM), endothelial growth factor receptor (EGFR), insulin, protein tyrosine phosphatase, non-receptor type 22, interleukin 2 receptor, alpha, interferon induced with helicase C domain 1, human epidermal growth factor receptor (HER2), glypican 3 (GPC3), disialoganglioside (GD2), mesiothelin, vesicular endothelial growth factor receptor 2 (VEGFR2), Smith antigen, Ro60, double stranded DNA, phospholipids, proinsulin, insulinoma antigen 2 (IA-2) , 65 kDa isoform of glutamic acid decarboxylase (GAD65), chromogranin A (CHGA), islet amyloid polypeptide (IAPP), islet-specific glucose-6-phosphatase catalytic subunit-related protein (IGRP), zinc transporter 8 (ZnT8).

In other embodiments, the NOI may encode a chimeric antigen receptor (CAR) against NKG2D ligands selected from the group comprising ULBP1, 2 and 3, H60, Rae-1a, b, g, d, MICA, MICB.

In further embodiments the NOI may encode SGSH, SUMF1, GAA, the common gamma chain (CD132), adenosine deaminase, WAS protein, globins, alpha galactosidase A, δ-aminolevulinate (ALA) synthase, δ-aminolevulinate dehydratase (ALAD), Hydroxymethylbilane (HMB) synthase, Uroporphyrinogen (URO) synthase, Uroporphyrinogen (URO) decarboxylase, Coproporphyrinogen (COPRO) oxidase, Protoporphyrinogen (PROTO) oxidase, Ferrochelatase, α-L-iduronidase, Iduronate sulfatase, Heparan sulfamidase, N-acetylglucosaminidase, Heparan-α-glucosaminide N-acetyltransferase, 3 N-acetylglucosamine 6-sulfatase, Galactose-6-sulfate sulfatase, β-galactosidase, N-acetylgalactosamine-4-sulfatase, β-glucuronidase and Hyaluronidase.

In addition to the NOI the vector may also comprise or encode a siRNA, shRNA, or regulated shRNA (Dickins et al. (2005) Nature Genetics 37: 1289-1295, Silva et al. (2005) Nature Genetics 37:1281-1288).

### Indications

The vectors, including retroviral and AAV vectors, according to the present invention may be used to deliver one or more NOI(s) useful in the treatment of the disorders listed in WO 1998/05635, WO 1998/07859, and WO 1998/09985. The nucleotide of interest may be DNA or RNA. Examples of such diseases are given below:
A disorder which responds to cytokine and cell proliferation/differentiation activity; immunosuppressant or immunostimulant activity (e.g. for treating immune deficiency, including infection with human immunodeficiency virus, regulation of lymphocyte growth; treating cancer and many autoimmune diseases, and to prevent transplant rejection or induce tumour immunity); regulation of haematopoiesis (e.g. treatment of myeloid or lymphoid diseases); promoting growth of bone, cartilage, tendon, ligament and nerve tissue (e.g. for healing wounds, treatment of burns, ulcers and periodontal disease and neurodegeneration); inhibition or activation of follicle-stimulating hormone (modulation of fertility); chemotactic/chemokinetic activity (e.g. for mobilising specific cell types to sites of injury or infection); haemostatic and thrombolytic activity (e.g. for treating haemophilia and stroke); anti-inflammatory activity (for treating, for example, septic shock or Crohn's disease); macrophage inhibitory and/or T cell inhibitory activity and thus, anti-inflammatory activity; anti-immune activity (i.e. inhibitory effects against a cellular and/or humoral immune response, including a response not associated with inflammation); inhibition of the ability of macrophages and T cells to adhere to extracellular matrix components and fibronectin, as well as up-regulated fas receptor expression in T cells.

Malignancy disorders, including cancer, leukaemia, benign and malignant tumour growth, invasion and spread, angiogenesis, metastases, ascites and malignant pleural effusion.

Autoimmune diseases including arthritis, including rheumatoid arthritis, hypersensitivity, psoriasis, Sjogren's syndrome, allergic reactions, asthma, chronic obstructive pulmonary disease, systemic lupus erythematosus, Type 1 diabetes mellitus, Crohn's disease, ulcerative colitis, collagen diseases and other diseases.

Vascular diseases including arteriosclerosis, atherosclerotic heart disease, reperfusion injury, cardiac arrest, myocardial infarction, vascular inflammatory disorders, respiratory distress syndrome, cardiovascular effects, peripheral vascular disease, migraine and aspirin-dependent anti-thrombosis, stroke, cerebral ischaemia, ischaemic heart disease or other diseases.

Diseases of the gastrointestinal tract including peptic ulcer, ulcerative colitis, Crohn's disease and other diseases.

Hepatic diseases including hepatic fibrosis, liver cirrhosis, amyloidosis.

Inherited metabolic disorders including phenylketonuria PKU, Wilson disease, organic acidemias, glycogen storage diseases, urea cycle disorders, cholestasis, and other diseases.

Renal and urologic diseases including thyroiditis or other glandular diseases, glomerulonephritis, lupus nephritis or other diseases.

Ear, nose and throat disorders including otitis or other oto-rhino-laryngological diseases, dermatitis or other dermal diseases.

Dental and oral disorders including periodontal diseases, periodontitis, gingivitis or other dental/oral diseases.

Testicular diseases including orchitis or epididimo-orchitis, infertility, orchidal trauma or other testicular diseases.

Gynaecological diseases including placental dysfunction, placental insufficiency, habitual abortion, eclampsia, pre-eclampsia, endometriosis and other gynaecological diseases.

Ophthalmologic disorders such as Leber Congenital Amaurosis (LCA) including LCA10, posterior uveitis, intermediate uveitis, anterior uveitis, conjunctivitis, chorioretinitis, uveoretinitis, optic neuritis, glaucoma, including open angle glaucoma and juvenile congenital glaucoma, intraocular inflammation, e.g. retinitis or cystoid macular oedema, sympathetic ophthalmia, scleritis, retinitis pigmentosa, macular degeneration including age related macular degeneration (AMD) and juvenile macular degeneration including Best Disease, Best vitelliform macular degeneration, Stargardt's Disease, Usher's syndrome, Doyne's honeycomb retinal dystrophy, Sorby's Macular Dystrophy, Juvenile retinoschisis, Cone-Rod Dystrophy, Corneal Dystrophy, Fuch's Dystrophy, Leber's congenital amaurosis, Leber's hereditary optic neuropathy (LHON), Adie syndrome, Oguchi disease, degenerative fondus disease, ocular trauma, ocular inflammation caused by infection, proliferative vitreo-retinopathies, acute ischaemic optic neuropathy, excessive scarring, e.g. following glaucoma filtration operation, reaction against ocular implants, corneal transplant graft rejection, and other ophthalmic diseases, such as diabetic macular oedema, retinal vein occlusion, RLBP1-associated retinal dystrophy, choroideremia and achromatopsia.

Neurological and neurodegenerative disorders including Parkinson's disease, complication and/or side effects from treatment of Parkinson's disease, AIDS-related dementia complex HIV-related encephalopathy, Devic's disease, Sydenham chorea, Alzheimer's disease and other degenerative diseases, conditions or disorders of the CNS, strokes, post-polio syndrome, psychiatric disorders, myelitis, encephalitis, subacute sclerosing pan-encephalitis, encephalomyelitis, acute neuropathy, subacute neuropathy, chronic neuropathy, Fabry disease, Gaucher disease, Cystinosis, Pompe disease, metachromatic leukodystrophy, Wiscott Aldrich Syndrome, adrenoleukodystrophy, beta-thalassemia, sickle cell disease, Guillaim-Barre syndrome, Sydenham chorea, myasthenia gravis, pseudo-tumour cerebri, Down's Syndrome, Huntington's disease, Frontotemporal dementia, CNS compression or CNS trauma or infections of the CNS, muscular atrophies and dystrophies, diseases, conditions or disorders of the central and peripheral nervous systems, motor neuron disease including amyotropic lateral sclerosis, spinal muscular atropy, spinal cord and avulsion injury.

Other diseases and conditions such as cystic fibrosis, mucopolysaccharidosis including Sanfilipo syndrome A, Sanfilipo syndrome B, Sanfilipo syndrome C, Sanfilipo syndrome D, Hunter syndrome, Hurler-Scheie syndrome, Morquio syndrome, ADA-SCID, X-linked SCID, X-linked chronic granulomatous disease, porphyria, haemophilia A, haemophilia B, post-traumatic inflammation, haemorrhage, coagulation and acute phase response, cachexia, anorexia, acute infection, septic shock, infectious diseases, diabetes mellitus, complications or side effects of surgery, bone marrow transplantation or other transplantation complications and/or side effects, complications and side effects of gene therapy, e.g. due to infection with a viral carrier, or AIDS, to suppress or inhibit a humoral and/or cellular immune response, for the prevention and/or treatment of graft rejection in cases of transplantation of natural or artificial cells, tissue and organs such as cornea, bone marrow, organs, lenses, pacemakers, natural or artificial skin tissue.

### siRNA, micro-RNA and shRNA

In certain other embodiments, the NOI comprises a micro-RNA. The micro-RNA which is the NOI (i.e. transgene) is distinct from the self-destabilisation or self-decay element described herein. Thus, a micro-RNA which is the transgene typically does not target the mRNA encoding the transgene. A micro-RNA which is the transgene may target the mRNA encoding another transgene (i.e. a second transgene) in order to regulate the second transgene mRNA, for example, as part of a gene switch system.

In addition to the NOI, the vector may also comprise or encode a miRNA, siRNA, shRNA, or regulated shRNA (Dickins et al. (2005) Nature Genetics 37: 1289-1295, Silva et al. (2005) Nature Genetics 37:1281-1288). Preferably, the vector may comprise or encode a miRNA or shRNA in addition to the NOI. The additional miRNA, siRNA, shRNA, or regulated shRNA is distinct from the self-destabilisation or self-decay element described herein. Thus, the additional miRNA, siRNA, shRNA, or regulated shRNA does not target the mRNA encoding the transgene.

### Pharmaceutical Compositions

The present disclosure provides a pharmaceutical composition comprising the lentiviral vector as described herein or a cell or tissue transduced with the viral vector as described herein, in combination with a pharmaceutically acceptable carrier, diluent or excipient.

The present disclosure provides a pharmaceutical composition for treating an individual by gene therapy, wherein the composition comprises a therapeutically effective amount of a lentiviral vector. The pharmaceutical composition may be for human or animal usage.

The composition may comprise a pharmaceutically acceptable carrier, diluent, excipient or adjuvant. The choice of pharmaceutical carrier, excipient or diluent can be made with regard to the intended route of administration and standard pharmaceutical practice. The pharmaceutical compositions may comprise, or be in addition to, the carrier, excipient or diluent any suitable binder(s), lubricant(s), suspending agent(s), coating agent(s), solubilising agent(s) and other carrier agents that may aid or increase vector entry into the target site (such as for example a lipid delivery system).

Where appropriate, the composition can be administered by any one or more of inhalation; in the form of a suppository or pessary; topically in the form of a lotion, solution, cream, ointment or dusting powder; by use of a skin patch; orally in the form of tablets containing excipients such as starch or lactose, or in capsules or ovules either alone or in admixture with excipients, or in the form of elixirs, solutions or suspensions containing flavouring or colouring agents; or they can be injected parenterally, for example intracavernosally, intravenously, intramuscularly, intracranially, intraoccularly intraperitoneally, or subcutaneously. For parenteral administration, the compositions may be best used in the form of a sterile aqueous solution which may contain other substances, for example enough salts or monosaccharides to make the solution isotonic with blood. For buccal or sublingual administration, the compositions may be administered in the form of tablets or lozenges which can be formulated in a conventional manner.

The lentiviral vector as described herein may also be used to transduce target cells or target tissue *ex vivo* prior to transfer of said target cell or tissue into a patient in need of the same. An example of such cell may be autologous T cells and an example of such tissue may be a donor cornea.

### Variants, Derivatives, Analogues, Homologues and Fragments

In addition to the specific proteins and nucleotides mentioned herein, the present invention also encompasses the use of variants, derivatives, analogues, homologues and fragments thereof.

In the context of the present invention, a variant of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally-occurring protein.

The term "derivative" as used herein, in relation to proteins or polypeptides of the present invention includes any substitution of, variation of, modification of, replacement of, deletion of and/or addition of one (or more) amino acid residues from or to the sequence providing that the resultant protein or polypeptide retains at least one of its endogenous functions.

The term "analogue" as used herein, in relation to polypeptides or polynucleotides includes any mimetic, that is, a chemical compound that possesses at least one of the endogenous functions of the polypeptides or polynucleotides which it mimics.

Typically, amino acid substitutions may be made, for example from 1, 2 or 3 to 10 or 20 substitutions provided that the modified sequence retains the required activity or ability. Amino acid substitutions may include the use of non-naturally occurring analogues.

Proteins used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" means an entity having a certain homology with the wild type amino acid sequence and the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 97 or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 97%, 98% or 99% identical to the subject sequence. Although homology can also be considered in terms of similarity, in the context of the present invention it is preferred to express homology in terms of sequence identity.

Homology comparisons can be conducted by eye, or more usually, with the aid of readily available sequence comparison programs. These commercially available computer programs can calculate percentage homology or identity between two or more sequences.

Percentage homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percentage homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Research 12:387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol Lett (1999) 174(2):247-50; FEMS Microbiol Lett (1999) 177(1):187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). For some applications, it is preferred to use the public default values for the GCG package, or in the case of other software, the default matrix, such as BLOSUM62.

Once the software has produced an optimal alignment, it is possible to calculate percentage homology, preferably percentage sequence identity. The software usually does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the break. The DNA is then expressed in accordance with the invention to make the encoded protein. These methods are only illustrative of the numerous standard techniques known in the art for manipulation of DNA sequences and other known techniques may also be used.

All variants, fragments or homologues of the regulatory protein suitable for use in the cells and/or modular constructs of the invention will retain the ability to bind the cognate binding site of the NOI such that translation of the NOI is repressed or prevented in a viral vector production cell.

All variants fragments or homologues of the binding site will retain the ability to bind the cognate RNA-binding protein, such that translation of the NOI is repressed or prevented in a viral vector production cell.

### Codon Optimisation

The polynucleotides used in the present invention (including the NOI and/or components of the vector production system) may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

Many viruses, including retroviruses, use a large number of rare codons and changing these to correspond to commonly used mammalian codons, increases expression of a gene of interest, e.g. a NOI or packaging components in mammalian production cells, can be achieved. Codon usage tables are known in the art for mammalian cells, as well as for a variety of other organisms.

Codon optimisation of viral vector packaging components has a number of other advantages. By virtue of alterations in their sequences, the nucleotide sequences encoding the packaging components of the viral particles required for assembly of viral particles in the producer cells/packaging cells have RNA instability sequences (INS) eliminated from them. At the same time, the amino acid sequence coding sequence for the packaging components is retained so that the viral components encoded by the sequences remain the same, or at least sufficiently similar that the function of the packaging components is not compromised. In lentiviral vector *gag*/*pol* expression cassettes codon optimisation also overcomes the Rev/RRE requirement for export, rendering optimised sequences Rev-independent. Codon optimisation also reduces homologous recombination between different constructs within the vector system (for example between the regions of overlap in the *gag-pol* and *env* open reading frames). The overall effect of codon optimisation is therefore a notable increase in viral titre and improved safety.

In one aspect only codons relating to INS are codon optimised. However, in a much more preferred and practical aspect, the sequences are codon optimised in their entirety, with some exceptions, for example the sequence encompassing the frameshift site of *gag-pol* (see below).

The *gag-pol* gene of lentiviral vectors comprises two overlapping reading frames encoding the *gag-pol* proteins. The expression of both proteins depends on a frameshift during translation. This frameshift occurs as a result of ribosome "slippage" during translation. This slippage is thought to be caused at least in part by ribosome-stalling RNA secondary structures. Such secondary structures exist downstream of the frameshift site in the *gag-pol* gene. For HIV, the region of overlap extends from nucleotide 1222 downstream of the beginning of *gag* (wherein nucleotide 1 is the A of the *gag* ATG) to the end of *gag* (nt 1503). Consequently, a 281 bp fragment spanning the frameshift site and the overlapping region of the two reading frames is preferably not codon optimised. Retaining this fragment will enable more efficient expression of the Gag-Pol proteins. For EIAV the beginning of the overlap has been taken to be nt 1262 (where nucleotide 1 is the A of the gag ATG) and the end of the overlap to be nt 1461. In order to ensure that the frameshift site and the *gag-pol* overlap are preserved, the wild type sequence has been retained from nt 1156 to 1465.

Derivations from optimal codon usage may be made, for example, in order to accommodate convenient restriction sites, and conservative amino acid changes may be introduced into the Gag-Pol proteins.

In one aspect, codon optimisation is based on lightly expressed mammalian genes. The third and sometimes the second and third base may be changed.

Due to the degenerate nature of the genetic code, it will be appreciated that numerous *gag-pol* sequences can be achieved by a skilled worker. Also there are many retroviral variants described which can be used as a starting point for generating a codon-optimised *gag-pol* sequence. Lentiviral genomes can be quite variable. For example there are many quasi-species of HIV-1 which are still functional. This is also the case for EIAV. These variants may be used to enhance particular parts of the transduction process. Examples of HIV-1 variants may be found at the HIV Databases operated by Los Alamos National Security, LLC at http://hiv-web.lanl.gov. Details of EIAV clones may be found at the National Center for Biotechnology Information (NCBI) database located at http://www.ncbi.nlm.nih.gov.

The strategy for codon-optimised *gag-pol* sequences can be used in relation to any retrovirus. This would apply to all lentiviruses, including EIAV, FIV, BIV, CAEV, VMR, SIV, HIV-1 and HIV-2. In addition this method could be used to increase expression of genes from HTLV-1, HTLV-2, HFV, HSRV and human endogenous retroviruses (HERV), MLV and other retroviruses.

Codon optimisation can render *gag-pol* expression Rev-independent. In order to enable the use of anti-rev or RRE factors in the lentiviral vector, however, it would be necessary to render the viral vector generation system totally Rev/RRE-independent. Thus, the genome also needs to be modified. This is achieved by optimising vector genome components. Advantageously, these modifications also lead to the production of a safer system absent of all additional proteins both in the producer and in the transduced cell.

It is to be understood that features disclosed herein may be used in combination with one another. Furthermore, it is to be understood that such features may possess different functionalities by virtue of the nucleotide sequence comprising them.

The practice of the present invention will employ, unless otherwise indicated, conventional techniques of chemistry, molecular biology, microbiology and immunology, which are within the capabilities of a person of ordinary skill in the art. Such techniques are explained in the literature. See, for example, J. Sambrook, E. F. Fritsch, and T. Maniatis (1989) Molecular Cloning: A Laboratory Manual, Second Edition, Books 1-3, Cold Spring Harbor Laboratory Press; Ausubel, F. M. et al. (1995 and periodic supplements) Current Protocols in Molecular Biology, Ch. 9, 13, and 16, John Wiley & Sons, New York, NY; B. Roe, J. Crabtree, and A. Kahn (1996) DNA Isolation and Sequencing: Essential Techniques, John Wiley & Sons; J. M. Polak and James O'D. McGee (1990) In Situ Hybridization: Principles and Practice; Oxford University Press; M. J. Gait (ed.) (1984) Oligonucleotide Synthesis: A Practical Approach, IRL Press; and, D. M. J. Lilley and J. E. Dahlberg (1992) Methods of Enzymology: DNA Structure Part A: Synthesis and Physical Analysis of DNA Methods in Enzymology, Academic Press. Each of these general texts is herein incorporated by reference.

This disclosure is not limited by the exemplary methods and materials disclosed herein, and any methods and materials similar or equivalent to those described herein can be used in the practice or testing of aspects of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of'.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

It is to be understood that any nucleotide sequence disclosed herein may be a DNA sequence or an RNA sequence. As such, a sequence recited comprising thymine nucleotides is considered disclosed as a corresponding RNA sequence comprising Uracil nucleotides in place of said Thymine nucleotides, and *vice versa.* Accordingly, DNA sequences contained within the corresponding sequence listing will be understood as also representing an RNA of identical sequence (with Thymines replaced by Uracils), and *vice versa.*

### EXAMPLES

### Materials and Methods

### Cell culture conditions

HEK293Ts (HEK293Ts) suspension cells were grown in FreeStyle^{™} 293 Expression Medium (Gibco) supplemented with 0.1% of Cholesterol Lipid Concentrate (Gibco) and incubated at 37 °C in 5% CO2, in a shaking incubator (25 mm orbit set at 190 RPM).

HEK293T adherent cells were maintained in complete media (Dulbecco's Modified Eagle Medium (DMEM) (Sigma) supplemented with 10% heat-inactivated fetal bovine serum (Gibco), 2 mM L-glutamine (Sigma) and 1% non-essential amino acids (NEAA) (Sigma)), at 37 °C in 5% CO2.

### Suspension cell culture, transfection and lentiviral vector production

Where indicated, vector production was carried out in suspension-adapted (serum-free) HEK293T cells. Cells were seeded at 8x10⁵ cells per ml in serum-free media and were incubated at 37 °C in 5% CO2, shaking, throughout vector production. Approximately 24 hours after seeding the cells were transfected using the following mass ratios of plasmids per effective final volume of culture at transfection: 0.95 µg/mL Genome, 0.1 µg/mL Gag-Pol, 0.06 µg/mL Rev, 0.07 µg/mL VSV-G and 0.18 µg/mL 256U1 plasmids. Transfection was mediated by mixing DNA with Lipofectamine 2000CD in Opti-MEM as per manufacturer's protocol (Life Technologies). Sodium butyrate (Sigma) was added ~18 hrs later to 10 mM final concentration. Typically, vector supernatant was harvested 20-24 hours later, and then filtered (0.22 µm) and frozen at -80°C.

In Example 23, all vector production was carried out in HEK293Ts cell, in 24-well plates (1mL volumes, on a shaking platform). 1.65s cells were seeded at 8 x 105 cells per ml in serum-free media and were incubated at 37 °C in 5% CO2, shaking, throughout vector production. Approximately 24 hours after seeding the cells were transfected using the following mass ratios of plasmids per effective final volume of culture at transfection: 0.95 µg/mL Genome, 0.1 µg/mL Gag-Pol, 0.06 µg/mL Rev, 0.07 µg/mL VSV-G, 0.150ug/ml pTK-dsRed, and 54 pmol/mL of siRNA. Transfection was mediated by mixing DNA with Lipofectamine 2000CD in Opti-MEM as per manufacturer's protocol (Life Technologies). Sodium butyrate (Sigma) was added ~18 hrs later to 10 mM final concentration. Typically, vector supernatant was harvested 20-24 hours later, and then filtered (0.22 µm) and frozen at -80°C.

### Flow cytometry (production cells)

At vector harvest, 200ul of end-of-production cells were assayed for GFP and dsRed expression by flow cytometry using an Attune NxT flow cytometer (Thermo). GFP and dsRed expression was scored by multiplying the median fluorescence intensity by the % of positive cells.

### Lentiviral vector titration by GFP/flow cytometry assay

For 'biological' titration of LVs, appropriate volumes of 1:10 to 1:150 diluted vector supernatants were used to transduce 2 x10⁴ HEK293T cells at 96-well scale in the presence of 8µg/mL polybrene. Cultures were incubated for 3 days (1:5 splits every 2-3 days) before flow cytometry was performed to generated percentage positive values, which were used to calculated GFP TU/mL titres using dilution factor and volumes used.

### Lentiviral vector titration by integration assay

For lentiviral vector titration by integration assay, 0.25mL volumes of 1:10 to 1:50 diluted vector supernatants were used to transduce 4.5 x10⁴ HEK293T cells at 24-well scale in the presence of 8µg/mL polybrene. Cultures were passaged for 10 days (1:5 splits every 2-3 days) before host DNA was extracted from ~1x10⁶ cell pellets using a QIAextractor (Qiagen) according to manufacturer's instructions. Duplex quantitative PCR was carried out using a FAM primer/probe set to the HIV packaging signal (Ψ) and to RRP1, and vector titres (TU/mL) calculated using the following factors: transduction volume, vector dilution, RRP1-normallised HIV-1 Ψ copies detected per reaction.

### Transcriptional read-through ('read-in') analysis of LV-transduced cells

The lentiviral vectors indicated were produced by transient transfection of suspension serum-free HEK293T cells and titrated to give Biological titres as described previously. GFP titres were then used accordingly to transduce 4.5x10⁴ adherent HEK293T with LVs at a multiplicity of infection (MOI) of 1. Transduced cells were passaged three times over ten days prior to being harvested and split into 2 aliquots; one was processed for total RNA extraction and one processed for genomic DNA extraction. 200ng (293T) of total RNA was DNAse I-treated and subjected to RT-PCR using the SSIV VILO RT system (Life Technologies). cDNA was diluted to 1ng/ul, and 5ul was subjected to SYBR qPCR using primer sets directed to the HIV Psi and cellular GAPDH. The Delta Ct method was used to normalise HIV Psi copies to GAPDH copies in order to generate expression score between samples. Genomic DNA extracts were prepared using the Qiacube extraction system (Qiagen) and 5ul of eluted DNA was subjected to HIV vector integration assay using qPCR. HIV Psi copies were normalised to cellular target RPPH1 in order to calculate the average of integrated vector genomes per cell. The HIV RNA expression score of a sample was then normalised to the number of integrated vector copies per cell in order to account for transduction efficiency. This final value is the relative HIV Psi RNA expression score, and values were normalised to the standard vector.

Alternatively, (e.g., as used for Example 27) the HIV vectors indicated were produced by transient transfection of suspension-mode HEK293-1.65s cells in the presence or absence of 256_U1 snRNA at 24 well plate scale. Supernatants were harvested after two days before being titrated by GFP expression using flow cytometry on HEK293T cells. An integration assay was subsequently performed in HEK293T cells and cells maintained for 10-days. Transduced cells were harvested 10-days post transduction. Genomic DNA extracts were prepared using the Qiacube extraction system (Qiagen) and 5ul of eluted DNA was subjected to HIV vector integration assay using qPCR. Integration titres were then used accordingly to transduce 4.5x10⁴ HEK293T or 92BR cells (Donkey primary fibroblasts) at a multiplicity of infection (MOI) of 1. Transduced cells were passaged three times over ten days prior to being harvested and split into 2 aliquots; one was processed for total RNA extraction and one processed for genomic DNA extraction. 200ng (293T) or 180ng (92BR) of total RNA was DNAse I-treated and subjected to RT-PCR using the SSIV VILO RT system (Life Technologies). cDNA was diluted 1 in 10, and 5ul was subjected to TAQman qPCR using primer sets directed to the HIV Psi and cellular GAPDH. The Delta Ct method was used to normalise HIV Psi copies to GAPDH copies in order to generate expression score between samples. Genomic DNA extracts were prepared using the Qiacube extraction system (Qiagen) and 5ul of eluted DNA was subjected to HIV vector integration assay using qPCR. HIV Psi copies were normalised to cellular target RPPH1 in order to calculate the average of integrated vector genomes per cell. The HIV RNA expression score of a sample was then normalised to the number of integrated vector copies per cell in order to account for transduction efficiency. This final value is the relative HIV Psi RNA expression score, and for each cell line tested all values were normalised to the standard vector (intact MSD) made in the absence of 256_U1 snRNA.

### Luciferase assay

All assays were performed on replicate cultures. For transfected adherent 293T cells, supernatants were removed and frozen on dry ice until assay. The remaining monolayers were then trypsinised and assayed for dsRed expression by flow cytometry using an Attune NxT flow cytometer. For transfected suspension 293Ts cells, 200ul of cell suspension was removed for dsRed assay as above. The remaining cells were first pelleted by centrifugation at low speed, and supernatants were then removed and frozen on dry ice.

At time of assay, cell supernatants were left to thaw to room temperature, then 5ul of each supernatant was assayed using the Pierce Gaussia luciferase assay detection system as per the manufacturers' instructions. The assay was performed using individual well dispensers of the Spectromax i3x system. Wells were assayed one at a time using the following protocol: 50ul of substrate was dispensed in the well, and plate was shaken for 2s. There was a further lag time of 2s prior to reading. Gaussia luciferase activity was then measured over the course of 10 seconds, and total RLU were recorded. For a given sample, total RLU were normalised to transfection efficiency using the dsRed expression score (% gated x MFI). Normalised scores were then displayed relative the score the control.

### Total RNA extraction from end-of-production cells and lentiviral vector virions

At the time of vector harvest, approximately 1e6 1.65s cells in 0.5ml Freestyle serum-free media were pelleted by centrifugation at 500g for 3 minutes. Supernatants were removed and filtered through a 0.22um Spin-X filter by centrifugation, and then stored at -80°C until vector titration and virion RNA extraction. The cell pellet was washed in 0.5ml PBS and cells were pelleted once again by centrifugation. RNA was extracted from fresh cell pellets or 125ul of crude vector filtrate using RNAeasy and QIAamp Viral RNA Mini Kits, respectively, according to the manufacturer's instructions (Qiagen).

### Reverse-transcription

500ng of total RNA from cells or 5ul of virion RNA eluate was used as input for reverse transcription. RNA samples were incubated with 1ul EZDNase (Life Technologies) at 37°C for 2 minutes. 1.1ul of 0.1M DTT were then added per sample, and reactions were left to incubate at 55°C for a further 5 minutes. DNAse-treated RNA was then subjected to reverse transcription using the Superscript IV first-strand synthesis system (Life Technologies), with OligodT primers being used to primer first-strand synthesis according to the manufacturer's instructions. Upon completion of reverse-transcription, samples were incubated with 1ul RNAse H (Life Technologies) at 37°C for 20 minutes. Upon completion, samples were diluted 10-fold using nuclease-free water.

### Polymerase chain reaction

PCR reactions were performed using CloneAmp polymerase (Takara). 1ul of diluted cDNA was used as template. Primers were design to span the HIV packaging sequence through to the GFP open reading frame or through to the wPRE. As an endogenous control for cellular RNA primers targeting human actin cDNA were employed. All primers were used at a final concentration of 200nM, and cycling conditions were as follows:
*HIV vector genome primers*
   98°C 3mins, 30 cycles of (98°C 10s; 53.9°C 15s; 72°C 60s), follows by a final incubation at 72°C for 5 minutes.
*Human actin cDNA primers*
   98°C 3mins, 30 cycles of (98°C 10s; 68°C 15s; 72°C 30s), follows by a final incubation at 72°C for 5 minutes.

**Table 5 - Primers used in this study**

| **Primer** | **Sequence 5'-3'** |
|---|---|
| HIV vector forward | AAAGCGAAAGGGAAACCAGAG |
| HIV vector reverse (GFP) | CTTGTGGCCGTTTACGTC |
| HIV vector reverse (wPRE) | AGCAGCGTATCCACATAG |
| Human Actin cDNA forward | TGACGGGGTCACCCACACTGTGCCCATCTA |
| Human Actin cDNA reverse | CTAGAAGCATTTGCGGTGGACGATGGAGGG |

Upon completion of reactions, PCR products were visualised by 1% agarose gel electrophoresis.

### Polyacrylamide gel electrophoresis and western blotting

45ul of crude vector was incubated with 15ul of 4X Laemmli buffer (BioRad) containing 25% β-Mercaptoethanol at 100°C for 5 minutes before being placed on ice. 20ul of sample was subjected to polyacrylamide gel electrophoresis using a 4-20% TGX Pre-cast gel (BioRad) and associated Criterion gel running system. Proteins were blotted onto nitrocellulose membrane using the Turbo Transblot system (BioRad). Membranes were blocked in blocking buffer (25mM Tris, 150mM NaCl, 0.1% v/v Tween 20, 5% w/v milk powder) prior to being probed with primary antibodies to VSVG (Thermo, PA1-30138) and HIV p24 (Abcam, ab9071) according to the manufacturer's instructions. Detection was performed using Starbright Blue 520 and Starbright Blue 700 fluorescent antibodies (BioRad, 12005870 and 12004158). Visualisation was performed using a ChemiDoc MP imager (BioRad).

### Transduction at matched multiplicity of infection (MOI)

Calculation of vector ul to achieve MOI ranging from 2 to 0.1 was based on integration titre calculated in HEK293T cells. HEK293Tcells were seeded at 9E4 cells per well in 12 well plates. Jurkat cells were seeded at 2E5 cells per well on a 12 well plate. The following day, cells were recounted and MOI calculated based on integration titre and number of cells per well. Cells were passaged for 10 days with splits every 2-3 days. Flow cytometry, using an Attune-NxT^{®} (Thermofisher), was performed at each split to determine the transgene (GFP) mean fluorescence intensity (MFI). At the end of 10 days, a Duplex quantitative PCR (see above) was performed to confirm the HIV-1 Ψ copies per cell. If necessary, the MFI values were normalized to the number of HIV-1 Ψ copies per cell.

### Cytosolic mRNA quantification

Sub-cellular fractionation into nucleus and cytosolic fractions was performed by lysing the cells with a hypotonic buffer (20mM Tris-HCL + 150mM NaCl) complemented with Igepal at final concentration of 0.2%. RNA extraction from the cytosolic fraction was performed using RNeasy kit (Qiagen), followed by DNAse treatment (ezDNase^{™}, Invitrogen) and reverse transcriptase (SuperScript^{™} IV VILO^{™}, Invitrogen). Quantification of cytosolic transgene mRNA was done by qPCR with primer and probes targeting GFP and GAPDH as a normalization control. Analyses was done by comparative quantification using the delta CT method.

### rAA V vector production

AAV vector was produced with the AAV-MAX Helper-Free AAV Production System (ThermoFisher). The manufacturer's protocol was followed with the exception that the production cells used were 1.65s cells. Cells were seeded at 3E+06 live cells/mL in Freestyle + 0.1% Cholesterol (hereafter FS + 0.1% CLC) in a total volume of 20ml and incubated in a Multitron set to 37°C with 5% CO2 and 200 rpm until transfection mixes were ready. A total of 1.5ug/ml of DNA was transfected into cells at a molar ratio of 1:1:1 (Transfer: AAV2Rep/Cap: Helper). Transfer plasmids used were pscAAV2-CMV-GFP and pscAAV2-EFS-GFP. After transfection, cells were returned to the Multitron and incubated for 72 hours before harvest with AAV-MAX lysis buffer (Thermo Fisher) following manufacturer's protocol.

### rAAV vector titration

The number of genome-containing particles present in the AAV preparation was determined by TaqMan Real-time PCR assay. Prior to the qPCR, vector was treated with DNAse and exonuclease in order to remove residual DNA. These enzymes were then heat-inactivated with a 95°C step, which simultaneously lyses AAV capsids to release vector genomes to allow analysis by PCR. TaqMan primers and probe were designed to target the GFP.

### rAA V transduction at matched multiplicity of infection (MOI)

HepG2 cells were seeded at 2E5 cells/ well in MEM supplemented with 10% FBS, L-glut (2mM), 1% NEAA in 12 well plates. The following day, cells were recounted, and MOI calculated based on the genome copy number/ml determined by qPCR and number of cells. HepG2 cells were transduced at MOI 250, and 500. Approximately 72 hours after infection, flow cytometry using an Attune-NxT^{®} (Thermofisher), was performed to determine the percentage of GFP positive cells and the transgene (GFP) mean fluorescence intensity (MFI).

### EXAMPLE 1: Exemplification of an 'R-embedded' heterologous polyadenylation sequence positioned at the 3' SIN-LTR using the SV40 late polyadenylation sequence.

To achieve the first three of the four main facets of the invention described above the inventors obtained a known, strong heterologous polyadenylation sequence known in the art (e.g. rabbit beta-globin polyA, SV40 late polyA, human/bovine growth hormone polyA) and inserted the sequence downstream of the 3' SIN-U3 region within the LV genome expression cassette, replacing the 3' R-U5 sequence in its entirety. Next, the first 18-20 nucleotides of the 5' R region is inserted immediately downstream of the PAS. It should be noted that for HIV-1 vRNA transcription initiation at the specific transcription start site (TSS) can vary across the first three 'G' nucleotides (i.e. nucleotides 1-3), resulting in '3G', '2G' or '1G' vRNA species (see Figure 17). It is believed that for wild type HIV-1 infection the specific TSS shifts from 3G/2G to 1G affecting the fate of vRNA, altering the requirements of expression of gagpol (3G/2G vRNA) or packaging (1G vRNA) (Kharytonchyk et al. (2016) Proc. Natl. Acad. Sci. U S A. 113: 13378-13383; and Brown et al (2020) Science 368: 413-417). Elsewhere in the invention a novel promoter has been developed enabling precise production of only the 1G vRNA (see Example 4, Figure 18). Therefore, when utilizing an LV expression cassette wherein only 1G vRNA is produced, the sequence inserted immediately downstream of the PAS may minimally encode nucleotides R.3-20 of HIV-1, which would correspond to nucleotides 1-18 of the 5' R region being employed. Therefore, the term '~20 nucleotides' of R can mean either scenario. Practically however, it is most simple to include nucleotides 1-20 (including all three Gs) downstream of the PAS within LV genome expression cassettes generating either just 1G vRNAs or all three variants (3G, 2G and 1G), since this will allow annealing of any of the variants at 1^{st} strand transfer resulting in no mis-matches at the primer extension point (see Figure 17). Practically, it is also simple to use an R region of a length as described herein in such a way. It is also recognized that the specific sequences used as 'R.1-20' or more generally 'embedded R region' (e.g. an R region of 14 nucleotides in length) may be heterologous to HIV-1 R, as long as [1] the R region sequence is used at both the 5' R region position and the 'embedded' 3' R region position to allow annealing at the RT step, [2] the 5' R region sequence is operably linked to a GU-rich DSE, and preferably [3] the 5' R region plus DSE sequence forms a stem loop (see Example 4, where this was demonstrated with R region sequence from RSV).

Next a 'CA' or a tandem set of 'CA' motifs can be inserted downstream of the embedded R.1-20 to 'offer' as a polyadenylation cleavage site; thus, this cleavage site will sit downstream of the R.1-20 sequence and upstream of the DSE, which allows for cleavage after nucleotides R.1-20, which will be retained for annealing to the ssDNA at 1^{st} strand transfer.

Figure 3 provides an overview of the above, and lays out the two main, related questions unanswered prior to the present invention. These were: [1] how much sequence from the R region can be embedded within the heterologous polyadenylation sequence (i.e., how much R region sequence can be retained between the PAS and the cleavage site/DSE?) to achieve efficient polyadenylation efficiency, and [2] what is the minimum amount of R region sequence homology required to allow [1] whilst maintaining efficient 1^{st} strand transfer? Thus, given the approximate distance tollerances of polyA sequences (USE, pA signal, cleavage site, DSE/GU box) reported to enable highly efficient polyadenylation, prior to the invention it was not known if sufficient 'R' sequence could be inserted between the pA signal and the cleavage site to allow both efficient polyadenylation and first strand transfer during the RT step. This was tested in this Example by generating three slightly different variants of the SV40 late polyadenylation sequences positioned within a reporter cassette (Figure 4). The SV40 late polyA sequence is well characterized and contains both a USE and a DSE. The SV40 late polyA sequence was positioned downstream of a Promoter-GFP-3'SIN-U3 sequence and upstream of an IRES-Luciferase open-reading frame. The three 'R-embedded' variants were: [1] nucleotides 1-20 from HIV-1 R (R.1-20), [2] nucleotides 1-60 of HIV-1 R (R.1-60), and [3] nucleotides 1-20 from HIV-1 R appended with downstream sequence designed to cause a stem loop to be formed (R.1-20c) (this was done to assess if the pA signal and GU-rich DSE might be brought closer spatially and potentially improve polyadenylation efficiency). The 'R.1-20c' variant was generated to assess if additional sequence could be inserted in this region, whilst perhaps still retaining close spatial proximity to the GU-rich DSE. Thus, the level of transcriptional read-through these R-embedded SV40 late polyA sequences was measured by Luciferase activity. These variants were compared to a SIN-LTR ('RU5') and an unmodified SV40 late polyA sequence (Figure 8). These data demonstrated that the SV40 late polyA was ~50-fold more efficient than the standard SIN-LTR, and the R.1-20 embedded SV40 late polyA was 20-fold more efficient. The other two variants were ≤ 5-fold more efficient than the standard SIN-LTR. Thus, the data show that the transcriptional read-though of the standard RU5 sequence is almost 50-fold greater than the strong SV40 late polyA sequence, and that replacement of the sequence between the pA signal and the cleavage site/GU-rich DSE of SV40 pA with nucleotides 1-20 of the HIV-1 R region still resulted in a polyA sequence that was 20-fold more effective than the standard RU5 sequence. Therefore, it was demonstrated that embedding of R sequence into the heterologous polyadenylation sequence still allow for efficient polyadenylation activity that was substantially improved over the SIN-LTR.

### EXAMPLE 2: LV expression cassettes employing 'R-embedded' SV40 late polyA-containing SIN-LTRs can generate high titre LV stocks.

The same R-embedded SV40 late polyA variants described in Example 1 were then cloned into a full LV genome expression cassette in order to evaluate polyadenylation in the proper context and to be able to assess the impact on LV titres (i.e. a surrogate measure of the 1^{st} strand transfer step). These LV genome expression cassettes therefore were appended with the IRES-Luc reporter sequence downstream (Figure 9). An additional LV genome construct ('RU5-pA_KO') was generated wherein the native HIV-1 PAS in the 3'SIN'LTR was mutated. HEK293T cells were transfected with these constructs together with LV packaging plasmids to generate clarified vector harvest for titration and post-production cells were assayed for Luciferase assay to measure read-through (polyA activity). Interestingly, the read-through activity for the 'RU5-pA_KO' control did not increase by more than 2-3 fold, supporting the premise that SIN-LTRs contain poor polyadenylation activity. Thus, the data shows that the standard 3'RU5 sequence is weakly active during LV production, since the pA knock-out RU5 variant gave only 2-fold greater read-through.

Strikingly, and by contrast, the unmodified SV40 late polyA construct had ~100-fold lower read-through in this experiment. Importantly, all three R-embedded SV40 late polyA variants produced 10-to-50 fold lower read-through than the standard SIN-LTR, with the R.1-20 variant within 2-3 fold of the unmodified SV40 late polyA (Figure 9A). In terms of the impact on LV titres, the R.1-20 embedded variant produced the highest titre of all LVs generated (Figure 9B), and was comparable to another control, which was a standard SIN-LTR cassette without the IRES-Luc reporter downstream ('RU5[ΔLuc]'). This provided strong evidence that in the R.1-20 embedded variant, all the transcription termination events occurred at the engineered SIN-LTR, whereas the standard SIN-LTR control (RU5) was mostly reading through to the SV40 polyA utilized downstream of the Luc reporter, leading to a longer vRNA (and hence lower titre compared to the 'RU5[ΔLuc]' control). As expected, the unmodified SV40 late polyA variant produced substantially less titre as expected due to the lack of R sequence available for efficient 1^{st} strand transfer, and the R.1-20c variant also produced lower levels of active LV (i.e. the R.1-20c variant did not produce LV efficiently), possibly due to the artificial stem loop impacting the 1^{st} strand step or some other aspect of reverse transcription. The R.1-20 and R.1-60 variants produced high LV titre but the R.1-20 variant was superior in terms of polyA activity. That being said, the R.1-60 variant gave both improved polyA activity and LV titre, but not to the levels observed for R.1-20. These data gave the first evidence that R-embedded heterologous polyadenylation sequences could be used to replace the 3' RU5 entirely, and LV genome transcription termination can be 'liberated' from requiring a 'back-up' polyadenylation sequence that is typically employed in standard SIN-LV genome expression plasmids (as modelled by the polyA site downstream of the IRES-Luc reporter in this case). Thus, it is not necessary to use an additional polyA site downstream of the 3' LTR with the lentiviral vector genomes of the invention. The length of the R region inserted could be up to 60 nucleotides but just 20 nucleotides appears to be close to the 'optimal' length. Example 8 provides granularity on the precise length requirements for 1^{st} strand transfer in balance with efficient polyadenylation activity.

### EXAMPLE 3: Design considerations for engineering a GU-rich DSE into the first stem loop of the LV genomic vRNA 5' R region.

Figure 5A demonstrates the consequences of employing the 3' SIN-LTR modifications of the invention (R-embedded polyA) within an LV genome expression cassette without any modifications to the 5' R region. In summary, the reformed SIN-LTRs flanking the integrated cassette would harbour the PAS (+/-USE) in the SIN-U3 region but the native HIV-1 GU-rich DSE would reside ~100 nucleotides downstream (also see Figure 6). Whilst the use of a USE (such as from the SV40 late polyA) may act from this distance to enhance polyadenylation from the native HIV-1 PAS, in order to position a GU-rich DSE closer to the heterologous PAS, the 5' R region of the LV genome expression cassette necessarily requires modification (see Figure 5B). It was noted that the first stem loop at the 5' terminus of other retroviruses such as RSV and MMTV contained GU-rich sequence that most likely 'service' the PAS within their U3 core promoter sequences. By 'service' it is meant that the GU-rich sequences function as an enhancer for the PAS within their U3 core promoter sequences. In general, there appears to be some selective pressure on retroviruses to maintain a stem-loop structure at their 5' terminus (for example, the TAR loop in HIV-1), perhaps to ensure vRNA stability or ensure that downstream secondary structure within the packaging sequence is formed correctly. Others have shown this to be the case for HIV-1 that was engineered to be independent of tat (i.e. the TAR loop is no longer required for transcription induction), and yet after passaging these tat-independent HIV-1 'regenerated' a 5' stem loop, which correlated with improved replication (Das et al. (2007) J. Virol. 81: 7742-8). Therefore, it is preferable that engineering of the TAR loop to contain a GU-rich DSE results in a sequence that is still predicted to form a stem loop structure. The engineering may or may not be further constrained depending on preferred sequences utilized in the R-embedded heterologous polyA sequence, which is preferably the same to allow for 1^{st} strand transfer.

Accordingly - and by non-limiting example - several 'hybrid' TAR loops were designed *in silico* such that the first 18/20 nucleotides of HIV-1 R were retained (as well as the nucleotides forming base-pairing on the other side of the stem) but the TAR loop was replaced with a heterologous sequence comprising GU-rich sequences based on the RSV, MMTV or beta-globin DSEs (Table 1 and Figure 10). The stem loop structures displayed in Figure 10 are based on 1G vRNA variants for clarity. These variants - together with versions wherein 44-45 nucleotides of the RSV or MMTV R regions were swapped in for HIV-1 R - were cloned into the polyA/Luciferase reporter construct reflecting different contexts (Figure 11). The different reporters contained additional downstream sequences present in the packaging signal (modelling an integrated 5' SIN-LTR) or without (modelling an integrated 3' SIN-LTR), as well as other optional features such as the presence/absence of a USE within the SIN-U3 and/or a mutated native HIV-1 PAS. Figure 12 presents the initial comparison of polyadenylation activity of the standard SIN-LTR, the unmodified SV40 late polyA, the R.1-20 embedded SV40 late polyA (3' supA-LTR) and some variants containing the R.1-44/45 of RSV or MMTV. These data show that whilst the use of just the SV40 USE improves polyadenylation activity in the SIN-LTR (as shown by Schambach et al. (2007) Molecular Therapy 15:1167-1173 and US20190276844A1), this was not as efficient as either the SV40 late polyA sequence nor the 3' supA-LTR configuration (relevant in LV production only) containing the embedded R.1-20 upstream of the SV40 late polyA GU-rich DSE. This indicated that positioning of a GU-rich DSE closer to the SIN-U3 containing PAS allowed further improvements in polyadenylation activity. The results of positioning either the R.1-44/45 sequence of RSV or MMTV indicated that the MMTV R sequence provided greatest benefit to polyadenylation activity, whereas for RSV the presence of the downstream native HIV-1 PAS/DSE also appeared to be necessary for full activity. The MMTV R sequence allowed for very efficient polyadenylation activity (equivalent to the unmodified SV40 late polyA) irrespectively of whether native HIV-1 PAS/DSE sequence was present downstream.

Next, the four of the TAR/Hybrid R variants (see Table 1 and Figure 10) were tested in the same polyA/Luciferase reporter cassettes to assess in the novel GU-rich sequences (based on RSV and MMTV DSEs) functioned as DSEs. Figure 15 displays the results of this experiment, indicating that whilst all four variants had improved polyadenylation activity compared to the standard SIN-LTR, variants '1GR-GU2' and '1GR-GU5' produced substantially better polyadenylation activity in a manner that was independent of the presence of downstream HIV-1 PAS/DSE sequence. Thus, of the variants compared here, a recapitulated supA-LTR containing either the '1GR-GU2 or the '1GR-GU5' variant gave the least transcriptional read-through, and very little of this termination activity was dependent on the presence of the native HIV-1 pA when modelling the 5' supA-LTR.

A further, deeper analysis of the cis-acting elements utilized in these supA-LTR sequences was performed in the polyA/Luciferase reporter cassettes, focusing on the '1GR-GU2' variant based on MMTV GU-rich DSE sequences. Figure 16 displays the results of this experiment where variants modelling just the used of R.1-20 embedded SV40 late polyA (mod SIN only) i.e. modelling Figure 5A, were compared to variants that modelled the full, recapitulated supA-LTR i.e. modelling Figure 5B. Sub-variants in which various combinations of USE, PAS, and knock-out of the downstream native HIV-1 PAS were generated and tested. The main conclusion from this experiment is that the USE alone provides a substantial benefit to polyadenylation activity but that the most efficient polyadenylation activity was only achieved when utilizing the full recapitulated supA-LTR when the downstream native HIV-1 PAS was inactivated.

**Table 1. Engineered TAR/stem loop 1 sequences used to exemplify one aspect of the invention, compared to wild type HIV-1 TAR, other rerovirus SL1s and a synthetic polyA sequence.** The HIV-1 TAR/SL1 sequence is shown (the HIV-1 polyA signal is downstream; not shown), with arrows indicating alternative transcription start sites known in the art. The black arrows represent '3G/2G' vRNA, which are considered to be a template for vRNA translation (gagpol), whereas the grey arrow is the '1G' vRNA, and is thought to be the most efficiently packaged form for HIV-1 genomic RNA. RSV and MMTV SL1 sequences are shown with associated upstream polyA signals. The hybrid TAR/SL1 sequences displayed were used to test whether a GU-rich DSE could be engineered into the TAR without compromising LV titre. Nucleotides shown in light grey are not likely to be forming dsRNA within the prediced SL structure (see Figure 10), whereas nucleotides shown underlined are. Nucleotides shown in bold and large font size indicate the location of a GU-rich DSE and nucleotides shown in smaller font size indicate the potential region for polyadenylation cleavage. The synthetic polyA (SPA) sequence is based on the beta-globin GU-rich DSE, and indicates relative positions of the pA signal, cleavage sites and DSE.

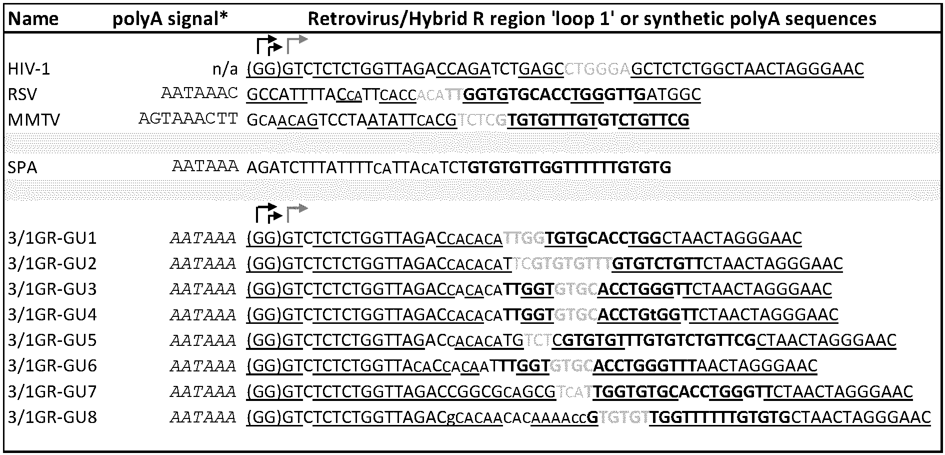

### EXAMPLE 4: Development of a novel CMV/RSV hybrid promoter to drive transcription of LV genome expression cassettes with a single G nucleotide at the transcription start site.

In demonstrating that the 5' R region could be engineered to improve polyadenylation activity in the recapitulated supA-LTR, prior to assessing the impact of these changes on LV titre, the inventors sought to engineer a promoter that enables production of just 1G vRNA. This was done partly to provide the option of employing a shorter embedded R region in the 3'supA-LTR but also because there may be some benefit in general to being able to synthesise 1G vRNA from every transcription event during LV production since for wild type HIV-1, 1G vRNA has been shown to preferentially dimerise compared to 3G/2G vRNA. The inventors noted that wild type RSV (as well as employing its PAS in the core promoter) harbours a single 'G' at its 5' terminus on the vRNA genome, indicating that this promoter is able to position the transcription initiation complex on a precise 'G' nucleotide. Production of a CMV promoter with 1G TSS surprisingly led to 3-4 fold lower titres within standard LV genome cassettes, indicating that the TSS is very sensitive to minor changes (data not shown). The CMV promoter is stronger than RSV, and so the inventors sought to generate a promoter that retained the power of the CMV enhancer/promoter sequence but also contained core sequence from RSV that allowed precise transcription initiation at the single 'G' of an HIV-1 based LV. Accordingly, two CMV-RSV hybrid promoters were designed: one, wherein the sequence between the CMV TATA box and the TSS was replaced with the analogous sequence from RSV U3 ('CMV-RSV1-1G'), and the second wherein the RSV TATA box (including 6 nts upstream) was additionally swapped in ('CMV-RSV2-1G') - see Figure 18A. These two variants contained a 5' R sequence starting at nucleotide #3 i.e. the single 'G' nucleotide. Additionally, a variant where the entire CMV promoter was swapped with RSV U3 was also generated ('RSV-3G'), which was used to assess relative power of the two promoters. All three of these variants were cloned into an LV genome expression cassette containing the 3'supA-LTR sequence with SV40 late poly-embedded R.1-20. A fourth construct was made where the entire RSV-U3 and stem loop 1 of RSV 5' R region was used to replace the CMV-TAR sequence, and this was cloned into an alternative 3'supA-LTR LV genome cassette where the same RSV R sequence was embedded into the SV40 late polyA to enable 1^{st} strand transfer ('RSV-1G[RSV SL1]'). These LV genome expression cassettes also contained the IRES-Luc reporter cassette downstream. Constructs were used to make LV in HEK293Ts cells, alongside a standard LV construct (lacking the IRES-Luc reporter sequence). Vector titres are reported in Figure 18B as a relative titre compared to the unmodified 'CMV-3G' cassette with 3'supA-LTR, and show that both CMV-RSV hybrid promoters were able to generate LV titre within 2-fold of the control; indeed, the CMV-RSV2-1G variant produced slightly more titre than the control. Interestingly, the RSV-1G[RSV SL1]/3'supA-LTR[RSV R.1-44] expression cassette produced LV titre within 4-fold of the control, demonstrating that the specific 'R' sequence utilized can be different to native HIV-1 sequence. Thus, the novel CMV-RSV hybrid promoters represent the first of their kind in specifically generating LV vRNA with only a single 'G' nucleotide at the TSS.

### EXAMPLE 5: Initial assessment the impact of insertion of GU-rich sequences into the 5' R on LV titres and transcriptional read-in in transduced cells.

To assess whether the engineering of the first stem loop of the LV vRNA R region to contain the GU-rich sequences impacts on LV titre, several of the variants were cloned into an LV vector genome cassette containing an EFS-GFP transgene and the 3'supA-LTR features (SIN-U3+R.1-20 embedded SV40 late polyA). These LV genome expression cassettes were driven by the CMV-RSV-1G hybrid promoter (Example 4). Vectors were initially produced in adherent HEK293T cells and clarified vector harvest titrated by transduction and flow cytometry. Figure 13 displays the results of this experiment demonstrating that indeed the TAR loop can be engineered in such a fashion without impacting on LV titres, as exampled by variants 1GR-GU1 (minor impact) and 1GR-GU4 (no impact).

These variants were then taken forward to assess transcriptional read-in through the 5'supA-LTR in target cells. HEK293T cells were transduced at matched multiplicity of infection (MOI) and passaged for 10 days in order dilute-out any RNA that might be derived from unintegrated LV cDNA. Using primers to the Psi region, mobilized vRNA was measured by RT-qPCR and displayed as either normalized relative values or normalized to integrated copies (Figure 14). These data show that all the variants protected the integrated LV cassette from transcriptional read-in through the 5' supA-LTR.

### EXAMPLE 6: Further characterisation of the impact of various combinations of the invention on LV titres.

Of the initial modified 5' R variants containing a GU-rich DSE, variants '1GR-GU2' and '1GR-GU5' produced the best polyadenylation activity when assessed within a re-capitulated integrated SIN-LTR (see Figure 15). However, initial evaluations of the impact of these modifications on titre of LVs produced in suspension serum-free HK293T cells indicated that these imparted attenuation on titre (and was the chief reason why assessment of transcription read-in of these variants in Figure 14 could not be done). Through various investigations it was surprisingly discovered that part or all of the magnitude of the observed loss in titre of these variants was due to the presence of the back-up polyadenylation sequence specifically when employing the USE within the 3'SIN LTR. This is evidenced by data presented in Figure 19, and shows that just employing the USE caused a ~5-fold drop in titre but this only occurred in LV genome plasmids utilizing the downstream back-up SV40 late polyA sequence. When the back-up polyA was deleted LV titres were increased only in LV genomes harbouring the USE. Thus, the data indicate that the back-up polyA sequence can be deleted and the deletion is even preferred when employing the SV40 late polyA USE in the 3'supA-LTR. This led to the generation of LV genome cassettes employing the 3GR-GU2 or 1GR-GU2 5' modified R region and the 3'supA-LTR, with no back-up polyA sequence, that were capable of generating maximal LV titre.

The features of the 5' and 3' supA-LTR were then evaluated in the context of constructs absent of the back-up polyA sequence, and also with other variable/optional aspects, namely a 5' LTR PAS mutation and the 'MSD-2KOm5' modification (Figure 20). The MSD-2KOm5 modification effectively ablates aberrant splicing from the major splice donor (MSD) region of the packaging signal by engineering of the SL2 loop within it. Mutation of the MSD leads to loss in LV titre but this can be restored by co-expression of a modified U1 snRNA that is able to anneal to sequences within the packaging region. Variant 256U1 was used to rescue titre of all MSD-2KO LV genomes tested. All genomes harbouring the mutated native 5' LTR PAS produced high titre LV, except for those where the 3'supA-LTR PAS had been mutated, demonstrating that this new approach in engineering LV SIN-LTRs was fully efficacious in supplying an efficient transcription termination signal for the transgene cassette, wholly independently of the presence of the native 5' LTR PAS. The experiment also demonstrated that an engineered 5' R sequence containing a GU-rich DSE could also be employed as the 'embedded' R sequence in the 3'supA-LTR sequence during LV production, and that the novel GU-rich DSE could functionally replace the DSE from the embedded heterologous polyA sequence (in this case the SV40 late polyA GU-rich DSE), as well as being independent of any 'back-up' polyA downstream of the LV genome expression cassette. This demonstrates that the 'R-embedded' heterologous polyA sequence can be comprised of a well characterized polyadenylation sequence or of functional component parts (i.e. synthetic).

### EXAMPLE 7: Development of a 'supA-2pA-LTR'.

The molecular gymnastics conducted by the LTRs during reverse transcription of the LV genome provides another opportunity for improving the polyadenylation sequences encoded within. Not only can cell promoter-derived transcription into the integrated LV cassette occur from upstream (i.e. read-in to the 5'SIN-LTR) but in principle read-in can occur into the 3'SIN-LTR from downstream (this essentially results from the LV integrating in either orientation with respect to the closest active cell promoter). Therefore, in order to fully insulate the integrated LV cassette from 'outside' transcription initiation events nearby, it is also desirable to encode effective polyadenylation sequences in the anti-sense orientation. This would be especially desirable if the LV transgene cassette is inverted (i.e. encoded anti-sense with respect to the vRNA), for example as part of a bi-directional cassette, to avoid transgene promoter interference and/or unwanted mobilsation of LV/transgene sequences. Indeed, it is conceivable that all integrated LVs that 'face' an active cell promoter may be subjected to dsRNA sensing mechanisms, since the probability of generating dsRNA in increased. Thus, a substantial number of transduced target cells may be further subjected to PKR-mediated apoptosis. Figure 21 provides a solution, whereby the supA-LTR approach is developed further by inserting a functional polyadenylation sequence in anti-sense orientation in the 3'supA-LTR between the ΔU3 and R-embedded heterologous polyadenylation sequence. The consequence and purpose of this feature is to reduce transcriptonal read-in from cellular promoters downstream of the integration site, and additionally would provide a 'back-up' polyadenylation sequence to an inverted transgene cassette, should it be employed alone or as part of a bi-directional transgene cassette. Thus, the result of this is to generate 5' and 3' 'supA-2pA-LTRs' after integration wherein effectively the LTRs contain strong, bi-directional polyadenylation sequences. This would result in 'protection' from transcriptional read-in and read-out from chromatin on both flanks of the integrated LV.

Figure 53 reports on an initial experiment to show that indeed an efficient bi-directional polyadenylation sequence could be engineered into supA-LTR sequences to generate novel configurations that modelled transcriptional 'insulation' from both upstream and/or downstream cellular promoters. Previous Examples utilising the SV40 late polyadenylation sequence use the 'late' orientation of this bi-directional polyA sequence; including the late USE-PAS sequence. Whilst this sequence also encompasses the two PAS's of the 'early' orientation (i.e. on the bottom strand), this sequence lacks the GU-rich DSE of the early polyA. Therefore, this native sequence was extended upstream of the late USE - effectively providing the early polyA sequence with its native DSE in the inverted direction. In addition, another variant utilised a GU-rich DSE based on the MMTV GU-rich DSE instead of the native SV40 early DSE. These data show that not only can the modifications described herein provide for improved transcription termination to stop transcription read-in from upstream by (i.e. on the top strand) by 10-fold compared to standard SIN-LTRs, but that transcription read-in from downstream (i.e. on the bottom strand) can also be reduced by ~100-fold compared to 'no polyA' (given that standard SIN LTRs do not provide any recognisable polyA sequence on the bottom strand). Sequences based on constructs 6/7 were cloned into 3' supA-LTRs of LV genome expression cassettes containing 5' R modifications (3GR-GU2) to produce supA-2pA-LTR LV stocks at the same (or within 2-fold) of standard LVs (data not shown), demonstrating that these additional inverted sequences within supA-2pA-LTRs were not detrimental to any other aspect of vector biology e.g. 1^{st} strand transfer/reverse transcription.

### EXAMPLE 8: Defining the minimum R sequence embedded within a heterologous polyadenylation sequence as part of the 3' supA-LTR that enables efficient polyadenylation/transcriptional termination and LV output titres.

The minimum R sequence that can embedded within a heterologous polyadenylation sequence as part of the 3' supA-LTR that enables efficient polyadenylation/transcriptional termination and allows efficient first strand transfer (measured by LV titres) was determined empirically. The 3' supA-LTR containing the SV40 late polyadenylation sequence immediately downstream of the SIN-U3 region (ΔU3) was embedded with HIV-1 R region sequence from nucleotide 1 up to nucleotide 20 i.e. these R sequences were inserted between the heterologous PAS and the cleavage site/GU-rich DSE. These 3' supA-LTRs were inserted and tested in the polyadenylation luciferase reporter cassette (see Figure 4 and 11), to empirically test sequence length requirements (as indicated in Figure 3B). The variants different in total length from 10 to 20 nucleotides, and also formed to groups where either the '3G' or '1G' contexts were tested. The 3G/1G sequence reflected scenarios where either 3G or 1G transcriptional start sites were employed as vRNA is transcribed from the 5' LTR, and therefore would match upon annealing during first strand transfer. Suspension (serum-free) suspension HEK293Ts cells were transfected with these constructs together with pDsRed-Express as a transfection efficiency control, to which luciferase activities were normalised. These data are presented in Figure 22, normalised to the '3G-R20' variant, which is the same as the 'R.1-20' sequence used elsewhere in the invention, and previously shown to mediate ~20-to-100 fold improved polyadenylation/termination activity compared to a standard SIN LTR (see Figures 8, 9, 12, 15). These data show that 2-to-3 fold improved polyadenylation/termination was achieved over the 3G-R20/R.1-20 variant by further reducing the length of the embedded R sequence, indicating that positioning of the PAS closer to the downstream cleavage site/GU-rich DSE was desirable.

To assess whether these 3' supA-LTR variants could be employed within an LV genome expression cassette leading to practicable output titres, they were employed within a vector cassette encoding an EFS-GFP reporter (Figure 23). All genomes used a 5' LTR driven by the CMV promoter and 3G TSS, and the native HIV-1 5' PAS was mutated. The data indicate that the embedded R sequence could be reduced to 12-14 nucleotides without impacting LV titres. Use of R sequences of 10 nucleotides reduced LV titres by up to 10-fold.

Therefore, an optimum 3' supA-LTR may contain just 14 nucleotides of R sequence embedded within a heterologous polyadenylation sequence, allowing for very efficient polyadenylation/termination of transcription whilst enabling high titre LV production (i.e. first strand transfer is not impacted).

### EXAMPLE 9: Use of lentiviral vectors harbouring supA-LTRs can increase transgene expression in target cells.

To examine whether the improved polyadenylation sequences of the invention might also lead to increased transgene protein expression (i.e. indirectly by improving transcription termination leading to increased mRNA availability for translation), several LV genome expression cassettes of the invention were used to generate clarified LV harvest material from suspension (serum-free) HEK293T cells. Figure 24 displays a summary of the main features of the LV genome expression cassettes (i.e. principally 5' and 3' modified LTRs) employed, the resultant supA-(SIN)-LTR generated upon reverse transcription (and integration), as well as the normalised transgene expression data in target cells. All the supA-LTR LV genome expression cassettes contained an R-embedded SV40 late polyadenylation sequence downstream of ΔU3 as part of the 3' supA-LTR. The embedded R was either the 3G-R20 sequence or the 3GR-GU2 sequence utilised as the 5' R modification in the invention; the latter also provided the GU-rich DSE, and so the SV40 late polyA GU-rich DSE was deleted from these constructs. At the 5' LTR, the CMV-3G promoter was used with either an unmodified 5' R (TAR/SL1 loop) or used the 3GR-GU2 sequence. The native HIV-1 5' polyA signal and major splice donor (MSD) were optionally mutated; MSD-mutated LV genomes (i.e. MSD-2KO LVs) were produced in the presence of 256U1 in order to boost production titres. LV stocks were titrated by transduction of adherent HEK293T cells by flow cytometry to generate biological titres (GFP TU/mL). Fresh adherent HEK293T cells were subsequently transduced at an MOI of 2 for all vectors, followed by a 10 day passage and integration assay by qPCR against the packaging signal (Ψ). Cells were also analysed by flow cytometry to obtain percent-positive and median fluorescence intensity values, which were multiplied to generate GFP Expression scores. These were normalised according to LV copy-number, and compared to the control LV with a standard SIN-LTR set to 100%. The data show that transgene expression of supA-LTR containing LVs could be increased up to 140% compared to the standard LV, and that the greatest increase was observed with LVs containing the USE-PAS-3GR-GU2 sequence, wherein the downstream native HIV-1 had been mutated.

These initial Examples demonstrate that native HIV-1 polyadenylation sequences within LVs can be deleted and/or made functionally redundant by replacement with more efficient heterologous sequences in both 5' and 3' LTRs in certain precise configurations to generate the supA-LTR LVs. These novel vectors can be produced to high output titres, reduce transcriptional read-in/out of the integrated LV, and lead to improved transgene expression.

### EXAMPLE 10: Positioning of novel cis-acting sequences in the 3'UTR of transgene cassettes within retroviral vector genomes (e.g. lentiviral vector genomes)

The invention describes use of a plurality of 'Cytoplasmic Accumulation Region' (CAR) elements (CARe) or 'tiles' based on the consensus sequence BMWGHWSSWS (SEQ ID NO: 92), for example CCAGATCCTG (SEQ ID NO: 98), within the 3'UTR of a viral vector transgene cassette either alone or in combination with other cis-acting elements such as post-transcription regulatory elements (PREs) and/or ZCCHC14 binding loops. The invention can be used in two main contexts: [1] within viral vector genomes where 'cargo' space is not limiting, and therefore in target cells the CAR elements further enhance expression of a transgene cassette containing another 3'UTR element, such as wPRE, or [2] within viral vector genomes where cargo space is limiting (i.e. at or above or substantially above the packaging 'limit' of the viral vector system employed), and therefore the CAR elements may be used instead of a larger 3'UTR element, such as the wPRE, thus reducing vector genome size, whilst also imparting an increase to transgene expression in target cells compared to a vector genome lacking any 3'UTR cis-acting element. Simplistically, a person skilled in the art will empirically determine the 'net' benefit in pursuing either of these two options. The net benefit is essentially determining the impact of total vector genome size on the maximum practical vector titres that can be achieved with or without the above stated combinations of cis-acting elements, whilst also leading to desirable levels transgene expression in target cells to mediate therapeutic effect. For example, if a lentiviral vector is being employed to deliver a large transgene resulting in a vector genome size of over ~9.5kb (the size of wild type HIV-1) - for example 10-to-13kb - then the viral vector titres are likely to be severely reduced (Sweeney and Vink, 2021). Output titres may be orders of magnitude lower than titres of vector genomes of <9.5kb. In such cases, it may be more advantageous to reduce the size of the vector genome by employing smaller cis-acting elements such as the CARe and/or ZCCHC14 binding loop(s) rather than larger elements such as the wPRE, even if transgene expression in the target cells is slightly or even modestly lower compared to use of wPRE (hence, the net benefit balances vector titre with transgene expression in target cells). For viral vector systems such as AAV and AdV, the threshold of vector genome packaging is much more stringent, with the optimum size for AAVs in the 4.7-5.3kb range (and in some cases ~6) (Wu et al., 2010). For AdVs, the maximum size of the vector genome is considered to be 34-37kb, and the available space for transgene sequences depends on whether 'gutted' or 1st/2nd generation vectors are being employed (Ricobaraza et al., 2020).

Generally the 3'UTR of a transgene cassette encoded within a retroviral vector genome - such as a lentiviral vector genome - also harbours elements required for reverse transcription and integration; namely, the 3' polypurine tract (3'ppt) and the DNA attachment (*att*) site, respectively. The 3'ppt is generated from viral RNA by the RNase H activity of RT plus-strand DNA during reverse transcription, resulting in a 15 nucleotide primer. The 3'ppt is highly conserved in most retroviruses and has been shown to be selectively used as the site of plus-strand initiation (Rausch and Grice, 2004). The *att* site is defined as those end sequences important for integration. The *att* site is comprised of U3 sequences at the terminus of the 5' LTR, and terminal U5 sequences at the end of the 3' LTR (Brown et al., 1999). In certain circumstances, the positioning of additional elements in the 3' UTR of the transgene cassette should be considered relative to these components when a retroviral viral vector is being used. For example, for integration-proficient retro/lentiviral vector genomes containing transgene cassettes in the same orientation as the vector genome, the transgene 3'UTR will necessarily contain the 3'ppt and U3 *att* sequences, since both the transgene cassette and vector genome cassette will utilizes the same polyadenylation (transcriptional terminator) sequence within the 3'LTR.

Accordingly, where the transgene cassette is in a forward orientation with respect to the retroviral vector genome expression cassette, special care should be taken to ensure that the novel cis-acting sequences described herein (e.g. the CAR sequence, and/or the ZCCHC14 protein-binding sequence) is positioned within the 3'UTR of the transgene cassette such that the 3'ppt and att site are not disrupted. Suitable positions are shown in Figure 25. Other suitable positions may also be identified by a person of skill in the art, based on the disclosure provided herein

For example the core sequence that comprises both the 3'ppt and the att site (e.g. of a lentiviral vector genome expression cassette as described herein) may have a sequence of SEQ ID NO: 93 (wherein 3'ppt is in bold, and *att* is underlined):
5'-**AAAAGAAAAGGGGGG**ACTGGAAGGGCTAATTCAC-3' (SEQ ID NO: 93)

Accordingly, where the transgene cassette is in a forward orientation with respect to the retroviral vector genome expression cassette, it is preferable if the sequence above (of SEQ ID NO: 93) is not disrupted by the novel cis-acting sequences described herein.

In one example, the sequence of SEQ ID NO: 94 may be used to provide the 3'ppt and att site (e.g. of a lentiviral vector genome expression cassette as described herein), (wherein 3'ppt is in bold, and *att* is underlined):

5’-
CCAATGACTTACAAGGCAGCTGTAGATCTTAGCCACTTTTTAAAAGAAAAGGGGGGAC
TGGAAGGGCTAATTCACTCCCAA-3’ (SEQ ID NO: 94)

Accordingly, where the transgene cassette is in a forward orientation with respect to the retroviral vector genome expression cassette, it is preferable if the sequence above (of SEQ ID NO: 94) is not disrupted by the novel cis-acting sequences described herein.

Preferably, where the transgene cassette is in a forward orientation with respect to the retroviral vector genome expression cassette cis-acting elements within the 3'UTR of the transgene cassette may be positioned upstream and/or downstream of the above uninterrupted sequences. Figure 25 also indicates how the CARe and/or ZCCHC14 binding loop(s) may be variably positioned within LV genome expression cassettes with or without a PRE, with transgene sequences in forward or reverse orientation.

The above considerations regarding the positioning of the novel cis-acting sequences relative to the 3'ppt and att site of the viral genome expression cassette only apply when the transgene cassette is in the forward orientation with respect to the retroviral vector genome expression cassette. When the transgene cassette employing such novel cis-acting elements is inverted with respect to the retro/lentiviral vector genome cassette (see Figure 25, with "inverted" transgene cassette shown), the 3' UTR of the transgene is spatially separated from the 3'ppt and att site of the retroviral vector genome expression cassette, such that the novel cis-acting sequences cannot disrupt 3'ppt and att sites when placed at any position within the transgene 3'UTR.

### EXAMPLE 11: General positioning of CAR sequences with or without other cis-acting elements in the 3'UTR of transgene cassettes for empirical testing

Figure 26 provides greater detail on how CAR sequences may be used alone or in combination with ZCCHC14 protein-binding sequence(s), with or without a PRE within the 3'UTR of a transgene cassette of a viral vector. Figure 26A shows the CAR element consensus sequence (adapted from Lei et al., 2013). The nucleotide sequences and structures for ZCCHC14-binding loops from HMCV RNA2.7 and WHV wPRE are shown in Figure 26B. Recruitment of ZCCHC14 to the 3' region of the transgene mRNA results in a complex of ZCCHC14-Tent4, which enables mixed tailing within polyA tails of the polyadenylated mRNA. Mixed tailing increases the stability of the transgene mRNA in target cells. A plurality of CARe sequences for example 6 or 8 or 10 or 16 CARe sequences (repeated tandemly) may be positioned throughout the 3'UTR and optionally mixed with one or more ZCCHC15 binding loop(s) upstream of the polyA sequence to generate a composite sequence that increases the expression of the transgene protein. This can be done by rational design, for example testing different transgene cassettes containing increasing numbers of CARe sequences, and optionally in combination with one or more ZCCHC14 protein-binding sequences at different positions. Alternatively, a (semi-)random library of transgene cassette may be produced to generate thousands to tens of millions or more variants, for example using golden gate cloning in combination with nucleic acid barcoding. Such cassettes may be inserted directly into the viral vector of choice and transgene expression levels evaluated in target cells. Figure 26C gives an overview of how such variants may be tested, screened and selected for high transgene expression in target cells to identify candidate composite cis-acting elements.

### EXAMPLE 12: Use of novel CARe-containing 3'UTRs within lentiviral vector transgene cassettes to increase transgene expression in different target cells

Lentiviral vector genomes were designed to contain GFP expression cassettes driven by a variety of different promoters; namely, EF1a (containing its own intron), EFS (EF1a lacking the intron), human phosphoglycerate kinase (huPGK), Ubiquitin (UBC; containing its own promoter) and UBCs (UBC lacking the intron). Transgene cassette 3'UTR variants were made by generally deleting the wPRE and adding a CAR sequence (160bp in size) composed of tandem 16x CARe sequences (CARe.16t) in this position ('Pos1'), and optionally inserting a ZCCHC14 protein-binding sequence (from HMCV RNA2.7 and WHV wPRE) downstream of position 1 ('Pos2'). Controls were made with wPRE-only, ΔwPRE or where the 160bp CAR sequence was inverted (CARe.16t). LVs were produced in suspension (serum-free) HEK293T cells by transient co-transfection, with packaging plasmids (gagpol, VSVG and rev for RRE/rev-dependent genomes). Some studies used standard LV genomes, whereas others used LV genomes wherein the major splice donor (MSD) site and adjacent cryptic splice donor (crSD) site within stem loop 2 (SL2) of the packaging signal has been mutated. This feature abolishes aberrant splicing from the SL2 region but results in reduction in LV titres. Titres of these 'MSD-2KO' genomes can be recovered by use of either [1] co-expression of a modified U1 snRNA (example '256U1' used herein) that anneals to SL1 of the packaging signal (see WO 2021/014157 and WO 2021/160993, incorporated herein by reference), or [2] replacement of the RRE with an intron or so-called 'Vector-Intron' (see Examples above). Clarified crude harvest material from LV productions were titrated on adherent HEK293T cells by either flow cytometry of transduced cells ('Biological titre GFP TU/mL) and/or by Integration assay (Integrating TU/mL). Upon attaining the integrating TU/mL of LV preps, alternative cells such as Jurkat (T-cell line), HEPG2 (Human hepatocyte carcinoma cell line) and equine primary cells '92BR' (testis fibroblasts) were transduced at matched multiplicities of infection (MOI), ranging from MOI 0.1-to-2. Transgene expression levels in these transduce cells was measured by flow cytometry and (GFP) transgene Expression Scores (ES) generated by multiplying %GFP-positive cells by the median fluorescence intensities (arbitrary units).

Tables 6 and 8 present the initial data for these experiments, and Figures 27-31 display some of these data graphically.

**Tables 6 and 8. A summary of lentiviral vectors produced and transgene expression within selected transduced target cell.** The tables display the types of lentiviral vectors produced: standard RRE/rev-dependent (STD RRE-LV), U1/RRE-dependent MSD-mutated LVs (MSD-2KOm5-RRE-LV [+256U1] or MSD-mutated, RRE/rev-independent Vector Intron (ΔRRE) LVs (MSD-2KOm5-ΔRRE-LV); transgene promoters and stated 3'UTR cis-acting elements employed at either upstream (Pos1) or downstream (Pos2) of each other when employing two cis-acting elements. Production titres from suspension (serum-free) HEK293T cells is stated as biological (GFP TU/mL) or integrating (Integrating TU/mL) titres (crude harvest). Median fluorescence intensities (Arb units) are displayed at indicated matched MOIs from transduction of the stated target cells (92BR are donkey primary cells). As can be seen from the data, the novel elements described herein boost expression compared to the ΔwPRE control. ND - not done, NT - not tested. Different 3' UTR cis-acting elements were employed: wPRE, wPRE3 (shortened wPRE), ΔwPRE (wPRE deleted), 16x 10bp CARe sequences in sense (CARe.16t) or antisense (CARe.inv16t), and/or single copy of the ZCCHC14 stem loop from either HCMV RNA2.7 (HCMV.ZSL1) or WHV wPRE (WPRE.ZSI1).

**Table 6**

| | | | | **HEK293T titre** | | **Transgene Expression Score (matched MOI)** | | | | | | | | | | |
|---|---|---|---|---|---|---|---|---|---|---|---|---|---|---|---|---|
| **Genome** | **Promoter** | **3'UTR cis-element** | | | | **HEK293T** | | | **Jurkat** | | **HEPG2** | | | **92BR** | | |
| | | **Pos1** | **Pos2** | **Integrating TU/mL** | **Biological TU/mL** | **MOI 2** | **MOI 1** | **MOI 0.5** | **MOI 1** | **MOI 0.5** | **MOI 2** | **MOI 1** | **MOI 0.5** | **MOI 2** | **MOI 1** | **MOI 0.5** |
| **STD RRE-LV** | **EFS** | **wPRE** | - | 1.6E+07 | 3.6E+07 | 1247591 | 632491 | 326565 | NT | | NT | | | NT | | |
| | | **[ΔwPRE]** | - | 9.9E+05 | 1.8E+07 | 471374 | 251913 | 130829 | | | | | | | | |
| | | **CARe.16t** | - | 7.0E+06 | 4.9E+07 | 548194 | 285569 | 153949 | | | | | | | | |
| | | **CARe.inv16t** | **-** | 9.5E+05 | 2.0E+07 | 355941 | 183557 | 101639 | | | | | | | | |
| | **EFS** | **wPRE** | **-** | 8.8E+07 | 3.8E+07 | 262597 | 129870 | 49713 | NT | | NT | | | NT | | |
| | | **[ΔwPRE]** | **-** | 6.7E+07 | 9.7E+06 | 46958 | 25353 | 12868 | | | | | | | | |
| | | **CARe.16t** | **-** | 8.8E+07 | 1.6E+07 | 96291 | 38012 | 21264 | | | | | | | | |
| | | **CARe.inv16t** | **-** | 4.4E+07 | 4.4E+06 | 63591 | 25186 | 12632 | | | | | | | | |
| | | **-** | **HCMV.ZSL1** | 6.6E+07 | 1.4E+07 | 95801 | 47076 | 21788 | | | | | | | | |
| | | **CARe.16t** | **HCMV.ZSL1** | 9.0E+07 | 2.4E+07 | 125717 | 62443 | 30446 | | | | | | | | |
| | | **CARe.inv16t** | **HCMV.ZSL1** | 4.1E+07 | 7.0E+06 | 101672 | 44098 | 23779 | | | | | | | | |
| **STD RRE-LV** | **EFla** | **wPRE** | **-** | 1.7E+07 | 1.3E+07 | 804059 | 352343 | 154514 | NT | | NT | | | NT | | |
| | | **[ΔwPRE]** | **-** | 2.8E+07 | 1.7E+07 | 484176 | 179367 | 94673 | | | | | | | | |
| | | **CARe.16t** | **-** | 3.0E+07 | 1.7E+07 | 478505 | 222793 | 110931 | | | | | | | | |
| | | **CARe.inv16t** | **-** | 2.1E+07 | 1.1E+07 | 373779 | 168468 | 78228 | | | | | | | | |
| | **huPGK** | **wPRE** | **-** | 6.1E+07 | 1.6E+07 | 162333 | 86049 | 45760 | NT | | NT | | | NT | | |
| | | **[ΔwPRE]** | **-** | 4.8E+07 | 2.7E+06 | 49647 | 24658 | 12371 | | | | | | | | |
| | | **CARe.16t** | **-** | 4.5E+07 | 4.4E+06 | 72519 | 35050 | 16763 | | | | | | | | |
| | | **CARe.inv16t** | **-** | 4.8E+07 | 2.5E+06 | 41151 | 19484 | 9020 | | | | | | | | |
| **STD RRE-LV** | **EFS** | **wPRE** | - | 1.3E+08 | 5.1E+07 | 1089273 | 618520 | 304897 | NT | | NT | | | NT | | |
| | | **[ΔwPRE]** | - | 8.4E+07 | 6.5E+06 | 183232 | 115109 | 55733 | | | | | | | | |
| | | **CARe.16t** | - | 1.1E+08 | 1.3E+07 | 292693 | 167590 | 90756 | | | | | | | | |
| | **EF1a** | **wPRE** | - | 4.5E+07 | 2.6E+07 | 2698168 | 1511460 | 831648 | NT | | NT | | | NT | | |
| | | **[ΔwPRE]** | - | 6.9E+07 | 3.0E+07 | 1410002 | 761009 | 369706 | | | | | | | | |
| | | **CARe.16t** | - | 5.6E+07 | 2.9E+07 | 1935064 | 1117901 | 473344 | | | | | | | | |
| | **huPGK** | **wPRE** | - | 9.6E+07 | 2.8E+07 | 293234 | 160592 | 82787 | NT | | NT | | | NT | | |
| | | **[ΔwPRE]** | - | 9.8E+07 | 5.0E+06 | 75645 | 36622 | 18074 | | | | | | | | |
| | | **CARe.16t** | - | 8.7E+07 | 7.4E+06 | 109503 | 57776 | 27314 | | | | | | | | |
| | **UBC** | **wPRE** | - | 3.7E+07 | 1.5E+07 | 294101 | 150325 | 77648 | NT | | NT | | | NT | | |
| | | **[ΔwPRE]** | - | 4.0E+07 | 7.2E+06 | 58695 | 27648 | 13270 | | | | | | | | |
| | | **CARe.16t** | - | 5.6E+07 | 1.3E+07 | 91615 | 48088 | 20619 | | | | | | | | |
| | **UBCs** | **wPRE** | - | 9.3E+07 | 1.4E+07 | 500992 | 271560 | 138104 | NT | | NT | | | NT | | |
| | | **[ΔwPRE]** | - | 8.2E+07 | 1.3E+06 | 297049 | 180390 | 78617 | | | | | | | | |
| | | **CARe.16t** | - | 1.0E+08 | 4.2E+06 | 291721 | 159021 | 71591 | | | | | | | | |

**Table 8**

| | | | | **HEK293T titre** | | **Transgene Expression Score (matched MOI)** | | | | | | | | | | |
|---|---|---|---|---|---|---|---|---|---|---|---|---|---|---|---|---|
| **Genome** | **Promoter** | **3'UTR cis-element** | | | | **HEK293T** | | | **Jurkat** | | **HEPG2** | | | **92BR** | | |
| | | **Pos1** | **Pos2** | **Integrating TU/mL** | **Biological TU/mL** | **MOI 2** | **MOI 1** | **MOI 0.5** | **MOI 1** | **MOI 0.5** | **MOI 2** | **MOI 1** | **MOI 0.5** | **MOI 2** | **MOI 1** | **MOI 0.5** |
| **STD RRE-LV** | **EFS** | **wPRE** | **-** | 7.0E+06 | ND | NT | | | 58539 | 33937 | NT | | | NT | | |
| | | **[ΔwPRE]** | **-** | 6.5E+06 | | | | | 31186 | 16991 | | | | | | |
| | | **CARe.16t** | **-** | 1.0E+07 | | | | | 59192 | 29766 | | | | | | |
| | | **-** | **HCMV.ZSL1** | 6.1E+06 | | | | | 31338 | 15096 | | | | | | |
| | | **CARe.16t** | **HCMV.ZSL1** | 8.7E+06 | | | | | 67576 | 33899 | | | | | | |
| | **EFla** | **wPRE** | **-** | 1.1E+06 | ND | NT | | | 204898 | 115583 | | | | | | |
| | | **[ΔwPRE]** | **-** | 1.9E+06 | | | | | 182003 | 87831 | | | | | | |
| | | **CARe.16t** | **-** | 1.9E+06 | | | | | 391180 | 201930 | | | | | | |
| | | **wPRE** | **-** | 3.6E+07 | 1.9E+07 | 610142 | 317856 | 166677 | | | | | | 10267 | 7796 | 7049 |
| | | **[ΔwPRE]** | **-** | 3.5E+07 | 4.9E+06 | 103810 | 48948 | 25026 | | | | | | 2163 | 1816 | 1540 |
| | | **CARe.16t** | **-** | 2.8E+07 | 5.6E+06 | 181922 | 94507 | 48215 | | | | | | 3828 | 3006 | 2736 |
| | | **-** | **HCMV.ZSL1** | 2.2E+07 | 4.6E+06 | 218375 | 108976 | 48470 | | | | | | 4209 | 3080 | 2613 |
| | **EFS** | **CARe.16t** | **HCMV.ZSL1** | 2.0E+07 | 6.4E+06 | 318723 | 142312 | 67325 | NT | | NT | | | 6083 | 5179 | 4964 |
| | | **-** | **WPRE.ZSL1** | 3.9E+07 | 8.2E+06 | 136401 | 67219 | 35293 | | | | | | 2004 | 2111 | 1749 |
| | | **CARe.16t** | **WPRE.ZSL1** | 3.6E+07 | 1.1E+07 | 268334 | 136131 | 69600 | | | | | | 4746 | 4099 | 3729 |
| **STD RRE-LV** | | **wPRE3** | **-** | 9.0E+06 | 8.1E+06 | 322180 | 151409 | 69671 | | | | | | 3633 | 2993 | 2378 |
| | | **wPRE** | **CARe.16t** | 1.3E+07 | 1.4E+07 | 563406 | 308401 | 141266 | | | | | | 9497 | 9754 | 7749 |
| | **EFla** | **wPRE** | **-** | 1.4E+07 | 8.1E+06 | 1914297 | 987455 | 475178 | NT | | NT | | | 43876 | 38987 | 37755 |
| | | **[ΔwPRE]** | **-** | 1.3E+07 | 8.0E+06 | 971945 | 418188 | 190283 | | | | | | 24431 | 17105 | 15371 |
| | | **CARe.16t** | **-** | 1.4E+07 | 1.0E+07 | 1182561 | 547375 | 251970 | | | | | | 34199 | 28883 | 24272 |
| | | **-** | **WPRE.ZSL1** | 2.5E+07 | 1.0E+07 | 1188685 | 508016 | 236951 | | | | | | 20374 | 16537 | 16008 |
| | | **CARe.16t** | **WPRE.ZSL1** | 2.2E+07 | 1.1E+07 | 1501211 | 687257 | 339840 | | | | | | 38114 | 35505 | 29263 |
| | **EFS** | **wPRE** | **-** | 3.4E+07 | 1.1E+07 | 26690 | 19101 | 26690 | NT | NT | | | NT | | | |
| | | **[ΔwPRE]** | **-** | 3.0E+07 | 2.4E+06 | 5036 | 4554 | 5036 | | | | | | | | |
| | | **CARe.16t** | **-** | 3.1E+07 | 4.1E+06 | 7875 | 6994 | 7875 | | | | | | | | |
| | | **CARe.inv16t** | **-** | 1.8E+07 | 1.3E+06 | 4626 | 4338 | 4626 | | | | | | | | |
| **MSD-2KOm5 -RRE-LV [+ 256U1]** | **EF1a** | **wPRE** | **-** | 2.0E+07 | 1.3E+07 | 92944 | 69371 | 92944 | NT | NT | | | NT | | | |
| | | **[ΔwPRE]** | **-** | 2.3E+07 | 1.1E+07 | 40945 | 32043 | 40945 | | | | | | | | |
| | | **CARe.16t** | **-** | 2.7E+07 | 1.6E+07 | 50331 | 42087 | 50331 | | | | | | | | |
| | | **CARe.inv16t** | **-** | 2.1E+07 | 1.0E+07 | 32043 | 27103 | 32043 | | | | | | | | |
| | **huPGK** | **wPRE** | **-** | 3.3E+07 | 6.5E+06 | 12822 | 11259 | 12822 | NT | NT | | | NT | | | |
| | | **[ΔwPRE]** | **-** | 2.9E+07 | 1.7E+06 | 4179 | 3566 | 4179 | | | | | | | | |
| | | **CARe.16t** | **-** | 3.2E+07 | 3.4E+06 | 5558 | 4751 | 5558 | | | | | | | | |
| | | **CARe.inv16t** | **-** | 2.7E+07 | 1.4E+06 | 3756 | 3258 | 3756 | | | | | | | | |
| **MSD-2KOm5 -ΔRRE-LV** | **EFS** | **wPRE** | **-** | 1.9E+07 | 2.6E+07 | NT | 11407 | NT | NT | NT | 30991 | NT | NT | | | |
| | | **[ΔwPRE]** | **-** | 1.5E+07 | 6.1E+06 | | 2158 | | | | 20529 | | | | | |
| | | **CARe.16t** | **-** | 2.2E+07 | 1.3E+07 | | 3024 | | | | 29330 | | | | | |
| | | **CARe.inv16t** | **-** | 1.3E+07 | 5.0E+06 | | 1700 | | | | 16648 | | | | | |
| | | **WPRE.ZSL1** | **-** | 1.3E+07 | 8.2E+06 | | 2904 | | | | 27254 | | | | | |
| | | **CARe.16t** | **WPRE.ZSL1** | 1.7E+07 | 1.6E+07 | | 4635 | | | | 35294 | | | | | |
| | | **CARe.inv16t** | **WPRE.ZSL1** | 1.6E+07 | 1.1E+07 | | 2897 | | | | 22093 | | | | | |

A number of general trends can be observed from the data presented in Table 6 and Figures 27-31. Firstly, the measure of LV titres by GFP ('biological' TU/mL) in adherent HEK293T cells tended to reveal the largest impacts of deleting the wPRE, and the partial 'rescue' of expression by the cis-acting sequences. This is to be expected, as loss of GFP transgene expression results in fewer cells being counted as transduced i.e. actual transduced cells appeared in the non-gated population. This is supported by the integration titre values, which are independent of transgene expression; here, LV titres were similar, although there were examples of 'rescued' integration titres when using CAR.16t with or without a ZCCHC14-protein-binding sequence (see Figures 27A, 27B, 30A). This trend generally held for intron-less promoters, since integrating titres for EF1a- or UBC- (both harbouring introns) containing LVs were actually slightly stimulated by deletion of wPRE. These effects were specific to the CAR sequences being employed in the sense orientation, since the inverted CAR sequences did not mediate the effect.

The benefit of the use of the CAR sequences with or without a ZCCHC14 protein-binding sequence in the context of wPRE-deleted LVs were tested in target cells different to adherent HEK293T cells, since the major purpose of viral vectors are to deliver transgenes to more relevant cell types rather than vector production cells. Initial experiments used Jurkat cells (to model T-cells i.e. for CAR-T therapy), HEPG2 cells (to model a liver indication) and a primary cell with limited cell doublings (92BR; a donkey testis cell). Interestingly, the largest effects of the CARe-based cis-acting elements were observed in these cell types. Figure 28 presents data in transduced Jurkat cells at three different MOls, and also reports on transgene mRNA levels in the same experiment. These data show that the CARe.16t sequence was able to boost expression from LVs delivering an EFS-GFP cassette lacking the wPRE to the same levels observed with a cassette containing the wPRE. Surprisingly, the CAR.16t sequence provided a boost to expression greater than cassettes containing the wPRE when employing the EF1a promoter (i.e. containing an intron). This was not expected, given that CAR element were generally expected to act on intronless transcripts in their natural context. Figure 28D confirmed that some aspect of all these effects were due to increased mRNA steady-state pools in transduced cells.

In Figure 30 the use of CARe.16t or a ZCCHC14 protein-binding sequence alone provided a substantial increase in expression in HEPG2 cells when using a wPRE-deleted LV. The inverted control did not provide this effect. Interestingly, the CAR.16t + ZCCHC14 protein-binding sequence cassette provided the highest level of expression in HEPG2 cells, which was greater than the wPRE-only LV control.

In Figure 30 the use of CARe.16t or ZCCHC14 protein-binding sequence alone provided an increase in expression in primary cells (92BR) when using a wPRE-deleted, EFS-GFP containing LV. The boost to expression was similar to that observed with the truncated wPRE element 'WPRE3' For EF1a-GFP containing LV genome, the boost to expression was greatest when both CARe.16t and ZCCHC14 protein-binding sequence were used together.

These elements could be used equally well when employing standard RRE/rev-dependent LV genomes, or when using the MSD-2KO vector genomes - either U1/rev-dependent (Figure 29) RE/rev-independent Vector Intron (ΔRRE) (Figure 30) versions.

### EXAMPLE 13: Pairing a ZCCHC14 protein-binding sequence with multiple numbers of CARe tiles leads to increased transgene expression in transduced cells

To further exemplify the paring of the ZCCHC14 protein-binding sequence with multiple CARe tiles to generate novel minimal transgene expression enhancer sequences in the 3' UTR, several variants were constructed wherein a single (downstream) ZCCHC14 protein-binding sequence was paired with tandem arrays of the 10bp CARe sequence, ranging from a single copy up to 20 copies. These were inserted into an EFS-GFP expression cassette within a standard RRE/rev-dependent LV genome cassette and used to produce LVs in suspension (serum-free) HEK293T cells. Controls LVs containing wPRE, no cis-acting element (ΔwPRE), the 16 × 10bp CARe variant (CARe.16t) or a single ZCCHC14 protein-binding sequence were also produced. Clarified LV supernatants were titrated to generate integrating titres (TU/mL), on adherent HEK293T cells. Following this, fresh adherent HEK293T cells were transduced at matched MOIs of 0.5, 1 or 2, and cultures incubated for 3 days. GFP expression in transduced cells was measured by flow cytometry, and median fluorescent intensity values generated (Arbitrary units). These were normalised to the wPRE-containing control (set to 100%). These data are displayed in Figure 32, and show that both the single ZCCHC14 protein-binding sequence and CARe.16t elements boosted expression on their own. Pairing of the single ZCCHC14 protein-binding sequence with multiple CARe tiles produced a further increase in transgene expression in a generally correlative manner (i.e. increasing CARe tile numbers produced a greater increase in transgene expression), although perhaps the greatest increase in expression was realised at 8 copies of the CARe tiles. The CARe.8t element in combination with the single ZCCHC14 protein-binding sequence was ~152bp, generating transgene expression of ~60% of that of the wPRE control, despite being only 20% of the size of the wPRE.

### EXAMPLE 14: Use of a Vector-Intron within LV genomes harbouring functional mutations within the SL2 loop of the packaging signal to ablate aberrant splicing allow for rev/RRE-independence.

Figure 33 displays the basic configuration of the Vector-Intron LV genome expression cassette in comparison to current, 'standard' LV genomes. Both types of genome expression cassettes are driven by powerful, constitutive promoters independently of HIV-1 tat, although the inventors have shown previously that HIV-1 tat can fully recover the titres of LV genomes harbouring a mutation in the MSD. However, contemporary 3^{rd} generation LV production systems have been purposely developed to remove tat-dependence due to the safety concerns associated with this potent transcriptional activator. Re-instating tat into LV production systems would be seen as a substantial retrograde step in vector development, and it may be difficult to justify to regulators in order to sanction their use for clinical applications. Standard and VI LV genomes both share the same packaging sequence from the 5' R to SL1 (dimerization loop) but then diverge principally in the SL2 loop, which contains the MSD. For VI genomes the MSD and a cryptic splice donor (crSD) site adjacent to it are both mutated to stop aberrant splicing into downstream positions. The inventors previously developed a substantive modification to the SL2 loop wherein the majority of the HIV-1 sequence is replaced with a heterologous stem loop absent of any splice donor site called 'MSD-2KOm5'. The MSD-2KOm5 variant is preferred in the present invention as it has a lower impact on LV titres compared to the 'MSD-2KO' mutation, which harbours GT>CA mutations at the MSD and crSD sites. Additionally, the MSD-2KOm5 variant also appears to suppress aberrant splicing from another cryptic splice donor site in the SL4 loop of the packaging signal.

Although optional, VI-containing LV genomes may also be deleted in the p17-INS sequence of the gag region that is typically retained in standard LV genomes as part of the wider packaging sequence. As for standard LV genomes, VI-containing LV genomes harbour the central poly purine tract (cppt) and a self-inactivating (SIN) 3'LTR. The transgene cassette typically contains a pol-II promoter and transgene ORF, with the 3'polyA site in the SIN LTR being used for both the vector genome vRNA and the transgene mRNA. The VI-containing LV genomes differ further from standard LV genomes in that they encode a functional intron in place of the RRE. Contemporary LVs also typically include a post-transcriptional regulatory element (PRE), such as the wPRE to enhance expression of (typically intron-less) transgene cassettes. Other transgenic sequences may also be encoded on the vector such as pol-III driven microRNA or gRNA for CRISPR-cas9 approaches. The VI-containing LV genomes differ further from standard LV genomes in that they encode a functional intron in place of the RRE. When considering the length of the wild type HIV-1 genome of ~9.5kb, the VI-containing LV in its basic form can package approximately ~1kb of additional transgene sequence relative to standard LV genomes.

Figure 34 provides evidence that combining both the MSD-2KO feature and the Vector-Intron is beneficial in achieving maximal LV titres in a rev/RRE-independent manner. HIV-CMV-GFP vectors were created from a standard LV genome, containing a wild type packaging signal (with intact MSD) and the RRE. Vectors were produced in both adherent (Figure 34A) and suspension [serum-free] (Figure 34B) mode in HEK293T cells, and efficient out-put titres were shown to be dependent on the presence of rev. Deletion of the RRE from the standard MSD-proficient LV genome resulted in loss of rev-responsiveness (as expected), although interestingly titres produced in the absence of rev increased 10-fold, confirming that the RRE is inherently unstabilising to vRNA. The insertion of a VI (the first variant VI_1.1, is the EF1a intron - see Table 7 below) in place of the deleted RRE had no substantive effect on LV titres. However, the same modifications tested in the context of the MSD-2KO LV genome produced surprisingly different results. Here, the attenuating impact of the MSD-2KO mutation was dramatic for the RRE-containing genome, as titres were three orders of magnitude reduced in the presence of rev. Addition of the VI to this RRE-containing LV genome dramatically increased vector titres independently of the presence of rev, although the highest titres were only realized in the presence of rev. Given the apparent destabilizing effect of the RRE observed previously, the RRE was deleted from the MSD-2KO LV genome containing the VI, which not only increased vector titres to within 2-fold of the standard LV genome (+rev) but this was not dependent on the presence of rev. Thus it was shown that the ability of a Vector-Intron to rescue titres of MSD-2KO modified LV genomes is maximized when the RRE is deleted.

### Table 7: A list of Vector-Intron variants designed and used to exemplify and optimize the invention, together with a visual representation of the functional sequences utilized in each variant.

Vector-Introns tested included native sequences from the EF1a (v1 series) and Ubiquitin (v2 series) introns, and also widely used chimeric/synthetic introns such as CAG (Chicken Actin + Rabbit beta-globin; v3 series) and the Rabbit beta-globin + human IgG chimeric intron used in the pCI/pSI series of expression plasmids sold by Promega; v4 series). Further Vector-Introns were designed to swap different functional parts of the intron, namely: the splice donor sequence, the branch-splice acceptor sequence, as well as appending upstream exonic splicing enhancer elements from HIV-1 (hESE, hESE2, hGAR). These novel Vector-Introns were improved in either their ability to splice 'cleanly' and/or ability to increase LV titre.

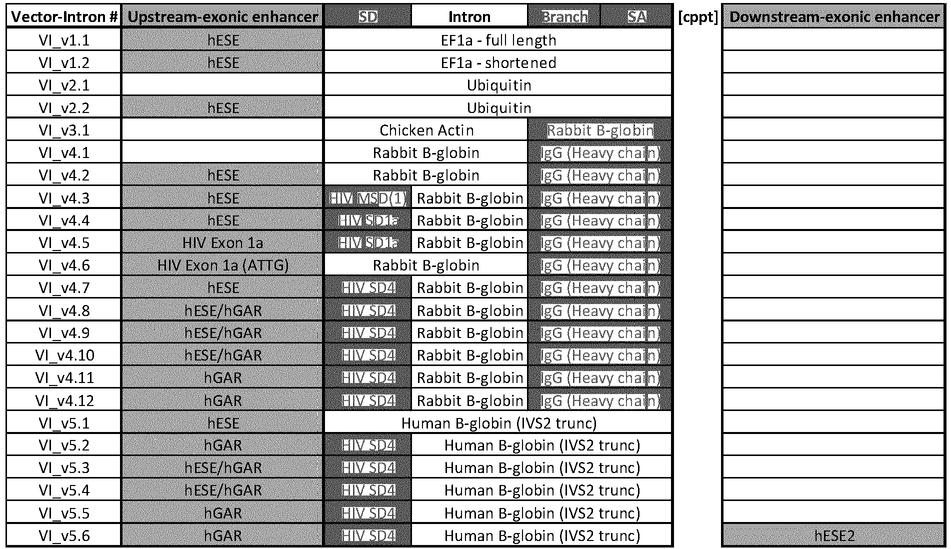

### EXAMPLE 15: The MSD-2KO and RRE-deletion work synergistically to generate correctly spliced and packaged LV vRNA when applied to VI-containing LV genome expression cassettes.

To understand the impact of the MSD-2KO, RRE and rev on the production of VI-'derived' LV vRNA in the cell and in virions, samples from the experiment performed in generating the data in Figure 34A were used (only samples where the VI was present in the LV genome). Total poly-A selected RNA from post-production cell lysates and crude LV harvest material were subjected to RT-PCR analysis using appropriately positioned forward and reverse primers as shown in Figure 35A. The same figure displays the VI-containing LV DNA expression cassette and the positions of relevant *cis*-acting sequences with regard to potential spliced products generated from transcription from the external promoter (here, the transgene mRNA is not shown for clarity). Thus, different sized RT-PCR products might be detected depending on the presence of the RRE and the potential for splicing from the MSD, crSD and the alternative crSD2 (active in MSD-2KO but not MSD-2KOm5 variants; see later Examples) to downstream splice acceptors (including the VI sa).

Figure 35B displays the result of this analysis and provides further support to the data shown in Figure 34. First of note is the stimulation of longer packageable vRNA by rev only when the RRE is resident on the genome. Interestingly, rev/RRE appeared to inhibit splicing-out of the VI by ~50%, resulting in a 50:50 mixture of LV vRNA retaining the VI or not. This in itself provided a secondary reason to pursue rev/RRE-independence of VI-LV genomes since it is desirable to generate LV populations with homogeneous vRNAs. Secondly, the results show an extremely large amount of aberrant splicing from the MSD to the VI splice acceptor, analogous to what is typically observed in standard LV genomes containing the EF1a promoter (with its own intron) as the internal transgene promoter (VI_v1.1 is the EF1a intron). The MSD-2KO mutation ablated this aberrant splicing, although this stimulated aberrant splicing from the crSD2 site in SL4 of the packaging signal into the VI splice acceptor. Interestingly, the deletion of the RRE minimized the amount of this specific aberrant splicing activity, although there were other products detected in the cells that were apparently not efficiently packaged. Finally, the MSD-2KO/ΔRRE VI-LV genome generated the highest levels of VI-spliced LV vRNA in both cells and vector virions, and this occurred independently of rev. Based on these data, other introns - both natural and synthetic - were evaluated for use as Vector-Introns in the invention as reported in following examples.

### EXAMPLE 16: Initial evaluation of different introns for use as the Vector-Intron

The principals of RNA splicing are well characterized, and the cis-acting elements required are well understood, as are the factors that interact with them (Bates et al. (2017), Pharmacological Reviews 69: 63-79). Figure 36 provides a basic overview of the core *cis-*acting sequences embedded within introns as well as adjacent exonic regions that are known to modulate splicing efficiency. Since intronic sequences are not constrained by protein-coding sequences, they tend to be more divergent from exonic sequences when comparing homologous genes from similar species. To assess the impact of varying the specific intronic sequence used as the Vector-Intron, several initial variants were generated based on well-characterized and/or widely used introns. An overview of the various introns and chimeric introns is shown in Table 7. Initial variants were based on the native intron from the human Ubiquitin gene (UBC), as well as synthetic/chimeric introns of the CAG promoter and the 'pCI' series of expression plasmids originally developed by Promega; these were compared to the native intron of the EF1a promoter used in previous Examples (VI_v1.1).

Figure 37 displays the results of similarly described previous comparisons of standard, MSD/RRE-containing LV vectors and these VI-LV vectors in adherent HEK293T cells. Whilst in this data set the VI_v1.1 variant did not restore vector titres to the same levels as seen in Figure 34A (perhaps due to some technical variability), the experiment did indicate that the general effect of titre enhancement by a VI appear to be generally independent of any specific intron i.e. the action of splicing was the most important aspect. The UBC intron appeared to be minimally active whereas the chimeric intron from pCI gave the greatest increase in titres in this experiment. It has been reported that splicing of the UBC intron from RRE-containing LV genomes behaves differently to other introns (such as EF1a; Cooper A., R., et al., Nucleic Acids Research, Volume 43, Issue 1, 9 January 2015, Pages 682-690*).* Given the potential for variable results with the UBC intron, it was not pursued further for further optimisation. Rather, VI_v4.2, based on the chimeric intron from pCI, was chosen for further optimization, and all subsequent evaluations were performed in suspension (serum-free) HEK293T cells, where more robust comparisons were gained.

### EXAMPLE 17: Optimisation of a Vector-Intron

The following example demonstrates how Vector-Intron sequences (and those flanking it) can be improved towards attaining better titre-enhancing effect. The variant VI_v4.1 is the chimeric intron from the pCI series of expression plasmids developed by Promega; this contains a fusion between the rabbit beta-globin intron (splice donor and most of the intronic sequence) and the human IgG heavy chain intron branch and splice acceptor sequence. Variant VI_v4.2 additionally includes a 39bp 'legacy' sequence from the RRE that was retained due to cloning restraints; upon *in silico* analysis this sequence is predicted to bind a number of splicing enhancers and therefore became a sequence of interest in a potential role in enhancing VI splicing. This sequence was found to improve the titre-boosting effect of VI_4.1 in Example 17 (Figure 37), and is herein referred to as 'hESE'. To assess how further improvements might be gained, other sequences were added or swapped-in with typical sequences shown in Figure 36, and MSD-2KO/ΔRRE VL LV genomes containing an EFS-GFP transgene produced in suspension (serum-free) HEK293T cells in the absence of rev (see Table 7 and Figure 38). First, the splice donor of the rabbit beta-globin intron of VI_4.2 was replaced with the major splice donor (MSD[1]) of HIV-1 in variant VI_v4.3, which indeed stimulated titre increase. An alternative HIV-1 splice donor sequence was assessed, optionally together with its upstream exonic sequence (VI_v4.4/4.5/4.6); this exon has previously shown to stabilize HIV-1 vRNA (Lützelberger, M., et al. Journal of Biological Chemistry, Volume 281, Issue 27, 18644 - 18651). VI_v4.6 additionally harboured ATG mutations to ensure no translation of HIV-1 sequences, and was tested with the rabbit beta-globin splice donor sequence. However, none of these features appeared to provide further benefit in VI LV genomes, and demonstrated that the novel hESE provided substantial benefit to titre increase. Next splice donor 4 (SD4) from HIV-1 was used to replace the rabbit beta-globin splice donor sequence, optionally together with another ESE from HIV-1 called 'GAR' (Kammler, S., et al. RNA. 2001;7(3):421-434*. &* Caputi, M, et al., J Virol. 2004; 78(12):6517-6526); the GAR-SD4 sequence is principally exon5 of wild type HIV-1. The titre of the VI_v4.8 variant was the highest of this set of variants tested, indicating that the GAR ESE provided some benefit.

### EXAMPLE 18: Vector Intron variants based on the human beta-globin intron-2

The human beta-globin gene transcription unit contains a well characterized and efficiently spliced intron (the second intron, also known as 'IVS2'). It is known that this intron is efficiently spliced from lentiviral vector genomes when inserted in the forward orientation, and is only retained when the RRE is inserted within intronic sequence (Uchida, N., et al. Nat Commun 10, 4479. 2019)*.* It was therefore hypothesized that this intron (herein referred to as 'hu B-Glo') might form an alternative for optimization as a Vector-Intron. Accordingly, VI_5.1 was generated, which is the entire hu B-Glo intron together with the hESE. Additionally, VI_5.2 was also generated, wherein the hu B-Glo splice donor was replaced with HIV-1 SD4 and the hESE was replaced with the hGAR. These were cloned and tested within an MSD-2KO/ΔRRE LV-EFS-GFP genomes to produce LV in suspension (serum-free) HEK293T cells in comparison to variants containing VI_v4.2/4.8 (Figure 39). These data show that indeed the hu B-Glo based VIs were indeed capable of producing high titre MSD-2KO/ΔRRE LV in a rev-independent manner, with VI_5.2 producing the highest boost in titre.

Next, these variant VIs were transferred into an alternative LV backbone wherein the preferred MSD/crSD mutant 'MSD-2KOm5' was present and the p17-INS sequence in the retained gag sequence (as part of packaging sequence) has been deleted to further increase capacity ('Δp17INS' or 'gag81'; see Example 21). These variant genomes were compared to previous variants in suspension (serum-free) HEK293T cells in the absence of rev (Figure 40). These results show that the variant VIs are able to produce LV titres of MSD-mutated genomes in a rev/RRE-independent manner irrespectively of the type of ablative mutation of the MSD.

### EXAMPLE 19: MSD-2KO/ΔRRE Vector-Intron LV production is stimulated by Prostratin

To assess if MSD-2KO/ΔRRE Vector-Intron LV production is stimulated by Prostratin, suspension (serum-free) HEK293T cells were transfected with LV packaging components and with either a standard LV genome plasmid +/- rev plasmid, or a MSD-2KO/ΔRRE VI LV genome plasmid, with or without addition of Prostratin post-transfection (Figure 41). The data demonstrates that the rev/RRE-independent production of MSD-2KO/ΔRRE VI LVs can be stimulated by Prostratin to a similar extent as observed with standard LVs.

### EXAMPLE 20: Use of 'self-cleaving' cis-elements within the 3' UTR of inverted transgene cassettes contained within Vector-Intron LV genome cassettes to rescue LV component expression from dsRNA sensing mechanisms and allow for efficient transgene repression during LV production

The unique features of the Vector-Intron genomes allows for other novel aspects of vector design that are advantageous. Since out-splicing of the VI stimulates splicing of other introns encoded within the transgene cassette, the retention of transgene introns (such as the EF1a promoter intron) when the transgene cassette is facing forward (i.e. encoded on the top strand) is not efficient/possible. Whilst there are good reasons why transgene introns perhaps should not be used in LVs (principally because splicing events into cellular genes can occur), nevertheless they provide a boost to expression in certain target cells. A way of ensuring transgene intron retention within VI LV vRNA is to invert the transgene cassette so that the transgene intron is not recognised as such in the anti-sense direction. If utilising a tissue specific promoter that is not/minimally active during production then this approach requires no further considerations. However, should the transgene promoter generate sufficient levels of transgene mRNA during LV production then the possibility of generating long dsRNA products via vRNA:mRNA annealing increases, and this will trigger innate dsRNA sensing pathways, such as those involving oligoadenylate synthetase-ribonuclease L (OAS-RNase L), protein kinase R (PKR), and interferon (IFN)/ melanoma differentiation-associated protein 5 (MDA-5). Whilst a number of these pathways are likely to be (partly) defective in HEK293(T) cells (Ferreira, C., B., et. al., Mol Ther Methods Clin Dev. (2019);17:209-219.), here the inventors provide evidence that generation of cytoplasmic dsRNA results in suppression of *de novo* protein synthesis. Figure 43 shows a simple experiment where a standard rev/RRE-dependent LV containing an inverted EFS-GFP-polyA cassette was produced in HEK293T cells with or without rev. Only when rev was present - and therefore full length vRNA could be transported to the cytoplasm where it could anneal to the transgene mRNA - was *de novo* expression of VSV-G/p24 suppressed, indicating that a cytoplasmic dsRNA response (e.g. PKR) was likely triggered. In the same experiment a VI LV genome was tested with the same transgene cassette in forward or inverted orientation without rev, and showed the same phenotype for the inverted variant. Therefore, one solution to avoid this response is to knock-down or knock out endogenous PKR in the LV production cell, or over-express protein factors shown to inhibit dsRNA sensing mechanisms, as indeed others have shown is possible (Hu, P., et. al., Gene Ther. 2018 Oct;25(7):454-472 & Maetzig T., et. al., Gene Ther. (2010);17(3):400-11).

An alternative to the above solutions, which is entirely dependent on the Vector-Intron approach, is to insert functional cis-elements within the 3' UTR of the inverted transgene cassette such that they are positioned within the VI sequence encoded on the top strand, and therefore are lost from the packaged vRNA and resulting integrated LV cassette (Figure 44). The cis-elements are 'self-cleaving' or de-stabilising elements such that the steady-state pool of transgene pre-/mRNA during LV production is reduced, and therefore limiting the amount of dsRNA that can be generated.

For example, the cis-elements can be one or more 'self-targeting' microRNA cassettes that are cleaved from the 3' UTR of the transgene mRNA (thus removing the polyA tail of the mRNA, leading to destabilisation), and then are processed by DROSHA/Dicer and loaded into the RISC complex to target 5' UTR and/or coding-region and/or 3'UTR sequences within the transgene mRNA such that further cleavage can occur. Preferably, the microRNAs are designed such that guide strand is fully matched to the target sequence in the transgene mRNA (allowing the further efficient cleavage) and the passenger strand is mis-matched, such that if the passenger strand was occasionally loaded as the guide strand it would only anneal to the vRNA and not efficiently cleave it (Figure 45).

For example, the cis-elements can be one or more 'self-cleaving' ribozymes that once transcribed in the pre-mRNA efficiently result in cleavage of the 3' UTR and loss of the polyA tail, and will therefore limit the amount of transgene mRNA being transported to the cytoplasm (Figure 45). The invention provides several examples of how self-cleaving ribozymes can be used to accomplish this in Figure 46. A VI LV genome containing an inverted EF1a-GFP-polyA cassette, as well as the MSD-2KOm5 modification, the gag-p17 deletion in Psi and the VI_v5.7 in place of the RRE, was generated to harbour various combinations of cis-acting elements within the 3' UTR of the inverted transgene cassette, and also embedded within the anti-sense VI sequence (see Figure 46B). These were ribozymes based on: Hammerhead ribozyme (HH_RZ), Hepatitis delta virus ribozyme (HDV-AG), and two modified *Schistosoma mansoni* hammerhead ribozymes (T3H38/T3H48) (De la Peña, M., et. al., EMBO J. (2003);22(20):5561-70; Webb, C., H., & Lupták, A., RNA Biol. (2011);8(5):719-27; & Zhong, G., et. al., Nat Biotechnol. (2020);38(2):169-175.). Additionally, other cis-elements tested were the 'negative regulator of splicing' (NRS) element from RSV, or a splice donor site, as these have been shown to modulate mRNA stability and/or nuclear retention via recruitment of U1 snRNA (Cochrane, A., W., et. al., Retrovirology 3, 18 (2006); and Lee, E., S., et. al., PLoS One. (2015);10(3):e0122743). These genomes plasmids were used to make LVs in suspension (serum-free) HEK293T cells, and VSVG/p24 levels or vRNA levels assessed in harvest supernatants (Figure 46A and 46C respectively). In summary, Figure 46A shows that a single, and preferably two ribozymes inserted within the 3' UTR of the inverted transgene cassette enables full reversal of the effect of dsRNA sensing on LV virion component protein expression levels in harvest supernatants. Figure 46C not only demonstrates that both 1x and 2x ribozymes within the 3' UTR of the inverted transgene results in substantial amount of vRNA packaged into LVs but also that the VI is still 'cleanly' spliced-out from the vRNA, since a single RT-PCR band of the expected size is generated. The impact of the self-cleaving ribozymes was a ~100-fold increase in LV titres (Figure 47).

The 'Transgene Repression In vector Production' (TRiP) system was previously developed to stop unwanted impacts of transgene protein on vector output titre during LV production. This is achieved by co-expressing the bacterial protein TRAP and inserting its target sequence (tbs) close to the transgene Kozak sequence; the TRAP/tbs complex functions to block translation initiation. In employing this system, LV titres can be substantially rescued from the impacts of the transgene proteins they encode. In the present invention, the novel configuration of 'sense' encoded VI and the use of self-cleaving ribozymes within the 3'UTR of an inverted transgene cassette allows for very efficient transgene repression during LV production in a manner independent of protein-encoded factors. During production of the described VI LV genomes, the GFP expression was assessed in post-production cells by flow cytometry, and Expression Scores (ES) generated by multiplying the % GFP positive by the median fluorescence intensity (Arb units). Figure 47 also shows this data and indicates that the utilisation of these novel, inverted transgene VI LV genome cassettes, transgene expression is suppressed by over 1000-fold.

### EXAMPLE 21: Further truncation of minimal gag sequences within the packaging region in combination with Vector-Intron

Previous examples utilised the 'Δp17INS' feature wherein the p17 instability element within retained gag sequences had been removed (to leave ~80 nucleotides of gag), and showed that this modification does not impact LV titres of genomes harbouring a Vector-Intron. Here, further variants of an MSD-2KO LV genome harbouring VI_5.5 were generated wherein the retained gag sequence (as part of extended packaging signal) was further reduced to 57, 31, 14 and essentially zero gag sequence. These sequences (including Δp17INS/gag81) also harboured an ATG>ACG mutation in the primary initiation codon of gag - a further safety feature to eliminate any possible expression of gag peptide in transduced cells should vector backbone sequences be mobilised by transcription read-in from cellular promoters. Figure 48 displays the results of producing these vectors in suspension (serum-free) HEK293T cells, and indicate that retained gag sequences can be minimised to ~30 nucleotides without affecting output titres of Vector-Intron containing LV genome cassettes.

### EXAMPLE 22: Optimisation of Vector-Intron LV production in suspension (serum-free) HEK293T cells in the absence of rev by Design-of-Experiment

Previous examples demonstrated that the use of optimised VIs enabled production of RRE-deleted LVs in the absence of rev to within 60-100% of standard, RRE/rev-dependent LVs. These studies used the same plasmid ratios for both standard and VI LV production, except that pRev was not included with VI LV transfections (pBluescript was used to ensure total DNA transfected was the same in comparisons). Interestingly and surprisingly, when concentrated preparations of VI LV and standard vectors (both encoding a CMV-GFP transgene cassette) were assessed for VSVG and capsid (p24) levels by immunoblotting, the VI LV vector sample appeared to contain substantially higher VSVG and capsid protein compared to the standard LV sample (Figure 49A), despite both vector samples having the very similar integrating titres of 2.2 x 10⁷ and 1.7 x 10⁷ TU/mL respectively. This indicated that the plasmid ratios previously optimised for standard RRE/rev-dependent LVs may not be optimal for rev-independent, VI LVs. Given that for the standard RRE/rev-dependent LVs the pRev represents just 5% of the pDNA mix in the optimised ratio, this also suggested that rev has some direct/indirect impact on the expression levels of GagPol and VSVG, as well as rev-dependent LV genomic RNA. Without wishing to be bound by theory, it is conceivable that rev may have some low level suppressive effect on expression cassettes containing introns (as both pGagPol and pVSVG did in this work), and that in the absence of rev (i.e. during VI LV production) the GagPol/VSVG expression levels are greater compared to standard LVs, even though the same pGagPol/pVSVG input levels were used.

The inventors therefore sought to optimise plasmid input ratio levels of VI LV genome plasmid, pGagPol and pVSVG, as well as assessing optimal harvest time post-sodium butyrate induction using a multifactorial approach (Design-of-Experiment, DoE). Figure 49B displays the results of this optimisation experiment performed in suspension (serum-free) HEK293T cells using lipofectamine, where the 'centre point' for the analysis was the standard plasmid ratios used in previous examples (950 ng/mL pVl-Genome, 100 ng/mL pGagPol and 70ng/mL pVSVG). It was surprisingly found that the optimal ratios for VI-containing RRE/rev-independent LV genome production differed substantially compared to previously optimised ratios for standard RRE/rev-dependent LVs. The titres of VI LVs could be increased 2-to-3 fold, compared to the centre point, and therefore indicated that in previous examples, where VI LV titres appeared to be slightly lower (i.e. within 2-fold) than standard LVs, this was likely to be due to the use of non-optimal plasmid ratios rather than any deficiency of the VI LV genomes themselves.

Thus, by use of optimal plasmid ratios, Vector-Intron LV genomes can be made to equivalent output titres compared to standard LVs but have the advantage of: [1] 1kb increased capacity (RRE eliminated and gag reduced to ~30 nucleotides), [2] simplified production (no pRev plasmid required for optimisation/implementation) and [3] the benefits of the MSD-2KO mutation i.e. no aberrant splicing during production, and reduced read-in from upstream chromatin as demonstrated elsewhere.

### EXAMPLE 23: Use of a transgene mRNA-targeted siRNA in transient co-transfection to reduce transgene expression as well as recover titres of a vector containing an inverted transgene cassette.

Standard RRE/rev-dependent LV genome expression cassettes harbouring the EFS-GFP reporter were generated with transgene cassette in either the forward or inverted orientation. For the inverted transgene cassette a heterologous polyadenylation sequence was provided to enable efficient transcription termination (see Figure 50). Vector supernatants were produced by transfection of suspension (serum-free) HEK293T cells with the vector component plasmids and optionally with 34pmol/mL (final concentration in the culture media) of 'Dicer-Substrate Short Interfering RNAs' (DsiRNAs [27-mer]; IDT) directed to either eGFP (Transgene; IDT Cat# 51-01-05-07) or Luciferase (Control; IDT Cat # 51-01-08-22). A pTK-DsRed-Xprs plasmid was also spiked into the DNA mix as a surrogate marker for dsRNA sensing effects on *de novo* protein synthesis during LV production. Post-production cells were analysed by flow cytometry to assess the levels of GFP transgene expression, and the level of DsRed-Xprs. Clarified vector supernatants were titrated by transduction of adherent HEK293T cells followed by flow cytometry.

Figure 43 shows a simple experiment where a standard rev/RRE-dependent LV containing an inverted EFS-GFP-polyA cassette was produced in HEK293T cells with or without rev. Only when rev was present - and therefore full length vRNA could be transported to the cytoplasm where it could anneal to the transgene mRNA - was *de novo* expression of VSV-G/p24 suppressed, indicating that a cytoplasmic dsRNA response (e.g. PKR) was likely triggered.

The results displayed in Figure 52 show that surprisingly the GFP siRNA led to substantial reduction (100-fold) in transgene expression of the forward facing LV genome without severely impacting LV titres. Moreover, the titres of the inverted transgene LV genome were substantially reduced compared to the forward facing LV genome, and DsRed-Xprs levels were also lower (indicating some potential dsRNA sensing, leading to translation suppression). However, the use of the GFP siRNA recovered titres by 100-fold and DsRed-Xprs level were restored. Strikingly, the level of GFP expression in cells transfected with the inverted transgene LV genome plus the GFP siRNA were 10,000 fold lower than the forward-facing LV genome alone. This demonstrated the utility of microRNA to rescue titres of LV containing inverted transgene cassettes, as well as efficiently suppressing transgene expression during LV production.

### EXAMPLE 24: Further development of a 'supA-2pA-LTR'.

Following on from Example 7, a second round of constructs were generated in which the inverted RU5 sequence with or without the GU box (resulting from 5'R modification) was introduced upstream of the supA-2pA-LTR, to better model the efficiency of transcription read-in from cellular promoters downstream of an integrated LV cassette (i.e. bottom strand 'read-in'). Figure 54 displays the sequences tested within the reporter cassette assay, wherein luciferase reports on read-through activity. Similarly to Example 7, the data demonstrate that the presence of the two polyA signals within the SV40 early polyA sequence provided ~10-fold increased transcriptional termination over the 'no polyA' control and polyA mutated variants (pAm1) - see Figure 54, construct 2 versus 1 and 3. This demonstrated that the inverted RU5 sequence imparted no effect on polyadenylation/termination reading-in on the bottom strand since a similar effect was observed (relative to 'no polyA') in Figure 53. Interestingly, and surprisingly, the inclusion of the 3R-GU2 sequence within the inverted RU5 (i.e. modelling supA-LTR modifications on the top strand) slightly stimulated transcriptional termination on the bottom strand - see Figure 54, construct 4 versus 2. Constructs 2-5 were appended with the SV40 early polyA DSE sequence (containing a GU box) to generate constructs 6-9, and use to show that the use of a GU-rich DSE was able to further stimulate transcriptional termination on the bottom strand by 4-5 fold. Overall, this first demonstration of an augmented 'supA-2pA-LTR' - as modelled by construct 8 of Figure 54 - displayed ~75-fold reduced transcriptional read-in on the bottom strand, as compared to having no polyA signals at all (i.e. current SIN-LTRs). Note that these constructs also model the potential to use supA-2pA-LTRs as primary or 'back-up' polyadenylation/termination signals for inverted transgene cassettes to minimise 'read-out' through the 5'LTR into adjacent chromatin.

Figure 55 provides the detailed sequence information for supA-2pA-LTR variant 'GU1', which comprises the SV40 early polyA DSE sequence (containing a GU box) in the inverted orientation to 'service' the inverted polyA sequences (the same variant tested in Figure 54). Moreover, and to provide further clarity to the invention, Figure 55 [A] provides (as an example) the complete LV-supA-2pA-LTR expression cassette from the 5'R (i.e. no promoter sequence is shown) to the 'R-embedded' heterologous polyadenylation sequence (in this case the SV40 polyA), at the 3'end of the expression cassette. In this case, the 5'R modification with the 'GU2' variant DSE/GU box (as per Table 1) is shown. At the 3'end, the inverted SV40early polyA DSE/GU box is shown in context to the R-embedded SV40 polyA sequence. As per one of the principal aspects of the invention, the polyA signal (pAS) is positioned upstream of the 3'R sequence, with the R.1-20 sequence positioned upstream of both the cleavage zone and DSE/GU box. Elsewhere in the present disclosure it is shown that as few as 12 nucleotides of R in this position may be sufficient to maintain LV titres (i.e. 1^{st} strand transfer). Figure 55 [B] displays the resultant LTR formed by reverse transcription/cDNA formation on the LV sequences of Figure 55 [A] (only on LTR is shown because they will be identical, and flanking the integrated LV. This more clearly shows the consequences of providing the 5'R GU-box modification; namely, that this DSE/GU-box is positioned downstream of the top strand pAS, since the DSE/GU-box of the R-embedded heterologous polyA sequence is not converted to DNA. Thus, a fully bi-directional polyA sequence is provided with both top and bottom pAS's 'serviced' with DSE/GU boxes, and the top strand pAS also serviced with a USE.

### EXAMPLE 25: Optimisation of the inverted/bottom strand DSE/GU-box within the 'supA-2pA-LTR'.

Further optimisation of the supA-2pA-LTR was pursued by assessing different GU-boxes downstream of the inverted SV40 early polyadenylation sequence. Table 2 shows the core sequences generated for each construct created and tested, and provides them 5'-3' with respect to the 'bottom strand'. The alternative GU boxes were from diverse sources, including cellular (e.g. β-globin, MC4R), viral (e.g. HSV-TK) and synthetic sequences. Together, Figure 55, Table 9 and Table 10 show how these variants differ. These variants were inserted into the luciferase reporter cassette such that transcriptional read-through modelled 'bottom strand' read-in from cellular promoters. Thus, a complete LTR was present, with inverted RU5 (with or without the R-GU2 modification) and SINΔA3(att) flanking the SV40 early sequence and GU boxes being tested. Constructs were transfected into HEK293T suspension cells, and luciferase measured to assess the degree of transcriptional termination (Figure 56). These data confirmed previous results (Constructs 2 to 7), indicating that neither the inverted RU5 or GU2 sequences adversely impacted the SV40 early polyA termination activity, and that the extended SV40 early sequences harbouring a DSE/GU-box improved this activity. The presence of polyA signals were paramount to this activity. When the 'GU2' box (same as 'GU5' of the 5'R modification), was used downstream of the SV40 early sequence, this resulted in the greatest level of transcriptional termination measured in the experiment (Figure 56, construct 8). However, when this was paired with the inverted GU2-embedded RU5 sequence upstream (which would be required to improve polyadenylation on the opposite (top) strand), the benefit of having the GU2 box downstream of the SV40 early sequence was lost (construct 9 versus 1 and 8). It's likely that this was due to base pairing between the two GU-box sequences due to sequence similarity, perhaps impairing binding of DSE factors. Generally, the other GU box variants tested enabled improved transcriptional termination compared to the 'no GU box' controls (compare constructs 10-to-19 with 1 and 3). The best supA-2pA-LTR variants harboured GU1, GU5 or GU7, and enabled 50-to-100 fold improved termination over the control (e.g. construct 19 versus 1), and 10-fold over the inverted supA-LTR (e.g. construct 19 versus 2).

To assess the transcriptional termination performance of these new supA-2pA-LTRs in the forward-facing direction, these variants were flipped around within the luciferase reporter cassette (Figure 57). Each supA-2pA-LTR variant containing the alternative GU box downstream of the SV40 early polyA sequence, was generated in the context of an RU5 containing a mutated HIV-1 native polyA, with or without the 3GR-GU2 modification, which supplies the DSE/GU box to the SV40 late polyadenylation signal that is upstream of the R boundary. Constructs were transfected into HEK293T suspension cells (as before), and luciferase measured to assess the degree of transcriptional termination. The results show that all supA-2pA-LTRs outperformed the standard SIN-LTR, but that importantly, the variants displaying the best transcriptional termination activity on the bottom strand (in Figure 56) also produced the best results on the top strand. The best version of supA-2pA-LTR contained the GU7 sequence to service the bottom strand, in combination with the GU2 modification to the R region to service the top strand, transcriptional termination activity was increased by 65-fold on the top strand and by 95-fold on the bottom strand.

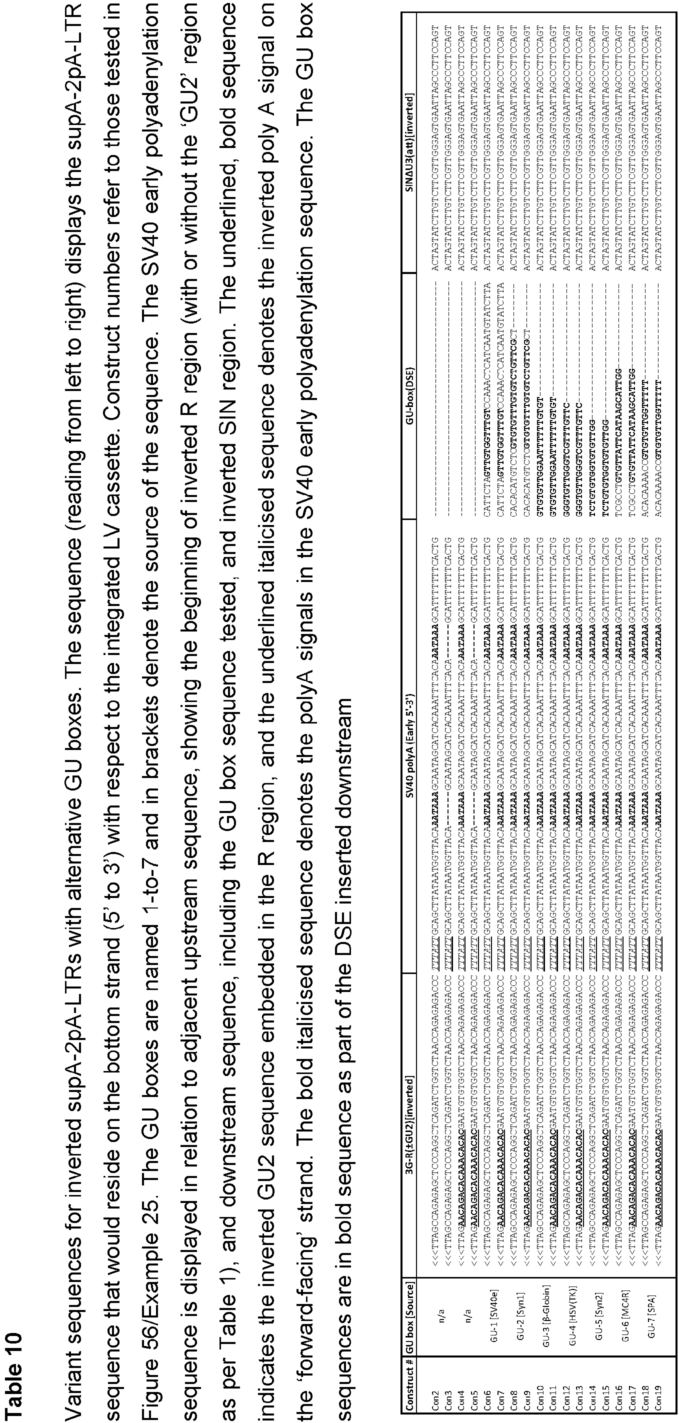

### EXAMPLE 26: Production titres for LVs bearing supA-2pA-LTRs.

Having demonstrated that the supA-2pA-LTRs could be optimised within the luciferase reporter assay, the supA-2pA-LTR variants harbouring the seven different inverted DSE/GU boxes were cloned into LV genome expression cassettes containing an EFS-GFP cassette. Figure 58 discloses both the structure of 5' and 3'LTRs in the production DNA expression cassette, as well as the final LTR structure that would be formed after integration (two LTRs flanking the integrated sequences). Variant LTR sequences were cloned into a major splice donor (MSD) mutated LV backbone, which eliminates aberrant splicing from the packaging region. The 5'R region of the DNA expression cassette contained the GU2 variant sequence to provide a DSE/GU box to the polyA signal on the top strand, and the R(1-20)-embedded SV40 polyA sequence was used at the 3' LTR position. SIN-LTR variants utilised an SV40 polyA sequence as a 'back-up', whereas the supA/supA-2pA-LTR variants did not (not shown). LV genome plasmids were used to make vector in serum-free suspension cells, and clarified harvest titrated on adherent HEK293T cells followed by flow cytometry to count GFP-positive cells, and passaged further for 10 days to harvest host cell DNA for integration assay. The data indicated that all the supA-2pA-LTR variants could be produced to practicable titres, similarly to the MSD-mutated LV harbouring the standard SIN-LTR, demonstrating that the sequences engineered into supA/supA-2pA-LTRs do not adversely affect output titres.

Further to this, the supA-2pA-LTR variant harbouring the inverted GU box from the SPA synthetic polyA sequence (GU7) was cloned into LV genome expression cassettes containing either EF1a or PGK promoter driven GFP cassettes, either in standard or MSD-mutated LVs (Figure 59). LV genome plasmids were used to make vector in serum-free suspension cells, and clarified harvest titrated on adherent HEK293T cells followed by flow cytometry to count GFP-positive cells, and passaged further for 10 days to harvest host cell DNA for integration assay. These data demonstrate that the sequences required to employ the supA/supA-2pA LTR modifications do not impact (and neither are impacted by) sequences internal to the lentiviral vector cassette, including the native 5'pA, MSD or internal promoter. Thus, the supA/supA-2pA-LTR may be applied to existing 3^{rd} generation LVs or to next generation LVs.

### EXAMPLE 27: Integrated LVs bearing supA-2pA-LTRs are subject to less transcriptional read-in through the 5'LTR.

Having demonstrated that the supA-2pA-LTRs greatly reduce transcriptional read-through on plasmid-bearing reporters (where transcriptional activity is very high), and that LVs bearing these upgraded polyA sequences could be produced to high titres, these vectors were used to transduce adherent HEK293T cells (immortalised) and primary donkey fibroblasts (92BR) at a target MOI of 1, followed by 10 days of passaging and integration assay (to obtain actual copies per cell), and then total RNA extracted to measure HIV-Psi RNA and GAPDH mRNA levels (the latter for normalisation). These results are displayed in Figure 60, which presents relative read-through for the integrated 5'LTR compared to a standard SIN-LTR. First, the MSD-mutation produced up to a 2.5-fold reduction in read-in (as the inventors have previously shown). Additionally, the supA-LTR variant further reduced read-in in HEK293T cells. Surprisingly, the majority of the supA-2pA-LTR variants provided the best reduction in transcriptional read-in, indicating that the recruitment of polyadenylation factors for termination on the bottom strand might aid with local termination on the top strand. These supA-2pA-LTR variants reduced transcriptional read-in by 8-to-33 fold compared to standard SIN-LTRs. These data also indicate that screening of supA/-2pA-LTR variants in actual integrated LVs may reveal the better variants compared to plasmid transfection (episomal cassette) reporter read-through assays.

### EXAMPLE 28: Integrated LVs bearing supA or supA2pA-LTRs are subject to orders of magnitude less transcriptional read-in from upstream of the 5'LTR.

To assess the degree of improvement in transcriptional insulation provided by the supA and supA-2pA LTRs over the standard SIN-LTRs with greater sensitivity, transcriptional read-in analysis of integrated LVs bearing supA or supA2pA-LTRs was measured by RNAseq. Seven LV genome constructs as outlined in Figure 61 were generated and used to produce LVs in suspension (serum-free) HEK293T cells. Two standard SIN-LTR bearing LVs were generated, one with an intact major splice donor and the other with the 2KOm5 mutation (i.e. it was a '2KO-LV', and was produced in the presence of a modified U1 snRNA able to bind to the packaging signal of the vRNA to restore titres - see WO 2021/160993). Four variants containing the supA-LTR (harbouring 3GR-GU2 5'modification, and SV40 late polyA USE-pAS in 3' LTR) were produced +/- 2KO5m mutation and +/- mutation of the native HIV-1 pA signal. A single supA-2pA-LTR variant was produced (as per supA-LTR but additionally with inverted SPA GU box [variant 'iGU7'] to service the two SV40 early pA signals in the USE region) with both the 2KOm5 and native HIV-1 pA signal mutations. All LVs had the identical EFS-GFP-wPRE expression cassette.

LVs were used to transduce adherent HEK293T cells at MOI of 1, and after a 10 day passage host cell genomic DNA extracted for integration assay to determine vector copy number (qPCR to HIV-Psi). PolyA-selected RNA was purified and subjected to RNAseq, assessing quantity of reads to the MSD-Psi region (MSD), the gag-Psi region (Ga) and the RRE region (RRE). Note that R-U5 reads were also measured but, since 3' R-U5 reads would also be included as transgene mRNA reads in the SIN-LTR constructs (at least reads to the polyA cleavage site) - and the fact that the supA(2pA)-LTRs have slightly different sequences in in these regions - the R-U5 data is not displayed. Details on RNA preparation, RNAseq and bioinformatics can be found in the materials and methods.

Figure 61 also displays the results of these analyses, and show the relative read-in to the 5'LTR of all constructs compared to the standard (MSD+)-LV-SIN vector for the 'MSD' read (set to 100%). It can be seen that read coverage diminishes from 5' to '3 (MSD-to-RRE) in the standard LV-SIN vector, with the number of reads to the RRE region being ~5-fold less compared to the MSD-Psi region; this is likely due to splicing from the MSD to a cryptic splice acceptor within the transgene cassette, thus elevating MSD reads over gag and RRE. This effect is seen in all LVs where the MSD is not mutated. The mutation of the MSD within the 2KO-LV genome resulted in a ~3-fold reduction in MSD reads, which is in agreement with other data that indicates that the MSD-mutation results in less read-in (WO 2021/160993). The supA-LTR variants provided further protection to transcriptional read-in to the MSD region by ~6-fold, and by ~122-fold when combined with MSD-mutation and native HIV-1 pA signal mutation. In some cases, the Gag and RRE regions generated no reads at all, but were generally reduced by ~36-fold to ~174-fold.

In summary, these data show that the supA/supA-2pA-LTRs provide substantial transcriptional insulation for integrated LVs, and represent a major step forward providing additional safety for LVs used as gene therapy vectors.

### EXAMPLE 29: Use of production cell-derived miRs to target the transgene mRNA derived from a Vector-Intron LV harbouring an inverted transgene cassette.

Other examples within the present invention demonstrate that ribozymes can be used as 'self-cleaving' elements with the 3'UTR of inverted transgene cassettes on-boarded to Vector-Intron LVs, in order to recover LV output titres. The lower output titre of these types of vectors containing inverted transgenes is likely due to the production of dsRNA, and resultant dsRNA-sensing pathways, for example leading to triggering of PKR. Thus, negation of dsRNA by degradation of transgene mRNA by use of self-cleaving 3'UTR elements block this potential signalling. Embedding such elements within the 3'UTR of the inverted transgene that is encompassed by the Vector-Intron on the top strand, ensures that packaged vRNA will not contain these self-cleaving elements. An alternative or additional type of element that can be used are target sequences for miRNAs expressed during LV production; these can either be endogenously expressed miRNAs by the host cell or by exogenously expressed miRNAs (e.g. by co-transfection of a U6-driven mi/shRNA cassette). This concept is described in Figure 62. The feasibility of this approach was carried out by producing Vector-Intron LVs (specifically, MSD-2KOm5/ΔRRE/Δp17INS + VI_v5.5) harbouring an inverted EF1a-GFP cassette, with endogenous miRNA target sequences (as 1x or 3x copies) within the 3'UTR. Two sets of variants were generated in which the ribozymes T3H38 and HDV_AG were optionally present (when present, the miRNA target sequences were between the two ribozymes). A third variant was generated (+ribozymes), wherein a single copy of each type of miRNA target sequence was present. LVs were produced in suspension (serum-free) HEK293T cells and GFP Expression scores generated (%GFP × MFI), and vector supernatants titrated on adherent HEK293T cells. These data are presented in Figure 63, and show that indeed endogenous miRNA target sequences can be used to rescue inverted transgene LV output titres, albeit to a lower extent than the ribozymes. Whilst the maximal recovery in output titres was observed with the ribozymes alone (i.e. miRNA paired with ribozymes offered no further boost to titres), this demonstrated that other elements of quite different sequence, structure and functional class could be used within the VI-encompassed portion of the inverted 3'UTR to boost titres.

### EXAMPLE 30: A side-by-side comparison of a standard LV, a '2KO-LV' and a 'MaxPax' LV (Vector-Intron-dependent) encoding a therapeutically relevant transgene: splicing profiles in production cells and virions, and output titres.

Example 30 demonstrates the following features of the present invention:;
[1] the use of the Vector-Intron to mitigate against the attenuating effect of the mutation of the major splice donor site in the packaging region,
[2] use of the Vector-Intron in combination with further deletions in the gag-Psi region increase the capacity of the LV vector, and
[3] the use of the MSD-mutation to enable both ablation of aberrant splicing from the packaging region, and allow clean splicing of the Vector-Intron,
a side-by-side comparison was performed with a transgene cassette encoding a chimeric antigen receptor (CAR) used for CAR-T therapy. For the purposes of ease of description, LVs containing the new architecture described above, were referred to as 'MaxPax' LVs. Accordingly, a standard LV and a '2KO-LVs' (harbouring the 2KOm5 mutation only) encoding an EF1a-CAR-wPRE transgene cassette were generated. In addition, a 2KO-LV and a MaxPax-LV were generated encoding an EFS-CAR-wPRE transgene; this is because MaxPax/Vector-Intron containing LV expression cassettes give rise to vRNA genomes that cannot retain introns encoded in the 5' to 3' direction, including the one present within the EF1a promoter (i.e. intron A). Therefore, MaxPax LVs are suited to gene therapy approaches wherein space may be at a premium, and cis-elements such as introns may be considered superfluous. It should be noted, that in instances where the retention of an intron within the transgene cassette is desirable, the inverted transgene approach (described elsewhere in the invention) may be taken, wherein the 'self-cleaving' elements may be utilised within the 3'UTR encompassed by the Vector-Intron sequence. This will depend on the activity of the transgene promoter, for example if a tissue-specific promoter is used that is not (very) active in production cells, then the self-cleaving elements may not be necessary since no/minimal dsRNA will be produced. In any case, the inverted transgene cassette encoded by the vRNA will be able to retain its intron(s) since it will not be functional in the vRNA 5' to 3' sense direction.

These LVs were produced in suspension (serum-free) HEK293T cells and post-production cells and resultant LV virions analysed by RT-PCR to assess full length vRNA and truncated vRNA production. These data are displayed in Figure 70 **[A],** together with the output titres of the produced LVs in Figure 70 **[B].** The results demonstrate that the MaxPax LVs (much like the 2KO LVs) are packaged as a single major species of vRNA, unlike the standard LVs that generate/package truncated forms. MaxPax LVs however, do not require either rev or the modified U1 snRNA to generate high titres, and also can on-board ~1kb additional space compared to the other vectors, since they lack the RRE (see 2KO-LV-EFS-CAR with MaxPax-EFS-CAR vRNA size difference in Figure 70 **[A]).**

### EXAMPLE 31: Transgene 3'UTRs bearing multiple numbers of CARe tiles optionally paired with a ZCCHC14 protein-binding sequence leads to increased transgene expression in transduced Jurkat cells.

To further exemplify the pairing of the ZCCHC14 protein-binding sequence (ZSL1) with multiple CARe tiles to generate novel minimal transgene expression enhancer sequences in the 3' UTR, several variants were constructed wherein a single (downstream) ZCCHC14 protein-binding sequence was paired with tandem arrays of the 10bp CARe sequence, ranging from a single copy up to 20 copies. These were inserted into an EFS-GFP expression cassette within a standard RRE/rev-dependent LV genome cassette and used to produce LVs in suspension (serum-free) HEK293T cells. Controls LVs containing wPRE, no cis-acting element (ΔwPRE), the 16 × 10bp CARe variant (CARe.16t) or a single ZCCHC14 protein-binding sequence were also produced. Clarified LV supernatants were titrated to generate integrating titres (TU/mL), on adherent HEK293T cells (Figure 65). Following this, fresh Jurkat cells were transduced at matched MOIs of 0.5 or 1, and cultures incubated for 3 days. GFP expression in transduced cells was measured by flow cytometry, and median fluorescent intensity values generated (Arbitrary units). These were normalised to the wPRE-containing control (set to 100%). These data are displayed in Figure 65, and show a similar pattern of expression in the T-cell line compared to that of the HEK293T cells in Figure 32. Having no 3'UTR element (ΔwPRE) had less impact on transgene expression in Jurkat cells, with transgene expression dropping to 40% compared to wPRE. However, similarly to HEK293T cells, there was an increase in transgene expression as more CARe tiles were used, particularly from 8x to 20x. Moreover, when at least 12 tiles were used, the level of transgene expression was greater than that of the wPRE control. In addition, when paired with the ZSL1 sequence, the highest transgene expression was observed with as few as 16x CARe tiles, and comparable expression to wPRE was achieve when ZSL1 was paired with just 8x CARe tiles. Thus, the CARe.8t or CAR.16t elements in combination with the single ZCCHC14 protein-binding sequence were ~150bp and ~230bp respectively, with each generating transgene expression of the same or 160% of that of the ~590bp wPRE control, respectively.

### EXAMPLE 32: Assessment of CARe consensus sequence variants as 16x tiles in combination with a ZCCHC14 protein-binding sequence on transgene expression in transduced Jurkat cells.

The CARe consensus sequence generated by Lei et al., 2013 - and tested in the present invention within transgene 3'UTRs - was engineered to reflect the variance within the consensus to generate alternative 'synthetic' tiles. These are shown in Figure 66, indicating how each variant differed within this semi-degenerate consensus sequence. Since it is shown in the present invention that 8x-to-16x copies of the CARe consensus tile produced increased transgene expression in a variety of different cell types/line, these synthetic variants were tandemised as 16x tiles and tested in combination with the HCMV.ZSL1 loop. Another variant was tested in which the CARe consensus 16x tile fragment was mutated in every other tile (i.e. alternate) to assess the impact of mutation across the CARe16t fragment (Cons/mut5). These were inserted into an EFS-GFP expression cassette within a standard RRE/rev-dependent LV genome cassette and used to produce LVs in suspension (serum-free) HEK293T cells. Controls LVs containing wPRE, no cis-acting element (ΔwPRE), the 16 × 10bp CARe variant (CARe.16t) or a single ZCCHC14 protein-binding sequence were also produced. Clarified LV supernatants were titrated to generate integrating titres (TU/mL), on adherent HEK293T cells (data not shown). Following this, fresh Jurkat cells were transduced at matched MOls of 1, and cultures incubated for 10 days. GFP expression in transduced cells was measured by flow cytometry, and median fluorescent intensity values generated (Arbitrary units). Host cell DNA was extracted and qPCR to HIV-1 Psi performed to generated vector-copy-number. Thus, Figure 10 displays transgene expression normalised to vector-copy-number (VCN). These data identity variants 4, 7, 10, and 12 has being able to boost transgene expression in the T-cell line, compared to no 3'UTR element, with variant 4 approaching similar expression levels compared to wPRE. The variant containing alternate mutated CARe tiles (cons/mut5) performed similarly to the CARe.16t consensus control without the ZSL1 loop (which produced slightly higher levels of expression than wPRE). Together, this data indicate that the CARe consensus tile is the most optimal 'version', and that each tile contributes to expression activity in a cumulative manner, since if only some consensus tiles were 'active' whilst others were acting as 'spacers', then the cons/mut5+ZSL1 variant would be expected to equally active as the CARe.16t+ZSL1 control. However, the cons/mut5 variant boosted expression to 2/3rds that of the CARe.16t+ZSL1 control, which is more inline with the activity of the CARe.8t+ZSL1 variant in Figure 65. In other words, only the 8 copies of the CARe consensus within the cons/mut5 variant were fully active. This also provides strong evidence that spacing nucleotides of up to 8 in length may be inserted between tandem CARe consensus tiles without affecting the total cumulative effect of the element on transgene expression. For this reason, and given that one aspect of the invention is to minimise sequence length for use in gene therapy vector transgene cassettes, the inventors did not pursue further variants containing spacers between CARe consensus tiles, as this would unnecessarily increase total element size.

Further CARe consensus variants were generated based on 'native' sequences as per Lei et al., 2013, namely, those found in c-Jun, HSPB3, IFNalpha and IFNbeta. These are displayed in Figure 11. These variants were tandemised into 16x CARe elements and paired with the HCMV/ZSL1 loop. These were inserted into an EFS-GFP expression cassette within a standard RRE/rev-dependent LV genome cassette and used to produce LVs in suspension (serum-free) HEK293T cells. The same set of experiments in Jurkat cells as described above was carried out, and data reported in Figure 67. In this case, variant 2 from c-Jun and variant 1 of IFNa1 provided similar levels of expression compared to wPRE.

### EXAMPLE 33: Use of CARe/ZSL1 variant as 3'UTR element within rAAV vectors.

To show the utility of the novel CARe/ZSL1 ('CAZL') composite element(s) in other viral vector systems, rAAV vector genomes were engineered to contain example CAZL elements, so that they could be compared 'empty' or cassettes using the wPRE, which is ~590 nts in length. The use of CAZL elements may be especially useful in rAAVs where the genome packaging size limit is a 'hard' one, at ~5kb. Figure 68 shows a schematic of these example rAAV vectors and of those generated for exemplification, with the CAZL elements tested ranging in size: 141 nt (CARe.4tZSL1), 181 nt (CARe.8t/ZSL1) and 261 nt (CARe.16t/ZSL1). Note that these variants contain 'stuffer' sequence between the tandemised CARe tiles and the ZSL1 sequence due to cloning sites, and thus may in practise be reduced further in size. Additional controls included the inverted CAZL element for each type to control both for rAAV genome size and for CAZL functionality, since the inverted elements are not expected to be functional. Two sets of the rAAVs were generated, wherein the GFP transgene was driven by the CMV or EFS promoters. rAAVs were produced by transient transfection of HEK293T cells, followed by qPCR titration (see details in Figure 69 and Materials and Methods). HEPG2 cells were transduced with the different rAAV vector stocks at matched MOls (250 and 500; see details in Figure 69 and Materials and Methods). Transgene expression data is presented in Figure 69 and demonstrates that the CAZL elements were able to increase transgene expression in HEPG2 cells compared to the empty control, and for the CMV-GFP cassette, the CARe.8t/ZSL1 and CARe.16t/ZSL1 CAZL variants enabled ~2-fold greater expression than the wPRE, despite being less than half the size of wPRE. Some variance was observed when employing different promoters, with the CARe.4t/ZSL1 increasing expression when using the EFS promoter to similar levels as the other two CAZL variants. Importantly, the inverted CAZL variants expressed transgene at similar levels as the empty control in all cases, demonstrating that orientation of the CAZL elements in the forward sense is necessary (i.e. these are functional sequences) and that the observed improved expression could not be explained by merely the increase in vector genome size or transgene cassette size relative to the empty control.

These data demonstrate that these novel, short CARe-ZSL1 composite elements function as 3'UTR transgene expression enhancers within rAAV vectors where cargo space is limited, and that expression may be boosted to similar or greater levels as observed for wPRE-containing rAAVs.

Sequences tested in rAAVs. CARe 10nt consensus tandomized tiles in bold. ZSL1 sequence underlined. *These are non-limiting examples, and it is recognised that intervening sequence between tandemised CARe tiles and the ZSL1 may be deleted, or reduced in length to accommodate different restriction enzyme sites.*
CARe.4t/ZL1 (SEQ ID NO: 240): 
CCAGTTCCTGCCAGTTCCTGCCAGTTCCTGCCAGTTCCTGACTAGGTACCTCGAGCGG
ATCCCATCGATTGCCGTCGCCACCGCGTTATCCGTTCCTCGTAGGCTGGTCCTGGGGA
ACGGGTCGGCGGCCGGTCGGCTTCT
CARe.8t/ZL1 (SEQ ID NO: 241): 
CCAGTTCCTGCCAGTTCCTGCCAGTTCCTGCCAGTTCCTGCCAGTTCCTGCCAGTTCC
TGCCAGTTCCTGCCAGTTCCTGACTAGGTACCTCGAGCGGATCCCATCGATTGCCGTC
GCCACCGCGTTATCCGTTCCTCGTAGGCTGGTCCTGGGGAACGGGTCGGCGGCCGGT
CGGCTTCT
CARe.16t/ZL1 (SEQ ID NO: 242):

CCAGTTCCTGCCAGTTCCTGCCAGTTCCTGCCAGTTCCTGCCAGTTCCTGCCAGTTCC
TGCCAGTTCCTGCCAGTTCCTGCCAGTTCCTGCCAGTTCCTGCCAGTTCCTGCCAGTT
CCTGCCAGTTCCTGCCAGTTCCTGCCAGTTCCTGCCAGTTCCTGACTAGGTACCTCGA
GCGGATCCCATCGATTGCCGTCGCCACCGCGTTATCCGTTCCTCGTAGGCTGGTCCTG
GGGAACGGGTCGGCGGCCGGTCGGCTTCT

### EXAMPLE 34: Combining the CARe/ZSL1 3'UTR element with supA-LTRs within RRE-dependent or Vector-Intron-dependent LVs - production titres.

Three aspects of the invention, namely, the Vector-Intron, supA(2pA)-LTRs, and the 3'UTR CARe/ZSL1 element, were tested in part or in full within lentiviral vector backbones. The structures of these LV DNA expression cassettes are presented in Figures 71-73, and tested these cis-acting elements either alone or in combination within standard LVs, or within LVs harbouring the 2KOm5 mutation within the major splice donor (see PCT WO 2021/160993). Of the 2KOm5 mutants, the two members of this family are the '2KO-LVs' and so-called 'MaxPax' LVs. 2KO-LVs contain the rev-response element and require both rev and a modified U1 snRNA molecule (see WO 2021/160993), in order to generate high LV output titres. MaxPax LVs - which but do not require the modified U1 snRNA for production - contain the Vector-Intron in place of RRE (but this is not counted in transgene 'space'), and are also truncated in gag-Psi (to just ~30 nts), liberating ~ 1kb of additional transgene space. Further, MaxPax LVs can be used with the CARe/ZSL1 element to replace the large wPRE, thus increasing transgene capacity by a total of ~1.5kb.

Accordingly, standard LVs, 2KO-LVs and MaxPax (Vector-Intron) LVs were constructed as detailed above and in Figures 71-73, and used to generate vector material by transfection of suspension (serum-free) HEK293T cells, followed by titration on adherent HEK293Ts by flow cytometry and integration assay - these output titres are also displayed in Figures 71-73. (Note that the 2KO-LVs were produced in co-transfection with a plasmid expressing 256U1). These results demonstrate that all vector types, containing different combinations of the aforementioned novel cis-acting elements, were capable of being produced at high titre, between 1×10⁷ and 1×10⁸ TU/mL.

### EXAMPLE 35: Combining the CARe/ZSL1 3'UTR element with supA-LTRs within RRE-dependent or Vector-Intron-dependent LVs - transcription read-through integrated LTR.

The vector preparations from Example 34 were used to transduce adherent HEK293T cells at an MOI of 1, followed by analysis of transcriptional read-in to the 5' LTR, in cultures 10 days post-transfection to ensure that only measures from integrated LV cassettes were being carried out. Total RNA was extracted and both HIV-Psi RNA and GAPDH mRNA signal measured by RT-qPCR. This gave a ratio of transcriptional read-in per 'culture' but these values were then normalised to actual vector copy number, and presented as relative values compared to the LV harbouring the wPRE/SIN-LTR combination (i.e. the standard configuration) for each internal promoter-GOI cassette variant. These data, as well as the final configuration of the integrated LV in each case, is displayed in Figures 74-76.

These results very clearly demonstrate that the novel supA(2pA)-LTR technology provides substantial protection from transcriptional read-in to the integrated LV cassettes, and this is universally true irrespective of sequences internal to the integrated LTRs, and also in a manner that is not dependent on the native HIV-1 polyA signal present in current standard LVs.

### EXAMPLE 36: Combining the CARe/ZSL1 3'UTR element with supA-LTRs within RRE-dependent or Vector-Intron-dependent LVs - transgene expression.

As part of the same experiment, described in Examples 34 and 35, the GFP expression within the transduced cells was evaluated by flow cytometry to generate a GFP Expression score (%GFP-positive cells × median fluorescence intensity). This score was divided by the vector copy number per cell (as determined in Example 35) and compared to the wPRE/SIN-LTR variant for each LV containing a different internal promoter (set to 100). These data are presented in Figures 77-79 and show that in adherent HEK293T cells, the supA(2pA)-LTRs provide increased expression by 116% to 150% compared to the wPRE/SIN-LTR control. Moreover, the use of supA(2pA)-LTRs increased transgene expression from the integrated LV irrespectively of vector backbone, internal promoter or 3'UTR element, including the CARe/ZSL1 element, which itself increased expression over the 'no element' control by ~2-fold. The use of supA(2pA)-LTRs together with the CARe/ZSL1 element attained ~50% of the transgene level of wPRE, despite the CARe/ZSL1 element being less than 40% of the size of the wPRE ('no element' was ~20% of wPRE).

To summarise Examples 71-73, the data show that the different aspects of the invention can be combined to powerful effect in generating a new generation of LVs with increased transgene capacity (up to 1.5kb), increased transgene expression (supA-2pA-LTRs and/or CARe/ZSL1) and improved safety, as demonstrated by reduced transcriptional read-in from upstream chromatin (supA(2pA)-LTR).

### EXAMPLE 37: Combining the CARe/ZSL1 3'UTR element within the inverted transgene 3' UTR within Vector-Intron-dependent LVs boosts ribozyme-mediated rescue of output titres.

Previous examples of the present invention demonstrated that the Vector-Intron configuration/concept could be further developed for inverted transgene payloads. This is desirable when using Vector-Intron LV genomes because no other introns can be retained within the vRNA (i.e. top strand transcription product). By inverting the transgene cassette, introns may be retained. However, due to the production of dsRNA (vRNA base-pairing with transgene mRNA), dsRNA-sensing pathways (such as PKR) are likely triggered during LV production, leading to substantially reduced titres. The concept of inserting self-cleaving and/or instability sequences within the 3'UTR of the transgene (in the section that is encompassed by the VI on the top strand) was demonstrated by use of ribozymes. The effect of self-cleaving ribozymes is to degrade the transgene mRNA, and thus no dsRNA is produced, and LV titres are recovered to a certain extent. These self-cleaving/instability sequences are removed from the final packaged vRNA, and so are not present in the target cell. The other advantage of this approach is that transgene expression is substantially suppressed during LV production, negating any potential negative effects of the transgene product on the production cell and/or LV output titres.

To further develop this approach, the CARe.ZSL1 3'UTR element was employed within these Vector-Intron LV genomes containing functionalised 3' UTRs, as per Figure 80. LVs were constructed harbouring an inverted EF1a-GFP cassette, +/- the ribozymes T3H38 and HDV_AG (residing within the sequence encompassed by the VI on the top strand), and +/- the CARe.ZSL1 3'UTR element, positioned outside the sequence encompassed by the VI on the top strand (in this case between the inverted polyA sequence and the VI). LVs were produced in suspension (serum-free) HEK293T cells and GFP Expression scores (%GFP × MFI) generated in end-of-production cells to measure transgene suppression. LVs were titrated by GFP/flow cytometry and integration assay of transduced adherent HEK293T cells. These data are displayed in Figure 81, and show that surprisingly, the presence of the CARe.8t/ZSL1 element rescued LV titres to within 2.5-fold of the standard LV (containing the EF1a-GFP-wPRE cassette in forward orientation). Moreover, the GFP expression within production cells was still suppressed to very low levels (over 3 orders of magnitude) compared to the standard LV production cells.

Various preferred features and embodiments of the present invention will now be described with reference to the following numbered paragraphs Set A:
1. A nucleotide sequence encoding a lentiviral vector genome or comprising a lentiviral genome expression cassette, wherein the 3' LTR of the lentiviral vector genome comprises a modified polyadenylation sequence, and wherein the modified polyadenylation sequence comprises a polyadenylation signal which is 5' of the 3' LTR R region.
2. The nucleotide sequence of paragraph 1, wherein the native 3' polyadenylation sequence has been mutated or deleted.
3. The nucleotide sequence of paragraph 2, wherein the native 3' polyadenylation signal has been mutated or deleted.
4. The nucleotide sequence of any one of paragraphs 1 to 3, wherein the modified polyadenylation sequence is a heterologous polyadenylation sequence.
5. The nucleotide sequence of any one of paragraphs 1 to 4, wherein the modified polyadenylation sequence comprises a heterologous polyadenylation signal.
6. The nucleotide sequence of any one of paragraphs 1 to 5, wherein the polyadenylation signal has the sequence AATAAA.
7. The nucleotide sequence of any one of paragraphs 1 to 6, wherein the modified polyadenylation sequence is a strong polyadenylation sequence.
8. The nucleotide sequence of any one of claims 1 to 7, wherein the modified polyadenylation sequence further comprises a polyadenylation upstream enhancer element (USE), preferably wherein the USE is 5' of the polyadenylation signal.
9. The nucleotide sequence of any one of claims 1 to 8, wherein the modified polyadenylation sequence comprises a GU rich downstream enhancer element, preferably wherein the GU rich downstream enhancer element is 3' of the polyadenylation signal.
10. The nucleotide sequence of claim 8 or claim 9, wherein the enhancer element is a strong enhancer element.
11. The nucleotide sequence of any one of claims 1 to 10, wherein the polyadenylation signal which is 5' of the 3' LTR R region is in the sense strand.
12. The nucleotide sequence of claim 11, wherein the 3' LTR further comprises a polyadenylation signal in the antisense strand which is (i) 5' of the polyadenylation signal in the sense strand and (ii) 3' of the attachment sequence of the 3' LTR.
13. The nucleotide sequence of any one of paragraphs 1 to 12, wherein the modified polyadenylation sequence is a sequence derived from Simian Virus 40 (SV40), a rabbit beta-globin gene, a human growth hormone gene, or a bovine growth hormone gene.
14. The nucleotide sequence of any one of paragraphs 1 to 13, wherein the modified polyadenylation sequence is a synthetic sequence.
15. The nucleotide sequence of any one of paragraphs 1 to 14, wherein the 3' LTR R region of the modified polyadenylation sequence is a minimal R region.
16. The nucleotide sequence of paragraph 15, wherein the minimal R region is:
   (a) at least 10 nucleotides;
   (b) at least 12 nucleotides;
   (c) at least 14 nucleotides;
   (d) at least 16 nucleotides;
   (e) at least 18 nucleotides; or
   (f) at least 20 nucleotides,
   in length.
17. The nucleotide sequence of paragraph 15 or paragraph 16, wherein the minimal R region comprises a sequence derived from a native R region.
18. The nucleotide sequence of any one of paragraphs 1 to 17, wherein the 3' LTR R region of the modified polyadenylation sequence is homologous to the native 5' LTR R region of the lentiviral vector genome.
19. The nucleotide sequence of any one of paragraphs 1 to 18, wherein the 3' LTR R region of the modified polyadenylation sequence is 0-6 nucleotides downstream of the 3' polyadenylation signal.
20. The nucleotide sequence of any one of paragraphs 1 to 19, wherein the 3' LTR R region of the modified polyadenylation sequence is immediately downstream of the 3' polyadenylation signal.
21. The nucleotide sequence of any one of paragraphs 1 to 20, wherein the modified polyadenylation sequence further comprises a polyadenylation cleavage site.
22. The nucleotide sequence of paragraph 21, wherein the polyadenylation cleavage site comprises at least one CA dinucleotide motif.
23. The nucleotide sequence of paragraph 21 or paragraph 22, wherein the polyadenylation cleavage site is 3' of the polyadenylation signal.
24. A nucleotide sequence encoding a lentiviral vector genome or comprising a lentiviral genome expression cassette, wherein the lentiviral vector genome comprises a modified 5' LTR, and wherein the R region of the modified 5' LTR comprises a polyadenylation downstream enhancer element (DSE).
25. The nucleotide sequence of paragraph 24, wherein the first 55 nucleotides of the modified 5' LTR comprises the polyadenylation DSE, preferably wherein the first 40 nucleotides of the modified 5' LTR comprises the at least one polyadenylation DSE.
26. The nucleotide sequence of paragraph 24 or paragraph 25, wherein the polyadenylation DSE is comprised within a stem loop structure of the modified 5' LTR.
27. The nucleotide sequence of any one of paragraphs 24 to 26, wherein the polyadenylation DSE is comprised within loop 1 of the modified 5' LTR.
28. The nucleotide sequence of any one of paragraphs 24 to 27, wherein the polyadenylation DSE is a GU-rich sequence.
29. The nucleotide sequence of paragraph 28, wherein the GU-rich sequence is derived from RSV or MMTV, or wherein the GU-rich sequence is synthetic.
30. The nucleotide sequence of any one of paragraphs 24 to 29, wherein the native polyadenylation signal of the modified 5' LTR has been mutated or deleted.
31. The nucleotide sequence of any one of paragraphs 24 to 30, wherein the modified 5' LTR does not comprise a native polyadenylation signal.
32. The nucleotide sequence of any one of paragraphs 24 to 31, wherein the 5' R region of the modified 5' LTR further comprises a polyadenylation cleavage site.
33. A nucleotide sequence encoding a lentiviral vector genome or comprising a lentiviral genome expression cassette, wherein the 3' LTR of the lentiviral vector genome is a modified 3' LTR as defined in any one of paragraphs 1 to 23 and the 5' LTR of the lentiviral vector genome is a modified 5' LTR as defined in any one of paragraphs 24 to 32.
34. The nucleotide sequence of paragraph 34, wherein the R region of the modified 5' LTR is homologous to the R region of the modified 3' LTR and wherein the R region of the modified 3' LTR is immediately downstream of the 3' polyadenylation signal within the modified 3' LTR.
35. The nucleotide sequence of paragraph 33 or paragraph 34, wherein the R region of the modified 5' LTR is identical to the R region of the modified 3' LTR and wherein the R region of the modified 3' LTR is immediately downstream of the 3' polyadenylation signal within the modified 3' LTR.
36. A nucleotide sequence comprising a lentiviral vector genome, wherein the lentiviral vector genome comprises a modified 3' LTR and a modified 5' LTR, wherein the modified 3' LTR comprises a modified polyadenylation sequence comprising a polyadenylation signal which is 5' of the R region within the 3'LTR and wherein the R region within the 3' LTR comprises a polyadenylation DSE, and wherein the modified 5' LTR comprises a modified polyadenylation sequence comprising a polyadenylation signal which is 5' of the R region within the 5'LTR and wherein the R region within the 5' LTR comprises a polyadenylation DSE.
37. The nucleotide sequence of paragraph 36, wherein the native 3' LTR polyadenylation sequence has been mutated or deleted.
38. The nucleotide sequence of paragraph 37, wherein the native 3' LTR polyadenylation signal and/or the native 5' LTR polyadenylation signal has been mutated or deleted.
39. The nucleotide sequence of any one of paragraphs 36 to 38, wherein the modified polyadenylation sequence is a heterologous polyadenylation sequence.
40. The nucleotide sequence of any one of paragraphs 36 to 39, wherein the modified 3' LTR and the modified 5' LTR are identical.
41. The nucleotide sequence of any one of paragraphs 1 to 40, wherein the major splice donor site in the lentiviral vector genome is inactivated, and optionally wherein the cryptic splice donor site 3' to the major splice donor site is inactivated.
42. The nucleotide sequence of paragraph 41, wherein the inactivated major splice donor site has the sequence set forth in SEQ ID NO: 4.
43. The nucleotide sequence of any one of paragraphs 1 to 42, wherein the lentiviral vector genome further comprises a tryptophan RNA-binding attenuation protein (TRAP) binding site.
44. The nucleotide sequence of any one of paragraphs 1 to 43, wherein the nucleotide sequence further comprises a nucleotide sequence encoding a modified U1 snRNA, wherein said modified U1 snRNA has been modified to bind to a nucleotide sequence within the packaging region of the lentiviral vector genome.
45. The nucleotide sequence of paragraph 44, wherein the nucleotide sequence encoding the lentiviral vector genome is operably linked to the nucleotide sequence encoding the modified U1 snRNA.
46. The nucleotide sequence according to any one of the preceding paragraphs, wherein the lentiviral vector genome comprises at least one modified viral cis-acting sequence, wherein at least one internal open reading frame (ORF) in the viral cis-acting sequence is disrupted.
47. The nucleotide sequence according to paragraph 46, wherein the at least one viral *cis-*acting sequence is a Woodchuck hepatitis virus (WHV) post-transcriptional regulatory element (WPRE) and/or a Rev response element (RRE).
48. The nucleotide sequence according to any one of the preceding paragraphs, wherein the lentiviral vector genome comprises a modified nucleotide sequence encoding gag, and wherein at least one internal open reading frame (ORF) in the modified nucleotide sequence encoding gag is disrupted.
49. The nucleotide sequence according to any one of paragraphs 46 to 48, wherein the at least one internal ORF is disrupted by mutating at least one ATG sequence within the nucleotide sequence, preferably wherein the first ATG sequence within the nucleotide sequence is mutated.
50. The nucleotide sequence according to any one of the preceding paragraphs, wherein the lentiviral vector genome lacks (i) a nucleotide sequence encoding Gag-p17 or (ii) a fragment of a nucleotide sequence encoding Gag-p17.
51. The nucleotide sequence according to paragraph 50, wherein the fragment of a nucleotide sequence encoding Gag-p17 comprises a nucleotide sequence encoding p17 instability element.
52. The nucleotide sequence according to any one of the preceding paragraphs, wherein the nucleotide sequence comprising a lentiviral vector genome does not express Gag-p17 or a fragment thereof.
53. The nucleotide sequence according to paragraph 52, wherein said fragment of Gag-p17 comprises the p17 instability element.
54. A lentiviral vector genome encoded by the nucleotide sequence of any one of paragraphs 1 to 35 or 41 to 53.
55. A lentiviral vector genome as defined in any one of paragraphs 1 to 54.
56. An expression cassette comprising a nucleotide sequence according to any one of paragraphs 1 to 53.
57. A viral vector production system comprising a set of nucleotide sequences, wherein the nucleotide sequences encode vector components including gag-pol, env, optionally rev, and the nucleotide sequence of any one of paragraphs 1 to 53.
58. A cell comprising the nucleotide sequence of any one of paragraphs 1 to 53, the expression cassette of paragraph 56, or the vector production system of paragraph 57.
59. A cell for producing lentiviral vectors comprising:
   (a)
      a. nucleotide sequences encoding vector components including gag-pol and env, and optionally rev, and the nucleotide sequence encoding the RNA genome of the lentiviral vector of any one of paragraphs 1 to 53 or the expression cassette of paragraph 56; or
      b. the viral vector production system of paragraph 57; and
   (b) optionally, a nucleotide sequence encoding a modified U1 snRNA and/or optionally a nucleotide sequence encoding TRAP.
60. The cell of paragraph 58 or paragraph 59, wherein the splicing activity from the major splice donor site and/or splice donor region of the RNA genome of the lentiviral vector is suppressed or ablated.
61. The cell of paragraph 60, wherein the splicing activity from the major splice donor site and/or splice donor region of the RNA genome of the lentiviral vector is suppressed or ablated during lentiviral vector production.
62. A method for producing a lentiviral vector, comprising the steps of:
   (a) introducing:
      a. nucleotide sequences encoding vector components including gag-pol and env, and optionally rev, and a nucleotide sequence encoding the RNA genome of the lentiviral vector of any one of paragraphs 1 to 53 or the expression cassette of paragraph 56; or
      b. the viral vector production system of paragraph 57, into a cell; and
   (b) optionally selecting for a cell that comprises nucleotide sequences encoding vector components and the RNA genome of the lentiviral vector; and
   (c) culturing the cell under conditions suitable for the production of the lentiviral vector.
63. A lentiviral vector produced by the method of paragraph 62.
64. The lentiviral vector of paragraph 63, wherein the lentiviral vector comprises the RNA genome of the lentiviral vector as defined in any one of paragraphs 1 to 53.
65. Use of the nucleotide sequence encoding the RNA genome of the lentiviral vector of any of paragraphs 1 to 53, the expression cassette of paragraph 56, the viral vector production system of paragraph 57, or the cell of paragraph 58 or paragraph 59, for producing a lentiviral vector.
66. A lentiviral vector comprising the lentiviral vector genome of paragraph 54 or paragraph 55.

Various preferred features and embodiments of the present invention will now be described with reference to the following numbered paragraphs Set B:
1. A nucleotide sequence comprising a transgene expression cassette, wherein the 3' UTR of the transgene expression cassette comprises at least one cis-acting sequence selected from:
   a) a *cis*-acting Cytoplasmic Accumulation Region (CAR) sequence, comprising at least one CAR element (CARe) sequence; and/or
   b) a *cis*-acting ZCCHC14 protein-binding sequence, comprising at least one CNGGN-type pentaloop sequence, wherein the cis-acting ZCCHC14 protein-binding sequence does not comprise a full length post-transcriptional regulatory element (PRE) α element and does not comprise a full length PRE γ element.
2. The nucleotide sequence of paragraph 1, wherein the CAR sequence comprises a plurality of CARe sequences.
3. The nucleotide sequence of paragraph 1 or paragraph 2, wherein the plurality of CARe sequences are in tandem.
4. The nucleotide sequence of paragraph 2 or 3, wherein the CAR sequence comprises at least two, at least four, at least six, at least eight, at least ten, at least twelve, at least fourteen, at least sixteen, at least eighteen, or at least twenty CARe sequences, optionally wherein the CARe sequences are in tandem.
5. The nucleotide sequence of paragraph 4, wherein the CAR sequence comprises at least six CARe sequences in tandem, or at least ten CARe sequences in tandem.
6. The nucleotide sequence of paragraph 5, wherein the CAR sequence comprises at least eight CARe sequences in tandem, at least twelve CARe sequences in tandem, or at least sixteen CARe sequences in tandem.
7. The nucleotide sequence of any one of the preceding paragraphs, wherein the CARe nucleotide sequence is BMWGHWSSWS (SEQ ID NO: 92) or BMWRHWSSWS (SEQ ID NO: 243).
8. The nucleotide sequence of paragraph 7, wherein the CARe nucleotide sequence is CMAGHWSSTG (SEQ ID NO: 96).
9. The nucleotide sequence of paragraph 7, wherein the CARe nucleotide sequence is selected from the group consisting of: CCAGTTCCTG (SEQ ID NO: 97), CCAGATCCTG (SEQ ID NO: 97), CCAGTTCCTC (SEQ ID NO: 99), TCAGATCCTG (SEQ ID NO: 100), CCAGATGGTG (SEQ ID NO: 101), CCAGTTCCAG (SEQ ID NO: 102), CCAGCAGCTG (SEQ ID NO: 103), CAAGCTCCTG (SEQ ID NO: 104), CAAGATCCTG (SEQ ID NO: 105), CCTGAACCTG (SEQ ID NO: 106), CAAGAACGTG (SEQ ID NO: 107), TCAGTTCCTG (SEQ ID NO: 227), GCAGTTCCTG (SEQ ID NO: 228), CAAGTTCCTG (SEQ ID NO: 229), CCTGTTCCTG (SEQ ID NO: 230), CCTGCTCCTG (SEQ ID NO: 231), CCTGTACCTG (SEQ ID NO:232), CCTGTTGCTG (SEQ ID NO: 233), CCTGTTCGTG (SEQ ID NO: 234), CCTGTTCCAG (SEQ ID NO: 235), CCTGTTCCTC (SEQ ID NO: 236), CCAATTCCTG (SEQ ID NO: 237) and GAAGCTCCTG (SEQ ID NO: 238); optionally wherein the CARe nucleotide sequence is selected from the group consisting of: CCAGTTCCTG (SEQ ID NO: 97), CCTGTTCCTG (SEQ ID NO: 230), CCTGTACCTG (SEQ ID NO: 232), CCTGTTCCAG (SEQ ID NO: 235), CCAATTCCTG (SEQ ID NO: 237), CCTGAACCTG (SEQ ID NO: 106), CCAGTTCCTC (SEQ ID NO: 99) and CCAGTTCCAG (SEQ ID NO: 102).
10. The nucleotide sequence of paragraph 9, wherein the CARe nucleotide sequence is CCAGTTCCTG (SEQ ID NO: 97).
11. The nucleotide sequence of any one of the preceding paragraphs, wherein the 3' UTR of the transgene expression cassette does not comprise additional post-transcriptional regulatory elements (PREs).
12. The nucleotide sequence of any one of paragraphs 1 to 10, wherein the 3' UTR of the transgene expression cassette comprises at least one additional post-transcriptional regulatory element (PRE).
13. The nucleotide sequence of paragraph 12, wherein the additional PRE is a Woodchuck hepatitis virus PRE (wPRE).
14. The nucleotide sequence of any one of the preceding paragraphs, wherein the *cis-*acting ZCCHC14 protein-binding sequence is a PRE α element fragment.
15. The nucleotide sequence of paragraph 14, wherein the cis-acting ZCCHC14 protein-binding sequence is:
   a) a fragment of a HBV PRE α element; or
   b) a fragment of HCMV RNA 2.7; or
   c) a fragment of a wPRE α element.
16. The nucleotide sequence of paragraph 14 or 15, wherein the PRE α element fragment is no more than 200 nucleotides in length.
17. The nucleotide sequence of paragraph 16, wherein the PRE α element fragment is no more than 90 nucleotides in length.
18. The nucleotide sequence of any one of the preceding paragraphs, wherein the CNGGN-type pentaloop sequence is comprised within a stem-loop structure having a sequence selected from the group consisting of:
   (i) TCCTCGTAGGCTGGTCCTGGGGA (SEQ ID NO: 108); and
   (ii) GCCCGCTGCTGGACAGGGGC (SEQ ID NO: 109).
19. The nucleotide sequence of any one of the preceding paragraphs, wherein the CNGGN-type pentaloop sequence is comprised within a heterologous stem-loop structure.
20. The nucleotide sequence of paragraph 18 or 19, wherein the ZCCHC14 protein-binding sequence comprises a sequence selected from the group consisting of:
   (i) 
TGCCGTCGCCACCGCGTTATCCGTTCCTCGTAGGCTGGTCCTGGGGAACGGGT
CGGCGGCCGGTCGGCTTCT (SEQ ID NO: 110);
 and
   (ii) 
CTATTGCCACGGCGGAACTCATCGCCGCCTGCCTTGCCCGCTGCTGGACAGGG
GCTCGGCTGTTGGGCACTGACAATTCCGTGGTGTTGT (SEQ ID NO: 111).
21. The nucleotide sequence of any one of the preceding paragraphs, wherein the 3' UTR of the transgene expression cassette comprises a *cis*-acting CAR sequence and a *cis*-acting ZCCHC14 protein-binding sequence, optionally wherein the ZCCHC14 protein-binding sequence is located 3' to the CAR sequence.
22. The nucleotide sequence of any one of the preceding paragraphs, wherein the 3' UTR of the transgene expression cassette comprises at least two spatially distinct *cis*-acting CAR sequences and/or at least two spatially distinct *cis*-acting ZCCHC14 protein-binding sequences.
23. The nucleotide sequence of any one of the preceding paragraphs, wherein the 3' UTR of the transgene expression cassette further comprises a polyA sequence located 3' to the *cis*-acting CAR sequence and/or *cis*-acting ZCCHC14 protein-binding sequence.
24. The nucleotide sequence of any one of the preceding paragraphs, wherein the transgene expression cassette further comprises a promoter operably linked to the transgene.
25. The nucleotide sequence of paragraph 24, wherein the promoter lacks its native intron, optionally wherein the promoter is selected from the group consisting of: an EFS promoter, a PGK promoter, and a UBCs promoter.
26. The nucleotide sequence of paragraph 24, wherein the promoter comprises an intron, optionally wherein the promoter is selected from the group consisting of: an EF1a promoter and a UBC promoter.
27. The nucleotide sequence of any one of the preceding paragraphs, wherein the transgene expression cassette is a viral vector transgene expression cassette.
28. The nucleotide sequence of paragraph 27, wherein the viral vector transgene expression cassette is selected from the group consisting of: a retroviral vector transgene expression cassette, an adenoviral vector transgene expression cassette, an adeno-associated viral vector transgene expression cassette, a herpes simplex viral vector transgene expression cassette, and a vaccinia viral vector transgene expression cassette.
29. The nucleotide sequence of paragraph 28, wherein the viral vector transgene expression cassette is a retroviral vector transgene expression cassette or an adeno-associated viral vector transgene expression cassette.
30. The nucleotide sequence of paragraph 29, wherein the retroviral vector transgene expression cassette is a lentiviral vector transgene expression cassette.
31. A nucleotide sequence comprising a viral vector genome expression cassette, wherein the viral vector genome expression cassette comprises the nucleotide sequence of any one of paragraphs 1 to 30.
32. The nucleotide sequence of paragraph 31, wherein the viral vector genome expression cassette is selected from the group consisting of: a retroviral vector genome expression cassette, an adenoviral vector genome expression cassette, an adeno-associated viral vector genome expression cassette, a herpes simplex viral vector genome expression cassette, and a vaccinia viral vector genome expression cassette.
33. The nucleotide sequence of paragraph 32, wherein the viral vector genome expression cassette is a retroviral vector genome expression cassette or an adeno-associated viral vector transgene expression cassette.
34. The nucleotide sequence of paragraph 33, wherein the 3' UTR of the retroviral vector genome expression cassette further comprises a 3' polypurine tract (3'ppt) that is located 5' to a DNA attachment (att) site, wherein, when the transgene expression cassette is in the forward orientation with respect to the genome expression cassette, the cis-acting sequence(s) are located 5' to the 3'ppt and/or 3' to the att site.
35. The nucleotide sequence of paragraph 33 or paragraph 34, wherein the retroviral vector genome expression cassette is a lentiviral vector genome expression cassette.
36. The nucleotide sequence of paragraph 35, wherein the major splice donor site in the lentiviral vector genome expression cassette is inactivated, optionally wherein the cryptic splice donor site 3' to the major splice donor site is also inactivated.
37. The nucleotide sequence of paragraph 36, wherein the inactivated major splice donor site has the sequence of GGGGAAGGCAACAGATAAATATGCCTTAAAAT (SEQ ID NO: 4).
38. The nucleotide sequence of any one of paragraphs 35 to 37, wherein the nucleotide sequence further comprises a nucleotide sequence encoding a modified U1 snRNA, wherein the modified U1 snRNA has been modified to bind to a nucleotide sequence within the packaging region of the lentiviral vector genome.
39. The nucleotide sequence of paragraph 38, wherein the viral vector genome expression cassette is operably linked to the nucleotide sequence encoding the modified U1 snRNA.
40. A viral vector comprising a viral vector genome encoded by the nucleotide sequence of any one of paragraphs 31 to 39.
41. A viral vector production system comprising a nucleotide sequence according to any one of paragraphs 1 to 39 and one or more additional nucleotide sequence(s) encoding viral vector components.
42. The viral vector production system of paragraph 41, wherein the one or more additional nucleotide sequence(s) encode gag-pol and env, and optionally rev.
43. A cell comprising a nucleotide sequence according to any one of paragraphs 1 to 39, a viral vector according to paragraph 40, or a viral vector production system according to paragraph 41 or 42.
44. A method for producing a viral vector, comprising the steps of:
   (a) introducing a viral vector production system of paragraph 41 or 42 into a cell; and
   (b) culturing the cell under conditions suitable for the production of the viral vector.
45. A viral vector produced by the method of paragraph 44.
46. Use of the nucleotide sequence of any of paragraphs 1 to 39, the viral vector production system of paragraph 41 or 42, or the cell of paragraph 43, for producing a viral vector.
47. A method for identifying one or more cis-acting sequence(s) that improve transgene expression in a target cell, the method comprising the steps of:
   (a) transducing target cells with a viral vector of paragraph 40 or 45;
   (b) identifying target cells with a high level transgene expression; and
   (c) optionally identifying the one or more cis-acting sequence(s) located within the 3' UTR of the transgene mRNA present within these target cells.
48. The method of paragraph 47, wherein step (c) comprises performing RT PCR and optionally sequencing the transgene mRNA.
49. The method of paragraph 47 or 48, wherein the method is performed using a plurality of viral vectors with:
   (i) different cis-acting sequences in the 3'UTR of the transgene expression cassette; and/or
   (ii) different cis-acting sequence locations within the 3'UTR of the transgene expression cassette; and/or
   (iii) different cis-acting sequence combinations in the 3'UTR of the transgene expression cassette;
   to identify one or more cis-acting sequence(s) that improve transgene expression in the target cell.

Various preferred features and embodiments of the present invention will now be described with reference to the following numbered paragraphs Set C:
1. A nucleotide sequence comprising a lentiviral vector genome expression cassette, wherein:
   i) the major splice donor site in the lentiviral vector genome expression cassette is inactivated;
   ii) the lentiviral vector genome expression cassette does not comprise a rev-response element;
   iii) the lentiviral vector genome expression cassette comprises a transgene expression cassette and a vector intron; and
   iv)
      a) when the transgene expression cassette is inverted with respect to the lentiviral vector genome expression cassette:
         i. the vector intron is not located between the promoter of the transgene expression cassette and the transgene; and/or
         ii. the nucleotide sequence comprises a sequence as set forth in any of SEQ ID NOs: 2, 3, 4, 6, 7, and/or 8, and/or the sequences CAGACA, and/or GTGGAGACT; and/or
         iii. the 3' UTR of the transgene expression cassette comprises the vector intron; and/or
      b) the vector intron comprises one or more transgene mRNA self-destabilization element(s), transgene mRNA self-decay element(s) or transgene mRNA nuclear retention signal(s); and/or
      c) the cryptic splice donor site adjacent to the 3' end of the major splice donor site in the lentiviral vector genome expression cassette is inactivated.
2. A nucleotide sequence comprising a lentiviral vector genome expression cassette, wherein:
   i) the major splice donor site in the lentiviral vector genome expression cassette is inactivated;
   ii) the lentiviral vector genome expression cassette does not comprise a rev-response element;
   iii) the lentiviral vector genome expression cassette comprises a transgene expression cassette and a vector intron; and
   iv) when the transgene expression cassette is inverted with respect to the lentiviral vector genome expression cassette, the vector intron is not located between the promoter of the transgene expression cassette and the transgene.
3. A nucleotide sequence comprising a lentiviral vector genome expression cassette, wherein:
   i) the major splice donor site in the lentiviral vector genome expression cassette is inactivated;
   ii) the lentiviral vector genome expression cassette does not comprise a rev-response element;
   iii) the lentiviral vector genome expression cassette comprises a transgene expression cassette and a vector intron; and
   iv) when the transgene expression cassette is inverted with respect to the lentiviral vector genome expression cassette, the nucleotide sequence comprises a sequence as set forth in any of SEQ ID NOs: 2, 3, 4, 6, 7, and/or 8, and/or the sequences CAGACA, and/or GTGGAGACT.
4. A nucleotide sequence comprising a lentiviral vector genome expression cassette, wherein:
   i) the major splice donor site in the lentiviral vector genome expression cassette is inactivated;
   ii) the lentiviral vector genome expression cassette does not comprise a rev-response element; and
   iii) the lentiviral vector genome expression cassette comprises a transgene expression cassette and a vector intron, and
   iv) when the transgene expression cassette is inverted with respect to the lentiviral vector genome expression cassette, the 3' UTR of the transgene expression cassette comprises the vector intron.
5. A nucleotide sequence comprising a lentiviral vector genome expression cassette, wherein:
   i) the major splice donor site and cryptic splice donor site adjacent to the 3' end of the major splice donor site in the lentiviral vector genome expression cassette are inactivated;
   ii) the lentiviral vector genome expression cassette does not comprise a rev-response element;
   iii) the lentiviral vector genome expression cassette comprises a transgene expression cassette and a vector intron.
6. The nucleotide sequence according to any one of paragraphs 2-5, wherein said vector intron comprises one or more transgene mRNA self-destabilization element(s), transgene mRNA self-decay element(s), or transgene mRNA nuclear retention signal(s).
7. The nucleotide sequence according to any one of paragraphs 3-6, wherein, when the transgene expression cassette is inverted with respect to the lentiviral vector genome expression cassette, the vector intron is not located between the promoter of the transgene expression cassette and the transgene.
8. The nucleotide sequence according to any one of paragraphs 2, or 4-6, wherein, when the transgene expression cassette is inverted with respect to the lentiviral vector genome expression cassette, the nucleotide sequence comprises a sequence as set forth in any of SEQ ID NOs: 2, 3, 4, 6, 7 and/or 8, and/or the sequences CAGACA, and/or GTGGAGACT.
9. The nucleotide sequence according to any one of paragraphs 2, 3, 5 or 6, wherein, when the transgene expression cassette is inverted with respect to the lentiviral vector genome expression cassette, the 3' UTR of the transgene expression cassette comprises the vector intron.
10. The nucleotide sequence according to any one of the preceding paragraphs, wherein the vector intron is not located between the promoter of the transgene expression cassette and the transgene.
11. The nucleotide sequence according to any one of the preceding paragraphs, wherein the nucleotide sequence comprises a sequence as set forth in any of SEQ ID NOs: 2, 3, 4, 6, 7, and/or 8, and/or the sequences CAGACA, and/or GTGGAGACT.
12. The nucleotide sequence according to any one of the preceding paragraphs, wherein:
   (a) when the transgene expression cassette is inverted with respect to the lentiviral vector genome expression cassette, the 3' UTR of the transgene expression cassette comprises the vector intron; or
   (b) when the transgene expression cassette is in the forward orientation with respect to the lentiviral vector genome expression cassette, the vector intron is located 5' of the transgene or the transgene expression cassette.
13. The nucleotide sequence of any one of the preceding paragraphs, wherein the transgene expression cassette is inverted with respect to the lentiviral vector genome expression cassette.
14. The nucleotide sequence of any one of the preceding paragraphs, wherein the transgene expression cassette is in the forward orientation with respect to the lentiviral vector genome expression cassette.
15. The nucleotide sequence according to any one of the preceding paragraphs, wherein the lentiviral vector genome expression cassette comprises at least one modified viral cis-acting sequence, wherein at least one internal open reading frame (ORF) in the viral cis-acting sequence is disrupted.
16. The nucleotide sequence according to any one of the preceding paragraphs, wherein the at least one viral cis-acting sequence is a Woodchuck hepatitis virus (WHV) post-transcriptional regulatory element (WPRE).
17. The nucleotide sequence according to any one of the preceding paragraphs, wherein the lentiviral vector genome expression cassette comprises a modified nucleotide sequence encoding gag, and wherein at least one internal open reading frame (ORF) in the modified nucleotide sequence encoding gag is disrupted.
18. The nucleotide sequence according to any one of paragraphs 15-17, wherein the at least one internal ORF in the modified nucleotide sequence is disrupted by mutating at least one ATG sequence within the nucleotide sequence.
19. The nucleotide sequence according to paragraph 18, wherein the first ATG sequence within the modified nucleotide sequence is mutated.
20. The nucleotide sequence according to any one of the preceding paragraphs, wherein the lentiviral vector genome expression cassette lacks (i) a nucleotide sequence encoding Gag-p17 or (ii) a fragment of a nucleotide sequence encoding Gag-p17.
21. The nucleotide sequence according to paragraph 20, wherein the fragment of a nucleotide sequence encoding Gag-p17 comprises a nucleotide sequence encoding p17 instability element.
22. The nucleotide sequence according to any one of the preceding paragraphs, wherein the nucleotide sequence comprising a lentiviral vector genome expression cassette does not express Gag-p17 or a fragment thereof.
23. The nucleotide sequence according to paragraph 22, wherein said fragment of Gag-p17 comprises the p17 instability element.
24. The nucleotide sequence of any one of the preceding paragraphs, wherein said vector intron is a synthetic vector intron.
25. The nucleotide sequence of any one of the preceding paragraphs, wherein said synthetic intron comprises the HIV-1 guanosine-adenosine rich (GAR) splicing element.
26. The nucleotide sequence of any one of the preceding paragraphs, wherein said vector intron comprises a splice donor sequence, wherein said splice donor sequence comprises from 7 to 11 nucleotides which are complementary to a portion of the U1 snRNA sequence.
27. The nucleotide sequence of paragraph 26, wherein said vector intron is a synthetic vector intron comprising the HIV-1 guanosine-adenosine rich (GAR) splicing element upstream of the splice donor sequence.
28. The nucleotide sequence of any one of the preceding paragraphs, wherein said vector intron is a synthetic intron comprising the branch site and splice acceptor of the human beta-globin intron-2.
29. The nucleotide sequence of any one of paragraphs 1, 6-8 or 10-28, wherein said transgene mRNA self-destabilization or self-decay element comprises at least one of the following:
   (a) a self-cleaving ribozyme;
   (b) an AU-rich element; and/or
   (c) an interfering RNA, preferably a miRNA or shRNA which targets the inverted transgene mRNA.
30. A nucleotide sequence comprising a lentiviral vector genome expression cassette, wherein the lentiviral vector genome expression cassette comprises:
   (a) a transgene expression cassette which is inverted with respect to the lentiviral vector genome expression cassette; and
   (b) a vector intron comprising a nucleotide expression cassette encoding a miRNA;
   wherein the miRNA targets mRNA encoding the transgene, preferably wherein the guide strand of the miRNA is fully complementary to the mRNA encoding the transgene, optionally wherein the nucleotide sequence is for use in a lentiviral vector production cell.
31. **The** nucleotide sequence of paragraph 30, wherein the passenger strand of the miRNA comprises at least one mismatch with its target sequence in the vector genome RNA, preferably wherein the miRNA does not target the vector genome RNA.
32. A viral vector production system comprising a set of nucleotide sequences, wherein the nucleotide sequences encode gag-pol and env, and the nucleotide sequence according to any one of paragraphs 1 to 31.
33. **The** viral vector production system according to paragraph 32, wherein the viral vector production system does not comprise a nucleotide sequence encoding rev.
34. A cell comprising the nucleotide sequence according to any one of paragraphs 1 to 31 or the viral vector production system according to paragraph 32 or paragraph 33.
35. A cell for producing lentiviral vectors comprising:
   (i)
      a) nucleotide sequences encoding gag-pol and env, and the nucleotide sequence according to any one of paragraphs 1 to 31; or
      b) the viral vector production system according to paragraph 32 or paragraph 33; and
   (ii) optionally, a nucleotide sequence encoding TRAP.
36. The cell according to paragraph 35, wherein the cell does not comprise a nucleotide sequence encoding rev.
37. A method for producing a lentiviral vector, comprising the steps of:
   (i) introducing:
      a)
         i. nucleotide sequences gag-pol and env, and the nucleotide sequence according to any one of paragraphs 1 to 31; or
         ii. the viral vector production system according to paragraph 32 or paragraph 33; and
      b) optionally, a nucleotide sequence encoding TRAP
         into a cell;
   (ii) optionally, selecting for a cell which comprises the nucleotide sequences encoding gag-pol and env and the nucleotide sequence according to any one of paragraphs 1 to 31; and
   (iii) culturing the cell under conditions suitable for the production of the lentiviral vector.
38. The method according to paragraph 37, wherein the cell does not comprise a nucleotide sequence encoding rev.
39. A lentiviral vector produced by the method according to paragraph 37 or paragraph 38.
40. A lentiviral vector according to paragraph 39 wherein the lentiviral vector comprises a splice junction sequence, preferably wherein the splice junction sequence has the sequence set forth in SEQ ID NO: 19
41. Use of the nucleotide sequence according to any one of paragraphs 1 to 31, the viral vector production system according to paragraph 32 or paragraph 33, or the cell according to any one of paragraphs 34 to 36 for producing a lentiviral vector.

Various preferred features and embodiments of the present invention will now be described with reference to the following numbered paragraphs Set D:
1. A set of nucleic acid sequences for producing a retroviral vector comprising:
   (i) a nucleic acid sequence encoding the retroviral vector genome, wherein the retroviral vector genome comprises a transgene expression cassette; and
   (ii) at least one nucleic acid sequence encoding an interfering RNA which is specific for mRNA encoding the transgene.
2. The set of nucleic acid sequences of paragraph 1, wherein the set of nucleic acid sequences further comprises nucleic acid sequences encoding Gag/pol and env or a functional substitute thereof.
3. The set of nucleic acid sequences of paragraph 1 or paragraph 2, wherein the set of nucleic acid sequences further comprises a nucleic acid sequence encoding rev or a functional substitute thereof.
4. The set of nucleic acid sequences of any one of the preceding paragraphs, wherein the transgene expression cassette is inverted with respect to the retroviral vector genome expression cassette.
5. The set of nucleic acid sequences of any one of the preceding paragraphs, wherein the transgene expression cassette is in the forward direction with respect to the retroviral vector genome expression cassette.
6. The set of nucleic acid sequences of any one of the preceding paragraphs, wherein the 3' UTR or the 5' UTR of the transgene expression cassette comprises at least one target nucleotide sequence.
7. The set of nucleic acid sequences of any one of the preceding paragraphs, wherein the transgene expression cassette is genetically engineered to comprise at least one target nucleotide sequence within the 3' UTR or 5' UTR.
8. The set of nucleic acid sequences of paragraph 6 or paragraph 7, wherein the interfering RNA is specific for the at least one target nucleotide sequence.
9. The set of nucleic acid sequences of paragraph 6 or paragraph 7, wherein the set of nucleic acid sequences comprises multiple nucleic acid sequences encoding a plurality of interfering RNAs specific for multiple target nucleotide sequences.
10. The set of nucleic acid sequences of any one of the preceding paragraphs, wherein the interfering RNA(s) promote cleavage of mRNA encoding the transgene.
11. The set of nucleic acid sequences of any one of the preceding paragraphs, wherein the interfering RNA(s) target mRNA encoding the transgene for cleavage.
12. The set of nucleic acid sequences of any one of the preceding paragraphs, wherein the interfering RNA(s) target mRNA encoding the transgene for cleavage by the RISC.
13. The set of nucleic acid sequences of any one of the preceding paragraphs, wherein the interfering RNA is an siRNA; a sisiRNA; a tsiRNA; a RNA-DNA chimeric duplex; a tkRNA; a Dicer-substrate dsRNA; a shRNA; a tRNA-shRNA; an aiRNA; a miRNA; a pre-miRNA; a pri-miRNA mimic; a pri-miRNA mimic cluster; a transcriptional gene silencing (TGS); and/or combinations thereof.
14. The set of nucleic acid sequences of any one of the preceding paragraphs, wherein the interfering RNA is a siRNA, a shRNA and/or a miRNA.
15. The set of nucleic acid sequences of any one of the preceding paragraphs, wherein the interfering RNA is a miRNA.
16. The set of nucleic acid sequences of any one of paragraphs 13 to 15, wherein the guide strand of the miRNA is fully complementary to the target sequence of the transgene mRNA.
17. The set of nucleic acid sequences of paragraph 14, wherein the miRNA comprises a passenger strand which comprises at least one mismatch with its complimentary sequence within the RNA genome of the retroviral vector.
18. The set of nucleic acid sequences of any one of the preceding paragraphs, wherein the nucleic acid encoding the retroviral vector genome comprises the nucleic acid sequence encoding the interfering RNA.
19. The set of nucleic acid sequences of any one of the preceding paragraphs, wherein the retroviral vector genome expression cassette further comprises a vector intron.
20. The set of nucleic acid sequences of paragraph 19, wherein the vector intron comprises the nucleic acid sequence encoding the interfering RNA.
21. The set of nucleic acid sequences of any one of paragraphs 1 to 17, wherein the set of nucleic acid sequences comprises a first nucleic acid sequence encoding the retroviral vector genome and at least a second nucleic acid sequence encoding the interfering RNA.
22. The set of nucleic acid sequences of any one of paragraphs 1 to 17 or 21, wherein the nucleic acid encoding the retroviral vector genome does not comprise the nucleic acid sequence encoding the interfering RNA.
23. The set of nucleic acid sequences of any one of the preceding paragraphs, wherein the retroviral vector genome further comprises a tryptophan RNA-binding attenuation protein (TRAP) binding site.
24. The set of nucleic acid sequences of any one of the preceding paragraphs, wherein the major splice donor site in the retroviral vector genome is inactivated, and optionally wherein the cryptic splice donor site 3' to the major splice donor site is inactivated.
25. The set of nucleic acid sequences of paragraph 24, wherein the inactivated major splice donor site has the sequence set forth in SEQ ID NO: 4.
26. The set of nucleic acid sequences of any one of the preceding paragraphs, wherein the set of nucleic acid sequences further comprises a nucleic acid sequence encoding a modified U1 snRNA, wherein the modified U1 snRNA has been modified to bind to a nucleotide sequence within the packaging region of the retroviral vector genome sequence.
27. The set of nucleic acid sequences of any one of the preceding paragraphs, wherein the transgene gives rise to a therapeutic effect.
28. The set of nucleic acid sequences of any one of the preceding paragraphs, wherein the retroviral vector is a lentiviral vector, preferably wherein the lentiviral vector is derived from HIV-1, HIV-2, SIV, FIV, BIV, EIAV, CAEV or Visna lentivirus.
29. The set of nucleic acid sequences of paragraph 28, wherein the lentiviral vector genome comprises at least one modified viral cis-acting sequence, wherein at least one internal open reading frame (ORF) in the viral cis-acting sequence is disrupted.
30. The set of nucleic acid sequences of paragraph 29, wherein the at least one viral *cis-*acting sequence is: (a) a Rev response element (RRE); and/or (b) a Woodchuck hepatitis virus (WHV) post-transcriptional regulatory element (WPRE).
31. The set of nucleic acid sequences of any one of paragraphs 28 to 30, wherein the lentiviral vector genome expression cassette comprises a modified nucleotide sequence encoding gag, and wherein at least one internal open reading frame (ORF) in the modified nucleotide sequence encoding gag is disrupted.
32. The set of nucleic acid sequences of any one of paragraphs 29 to 31, wherein the at least one internal ORF is disrupted by mutating at least one ATG sequence.
33. The set of nucleic acid sequences according to any one of paragraphs 30 to 32, wherein: (a) the modified RRE comprises less than eight ATG sequences; and/or (b) the modified WPRE comprises less than seven ATG sequences.
34. The set of nucleic acid sequences of paragraph 31, wherein the first ATG sequence within the nucleotide sequence encoding gag is mutated.
35. The set of nucleic acid sequences of any one of paragraphs 28 to 34, wherein the lentiviral vector genome expression cassette lacks (i) a nucleotide sequence encoding Gag-p17 or (ii) a fragment of a nucleotide sequence encoding Gag-p17, preferably wherein the fragment of a nucleotide sequence encoding Gag-p17 comprises a nucleotide sequence encoding p17 instability element.
36. The set of nucleic acid sequences of any one of paragraphs 28 to 35, wherein the nucleotide sequence comprising a lentiviral vector genome expression cassette does not express Gag-p17 or a fragment thereof, preferably wherein said fragment of Gag-p17 comprises the p17 instability element.
37. A retroviral vector production system comprising the set of nucleic acid sequences of any one of the preceding paragraphs.
38. A retroviral vector production system comprising a viral vector production cell, wherein the viral vector production cell comprises the set of nucleic acid sequences of any one of paragraphs 1 to 36.
39. An expression cassette encoding a retroviral vector genome comprising:
   (i) a transgene expression cassette; and
   (ii) a vector intron comprising at least one interfering RNA as defined in any one of paragraphs 1 to 36.
40. The expression cassette of paragraph 39, wherein the transgene expression cassette is inverted with respect to the retroviral vector genome expression cassette.
41. A nucleotide sequence comprising the expression cassette of paragraph 39 or paragraph 40.
42. A retroviral vector genome comprising a transgene expression cassette and a vector intron, optionally wherein the transgene expression cassette is inverted with respect to the retroviral vector genome expression cassette, and wherein the vector intron comprises at least one interfering RNA as defined in any one of paragraphs 1 to 36.
43. A cell comprising the set of nucleic acid sequences of any one of paragraphs 1 to 36, the retroviral vector production system of paragraph 37, the expression cassette of paragraph 39 or paragraph 40, the nucleotide sequence of paragraph 41 or the retroviral vector genome of paragraph 42.
44. A cell for producing retroviral vectors comprising:
   a) the set of nucleic acid sequences of any one of paragraphs 1 to 36, the retroviral vector production system of paragraph 37, the expression cassette of paragraph 39 or paragraph 40, the nucleotide sequence of paragraph 41 or the retroviral vector genome of paragraph 42; and
   b) optionally, a nucleic acid sequence encoding a modified U1 snRNA and/or a nucleic acid sequence encoding TRAP.
45. A method for producing a retroviral vector, comprising the steps of:
   (i) introducing:
      a) the set of nucleic acid sequences of any one of paragraphs 1 to 36, the retroviral vector production system of paragraph 37, the expression cassette of paragraph 39 or paragraph 40, the nucleotide sequence of paragraph 41 or the retroviral vector genome of paragraph 42; and
      b) optionally, a nucleic acid sequence encoding a modified U1 snRNA and/or a nucleic acid sequence encoding TRAP
         into a cell;
   (ii) optionally, selecting for a cell which comprises the nucleic acid sequences encoding vector components; and
   (iii) culturing the cell under conditions suitable for the production of the retroviral vector.
46. A method for producing a retroviral vector, comprising the step of culturing the cell of paragraph 43 or paragraph 44 under conditions suitable for the production of the retroviral vector.
47. A retroviral vector produced by the method of paragraph 45 or paragraph 46.
48. Use of the set of nucleic acid sequences of any one of paragraphs 1 to 36, the retroviral vector production system of paragraph 37 or paragraph 38, the expression cassette of paragraph 39 or paragraph 40, the nucleotide sequence of paragraph 41, the retroviral vector genome of paragraph 42, or the cell of paragraph 43 or paragraph 44 for producing a retroviral vector.
49. Use of at least one nucleic acid sequence encoding an interfering RNA as defined in any one of paragraphs 1-36 for repressing expression of a transgene and/or increasing retroviral vector titre during retroviral vector production.

### NUMBERED EMBODIMENS

1. A nucleotide sequence comprising a lentiviral vector genome expression cassette, wherein:
   a) the 3' LTR of the lentiviral vector genome comprises a modified polyadenylation sequence, and wherein the modified polyadenylation sequence comprises a polyadenylation signal which is 5' of the 3' LTR R region;
   b) the lentiviral vector genome further comprises a modified 5' LTR, and wherein the R region of the modified 5' LTR comprises at least one polyadenylation downstream enhancer element (DSE);
   c) the lentiviral vector genome further comprises a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence; and
   d) the lentiviral vector genome further comprises a vector intron.
2. A nucleotide sequence comprising a lentiviral vector genome expression cassette, wherein the 3' LTR of the lentiviral vector genome comprises a modified polyadenylation sequence, and wherein the modified polyadenylation sequence comprises a polyadenylation signal which is 5' of the 3' LTR R region.
3. A nucleotide sequence comprising a lentiviral vector genome expression cassette, wherein the lentiviral vector genome comprises a modified 5' LTR, and wherein the R region of the modified 5' LTR comprises at least one polyadenylation downstream enhancer element (DSE).
4. A nucleotide sequence comprising a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.
5. A nucleotide sequence comprising a lentiviral vector genome expression cassette, wherein the lentiviral vector genome expression cassette comprises a transgene expression cassette and a vector intron.
6. The nucleotide sequence according to embodiment 2, wherein the lentiviral vector genome further comprises:
   a) a modified 5' LTR, and wherein the R region of the modified 5' LTR comprises at least one polyadenylation downstream enhancer element (DSE);
   b) a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence; and/or
   c) a transgene expression cassette and a vector intron.
7. The nucleotide sequence according to embodiment 2 or embodiment 6, wherein the lentiviral vector genome further comprises:
   a) a modified 5' LTR, and wherein the R region of the modified 5' LTR comprises at least one polyadenylation downstream enhancer element (DSE);
   b) a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence; and
   c) a transgene expression cassette and a vector intron.
8. The nucleotide sequence according to embodiment 3, wherein the lentiviral vector genome further comprises:
   a) a modified 3' LTR, wherein the 3' LTR of the lentiviral vector genome comprises a modified polyadenylation sequence, and wherein the modified polyadenylation sequence comprises a polyadenylation signal which is 5' of the 3' LTR R region;
   b) a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence; and/or
   c) a transgene expression cassette and a vector intron.
9. The nucleotide sequence according to embodiment 3 or embodiment 8, wherein the lentiviral vector genome further comprises:
   a) a modified 3' LTR, wherein the 3' LTR of the lentiviral vector genome comprises a modified polyadenylation sequence, and wherein the modified polyadenylation sequence comprises a polyadenylation signal which is 5' of the 3' LTR R region;
   b) a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence; and
   c) a transgene expression cassette and a vector intron.
10. The nucleotide sequence according to embodiment 2 or embodiment 3, wherein the lentiviral vector genome further comprises a transgene expression cassette, wherein the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.
11. The nucleotide sequence according to embodiment 5, wherein:
   a) the lentiviral vector genome further comprises a modified 3' LTR, wherein the 3' LTR of the lentiviral vector genome comprises a modified polyadenylation sequence, and wherein the modified polyadenylation sequence comprises a polyadenylation signal which is 5' of the 3' LTR R region;
   b) the lentiviral vector genome further comprises a modified 5' LTR, and wherein the R region of the modified 5' LTR comprises at least one polyadenylation downstream enhancer element (DSE); and/or
   c) the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.
12. The nucleotide sequence according to embodiment 5 or 11, wherein:
   a) the lentiviral vector genome further comprises a modified 3' LTR, wherein the 3' LTR of the lentiviral vector genome comprises a modified polyadenylation sequence, and wherein the modified polyadenylation sequence comprises a polyadenylation signal which is 5' of the 3' LTR R region;
   b) the lentiviral vector genome further comprises a modified 5' LTR, and wherein the R region of the modified 5' LTR comprises at least one polyadenylation downstream enhancer element (DSE); and
   c) the 3' UTR of the transgene expression cassette comprises at least one cis-acting sequence selected from (a) a cis-acting Cytoplasmic Accumulation Region (CAR) sequence; and/or (b) a cis-acting ZCCHC14 protein-binding sequence.
13. The nucleotide sequence according any one of embodiments 1 or 5 to 12, wherein:
   a) the transgene expression cassette is in the forward orientation with respect to the lentiviral vector genome expression cassette; or
   b) the transgene expression cassette is inverted with respect to the lentiviral vector genome expression cassette.
14. The nucleotide sequence according to any one of embodiments 1, 5 to 9 or 11 to 13, wherein:
   a) the vector intron comprises one or more transgene mRNA self-destabilization or self-decay element(s) or one or more transgene mRNA nuclear retention signal(s), preferably wherein the vector intron comprises one or more transgene mRNA self-destabilization or self-decay element(s);
   b) the vector intron is not located between the promoter of the transgene expression cassette and the transgene; and/or
   c) the 3' UTR of the transgene expression cassette comprises the vector intron.
15. The nucleotide sequence according to any one of embodiments 1 to 3 or 5 to 14, wherein the major splice donor site in the lentiviral vector genome is inactivated, preferably wherein the nucleotide sequence comprises a sequence as set forth in any of SEQ ID NOs: 2, 3, 4, 6, 7, and/or 8, and/or the sequences CAGACA, and/or GTGGAGACT.
16. The nucleotide sequence according to any one of embodiments 1 to 3 or 5 to 15, wherein the cryptic splice donor site adjacent to the 3' end of the major splice donor site in the lentiviral vector genome is inactivated.
17. The nucleotide sequence according to any one of embodiments 1to 3 or 5 to 16, wherein the lentiviral vector genome does not comprise a rev-response element (RRE).
18. The nucleotide sequence according to any one of embodiments 1, 2 or 6 to 17, wherein the native 3' polyadenylation sequence has been mutated or deleted, preferably wherein the native 3' polyadenylation signal has been mutated or deleted.
19. The nucleotide sequence according to any one of embodiments 1, 2 or 6 to 18, wherein the modified polyadenylation sequence is a heterologous polyadenylation sequence, preferably wherein the modified polyadenylation sequence comprises a heterologous polyadenylation signal.
20. The nucleotide sequence according to any one of embodiments 1, 2 or 6 to 19, wherein the modified polyadenylation sequence further comprises a polyadenylation upstream enhancer element (USE), preferably wherein the USE is 5' of the polyadenylation signal.
21. The nucleotide sequence according to any one of embodiments 1, 2 or 6 to 20, wherein the modified polyadenylation sequence further comprises a GU rich downstream enhancer element, preferably wherein the GU rich downstream enhancer element is 3' of the polyadenylation signal.
22. The nucleotide sequence according to any one of embodiments 1, 2 or 6 to 21, wherein the polyadenylation signal which is 5' of the 3' LTR R region is in the sense strand, preferably wherein the 3' LTR further comprises a polyadenylation signal in the antisense strand which is (i) 5' of the polyadenylation signal in the sense strand and (ii) 3' of the attachment sequence of the 3' LTR.
23. The nucleotide sequence according to any one of embodiments 1,2 or 6 to 22, wherein:
   a) the 3' LTR R region of the modified polyadenylation sequence is a minimal R region; and/or
   b) the 3' LTR R region of the modified polyadenylation sequence is homologous to the native 5' LTR R region of the lentiviral vector genome.
24. The nucleotide sequence according to any one of embodiments 1, 2 or 6 to 23, wherein the modified polyadenylation sequence further comprises a polyadenylation cleavage site, preferably wherein the polyadenylation cleavage site is 3' of the polyadenylation signal.
25. The nucleotide sequence according to any one of embodiments 1, 3 or 6 to 24, wherein the first 55 nucleotides of the modified 5' LTR comprises the polyadenylation DSE, preferably wherein the first 40 nucleotides of the modified 5' LTR comprises the at least one polyadenylation DSE.
26. The nucleotide sequence according to any one of embodiments 1, 3 or 6 to 25, wherein the polyadenylation DSE is comprised within a stem loop structure of the modified 5' LTR.
27. The nucleotide sequence according to any one of embodiments 1, 3 or 6 to 26, wherein the polyadenylation DSE is a GU-rich sequence, preferably wherein the GU-rich sequence is derived from RSV or MMTV, or wherein the GU-rich sequence is synthetic.
28. The nucleotide sequence according to any one of embodiments 1, 3 or 6 to 27, wherein the native polyadenylation signal of the modified 5' LTR has been mutated or deleted.
29. The nucleotide sequence according to any one of embodiments 1, 3 or 6 to 28, wherein the 5' R region of the modified 5' LTR further comprises a polyadenylation cleavage site.
30. The nucleotide sequence according to any one of embodiments 1, 4 or 6 to 29, wherein the CAR sequence comprises a plurality of CARe sequences, preferably wherein the plurality of CARe sequences are in tandem.
31. The nucleotide sequence according to any one of embodiments 1, 4 or 6 to 30, wherein the CAR sequence comprises at least two, at least four, at least six, at least eight, at least ten, at least twelve, at least fourteen, at least sixteen, at least eighteen, or at least twenty CARe sequences, optionally wherein the CARe sequences are in tandem.
32. The nucleotide sequence according to embodiment 31, wherein the CAR sequence comprises at least six CARe sequences in tandem, least eight CARe sequences in tandem, at least ten CARe sequences in tandem, at least twelve CARe sequences in tandem, or at least sixteen CARe sequences in tandem.
33. The nucleotide sequence according to any one of embodiments 1, 4 or 6 to 32, wherein the CARe nucleotide sequence is BMWGHWSSWS (SEQ ID NO: 92) or BMWRHWSSWS (SEQ ID NO: 243), preferably wherein the CARe nucleotide sequence is CMAGHWSSTG (SEQ ID NO: 96).
34. The nucleotide sequence according to any one of embodiments 1, 4 or 6 to 33, wherein the CARe nucleotide sequence is selected from the group consisting of: CCAGTTCCTG (SEQ ID NO: 97), CCAGATCCTG (SEQ ID NO: 97), CCAGTTCCTC (SEQ ID NO: 99), TCAGATCCTG (SEQ ID NO: 100), CCAGATGGTG (SEQ ID NO: 101), CCAGTTCCAG (SEQ ID NO: 102), CCAGCAGCTG (SEQ ID NO: 103), CAAGCTCCTG (SEQ ID NO: 104), CAAGATCCTG (SEQ ID NO: 105), CCTGAACCTG (SEQ ID NO: 106), CAAGAACGTG (SEQ ID NO: 107), TCAGTTCCTG (SEQ ID NO: 227), GCAGTTCCTG (SEQ ID NO: 228), CAAGTTCCTG (SEQ ID NO: 229), CCTGTTCCTG (SEQ ID NO: 230), CCTGCTCCTG (SEQ ID NO: 231), CCTGTACCTG (SEQ ID NO:232), CCTGTTGCTG (SEQ ID NO: 233), CCTGTTCGTG (SEQ ID NO: 234), CCTGTTCCAG (SEQ ID NO: 235), CCTGTTCCTC (SEQ ID NO: 236), CCAATTCCTG (SEQ ID NO: 237) and GAAGCTCCTG (SEQ ID NO: 238); preferably wherein the CARe nucleotide sequence is CCAGTTCCTG (SEQ ID NO: 97), CCTGTTCCTG (SEQ ID NO: 230), CCTGTACCTG (SEQ ID NO: 232), CCTGTTCCAG (SEQ ID NO: 235), CCAATTCCTG (SEQ ID NO: 237), CCTGAACCTG (SEQ ID NO: 106), CCAGTTCCTC (SEQ ID NO: 99) and CCAGTTCCAG (SEQ ID NO: 102).
35. The nucleotide sequence according to any one of embodiments 1, 4 or 6 to 34, wherein the 3' UTR of the transgene expression cassette does not comprise additional post-transcriptional regulatory elements (PREs).
36. The nucleotide sequence according to any one of embodiments 1, 4 or 6 to 35, wherein the 3' UTR of the transgene expression cassette comprises at least one additional post-transcriptional regulatory element (PRE), preferably wherein the additional PRE is a Woodchuck hepatitis virus PRE (wPRE).
37. The nucleotide sequence according to any one of embodiments 1, 4 or 6 to 36, wherein the cis-acting ZCCHC14 protein-binding sequence is a PRE α element fragment, preferably wherein the cis-acting ZCCHC14 protein-binding sequence is:
   a) a fragment of a HBV PRE α element; or
   b) a fragment of HCMV RNA 2.7; or
   c) a fragment of a wPRE α element.
38. The nucleotide sequence according to embodiment 37, wherein the PRE α element fragment is no more than 200 nucleotides in length, preferably wherein the PRE α element fragment is no more than 90 nucleotides in length.
39. The nucleotide sequence according to any one of embodiments 1, 4 or 6 to 38, wherein the CNGGN-type pentaloop sequence is comprised within a stem-loop structure having a sequence selected from the group consisting of:
   (i) TCCTCGTAGGCTGGTCCTGGGGA (SEQ ID NO: 108); and
   (ii) GCCCGCTGCTGGACAGGGGC (SEQ ID NO: 109).
40. The nucleotide sequence according to any one of embodiments 1, 4 or 6 to 39, wherein the CNGGN-type pentaloop sequence is comprised within a heterologous stem-loop structure.
41. The nucleotide sequence according to any one of embodiments 1, 4 or 6 to 40, wherein the ZCCHC14 protein-binding sequence comprises a sequence selected from the group consisting of:
   (i) 
TGCCGTCGCCACCGCGTTATCCGTTCCTCGTAGGCTGGTCCTGGGGAACGGGT
CGGCGGCCGGTCGGCTTCT (SEQ ID NO: 110);
 and
   (ii) 
CTATTGCCACGGCGGAACTCATCGCCGCCTGCCTTGCCCGCTGCTGGACAGGG
GCTCGGCTGTTGGGCACTGACAATTCCGTGGTGTTGT (SEQ ID NO: 111).
42. The nucleotide sequence according to any one of embodiments 1, 4 or 6 to 41, wherein the 3' UTR of the transgene expression cassette comprises a cis-acting CAR sequence and a cis-acting ZCCHC14 protein-binding sequence, optionally wherein the ZCCHC14 protein-binding sequence is located 3' to the CAR sequence.
43. The nucleotide sequence according to any one of embodiments 1, 4 or 6 to 42, wherein the 3' UTR of the transgene expression cassette comprises at least two spatially distinct *cis-*acting CAR sequences and/or at least two spatially distinct cis-acting ZCCHC14 protein-binding sequences.
44. The nucleotide sequence according to any one of embodiments 1, 4 or 6 to 43, wherein the 3' UTR of the transgene expression cassette further comprises a polyA sequence located 3' to the cis-acting CAR sequence and/or cis-acting ZCCHC14 protein-binding sequence.
45. The nucleotide sequence of any one of embodiments 1, or 4 to 44, wherein the transgene expression cassette further comprises a promoter operably linked to the transgene.
46. The nucleotide sequence according to any one of embodiments 1, 2 or 4 to 45, wherein the lentiviral vector genome further comprises a tryptophan RNA-binding attenuation protein (TRAP) binding site.
47. The nucleotide sequence according to any one of the preceding embodiments, wherein the nucleotide sequence further comprises a nucleotide sequence encoding a modified U1 snRNA, wherein said modified U1 snRNA has been modified to bind to a nucleotide sequence within the packaging region of the lentiviral vector genome, preferably the nucleotide sequence encoding the lentiviral vector genome is operably linked to the nucleotide sequence encoding the modified U1 snRNA.
48. The nucleotide sequence according to any one of embodiments 1, 2 or 4 to 47, wherein the lentiviral vector genome comprises at least one modified viral cis-acting sequence, wherein at least one internal open reading frame (ORF) in the viral cis-acting sequence is disrupted, preferably wherein the at least one viral cis-acting sequence is a Woodchuck hepatitis virus (WHV) post-transcriptional regulatory element (WPRE) and/or a Rev response element (RRE).
49. The nucleotide sequence according to any one of embodiments 1, 2 or 4 to 48, wherein the lentiviral vector genome comprises a modified nucleotide sequence encoding gag, and wherein at least one internal open reading frame (ORF) in the modified nucleotide sequence encoding gag is disrupted.
50. The nucleotide sequence according to embodiment 48 or embodiment 49, wherein the at least one internal ORF is disrupted by mutating at least one ATG sequence within the nucleotide sequence, preferably wherein the first ATG sequence within the nucleotide sequence is mutated.
51. The nucleotide sequence according to any one of embodiments 1, 2 or 4 to 50, wherein the lentiviral vector genome lacks (i) a nucleotide sequence encoding Gag-p17 or (ii) a fragment of a nucleotide sequence encoding Gag-p17, preferably wherein the fragment of a nucleotide sequence encoding Gag-p17 comprises a nucleotide sequence encoding p17 instability element.
52. The nucleotide sequence according to any one of embodiments 1, 2 or 4 to 51, wherein the nucleotide sequence comprising a lentiviral vector genome does not express Gag-p17 or a fragment thereof, preferably wherein said fragment of Gag-p17 comprises the p17 instability element.
53. A set of nucleotide sequences for producing a lentiviral vector comprising:
   (i) a nucleic acid sequence comprising a lentiviral vector genome expression cassette, wherein the lentiviral vector genome expression cassette comprises a transgene expression cassette; and
   (ii) at least one nucleic acid sequence encoding an interfering RNA which is specific for mRNA encoding the transgene.
54. A set of nucleotide sequences for producing a lentiviral vector comprising:
   (i) the nucleotide sequence according to any one of embodiments 1 to 3 or 5 to 52, wherein the lentiviral vector genome expression cassette further comprises a transgene expression cassette; and
   (ii) nucleotide sequences encoding lentiviral vector components.
55. A set of nucleotide sequences for producing a lentiviral vector comprising:
   (i) the nucleotide sequence according to any one of embodiments 1to 3 or 5 to 52, wherein the lentiviral vector genome expression cassette further comprises a transgene expression cassette; and
   (ii) at least one nucleic acid sequence encoding an interfering RNA which is specific for mRNA encoding the transgene.
56. The set of nucleotide sequences according to embodiment 53 or embodiment 55, wherein the nucleotide sequence comprising the lentiviral vector genome expression cassette does not comprise the nucleic acid sequence encoding the interfering RNA.
57. The set of nucleotide sequences according to embodiment 53 or embodiment 55, wherein the nucleotide sequence comprising the lentiviral vector genome expression cassette comprises a vector intron and wherein the vector intron comprises the nucleic acid sequence encoding the interfering RNA which is specific for mRNA encoding the transgene.
58. The set of nucleotide sequences according to any one of embodiments 53 or 55 to 57, wherein the set of nucleotide sequences further comprises nucleotide sequences encoding lentiviral vector components, preferably wherein the lentiviral vector components are gag-pol and env, and optionally rev.
59. The set of nucleotide sequences according to any one of embodiments 53 to 58, wherein the transgene expression cassette comprises at least one cis-acting sequence as defined in any one of embodiments 4 or 30 to 44.
60. The set of nucleotide sequences according to any one of embodiments 53 to 59, wherein the 3' UTR or the 5' UTR of the transgene expression cassette comprises at least one target nucleotide sequence.
61. The set of nucleotide sequences according to any one of embodiments 53 to 60, wherein the transgene expression cassette is genetically engineered to comprise at least one target nucleotide sequence within the 3' UTR or 5' UTR.
62. The set of nucleotide sequences according to embodiment 60 or embodiment 61, wherein the interfering RNA is specific for the at least one target nucleotide sequence.
63. The set of nucleotide sequences according to any one of embodiments 53 or 55 to 62, wherein the set of nucleic acid sequences comprises multiple nucleic acid sequences encoding a plurality of interfering RNAs specific for multiple target nucleotide sequences.
64. The set of nucleotide sequences according to any one of embodiments 53 or 55 to 63, wherein the interfering RNA(s) promote cleavage of mRNA encoding the transgene.
65. The set of nucleotide sequences according to any one of embodiments 53 or 55 to 64, wherein the interfering RNA(s) target mRNA encoding the transgene for cleavage, preferably wherein the interfering RNA(s) target mRNA encoding the transgene for cleavage by the RISC
66. The set of nucleotide sequences according to any one of embodiments 53 or 55 to 65, wherein the interfering RNA is an siRNA; a sisiRNA; a tsiRNA; a RNA-DNA chimeric duplex; a tkRNA; a Dicer-substrate dsRNA; a shRNA; a tRNA-shRNA; an aiRNA; a miRNA; a pre-miRNA; a pri-miRNA mimic; a pri-miRNA mimic cluster; a transcriptional gene silencing (TGS); and/or combinations thereof, preferably wherein the interfering RNA is a siRNA, a shRNA and/or a miRNA.
67. The set of nucleotide sequences according to embodiment 66, wherein the guide strand of the miRNA is fully complementary to the target sequence of the transgene mRNA.
68. The set of nucleotide sequences according to embodiment 66 or embodiment 67, wherein the miRNA comprises a passenger strand which comprises at least one mismatch with its complimentary sequence within the RNA genome of the retroviral vector.
69. A nucleotide sequence comprising a lentiviral vector genome, wherein the lentiviral vector genome comprises a modified 3' LTR and a modified 5' LTR, wherein the modified 3' LTR comprises a modified polyadenylation sequence comprising a polyadenylation signal which is 5' of the R region within the modified 3' LTR, and wherein the modified 5' LTR comprises a modified polyadenylation sequence comprising a polyadenylation signal which is 5' of the R region within the modified 5' LTR.
70. A nucleotide sequence comprising a lentiviral vector genome, wherein the lentiviral vector genome comprises a modified 3' LTR and a modified 5' LTR, wherein the R region within the modified 3' LTR comprises at least one polyadenylation DSE wherein the R region within the modified 5' LTR comprises at least one polyadenylation DSE.
71. A nucleotide sequence comprising a lentiviral vector genome, wherein the lentiviral vector genome comprises a modified 3' LTR and a modified 5' LTR, wherein the modified 3' LTR comprises a modified polyadenylation sequence comprising a polyadenylation signal which is 5' of the R region within the modified 3' LTR and wherein the R region within the 3' LTR comprises at least one polyadenylation DSE, and wherein the modified 5' LTR comprises a modified polyadenylation sequence comprising a polyadenylation signal which is 5' of the R region within the 5' LTR and wherein the R region within the modified 5' LTR comprises at least one polyadenylation DSE.
72. The nucleotide sequence according to any one of embodiments 69 to 71, wherein the lentiviral vector genome further comprises:
   a) a transgene expression cassette as defined in any one of embodiments 4 or 30 to 44; and/or
   b) a transgene expression cassette and a vector intron as defined in embodiment 5 or embodiment 14.
73. The nucleotide sequence according to any one of embodiments 69 to 72, wherein the lentiviral vector genome is as defined in any one of embodiments 15 to 29, 46, 48 or 52.
74. The nucleotide sequence according to any one of embodiments 4 to 52 or 69 to 73, or the set of nucleotide sequences according to any one of embodiments 53 to 68, wherein the transgene gives rise to a therapeutic effect.
75. The nucleotide sequence according to any one of embodiments 4 to 52 or 69 to 74, or the set of nucleotide sequences according to any one of embodiments 53 to 68 or 74, wherein the lentiviral vector is derived from HIV-1, HIV-2, SIV, FIV, BIV, EIAV, CAEV or Visna lentivirus.
76. A lentiviral vector genome encoded by the nucleotide sequence according to any one of embodiments 1 to 52 or 69 to 75.
77. A lentiviral vector genome as defined in any one of embodiments 1 to 3 or 5 to 75.
78. An expression cassette comprising a nucleotide sequence according to any one of embodiments 1 to 52 or 69 to 75 or a nucleic acid sequence encoding an interfering RNA which is specific for mRNA encoding the transgene as defined in any one of embodiments 53 or 55 to 68.
79. A viral vector production system comprising the set of nucleotide sequences according to any one of embodiments 53 to 68.
80. A cell comprising the nucleotide sequence according to any one of embodiments 1 to 52 or 69 to 75, the set of nucleotide sequences according to any one of embodiments 53 to 68, the expression cassette according to embodiment 78 or the viral vector production system according to embodiment 79.
81. A cell for producing lentiviral vectors comprising:
   (a)
      (i) nucleotide sequences encoding vector components including gag-pol and env, and optionally rev, and the nucleotide sequence according to any one of embodiments 1 to 52 or 69 to 75 or the expression cassette according to embodiment 78, or the set of nucleotide sequences according to any one of embodiments 53 to 68; or
      (ii) the viral vector production system according to embodiment 79; and
   (b) optionally, a nucleotide sequence encoding a modified U1 snRNA and/or optionally a nucleotide sequence encoding TRAP.
82. A method for producing a lentiviral vector, comprising the steps of:
   (a) introducing:
      (i) nucleotide sequences encoding vector components including gag-pol and env, and optionally rev, and the nucleotide sequence according to any one of embodiments 1 to 52 or 69 to 75 or the expression cassette according to embodiment 78, or the set of nucleotide sequences according to any one of embodiments 53 to 68; or
      (ii) the viral vector production system according to embodiment 79,
         into a cell; and
   (b) optionally selecting for a cell that comprises nucleotide sequences encoding vector components and the RNA genome of the lentiviral vector; and
   (c) culturing the cell under conditions suitable for the production of the lentiviral vector.
83. A lentiviral vector produced by the method according to embodiment 82.
84. Use of the nucleotide sequence according to any one of embodiments 1 to 52 or 69 to 75, the set of nucleotide sequences according to any one of embodiments 53 to 68, the expression cassette according to embodiment 78, the viral vector production system according to embodiment 79, or the cell according to embodiment 80 or embodiment 81, for producing a lentiviral vector.
85. A lentiviral vector comprising the lentiviral vector genome as defined in any one of embodiments 1 to 3 or 5 to 75 or the lentiviral vector genome according to embodiment 76 or embodiment 77.

## Claims

1. A nucleotide sequence comprising a lentiviral vector genome expression cassette, wherein:
a) the 3' LTR of the lentiviral vector genome comprises a modified polyadenylation sequence, and wherein the modified polyadenylation sequence comprises a polyadenylation signal which is 5' of the 3' LTR R region; and/or
b) the lentiviral vector genome comprises a modified 5' LTR, and wherein the R region of the modified 5' LTR comprises at least one polyadenylation downstream enhancer element (DSE).

2. The nucleotide sequence according to claim 1, wherein the lentiviral vector genome further comprises:
a) a transgene expression cassette, wherein the 3' UTR of the transgene expression cassette comprises at least one cis-acting sequence selected from:
(i) a cis-acting Cytoplasmic Accumulation Region (CAR) sequence; and/or
(ii) a cis-acting ZCCHC14 protein-binding sequence;
and/or
b) a transgene expression cassette and a vector intron.

3. The nucleotide sequence according to claim 1 or claim 2, wherein:
a) the transgene expression cassette is:
(i) in the forward orientation with respect to the lentiviral vector genome expression cassette; or
(ii) the transgene expression cassette is inverted with respect to the lentiviral vector genome expression cassette;
b) the vector intron comprises one or more transgene mRNA self-destabilization or self-decay element(s) or one or more transgene mRNA nuclear retention signal(s), preferably wherein the vector intron comprises one or more transgene mRNA self-destabilization or self-decay element(s);
c) the vector intron is not located between the promoter of the transgene expression cassette and the transgene;
d) the 3' UTR of the transgene expression cassette comprises the vector intron;
e) the major splice donor site in the lentiviral vector genome is inactivated, preferably wherein the nucleotide sequence comprises a sequence as set forth in any of SEQ ID NOs: 2, 3, 4, 6, 7, and/or 8, and/or the sequences CAGACA, and/or GTGGAGACT;
f) the cryptic splice donor site adjacent to the 3' end of the major splice donor site in the lentiviral vector genome is inactivated;
g) the lentiviral vector genome does not comprise a rev-response element (RRE);
h) the native 3' polyadenylation sequence has been mutated or deleted, preferably wherein the native 3' polyadenylation signal has been mutated or deleted;
i) the modified polyadenylation sequence is a heterologous polyadenylation sequence, preferably wherein the modified polyadenylation sequence comprises a heterologous polyadenylation signal;
j) the modified polyadenylation sequence further comprises a polyadenylation upstream enhancer element (USE), preferably wherein the USE is 5' of the polyadenylation signal;
k) the modified polyadenylation sequence further comprises a GU rich downstream enhancer element, preferably wherein the GU rich downstream enhancer element is 3' of the polyadenylation signal;
l) the polyadenylation signal which is 5' of the 3' LTR R region is in the sense strand, preferably wherein the 3' LTR further comprises a polyadenylation signal in the antisense strand which is:
(i) 5' of the polyadenylation signal in the sense strand; and
(ii) 3' of the attachment sequence of the 3' LTR;
m) the 3' LTR R region of the modified polyadenylation sequence is a minimal R region;
n) the 3' LTR R region of the modified polyadenylation sequence is homologous to the native 5' LTR R region of the lentiviral vector genome; and/or
o) the modified polyadenylation sequence further comprises a polyadenylation cleavage site, preferably wherein the polyadenylation cleavage site is 3' of the polyadenylation signal.

4. The nucleotide sequence according to any one of claims 2 or 3, wherein:
a) the first 55 nucleotides of the modified 5' LTR comprises the polyadenylation DSE, preferably wherein the first 40 nucleotides of the modified 5' LTR comprises the at least one polyadenylation DSE;
b) the polyadenylation DSE is comprised within a stem loop structure of the modified 5' LTR;
c) the polyadenylation DSE is a GU-rich sequence, preferably wherein the GU-rich sequence is derived from RSV or MMTV, or wherein the GU-rich sequence is synthetic;
d) the native polyadenylation signal of the modified 5' LTR has been mutated or deleted;
e) the 5' R region of the modified 5' LTR further comprises a polyadenylation cleavage site;
f) the CAR sequence comprises a plurality of CARe sequences, preferably wherein the plurality of CARe sequences are in tandem;
g) the CAR sequence comprises at least two, at least four, at least six, at least eight, at least ten, at least twelve, at least fourteen, at least sixteen, at least eighteen, or at least twenty CARe sequences, optionally wherein the CARe sequences are in tandem;
h) the CAR sequence comprises at least six CARe sequences in tandem, least eight CARe sequences in tandem, at least ten CARe sequences in tandem, at least twelve CARe sequences in tandem, or at least sixteen CARe sequences in tandem;
i) the CARe nucleotide sequence is BMWGHWSSWS (SEQ ID NO: 92) or BMWRHWSSWS (SEQ ID NO: 243), preferably wherein the CARe nucleotide sequence is CMAGHWSSTG (SEQ ID NO: 96);
j) the CARe nucleotide sequence is selected from the group consisting of: CCAGTTCCTG (SEQ ID NO: 97), CCAGATCCTG (SEQ ID NO: 97), CCAGTTCCTC (SEQ ID NO: 99), TCAGATCCTG (SEQ ID NO: 100), CCAGATGGTG (SEQ ID NO: 101), CCAGTTCCAG (SEQ ID NO: 102), CCAGCAGCTG (SEQ ID NO: 103), CAAGCTCCTG (SEQ ID NO: 104), CAAGATCCTG (SEQ ID NO: 105), CCTGAACCTG (SEQ ID NO: 106), CAAGAACGTG (SEQ ID NO: 107), TCAGTTCCTG (SEQ ID NO: 227), GCAGTTCCTG (SEQ ID NO: 228), CAAGTTCCTG (SEQ ID NO: 229), CCTGTTCCTG (SEQ ID NO: 230), CCTGCTCCTG (SEQ ID NO: 231), CCTGTACCTG (SEQ ID NO:232), CCTGTTGCTG (SEQ ID NO: 233), CCTGTTCGTG (SEQ ID NO: 234), CCTGTTCCAG (SEQ ID NO: 235), CCTGTTCCTC (SEQ ID NO: 236), CCAATTCCTG (SEQ ID NO: 237) and GAAGCTCCTG (SEQ ID NO: 238); preferably wherein the CARe nucleotide sequence is CCAGTTCCTG (SEQ ID NO: 97), CCTGTTCCTG (SEQ ID NO: 230), CCTGTACCTG (SEQ ID NO: 232), CCTGTTCCAG (SEQ ID NO: 235), CCAATTCCTG (SEQ ID NO: 237), CCTGAACCTG (SEQ ID NO: 106), CCAGTTCCTC (SEQ ID NO: 99) and CCAGTTCCAG (SEQ ID NO: 102);
k) the 3' UTR of the transgene expression cassette does not comprise additional post-transcriptional regulatory elements (PREs);
l) the 3' UTR of the transgene expression cassette comprises at least one additional post-transcriptional regulatory element (PRE), preferably wherein the additional PRE is a Woodchuck hepatitis virus PRE (wPRE);
m) the cis-acting ZCCHC14 protein-binding sequence is a PRE α element fragment preferably wherein the cis-acting ZCCHC14 protein-binding sequence is:
(i) a fragment of a HBV PRE α element; or
(ii) a fragment of HCMV RNA 2.7; or
(iii) a fragment of a wPRE α element;
n) the cis-acting ZCCHC14 protein-binding sequence is a PRE α element fragment that is is no more than 200 nucleotides in length, preferably wherein the PRE α element fragment is no more than 90 nucleotides in length;
o) the CNGGN-type pentaloop sequence is comprised within a stem-loop structure having a sequence selected from the group consisting of:
(i) TCCTCGTAGGCTGGTCCTGGGGA (SEQ ID NO: 108); and
(ii) GCCCGCTGCTGGACAGGGGC (SEQ ID NO: 109);
p) the CNGGN-type pentaloop sequence is comprised within a heterologous stem-loop structure;
q) the ZCCHC14 protein-binding sequence comprises a sequence selected from the group consisting of:
(i) 
TGCCGTCGCCACCGCGTTATCCGTTCCTCGTAGGCTGGTCCTGGGGAA
CGGGTCGGCGGCCGGTCGGCTTCT (SEQ ID NO: 110);
 and
(ii) 
CTATTGCCACGGCGGAACTCATCGCCGCCTGCCTTGCCCGCTGCTGGA
CAGGGGCTCGGCTGTTGGGCACTGACAATTCCGTGGTGTTGT (SEQ ID
NO: 111);
r) the 3' UTR of the transgene expression cassette comprises a cis-acting CAR sequence and a cis-acting ZCCHC14 protein-binding sequence, optionally wherein the ZCCHC14 protein-binding sequence is located 3' to the CAR sequence;
s) wherein the 3' UTR of the transgene expression cassette comprises at least two spatially distinct cis-acting CAR sequences and/or at least two spatially distinct *cis-*acting ZCCHC14 protein-binding sequences;
t) the 3' UTR of the transgene expression cassette further comprises a polyA sequence located 3' to the cis-acting CAR sequence and/or cis-acting ZCCHC14 protein-binding sequence;
u) the transgene expression cassette further comprises a promoter operably linked to the transgene; and/or
v) the lentiviral vector genome further comprises a tryptophan RNA-binding attenuation protein (TRAP) binding site.

5. The nucleotide sequence according to any one of the preceding claims, wherein:
a) the nucleotide sequence further comprises a nucleotide sequence encoding a modified U1 snRNA, wherein said modified U1 snRNA has been modified to bind to a nucleotide sequence within the packaging region of the lentiviral vector genome, preferably the nucleotide sequence encoding the lentiviral vector genome is operably linked to the nucleotide sequence encoding the modified U1 snRNA;
b) the lentiviral vector genome comprises at least one modified viral cis-acting sequence, wherein at least one internal open reading frame (ORF) in the viral cis-acting sequence is disrupted, preferably wherein the at least one viral cis-acting sequence is a Woodchuck hepatitis virus (WHV) post-transcriptional regulatory element (WPRE) and/or a Rev response element (RRE), optionally wherein the at least one internal ORF is disrupted by mutating at least one ATG sequence, preferably the first ATG sequence;
c) the lentiviral vector genome comprises a modified nucleotide sequence encoding gag, and wherein at least one internal open reading frame (ORF) in the modified nucleotide sequence encoding gag is disrupted, optionally wherein the at least one internal ORF is disrupted by mutating at least one ATG sequence, preferably the first ATG sequence;
d) the lentiviral vector genome lacks (i) a nucleotide sequence encoding Gag-p17 or (ii) a fragment of a nucleotide sequence encoding Gag-p17, preferably wherein the fragment of a nucleotide sequence encoding Gag-p17 comprises a nucleotide sequence encoding p17 instability element;
e) the nucleotide sequence comprising a lentiviral vector genome does not express Gag-p17 or a fragment thereof, preferably wherein said fragment of Gag-p17 comprises the p17 instability element;
f) the transgene gives rise to a therapeutic effect; and/or
g) the lentiviral vector is derived from HIV-1, HIV-2, SIV, FIV, BIV, EIAV, CAEV or Visna lentivirus.

6. A set of nucleotide sequences for producing a lentiviral vector comprising:
a) the nucleotide sequence according to any one of claims 1 to 5, wherein the lentiviral vector genome expression cassette further comprises a transgene expression cassette; and
b) nucleotide sequences encoding lentiviral vector components.

7. The set of nucleotide sequences according to claim 6, wherein:
a) the transgene expression cassette comprises at least one cis-acting sequence as defined in claim 4;
b) the 3' UTR or the 5' UTR of the transgene expression cassette comprises at least one target nucleotide sequence;
c) the transgene expression cassette is genetically engineered to comprise at least one target nucleotide sequence within the 3' UTR or 5' UTR;
d) the transgene gives rise to a therapeutic effect; and/or
e) the lentiviral vector is derived from HIV-1, HIV-2, SIV, FIV, BIV, EIAV, CAEV or Visna lentivirus.

8. A nucleotide sequence comprising a lentiviral vector genome, wherein:
a) the lentiviral vector genome comprises a modified 3' LTR and a modified 5' LTR, wherein the modified 3' LTR comprises a modified polyadenylation sequence comprising a polyadenylation signal which is 5' of the R region within the modified 3' LTR, and wherein the modified 5' LTR comprises a modified polyadenylation sequence comprising a polyadenylation signal which is 5' of the R region within the modified 5' LTR;
b) the lentiviral vector genome comprises a modified 3' LTR and a modified 5' LTR, wherein the R region within the modified 3' LTR comprises at least one polyadenylation DSE wherein the R region within the modified 5' LTR comprises at least one polyadenylation DSE; or
c) the lentiviral vector genome comprises a modified 3' LTR and a modified 5' LTR, wherein the modified 3' LTR comprises a modified polyadenylation sequence comprising a polyadenylation signal which is 5' of the R region within the modified 3' LTR and wherein the R region within the 3' LTR comprises at least one polyadenylation DSE, and wherein the modified 5' LTR comprises a modified polyadenylation sequence comprising a polyadenylation signal which is 5' of the R region within the 5' LTR and wherein the R region within the modified 5' LTR comprises at least one polyadenylation DSE.

9. A lentiviral vector genome as defined in any one of claims 1 to 8.

10. An expression cassette comprising a nucleotide sequence according to any one of claims 1 to 5 or 8.

11. A viral vector production system comprising the set of nucleotide sequences according to claim 6 or claim 7.

12. A cell comprising the nucleotide sequence according to any one of claims 1 to 5 or 8, the set of nucleotide sequences according to claim 6 or claim 7, the expression cassette according to claim 10 or the viral vector production system according to claim 9, optionally, wherein the cell also comprises a nucleotide sequence encoding a modified U1 snRNA and/or optionally a nucleotide sequence encoding TRAP.

13. A method for producing a lentiviral vector, comprising the steps of:
(a) introducing:
(i) nucleotide sequences encoding vector components including gag-pol and env, and optionally rev, and the nucleotide sequence according to any one of claims 1 to 5 or 8, or the expression cassette according to claim 10, or the set of nucleotide sequences according to claim 6 or claim 7; or
(ii) the viral vector production system according to claim 11,
into a cell; and
(b) optionally selecting for a cell that comprises nucleotide sequences encoding vector components and the RNA genome of the lentiviral vector; and
(c) culturing the cell under conditions suitable for the production of the lentiviral vector.

14. Use of the nucleotide sequence according to any one of claims 1 to 5 or 8, the set of nucleotide sequences according to claim 6 or claim 7, the expression cassette according to claim 10, the viral vector production system according to claim 11, or the cell according to claim 12, for producing a lentiviral vector.

15. A lentiviral vector comprising the lentiviral vector genome as defined in any one of claims 1 to 8 or the lentiviral vector genome according to claim 9.
